# EUROPEAN PATENT APPLICATION

(11) **EP 1 715 036 A1**
(43) Date of publication of application: **25.10.2006**
(21) Application number: 05008732.9
(22) Date of filing: 20.04.2005
(51) Int. Cl.: C12N 9/12

(54) **Crystal structure of an Aurora-B/INCENP complex**

(71) Applicant: Boehringer Ingelheim International GmbH, 55216 Ingelheim (DE)
(72) Inventor: Boehmelt, Guido, 2531 Gaaden (AT); Musacchio, Andrea, p.A. European Inst. of Oncology, 20141 Milan (IT); Sessa, Fabio, p.A. European Inst. of Oncology, 20141 Milan (IT)
(74) Representative: Hammann, Heinz

(57) **Abstract**

The present invention provides crystalline molecules or molecular complexes which comprise binding sites of Aurora-B-like polypeptides. The invention also relates to the structural coordinates of Aurora-B/INCENP complexes and methods of using computational means to evaluate the ability of compounds to bind to the molecule or molecular complexes. This invention also provides methods of using the structure coordinates to solve the structure of homologous proteins or protein complexes. In addition, this invention provides methods of using the structure coordinates to screen and design compounds, including inhibitory compounds that bind to Aurora-B-like polypeptides.

## Description

The present invention relates to compositions and crystals of an Aurora-B kinase in complex with INCENP. This invention also relates to methods of using the structure coordinates of Aurora-B kinase in complex with INCENP to solve the structure of similar or homologous proteins or protein complexes and to identify inhibitors of Aurora-B kinase or homologues thereof.

### BACKGROUND OF THE INVENTION

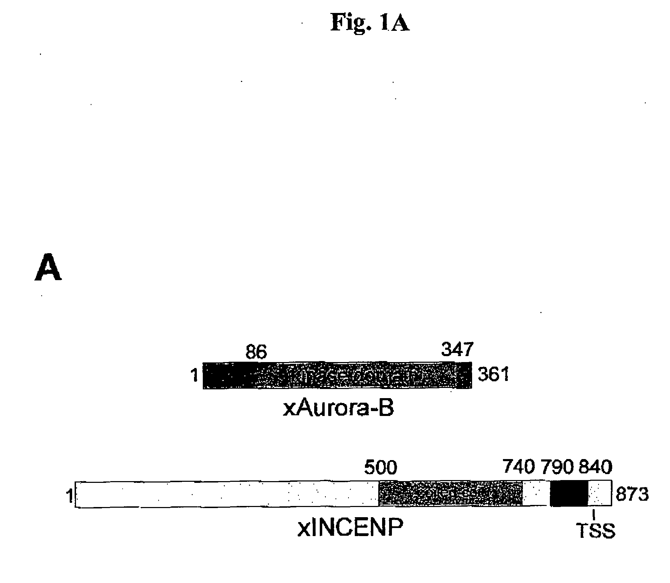

Serine/threonine kinases of the Aurora family regulate many processes in cell division, including chromosome condensation, spindle dynamics, kinetochore-microtubule interactions, chromosome orientation, the establishment of the metaphase plate, and cytokinesis (Andrews, 2003; Carmena, 2003; Meraldi, 2004). In mammals, three members of the Aurora family, known as A, B, and C have been identified. A- and B-type Aurora kinases also exist in *Caenorhabditis elegans* and *Drosophila melanogaster,* while *Saccharomyces cerevisiae* and *Schizosaccharomyces pombe* have single Aurora genes known as *IPL1* and *ARK1,* respectively. All Aurora proteins share a similar structure, with a variable N-terminal domain, followed by a well conserved catalytic domain and a short C-terminal extension (Figure 1A).

In spite of high sequence similarity, mammalian Aurora family members display different subcellular localization and have specialized functions (Andrews, 2003; Carmena, 2003; Meraldi, 2004). Aurora-A localizes to centrosomes in interphase and to centrosomcs and spindle microtubules near the poles during mitosis. Aurora-A is implicated in centrosome maturation and separation (Andrews, 2003; Carmena, 2003; Mcraldi, 2004). There arc multiple activators of Aurora-A including TPX2, Ajuba and Inhibitor-2 (Bayliss, 2003; Eyers, 2004; Kufer, 2002; Satinover, 2004; Tsai, 2003). The best characterized of these is TPX2, which localizes Aurora-A to spindle microtubules proximal to the poles and is thought to specifically activate it there (Kufer, 2002; Tsai, 2003).

Aurora-B first associates with centromeres, then relocalizes to the midzone of the central spindle and finally concentrates at the midbodybetween dividing cells, which characterizes Aurora-B as a so-called chromosomal passenger protein (Andrews, 2003; Carmena, 2003; Meraldi, 2004). At least three more chromosomal passenger proteins, INCENP (inner centromere protein), survivin, and borcalin participate in a multi protein complex (the chromosomal passenger complex) with Aurora B (Andrews, 2003; Carmena, 2003; Meraldi, 2004). A well-defined interaction within this complex involves the C-terminal segment of INCENP, the IN-box (Adams, 2000; Bishop, 2002; Kaitna, 2000). The IN-box is the most strongly conserved region of INCENP. It binds and activates Aurora-B, and is phosphorylated by this kinase (Bishop, 2002; Bolton, 2002; Honda, 2003; Kang, 2001).

Aurora-C, the least characterized Aurora family member in mammals, has recently been shown also to interact with INCENP and has overlapping function with Aurora-B (Li, 2004; Sasai, 2004).

Aurora-B phosphorylates histone H3 on Ser1 0 and is required to both define the cleavage furrow and for cytokinesis when it phosphorylates substrates that include MgcRacGrAY, vimcntin, desmin, myosin II regulatory light chain, GFAP and centralspindlin (Andrews, 2003; Carmena, 2003; Meraldi, 2004). Aurora-B recruits several proteins to the kinetochore, including components of the spindle assembly checkpoint (Ditchfield, 2003; Murata-hlori, 2002; Vigneron, 2004). Aurora-B also seems to play a direct role in a pathway that senses and corrects syntelic microtubule/kinetochore attachments, in which both sister kinetochores are attached to microtubules from the same spindle pole (Andrews, 2003; Carmena, 2003; Meraldi, 2004). This condition might be a cause of chromosome segregation errors and must be corrected by destabilizing improper microtubulekinetochore interactions. The phosphorylation of the microtubule depolymerase MCAK by Aurora-B has been implicated as a putative mechanism of correction (Gorbsky, 2004).

The activation of Aurora kinases is a multistep process. The mechanisms of Aurora-A activation have been revealed by crystal structures of Aurora-A and of the Aurora-A/TPX2 complex (Bayliss, 2003; Cheetham, 2002; Nowakowski, 2002). The N-terminal segment of TPX2 binds near the small lobe of Aurora-A, at a site that is roughly equivalent to the site recognized by cyclins on their cyclin-dependent kinase (CDK) targets (Pavletich, 1999). Differently from the effect of cyclin binding on their CDK partners, TPX2 does not impart a large-scale conformational rearrangement of Aurora-A. The effects of its intervention, however, are sufficient to form an extended, active conformation of the activation loop of Aurora-A, which needs to be phosphorylated on the conserved residue Thr288, equivalent to Thr248 of Xcnopus laevis (x) Aurora-B (Bayliss, 2003; Walter, 2000). Both sites arc embedded in sequences that fit the consensus target sequence for Aurora kinases (R/K)X(T/S)(I/L/V) (Bishop, 2002; Bolton, 2002; Cheeseman, 2002; Honda, 2003) and represent autophosphoryiation sites (Bayliss, 2003; Yasui, 2004). It is believed that kinase activity is restricted by interaction with PP1, which removes this activation loop phosphate until activator binding (Katayama, 2001; Tsai, 2003).

Given the high sequence similarity between Aurora-A and Aurora-B (57% overall identity for the human proteins), and more generally the structural similarity among active kinases, the structure of Aurora-A/TPX2 must be regarded as a good model for the fully active Aurora-B/INCENP complex (Bayliss, 2003). Recently, however, evidence has emerged that the activation of Aurora-B by INCENP is a two-step process in which binding of INCENP first stimulates the activity of Aurora-B, whilst full activation of Aurora-B requires the phosphorylation of INCENP on a conserved Tbr-Ser-Ser (TSS) motif near the C-terminus (Bishop, 2002; Bolton, 2002; Honda, 2003).

The International Patent Application WO 03/092607 discloses the crystal structure of the Aurora A protein and binding pockets thereof. Crystalline forms of a polypeptide corresponding to the catalytic domain of Aurora-A kinase are described in WO 03/031606.

The Aurora B kinase is considered a potential target for antimitotic agents. However, drug discovery efforts directed towards the Aurora-B protein have been hampered by the lack of structural information about Aurora-B and its complex with INCENP. Such structural information would provide valuable information in discovery of Aurora-B kinase inhibitors. There have been no crystals reported of any Aurora-B domain protein. Thus, X-ray crystallographic analysis of such proteins has not been possible

### BRIEF SUMMARY OF THE INVENTION

The present invention relates to the previously unknown three-dimensional structure of a complex of Aurora-B with INCENP. The invention also provides methods for screening, designing, optimizing, evaluating and identifying compounds which bind to Aurora-B or to an Aurora-B/INCENP complex or binding pockets thereof.

### BRIEF DESCRIPTION OF THE DRAWINGS

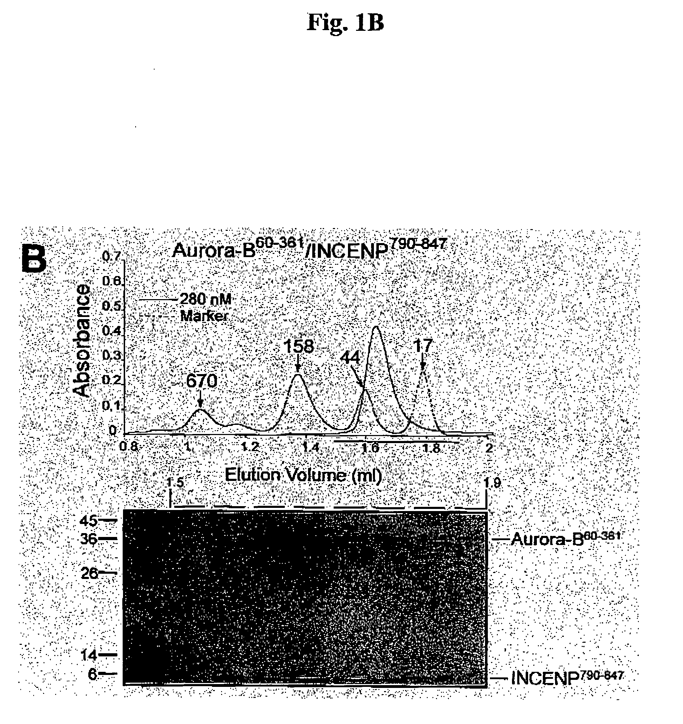

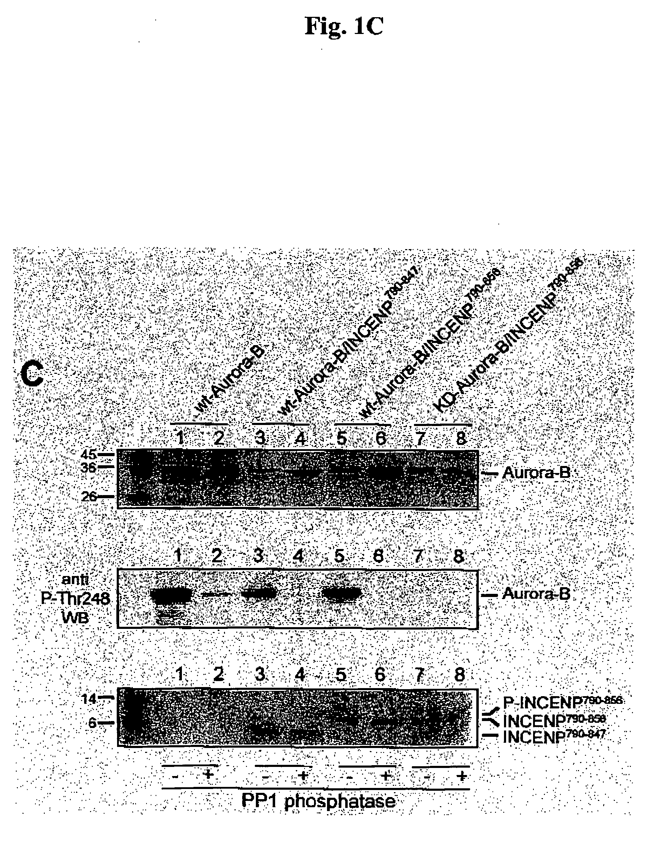

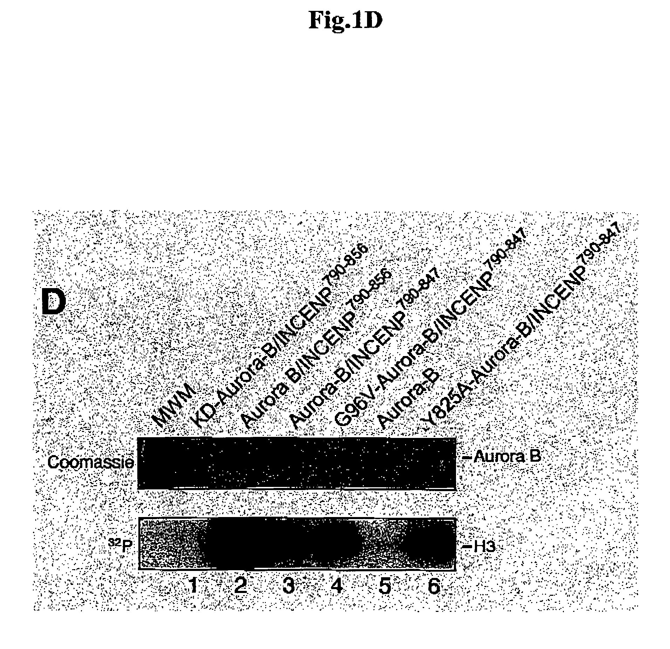

**Figure 1** Characterization of the Aurora-B/IN box complex
   A) Schematic view of xAurora-B and INCENP.
   B) Elution profile of Aurora-B/INCENP790-847 from a Superdex-200 PC 3.2/30 size-exclusion chromatography column. The content of eight 50 µl fractions between 1.5 and 1.9ml is shown.
   C) Phosphorylation of Aurora-B/INCENP. Upper panel: 17.5 % Coomassie-stained polyacrylamide gel of the indicated Aurora-B and Aurora-B/INCENP samples.
      Middle panel: Western blotting with an antibody against phospho-Thr248. Aurora-B (lanes 1 and 2) is phosphorylated on Thr248 even in the absence of INCENP. Treatment with PP1 phosphatase removes phosphorylation on Thr248.
      Bottom panel: The mobility of INCENP790-856 (lanes 5 and 6) is modified by PP1 treatment.
   D) The indicated samples of Aurora-B are incubated in a kinase assay with histone H3 and analysed using autoradiography. Upper panel: loading control.
      Lower panel: a representative example of kinase assay. Aurora-B/lNCENP790-856 consistently shows a ~7-fold activation relative to the complex containing unphosphorylated INCENP.

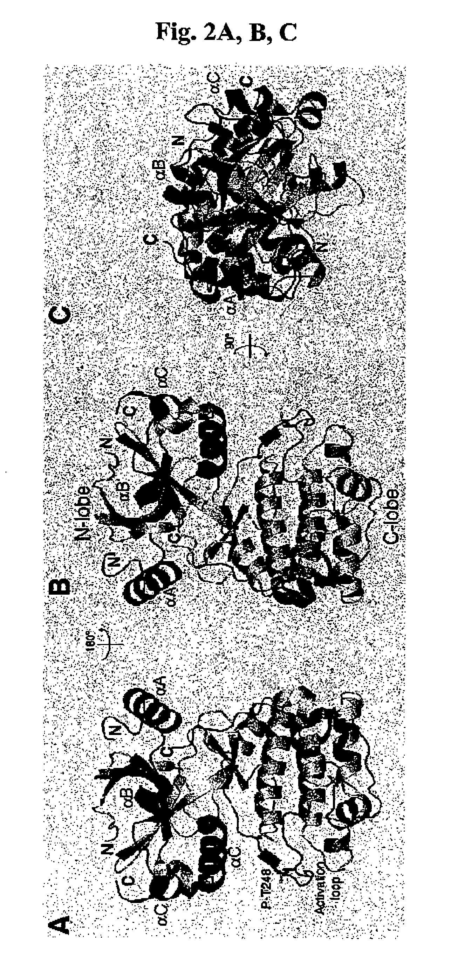

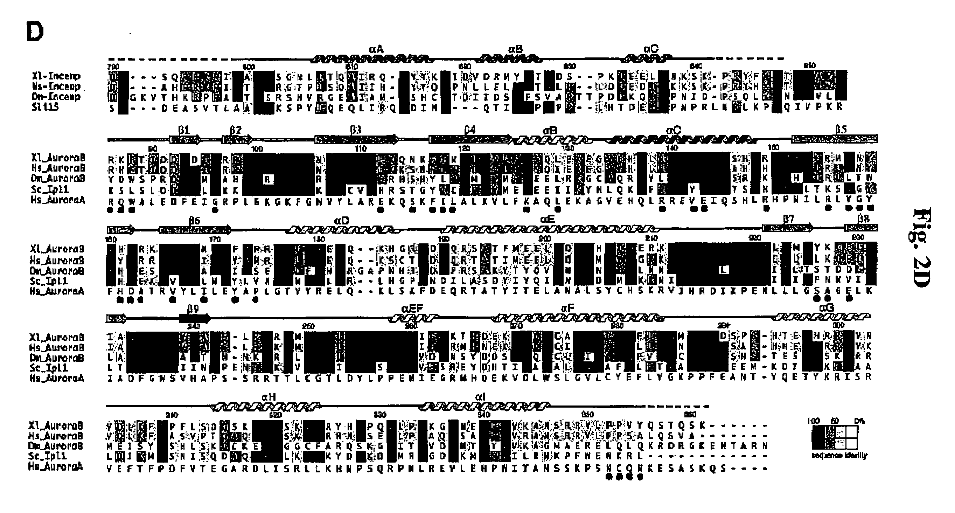

**Figure 2** Overall view of the Aurora-B/INCENP complex
   A to C) Three views related by 90° rotations of Aurora-B/INCENP790-847
      The main features of Aurora-B (light grey) are shown, including activation segment, phosphorylated Thr248 (P-T248), C-terminal tail, and αC helix (αC, horizontal helix, not labeled in the 180°C rotated view). INCENP (dark grey) forms a crown around the N-lobe including segments αA, αB, αC. N and C represent N- and C-termini, respectively.
   D) Alignment of Aurora and INCENP sequences with secondary structure.
      The alignment is shaded based on conservation measured on a larger alignment of 7 Aurora-B and 8 INCENP sequences. Aurora-B residues contacting INCENP are marked with a grey dot.

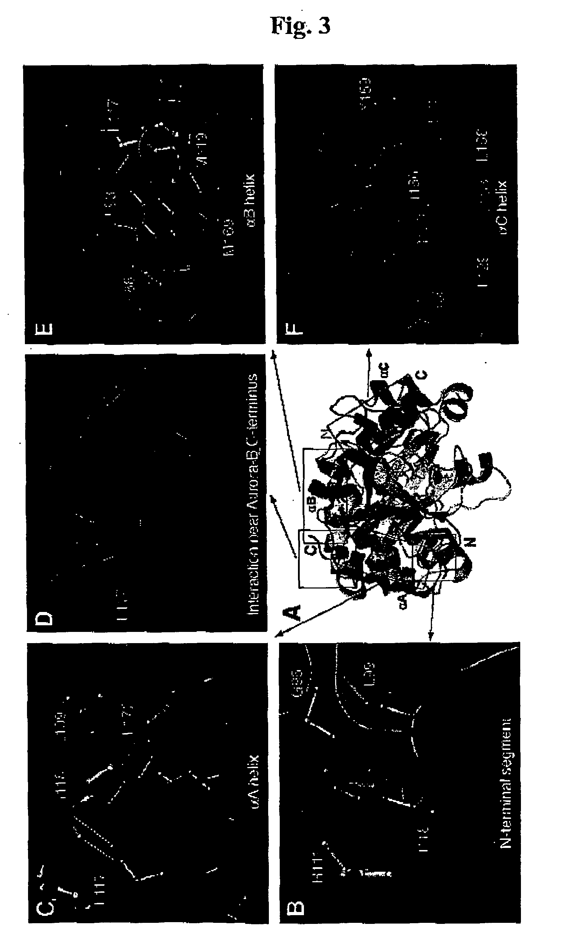

**Figure 3** Details of the Aurora-B/IN-box interaction
   A) The Aurora-B/INCENP complex is viewed as in Panel 2C.
   B-F) Close-ups of the interaction of INCENP790-847 with Aurora-B. The color scheme used in Figure 2 is maintained. The INCENP chain is shown from N- to C-terminus in subsequent snapshots as indicated in panel A. To preserve clarity, only the key contacts are shown.

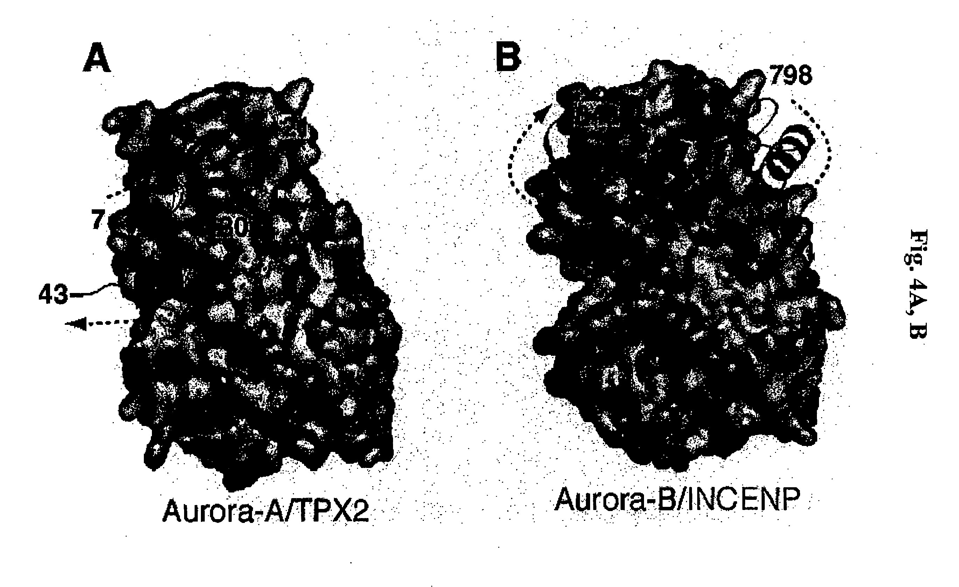

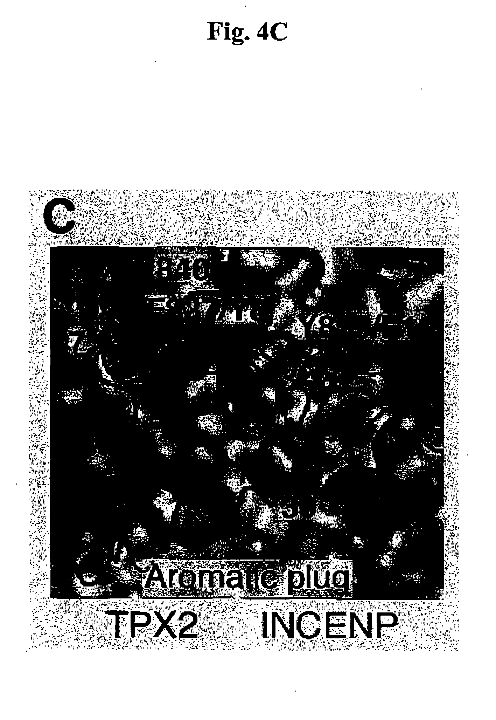

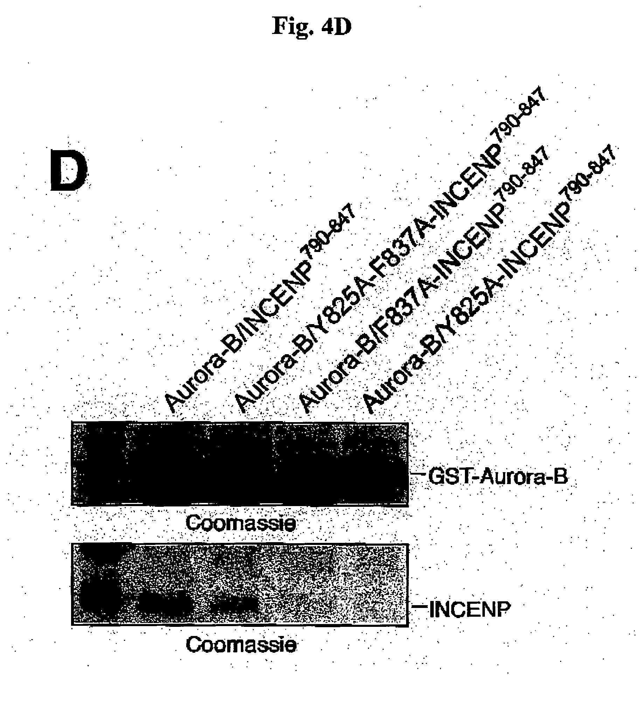

**Figure 4** Comparison of Aurora-B/INCENP and Aurora-A/TPX2
   A) Surface of Aurora-A with ribbon model of TPX2 (dark grey). The dashed arrow represents the direction of the TPX2 chain.
   B) Aurora-B/INCENP is shown in the same orientation and style of the Aurora-A/TPX2 shown in A. The dashed arrow shows the direction of the INCENP chain.
   C) The main chains of TPX2 (dark grey) and INCENP (light grey) arc shown on the surface of Aurora-B. The position of TPX2 was derived from the superposition of the N-lobes of Aurora-A and Aurora-B. Tyr825/Phe837 of INCENP and Tyr8/Phe19 of TPX2 occupy similar positions on their cognate kinases despite opposite directions of the main chains.
   D) GST-Aurora-B is co-expressed with INCENP790-847 or the indicated mutants. The Coomassie-stained gels indicate the amounts of GST-Aurora-B purified by affinity chromatography using glutathione-agarose beads (upper panel) and the amount of co-purifying INCENP (lower panel).

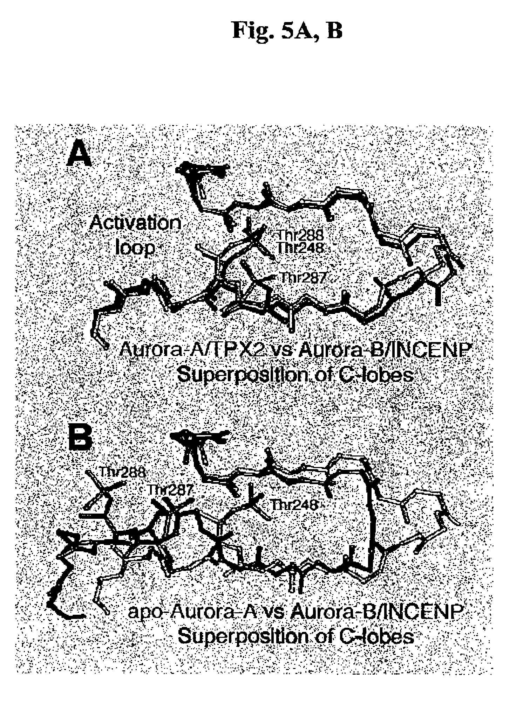

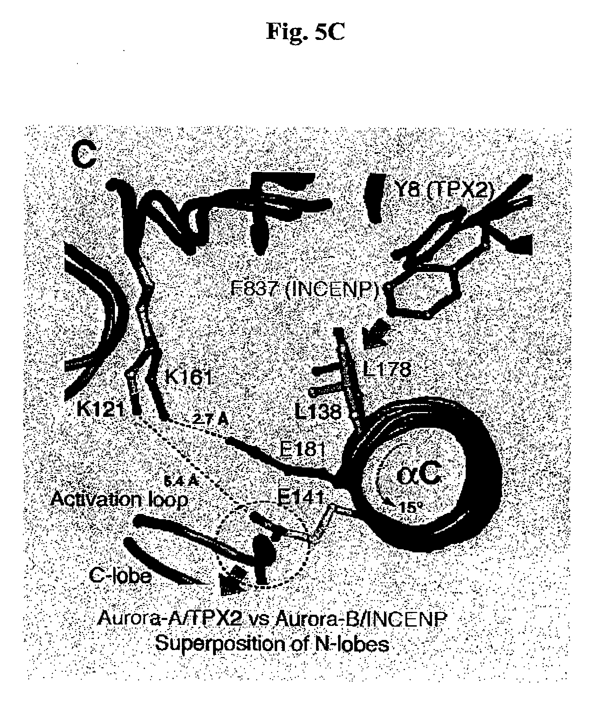

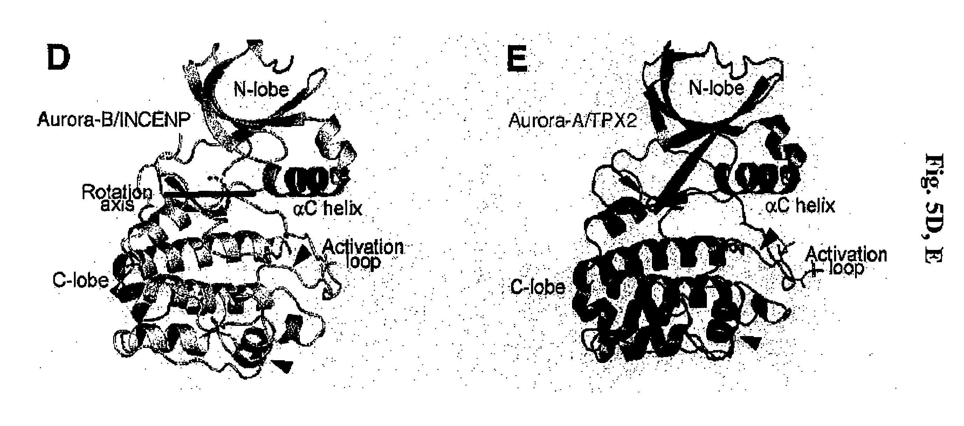

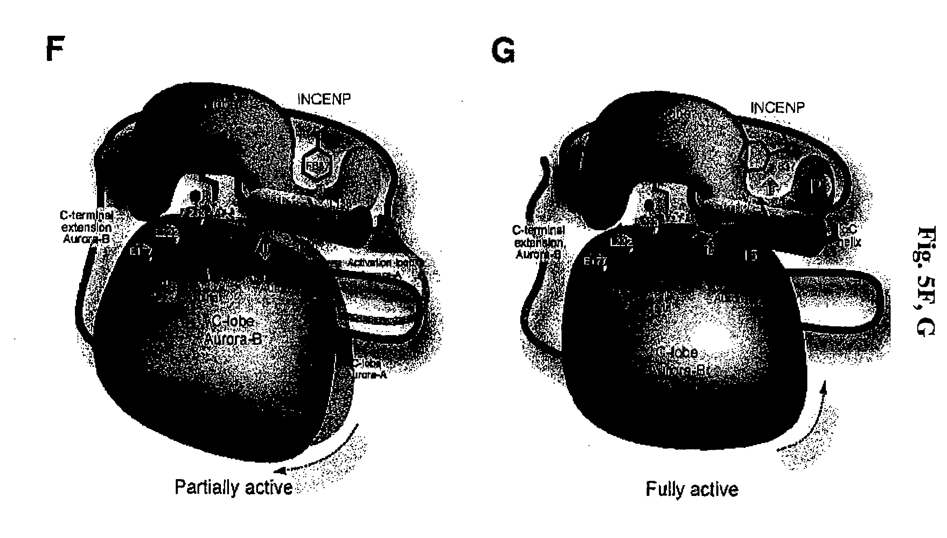

**Figure 5** Activation mechanism of Aurora-B
   A) The activation loop in Aurora-B/INCENP is phosphorylated on Thr248 and is essentially identical to that of Aurora-A/TPX2, which is doubly phosphorylated on Thr288 (equivalent to Tht248 Aurora-B) and Thr287.
   B) A simi lar comparison with the activation loop of doubly phosphorylated apo-Aurora-A shows poor fitting.
   C) Superposition of the N-lobes of Aurora-B/INCENP and Aurora-A/TPX2. K122Aurora-B and K161Aurora-A occupy similar positions. E141Aurora-B is rotated away from the position of E181Aurora-A, and its distance from K122Aurora-B in ~5.4 Å. The rotation depends on F837INCENP, which pushes against L138. The new position of E141Aurora-B is incompatible with the small interlobar angle of Aurora-A/TPX2, as there would be a clash of the E141 side chain with the activation loop (dashed circle). As a consequence, the two lobes of Aurora-B rotate.
   D) The catalytic cleft of Aurora-B/INCENP is about 15° more open than in Aurora-A/TPX2, shown in E). The approximate position of a rotation axis describing the rotation is shown.
      The arrowheads in panels D and E indicate equivalent points in the two structures that reveal the rotation.
   F) A model describing the state of activation of Auroza-B/TNCENP in the absence of INCENP phosphorylation. The activation loop is fully stretched. F837 pushes on L138 to rotate the αC helix. The rotated E141 is unable to bind K122 and pushes on the activation loop causing the opening of the cleft. The C-terminal extension of Aurora-B stabilizes this conformation.
   G) The phosphorylation of the TSS motif of INCENP releases the pressure of F837. The αC helix rotates back in its normal position. This causes the closure of the cleft and the release of the C-terminal tail. Restoration of the E141/K122 interaction elicits full activation.

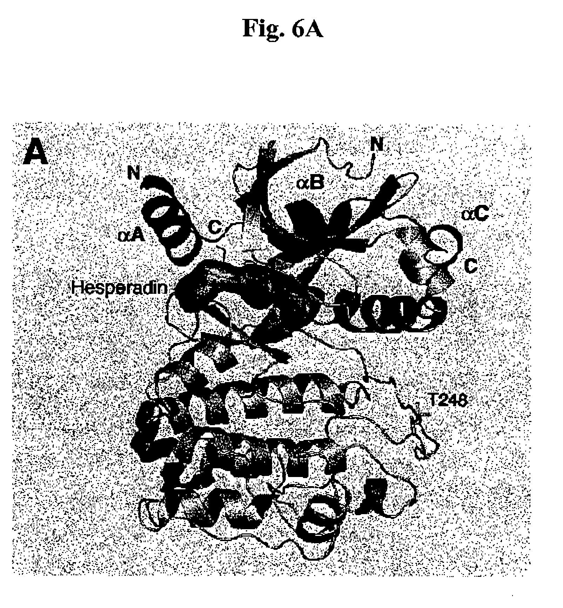

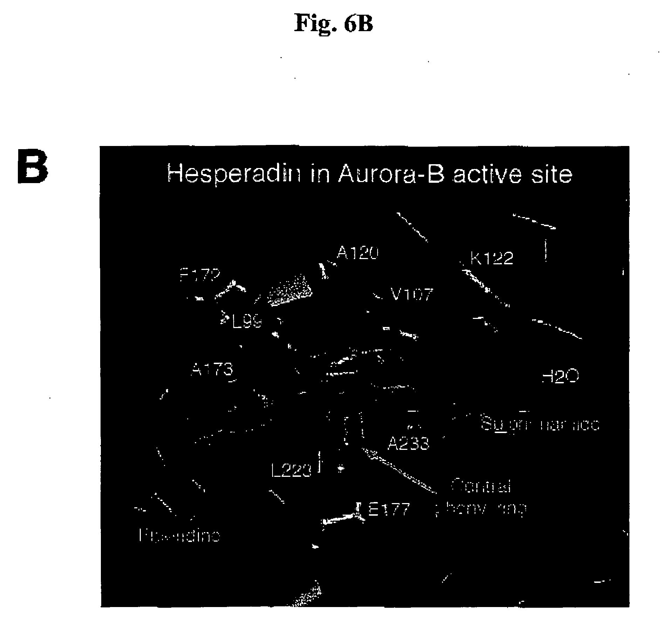

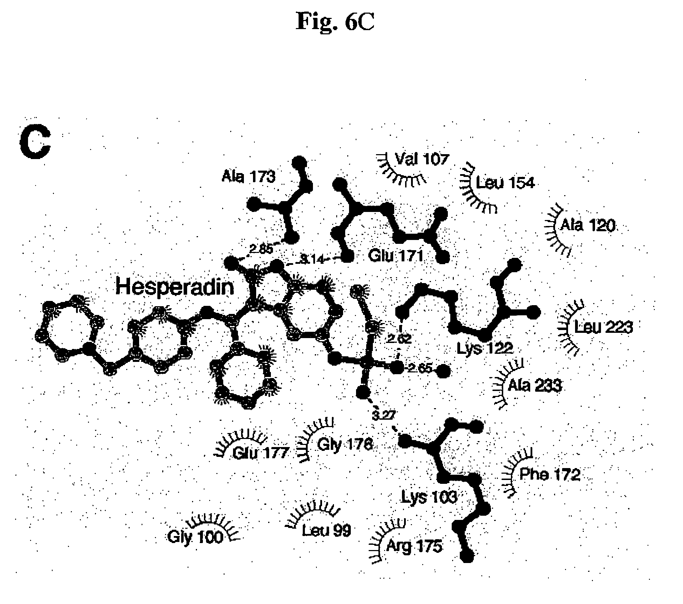

**Figure 6** Binding of Hesperadin to Aurora-B/INCENP
   A) Hesperadin is an active site inhibitor of Aurora-B.
   B) Electron density 'omit' map of Hcspcradin in the active site of Aurora B.
   C) Schematic view of the interactions of Hesperadin with the active site of Aurora-B created with LIGPLOT (Wallace, 1995).
**Figure 7**
   The activity of the G96V-Aurora-B⁶⁰⁻³⁶¹/INCENP⁷⁹⁰⁻⁸⁴⁷ (G96V) complex is equivalent to the wildtype Aurora-B⁶⁰⁻³⁶¹/INCENP⁷⁹⁰⁻⁸⁴⁷ (WT) complex.
   Activity of the xAurora-B/INCENP enzyme complexes is determined in the presence (WT, G96V) or absence (WT, no pep.; G96V, no pep.) of substrate peptide. Indicated amounts of protein are used in the radioactive filterbinding assay under otherwise identical assay conditions.
**Figure 8**
   List of amino acid residues forming the Hesperadin binding site of the G96V-Aurora-B/INCENP complex as set forth in Table 3.
**Figure 9**
   List of amino acid residues forming the INCENP binding site of Aurora-B as set forth in Tables 2-4.

### DETAILED DESCRIPTION OF THE INVENTION

### DEFINITIONS

"Conservative substitutions" are those amino acid substitutions which are functionally equivalent to the substituted amino acid residue, either by way of having similar polarity, steric arrangement, or by belonging to the same class as the substituted residue (e. g., hydrophobic, acidic or basic), and includes substitutions having an inconsequential effect on the three-dimensional structure of Aurora-B/INCENP with respect to the use of said structure for the identification and design of inhibitors of Aurora-B or of the Aurora-B/INCENP complex, for molecular replacement analyses and/or for homology modeling. "Amino acid sequence similarity" is a measure of the degree to which aligned amino acid sequences possess identical amino acids or conservative amino acid substitutions at corresponding positions.

A "fragment" of Aurora-B refers to a protein molecule which contains a portion of the complete amino acid sequence of the wild type or reference protein.

Unless stated otherwise, "Aurora-B" encompasses both Aurora-B and an"Aurora-B-like polypeptide".

As used herein, an "Aurora-B-like polypeptide" refers to a polypeptide having an amino acid sequence as described herein or with one or more amino acid substitutions, insertions, and/or deletions compared to the protein sequence described herein.

Preferably, the Aurora-B-like polypeptide has an amino acid sequence sharing at least about 70% amino acid sequence identity with the sequences shown in SEQ ID NO: 1 or SEQ ID NO:2, preferably at least about 80%, more preferably at least about 90%, and most preferably at least about 95%. Amino-acid substitutions are preferably substitutions of single amino-acid residues or small segments thereof. Such polypeptides also possess characteristic structural features and biological activity of a native Aurora-B polypeptide. For example, Aurora-B-like polypeptides arc characterized as containing key functional residues that participate in ligand binding.

Unless stated otherwise, "INCENP" encompasses both INCENP and an "INCENP-like polypeptide".

An "INCENP-like polypeptide" refers to a polypeptide having an amino acid sequence as described herein or with one or more amino acid substitutions, insertions, and/or deletions compared to the protein sequence described herein.

Preferably, the INCENP-like polypeptide has an amino acid sequence sharing at least about 70% amino acid sequence identity with the sequences shown in SEQ ID NO:3 or SEQ ID NO:4, preferably at least about 80%, more preferably at least about 90%, and most preferably at least about 95%. Amino-acid substitutions are preferably substitutions of single amino-acid residues or small segments thereof.

As used herein, an "Aurora-B domain-like polypeptide" and an "INCENP domain-like polypeptide" refers to a polypeptide having an amino acid sequence as described herein or with one or more amino acid substitutions, insertions, and/or deletions compared to the protein sequence described herein.

Generally, differences in amino acid sequences are limited so that the sequences of the reference (SEQ ID NO:1-4) and the variant are closely similar overall. Such variants are generally biologically active and necessarily have less than 100% sequence identity with the reference.

A "ligand" as used herein includes, in its general definition, a biological or chemical molecule which contacts and associates with a binding site. In the meaning of the present invention, a ligand is a molecule that binds to the ligand binding site of Aurora-B and/or an Aurora-B/INCENP complex as defined herein. Such ligands encompass, but are not limited to, DNA and RNA molecules, nucleosides, nucleotides, (poly)peptides, peptidomimetics (molecules which mimic aspects of polypeptide structure), small molecules, new chemical entities, (test) compounds, active ingredients of natural products or extracts thereof, substrate molecules, and drugs. Such ligands have the potential to inhibit the activity of Aurora-B and/or Aurora-B/1NCENP and may be developed as drugs for the treatment of cell proliferation disorders, in particular cancer.

As used herein, a "binding site" refers to a region of Aurora-B-like or an Aurora-B/INCENP complex that, as a result of its shape and charge potential, favorably interacts or associates with a ligand via various covalent and/or non-covalent binding forces. The terms "ligand binding site" and "binding site" are used interchangeably. The binding of a ligand to its binding site may or may not induce global conformational changes to Aurora-B, yet could potentially modulate the kinase activity of the protein. A ligand binding site according to the present invention includes the site of Aurora-B to which INCENP binds. Furthermore, a ligand-binding site on Aurora-B may be shared by various ligands. A compound could compete for or prevent binding of an activating ligand, e.g. INCENP, to Aurora-B and thereby inhibit kinase activity. A ligand binding site according to the present invention may also include a binding site on INCLNP responsible for interaction with Aurora-B. A ligand binding site according to the present invention also includes the actual binding site of Hesperadin.

As used herein, a ligand binding site also includes residues of Aurora-B/INCENP or of an Aurora-B/INCENP variant which exhibit observable NMR perturbations in the presence of a binding ligand, such as Hesperadin or any other ligand. While such residues exhibiting observable NMR perturbations may not necessarily be in direct contact with or immediately proximate to ligand binding residues, they may be critical to Aurora-B/INCENP residues for rational drug design protocols. For example, knowledge of the three-dimensional structure of the ligand binding site allows one to design molecules, preferably pharmaceutical agents, capable of binding thereto, including molecules which are thereby capable of inhibiting the interaction of Aurora-B/INCENP with its native ligands.

As used herein, the terms "bind" and "binding", when used to describe the interaction of a ligand with a binding site or a group of amino acids, means that the binding site or group of amino acids are capable of forming a covalent or non-covalent bond or bonds with the ligand.

Preferably, the binding between the ligand and the binding site is non-covalent. Such a non-covalent bond includes a hydrogen bond, an electrostatic bond, a van der Waals bond or the like. The binding of the ligand to the binding site may also be characterized by the ability of the ligand to co-crystallize with Aurora-B/INCENP within the binding pocket of the instant invention. It is further understood that the use of the terms "bind" and "binding" when referring to the interaction of a ligand with the novel binding site of the instant invention includes the covalent or non-covalent interactions of the ligand with all or some of the amino acid residues comprising the binding site.

An "Aurora-B/INCENP complex" refers to a complex of Aurora-B with INCENP including complexes comprising Aurora-B/INCENP in bound association with a ligand either by covalent or non-covalent binding forces at the binding sites disclosed herein. A non-limiting example of an Aurora-B/INCENP complex includes Aurora-B/INCENP/Hesperadin. As used herein the term "variant" refers to Aurora-B and/or INCENP or a portion thereof which may have deletions, insertions or substitutions of amino acids residues as long as the binding specificity of the molecules to each other in an Aurora/INCENP complex is retained to the extent desired. In this regard, deliberate amino acid substitutions may be made on the basis of similarity in polarity, charge, solubility, hydrophobicity, hydrophilicity and/or the amphiphatic nature of the residues.

As used herein, the term "composition" is intended to encompass a product comprising the specified ingredients in the specific amounts, as well as any product which results, directly or indirectly, from combination of the specific ingredients in the specified amounts.

As used herein the term "substantially pure composition" is intended to indicate that the composition does readily crystallize, when kept at the appropriate protein concentration.

The term "rool mean square deviation" means the square root of the arithmetic mean of the squares of the deviations from the mean. It is a way to express the deviation or variation from a trend or object. For purposes of this invention, the "root mean square deviation" defines the variation in the backbone atoms of a protein or protein complex from the relevant portion of the backbone of the Aurora-B-like polypeptide portion of the complex as defined by the structure coordinates described herein.

The invention therefore relates to a crystallizable composition comprising an Aurora-B-like polypeptide complexed with an INCENP-like polypeptide.

A further embodiment of the invention relates to a crystallizable composition wherein the Aurora-B-like polypeptide is a Xenopus laevis Aurora-B (xAurora-B)-like polypeptide.

A further embodiment of the invention relates to a crystallizable composition wherein the INCENP-like polypeptide is a Xenopus laevis INCENP (xINCENP)-like polypeptide.

A further embodiment of the invention relates to a crystallizable composition wherein the Aurora-B-like polypeptide is an Aurora-B kinase domain polypeptide or an Aurora-B kinase domain-like polypeptide.

Another embodiment of the invention relates to a substantially pure isolated complex comprising a polypeptide with an amino acid sequence as set forth in SEQ ID NO:1 or SEQ ID NO:2 and and a polypeptide with an amino acid sequence as set forth in SEQ ID NO:3 or SEQ ID No:4. Further aspects of the invention relate to a complex comprising variants of the aforementioned polypeptides.

A further embodiment of the invention relates to a crystallizable composition wherein the Aurora-B-like polypeptide has the amino acid sequence shown in SEQ ID NO:1 and the INCENP-like polypeptide has the amino acid sequence shown in SEQ ID NO:3.

A further embodiment of the invention relates to a crystallizable composition wherein the Aurora-B-like polypeptide has the amino acid sequence shown in SEQ ID NO:2 and INCENP-like polypeptide has the amino acid sequence shown in SEQ ID NO:3.

A further embodiment of the invention relates to a crystallizable composition obtainable by coexpression of an Aurora-B-like polypeptide and an INCENP-like polypeptide.

A further embodiment of the invention provides protein crystals of Aurora-B/INCENP. The crystals provide means to obtain the relative structural coordinates of the specific amino acids and their atoms that form the putative binding site and can interact with ligands that potentially bind to Aurora-B.

A further embodiment of the invention provides crystals of an Aurora-B/INCENP complex.

A further embodiment of the invention provides crystals of Aurora-B/INCENP complexed with a ligand bound to the ligand binding site disclosed herein. The crystals provide means to obtain the relative structural coordinates of the specific amino acids and their atoms that form the binding site and interact with ligands that bind to Aurora-B.

A further embodiment of the invention provides protein crystals of Aurora-BIINCENP complexed with a ligand bound to the ligand binding site disclosed herein. The crystals provide means to obtain the structural coordinates of the atoms of the ligands that bind to Aurora-B.

The Aurora-B/INCENP crystal can be obtained by crystallizing it with one or more ligands which bind to Aurora-B. Alternatively, the preformed Aurora-B-like polypeptide/INCENP-like polypeptide crystals and in particular the Aurora-B/INCENP crystals provided by this invention may be soaked in the presence of one or more ligands, such as Aurora-B kinase inhibitors, to provide Aurora-B-like polypeptide/INCENP-like polypeptide/ligand complex crystals. As used herein the term "soaking" refers to a process in which the crystal is transferred to a solution containing the ligand or ligands of interest.

Knowledge of the three-dimensional structure of the binding sites of Aurora-B/INCENP provides the basis for investigating the mechanism of action of the protein and tools for identifying inhibitors of its function.

Preferred crystalline compositions of this invention are capable of diffracting X-rays to a resolution of better than about 3.5 Å, and more preferably to a resolution of about 2.6 Å or better, and even more preferably to a resolution of about 2.0 Å or better, and are useful for determining the three-dimensional structure of the complex. The relative structural coordinates of the amino acid residues of the Aurora-B/INCENP complex, when the X-ray diffraction is obtained for the crystalline composition of Aurora-B/INCENP are shown in Tables 2 and 4. The relative structural coordinates of the amino acid residues of the Aurora-B/INCENP complex, when the X-ray diffraction is obtained for the crystalline composition of Aurora-B/INCENP and Hesperadin are shown in Table 3.

The three-dimensional structure of the Aurora-B/INCENP complex of this invention is defined by a set of structure coordinates as set forth in Tables 2 and 4. The three-dimensional structure of the Aurom-B/INCENP/ligand complex of this invention is defined by a set of structure coordinates as set forth in Table 3. The term "structure coordinates" refers to Cartesian coordinates derived from mathematical equations related to the patterns obtained on diffraction of a monochromatic beam of X-rays by the atoms (scattering centers) of an Aurora-B/INCENP complex with or without a bound ligand in crystalline form. The diffraction data are used to calculate an electron density map of the repeating unit of the crystal. The electron density maps are then used to establish the positions of the individual atoms of the Aurora-B/INCENP or Aurora-B/INCENP/ligand complex(es).

Those of skill in the art will understand that a set of structure coordinates for a protein or a protein-complex or a portion thereof, is a relative set of points that define a shape in three dimensions. Thus, it is possible that an entirely different set of coordinates could define a similar or identical shape. Moreover, slight variations in the individual coordinates will have little effect on overall shape.

The variations in coordinates discussed above may be generated using mathematical manipulations of the structure coordinates. For example, the structure coordinates set forth in Tables 2-4 could be manipulated by crystallographic permutations of the structure coordinates, fractionalization of the structure coordinates, integer additions or subtractions to sets of the structure coordinates, inversion of the structure coordinates, rotation of the structure coordinates or any combination of the above, but the new structure coordinates will still define the relationship between the multiple components of the crystal structures disclosed herein.

Alternatively, modifications in the crystal structures due to mutations, additions, substitutions, and/or deletions of amino acids, or other changes in any of the components that make up the crystal could also account for variations in structure coordinates. If such variations arc within an acceptable standard error as compared to the original coordinates, the resulting three-dimensional shape is considered to be the same.

In another aspect of the instant invention, the high quality single crystals of Aurora-B/INCENP complex with a bound ligand can be used to obtain single crystals of an Aurora-B/INCENP complex with another ligand that binds to Aurora-B/INCENP. Thus, for example and not limiting the scope of this embodiment, high quality single crystals of an Aurora-B/INCENP/ligand complex are soaked with another ligand that binds more strongly to Aurora-B/INCENP than the bounded one. It is expected that the ligand will intercalate into the crystal and replace the previously bound ligand in the binding site. One or more molecular fragments of ligands that bind to Aurora-B/INCENP may also be utilized in this technique. X-ray diffraction data are collected from the high quality single crystals obtained by the intra-crystal ligand exchange technique.

The X-ray diffraction patterns obtained by methods of the present invention can be provided on computer readable media, and used to provide electron density maps.

The structure coordinates set forth in Tables 2-4 can also be used to aid in obtaining structural information about any crystallised molecule or molecular complex of interest. This may be achieved by any of a number of well-known techniques, such as molecular replacement analysis, 2D, 3D and 4D isotope filtering, editing and triple resonance NMR techniques, and computer homology modeling

The structure coordinates set forth in Tables 2-4 can also be used for determining at least a portion of the three-dimensional structure of a molecule or a molecular complex of interest which contains at least some features that are structurally similar to Aurora-B and/or Aurora-B/INCENP. In particular, structural information about any other crystallized molecule or molecular complex of interest can be obtained.

Therefore, in another embodiment this invention provides a method of utilizing molecular replacement to obtain structural information about a crystallized molecule or molecular complex of interest whose structure is unknown comprising the steps of
a) generating an X-ray diffraction pattern from said crystallized molecule or molecular complex of interest; and b) applying at least a portion of the structure coordinates set forth in Tables 2-4 to the X-ray diffraction pattern to generate a three-dimensional electron density map of said molecule or molecular complex.

Molecular replacement provides an accurate estimation of the phases for an unknown structure. Phases are a factor in equations used to solve crystal structures that can not be determined directly. Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory estimate of the phases for the unknown structure.

Thus, this method involves generating a preliminary model of a molecule or molecular complex whose structure coordinates are unknown, by orienting and positioning the relevant portion of the Aurora-B/INCENP complex according to Tables 2-4 within the unit cell of the crystal of the unknown molecule or molecular complex so as best to account for the observed X-ray diffraction pattern of the crystal of the molecule or molecular complex whose structure is unknown. Phases can then be calculated from this model and combined with the observed X-ray diffraction pattern amplitudes to generate an electron density map of the structure whose coordinates are unknown. This, in turn, can be subjected to any well-known model building and structure refinement techniques to provide a final, accurate structure of the unknown crystallized molecule or molecular complex (Lattman, 1985; M. G. Rossmann, 1972).

The structure of any portion of any crystallized molecule or molecular complex that is sufficiently homologous to any portion of the Aurora-B/INCENP complex can be solved by this method.

The structure coordinates of Aurora-B/INCENP as provided by this invention are particularly useful in solving the structure of other crystal forms of Aurora-B.

The structure coordinates of Aurora-B/INCENP as provided by this invention are also particularly useful to solve the structure of crystals of Aurora-B/INCENP co-complexed with a variety of ligands. This approach enables the determination of the optimal sites for interaction between ligands, including candidate Aurora-B inhibitory compounds, with Aurora-B or the Aurora-B/INCENP complex. For example, high resolution X-ray diffraction data collected from Aurora-B/INCENP crystals exposed to different types of solvents allows the determination of where each type of solvent molecule resides. Ligands that bind to those sites can then be designed and synthesized and tested for their Aurora-B inhibitory activity.

All of the complexes referred to above may be studied using well-known X-ray diffraction techniques and may be refined against X-ray data using computer software, such as Rcfmac (distributed by the Collaborative Computational Project, Number 4). This information may thus be used to optimize known Aurora-B inhibitors, and more importantly, to design new Aurora-B inhibitors.

Furthermore, alternative methods of tertiary stmcture determination that do not rely on X-ray diffraction techniques and thus do not require crystallization of the protein, such as NMR techniques, are simplined if a model of the structure is available for refinement using the additional data gathered by the alternative method.

The present invention is additionally directed to a method of determining the three-dimensional structure of a molecule or molecular complex of interest whose structure is unknown, comprising the steps of generating NMR data from the solution of said molecule or molecular complex. The generated diffraction or spectroscopy data from the molecule or molecular complex is then compared with the coordinates or three-dimensional structure of Aurora-B/INCENP as disclosed herein, and the three-dimensional structure of the unknown molecule or molecular complex is conformed to the Aurora-B/INCENP structure using standard techniques.

Knowledge obtained concerning Aurora-B including a binding site defined herein can also be used to model the tertiary structure of related kinase proteins, in particular members of the Aurora family of kinases.

The invention also provides methods for determining the three-dimensional structure of a protein of interest containing the ligand binding site as disclosed herein, or a complex of said protein with a ligand thereof, using homology modeling techniques and structural coordinates of a composition of this invention. Homology modeling involves coinstructing a model of an unknown structure using structural coordinates of one or more related proteins, protein domains and/or subdomains. Homology modeling may be conducted by fitting common or homologous portions of the protein or peptide whose three-dimensional structure is to be solved to the three-dimensional structure of homologous structural elements. Homology modeling may include rebuilding part or all of a three-dimensional structure with replacement of amino acids (or other components) by those of the related structure to be solved. This invention is also useful for providing threc-dimensional structural information on new complexes of Aurora family members of which Aurora-B is a member with various ligands, as well as mutations or other variants of any of the foregoing.

Alternatively, a three-dimensional model of the unknown molecule may be generated by generating a sequence alignment between Aurora-B and the unknown molecule, based on any or all of amino acid sequence identity, secondary structure elements or tertiary folds, and then generating by computer modeling a three-dimensional structure for the molecule using the three-dimensional structure of, and sequence alignment with, Aurora-B.

Examples of programs for homology modeling include but are not limited to MOE (Chemical Computing Group, Inc.) and MODELLER (Accelrys Inc).

In accordance with the above, a three-dimensional structure of a molecule/molecular complex of unknown structure may be generated using the three-dimensional structure of the Aurora-B/INCENP molecule of the present invention (Tables 2-4) refined using a number of techniques well known in the art, and then used in the same fashion as the structural coordinates of the present invention, for instance, in applications involving molecular replacement analysis, homology modeling, and rational drug design.

Still further, the invention also encompasses compositions and methods for identifying binding sites of other members of the Aurora protein family. The methods involve examining the surface of a protein of interest, preferably a kinase, to identify residues that facilitate binding to the binding site. The residues can be identified by homology to the ligand binding site of xAurora-B described herein. Overlays and super-positioning with a three-dimensional model of a Aurora-B binding site, or a portion thereof that contains a ligand binding site, also can be used for this purpose.

One embodiment of the invention is a method for determining whether a test compound has the ability to inhibit the kinase activity and/or the ATP-consuming activity of Aurora B, the method comprising the steps of: (a) contacting an Aurora-B-like polypeptide/INCENP-like polypeptide composition with said test compound and (b) determining (i) the interaction of said test compound with said Aurora-B-like polypeptide/INCENP-like polypeptide composition and/or (ii) the effect of said compound on the kinase activity or the ATP-consuming activity of said composition.

The interaction of said test compound with said composition can be determined according to known methods, which allow determination of dissociation constants, e.g. using assays based on the principle of surface plasmon resonance, fluorescence anisotropy or polarization, thermal denaturation, or isothermal titration calorimetry (ITC). A quantitative description of the forces that govern molecular associations requires determination of changes of all thermodynamic parameters (free energy of binding, enthalpy, and entropy of binding and the heat capacity change). A method to measure the heat change during interaction between the compound and the Aurora-B/INCENP composition at constant temperature is provided by ITC. With this method one binding partner, usually the test compound, is titrated into a solution containing the interaction partner, usually the complex, thereby generating or absorbing heat. This heat is the direct observable that can be quantified by the calorimeter (Perozzo, 2004).

Kinase or ATP-consuming activity can be determined in a radioactive or a non-radioactive format using known technologies. Usually, readouts for such assays are optical and encompass scintillation proximity assays (SPA), filter-binding assays, homogenous time-resolved fluorescence (HTRF) assays, fluorescence polarization (FP) assays, amplified luminescence proximity homogenous (ALPHA) screen assays, ATP consumption assays and others (von A6sen, 2005).

In brief, such assays, which can be carried out on a laboratory scale or in the high-throughput format, involve the steps of contacting the composition with the test compound - usually in the presence of substrate and ATP - and detecting, by a method mentioned above, the phosphorylated state of the substrate (or the kinase itself), or the decrease in ATP-levels as a result of the kinase and/or ATP-consuming activity of Aurora B.

A further aspect of the invention is a method for identifying an agent which upon binding to Aurora-B inhibits kinase and/or ATP-consuming activity wherein said Aurora-B-like polypepnde/INCENP-like polypeptide composition is substantially pure.

A further aspect of the invention is a method for identifying a ligand which upon binding to Aurora-B has potential anti-mitotic activity, comprising the steps of:
(a) obtaining a crystal of Aurora-B/INCENP, where said Aurora-B/lNCBNP has been crystallized while exposed to one or more potential ligands;
(b) determining whether a ligand/Aurora-B/INCENP complex is formed in said crystal; and
(c) identifying a potential anti-mitotic ligand as one that binds to said Aurora-B/INCENP.

A further aspect of the invention is a method for identifying a ligand which upon binding to Aurora-B inhibits cell proliferation, comprising the steps of:
(a) soaking an pre-formed crystal of Aurora-B/INCENP with one or more potential ligands;
(b) determining whether a ligand/Autora-B/INCENP complex is formed in said crystal; and
(c) identifying a potential anti-mitotic ligand as one that binds to said Aurora-B/INCENP.

Also disclosed herein is a process for identifying a potential ligand which upon binding to Aurora-B inhibits cell proliferation, comprising the steps of:
a) crystallizing said Aurora-B/INCENP composition in the presence of one or more ligands or soaking a pre-formed Aurora-B/INCENP crystal with one or more ligands.
b) obtaining an X-ray diffraction pattern of the said crystal; and
c) determining whether an Aurora-B/INCENP/ligand complex is formed by comparing the electron density map calculated from the X-ray diffraction pattern of said crystal to the electron density map calculated from the X-ray diffraction pattern set forth in a table selected from Tables 2-4.

The structural coordinates of the present invention enable one to use various molecular design and analysis techniques in order to (i) solve the three-dimensional structures of related molecules, preferably molecular complexes such as those of other species (esp. human) or members of Aurora family of proteins; as well as (ii) design, select, and synthesize ligands capable of favorably associating or interacting with a ligand binding site of an Aumra-B molecule, wherein the ligand would preferably inhibit Aurora-B function and induce in cells contacted therewith a type of mitotic arrest which mimics the phenotype observed following genetic inactivation of Aurora-B (Hauf, 2003).

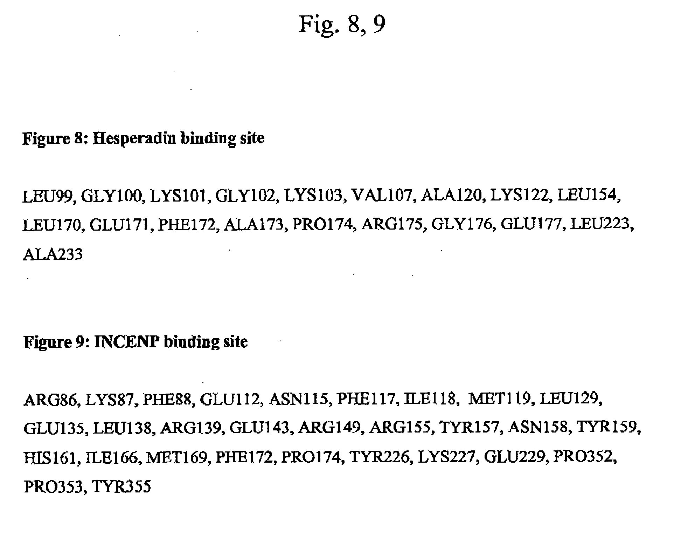

The Aurora-B/INCENP complex of the invention comprises a ligand binding site characterized by the amino acid residues as set forth in Figure 8 or the relative structural coordinates of those amino acid residues according to Table 3 with a root mean square deviation from the conserved backbone atoms of said amino acids of not more than about 2.0 Å (or more preferably, not more than about 1.0 Å, and most preferably, not more than about 0.5 Å).

The Aurora-B/INCENP complex of the invention further comprises a binding site characterized by the amino acid residues as set forth in Figure 9 or the relative structural coordinates of those amino acid residues according to Table 2 or 4 with a root mean square deviation from the conserved backbone atoms of said amino acids of not more than about 2.0 Å (or more preferably, not more than about 1.0 Å, and most preferably, not more than about 0.5 Å).

It is understood that the amino acids listed in Figure 8 represent the residues defining the ligand binding site formed upon the complexation Hesperadin with the Aurora-B/INCENP complex. It is understood that the amino acids listed in Figure 9 represent the residues defining the binding site formed upon the complexation of Aurora-B with INCENP. It is further understood that specific binding interactions between the listed residues may or may not occur based on the size of another ligand and structure of that ligand. It is also understood that the computational length of the allowable van der Waals interactions is also a factor when determining whether an amino acid residue binds to a ligand. It is therefore understood that the binding of a ligand of the instant invention may take place between those residues listed in Figure 8 and/or 9 or a subset thereof.

It is understood that the method of the present invention includes additional embodiments comprising conservative substitutions of the noted amino acids which result in the same structural coordinates of the corresponding residues in Tables 2-4 within the stated root mean square deviation.

Computer modeling technologies allow researchers to visualize the three-dimensional structure of a targeted compound (e.g. Aurora-B/INCENP), and using such a three-dimensional structure to identify putative binding sites and then identify or design ligands to interact with these binding sites. These ligands can thereafter be screened for an inhibitory effect upon the target molecule.

One skilled in the relevant art may use molecular modeling methods to identify a ligand binding site of an Aurora-B-like polypeptide. Specifically, coordinates provided by the present invention may be used to characterize a three-dimensional structure of the target Aurora-B molecule, liganded or unliganded. Importantly, such a skilled artisan may, from such a structure, computationally visualize a putative binding site and identify and characterize other features based upon the coordinates provided herein. Such putative ligand binding sites may be further refined using chemical shift perturbations of spectra generated from various and distinct Aurora-B complexes, e.g. from other species, competitive and non-competitive inhibition experiments, and/or by the generation and characterization of Aurora-B or ligand mutants to identify critical residues or characteristics of the ligand binding site.

This invention further provides for the use of the structural coordinates of a crystalline composition of this invention, or portions thereof, to identify reactive amino acids within the three-dimensional structure, preferably within or adjacent to a ligand binding site; to generate and visualize a molecular surface, such as a water-accessible surface or a surface comprising the space-filling van der Waals surface of all atoms; to calculate and visualize the size and shape of surface features of the molecule or molecular complex, e.g. ligand binding pockets; to locate potential H-bond donors and acceptors within the three-dimensional structure, preferably within or adjacent to a ligand binding site; to calculate regions of hydrophobicity and hydrophilicity within the three-dimensional structure, preferably within or adjacent to a ligand binding site; and to calculate and visualize regions on or adjacent to the protein surface of favorable interaction energies with respect to selected functional groups of interest (e.g. amino, hydroxyl, carboxyl, methylene, alkyl, alkenyl, aromatic carbon, aromatic rings, heteroaromatic rings, substituted and unsubstituted phosphates, substituted and unsubstituted phosphonates, substituted and unsubstituted fluoro- and difluorophosphonates; etc.). One may use the foregoing approaches for characterizing the protein and its interactions with moieties of potential ligands to design or select compounds capable of specific covalent attachment to reactive amino acids (e.g. cysteine) and to design or select compounds of complementary characteristics (e.g. size, shape, charge, hydrophobicity/hydrophilicity, ability to participate in hydrogen bonding, etc.) to surface features of the protein, a set of which may be preselected.

In another aspect, this invention envisions use of atomic coordinates of the Aurora-B/INCENP complex disclosed herein, to design a ligand capable of associating with an Aurora-B like polypeptide or a fragment thereof

Thus, an embodiment of the invention envisions use of the structural information from the ligand/protein complexes found herein including the information derived therefrom in designing new ligands that bind more tightly, bind more specifically, have better biological activity or have better safety profile than known ligands that bind to Aurora-B. The computer modeling method disclosed herein can also be used to remodel the ligands to improve the affinity or solubility, and produce an optimized pharmaceutical agent The resulting optimized compound can thereafter be synthesized and the inhibitory activity for Aurora-B can be tested in vitro, e.g. in kinase assays or in cell culture, and in vivo, e.g. animal models. If the test confirms that the material does indeed inhibit Aurora-B, then the compound or a derivative can be used as an anti-mitotic agent. Using the method as described above, the ligand identified to have inhibitory activity may thereafter be used as a lead compound to obtain an improved Aurora-B inhibitor. In order to confirm the affinity predicted by the computer modeling method, the dissociation constant of the complex may be experimentally measured. The resulting ligands arc then provided by methods of the present invention and are useful for treating, inhibiting or preventing Aurora-B-modulated diseases in animals, including humans. Preferably the ligands of the novel binding site provided herein are useful in the treatment or prevention of a hyper-proliferative disease, preferably cancer.

Also provided is a method for obtaining a potential inhibitor of Aurora-B, comprising the steps of a) providing a three-dimensional structure of a binding site as defined by the relative structural coordinates of the amino acids in Figure 8 or 9 as set forth in Tables 2-4, b) designing or selecting a potential inhibitor, and c) synthesizing said potential inhibitor. The inhibitor may be selected by screening an appropriate database, may be designed de novo by analyzing the steric configurations and charge potentials of an empty Aurora-B binding site in conjunction with the appropriate software programs, or may be designed using characteristics of known inhibitors to create "hybrid" inhibitors. The inhibitor may then be contacted with Aurora-B, and the effect of the inhibitor on Aurora-B related function may be assessed. For instance, a potential inhibitor identified by this method may be contacted with Aurora-B in the presence of Aurora-B substrate(s) and determining the effect the potential inhibitor has on Aurora-B kinase activity.

Thus, an exemplary embodiment of the invention envisions a method of three-dimensional modeling of an Aurora-B/INCENP protein, comprising the steps of: (a) providing three-dimensional atomic coordinates derived from X-ray diffraction measurements of a Aurora-B/INCENP crystal in a computer readable format; (b) inputting the data from step (a) into a computer with appropriate software programs; and (c) generating a three-dimensional structural representation of the Aurora-B/INCENP protein suitable for visualization and further computational manipulation.

There are many currently available computer programs for the expression of the three-dimensional structure of a molecule, for example SYBYL (Tripos, Inc.) or MOE (Chemical Computing Group, Inc.). Generally, these programs provide for inputting of the coordinates for the three-dimensional structure of a molecule (i.e., for example, a numerical assignment for each atom of a Aurora-B/INCETTP molecule along an x, y, and z axis or the assignment for each atom of the binding sites as defined by the relative structural coordinates of the amino acids in Figure 8 or 9 as set forth in Tables 2-4), means to express (such as visually display) such coordinates, means to alter such coordinates and means to express an image of a molecule having such altered coordinates. Preferred expression means are well known to a skilled artisan.

An exemplary embodiment of the invention provides methods for identifying and designing ligands that bind to the binding site using atomic models of Aurora-B provided herein. The method involves modeling test compounds that fit spatially into the binding site of interest using an atomic structural model comprising an Aurora-B/INCENP binding site or portion thereof. Preferably, the ligand designed or selected to interact with Aurora-B is capable of associating with Aurora-B and of assuming a three-dimensional configuration and orientation that complements the relevant ligand binding site of Aurnra-B. The effect of a ligand identified by computer fitting analyses on Aurora-B activity may be further evaluated computationally or experimentally by competitive binding experiments or by contacting the identified ligand with Aurora B and measuring the effect of the ligand on the target's biological activity. Standard enzymatic assays may be performed and the results analyzed to determine whether the ligand is an inhibitor of Aurora-B activity (i. e., induce cell cycle arrest). Further tests may be performed to evaluate the selectivity of the identified ligand to Aurora-B with regard to other Aurora-B proteins (other species), other members of the Aurora protein family or other kinase family members.

After an Aurora-B and/or Aurora-B/INCENP binding ligand has been selected or designed, as described above, substitutions may then be made in some of its atoms or side groups in order to improve or modify its selectivity and binding properties - that is its affinity for the ligand binding site disclosed herein. Such substituted chemical compounds may then be analyzed for efficiency of fit to the Aurora-B/INCENP by computer methods.

For example, once a Aurora-B binding ligand has been optimally selected or designed, as described above, substitutions may then be made in some of its atoms or side groups in order to improve or modify its selectivity and binding properties for Aurora-B. Generally, initial substitutions are conservative, i.e., the replacement group will have approximately the same size, shape, hydrophobicity and charge as the original group. Such substituted chemical compounds may then be analyzed for efficiency of fit to Aurora-B/INCENP by the same computer methods described in detail above.

After a ligand designed by the computer method described above is prepared and bound to Aurora-B/INCENP to produce a crystal, the 3-dimensional structure of the complex may be determined using X-ray crystallographic methods. The information gained therefrom e. g., about the interaction between Aurora-B/INCENP and the inhibitor obtained from this can then be used to modify the inhibitor and to increase the affinity of the inhibitor for the ligand binding site of Aurora-B/INCENP.

Also provided herein is a method of identifying a compound that modulates the binding of a ligand to a ligand binding site of Aurora-B or Aurora B/INCENP, said method comprising: modeling test compounds that fit spatially into a Aurora-B or Aurora-B/INCENP ligand binding site using an atomic structural model of a Aurora-B or Aurora-B/INCENP binding site having the relative structural coordinates as set forth in Tables 2, 3 and 4 for the Aurora-B amino acid residues stated in Figure 8 or 9 ± the root mean square deviation from the backbone atoms of said amino acids of not more than about 2.0 Å; screening the test compounds in an assay characterized by binding of a ligand to the ligand binding site; and identifying a test compound that modulates binding of said ligand to the Aurora-B at its binding site.

It is also within the confines of the present invention that a potential inhibitor may be designed or selected by identifying chemical entities or fragments capable of associating with Aurora-B; and assembling the identified chemical entities or fragments into a single molecule to provide the structure of the potential inhibitor.

In the pharmaceutical industry, new or known compounds are routinely screened for new uses employing a variety of known in vitro or in vivo screens. Often such screens involve complex natural substances and are correspondingly expensive to carry out, and the result may be difficult to interpret. The knowledge of the three-dimensional protein structure according to the invention allows a preliminary screening to be carried out on the basis of the three-dimensional structure or a region thereof, and the structural similarity of a molecule which is being screened. This is usually carried out in conjunction with knowledge of the amino sequence of the region. Such screening can conveniently be carried out using computer modeling techniques, which match the three-dimensional structure of the protein or part thereof with the structure of the molecule being screened, thereby allowing one to predict potential inhibitor activity.

Consequently, an embodiment of the invention relates to a method for identifying a potential inhibitor of Aurora-B. The proposed method comprises using the three-dimensional structure of Aurora-B/INCENP and the ligand binding site of the invention as defined by the relative structural coordinates of the amino acid residues in Figure 8 as set forth in Table 3 to select a potential inhibitor of Aurom-B activity by screening an appropriate database, followed by synthesizing or obtaining the said potential inhibitor.

An alternative method of this invention provides for, selecting from a database of chemical structures a compound capable of binding to Aurora-B. The method starts with structural coordinates of a crystalline composition of the invention, e.g. coordinates defining the three-dimensional structure of Aurora-B or a portion thereof (e.g. the herein provided coordinates of Aurora-B/INCENP). Points associated with that three-dimensional structure are characterized with respect to the extent of favorable interactions with one or more functional groups of a chemical moiety. A database of chemical structures is then searched for candidate compounds containing one or more functional groups disposed for favorable interaction with the protein based on the prior characterization. Compounds having structures which best fit the points of favorable interaction with the three-dimensional structure are thus identified.

Further provided is a method for identifying a potential inhibitor of Aurora-B, the method comprising the steps of: (i) providing the three-dimensional structure of a ligand-bound Aurora-B/INCENP; (ii) comparing the three-dimensional coordinates of the ligand when it is bound to Aurora-B/INCENP (e.g. the coordinates of Hesperadin as set forth in Table 3) to the three-dimensional coordinates of a compound in a database of compound structures; and (iii) selecting from said database at least one compound that is structurally similar to said ligand when it is bound to said Aurora-B/INCENP, wherein the selected compound is a potential inhibitor of said Aurora-B/INCENP.

As such, an embodiment of the invention proposes using the structural coordinates of Tables 2-4 of the present invention, or structural coordinates derived therefrom using molecular replacement or homology modeling techniques as discussed above to screen a database for agents that may act as potential inhibitors of Aurora-B activity. As an example, the obtained structural coordinates of the present invention may be read into a software package and the three-dimensional structure analyzed graphically. A number of computational software packages may be used for the analysis of structural coordinates, e.g. Sybyl (Tripos Inc.). Additional software programs maybe optionally used to check the coordinates with regard to features such as bond and atom types. Ifnecessary, the three-dimensional structure may be modified and then energy minimized using the appropriate software until all of the structural parameters are at their equilibrium/optimal values. The energy minimized structure can then be superimposed against the original structure to make sure there are no significant deviations between the original and the energy minimized coordinates. Docking studies with different ligands will allow one skilled in the art to generate initial models of new ligands bound to Aurora-B. The integrity of these new models may be evaluated a number of ways, including constrained conformational analysis using molecular dynamics methods; that is where both Aurora-B/INCENP and the bound ligand are allowed to sample different three-dimensional conformational states until the most favorable state is reached or found to exist between the protein and the bound ligand etc. Once models are obtained of the original known ligand bound to Aurora-B/INCBNP (e.g. Hesperadin, Table 3) and computer models of other ligands hound to Aurora-B are as well obtained, strategies maybe proposed determined for designing modifications into the ligands to improve their inhibitory activity and/or enhance their selectivity.

Compounds of the structures selected or designed by any of the foregoing means may be tested for their ability to bind to an Aurora-B protein, inhibit the binding of an Aurora-B protein to a natural or non-natural ligand, and/or inhibit a biological function mediated by an Aurora-B.

An entity/agent that interacts or associates with the ligand binding site of Aurora-B may be identified by performing computer fitting analyses to identify a ligand which interacts or associates with said site.

Computer fitting analyses utilize various computer software programs that evaluate the "fit" between the binding site and the identified ligand, by (a) generating a three-dimensional model of the ligand binding site using homology modeling or the atomic structural coordinates of the binding site as defined by the relative structural coordinates of the amino acids in Figure 8 or 9 as set forth in Tables 2-4, and (b) determining the degree of association between the binding site and the identified ligand. The degree of association may be determined computationally by any number of commercially available software programs, or may be determined experimentally using standard binding assays.

Using the structural coordinates, one may also predict or calculate the orientation, binding constant or relative affinity of a given ligand to the protein in the complexed state, and use that information to design or select compounds of improved affinity.

Fitting of a compound to the ligand binding site volume can be done in a number of different ways using computational methods well known by those skil led in the art. Visual inspection and manual docking of compounds into the induced-fit active site volume can be done using molecular modeling software such as SYBYL (Tripos, Inc.), AMBER (Weiner, 1984), CHARMM (Brooks,1983) or other modeling programs known to those of skill in the art. This modeling step may be followed by energy minimization using standard force fields, such as CHARMM and AMBER., or others. More specialized modeling programs include GRID (Goodford,1985), AUTODOCK (Goodsell, 1990), and DOCK (Kuntz, 1982).

For example, one method of this invention for evaluating the ability of a ligand to associate with any of the proteins or protein-ligand complexes set forth herein comprises the steps of: a) employing computational means to perform a fitting operation (docking) between the ligand and a binding pocket or other surface feature of the molecule or molecular complex; and b) analyzing the results of said fitting operation to quantify the association between the ligand and the binding pocket.

In addition, inhibitory compounds may be constructed de novo in the empty active site or in the active site including some portions of a known inhibitor using computer programs such as LEGEND (Nishibata, 1991), LeapFrog (Tripos Associates, St. Louis, MO), LUDI (Bohm, 1992), AutoLudi (Accelrys, Inc.) or others.

### MATERIALS AND METHODS

Materials and methods provided are intended to assist in a further understanding of the invention and are not to limit the reasonable scope thereof

The amino acid sequence of the kinase domain ofxAurora-B is depicted in SEQ ID NO: 1. These amino acids correspond to residues 60-361 of the native protein.

SEQ ID NO:2 xAuroTa-B kinase domain, G96V mutant

The amino acid sequence of the XINCENP (Xenopus laevis INCENP) fragment is depicted in SEQ ID NO:3. These amino acids correspond to residues 790-847 of the native protein.

The amino acid sequence of the xINCENP fragment is depicted in SEQ ID NO:4. These amino acids correspond to residues 790-856 of the native protein.

### Epression vectors

The coding sequence for Aurora-B⁶⁰⁻³⁶¹ from *Xenopus laevis* is subcloned using BamHI and SalI restriction sites in the first cassette of a modified version of pGEX-6T (Amersham Biotech) generate. The vector contains two cloning cassettes separated by a ribosome-binding site, and allows dicistronic expression. In this configuration, the protein is expressed from the first cassette as a C-terminal fusion to the GST. The sequences encoding *Xenopus laevis* INCENP⁷⁹⁰⁻⁸⁴⁷ and INCENP⁷⁹⁰⁻⁸⁵⁶ arc subcloned into the BglII and Hindlll sites of the second cassette. The resulting polycistronic vectors will be refered as pAUB-IN⁸⁴⁷ and pAUB-IN⁸⁵⁶. Point mutants and deletion mutants are created using the QuikChange^{™} Kit (Stratagene). All constructs are confirmed by sequencing. The KD mutant is generated by mutation of Lys 122 into Arg.

### Protein expression

pAUB-IN847 and pAUB-1N856 are used to transform the E. coli strain BL21(DE3) containing the pUBS520 helper plasmid. Both proteins and their mutants are expressed and purified under essentially identical conditions. Protein expression is induced with 0.3 mM IPTG at an OD600 of 0.45-0.7. Expression is then continued for about 12-16 hours at 23-25°C with agitation. Bacterial cells are harvested by centrifugation at 4000 rpm x 15 min in a Beckman JLA 8.1 rotor, and the pellets resuspended in lysis buffer (50 mM Tris HCl pH 7.6, 300 mM NaCl, 1 mM DTT, 1 mM EDTA, 5 % Glycerol, Roche Complete protease inhibitor tablets). 20-30 ml Lysis buffer are used per liter of E. coli culture. Cells arc lysed by sonication, and the lysates cleared by centrifugation at 12000 rpm for 45-60 min on a JA20 rotor. The supernatants are incubated with 300 µl of GST Sepharose Fast Flow (Amersham Biosciences) per liter of bacterial culture. The resin is first washed with PBS buffer and finally equilibrated with lysis buffer. After a 4-5 hour agitation at 4°C, the beads are washed with 30 volumes of lysis buffer, and then equilibrated with 30 volumes of cleavage buffer (50 mM Tris pH 7.6,150 mM NaCl, 1 mM DTT, 1 mM EDTA). To cleave the GST from Aurora-B, 10 units of Prescission protease (Amersham Biosciences) per milligram of substrate are added and the incubation is protracted for 16 hours at 4 °C. The supernatant, which contains the cleaved product, is collected and loaded onto a 6 ml Resource Q column (Amersham Bioscicnces) equilibrated with Ion Exchange buffer (50 mM Tris pH 7.6, 150 mM NaCl, 1 mM DTT, 1 mM EDTA). The Aurora-BIINCENP complex is collected in the How through of the column. The flow-through of the Resource Q column is concentrated and loaded onto a Superdex 200 size-exclusion chromatography (SEC) column equilibrated with SEC buffer (Tris HCl 10 mM pH 7.6, NaCl 150 mM, DTT 1 mM, EDTA 1 mM). Fractions containing Aurora-B/INCENP are collected and concentrated using Vivaspin concentrators (MW cutoff 3-5 K) to a final concentration of 12 mg/ml. The final yield is about 1-2 mg of pure complex per liter of bacteria.

### Crystallization and Structure Determination

Aurora-B/INCENP⁷⁹⁰⁻⁸⁴⁷ is crystallized by vapor diffusion. Initial crystals are obtained with a complex containing an Aurora-B mutant, in which Gly96 had been mutated into valine (G96V-Aurora-B) during the polymerase chain reaction. The G96V-Aurora-B/INCENP⁷⁹⁰⁻⁸⁴⁷ complex has the same biochemical and biophysical properties as the wild type complex, including phosphorylation on Thr248, elution from a size-exclusion chromatography column as a 1:1 complex, and kinase activity indistinguishable from that of its wild type counterpart.

Crystals are improved by microseeding. Initial crystals arc crashed with a needle and used for seeding drops that had equilibrated for 5-8 hours against the same reservoir solution. Seeds derived from crystals of G96V-Aurora-B⁶⁰⁻³⁶¹/INCENP⁷⁹⁰⁻⁸⁴⁷ are later used for micro seeding against wild type Aurora-B⁶⁰⁻³⁶¹/INCENp790-847.

Crystals are gradually transferred to cryo-buffer (19% PEG400, 100 mM Bis-Tris-Propane pH 6.5, 2 mM TCEP) and flash-frozen prior to data collection. Initial X-ray diffraction data to a resolution of 1.8 A are collected from a single frozen crystal at beam line ID14-1 at the European Synchroton Radiation Facility in Grenoble (France). Data processing is carried out with DL7NZ0 and SCALEPACK (Otwinowski, 1993). For subsequent calculations, the CCP4 (Collaborative Computational Project, 1994) and CNS (Neumann, 1998) suites are used. The crystals belong to the monoclinic space group P2, and contain two 1:I 1 Aurora-B/INCENP complexes in the asymmetric unit (Table I). The structure is determined by molecular replacement (MR) using the atomic model of Aurora-A (PDB code 1OL5) as a search model. Model building is carried out with program O (Jones, 1991). This search model provides excellent solutions for the rotation and translation function that reveals the position of the two Aurora-B molecules in the crystal's asymmetric unit The resulting sigma-weighted electron density maps (Collaborative Computational Project, 1994) calculated with model phases and native amplitudes, however, shows well defined changes in the relative orientation of the small and large lobes of Aurora-B relative to Aurora-A. These changes arc modeled and the structure of the INCENP IN-box is built into the electron density. The final model of the G96V-Aurora-B/INCENP⁷⁹⁰⁻⁸⁴⁷ complex is refined to an R-factor of 23.0 with a frcc-R factor of 27.0 (Table 1).

The final model of Aurora-B/INCENP⁷⁹⁰⁻⁸⁴⁷ complex is refined to an R-factor of 19.7 and a free-R factor of 22.6 (Table 1).

For co-crystallization, Hesperadin is dissolved to a final concentration of 100 mM in pure DMSO, and the solution is diluted 50-100 times upon mixing with the Aurora-B/INCENP complex. Crystals of the complex with Hesperadin are obtained with G96V-Aurora-B⁶⁰⁻³⁶¹/INCENP⁷⁹⁰⁻⁸⁴⁷ that had been previously incubated with 1. mM Hesperadin for 1 hour and later co-crystallized in the presence of 1 mM Hesperadin by microseeding against the same reservoir condition. Also in this case, diffraction quality crystals are obtained by microseeding with seeds derived from crystals of G96V-Aurora-B⁶⁰⁻³⁶¹/INCENP⁷⁹⁰⁻⁸⁴⁷.

### Example 1

### Generation of active Aurora-B/INCENP

Xenopus laevis (x) INCENP is an 873-residue protein (Figure 1A). Its first about 500 residues, of unknown structure, are not significantly related to other proteins. Residues 500-730 are predicted to form a coiled-coil. This segment is followed by the IN-box, which binds Aurora-B (Adams, 2000; Bishop, 2002; Kaitna, 2000). To identify the minimal Aurora-B-binding domain, different C-terminal segments of xINCENP for binding to the kinase domain of xAurora-B are tested. For this, the backbone of the pGEX-6P vector to support polycistronic expression in bacteria is modified. The N-terminal domain (NTD) of Aurora kinases does not influence kinase activity and is not important for binding INCENP (Cheetham, 2002), and is omitted from the expression constructs. The NTD may be important for sub-cellular localization of Aurora kinases, although this may not be the case for Aurora-B (Scrittori, 2004). Residues 60-361 of x Aurora-B (Aurora-B60-361) are expressed from the first cassette of the vector as a C-terminal fusion to the glutathione-S-transferase (GST). The second cassette is used to express different untagged segments of the IN-box. Two segments of xINCENP, INCENP790-847 and INCENP790-856, are chosen for further studies. After affinity purification and proteolytic removal of GST, the Aurora-B/IN-box complexes are found to co-purify through ion exchange and size exclusion chromatography (SEC). Aurora-B60-361/INCENP790-847 and Aurora-B60-361/INCENP790-856 elute from a Superdex 200 SEC column with an apparent molecular weight (MW) of about 40 kDa, in good agreement with the sum of the theoretical individual MWs (42743 or 43599, respectively), indicative of a 1:1 complex (Figure 1B).

Bacterially expressed Aurora-A and Aurora-A/TPX2 are active and auto-phosphorylate on Thr288 in the activation loop (Bayliss, 2003). Western blotting (WB) of Aurora-B/INCENP before and after treatment with protein phosphatase 1 (PP1) revealed that both Aurora-B/INCENP790-847 and Aurora-B/INCENP790-856 are phosphorylated on Thr248. Phosphorylation of Thr248 is not observed with a kinase-dead (KD) mutant of Aurora-B/INCENP, suggesting that the phosphorylation of Thr248 in bacteria is due to autocatalysis.

When Aurora-B/INCENP is treated with PP1 phosphatase, the mobility of INCENP790-856 is shifted, while no equivalent mobility shift is observed for INCENP790-847 or for an lNCENP790-856 segment bound to KD-Aurora-B, suggesting that the phosphorylation of INCENP is due to Aurora.-B (Figure 1C). Using mass spectrometry, two prominent phosphorylation sites in INCENP790-856 are found that are concomitantly phosphorylated. These are determined to correspond to the serines of the Thr-Ser-Ser (TSS) motif encompassing residues 848-850 ofxINCENP, explaining the different mobility behavior ofINCENP790-847 (lacking the TSS motif) and INCENP790-856. In summary, both Aurora-B60-361/TNCENP790-847 and Aurora-B6Q-361/INCENP790-856 complexes purified from bacteria are phosphorylated on the activation loop. Aurora-B60-361/INCENP790-856 is also phosphorylated on both serines of the TSS motif. The phosphorylation of the TSS motif is important for Aurora-B activation (Bishop, 2002; Bolton, 2002; Honda, 2003; Kang, 2001). It is found that Aurora-B/INCENP790-856 is about 7-fold more active than Aurora-B/INCENP790-847 on substrates such as histone H3 and MBP (Figure 1D). The results confirm that Aurora-B activation is a two-step process. INCENP binding activates Aurora-B (compare lanes 3 and 5 in Figure 1D). INCENP phosphorylation on the TSS motif elicits maximal activation. Lacking the TSS motif, Aurora-B/INCENP790-847 is a state of intermediate activation of Aurora-B.

### Example 2

### a) Crystallization and structure determination

Aurora-B/INCENP790-847 is crystallized and its crystal structure determined to a resolution of 1.8 Å. The crystals belong to space group P21 and contain two Aurora-B/INCENP complexes in the asymmetric unit (Table 1). The structure is determined by molecular replacement (MR) using the atomic model of Aurora-A as search model (PDB code 1OL5). After MR, σA-weighted electron density maps (Collaborative Computational Project, 1994) calculated with model phases and native amplitudes revealed changes in the relative orientation of the small and large lobes of Aurora-B relative to Aurora-A (detailed below). These changes are modeled and the structure of the IN-box is built in the electron density. The final model of Aurora-B/INCENP790-847 complex is refined to an R-factor of 19.7 and a free-R factor of 22.6 (Table 1).

### b) Overall structure of Aurora-B/INCENP

Aurora-B/INCENP⁷⁹⁰⁻⁸⁴⁷ is shown in Figure 2. Aurora-B has the classical bilobal protein kinase fold. The N-terminal lobe (N-lobe, residues 86-174) is rich in β-strands and is implicated in nucleotide binding and the interaction with kinase regulators. The C-terminal lobe (C-lobe, residues 175-347) is mainly α-helical and serves as a docking site for the substrate and also contains the conserved residues that direct phosphate transfer. The ATP-binding pocket lies at the interface between the lobes. The IN-box forms a molecular crown that extends linearly for about 70 Å over three quarters of the perimeter of the small lobe. In its revolution, the IN-box also makes extensive contacts with the second half of the C-terminal extension of Aurora-B (residues 348-361), which is well ordered until residue 355 (Figure 2). Residues 388-403 in the equivalent extension of human Aurora-A are disordered in the Aurora-A/TPX2 crystals (Bayliss, 2003). About 3600 Å² of surface area are buried in the Aurora-B/INCENP complex. The IN-box does not have a hydrophobic core, and uses its hydrophobicity to pack against the surface of Aurora-B.

### c) The Aurora-B/INCENP interface

Only four residues in INCENP⁷⁹⁰⁻⁸⁴⁷ are fully conserved, Glu791^{IN}) Trp801^{IN}, Pro818^{IN} and Phe837^{IN} (Superscripts 'IN', 'Au-A' and 'Au-B' will be used to identify residues in INCENP, Aurora-A and Aurora-B, respectively). Another half-dozen residues are conserved in more than 75% of INCENP orthologues (Figure 2D). The Asp790^{IN}-Glu791^{IN} pair is disordered in the electron density. The visible part of the IN-box starts at residues 798^{IN}. This segment precedes the αA^{IN} helix, and sits at the interface with the small lobe near the glycine-rich loop. Pro799^{IN}, Trp801^{IN} and Ala802^{IN} pack against an exposed hydrophobic pocket of Aurora-B (Figure 3B), and the side chain of Trp801^{IN} is stacked against the side chain of Arg111^{Au-B}. The Aurora-B pocket is lined by Gly96^{Au-B}, and the absence of a side chain at this position allows the side chains of Trp801^{IN} and Ala802^{IN} to approach Aurora-B closely. Substitution of Gly96^{Au-B} with valine does not disrupt the interaction of Aurora-B with the IN-box, and a G96V-Aurora-B/INCENP⁷⁹⁸⁻⁸⁴⁷ complex crystallizes like the wild type complex. The only difference with the structure of the wild type complex is that Val96 prevents the occupation of the hydrophobic patch by the Trp801^{IN} side chain, so that the N-terminal segment of INCENP is pushed into solvent and is disordered. Thus, loss of the binding contribution of Trp801^{IN} is not sufficient to disrupt the Aurora-B/INCENP complex.

Helix αA^{IN} is amphipathic and binds Aurora-B with several hydrophobic side chains that pack against an equally hydrophobic exposed patch of Aurora-B (Figure 3C). Gln814^{IN} is also part of the helical interface and its side chain is hydrogen-bonded to the main chain amide and carbonyl groups of Ile 118^{Au-B}. This interaction docks the C-terminal part of the αA^{IN} helix onto Aurora-B. The side chains of the last two residues in the αA^{IN} helix, Tyr815^{IN} and Tyr816^{IN}, orient approximately at a right angle, creating a pocket that hosts the side chain of Pro353^{Au-B} in the C-tcrminal extension ofAurora-B (Figure 3D). The INCENP chain turns sharply with Pro818^{IN} to follow the surface of the small lobe, leading into the αB^{IN} helix. This is also amphipathic, and engages its hydrophobic residues to bind Aurora-B (Figure 3E). Finally the INCENP chain progresses into an irregular helical segment, αC, which has the character of a 3₁₀ helix. The hydrophobic side chains of Pro831^{IN}, Leu833^{IN}, Leu836^{IN}, and Phe837^{IN} mediate the interaction with Aurora-B in this region (Figure 3F). Phe837^{IN} is fully conserved in INCENP sequences. Its side-chain points vertically into a deep pocket on the Aurora-B surface lined by Ile166^{Au-B}, Leu129^{Au-B}, Glu135^{Au-B}, Leu136^{Au-B} and Arg139^{Au-B}.

### d) Comparison of Aurora-B/INCENP with Aurora-A/TPX2

TPX2 binds Aurora-A with two separate stretches of polypeptide chain (Figure 4A), TPX2⁷⁻²¹ and TPX2³⁰⁻⁴³, separated by an intervening flexible and disordered segment (Bayliss, 2003). TPX2⁷⁻²¹ sits on Aurora-A roughly where the αB-αC loop and the helix αC of the IN-box dock on Aurora-B (Figure 4B), but the main chains of TPX2⁷⁻²¹ and INCENP run in opposite directions. C-terminally to the TPX2⁷⁻²¹ segment, the TPX2 chain inverts its direction. TPX2³⁰⁻⁴³ binds in a α-helical conformation between the N- and C-terminal lobes of Aurora-A. There is no structural equivalent of the TPX2³⁰⁻⁴³ segment in INCENP, as the INCENP main chain emerging from the rear face of the Aurora-B small lobe broadly overlaps with TPX2⁷⁻²¹

The main chains of TPX2⁷⁻²¹ and of INCENP arc not directly superimposable. The only noteworthy element of similarity in these segments is an 'aromatic plug' formed by the side chains of two similarly spaced residues, Phe19^{TPX2}/Tryr8^{TPX2} and Tyr825^{IN}/Phe837^{IN}, (Figure 4C). The side chains of these aromatic residues orient differently on the surface of their cognate Aurora partners, but insert into equivalent cavities (Figure 4C).

To test the importance of the aromatic plug of INCENP for Aurora-B binding, alanine mutants of Tyr825 (Y825A), Phe837 (F837A) and their combination are created within the frame of the bacterial co-expression system. Y825A-INCENP⁷⁹⁰⁻⁸⁴⁷ binds almost normally to Aurora-B, as judged by the amounts of co-expressed mutant INCENP co-purifying with Aurora-B upon affinity purification on glutathione-sepharose beads (Figure 4D). The presence of an aromatic residue at position 825 is a conserved feature of INCENP, and this residue is likely to contribute significantly to binding. It is possible that this mutation is tolerated because of the docking on Aurora-B of αA^{IN} and αC^{IN}, which are positioned upstream and downstream from the αB^{IN} helix containing Y825. F837A-INCENP⁷⁹⁰⁻⁸⁴⁷, on the other hand, is unable to bind Aurora-B, and so is the double mutant Y825A-F837Λ, underscoring the importance of this residue for the Aurora-B/INCENP interaction.

### e) Activation of Aurora-B by INCENP⁷⁹⁰⁻⁸⁴⁷

If one excludes the 'aromatic plug' described above, the comparison of Aurora-A/TPX2 and Aurora-B/INCENP reveals genuinely distinct binding modes. In the Aurora-A/TPX2 complex, TPX2³⁰⁻⁴³ triggers a lever-arm-like movement on the activation loop of Aurora-A, which as a consequence adopts a fully extended conformation (Bayliss, 2003). The activation loop of protein kinases plays a critical role in substrate recognition for phosphotransfer and its fully extended conformation is the most characterizing hallmark of active protein kinases (Nolen, 2004). In the absence of TPX2, the activation loop of Aurora-A adopts a considerably less stretched conformation, and correspondingly the kinase activity of apo-Aurora-A is reduced (Bayliss, 2003; Nowakowski, 2002).

In contrast with Aurora-A/TPX2, INCENP does not make any direct contacts with the activation loop along its revolution around the small lobe of Aurora-B (Figure 3). The C-lobe of Aurora-B is aligned onto the C-lobe of Aurora-A and the conformation of the activation loop compared (Figure 5A). The activation loop of INCENP⁷⁹⁰⁻⁸⁴⁷-bound Aurora-B is fully extended and perfectly superimposable to that of Aurora-A in Aurora-A/TPX2. There is very clear electron density for the phosphate group on Thr248 ^{Au-B}, suggesting that most if not all of Aurora-B is phosphorylated on this residue.

This observation suggests that INCENP forces an extended conformation of the Aurora-B activation loop through an allosteric mechanism. In the absence of a structure of apo-Aurora-B, it cannot be excluded that the phosphorylation of Thr248^{Au-B} is sufficient to trigger the active conformation independently of INCENP. Due to the fact that the phosphorylated activation loop of apo-Aurora-A is unable to adopt a fully active conformation (Bayliss, 2003; Nowakowski, 2002), it is suspected that the activation loop of apo-Aurora-B phosphorylated on Thr248 is also unlikely to attain the fully active conformation unless it is bound to INCENP.

### f) Aurora-B/INCENP⁷⁹⁰⁻⁸⁴⁷: an intermediate state of activation

If the conformation of the activation loop in Aurora-B/INCENP⁷⁹⁰⁻⁸⁴⁷ is indicative of an active kinase, two more features may explain why Aurora-B/INCENP⁷⁹⁰⁻⁸⁴⁷ represents an intermediate state of activation rather than the fully active state exemplified by Aurora-A/TPX2. The first feature is the suppression of the interaction between Lys122^{Au-B} and Glul41^{Au-B}. This is probably the primary reason why Aurora-B/INCENP⁷⁹⁰⁻⁸⁴⁷ is only partially active relative to Aurora-B/INCENP⁷⁹⁰⁻⁸⁵⁶. In all active kinases this pair of residues engages in a buried ion pair required to orient the α and β-phosphates of ATP for phosphotransfer (Huse, 2002). The distance of the ε amino group of Lys 122 and the carboxylate oxygens of Glu141 increases to ~5.5 Å in the Aurora-B/INCENP complex, too far to delineate a significant interaction (Figure 5C). On the other hand, the ion pair is perfectly aligned in the Aurora-A/TPX2 structure (2.7 Å). Because both residues reside in the N-lobe, the N-lobes of Aurora-B and Aurora-A are superimposed to identify the possible causes of this structural difference. The N-lobe of Aurora-A and Aurora-B can be superimposed with a root mean square (rms) deviation of 0.70 Å over 88 equivalent Cα atoms. The equivalent Cα atoms of Lys122^{Au-B} and Lys162^{Au-A} arc only 0.1 Å apart, and somewhat unexpectedly also the side chains of these residues superimpose well. The temperature factors of the side chain atoms of Lys 122^{Au-B} are low (25 Å² for the ε amino nitrogen). The conformational stability of Lys122^{Au-B} indicates that Glu141^{Au-B} does not play *per se* a tethering function on Lys122^{Au-B}. It is suspected that the function of Glu141^{Au-B} may become important for catalysis when the phosphates of ATP are in the active site.

Being Lys122^{Au-B} and Lys162^{Au-A} in equivalent positions, the loss of the ion pair with Glu141^{Au-B} must be due to a difference in the position of the latter. Indeed, the Cα atoms of Glu141^{Au-B} and Glu181^{Au-A} are 1.2 Å apart, and the side chain of Glu141^{Au-B} is rotated towards the C-lobe relative to its Aurora-A equivalent (Figure 5C). Glu141^{Au-B} resides on the αC helix of Aurora-B. The deviation of its position relative to the equivalent residue in Aurora-A could be explained by a ~15° counterclockwise rotation of the αC^{Au-B} helix along its axis (Figure 5C). Likely, this rotation is caused by Phe837^{IN}, whose aromatic side chain pushes against the side chain of Leu138^{Au-B} (Figure 5C). The relatively small rotation imposed by Phe837^{IN} on the αC helix produces significant effects at a distance from Phe837, resulting in the unusual position of Glu141^{Au-B}.

The second feature characterizing Aurora-B/INCENP⁷⁹⁰⁻⁸⁴⁷ as an intermediate state of activation is the opening of the catalytic cleft at the interface between the N- and C-lobes, which in Aurora-B is 15° more open than in Aurora-A (Figure 5D-E). This is seen without significant variations in all independent views of Aurora-B/INCENP⁷⁹⁰⁻⁸⁴⁷ (Table 1). Using ESCET (Schneider, 2002), it is determined that the N- and C-lobes of Aurora-B behave essentially as rigid-bodies in the rotation, and that the opening of the lobes results from a ~15° rotation around an axis passing approximately through three hinge residues, Glu177^{Au-B}, Leu222^{Au-B}, and Ala233^{Au-B}, whose main chain φ and ψ angles diverge significantly in the Aurora-B/INCENP and Aurora-A/TPX2 complexes (Figure 5D-E). A model of Aurora-B/INCENP is created with a reduced interlobal angle by superimposing the N- and C-terminal lobes of Aurora-B on the equivalent lobes of the crystallographic model of Aurora-A/TPX2. We analyzed the regions of communication between the N- and C-lobe, concentrating in particular on the C-terminal tail of Aurora-B and on the catalytic cleft at the interface of these domains (Figure 2A). As observed before, the C-terminal segment of Aurora B is fully extended. The side chain of Pro353^{Au-B} fits snugly into the pocket formed by Tyr815^{IN} and Tyr816^{IN} (Figure 3D). Because the C-terminal tail cannot be stretched further, closure of the catalytic cleft of Aurora-B would cause the proline side chain to slip out of the pocked formed by Tyr815^{IN} and Tyr816^{IN}. This analysis suggests that the C-terminal tail of Aurora-B, which is connected to the Aurora-B N-lobe via INCENP, might stabilize an open and partially inactive state of Aurora-B, as shown schematically in Figure 5F-G.

Remarkably, modeling of the Aurora-B domains onto the Aurora-A scaffold also revealed a single severe steric clash in the catalytic cleft between the N- and C-lobes. With a narrower cleft, Glu141^{Au-B} (N-lobe) clashes against Gly236^{Au-B} in the activation loop (C-lobe, Figure 5C). Because the unusual position ofGlu141^{Au-B} causing this clash is due to the rotation of the αC^{Au-B} helix towards the C-lobe (Figure 5C), this observation suggests very strongly that the rotation of the αC^{Au-B} helix and the increased intcrlobal angle in Aurora-B are related phenomena. The significance of this analysis is that the position of the αC^{Au-B} helix and more specifically of Glu141^{Au-B} in Aurora-B/INCENP⁷⁹⁰⁻⁸⁴⁷ is incompatible with the narrow catalytic cleft observed in Aurora-A. It is conceivable that a back rotation of αC^{Au-B} in a position that would restore the interaction of Glu 141 with Lys122 would also allow the closure of the catalytic cleft.

### g) Becoming fully active

Two hypotheses are proposed relating to the observations described in the previous paragraph. In the first hypothesis, the opening of the catalytic cleft is envisioned as a consequence of the rotation of the αC^{Au-B} helix. This rotation can be ascribed to the side chain of Phe837^{IN}, which pushes against the side chain of Leu138^{Au-B} on the αC^{Au-B} helix (Figure 5C). Once rotated towards the large lobe, the side chain ofGlu141^{Au-B} pushes on the main chain of the N-terminal portion of the activation loop, acting as a wedge to promote the opening of the catalytic cleft. In the second hypothesis, the C-terminal tail of Aurora-B is key to force an open state of the catalytic cleft via the interaction of Pro353^{Au-B} with Tyr 815^{IN} and Tyr 816^{IN}. With an open cleft, the αC helix may relax into an energetically more favorable position by rotating away from its expected position in the active kinase, exemplified by the Aurora-A/TPX2 complex. These effects are described schematically in Figures 5F-G. The effects are not exclusive, but it seems that Phe837^{IN} is more likely to take the center stage, given its short distance from the TSS motif, whose phosphorylation is expected to revert the impediments towards full activation. Furthermore, whilst Phe837^{IN} is fully conserved in INCENP sequences, the C-terminal tail of Aurora-B is poorly conserved, and so are Tyr815^{IN} and Tyr816^{IN} with which it interacts.

### Examples 3

### a) Mechanism of Hesperadin binding to the Aurora-B active site

Aurora-A and Aurora-B are overexpressed in a wide range of human tumors and are thought to represent promising targets for anti-cancer pharmacological therapy (Keen, 2004; Sausville, 2004). How Aurora kinases contribute to cellular transformation is still debated, but their involvement in different aspects of mitosis suggests that their deregulation might cause chromosome instability and aneuploidy, a frequent feature of tumor cells (Keen, 2004). The ATP-binding pocket of protein kinases represents an ideal target for pharmacological therapy, and differences in the nature of residues lining the ATP-binding cavity explains the remarkable selectivity of many kinase inhibitors (Kecn, 2004; Sausville, 2004). The effect of Hespcradin has been described in Hauf, 2003. At the cellular level, the inhibitory effects of this compound phenocopy loss of Aurora-B function by RNA interference or dominant-negative approaches, whilst the phenotype bears no resemblance to that observed upon loss of Aurora-A function by similar methods (Hauf, 2003).

With the availability of crystallization conditions for the Aurora-B/INCENP complex, it is possible to gain an insight into the mechanism of inhibition of Aurora-B by the indolinone Hesperadin. The 1.8 Å resolution crystal structure of the ternary complex G96V-Aurora B⁶⁰⁻³⁶¹/INCENP⁷⁹⁰⁻⁸⁴⁷/Hesperadin is determined. Hesperadin binds in the ATP site of Autora-B (Figure 6A), and is therefore an ATP competitive inhibitor. Excellent density for Hesperadin is visible in 'omit' electron density maps and allows unambiguous identification of the orientation of Hesperadin in the Aurora-B active site (Figure 6B-C). The indolinone ring system of Hesperadin sits in the catalytic cleft so that the oxygen and nitrogen atoms are hydrogen-bonded to the main chain carbonyl and amide groups of Glu171 and Ala173, respectively. At one end of the indolinone ring, the central phenyl ring is in van der Waals contact with the side chains of Leu99, Vall 07 and Glu177 and points towards the entry site to the catalytic cleft. The following phenylaznine is squeezed between Gly176 and the side chain of Leu99, and precedes a piperidine group that is exposed to solvent At the opposite end of the indolinone ring, the sulfonamide moiety points into the active site, with the sulfur and oxygen atoms roughly occupying the same position of the α-phosphate of ATP.

### b) Description of activity and inhibition by Hesperadin

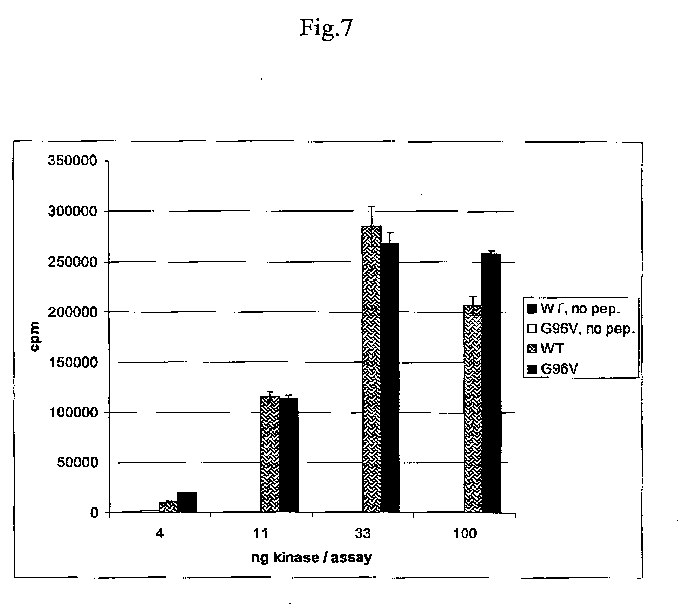

In order to determine whether the G96V-Aurora-B⁶⁰⁻³⁶¹/INCENP⁷⁹⁰⁻⁸⁴⁷ complex exhibits a change in its activity, a radioactive kinase filterbinding assay is applied to compare it to wildtype (wt) Aurora-B⁶⁰⁻³⁶¹/INCENP⁷⁹⁰⁻⁸⁴⁷. The ATP-K_{M} value for the G96V mutant (83 µM) is similar to the wt enzyme (61 µM). The kinase assays are run in the presence of 100 µM ATP using 10 µM of a substrate peptide (see Materials and Methods). As can be seen in Figure 7, both protein complexes display almost identical activity over a wide concentration range, while no activity is determined in the absence ofpeptide.

Hesperadin is a potent inhibitor of human Aurora-B (Hauf, 2003). To determine whether Hesperadin inhibits also xAurora-B /INCENP complexes, a kinase filterbinding assay is employed for IC₅₀-determinations. Both, the G96V and the wt enzyme complex are used at 5 nM concentration in the assays. An IC₅₀ of 25 nM ±13 nM for G96V-Aurora-B⁶⁰⁻³⁶¹/INCENP⁷⁹⁰⁻⁸⁴⁷ and 7 nM ± 2 nM for wt-Aurora-B⁶⁰⁻³⁶¹/INCENP⁷⁹⁰⁻⁸⁴⁷ is determined, which is comparable to the value determined for monomeric human Aurora-B (44 nM) using assay conditions which allow similar signal/noise ratios. Similar results are obtained using a non-radioactive ATP-consumption assay.

### Example 4

### Assays to determine activity of an Aurora-B/INCENP complex

Purified xAurora-B/INCENP is snap frozen and stored for further use at -80°C in desalting buffer (50 mM Tris/Cl pH 8.0, 150mM NaCl, 0.1mM EDTA, 0.03% Brij-35, 10% glycerol, 1mM DTT). xAurora-B/INCENP is incubated in the presence or absence of a test compound dilutions in DMSO prior to addition of substrate peptide and P33-γ-ATP. Samples without a test compound or without substrate peptide are used as high or low-controls, respectively. IC50 values are determined using Graph Pad Prism software.

For the filter-binding kinase assay (reaction volume 50 ul /well), 96-well PP-Microplates (Greiner, 655 201) are used. To 10µl compound in 25% DMSO are added 30µl PROTEIN-MIX (166 µM ATP,1x kinase buffer [50mM Tris/Cl pH7.5,25mM MgC12, 25mM NaCl], 10-20 ng xAurora-B/INCENP) followed by an 15 min incubation at room temperature (agitating, 350rpm). To this, 10µl PEPTIDE-MIX (2x kinase buffer, 5mM NaF, 5mM DTT, 1 µCi P33-γ-ATP, 50 µM peptide (e.g. Biotin-EPLERRLSLVPDS (SEQ ID NO:5), or Biotin-EPLERRLSLVPDSEPLERRLSLVPDS, or Biotin-EPLERRLSLVPKM (SEQ ID NO:6)or multimers thereof, or Biotin-LRRWSLGLRRWSLGLRRWSLGLRRWSLG (SEQ ID NO:7)) is added. The mixture is incubated for 60 min at room temperature (agitating, 350rpm), followed by addition of 180 µl 6.4% TCA (final concentration: 5%) to stop the reaction. Subsequently, a Multiscreen filtration plate (Millipore, MAIP N0B 10) is equilibrated with 100 µl 70% ethanol and 1 % TCA prior to addition of the stopped kinase reaction. Following 5 washes with 180 µl 1% TCA, the lower part of the plate is dried, 25 µl scintillation cocktail (Microscint, High Efficiency LSC-Cocktail, Packard, 6013611) is added and samples are counted using a Wallac 1450 MicroBeta Liquid Scintillation counter at the appropriate settings.

Activity is assessed using an ATP-consumption assay in the presence of substrate peptide: To 10 µl compound/DMSO mix (final assay DMSO-concentration 5%), 20 µl protein mix (1.5x kinase buffer, 1.5mM NaF, 2.5mM DTT, 40-80ng xAurora-B/INCENP) are incubated in an OptiPlate-96 (opaque, Perkin Elmer) for 15 min, (room temperature, agitating). To this, 10 µl ATP-mix (1x kinase buffer, 1.25 µM ATP) is added, followed by addition of 10 µl peptide mix (1x kinase buffer, 75 µM peptide, for sequences see above). The mixture is incubated for 90 min (room temperature, agitating), followed by addition of 20 µl of PKlight ATP detection reagent (Cambrex, #LT07-200). The mixture is incubated (10 min, room temperature, agitating) and samples are measured at 565 nm using a luminescence-reader (Wallac Victor 2,1240 Multilabel Counter) at 0.1 sec integration time.

One futher aspect of the invention is a peptide selected from the group consisting of SEQ TD NO:5 and SEQ ID NO:6 or multimers thereof.

The compounds designed or selected using the methods of the invention may find use in a variety of applications. As will be appreciated by those in the art, mitosis may be altered in a variety of ways; that is, one can affect mitosis either by increasing or decreasing the activity of a component in the mitotic pathway. Stated differently, mitosis may be affected. (e.g. disrupted) by disturbing equilibrium, either by inhibiting or activating certain components. Also included within the definition of Aurara-B for these purposes are variants and/or fragments of Aurora-B. The compounds designed or selected using the methods of the invention are used to treat cellular proliferation diseases. Disease states which can be treated by the methods and compositions provided herein include, but are not limited to, cancer (further discussed below), autoimmune disease, arthritis, graft rejection, inflammatory bowel disease, proliferation induced after medical procedures, including, but not limited to, surgery, angioplasty, and the like.

Thus, in one embodiment, the invention herein includes application of the compounds to cells or individuals afflicted or impending affliction with any one of these disorders or states. The compounds, compositions and methods provided herein are particularly deemed useful for the treatment of cancer including but not limited to AIDS-related cancer such as Kaposi's sarcoma; bone related cancer such as Ewing's family of tumors and asteosarcoma; brain related cancer such as adult brain tumor, childhood brain stem glioma, childhood cerebellar astrocytoma, childhood cerebral astrocytoma/malignant glioma, childhood ependymoma, childhood medulloblastoma, childhood supratentorial primitive neurocctodermal tumors, childhood visual pathway and hypothalamic glioma and other childhood brain tumors; breast cancer; digestive/gastrointestinal related cancer such as anal cancer, extrahepatic bile duct cancer, gastrointestinal carcinoid tumor, colon cancer, esophageal cancer, gallbladder cancer, adult primary liver cancer, childhood liver cancer, pancreatic cancer, rectal cancer, small intestine cancer and stomach (gastric) cancer; endocrine related cancer such as adrenocortical arcinoma, gastrointestinal carcinoid tumor, islet cell carcinoma (endocrine pancreas), parathyroid cancer, pheochromocytoma, pituitary tumor and thyroid cancer; eye related cancer such as intraocular melanoma, and retinoblastoma; genuitourinary related cancer such as bladder cancer, kidney (renal cell) cancer, penile cancer, prostate cancer, transitional cell renal pelvis and ureter cancer, testicular cancer, urethral cancer, Wilms' tumor and other childhood kidney tumors; germ cell related cancer such as childhood extracranial germ cell tumor, extragonadal germ cell tumor, ovarian germ cell tumor and testicular cancer; gynecologic related cancer such as cervical cancer, endometrial cancer, gestational trophoblastic tumor, ovarian epithelial cancer, ovarian germ cell tumor, ovarian low malignant potential tumor, uterine sarcoma, vaginal cancer and vulvar cancer; head and neck related cancer such as hypopharyngeal cancer, laryngeal cancer, lip and oral cavity cancer, metastatic squamous neck cancer with occult primary, nasopharyngeal cancer, oropharyngeal cancer, paranasal sinus and nasal cavity cancer, parathyroid cancer and salivary gland cancer; hematologic/blood related cancer such as leukemias, such as adult acute lymphoblastic leukemia, childhood acute lymphoblastic leukemia, adult acute myeloid leukemia, childhood acute myeloid leukemia, chronic lymphocytic leukemia, chronic myelogenous leukemia and hairy cell leukemia; and lymphomas, such as AIDS-related lymphoma, cutaneous T-cell lymphoma, adult Hodgkin's lymphoma, childhood Aodgkin's lymphoma, Flodgkin's lymphoma during pregnancy, mycosis fungoides, adult non-Hodgkin's lymphoma, childhood non-Hodgkin's lymphoma, non-Hodgkin's lymphoma during pregnancy, primary central nervous system lymphoma, Sezary syndrome, cutaneous T-cell lymphoma and Waldenstrom's macroglobulinemia and other hematologic/blood related cancer such as chronic myeloproliferative disorders, multiple myeloma/plasma cell neoplasm, myelodysplastic syndromes and myelodysplastic/myeloproliferative diseases; lung related cancer such as non-small cell lung cancer and small cell lung cancer musculoskeletal related cancer such as Ewing's family of tumors, osteosarcoma, malignant fibrous histiocytoma of bone, childhood rhabdomyosarcoma, adult soft tissue sarcoma, childhood soft tissue sarcoma and uterine sarcoma; neurologic related cancer such as adult brain tumor, childhood brain tumor, brain stem glioma, cerebellar astrocytoma, cerebral astrocytoma/malignant glioma, ependmoma, medulloblastoma, supratentorial primitive neuroectodermal tumors, visual pathway and hypothalamic glioma and other brain tumors such as neuroblastoma, pituitary tumor and primary central nervous system lymphoma; respiratory/thoracic related cancer such as non-smal cell lung cancer, smal cell lung cancer, malignant mesothelioma, thymoma and thymic carcinoma; skin related cancer such as cutaneous T-cell lymphoma, Kaposi's sarcoma, melanoma; Merkel cell carcinoma and skin cancer.
The compounds of the invention may be used in combination with other therapies or anticancer agents including surgery, radiotherapy, endocrine therapy, biologic response modifiers, hyperthermia and cryotherapy, agents to attenuate any adverse effect (e.g. anti emetics) and other chemotherapeutic drugs. Such conjoint treatment may be achieved by way of simultaneous, sequential or separate administration of the individual components of the treatment. Chemotherapeutics that may be used in combination with the compounds of the present invention are selected from, but not limited to hormones, hormonal analogues and antihormonals (e.g. tamoxifen, torenufene, raloxifene, fulvestrant, megestrol acetate, flutamide, nilutamidc, bicalutamide, arninogluteddmide, cyproterone acetate, finasteride, buserelin acetate, fludrocortinsone, Buoxymesterone, medroxyprogesterone, octreotide), aromatase inhibitors (e.g. anastrozole, letrozole, liar6zole, vorozole, exemcstane, atamestane,), LHRH agonists and antagonists (e.g. goserelin acetate, luprolide), inhibitors of growth factor function, (such growth factors include for example platelet derived growth factor and hepatocyte growth factor such inhibitors include growth factor antibodies, growth factor receptor antibodies and tyrosine kinase inhibitors such as gefitinib, imatinib, lapatinib and trastuzumab); antimetabolites (e.g. antifolates like methotrexate, raltitrexed, pyrimidine analogues like 5-fluorouracil capecitabine and gemeitabine, purine and adenosinc analogues such as mercaptopurinc thioguanine, cladnbine and pentostatin, cytarabine, fludarabine); antitumor antibiotics (e.g. anthracyclines like doxorubicin, daunorubicin, cpirubicin and idarubicin, mitomycin-C, bleomycin dactinomycin, plicamycin,, streptozocin); platinum derivatives (e.g. cisplatin, oxaliplatin, carboplatin); alkylating agents (e.g. estramustine, meclorethamine, mclphalan, chlorambucil, busulphan, dacarbazine, cyclophosphamide, ifosfamide, temozolomide, nitrosoureas such as carmustinc and lornustine, thiotepa) ; antimitotic agents (e.g. vinca alkaloids like vinblasttt1e. vindesine, vinorelbine and vincristine; and taxabes like paclitaxel, docetaxel); topoisomerase inhibitors (e.g. epipodophyllotoxins like etoposide and etopophos, teniposide, amsacrine, topotecan, irinotecan, mitoxantrone) and miscellaneous chemotherapeutics such as hydroxyurea, amifostine, anagrelide, clodmnate, filgrastin, interferone alpha, leucovorin, rituximab, procarbazine, levamisole, mesna, mitotane, pamidronate and porfimer.

The compounds according to the invention may be administered by oral, transdennal or parenteral route or by inhalation. The compounds according to the invention are present as active ingredients in conventional preparations, e.g. in compositions consisting essentially of an inert pharmaceutical carrier and an effective dose of the active substance, such as for example plain or coated tablets, capsules, lozenges, powders, solutions, suspensions, emulsions, syrups, suppositories, transdermal systems, etc. An effective dose of the compounds according to the invention is between 1 and 100, preferably between 1 and 50, most preferably between 5-30 mg/dose, for oral administration, and between 0.001 and 50, preferably between 0.1 and 10 mg/dose for intravenous or intramuscular administration. For inhalation, solutions containing 0.01 to 1.0, preferably 0.1 to 0.5% of active substance are suitable according to the invention. For inhalation, the use of powders is preferred. It is also possible to use the compounds according to the invention as a solution for infusion, preferably in physiological saline or nutrient salt solution.

### REFERENCES

Adams, R. R., Wheatley, S. P., Gouldsworthy, A. M., Kandels-Lewis, S. E., Carmena, M., Smythe, C., Gerloff, D. L., and Earnshaw, W. C. (2000). INCENP binds the Aurora-related kinase AIRK2 and is required to target it to chromosomes, the central spindle and cleavage furrow. Curr Biol 10, 1075-1078..

Andrews, P. D., Knatko, E., Moore, W. J., and Swedlow, J. R. (2003). Mitotic mechanics: the auroras come into view. Curr Opin Cell Biol 15, 672-683.

Bayliss, R., Sardon, T., Vernos, I., and Conti, E. (2003). Structural basis of Aurora-A activation by TPX2 at the mitotic spindle. Mol Cell 12, 851-862.

Bishop, J. D., and Schumacher, J. M. (2002). Phosphorylation of the carboxyl terminus of inner centromere protein (INCENP) by the Aurora-B Kinase stimulates Aurora-B kinase activity. J Biol Chem 277, 27577-27580.
Bohm, H.J., (1992). The Computer Program LUDI: A New Method for the De Novo Design of Enzyme Inhibitors. J. Comp. Aid. Molec. Design, 6, 61-78.

Bolton, M. A., Lan, W., Powers, S. E., McClcland, M. L., Kuang, J., and Stukenberg, P. T. (2002). Aurora-B Kinase Exists in a Complex with Survivin and INCENP and Its Kinase Activity Is Stimulated by Survivin Binding and Phosphorylation. Mol Biol Cell 13, 3064-3077.

Brooks, B.R., Bruccoleri R.E., Olafson B.D., States D.J., Swaminathan S., and Karplus M. (1983) CHARMM: A Program for Macromolecular Energy, Minimization, and Dynamics Calculations. J. Comp. Chem., 4, 187-217.

Carmma, M., and Earnshaw, W. C. (2003). The cellular geography of aurora kinases. Nat Rev Mol Cell Biol 4, 842-854.

Cheetham, G. M., Knegtel, R. M., Coll, J. T., Renwick, S. B., Swenson, L., Weber, P., Lippke, J. A., and Austen, D. A. (2002). Crystal structure of aurora-2, an oncogenic serine/threonine kinase. J Biol Chem 277, 42419-42422.

Collaborative Computational Project, N. (1994). The CCP4 Suite: Programs for Protein Crystallography. Acta Crystallogr D 50, 760-763.

Ditchfield, C., Johnson, V. L., Tighe, A., Elision, R., Haworth, C., Johnson, T., Mortlock, A., Keen, N., and Taylor, S. S. (2003). Aurora-B couples chromosome alignment with anaphase by targeting BubR1, Mad2, and Cenp-E to kinetochores. J Cell Biol 161, 267-280.

Eyers, P. A., and Maller, J. L. (2004). Regulation of Xenopus Aurora A activation by TPX2. J Biol Chem 279, 9008-9015.

Goodford, P. J. (1985). A computational procedure for determining energetically favorable binding sites on biologically important macromolecules. J. Mcd. Chem., 28, 849-857

GOODSELL, D.S. and OLSEN, A.J. (1990). Automated Docking of Substrates to Proteins by Simulated Annealing. Proteins: Sir, Func. and Genet., 8,195-202.

Gorbsky, G. J. (2004). Mitosis: MCAK under the aura of Aurora-B. Curr Biol 14, R346-348.

Hauf, S., Cole, R W., LaTerra, S., Zimmer, C., Schnapp, G., Walter, R, Heckel, A., van Mecl, J., Rieder, C. L., and Peters, J.-M. (2003). The small molecule Hesperadin reveals a role for Aurora-B in correcting kinetochore-microtubule attachment and in maintaining the spindle assembly checkpoint. J Cell Biol 161, 281-94.

Honda, R, Korner, R, and Nigg, E. A. (2003). Exploring the functional interactions between Aurora-B, INCENP, and survivin in mitosis. Mol Biol Cell 14,3325-3341.

Huse, M., and Kuriyan, J. (2002). The conformational plasticity of protein kinases. Cell 109,275-282.

Jones, T. A., Zou, J.-Y., and Cowan, S. W. (1991). Improved methods for building protein models in electron density map and the location of errors in these models. Acta Crystallogr A 47, 110-119.

Kaitna, S., Mendoza, M., Jantsch-Plunger, V., and Glotzer, M. (2000). Incenp and an aurora-like kinase form a complex essential for chromosome segregation and efficient completion of cytokinesis. Curr Biol 10,1172-1181.

Kang, J., Cheescman, T. M., Kallstrom, G., Velmurugan, S., Barnes, G., and Chan, C. S. (2001). Functional cooperation of Dam1, Ipll, and the inner centromere protein (INCENP)-related protein Slil5 during chromosome segregation. J Cell Biol 155, 763-774. Katayama, H., Zhou, H., Li, Q., Tatsuka, M., and Sen, S. (2001). Interaction and feedback regulation between STK15/BTAK/Aurora-A kinase and protein phosphatase 1 through mitotic cell division cycle. J Biol Chem 276,46219-46224.

Keen, N., and Taylor, S. (2004). Aurora-kinase inhibitors as anticancer agents. Nat Rev Cancer 4, 927-936.

Kuntz, I.D., Blancy, J.M., Oatley, S.J., Langridge, R., Ferrin, T.E. (1982). A Geometric Approach to Macromolecule-Ligand Interactions. J Mol Biol 161, 269-288.

Kufer, T. A., Sillje, H. H., Korner, R., Gruss, O. J., Meraldi, P., and Nigg, E. A. (2002). Human TPX2 is required for targeting Aurora-A kinase to the spindle. J Cell Biol 158, 617-623.

E. Lattman, "Use of the Rotation and Translation Functions", in Meth. Enzymol., 115, pp. 55-77 (1985);

Li, X., Sakashita, G., Matsuzaki, H., Sugimoto, K., Kimura, K., Hanaoka, F., Taniguchi, H., Furukawa, K., and Urano, T. (2004). Direct association with inner centromere protein (INCENP) activates the novel chromosomal passenger protein, Aurora-C. J Biol Chem 279, 47201-47211.

Meraldi, P., Honda, R., and Nigg, E. A. (2004). Aurora kinases link chromosome segregation and cell division to cancer susceptibility. Curr Opin Genet Dev 14, 29-36.

Murata-Hori, M., and Wang, Y.-L. (2002). The kinase activity of Aurora-B is required for kinetochore-microtubule interactions during mitosis. Current Biology 72, 894-899. Neumann, A., Wohlfarth, G., and Diekert, G. (1998). Tetrachloroethene dehalogenase from Dehalospirillum multivorans: cloning, sequencing of the encoding genes, and expression of the pccA gene in Escherichia coli. J Bacteriol 180, 4140-4145.

Nishibata, Y. and Itai, A. (1991). Automatic creation of drug candidate structures based on receptor structure. Starting point for artificial lead generation. Tetrahedron 41, 8985-8990 Nolen, B., Taylor, S., and Ghosh, G. (2004). Regulation of Protein Kinases; Controlling Activity through Activation Segment Conformation. Mol Cell 15, 661-675.

Nowakowski, J., Cronin, C. N., McRee, D. E., Knuth, M. W., Nelson, C. G., Pavletich, N. P., Rogers, J., Sang, B. C., Scheibe, D. N., Swanson, R. V., and Thompson, D. A. (2002). Structures of the cancer-related Aurora-A, FAK, and EphA2 protein kinases from nanovolume crystallography. Structure 10, 1659-1667.

Otwinowski, Z. (1993). Oscillation data reduction program. (Warrington, UK, Science and Engineering Research Council/Darcsbury Laboratory).

Pavletich, N. P. (1999). Mechanisms of cyclin-dependent kinase regulation: structures of Cdks, their cyclin activators, and Cip and INK4 inhibitors. J Mol Biol 287, 821-828.

Perozzo, R., Folkers, G., and Scapozza, L. (2004). Thermodynamics of protein-ligand interactions; history, presence, and future aspects. J. Recept. Signal Transduct. Res. Feb; 24(1-2):1-52.

Rossmann, M. G., ed., "The Molecular Replacement Method", Int. Sci. Rev. Ser., No. 13, Gordon & Breach, New York (1972)

Sasai, K., Katayama, H., Stenoien, D. L., Fujii, S., Honda, R., Kimura, M., Okano, Y., Tatsuka, M., Suzuki, F., Nigg, E. A., et al. (2004). Aurora-C kinase is a novel chromosomal passenger protein that can complement Aurora-B kinase function in mitotic cells. Cell Motil Cytoskeleton 59, 249-263.

Satinover, D. L., Leach, C. A., Stukenberg, P. T., and Brantigan, D. L. (2004). Activation of Aurora-A kinase by protein phosphatase inhibitor-2, a bifunctional signaling protein. Proc Natl Acad Sci U S A 101, 8625-8630.

Sausville, E. A. (2004). Aurora kinases dawn as cancer drug targets. Nat Med 10, 234-235.

Schneider, T. R. (2002). A genetic algorithm for the identification of conformationally invariant regions in protein molecules. Acta Crystallogr D Biol Crystallogr 58, 195-208.

Scrittori, L., Skoufias, D., Hans, F., Gerson, V., Sassone-Corsi, P., Dimitrov, S., and Margolis, R. (2004). A Small C-Terminal Sequence of Aurora-B Is Responsible for Localization and Function. Mol Biol Cell.

Tsai, M. Y., Wiese, C., Cao, K, Martin, O., Donovan, P., Ruderman, J., Prigent, C., and Zheng, Y. (2003). A Ran signalling pathway mediated by the mitotic kinase, Aurora A in spindle assembly. Nat Cell Biol 5, 242-248.

Vigneron, S., Prieto, S., Bernis, C., Labbe, J. C., Castro, A., and Lorca, T. (2004). Kinetochore localization of spindle checkpoint proteins: who controls whom? Mol Biol Cell 15, 4584-4596. von Ahsen, O., Bömer, T. (2005). High-Throughput Screening for Kinase Inhibitors.Chem. BioChem 6, 481-490.

Wallace, A. C., Laskowski, R. A., and Thornton, J. M. (1995). LIGPLOT: a program to generate schematic diagrams of protein-ligand interactions. Protein Eng 8, 127-134.

Walter, A. O. Seghezzi, W., Korver, W., Sheung, J., and Lees, F. (2000). The mitotic scrinelthreonine kinase Aurora2/ATK is regulated by phosphorylation and degradation. Oncogcne 19, 4906-4916.

Weiner, S.J., Kollman P.A., Case D.A., Singh U. C., Ghio C., Alagona G., Profeta S., Jr., and Weiner P. (1984). A new force field for molecular mechanical simulation of nucleic acids and proteins. J Am Chem Soc 106, 765-784.

Yasui, Y., Urano, T., Kawajiri, A., Nagata, K., Tatsuka, M., Saya, H., Furukawa, K., Takahashi, T., Izawa, I., and Inagaki, M. (2004). Autophosphorylation of a newly identified site of Aurora-B is indispensable for cytokinesis. J Biol Chem 279, 12997-13003.

**Table 1 Data Collection and Refinement Statistics**

| **Data Collection** | **G96V-AuroraB**^{**60- 361**}**/INCENP**^{**790-847**} | **AuroraB**^{**60 361**}**/ INCENP**^{**790-847**} | **G96V -AuroraB**^{**60-361**}**/ INCENP**^{**790-847**} **+ Hesperadin** |
|---|---|---|---|
| Space group | P2₁ | P2₁ | P2₁ |
| Beamline | ID14-1 | ID14-1 | ID14-1 |
| | 45.91 | 45.98 | 45.94 |
| Unit cell dimension (') | 66.28 | 66.86 | 67.04 |
| and angles (°) | 116.39 | 116.53 | 116.46 |
| | β=96.59 | β=96.78 | β=96.51 |
| Resolution (Å)^{a} | 25.0-1.9 (2.02-1.9) | 30.0-1.8 (1.86-1.8) | 20.0-1.8 (1.86-1.8) |
| Total observations | 377,259 | 629,603 | 365,547 |
| Unique reflections | 53258 | 64,766 | 67,250 |
| Data Completeness (%) | 96.4 (83.8) | 99.3 (94.4) | 96.9 (79.0) |
| Rsym (%)^{b} | 6.0 (29.U) | 5.1 (19.4) | 6.3 (22.1) |
| I/σI | 8.92 (2.16) | 29.3 (8.4) | 25.0 (2.9) |
| | | | |
| **Refinement** | | | |
| Resolution range (Å) | 20.0-1.9 | 20.0-1.8 | 20.0-1.8 |
| Rconv^{c}/Rfree^{d} | 20.7/24.5 | 19.7/22.6 | 20.3/22.6 |
| Number of protein atoms | 5,174 | 5,228 | 5,183 |
| Number of inhibitor atoms | - | - | 74 |
| Number of solvent atoms | 221 | 268 | 383 |
| Rmsd bond lengths (Å) | 0.009 | 0.016 | 0.012 |
| Rmsd bond angles (°) | 1.6 | 2.0 | 1.8 |
| Mean B-factor protein (Å²) | 38.8 | 19.5 | 30.2 |
| Mean B-factor inhibitor (Å²) | - | - | 34.1 |

| | | | |
|---|---|---|---|
| ^{a} Values in parentheses refer to the outer resolution shell. ^{b} R_{symm} = Σ\|1 - <I>\| / Σ I , where I is the observed intensity of a reflection and <I> is the average intensity obtained from multiple observations of symmetry-related reflections. ^{c} R_{conv} = Σ ∥Fc\| -\|Fc∥ /Σ \|Fₒ\|, where Fₒ and F_{c} are the observed and calculated structure factor amplitudes respectively. ^{d} R_{free} is equivalent to R_{conv} for a 5% subset of reflections not used in the refinement. | | | |

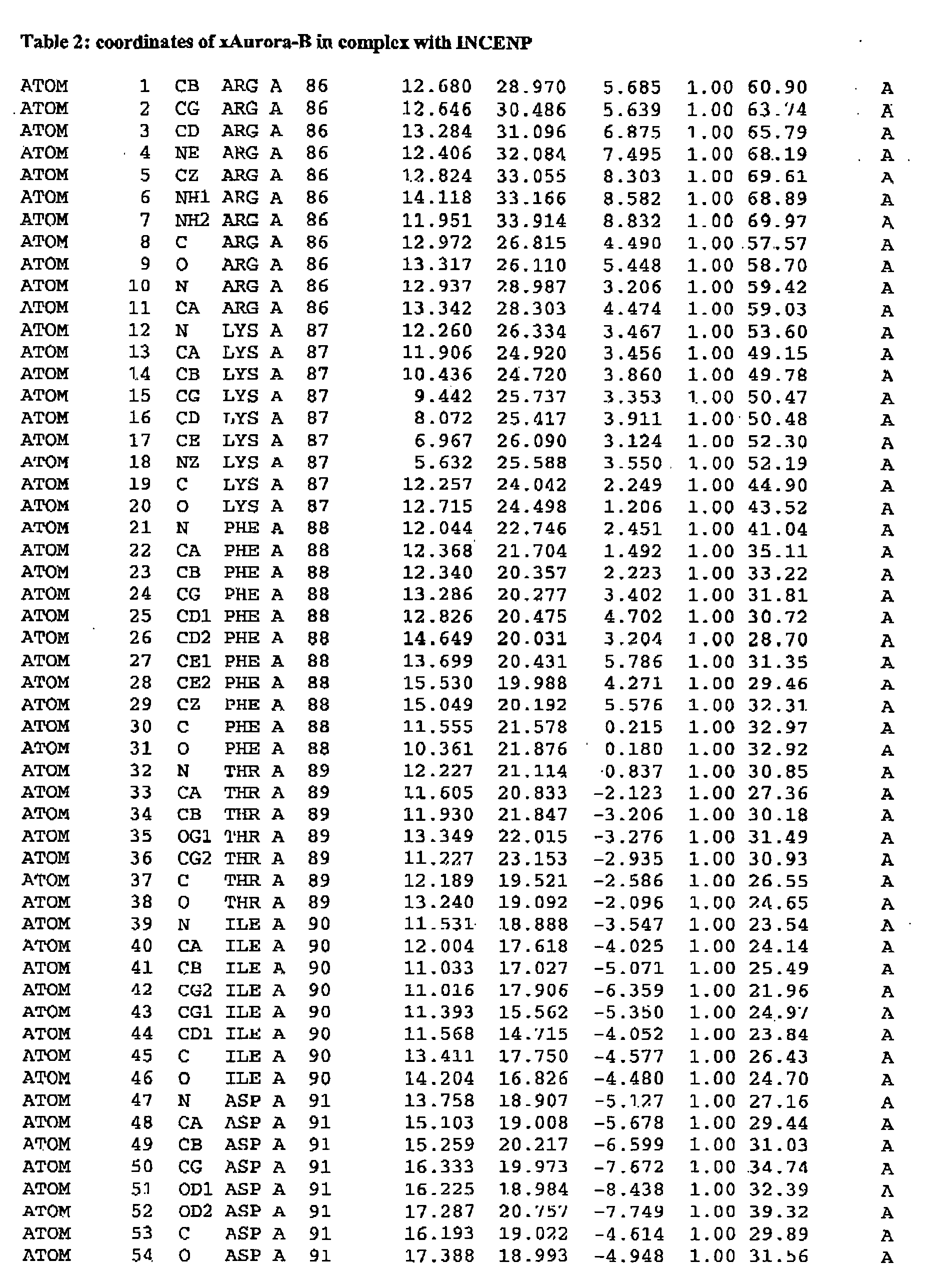

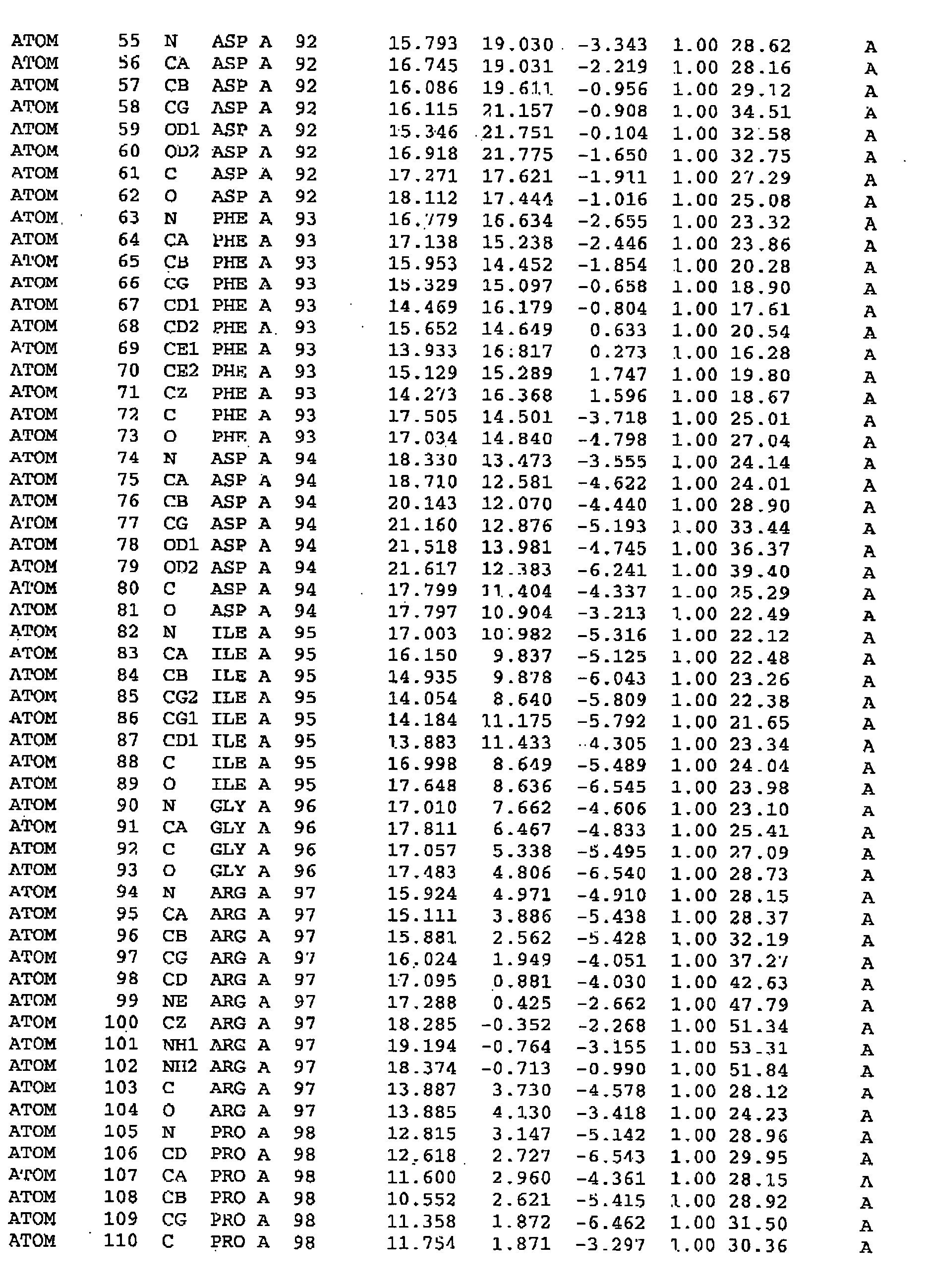

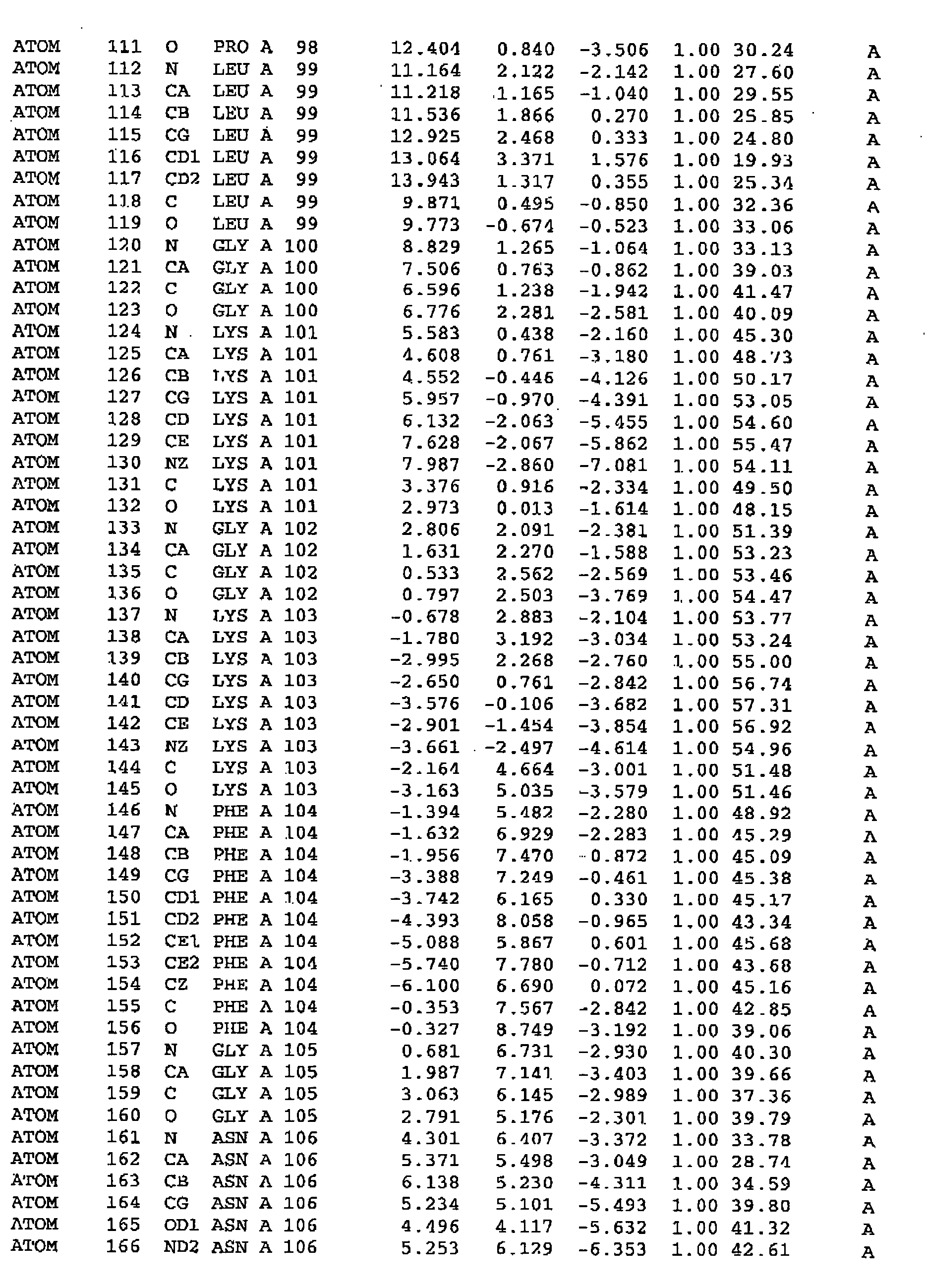

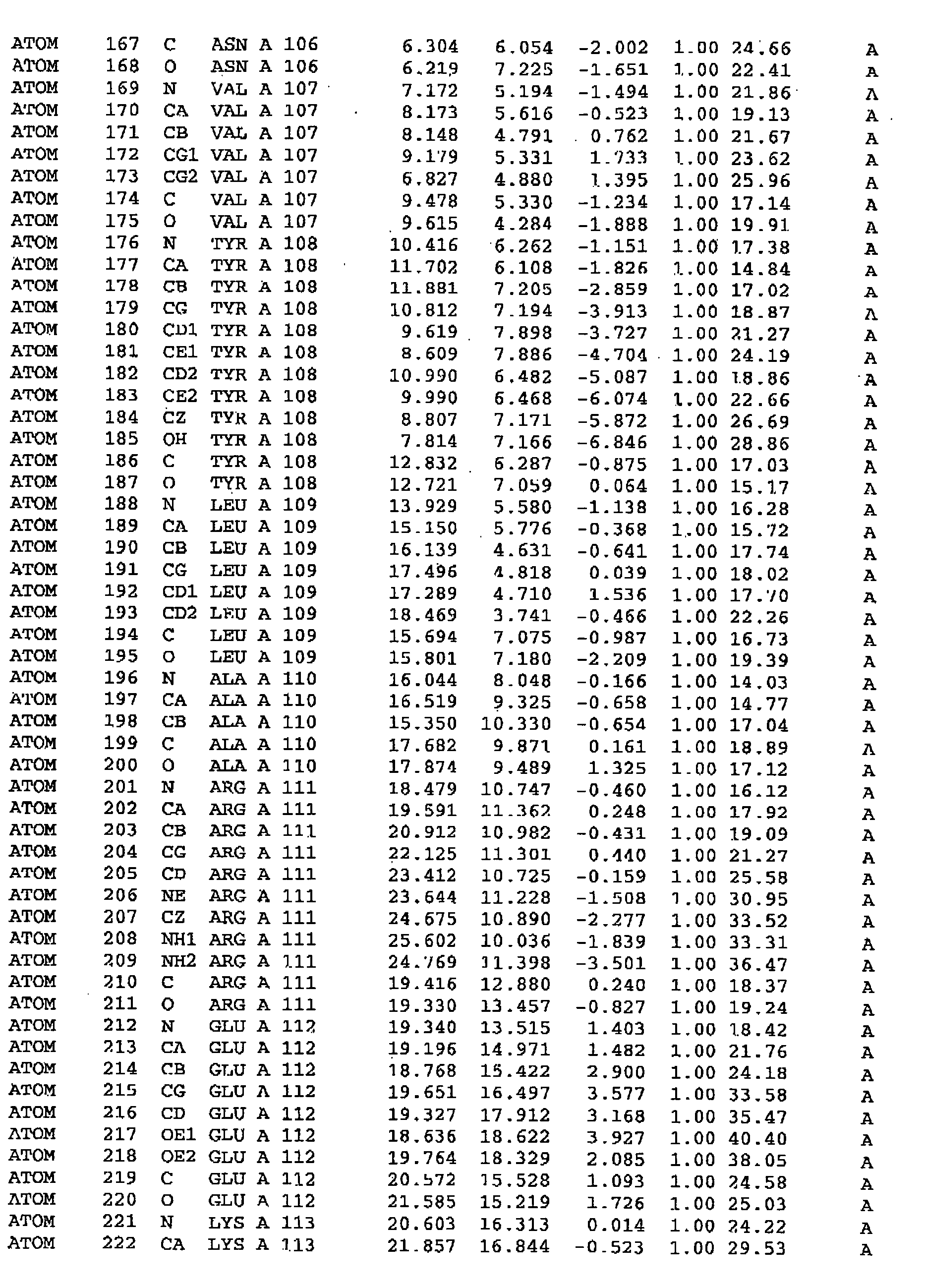

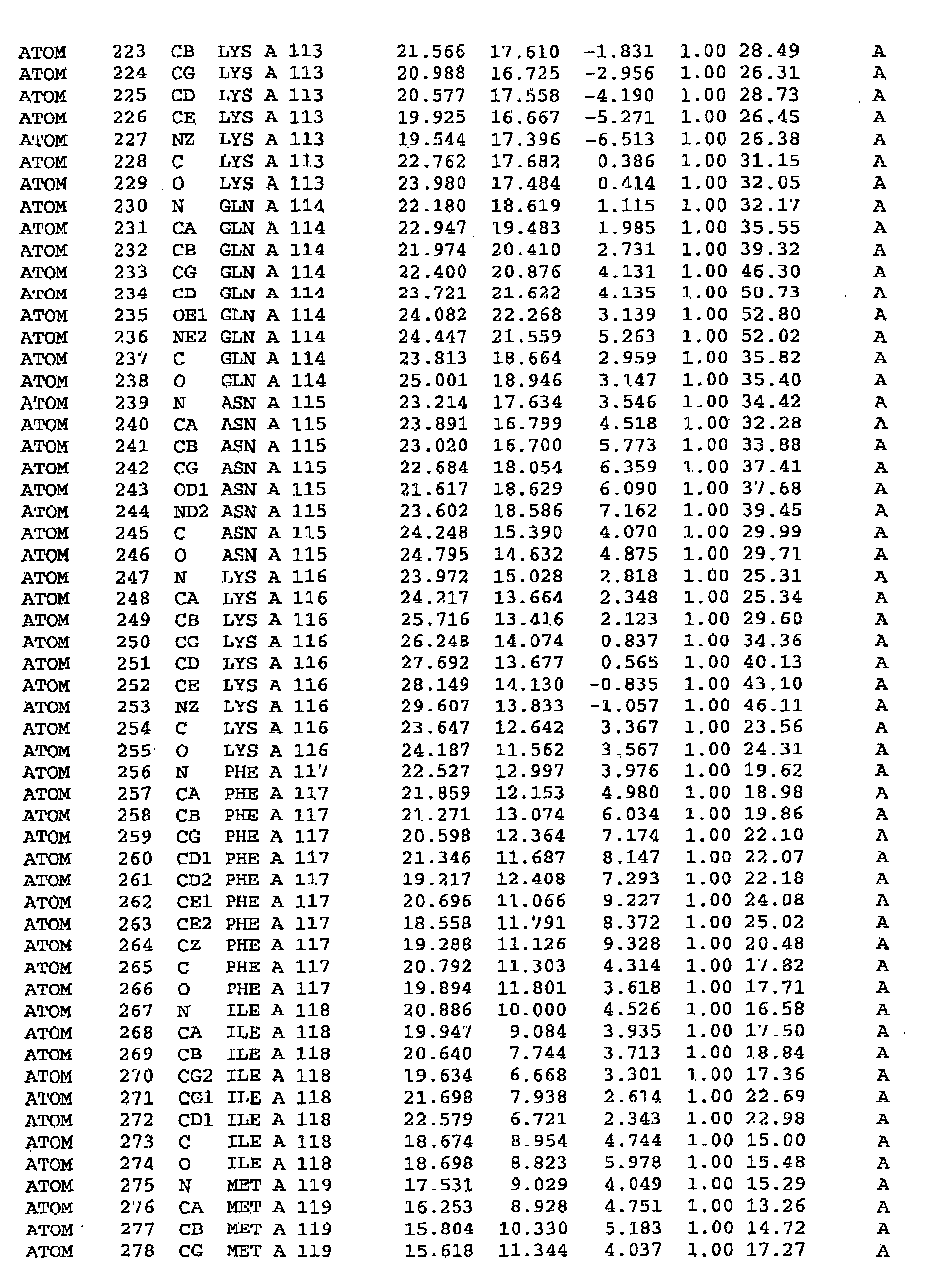

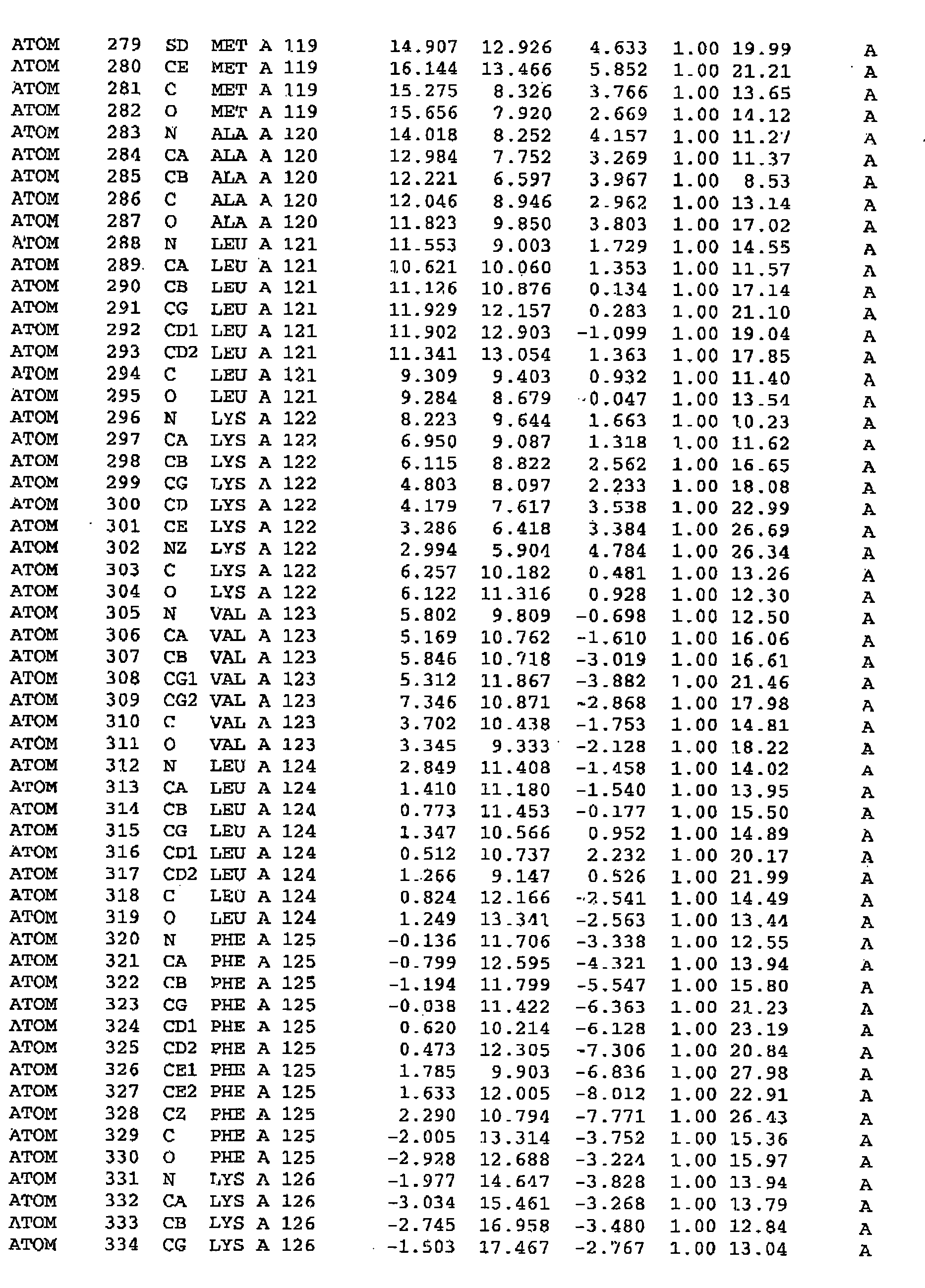

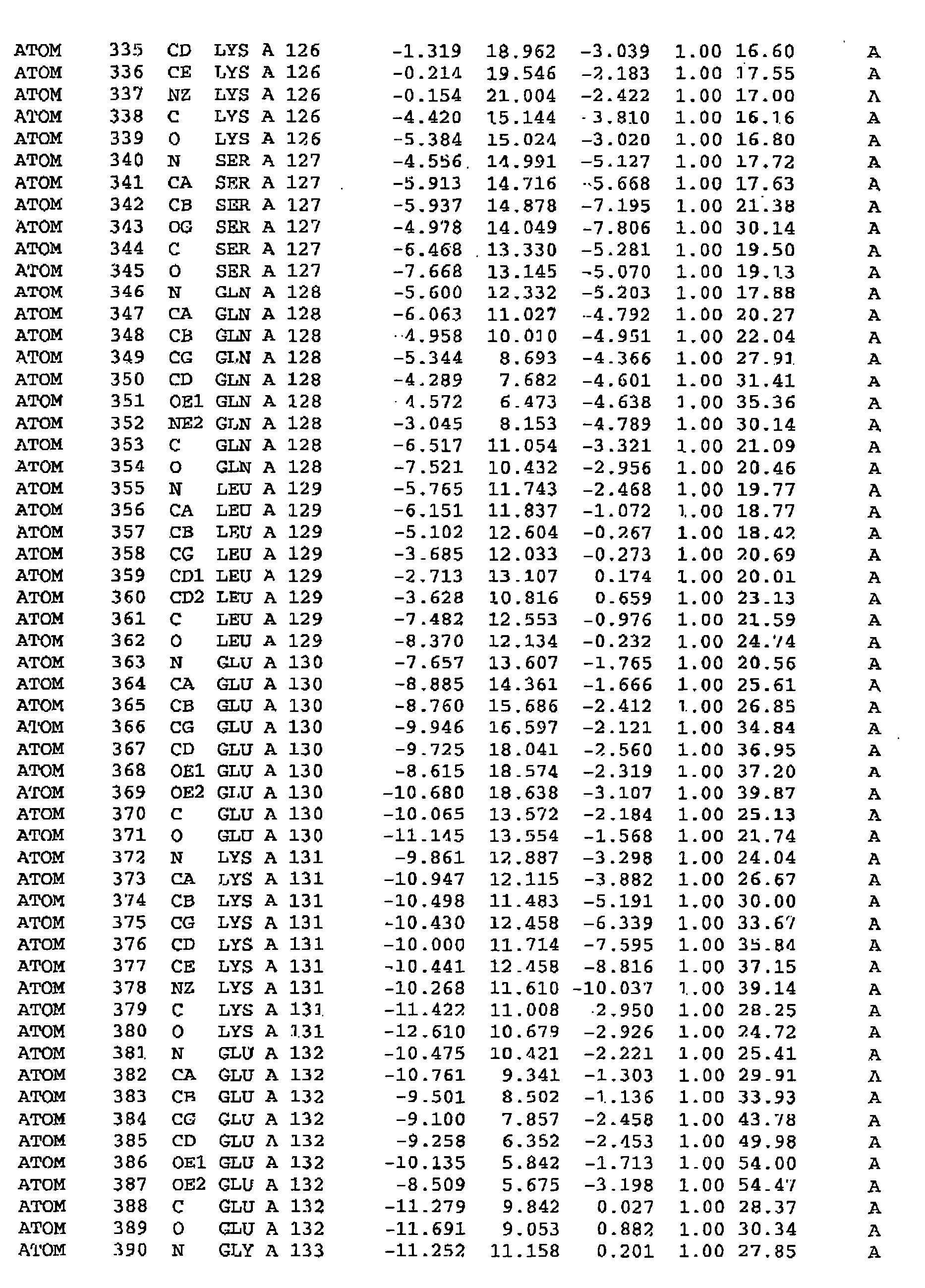

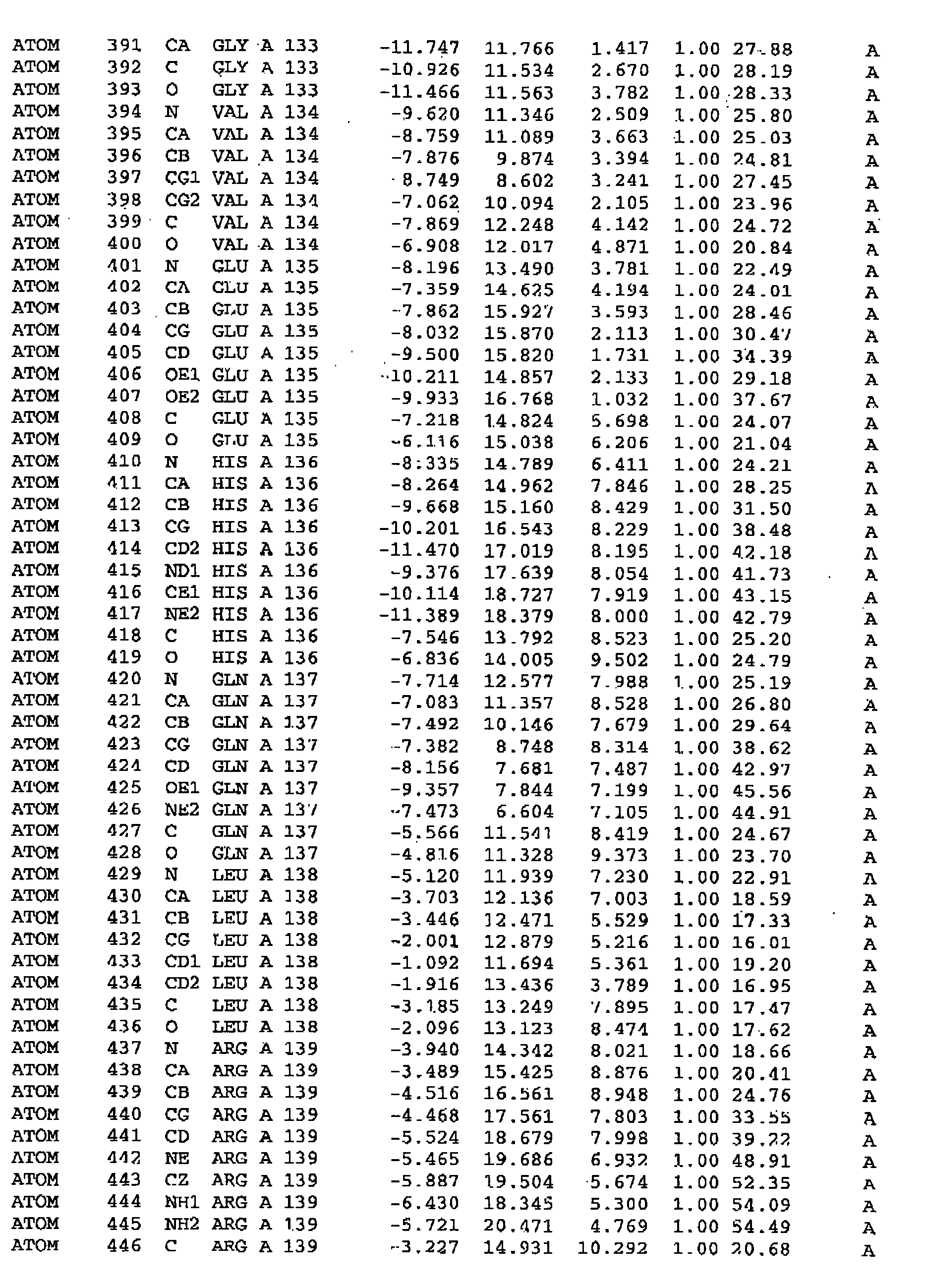

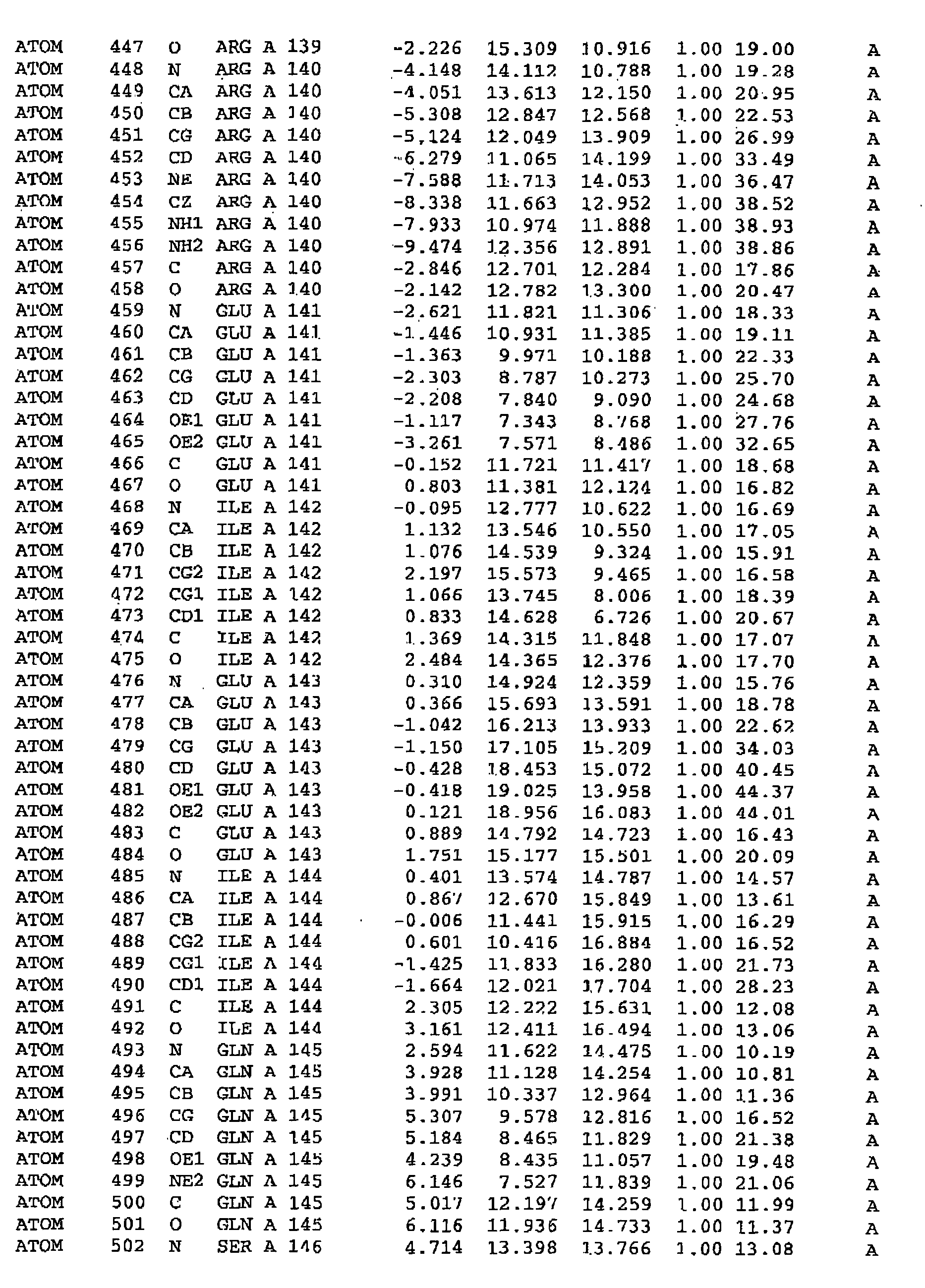

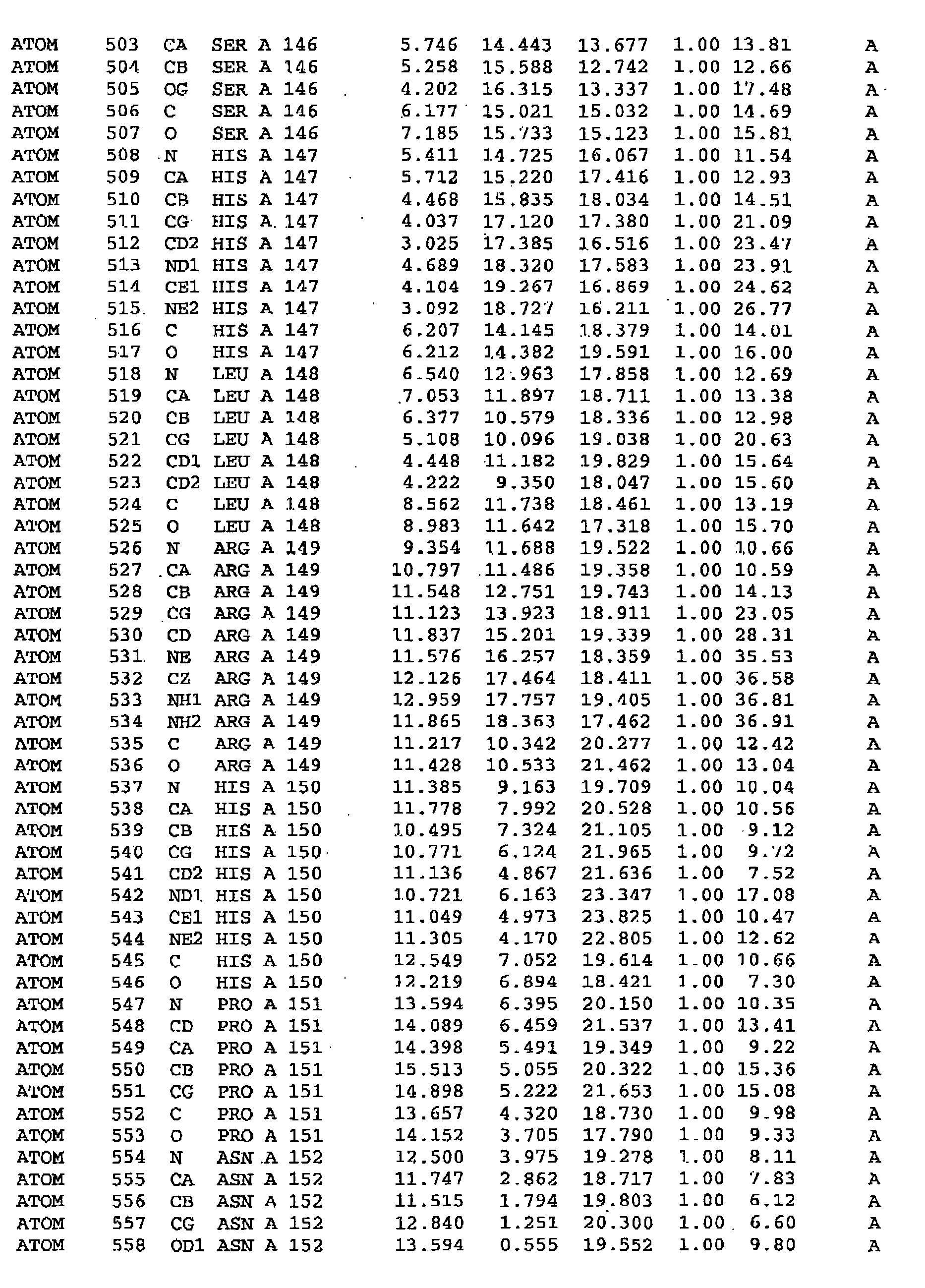

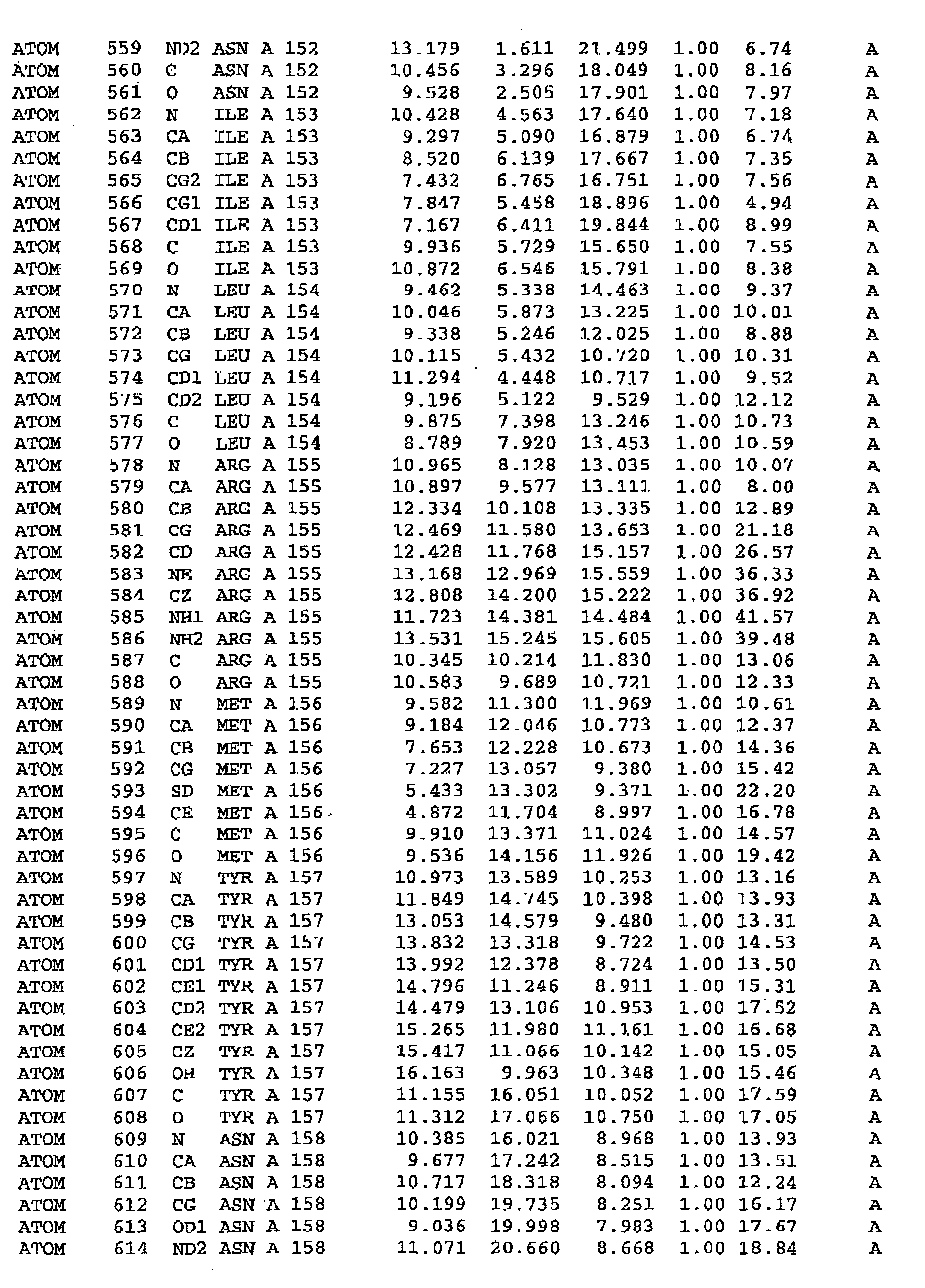

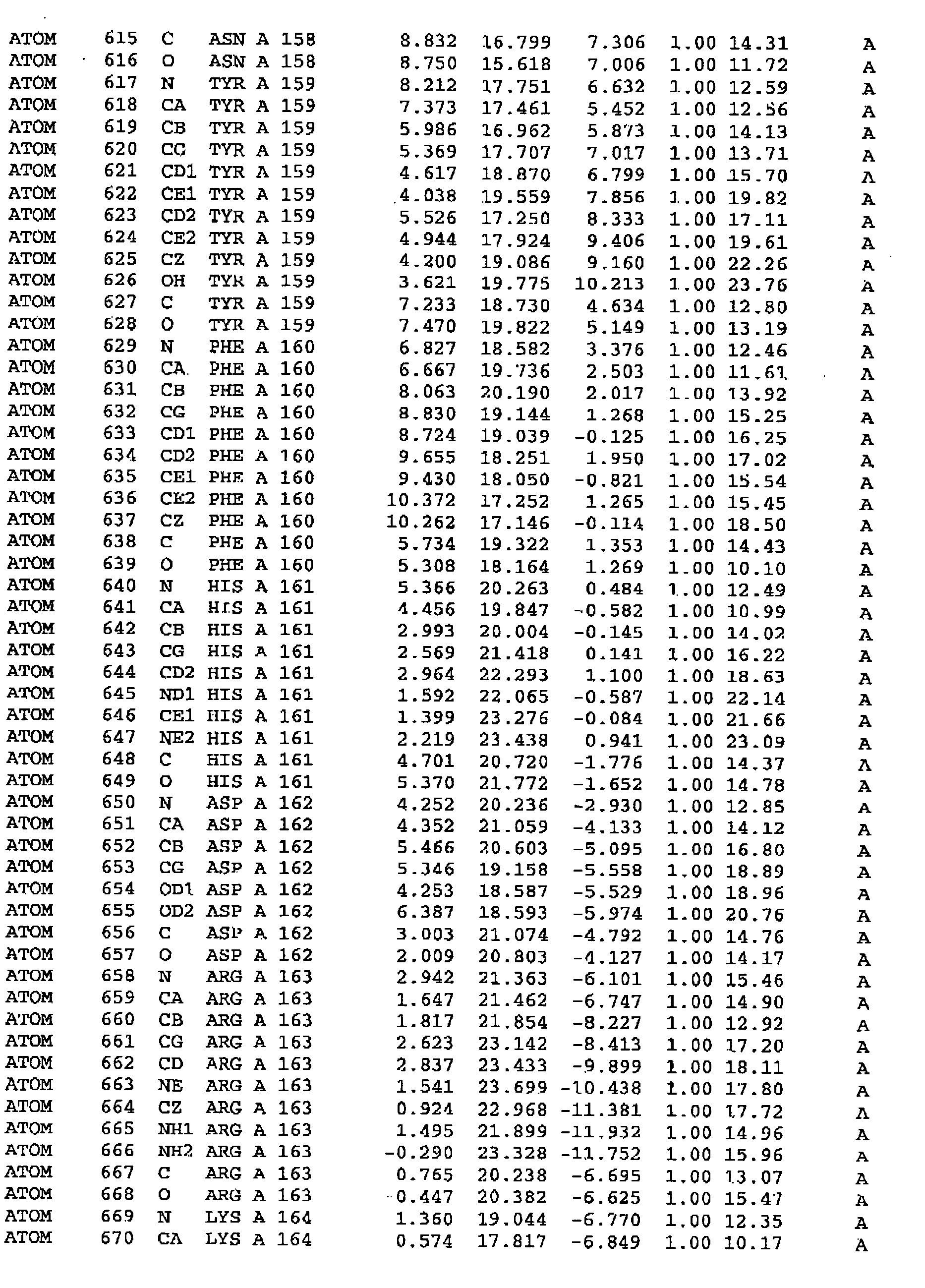

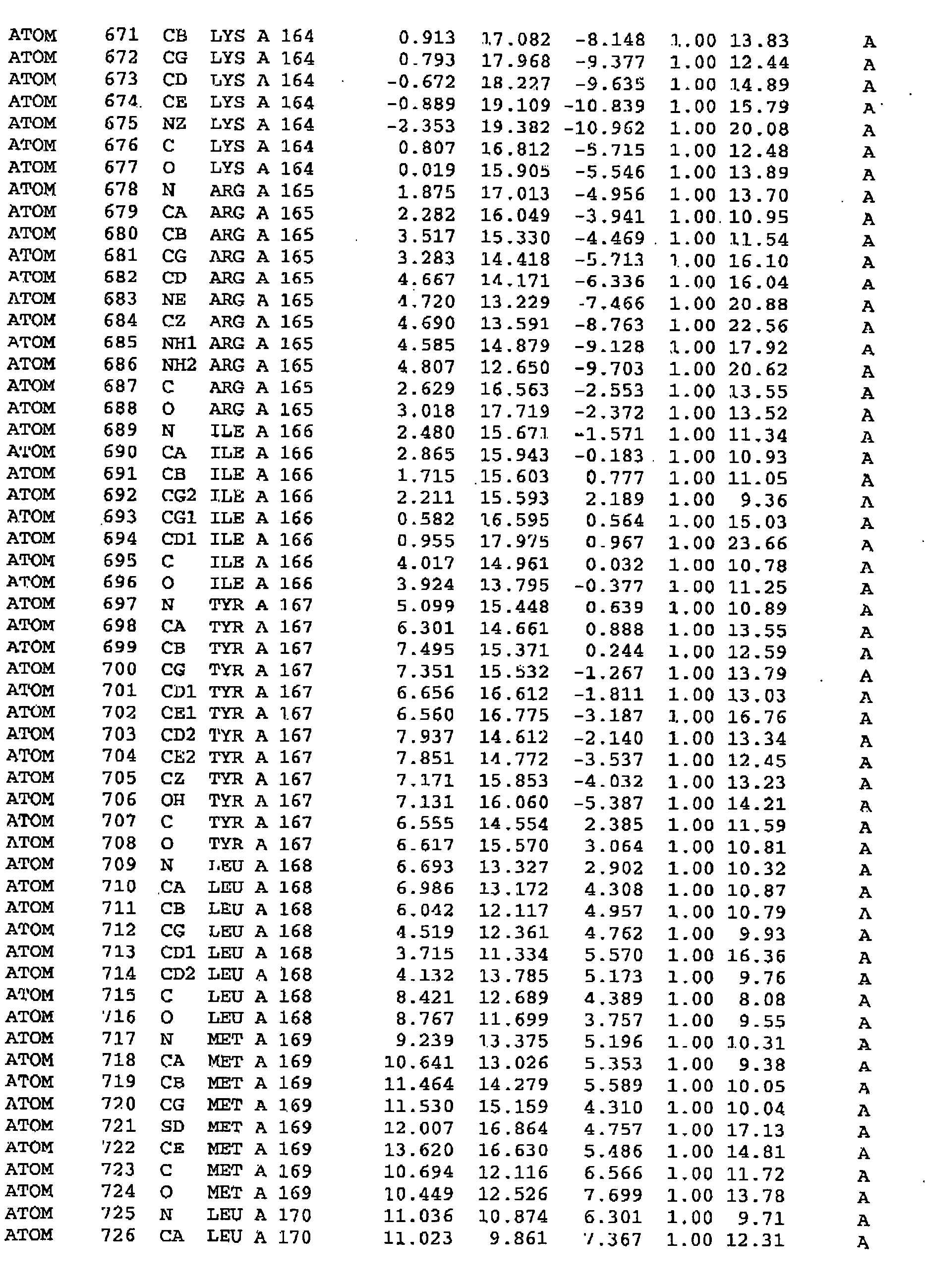

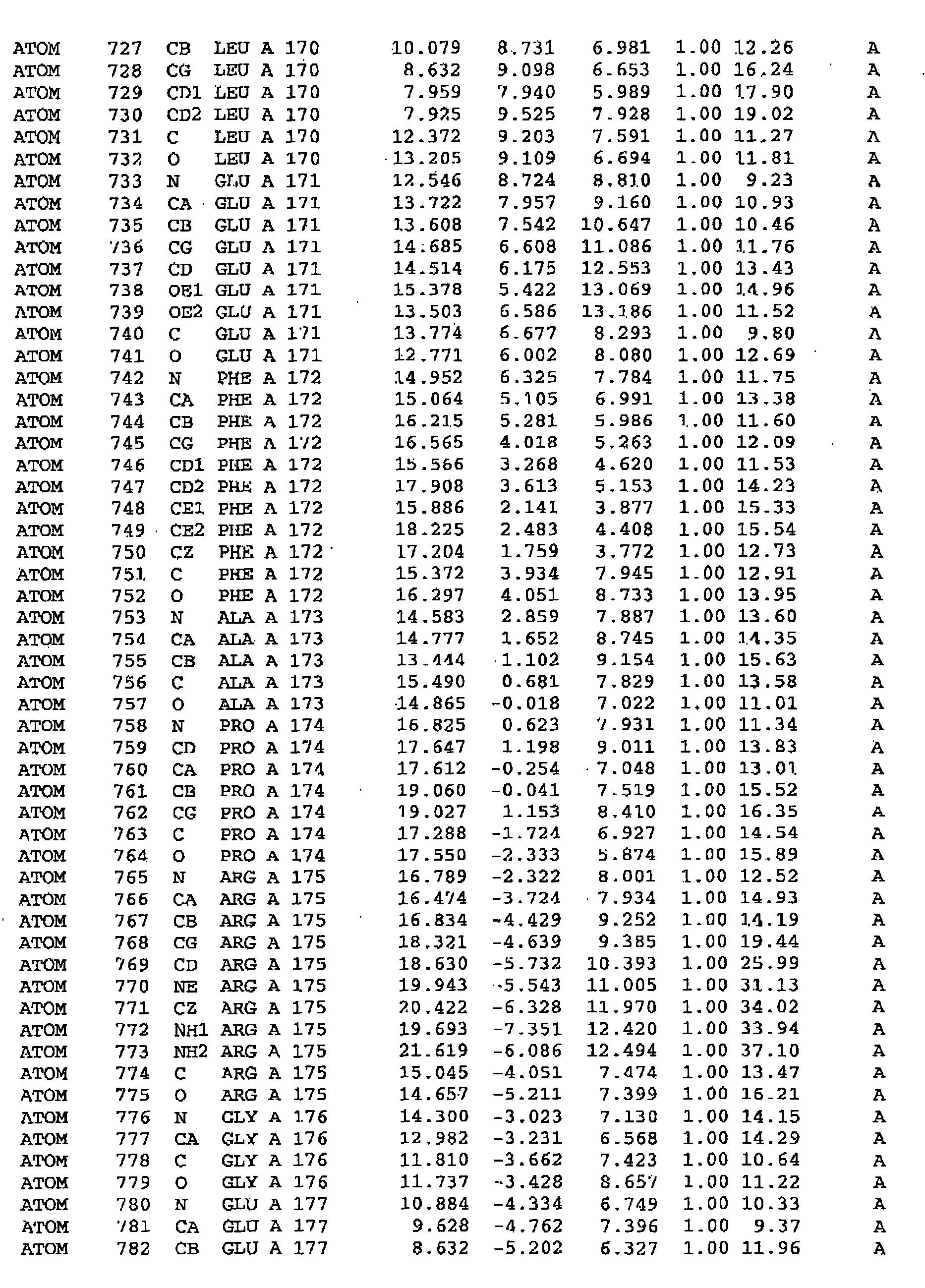

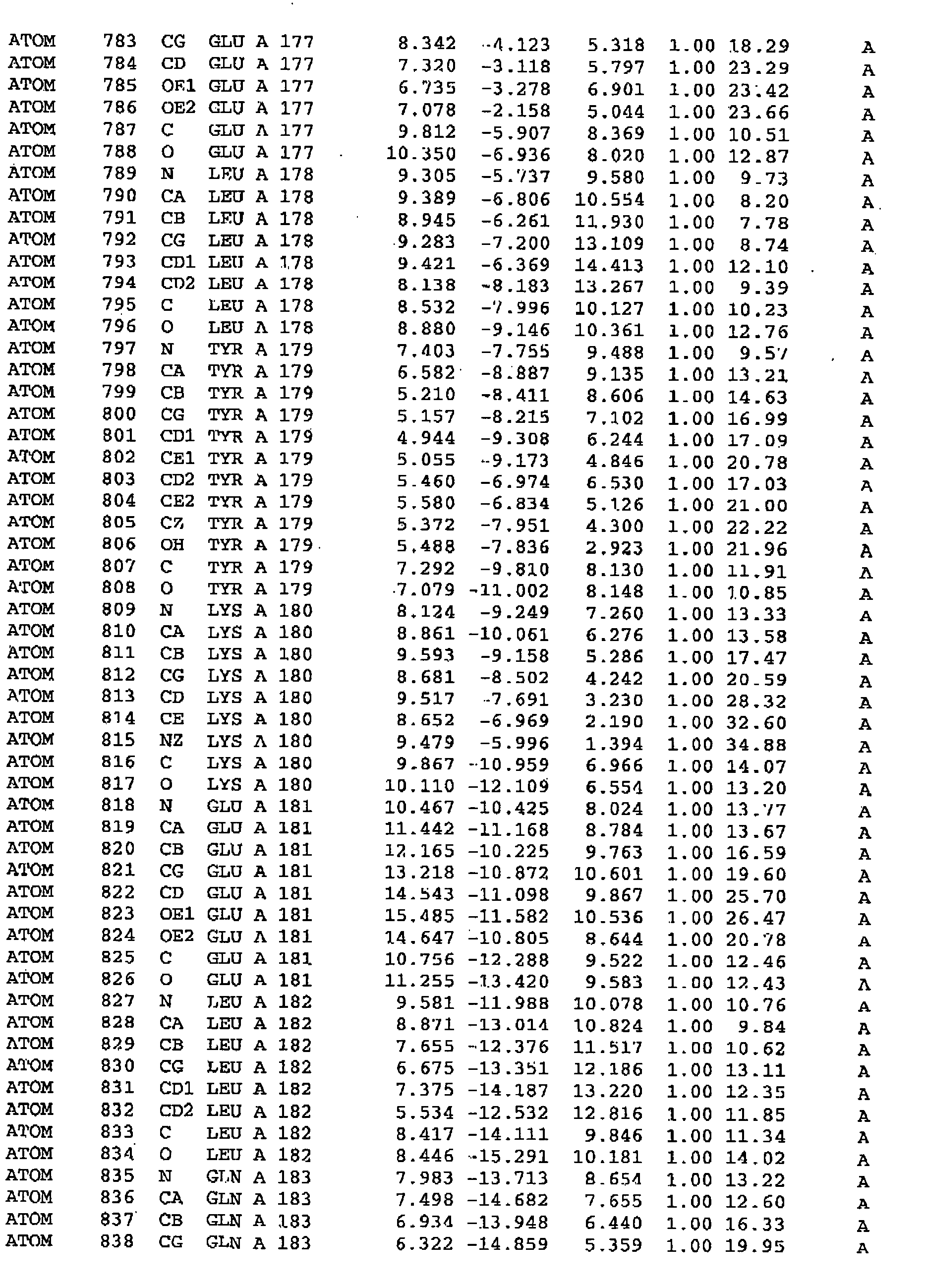

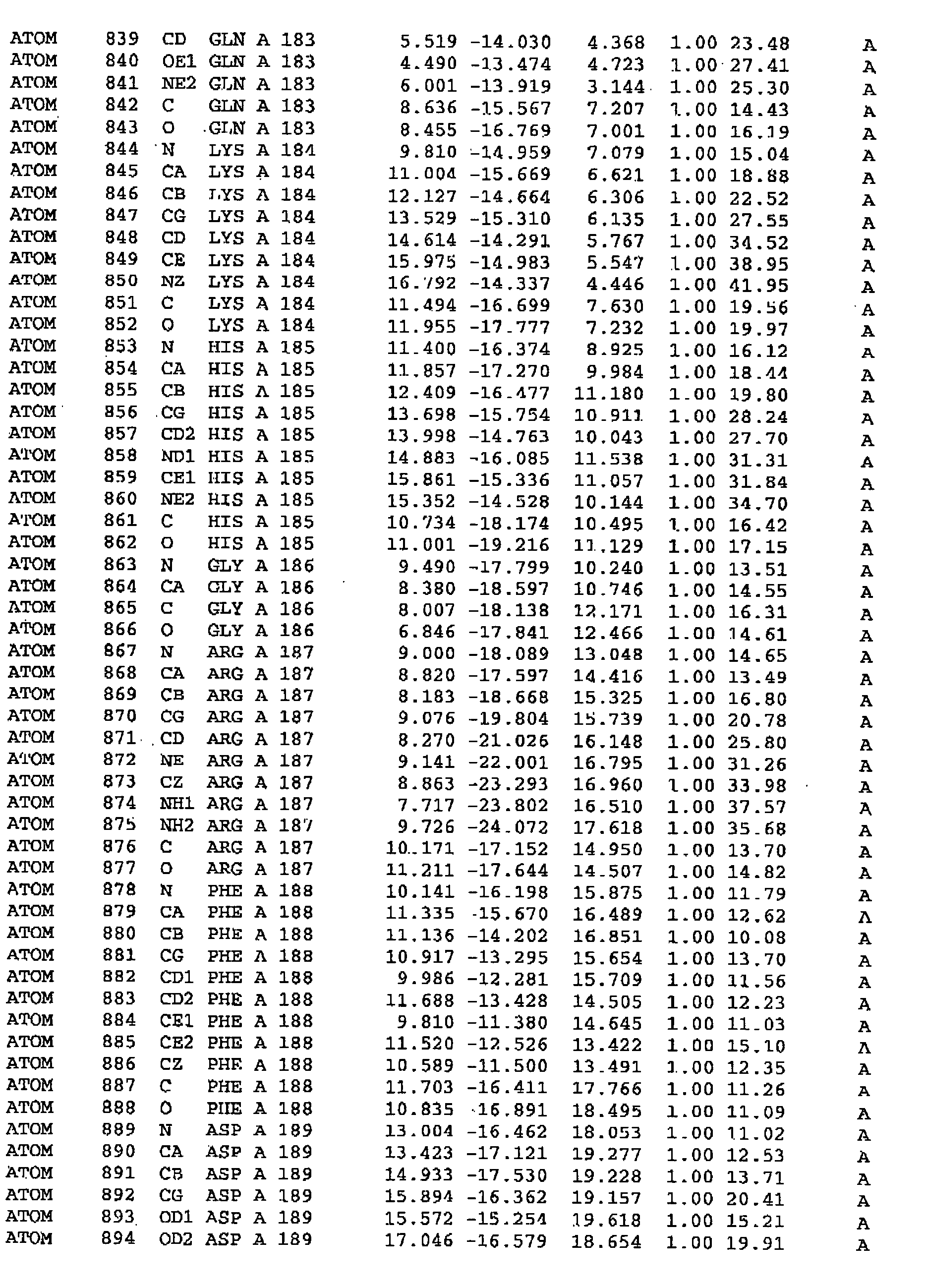

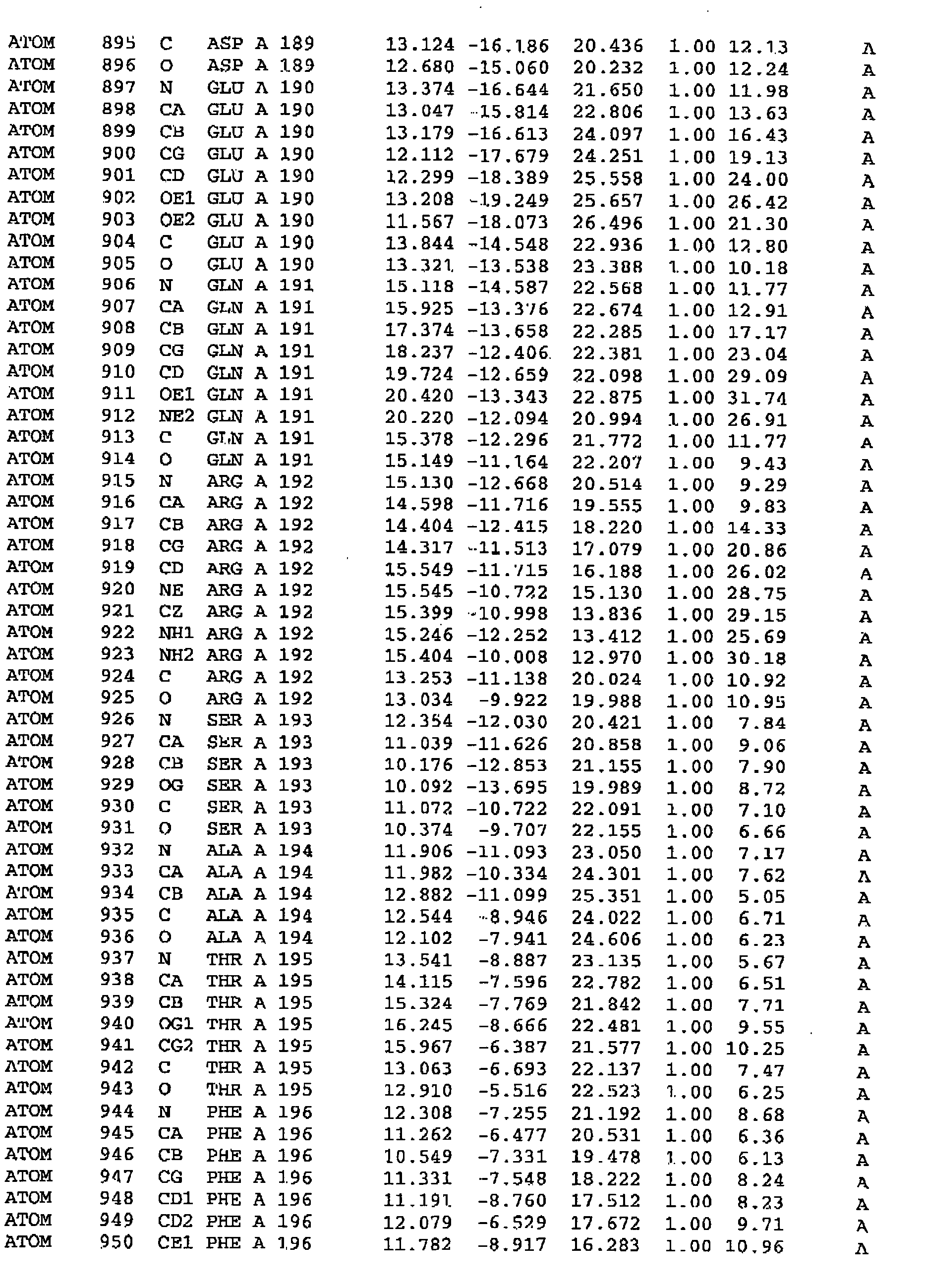

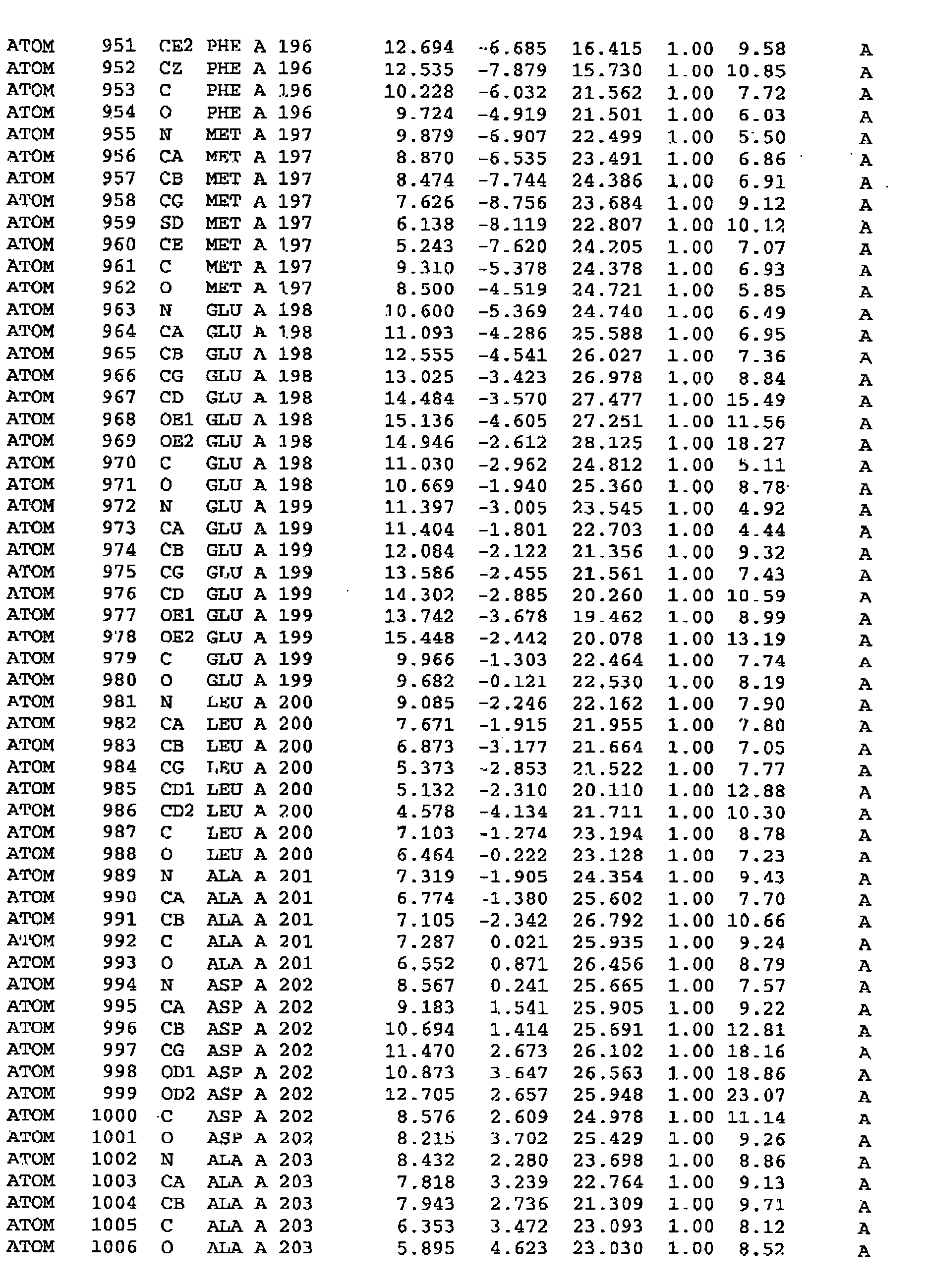

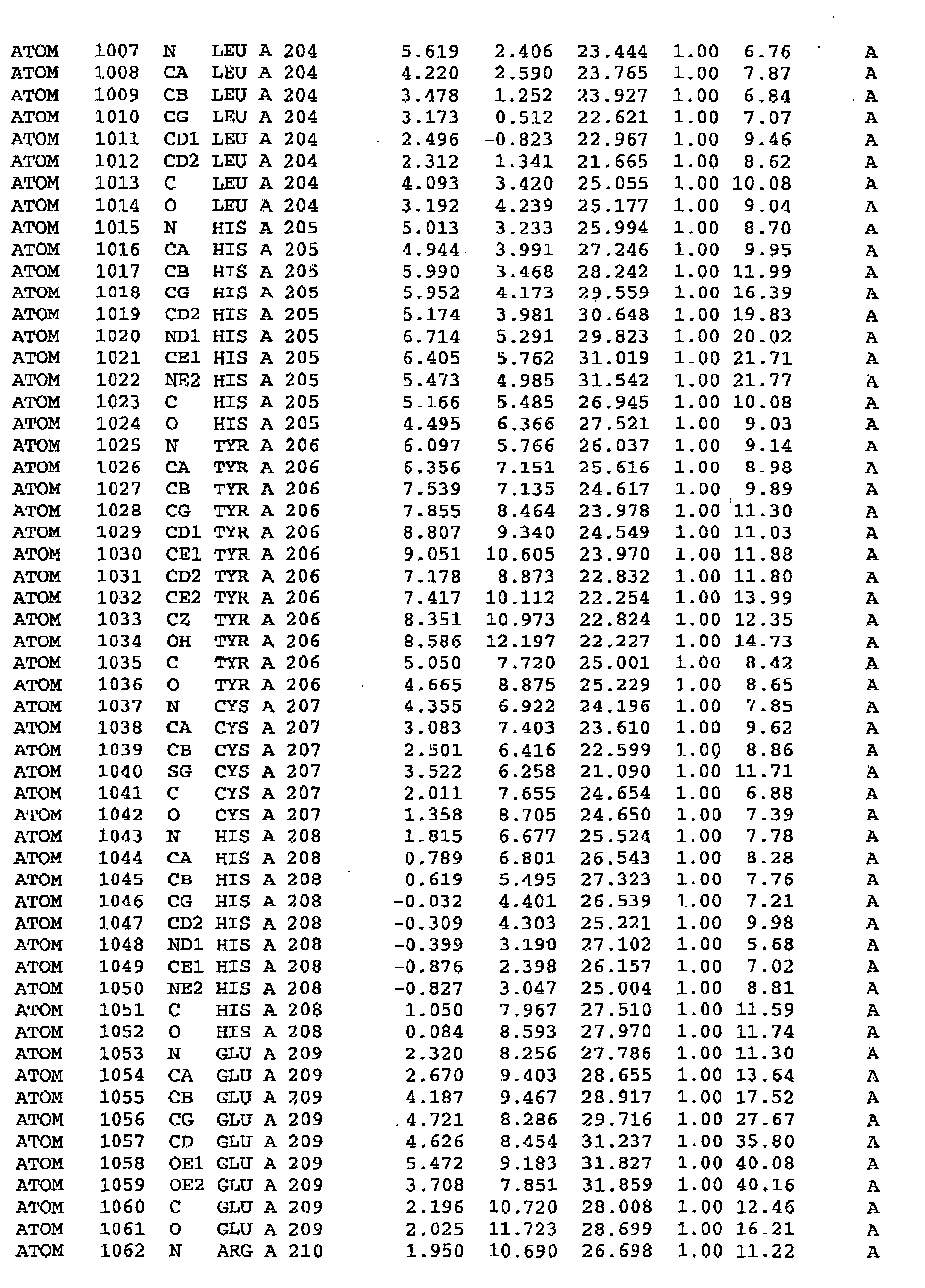

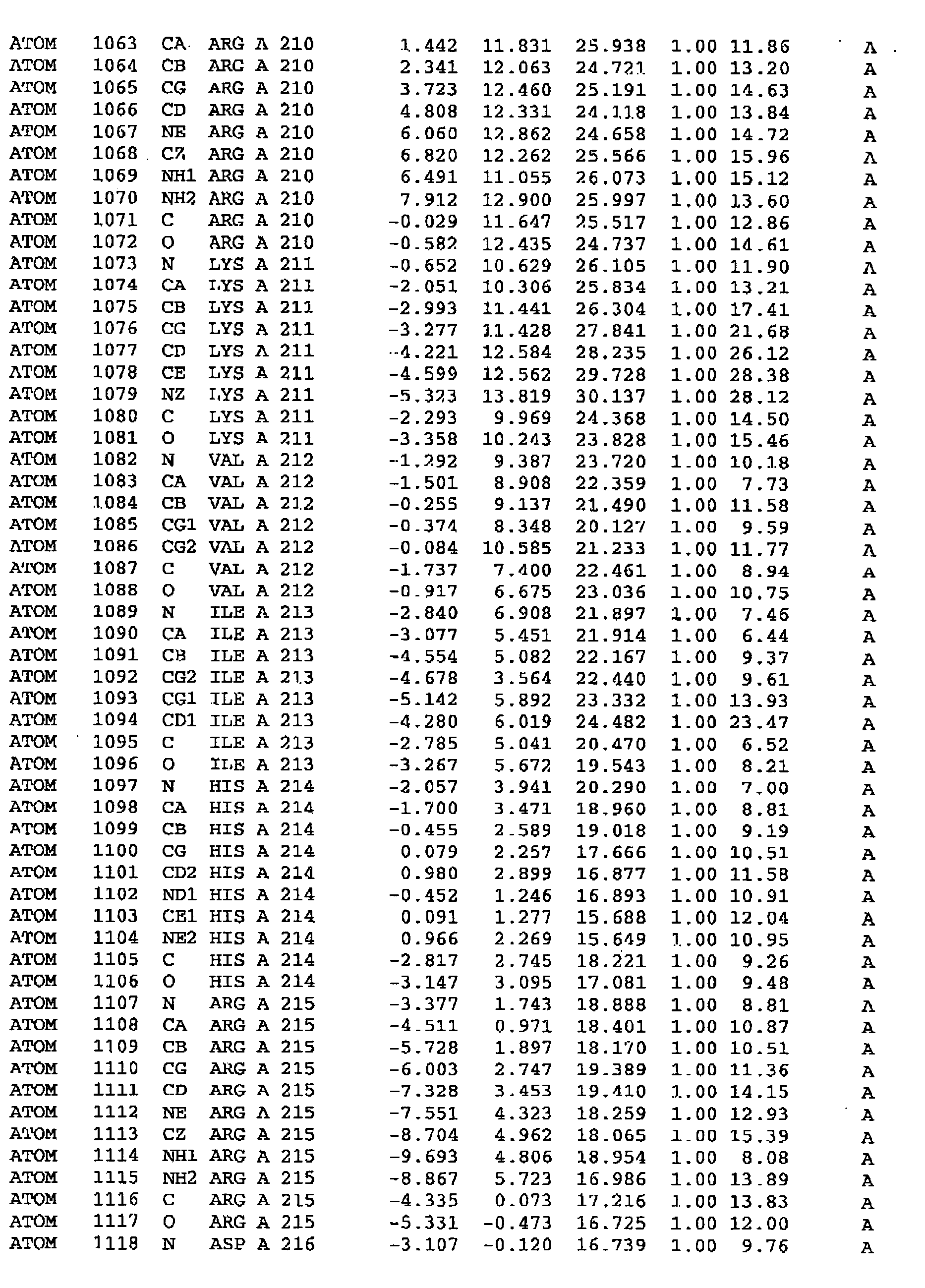

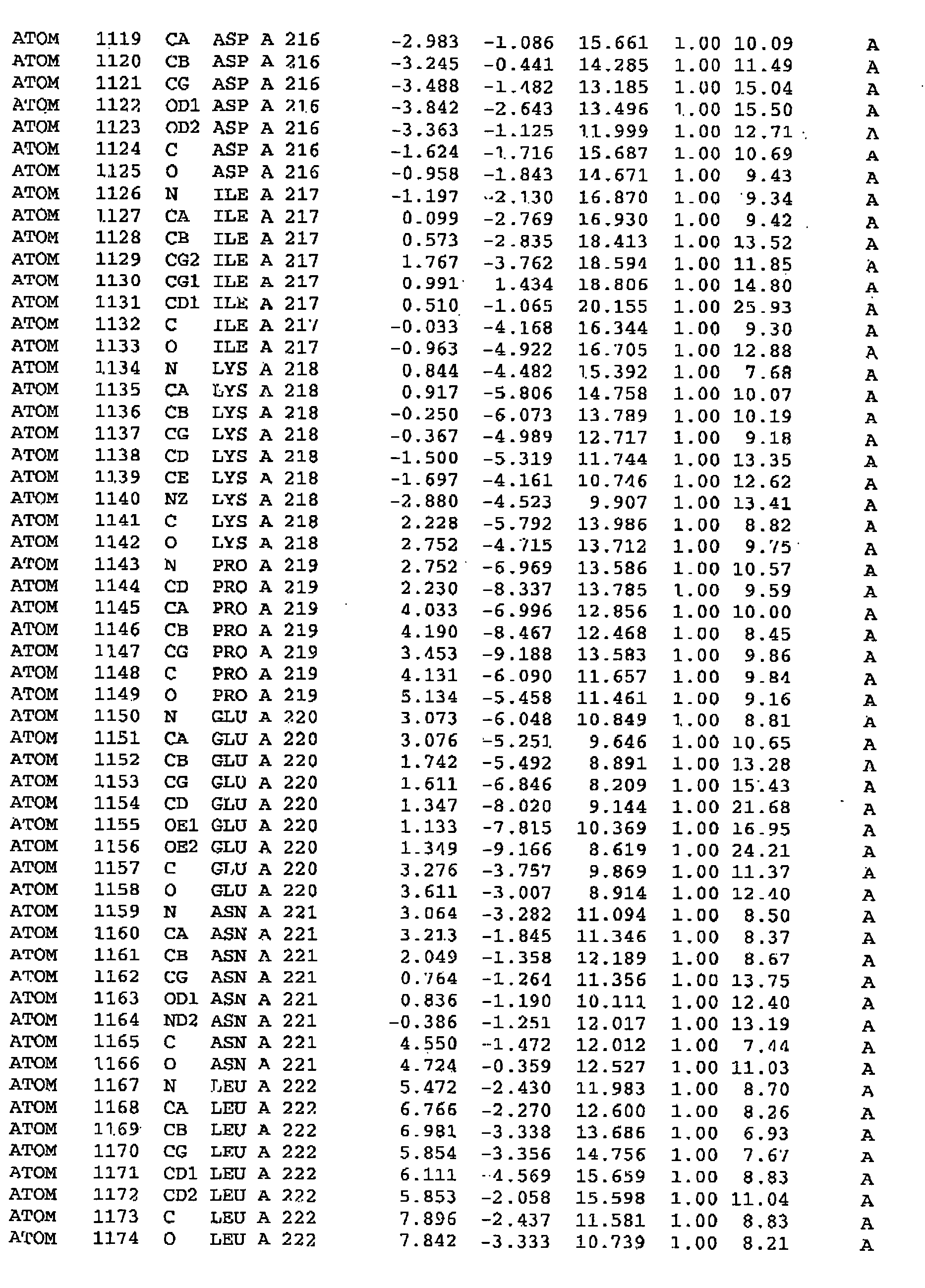

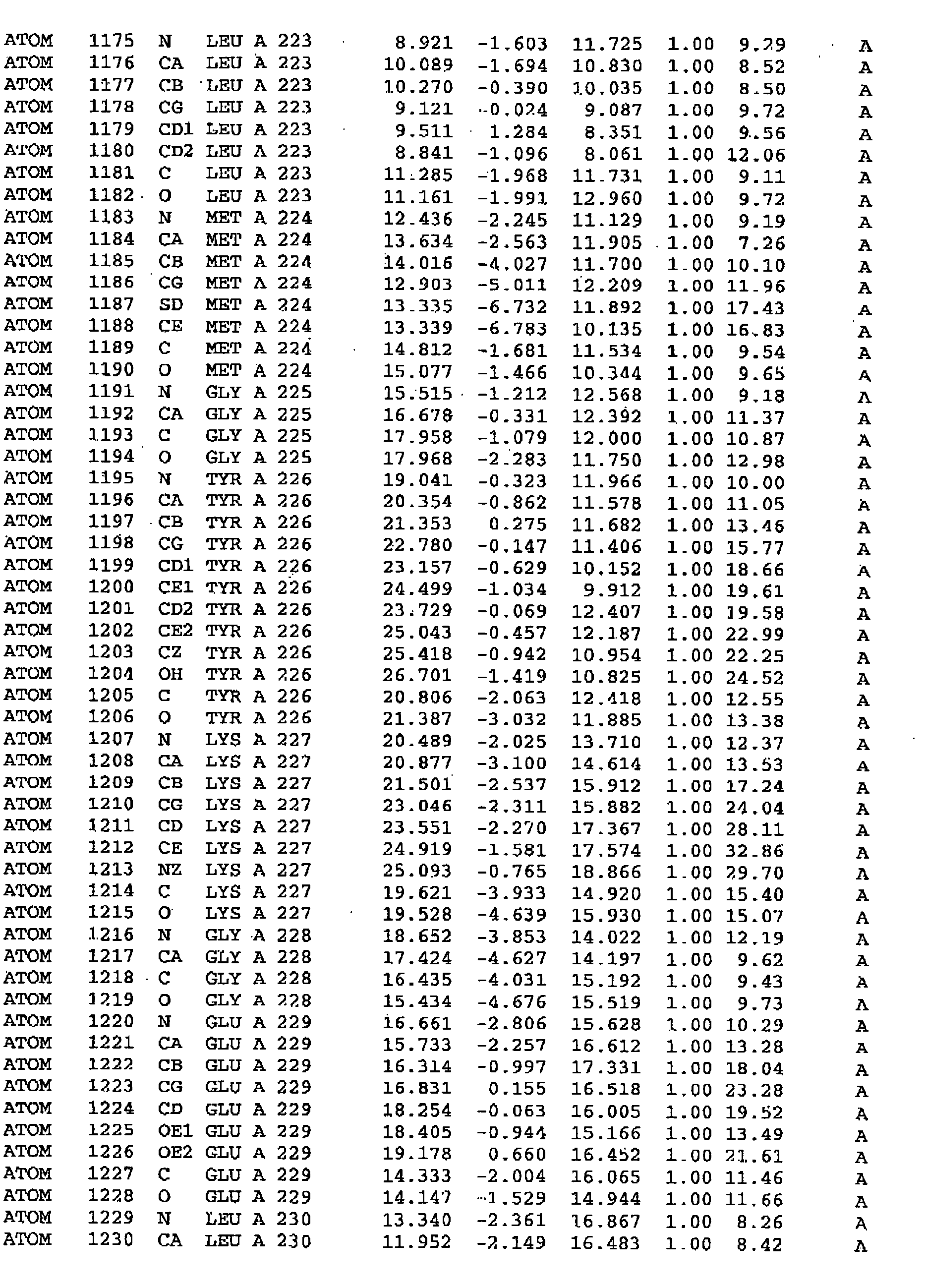

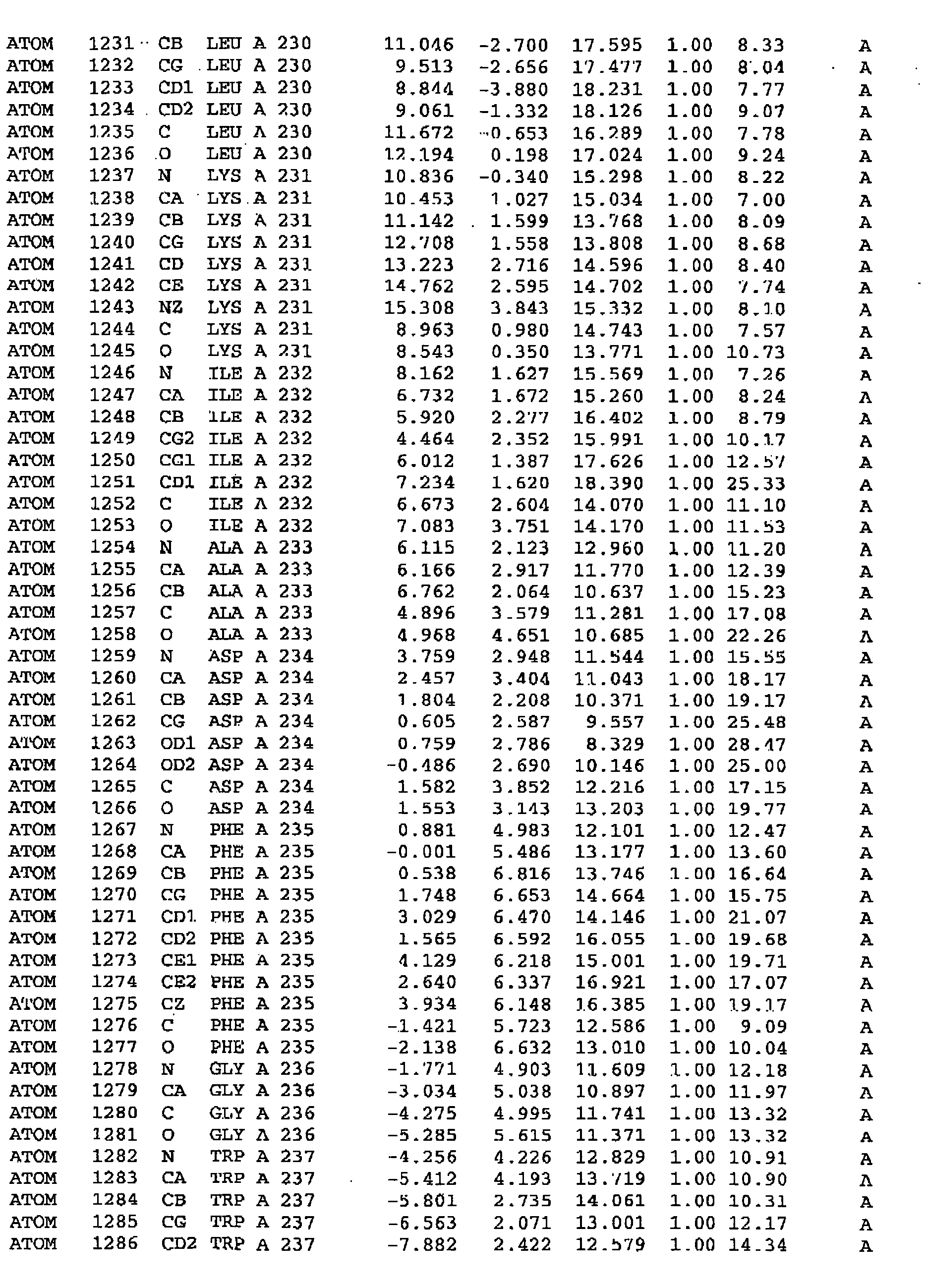

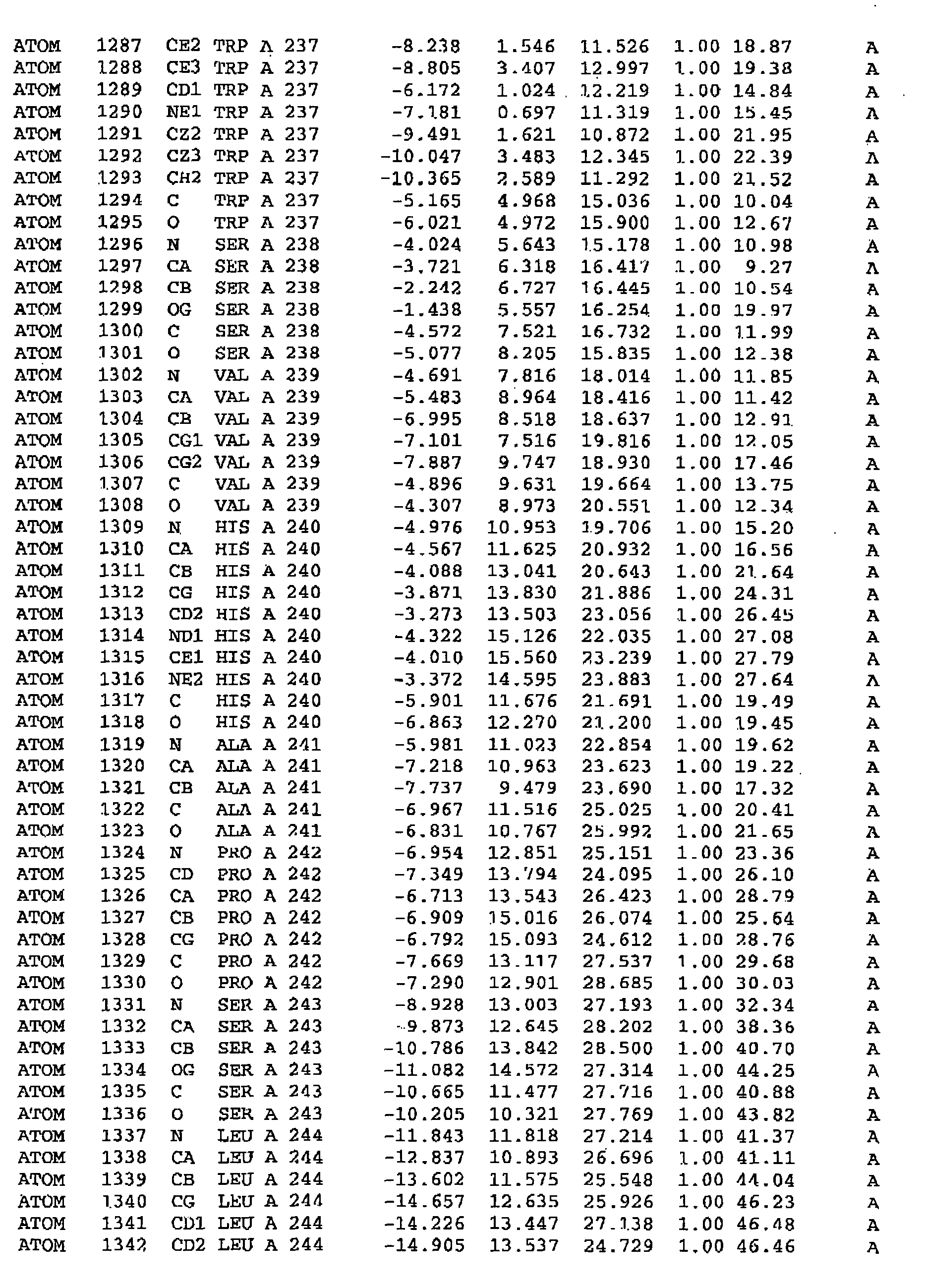

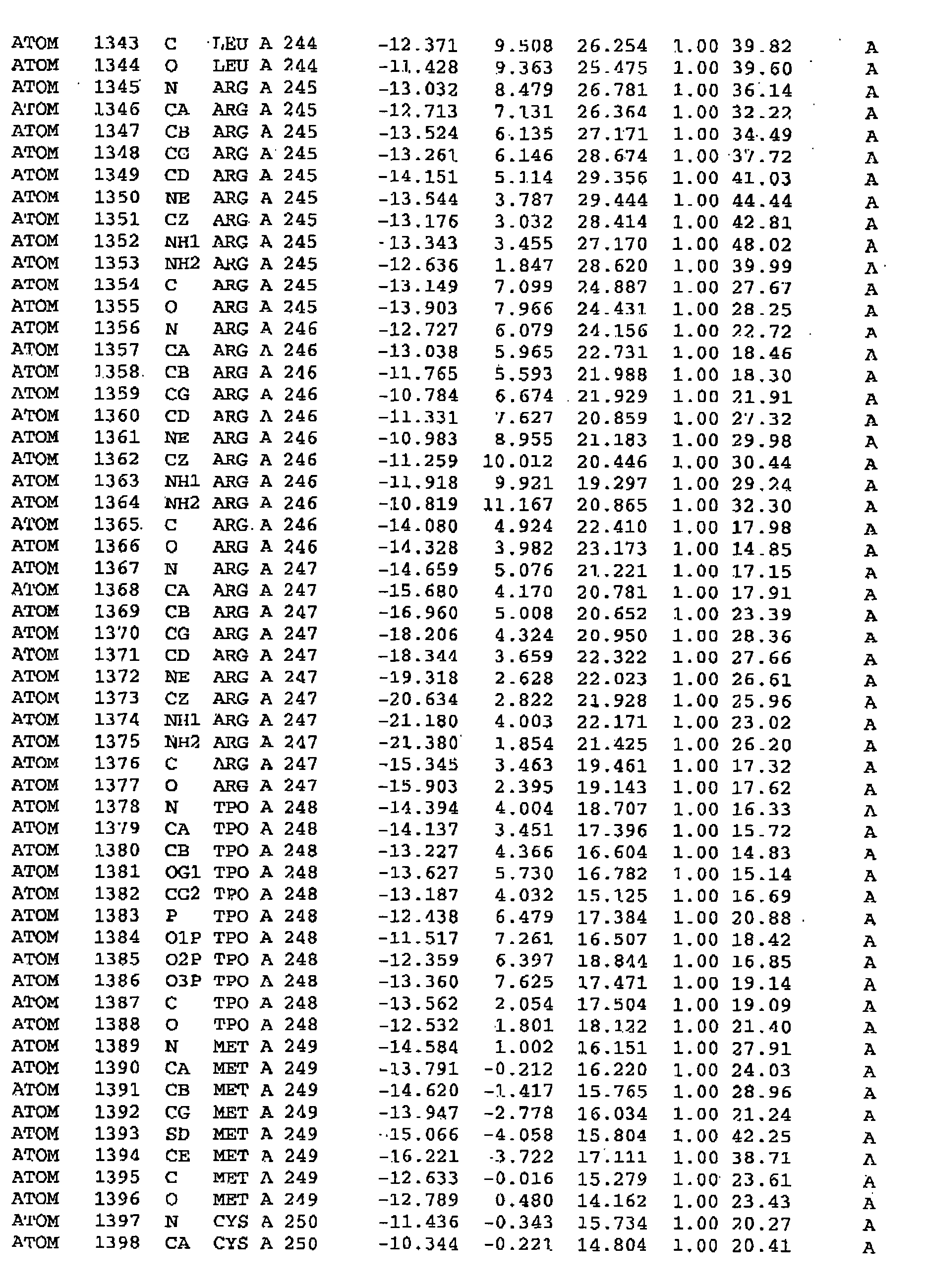

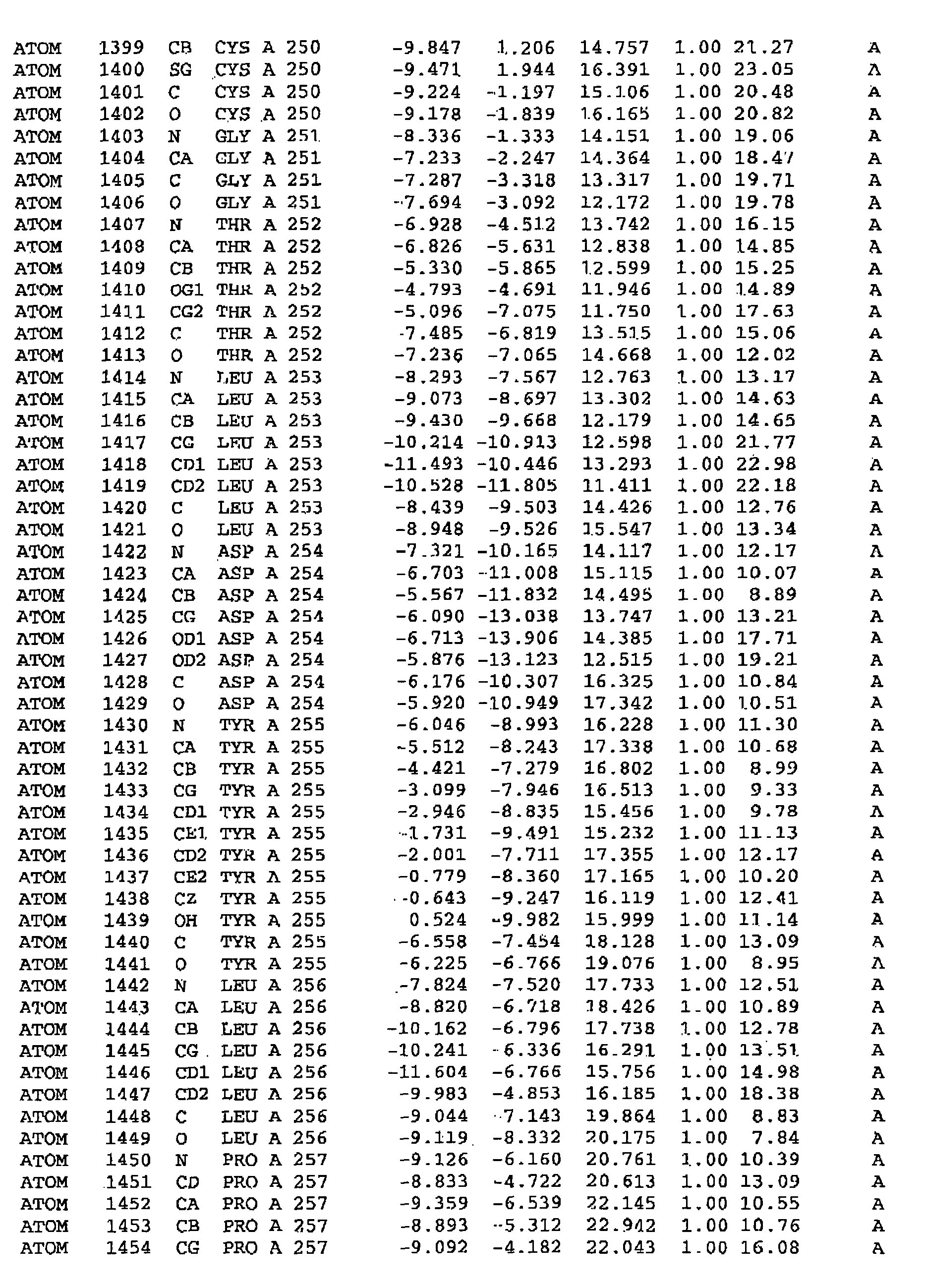

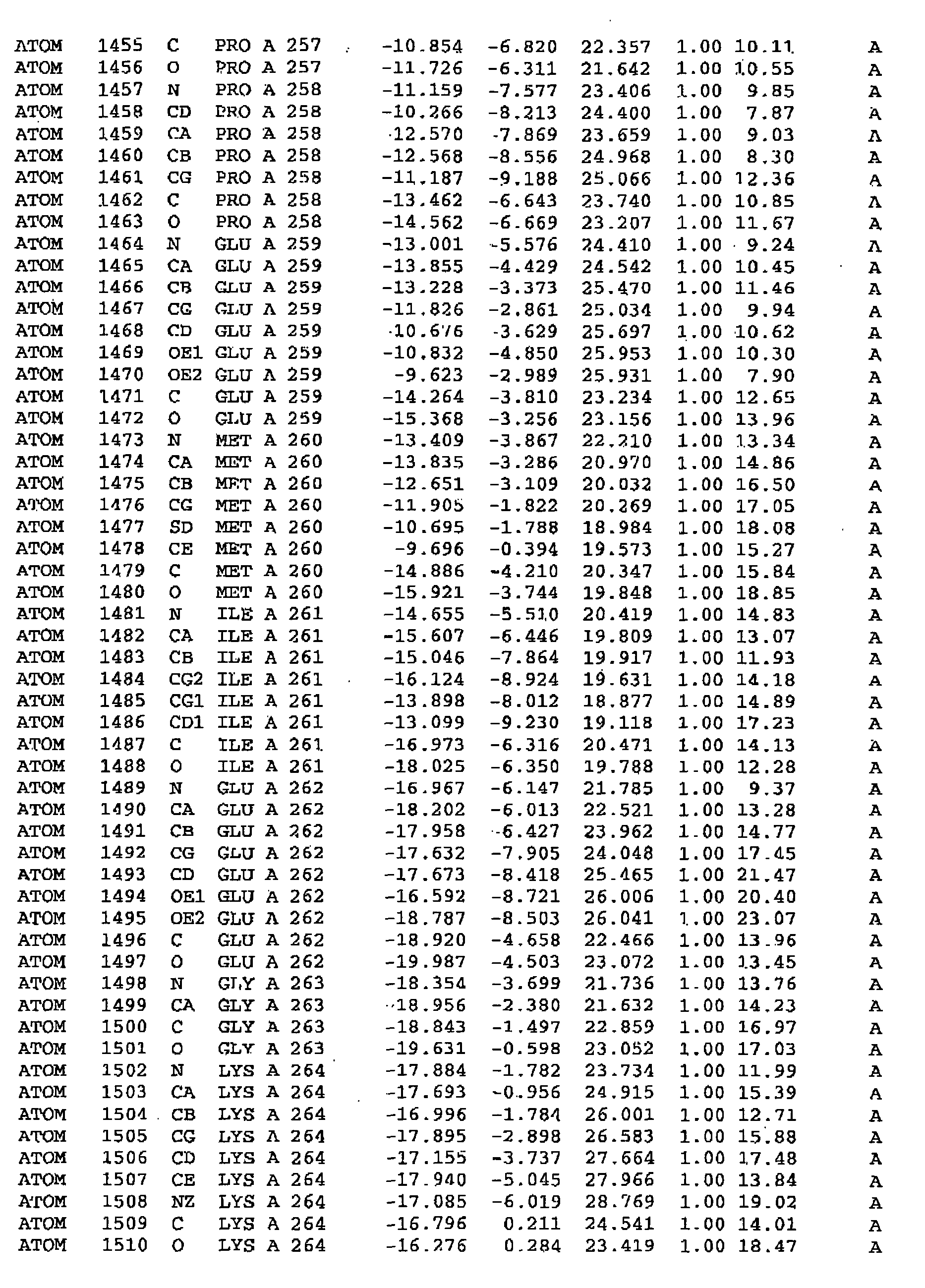

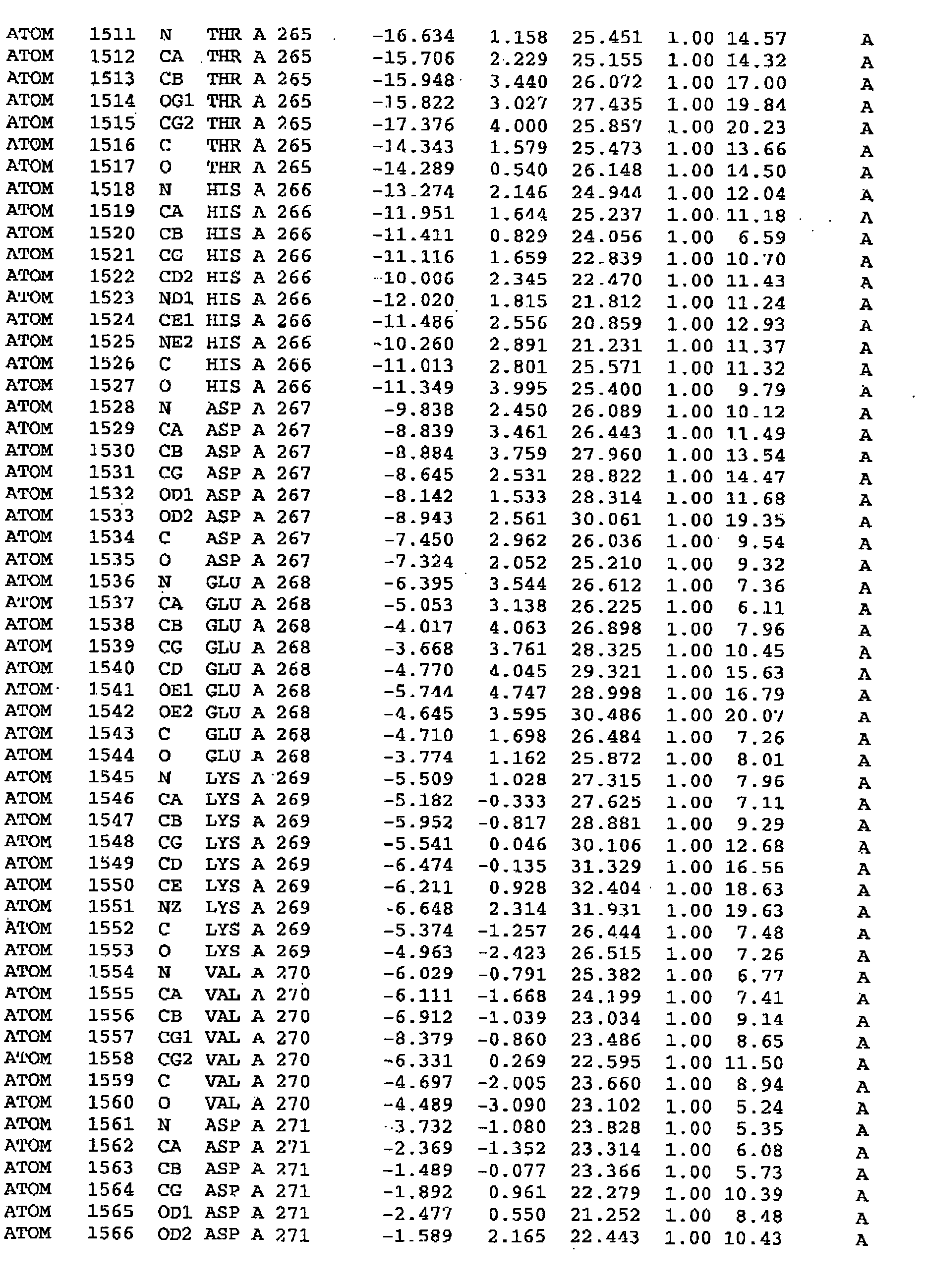

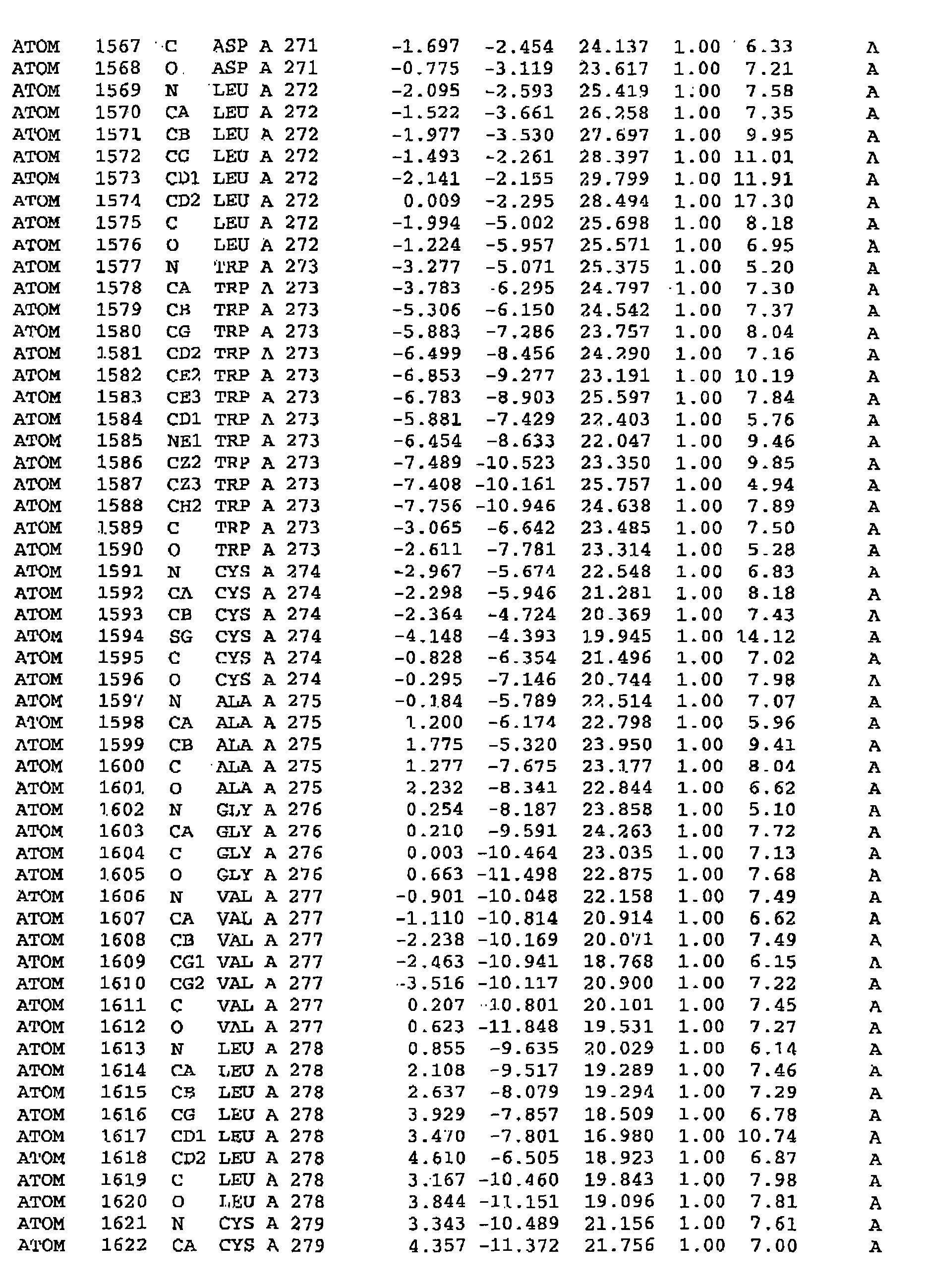

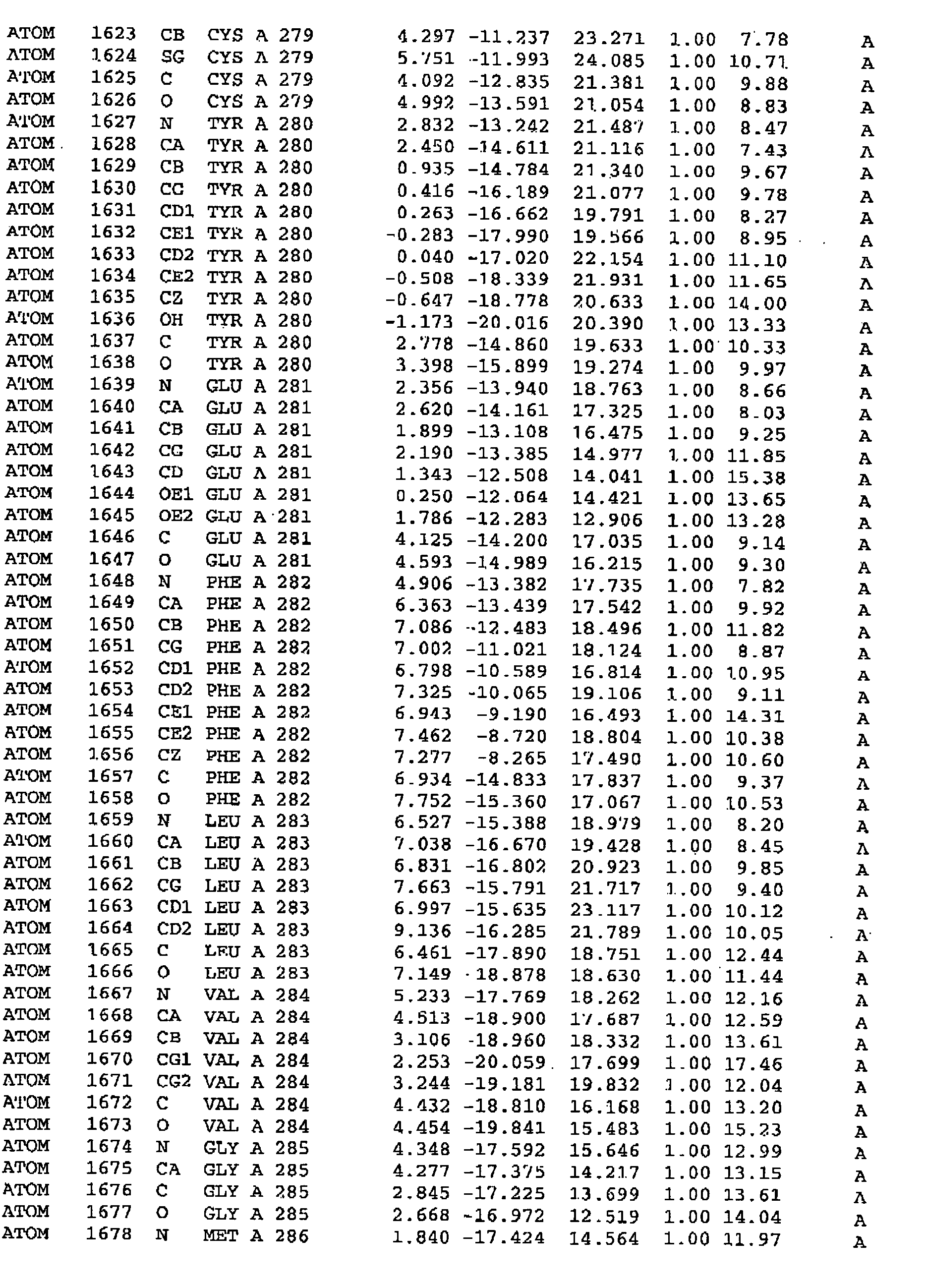

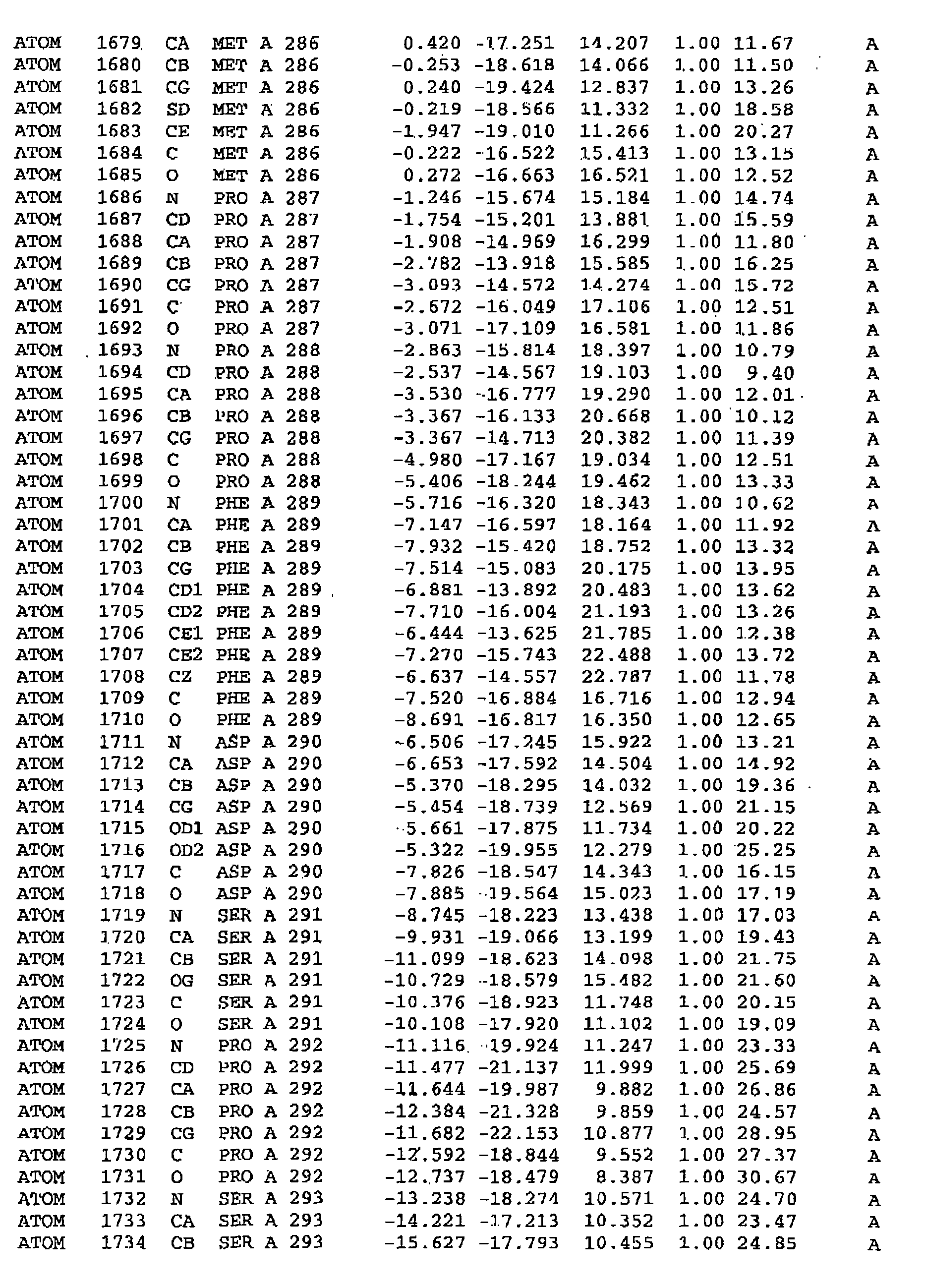

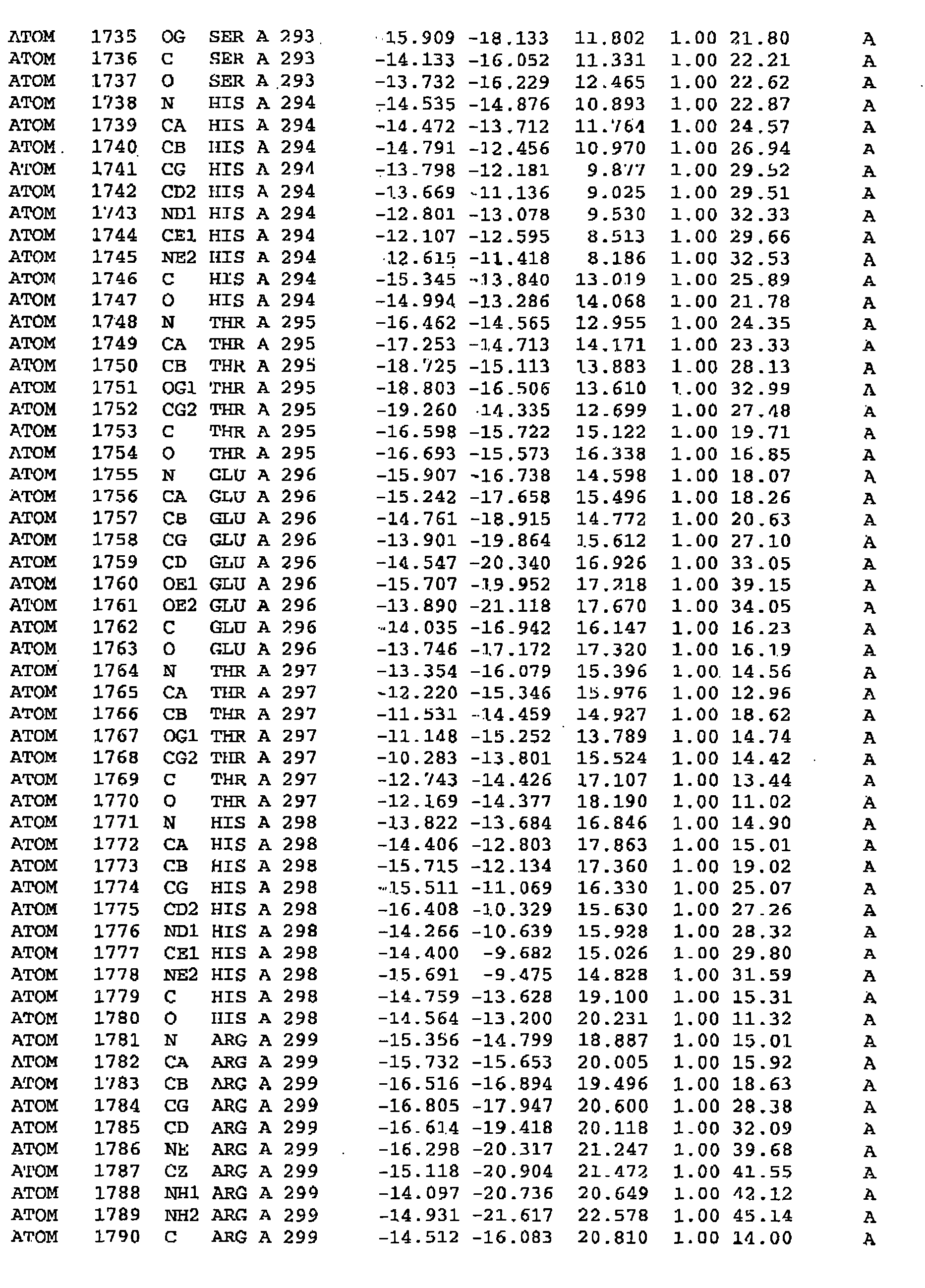

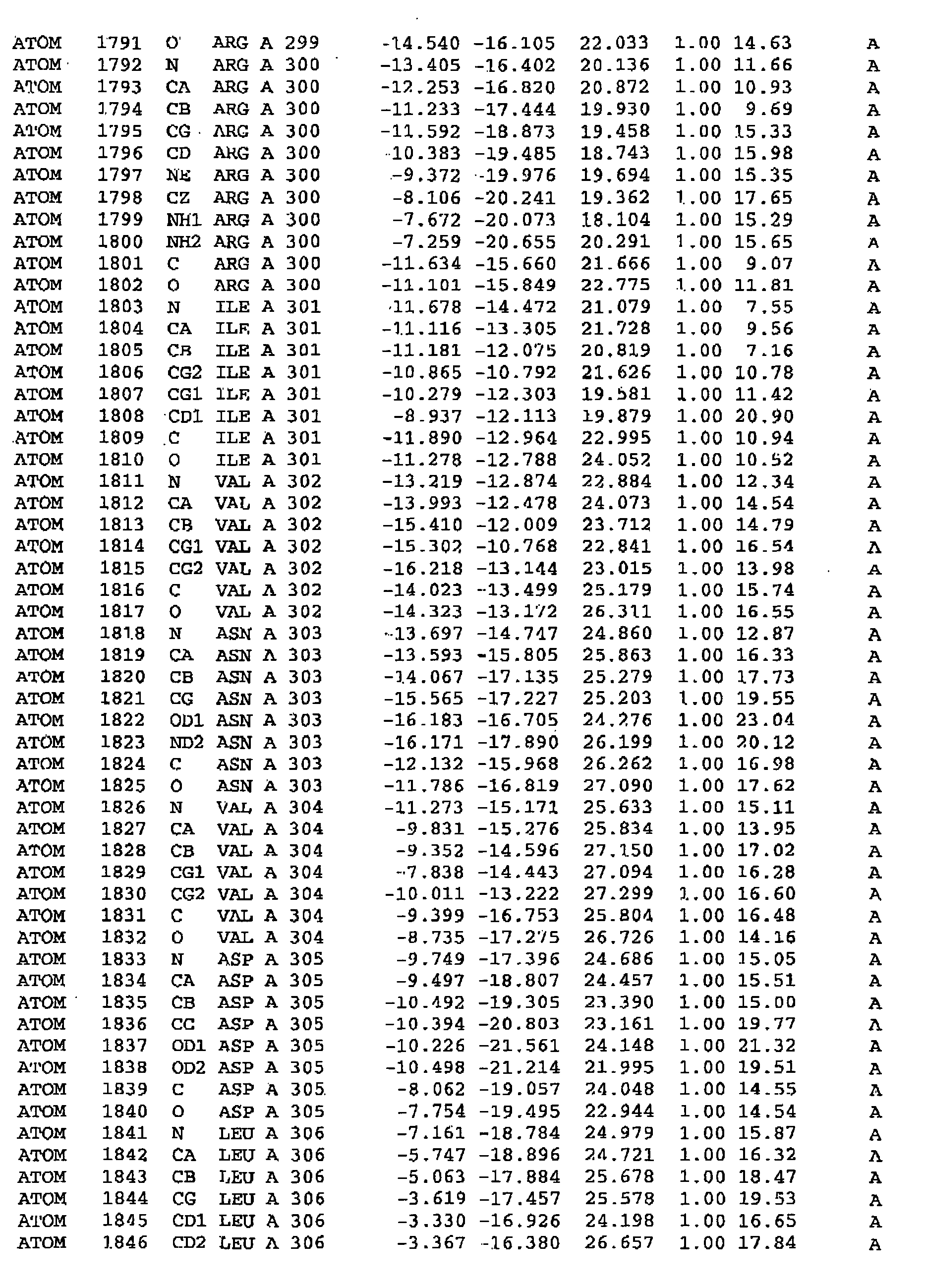

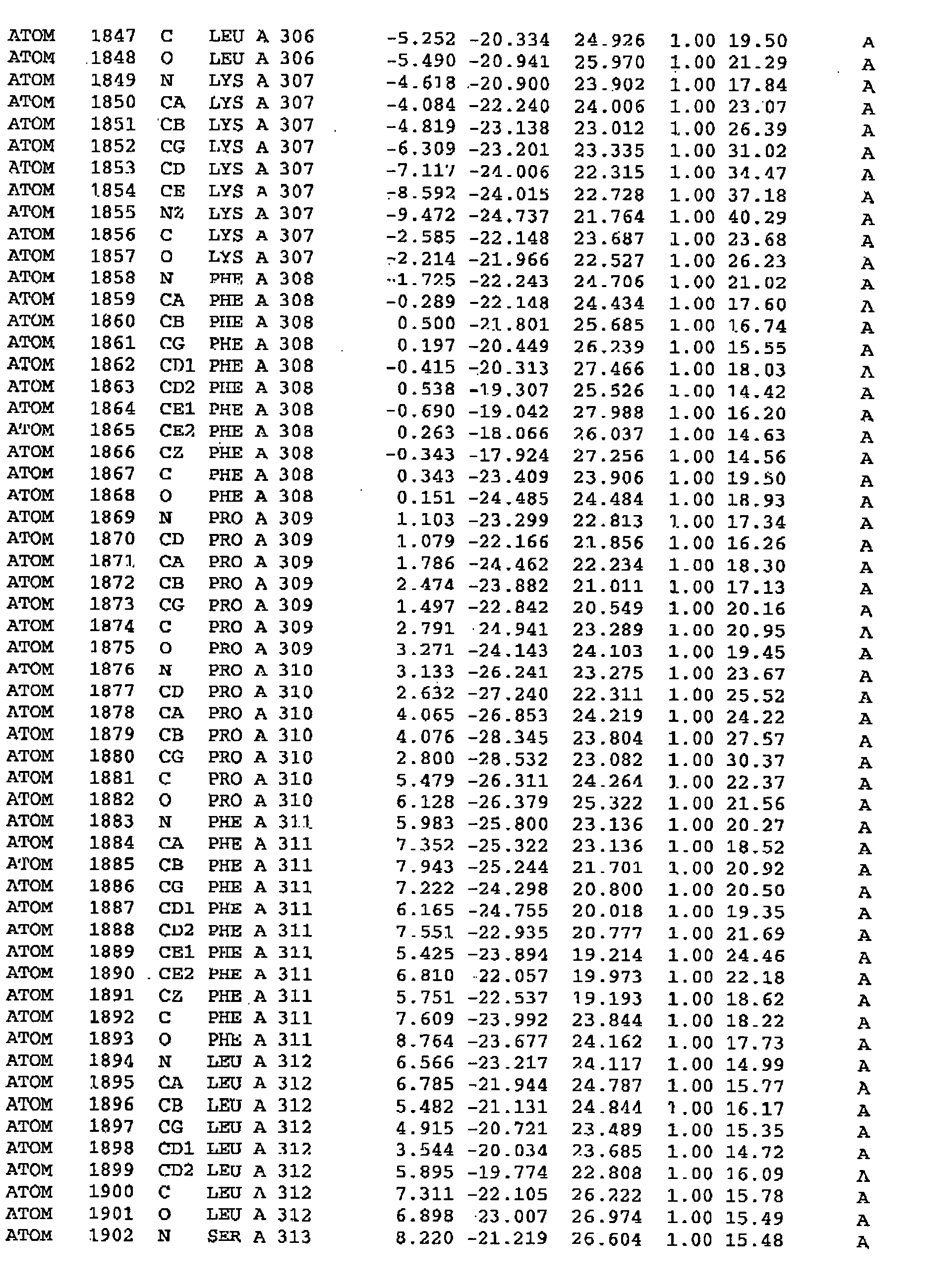

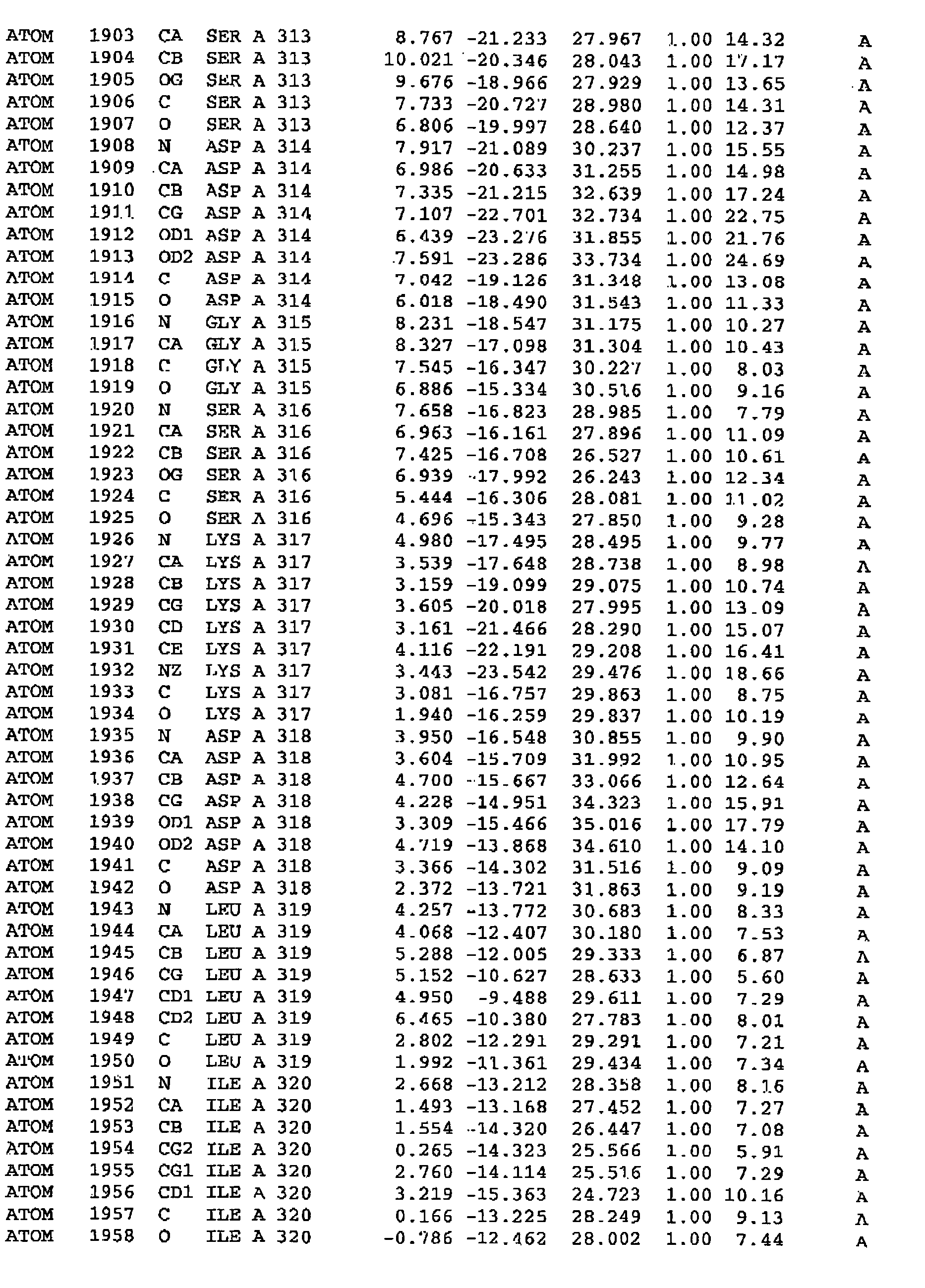

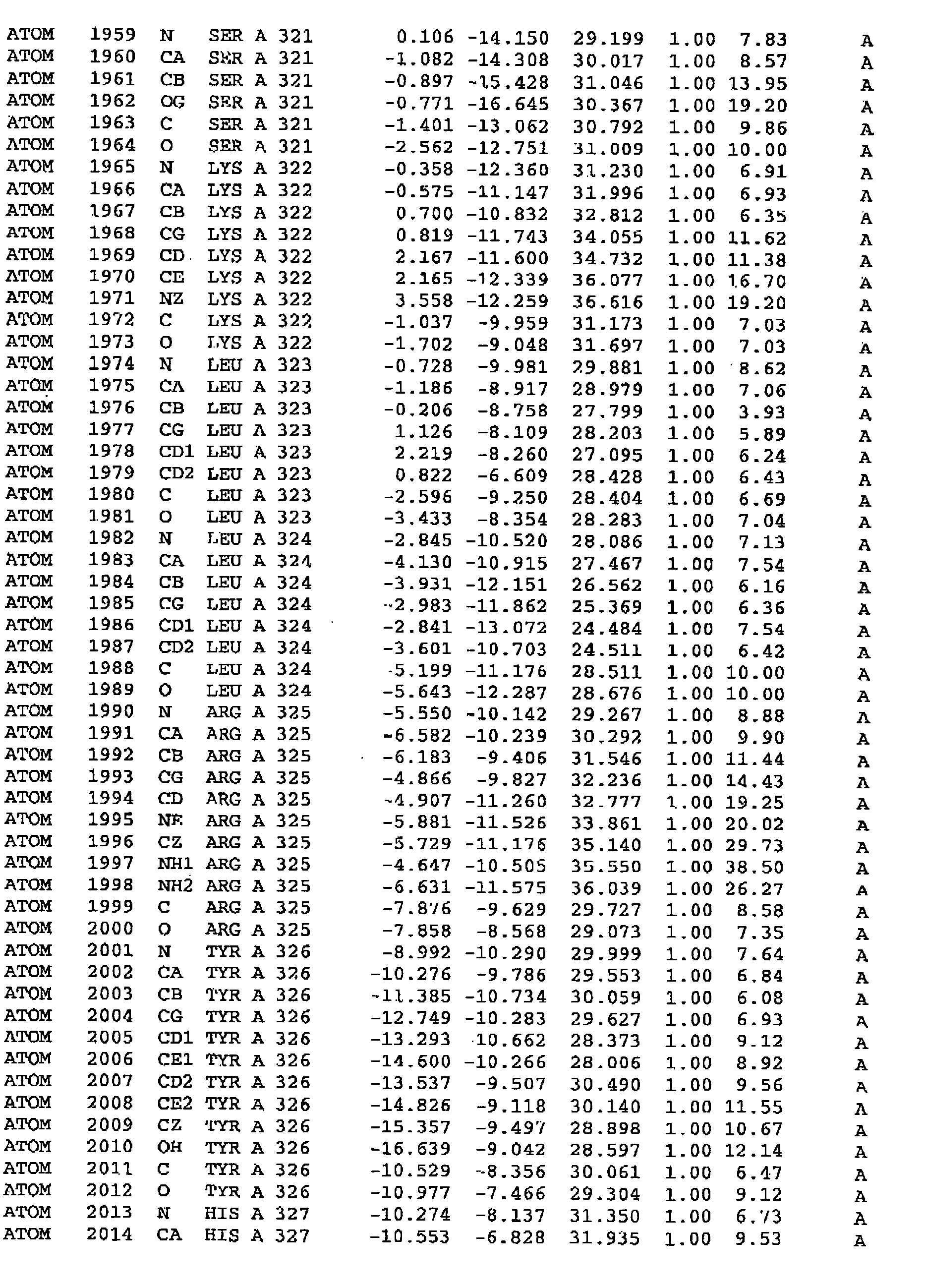

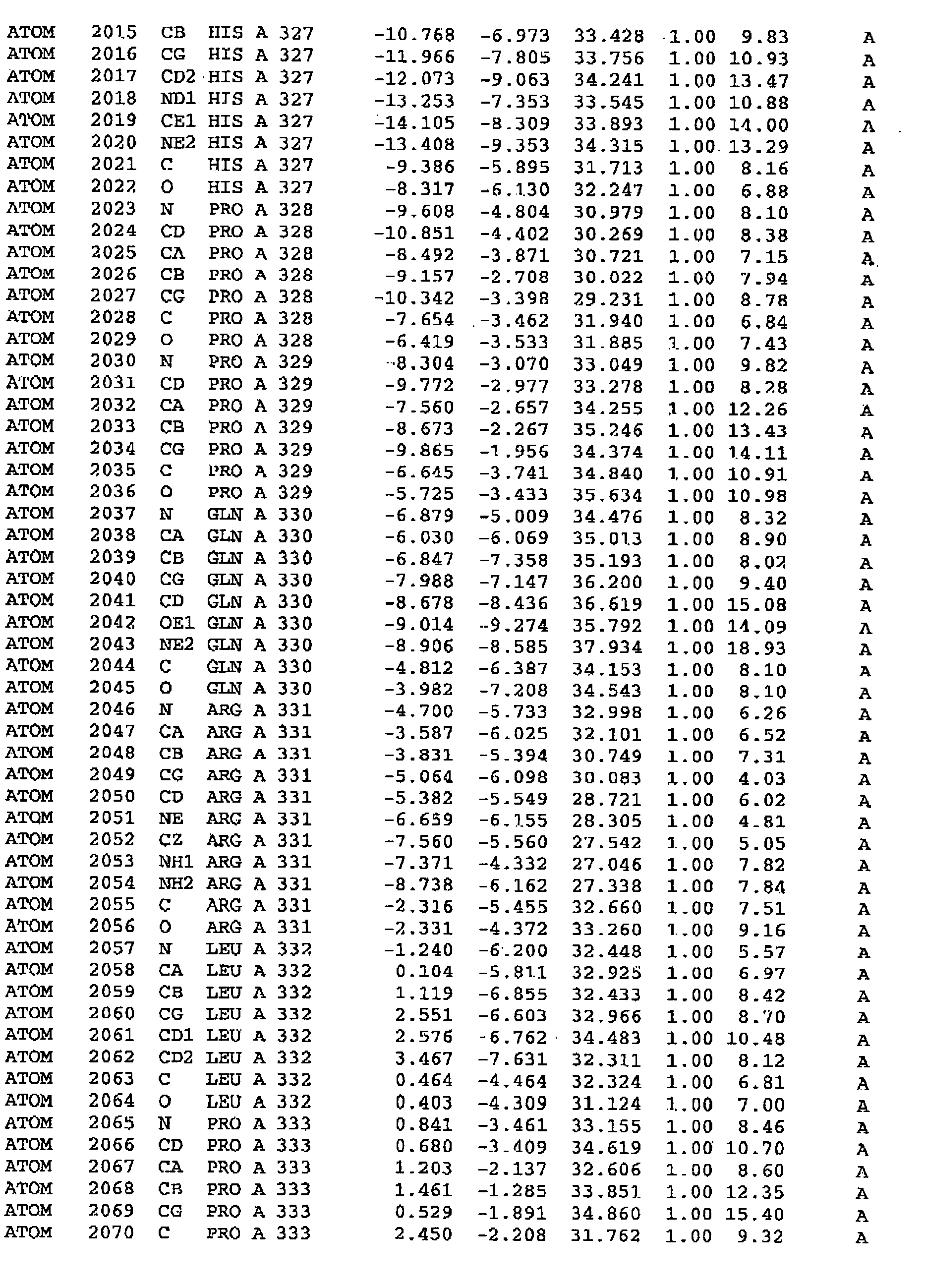

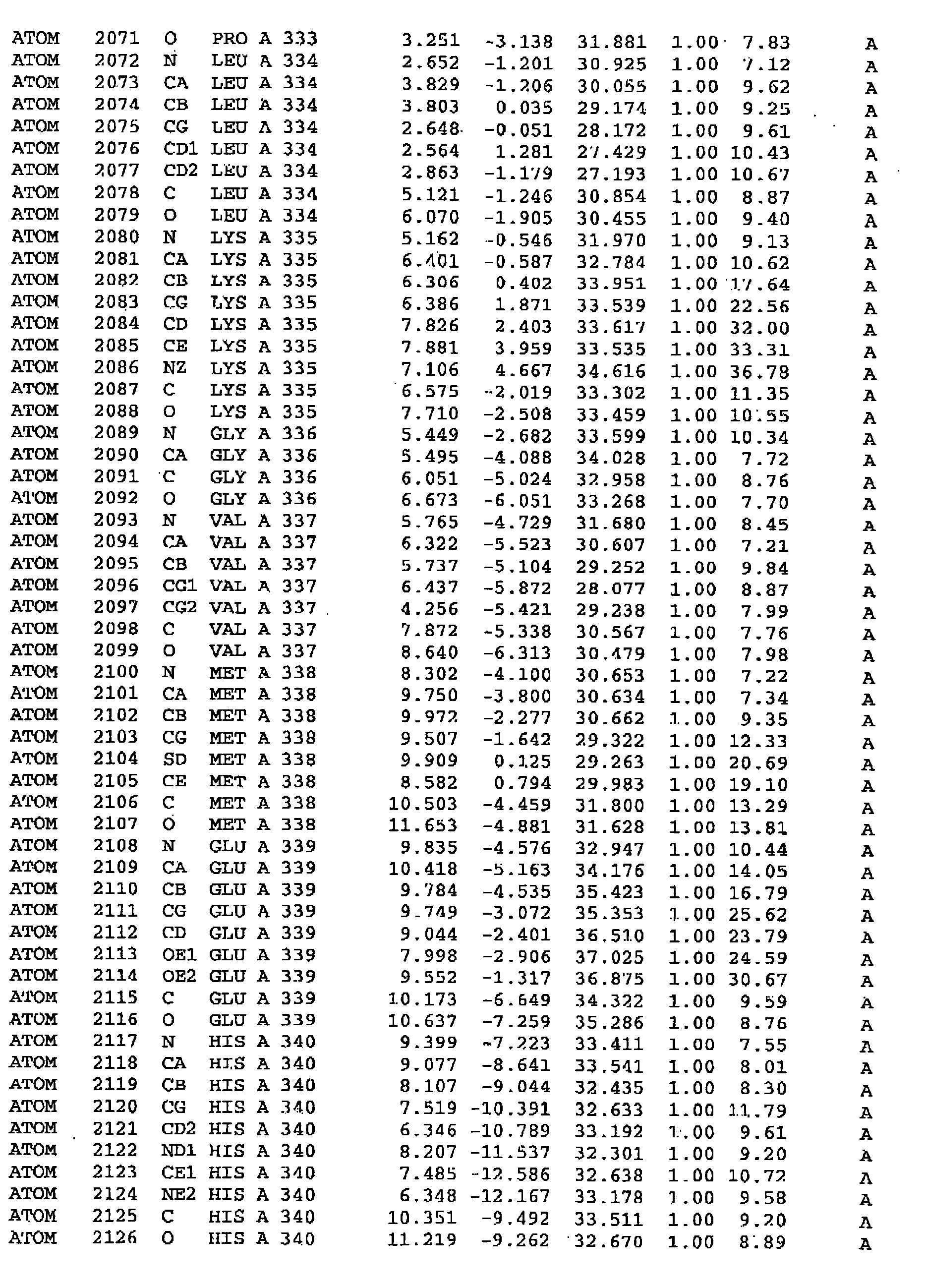

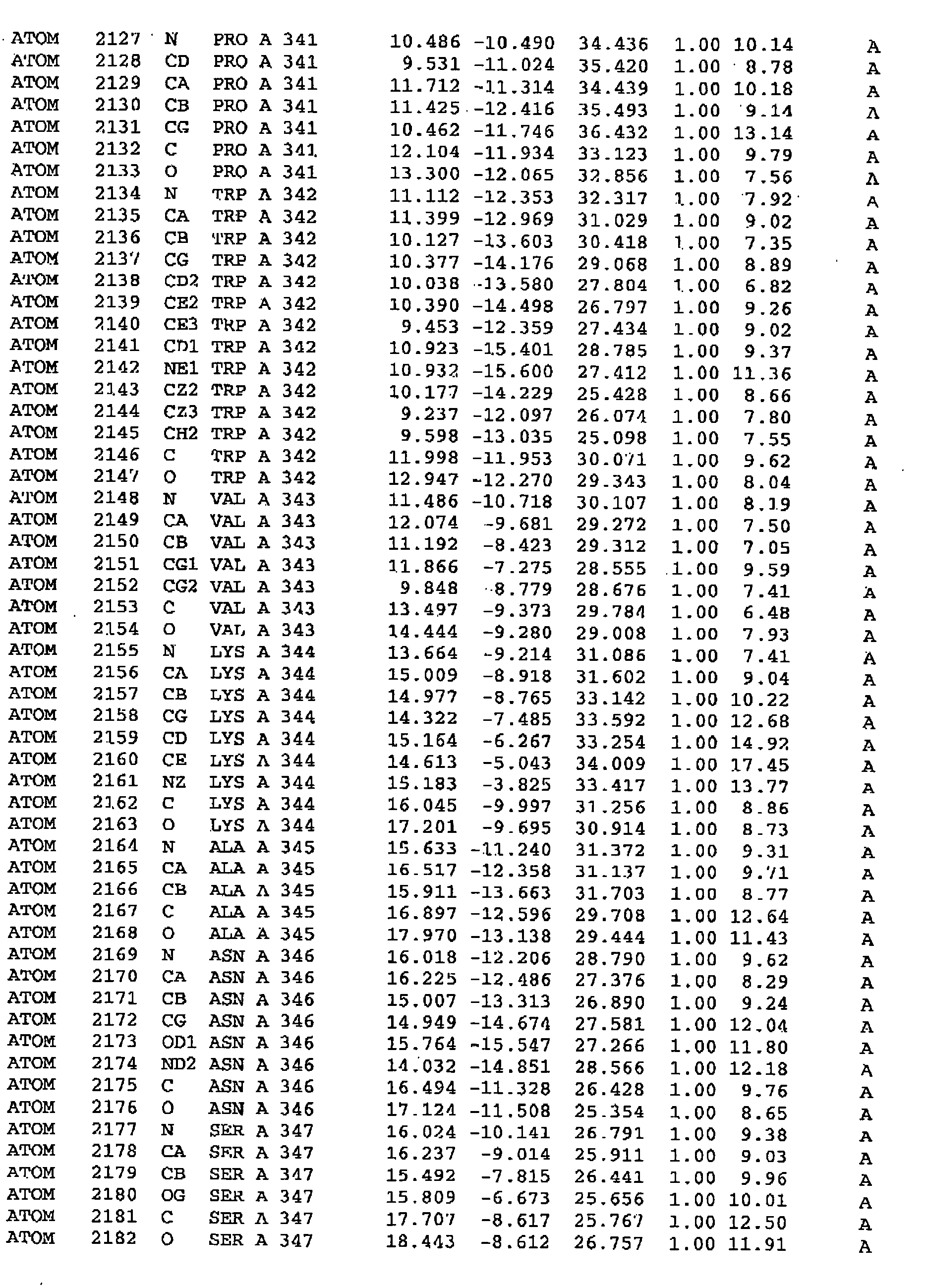

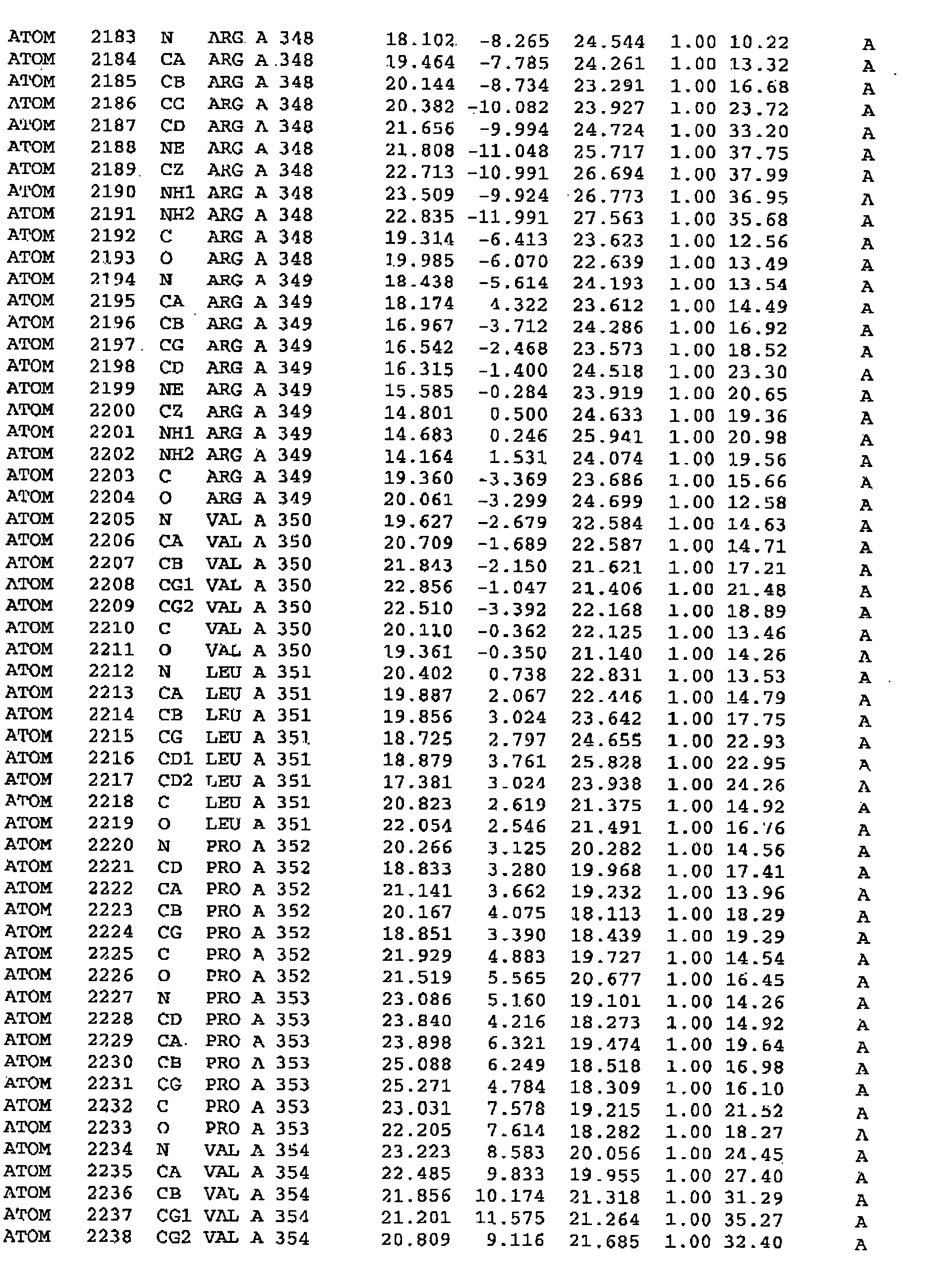

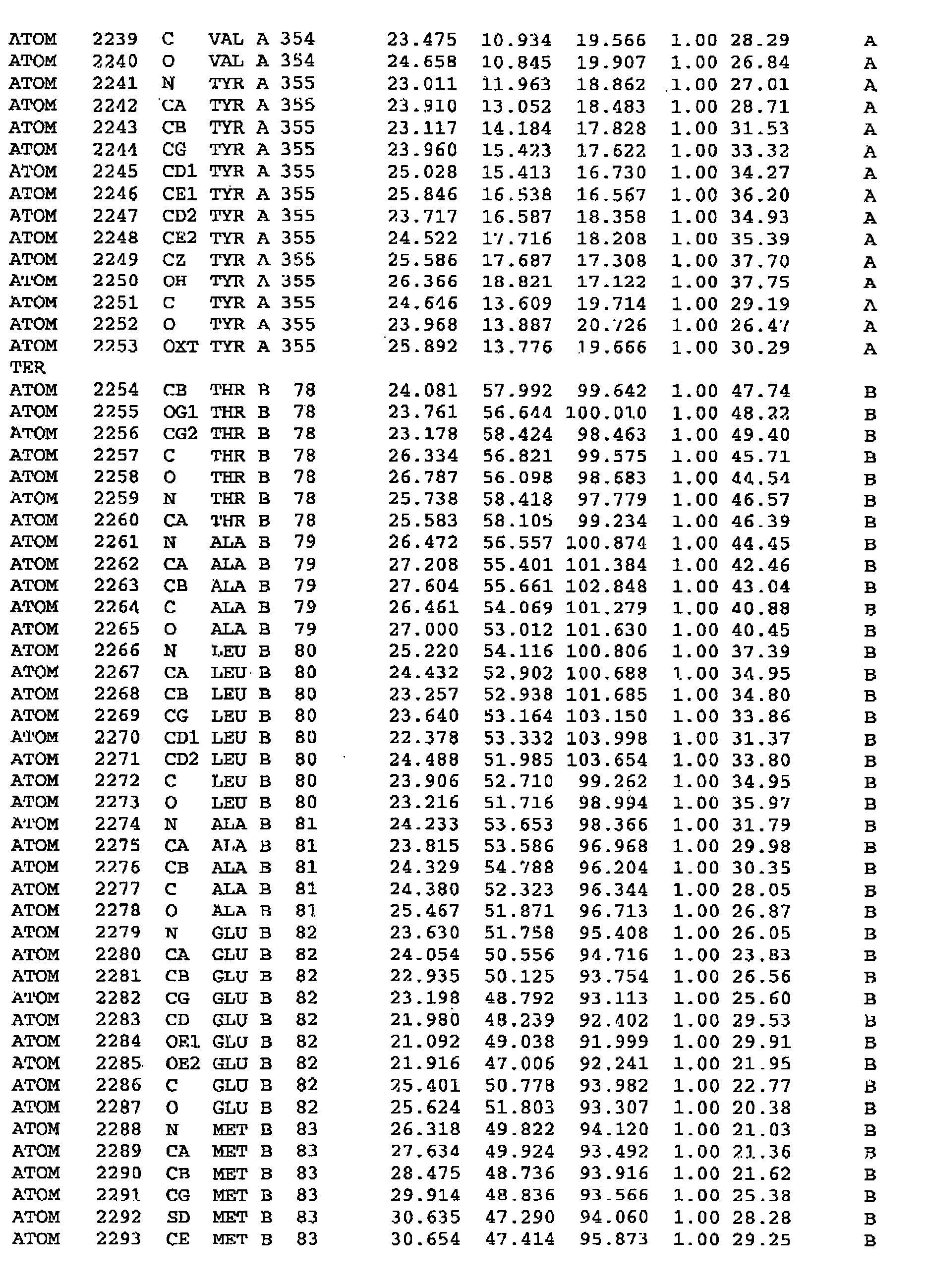

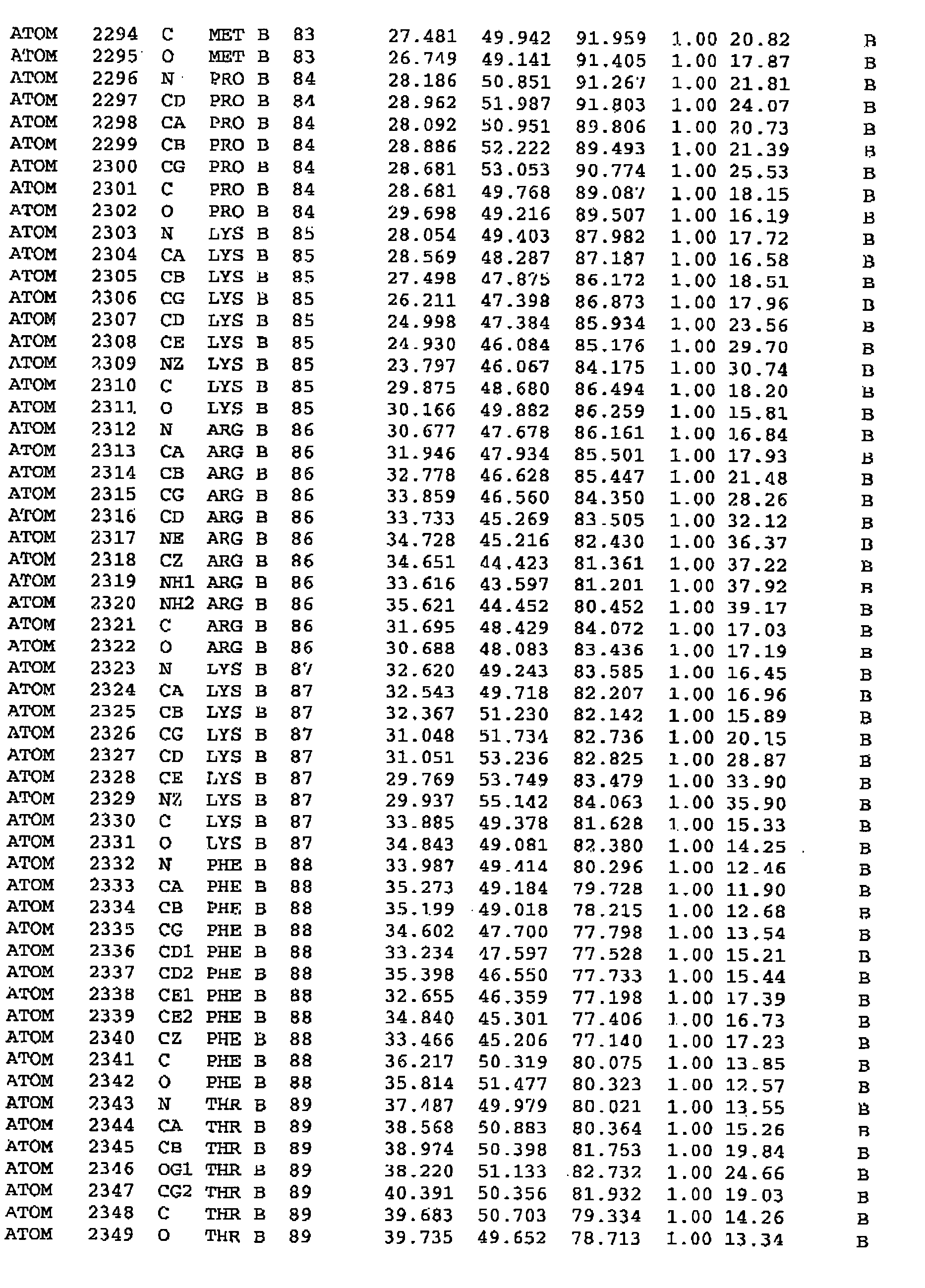

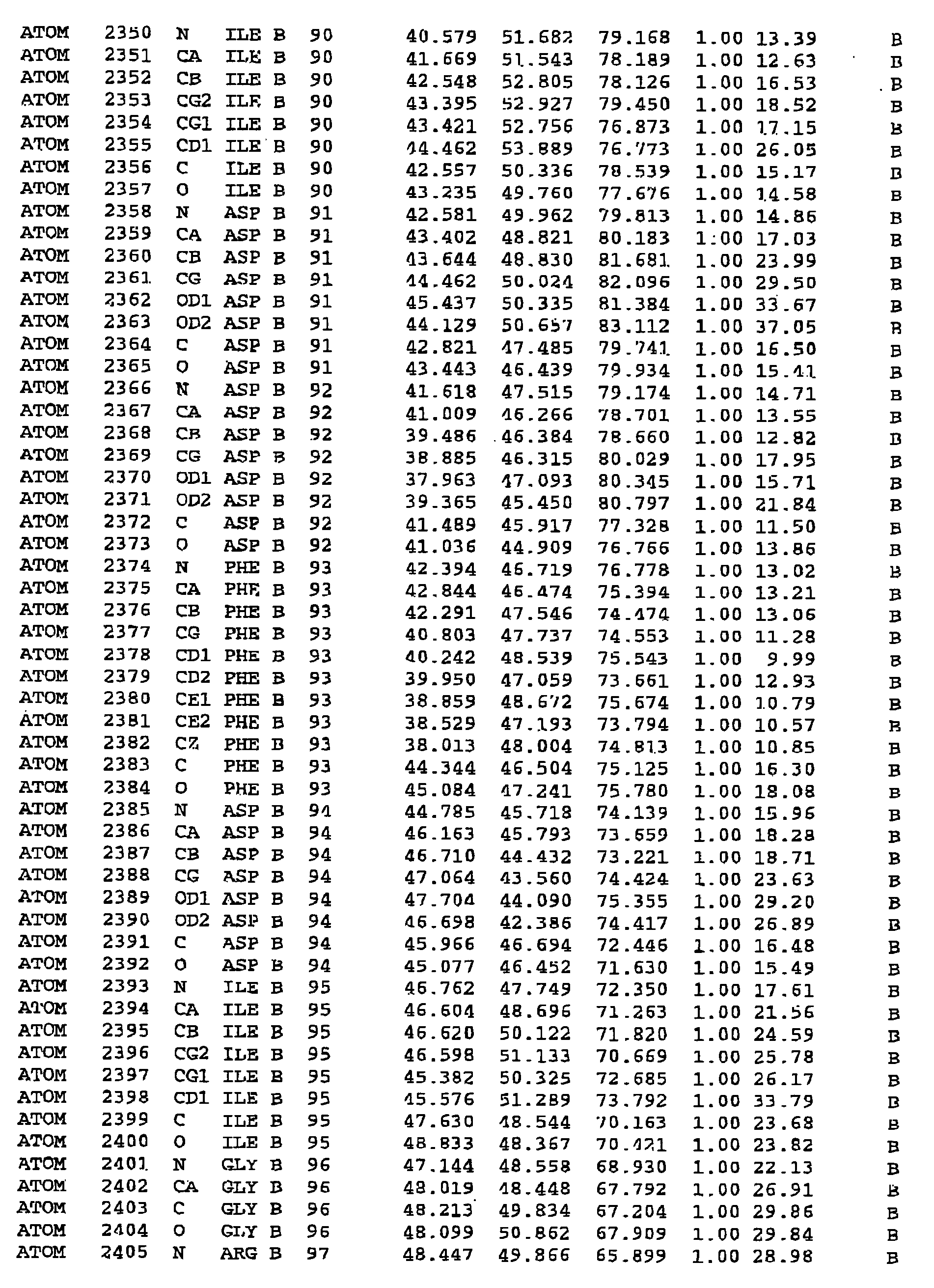

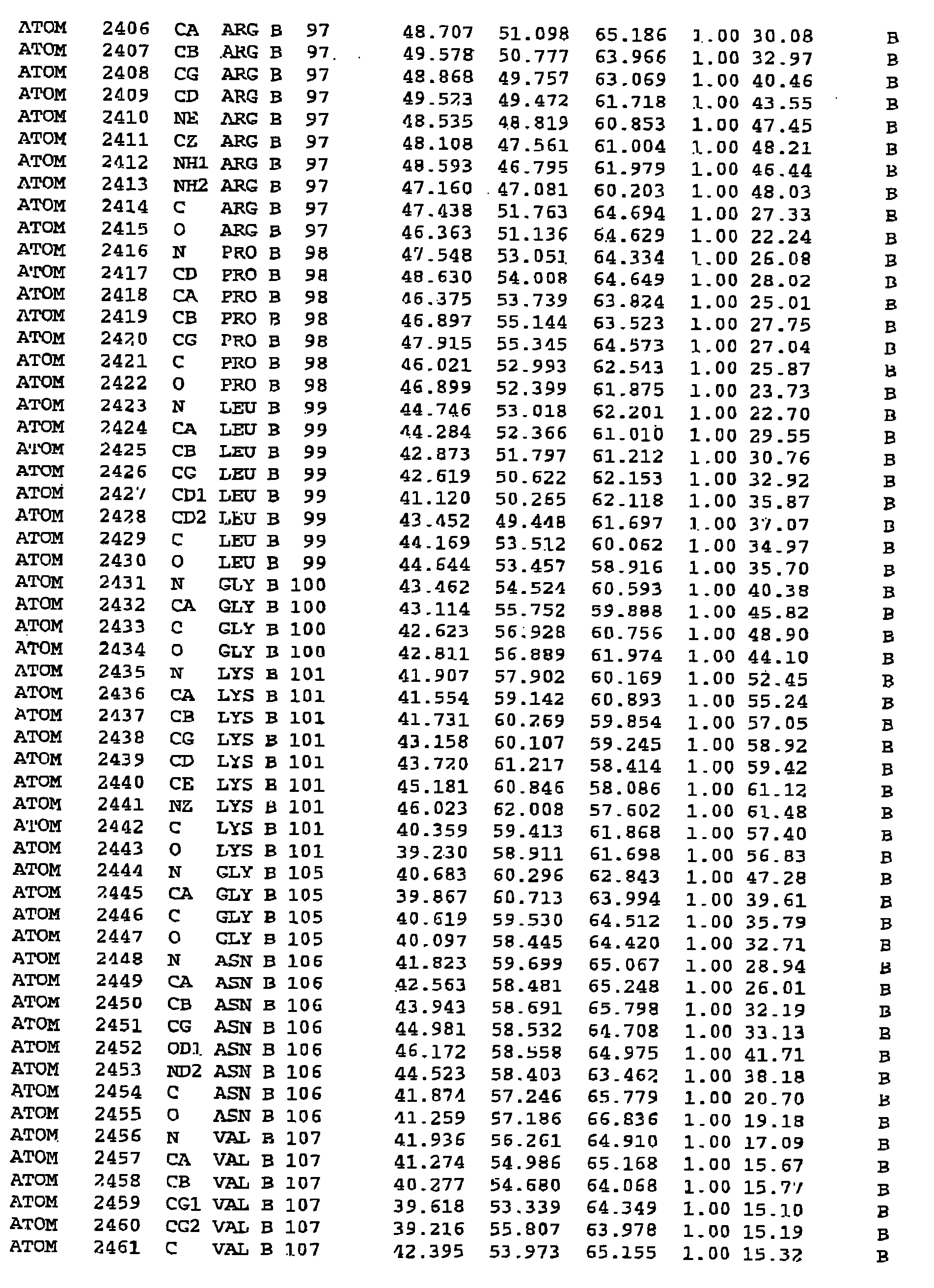

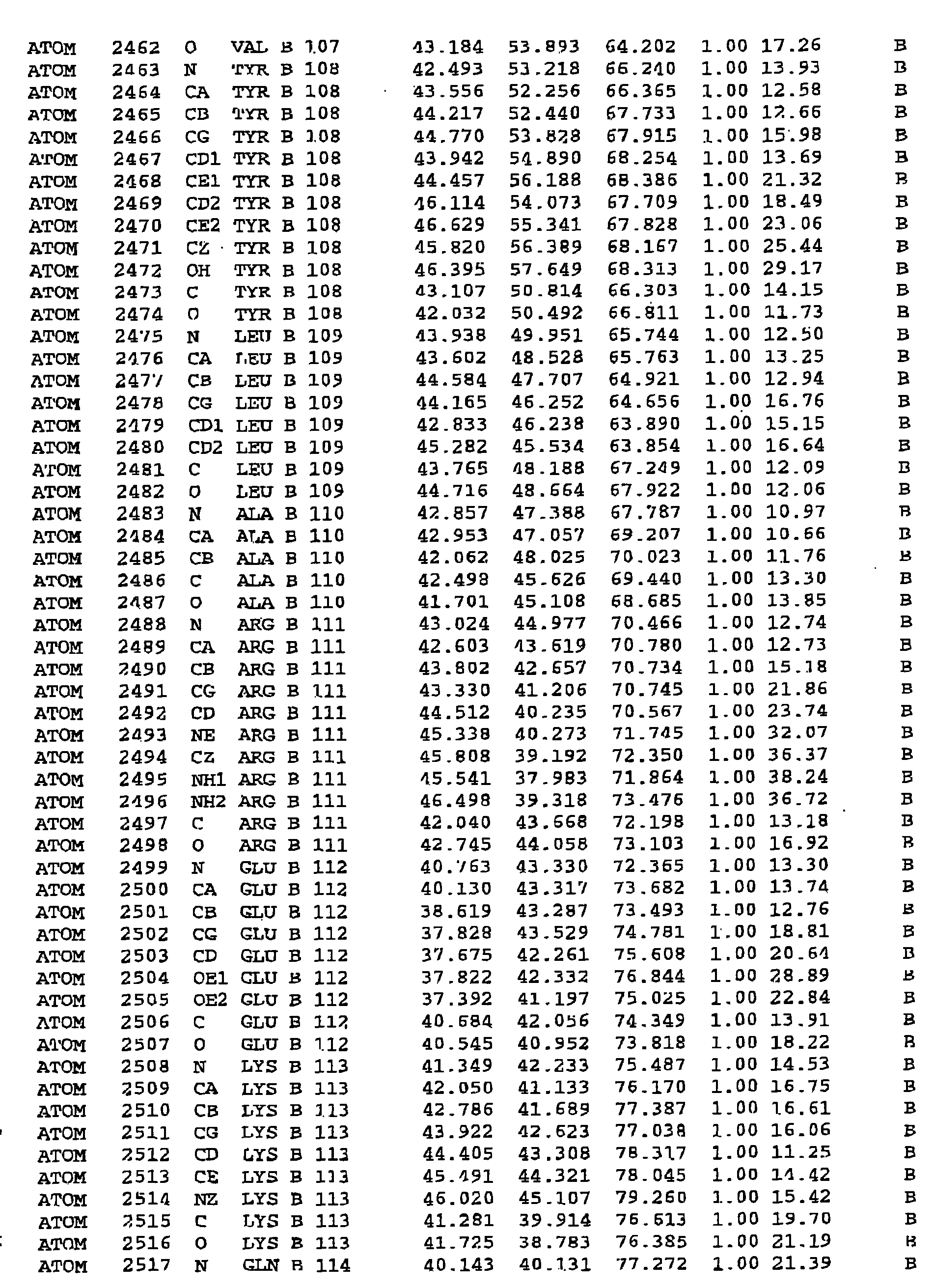

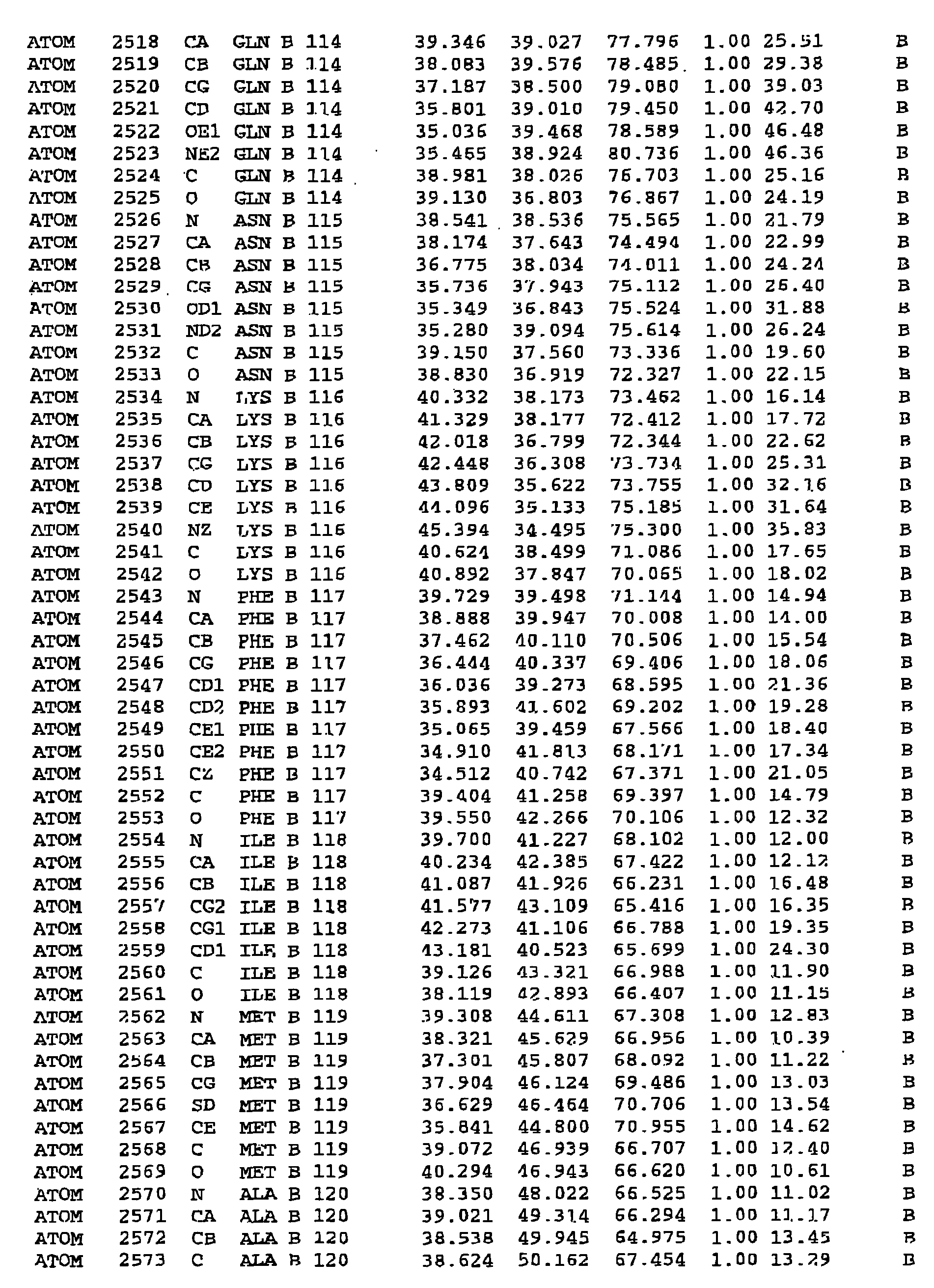

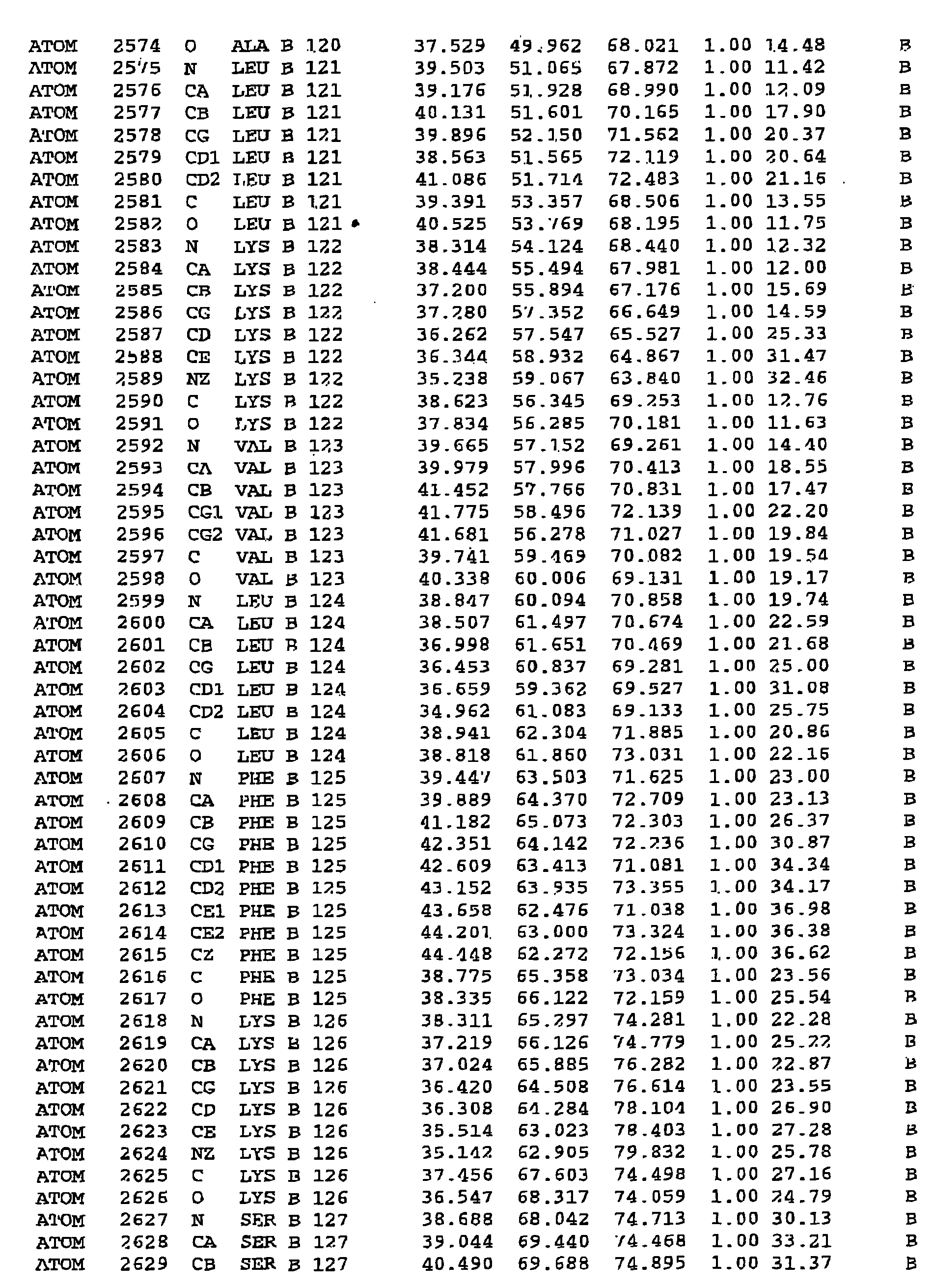

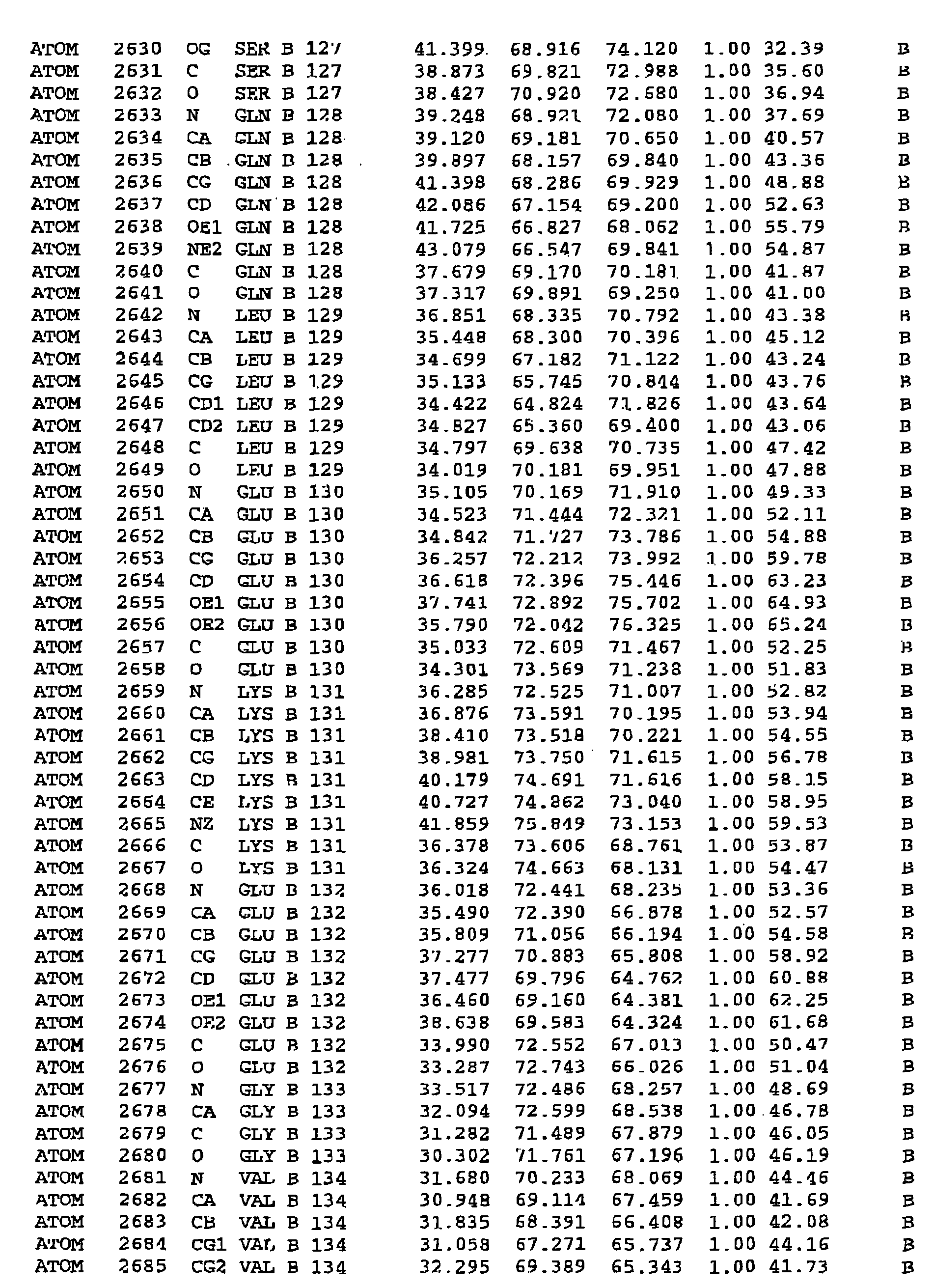

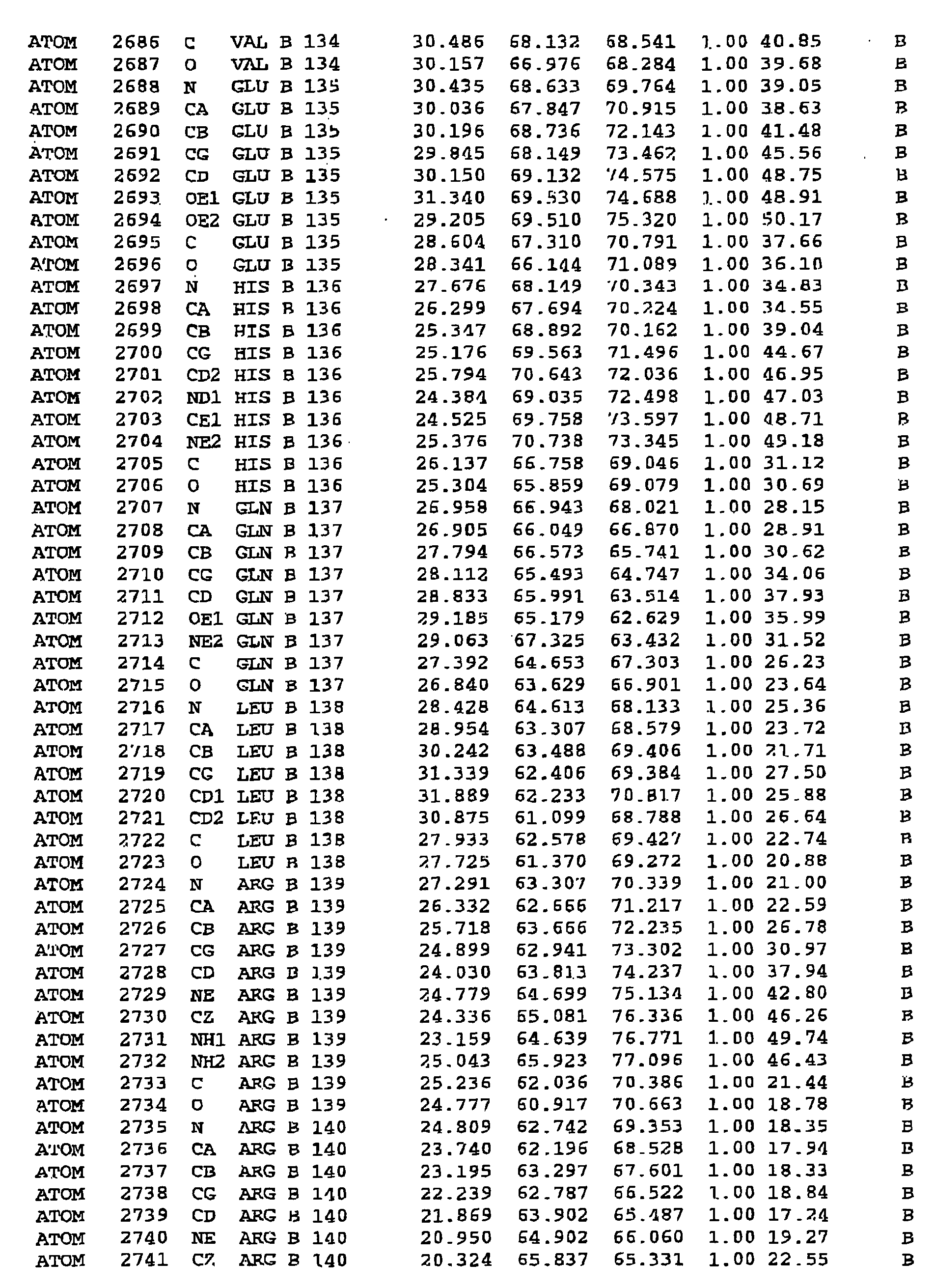

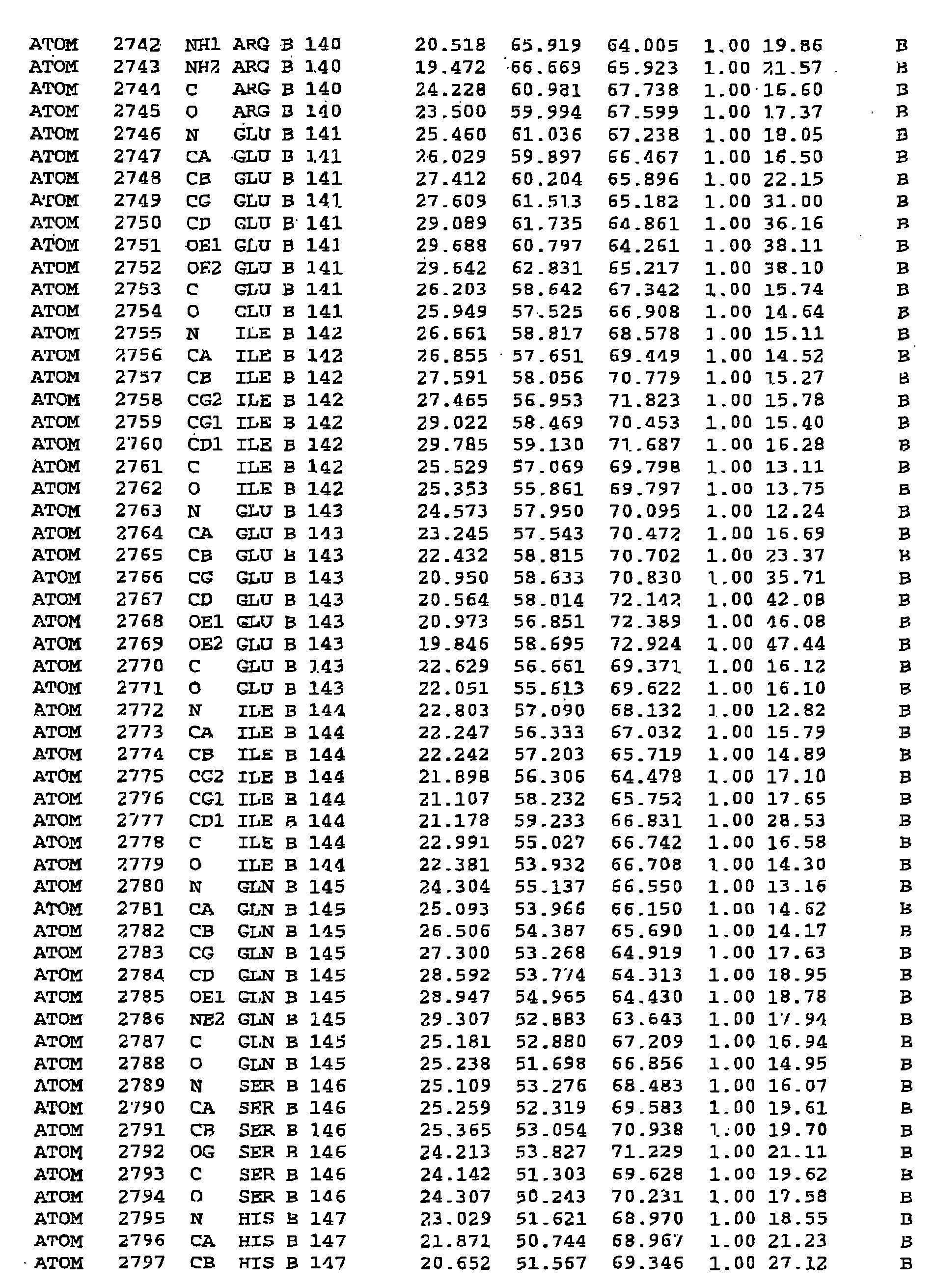

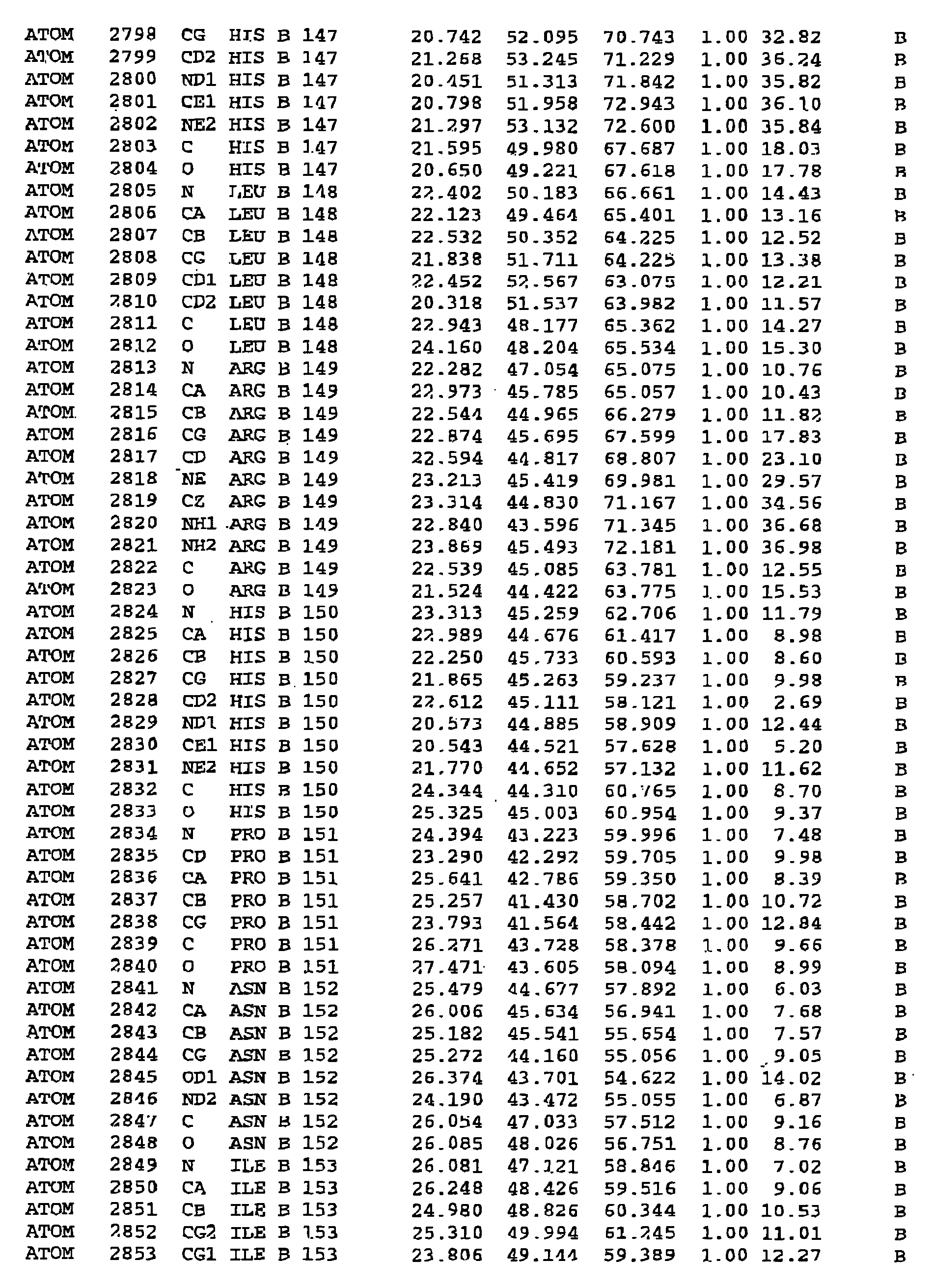

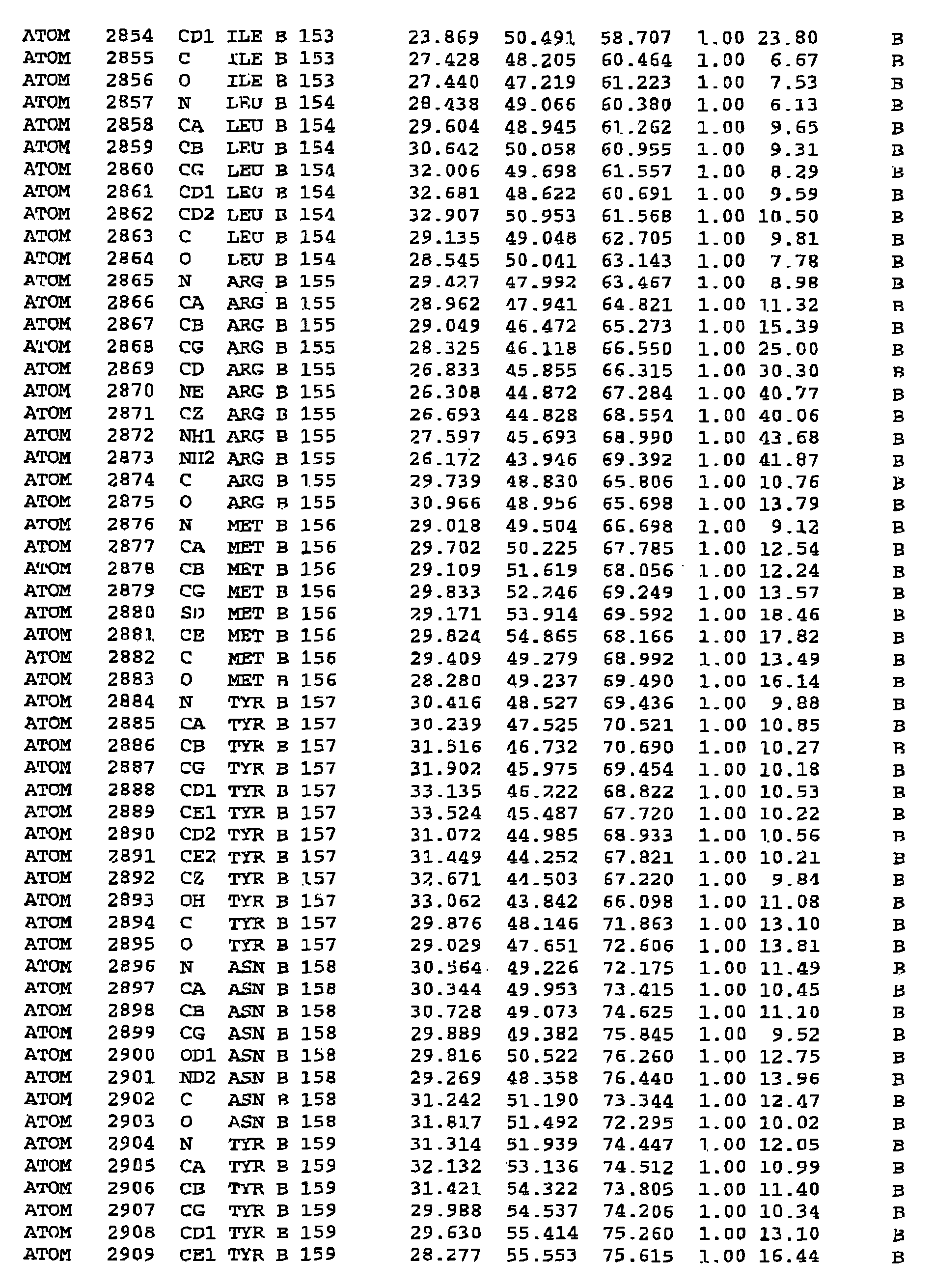

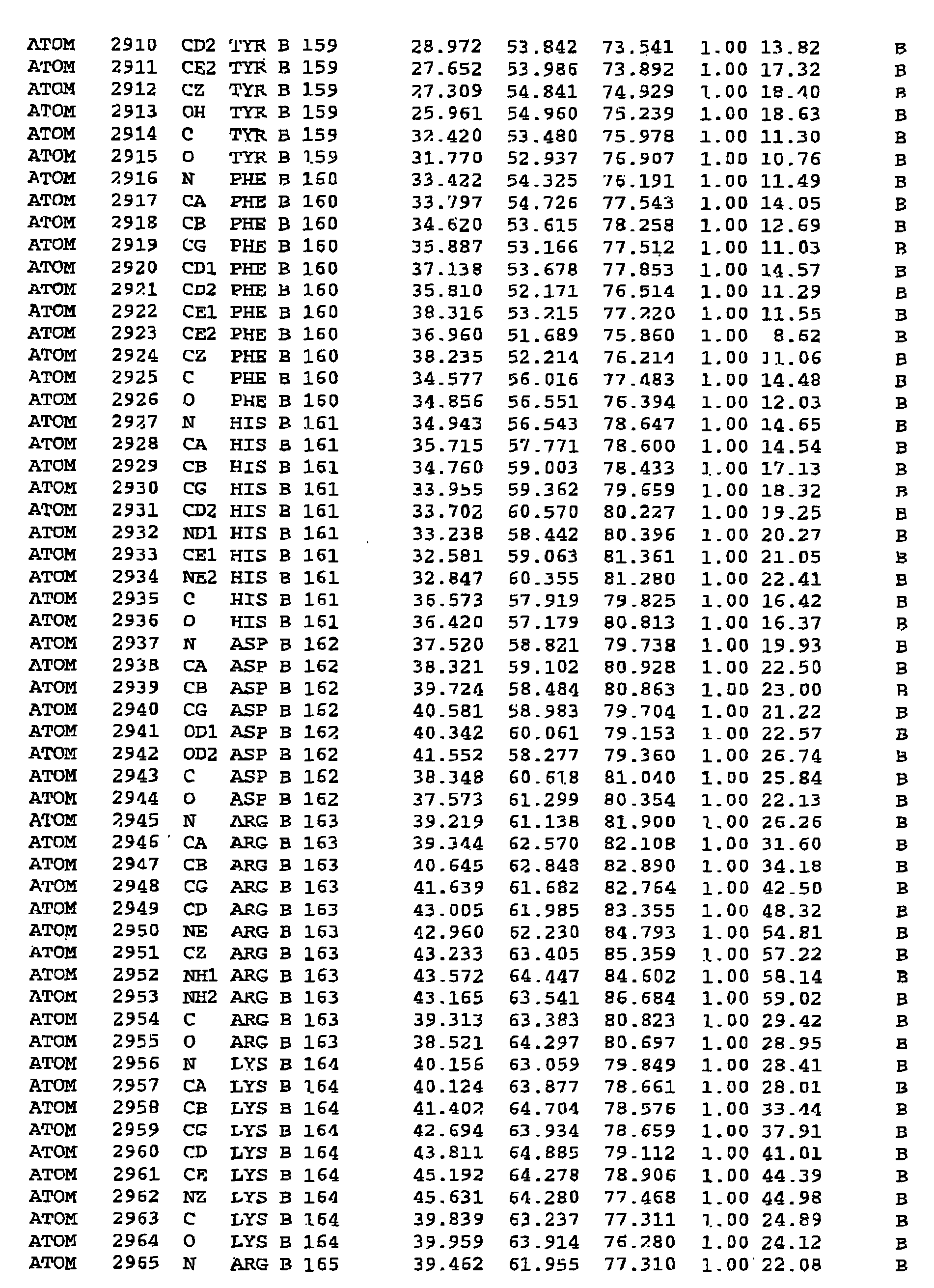

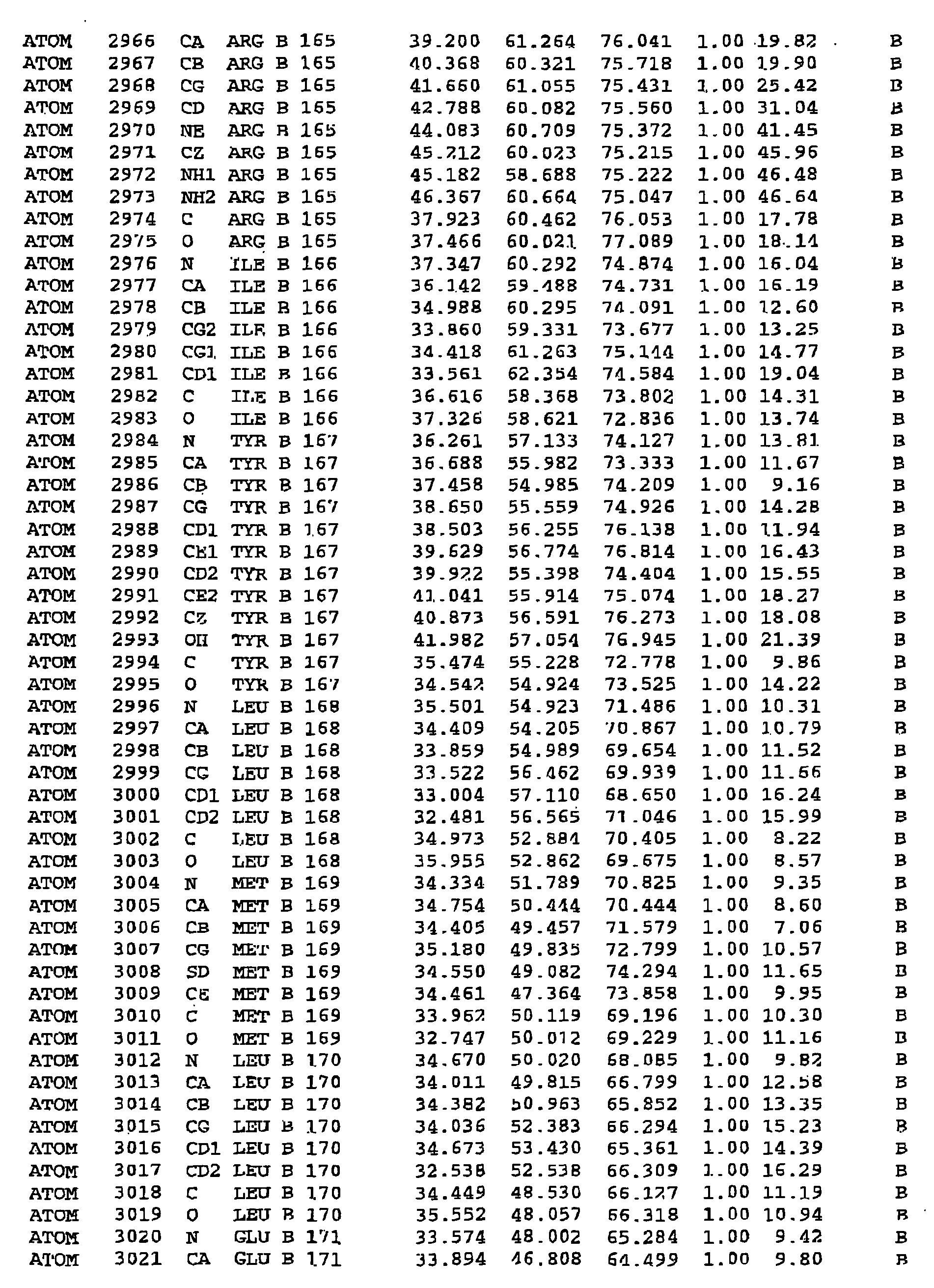

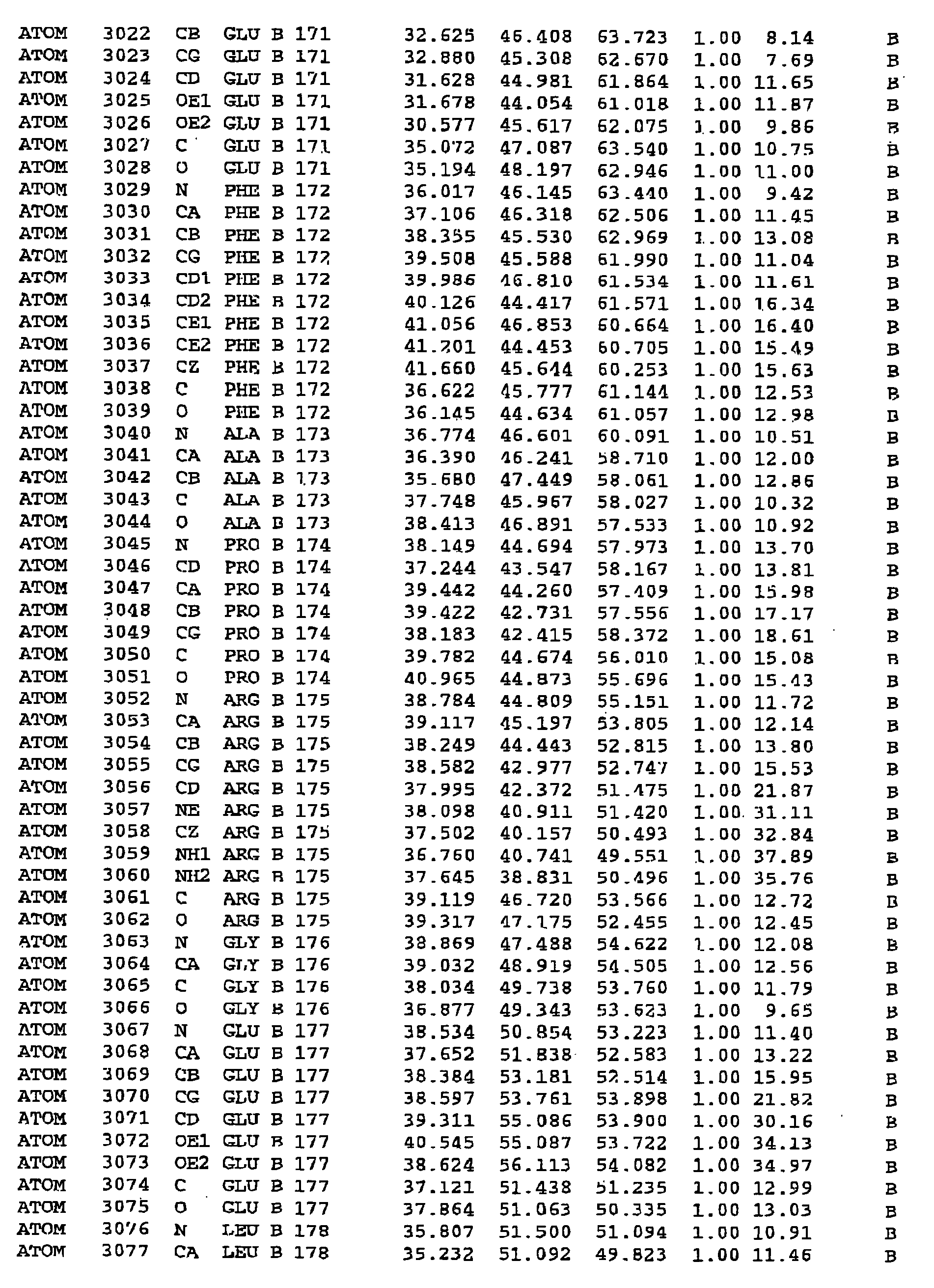

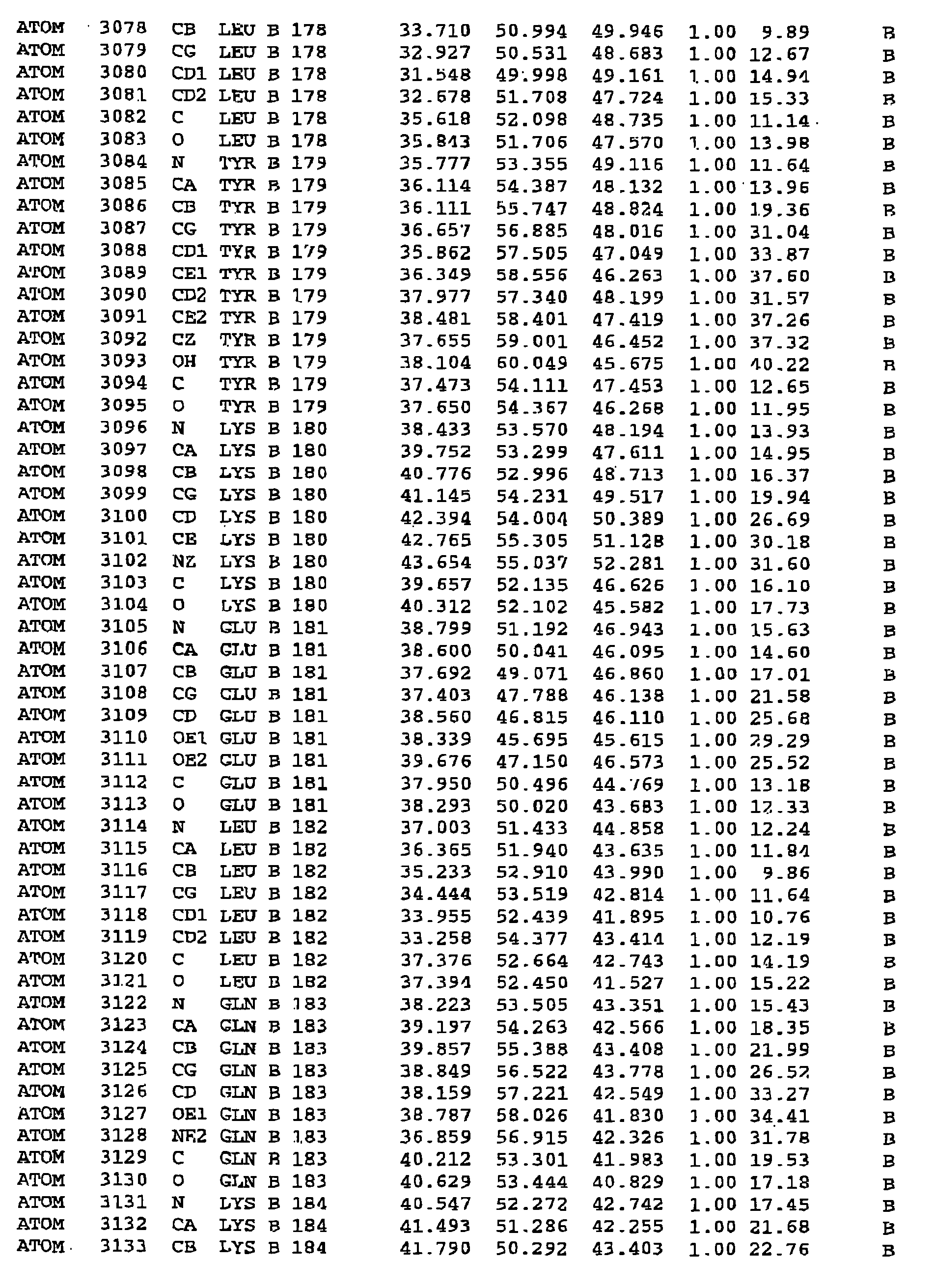

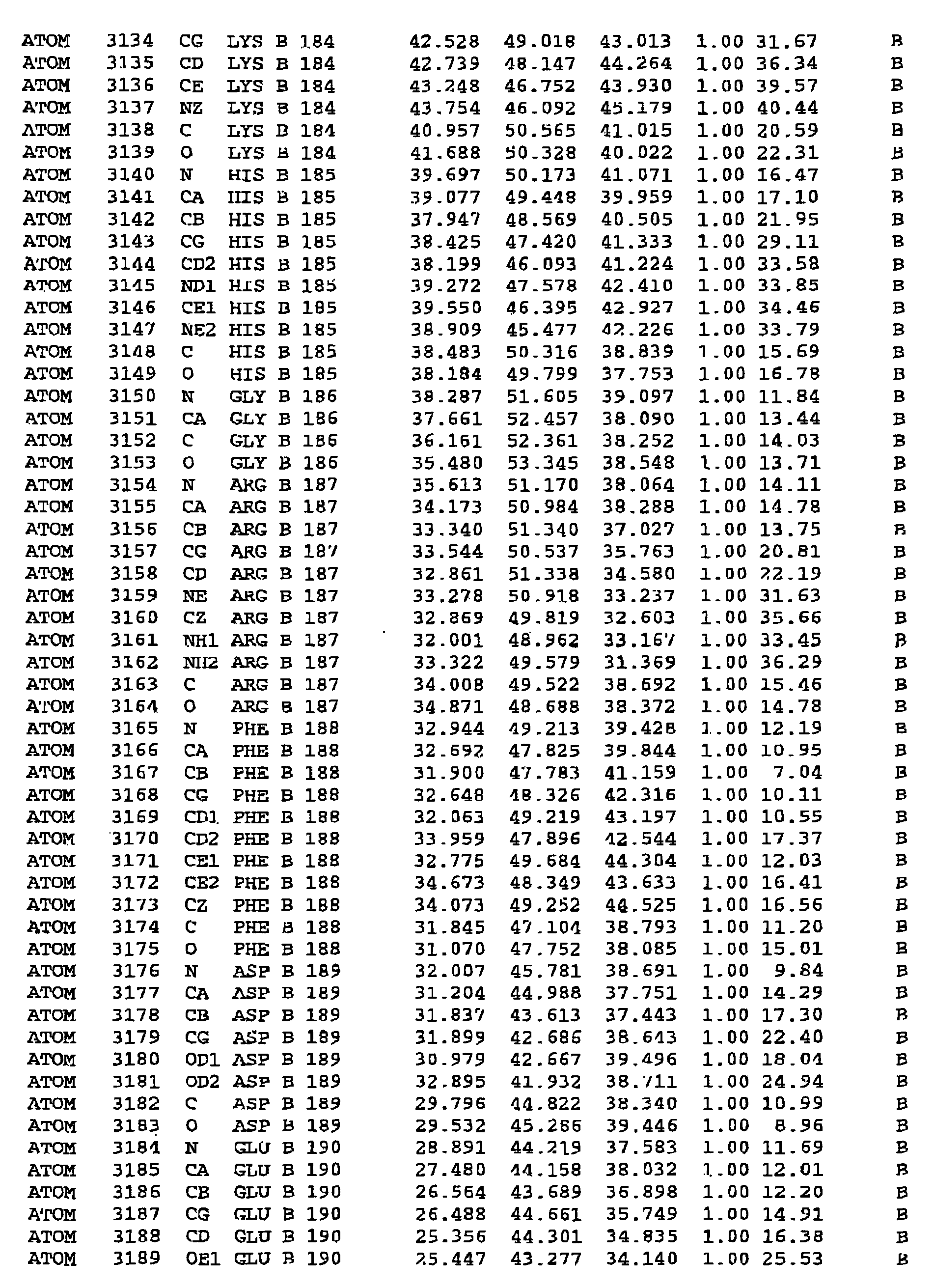

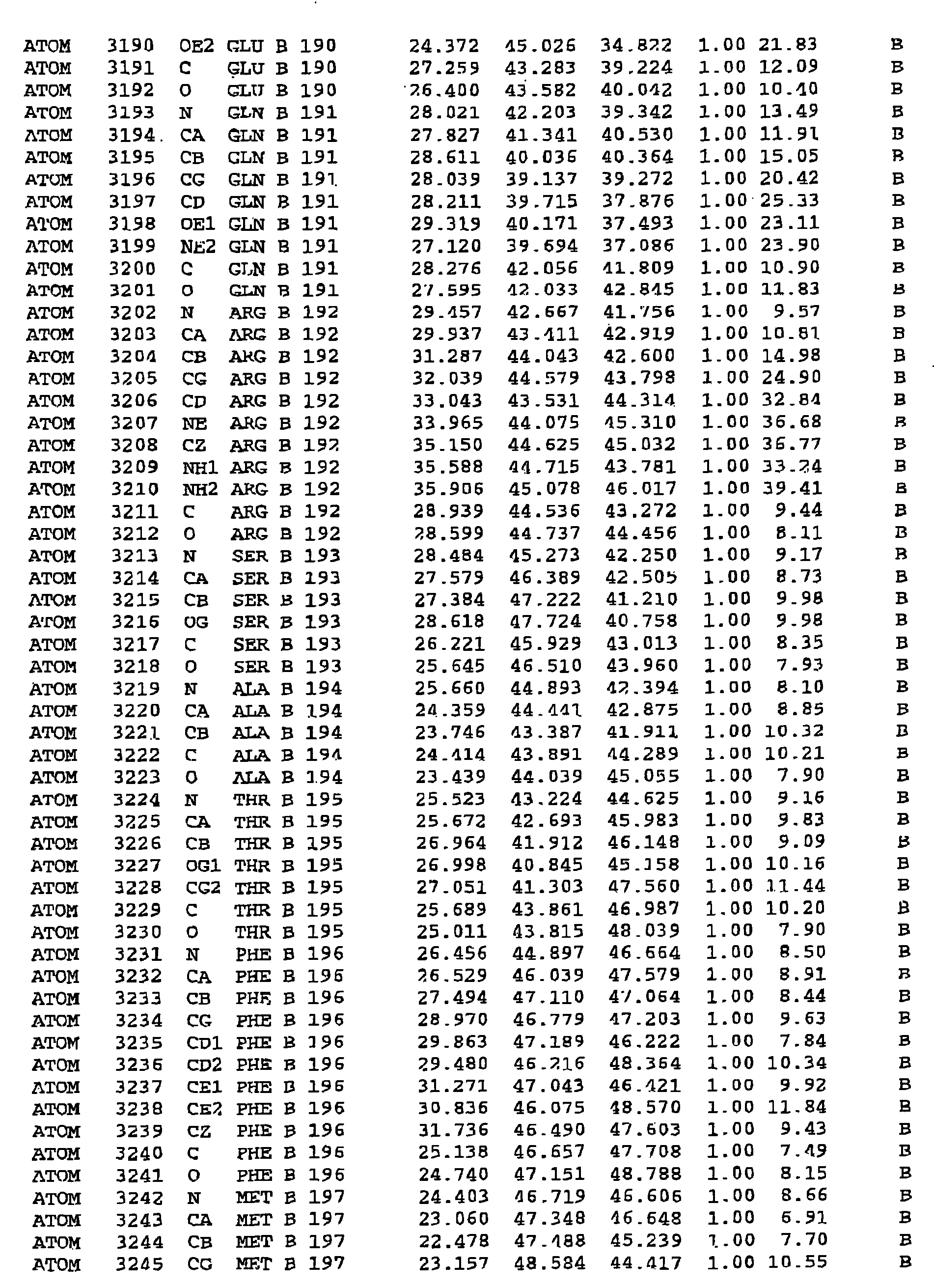

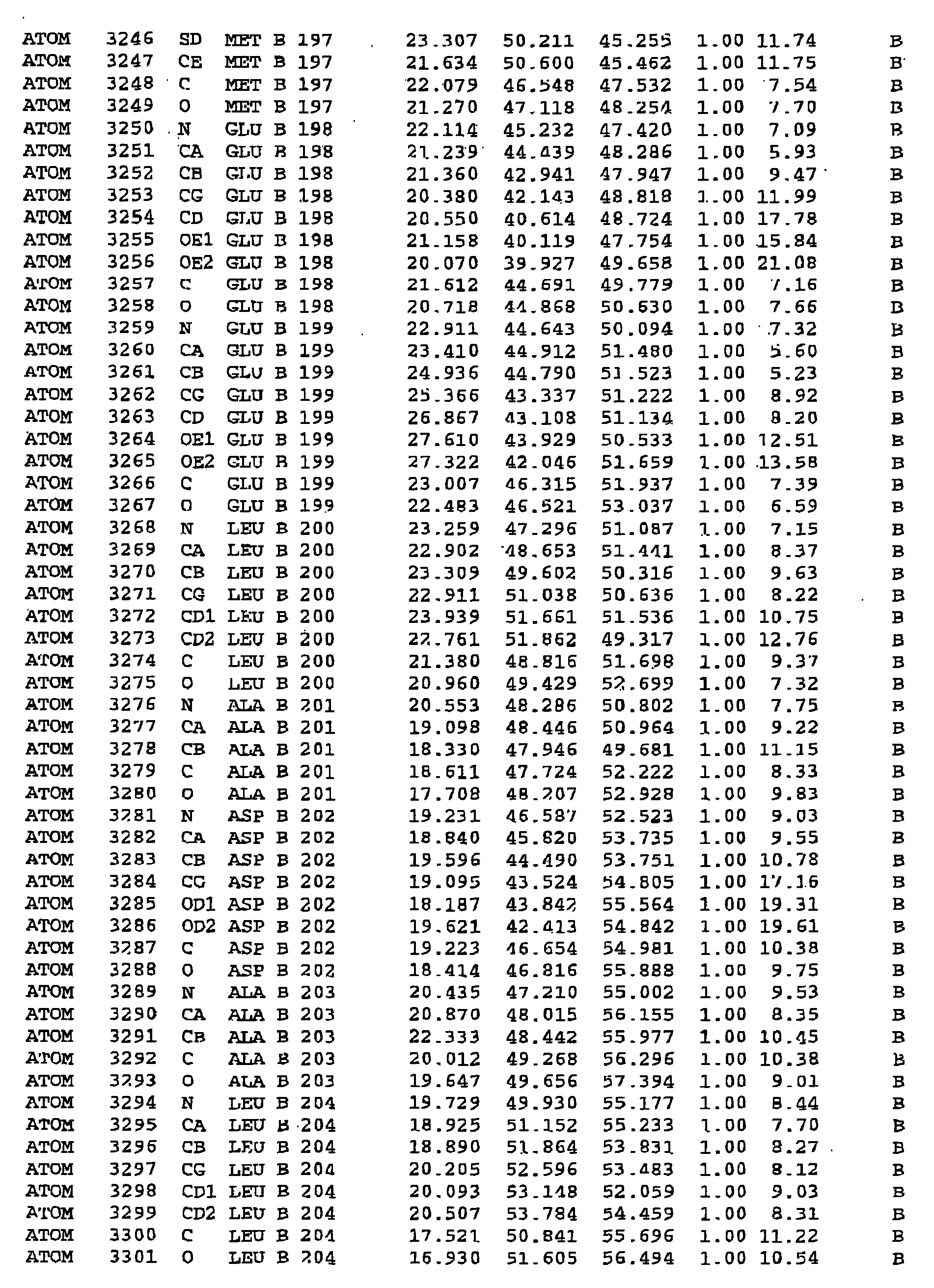

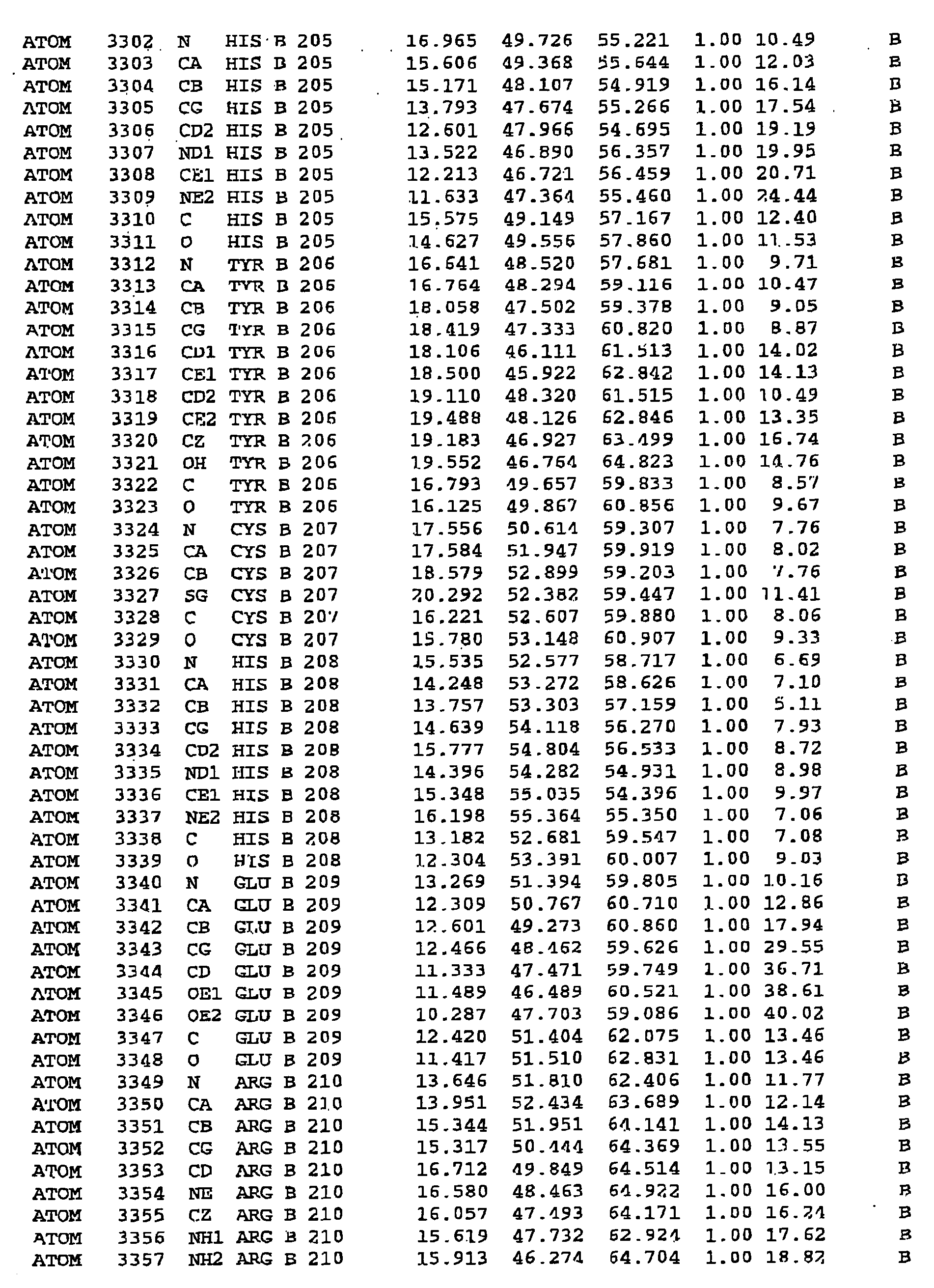

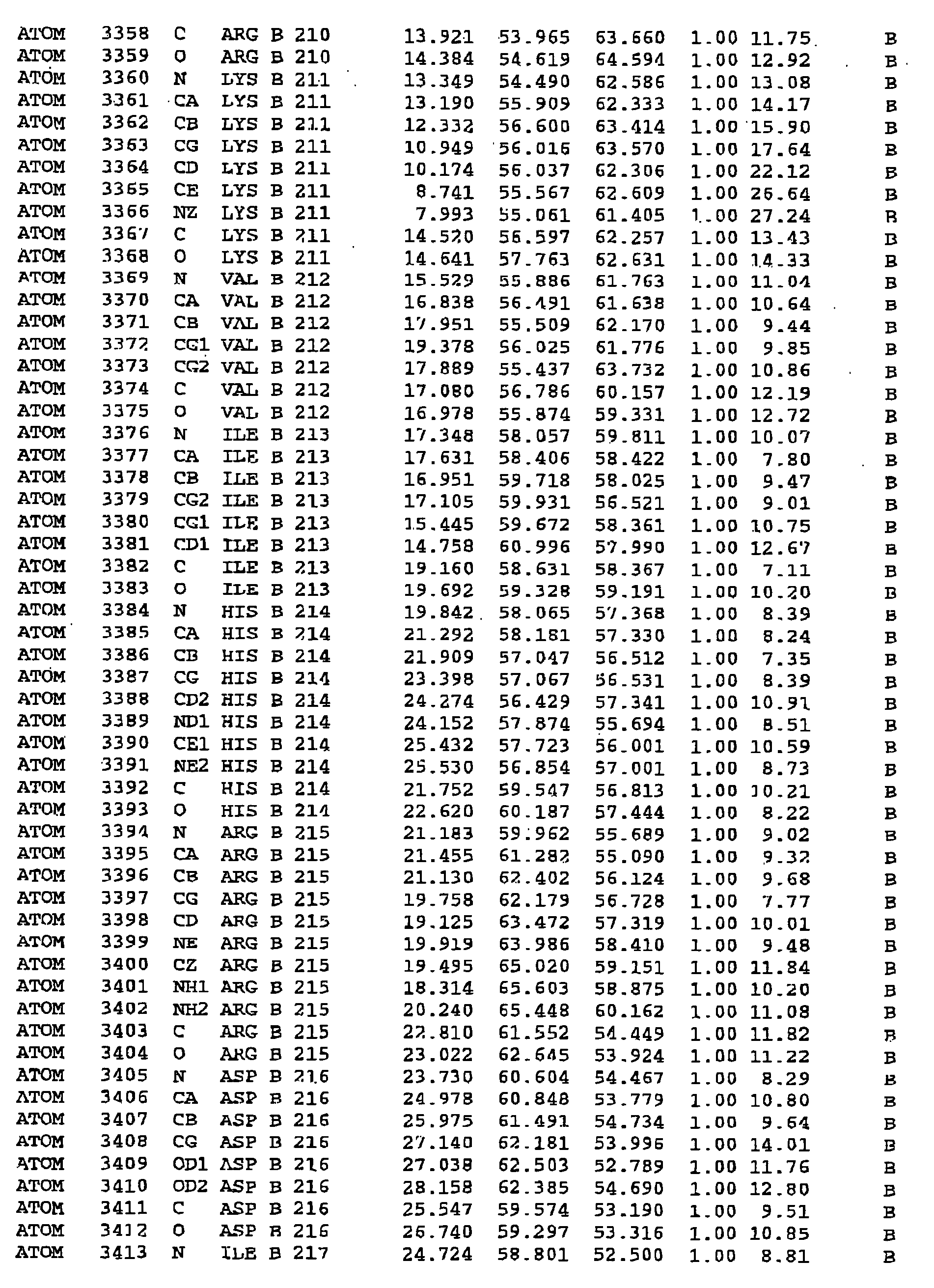

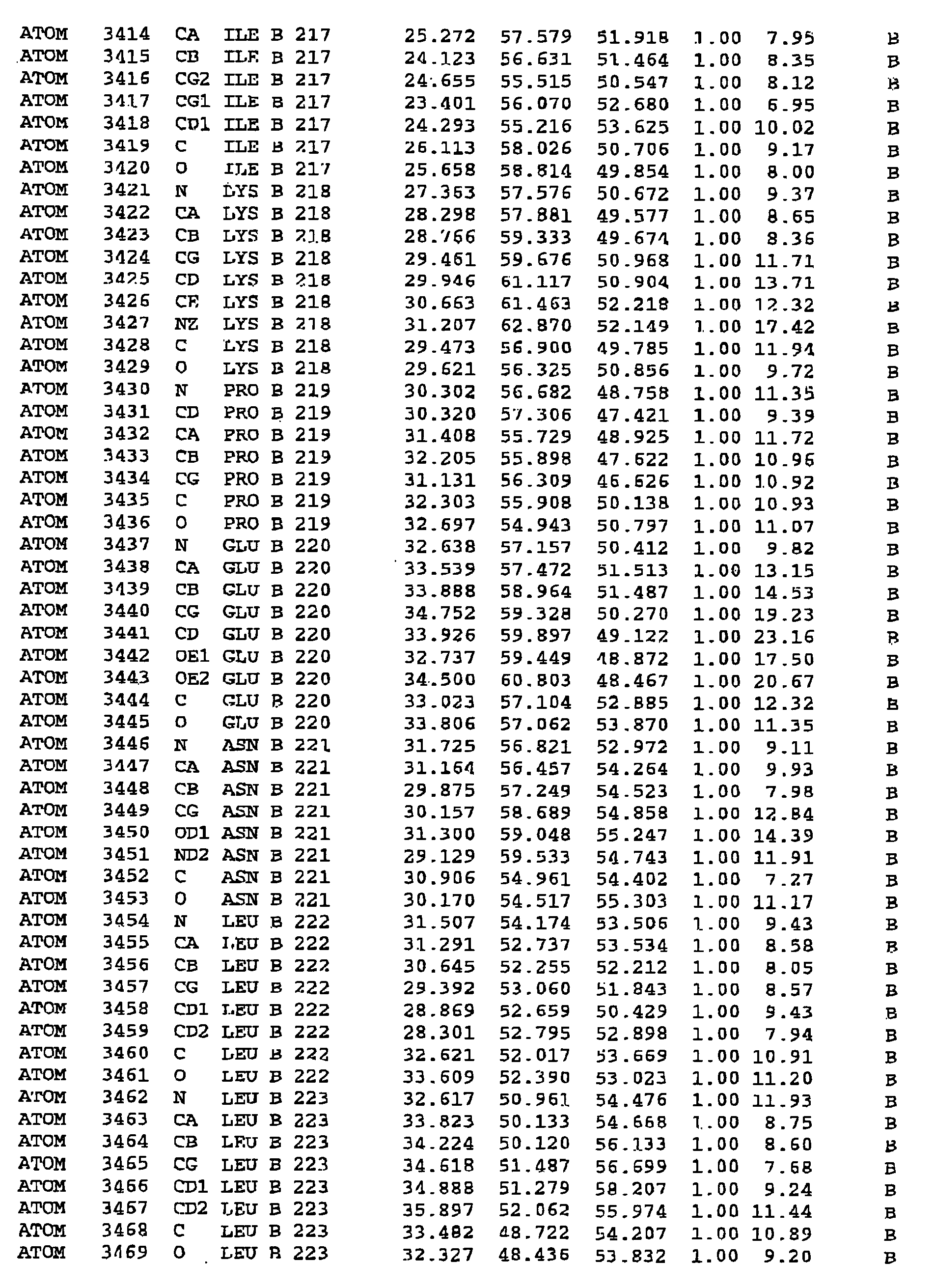

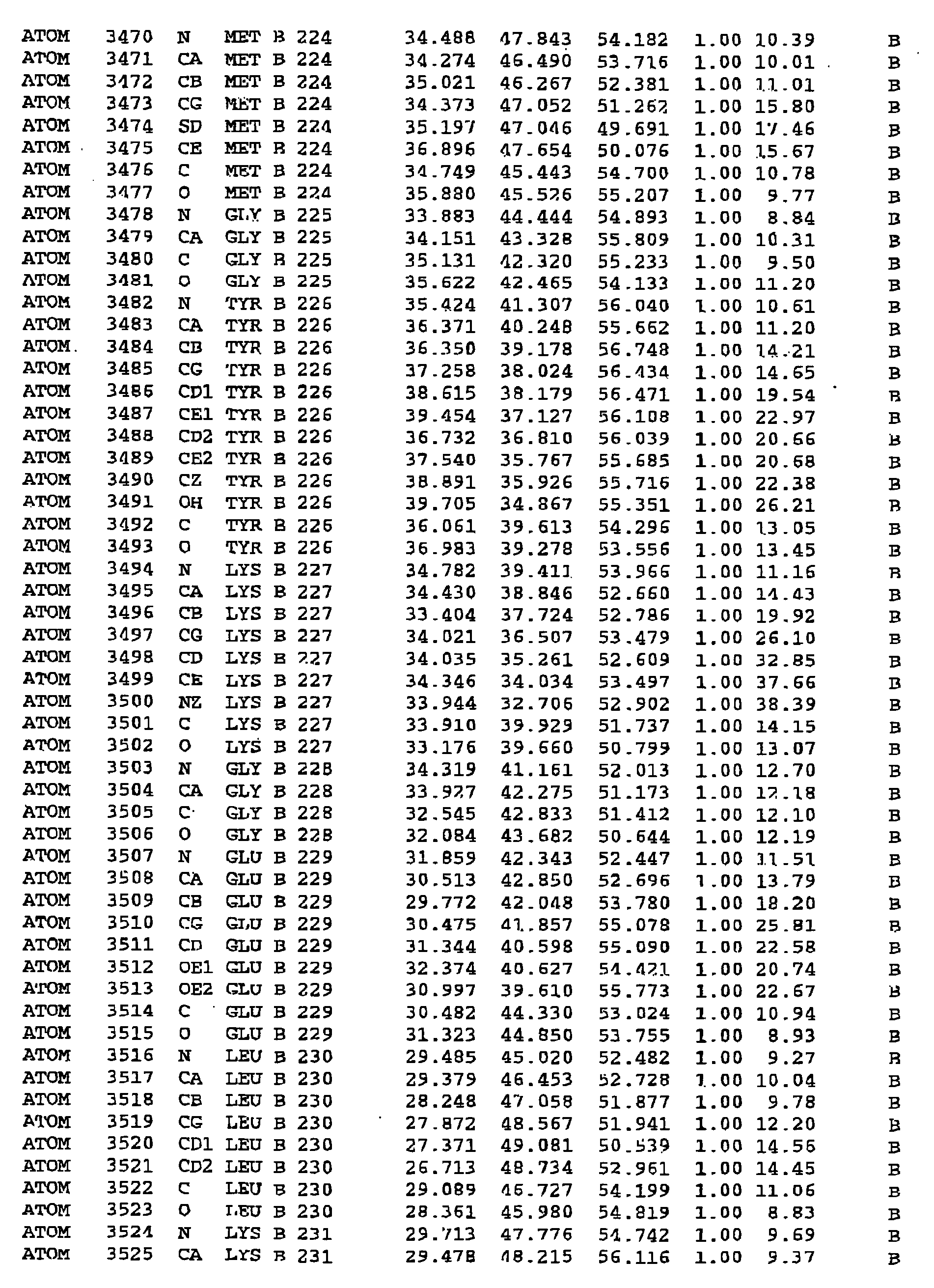

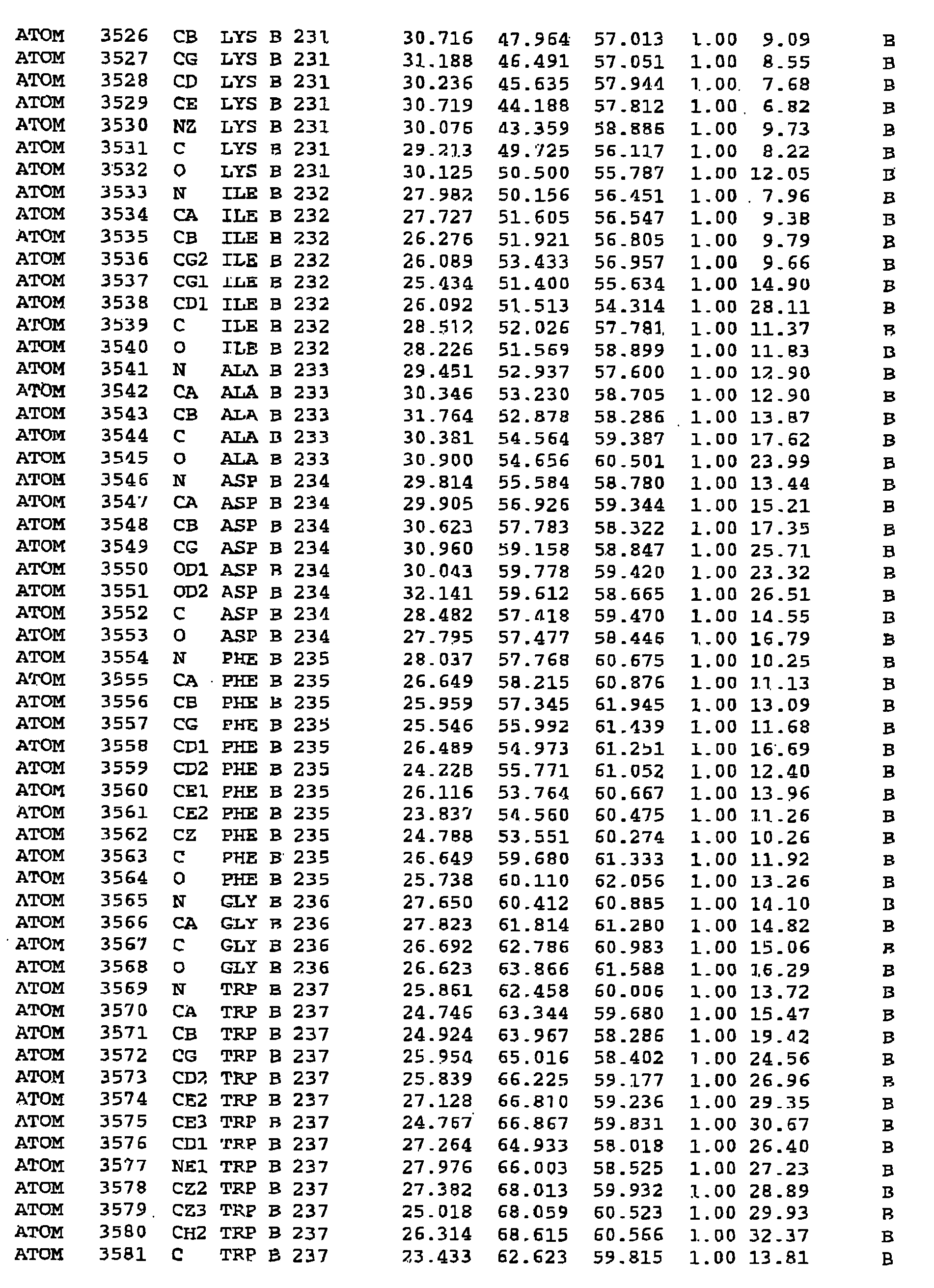

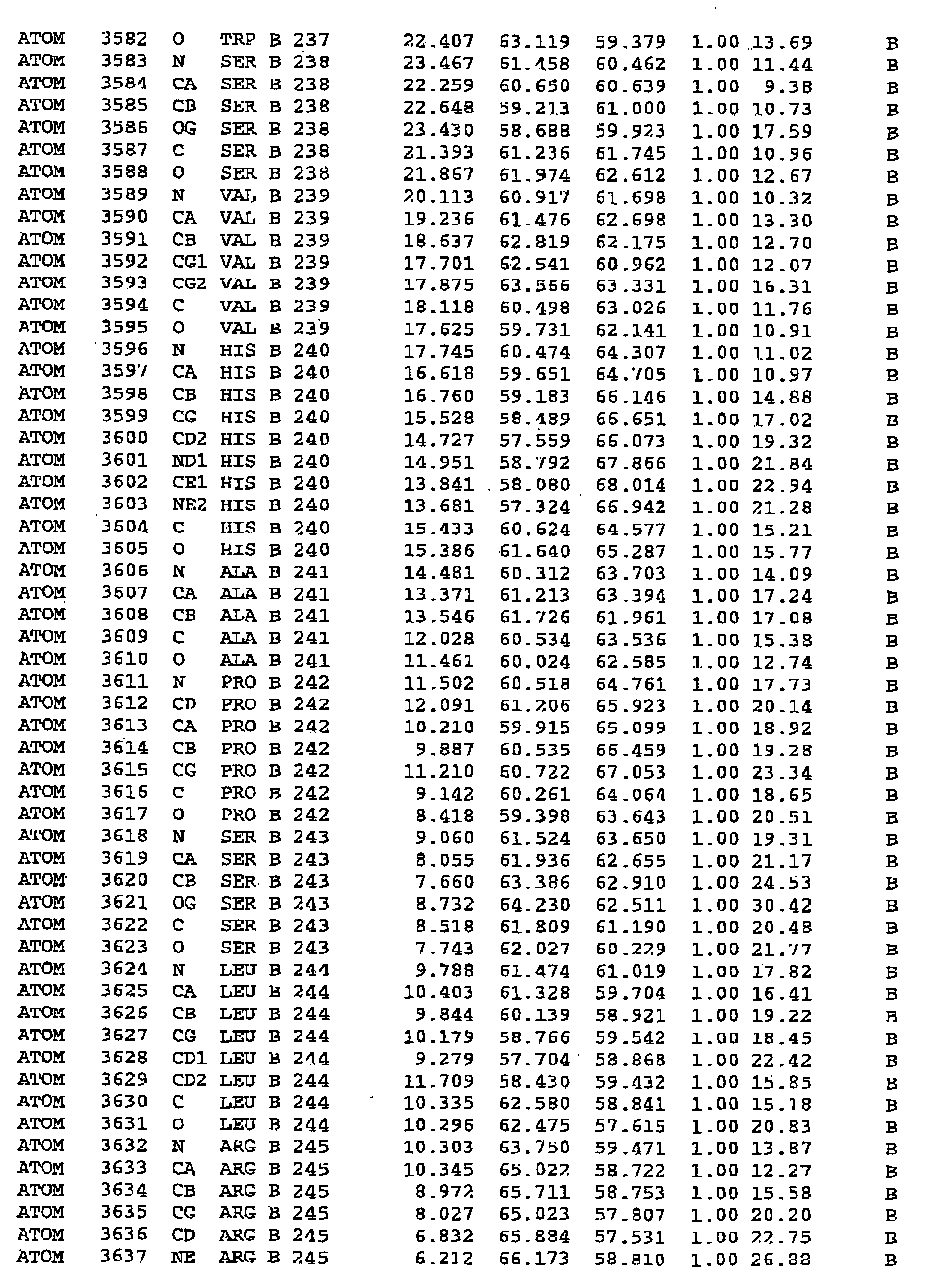

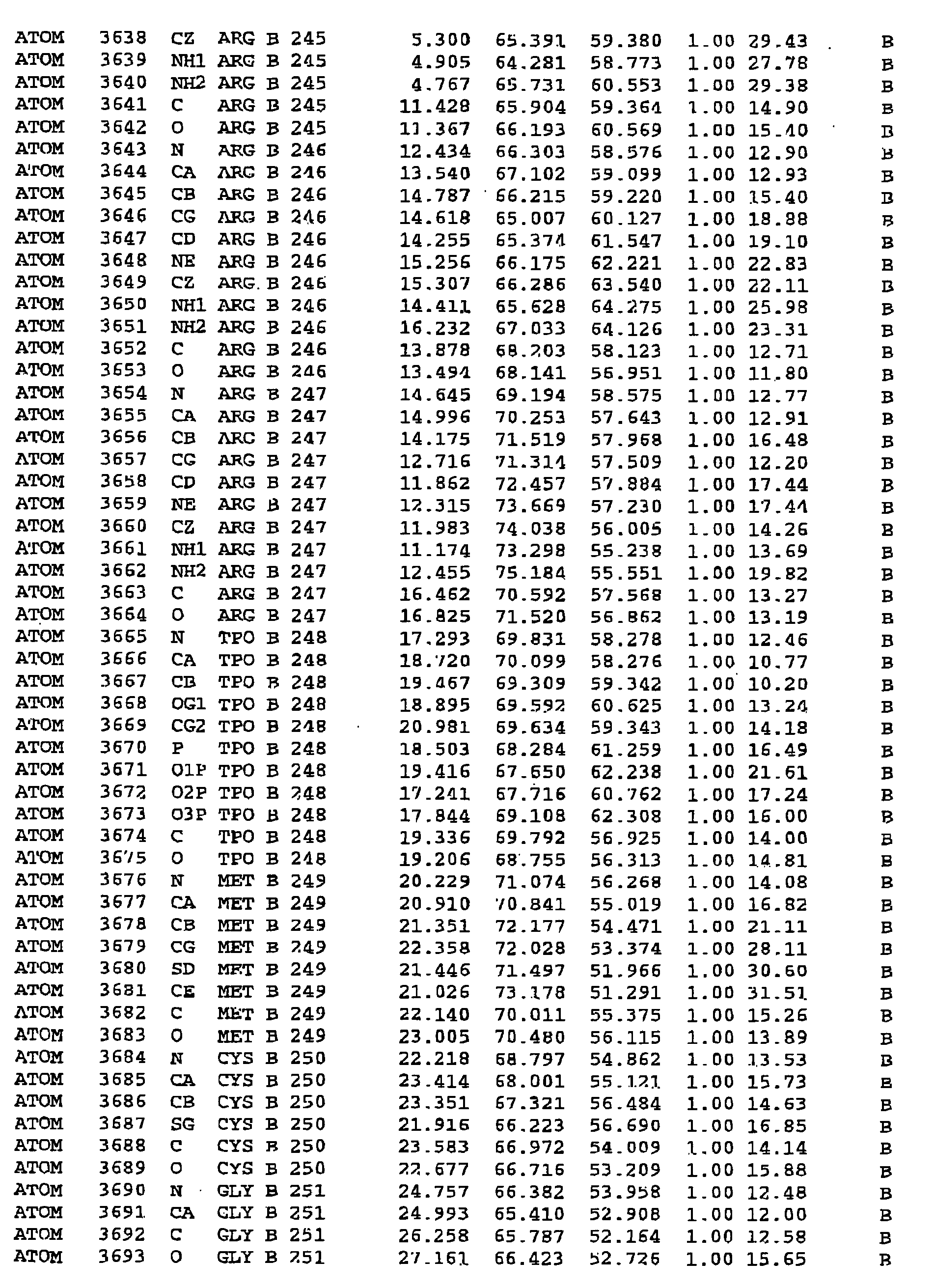

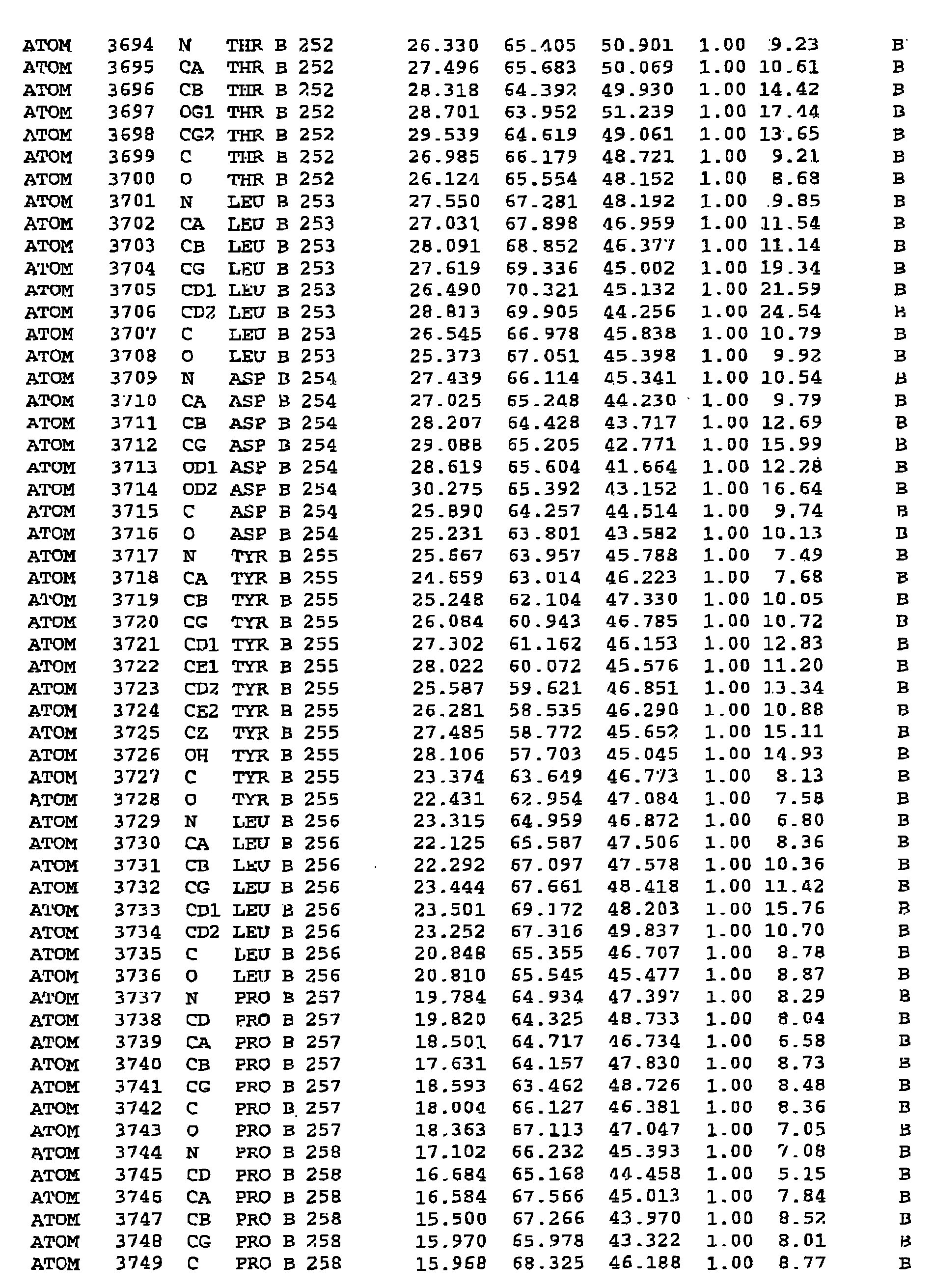

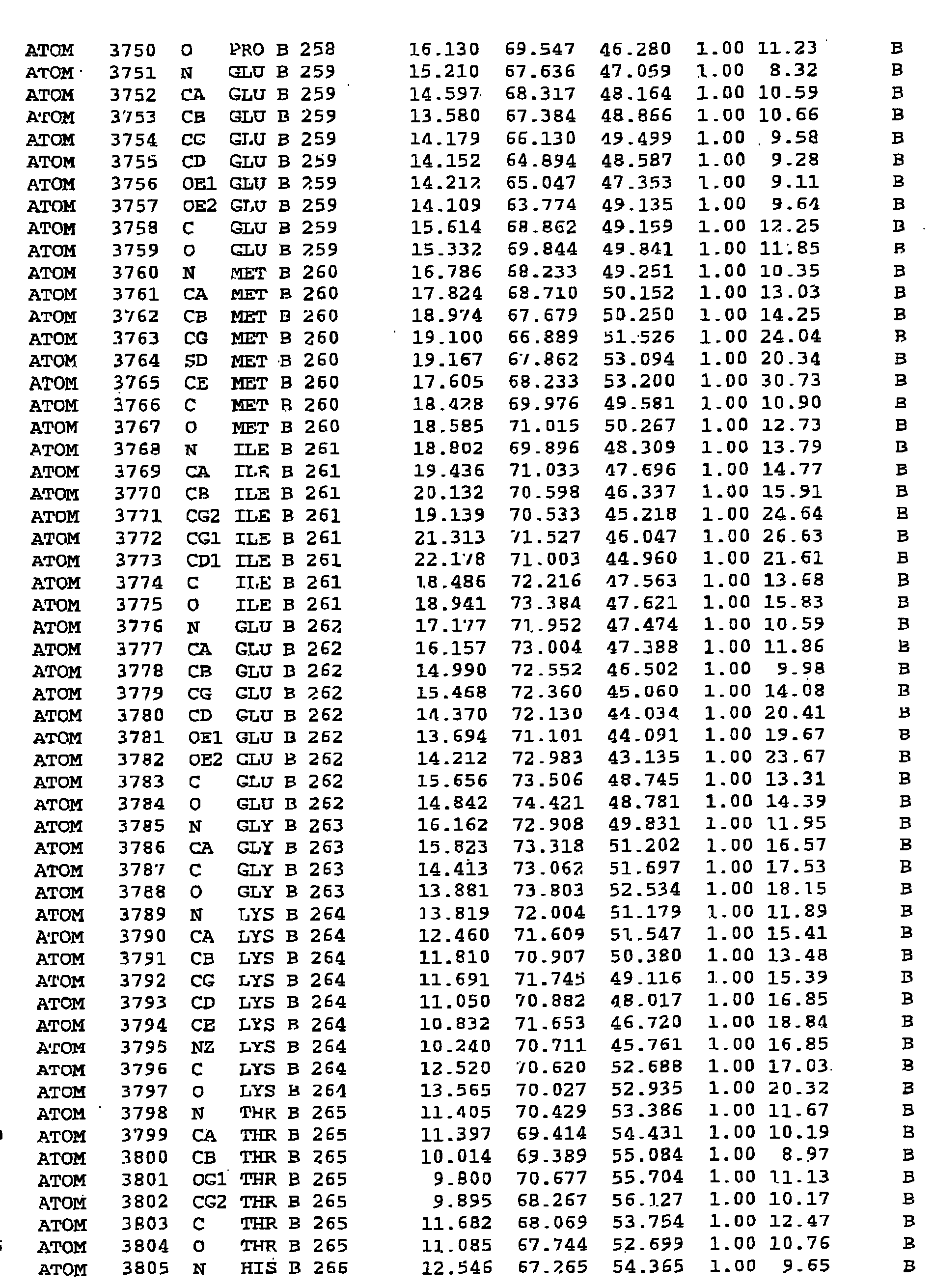

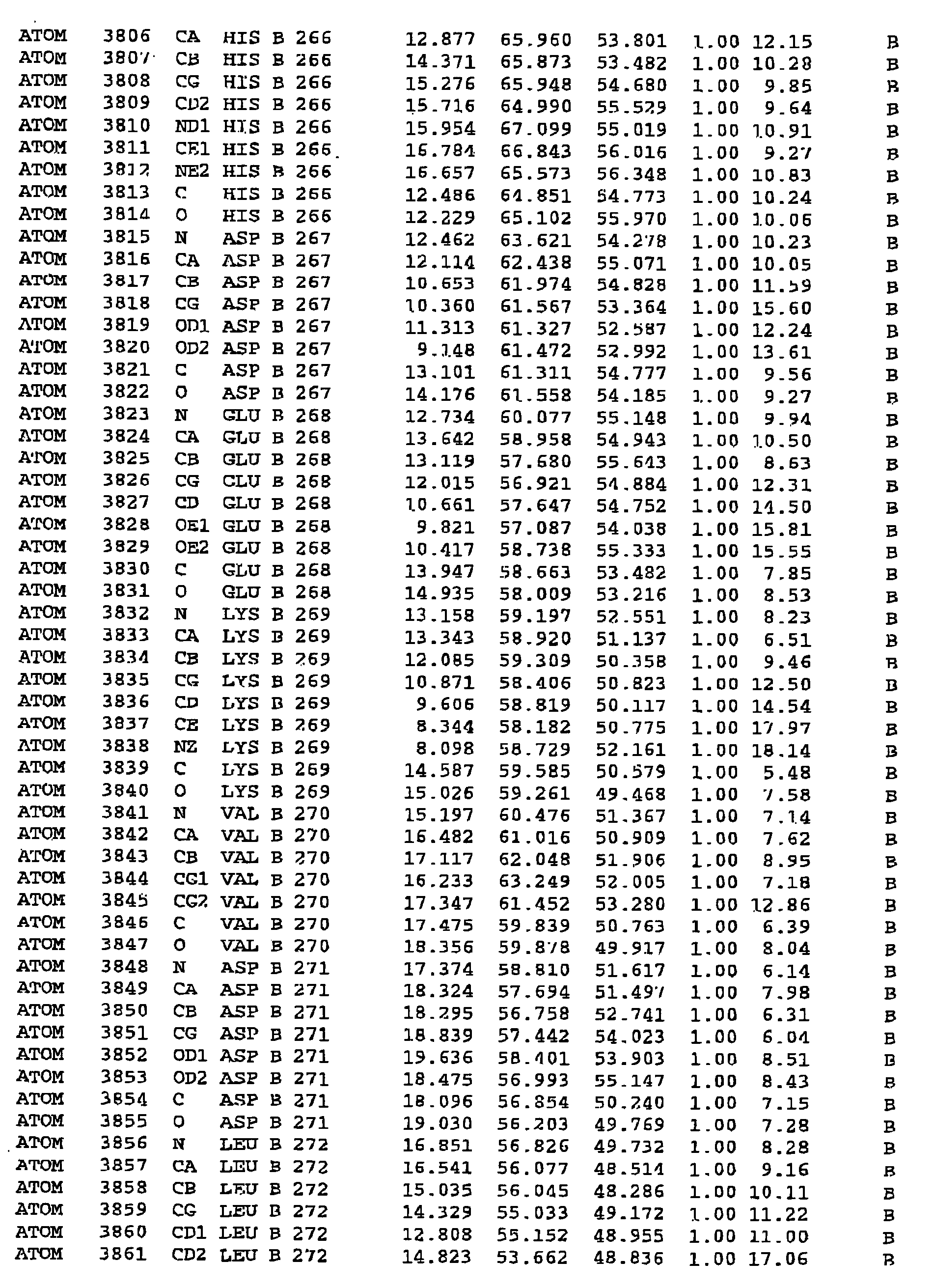

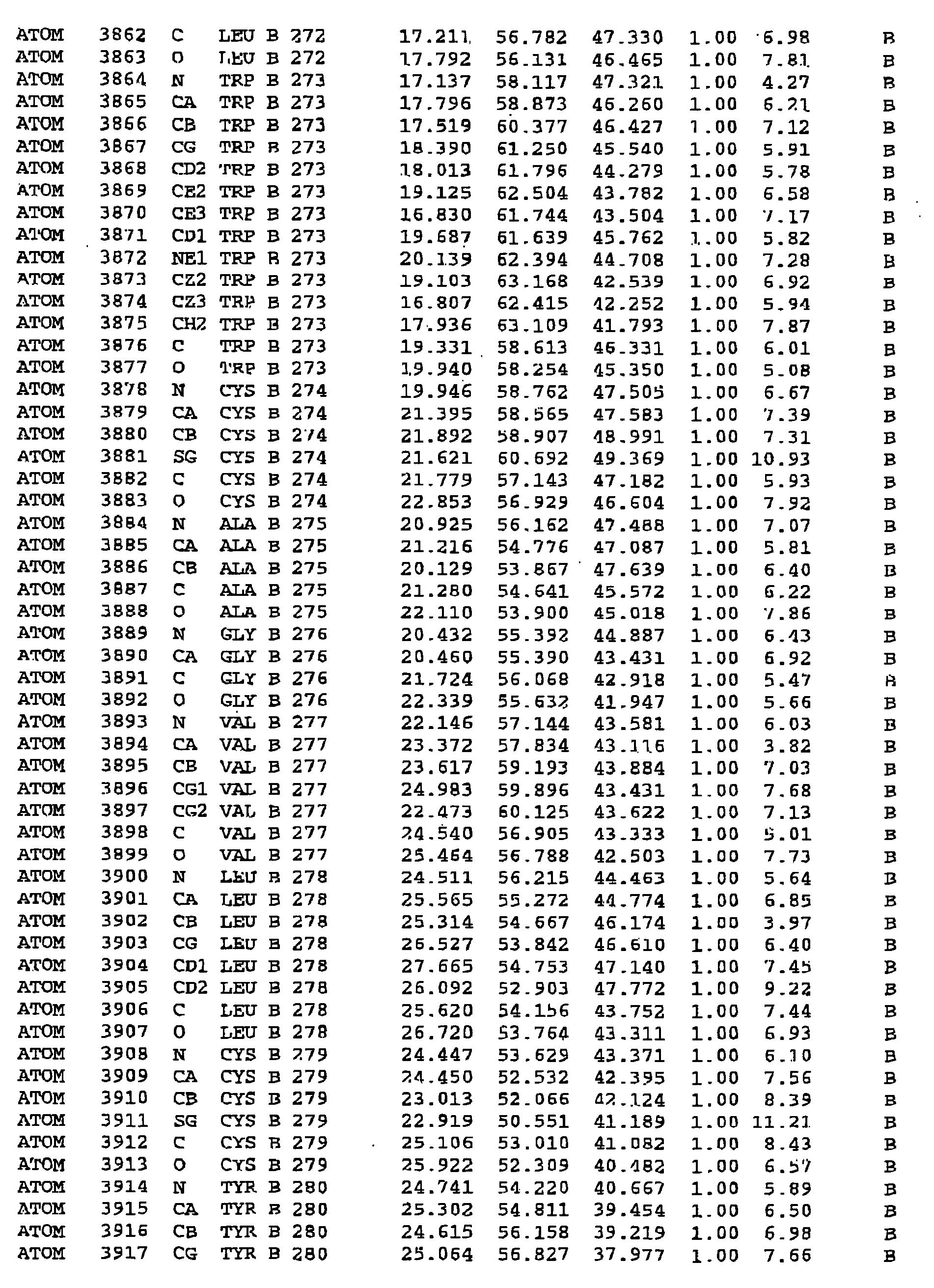

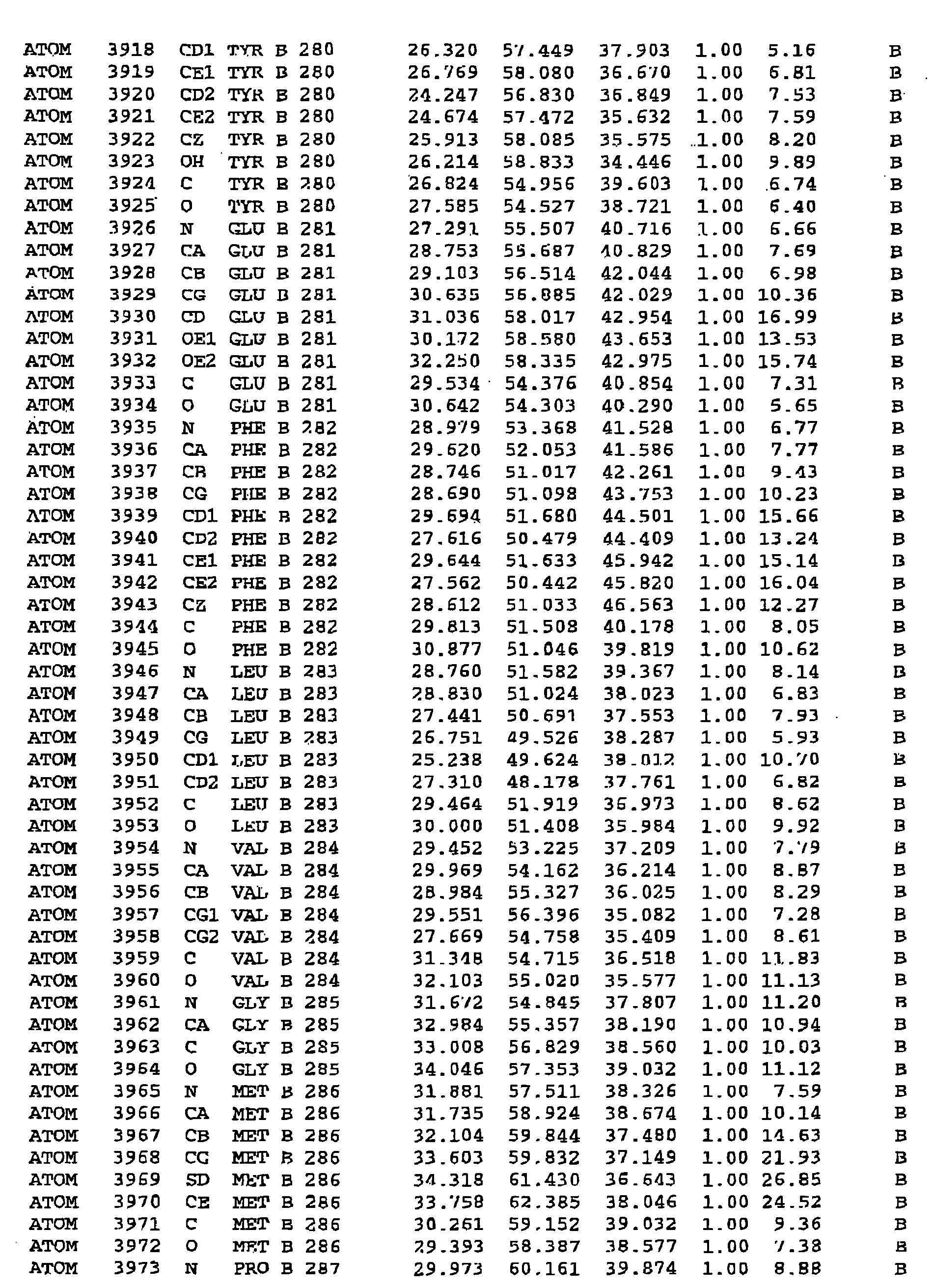

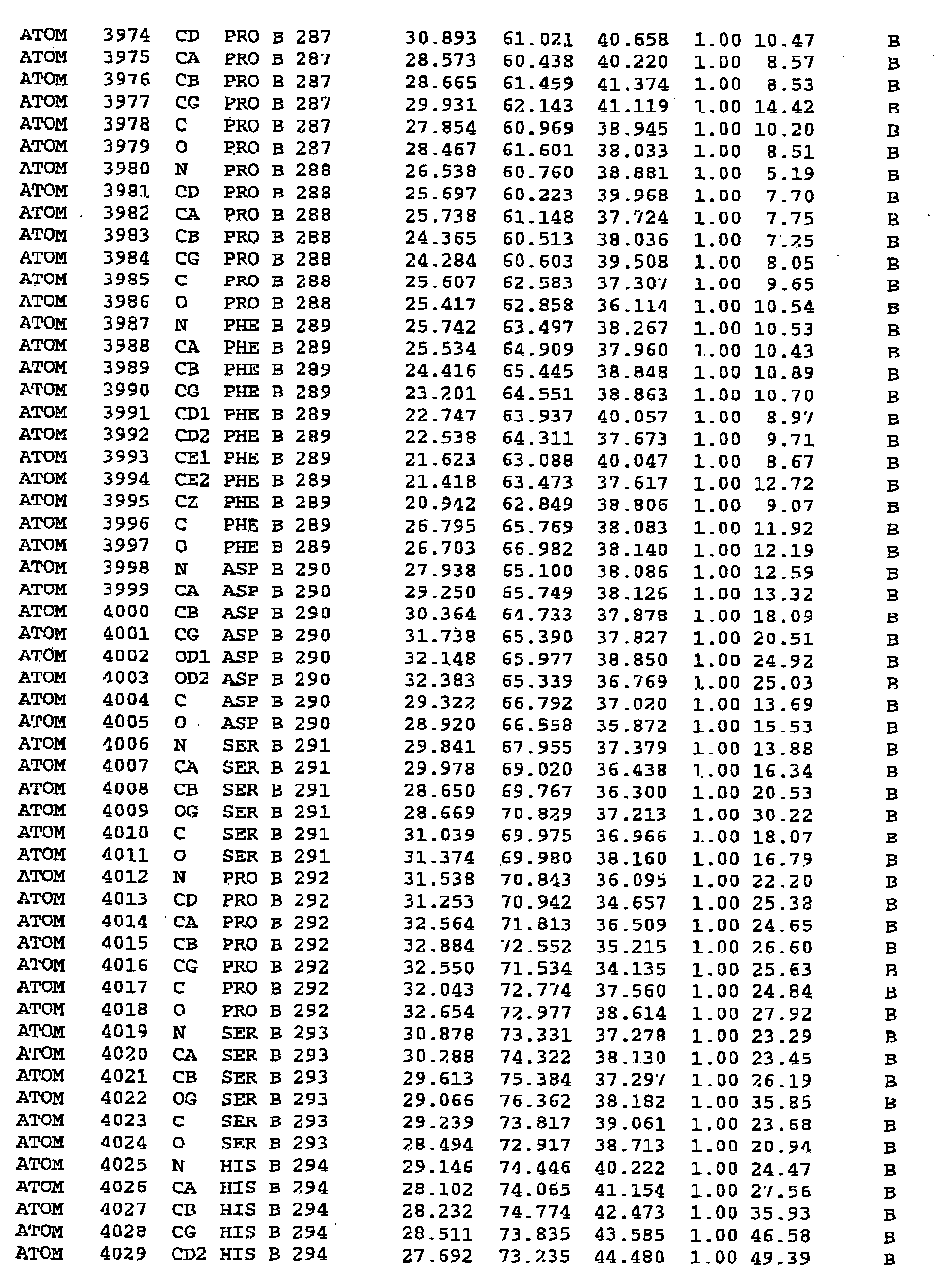

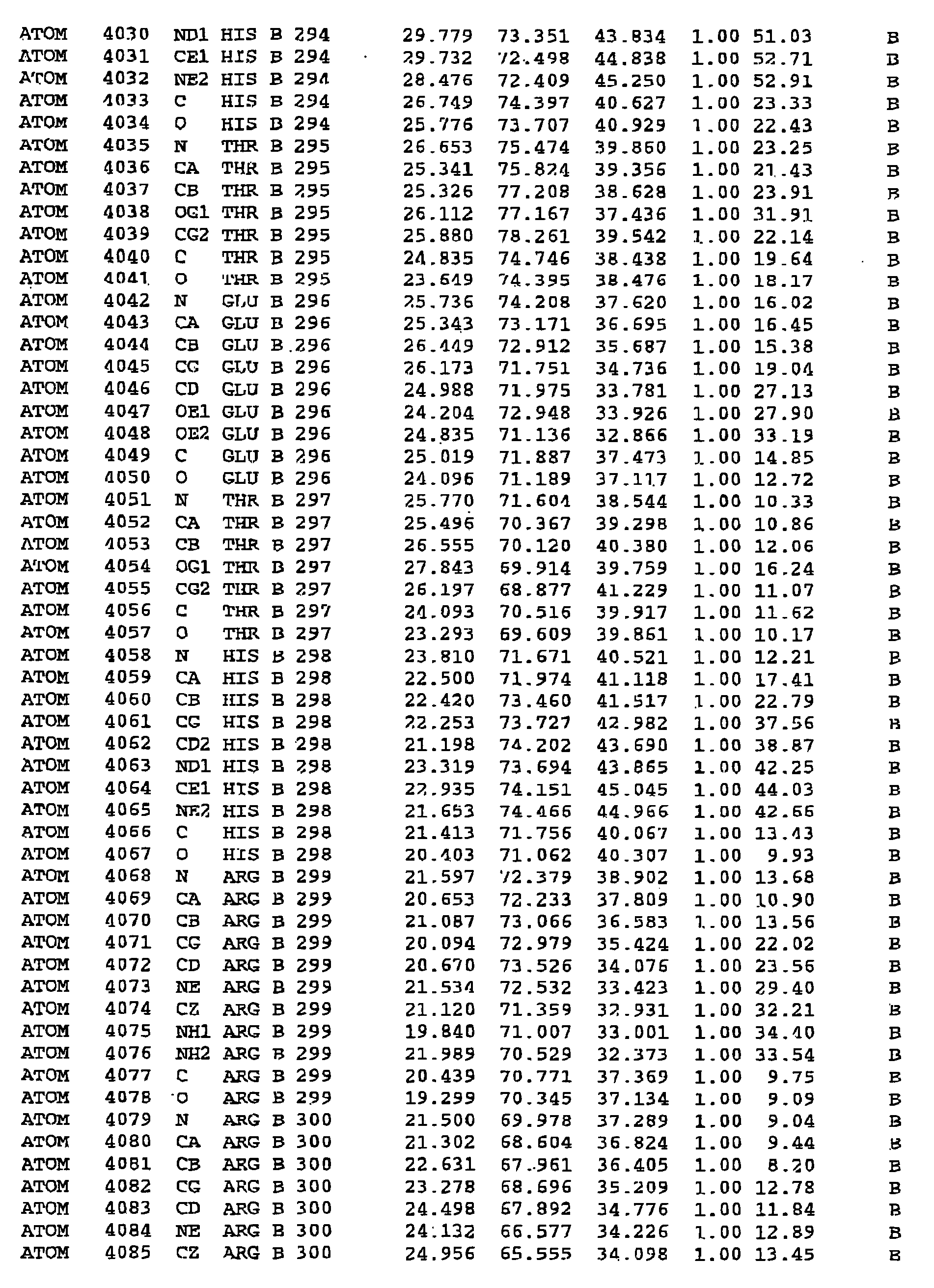

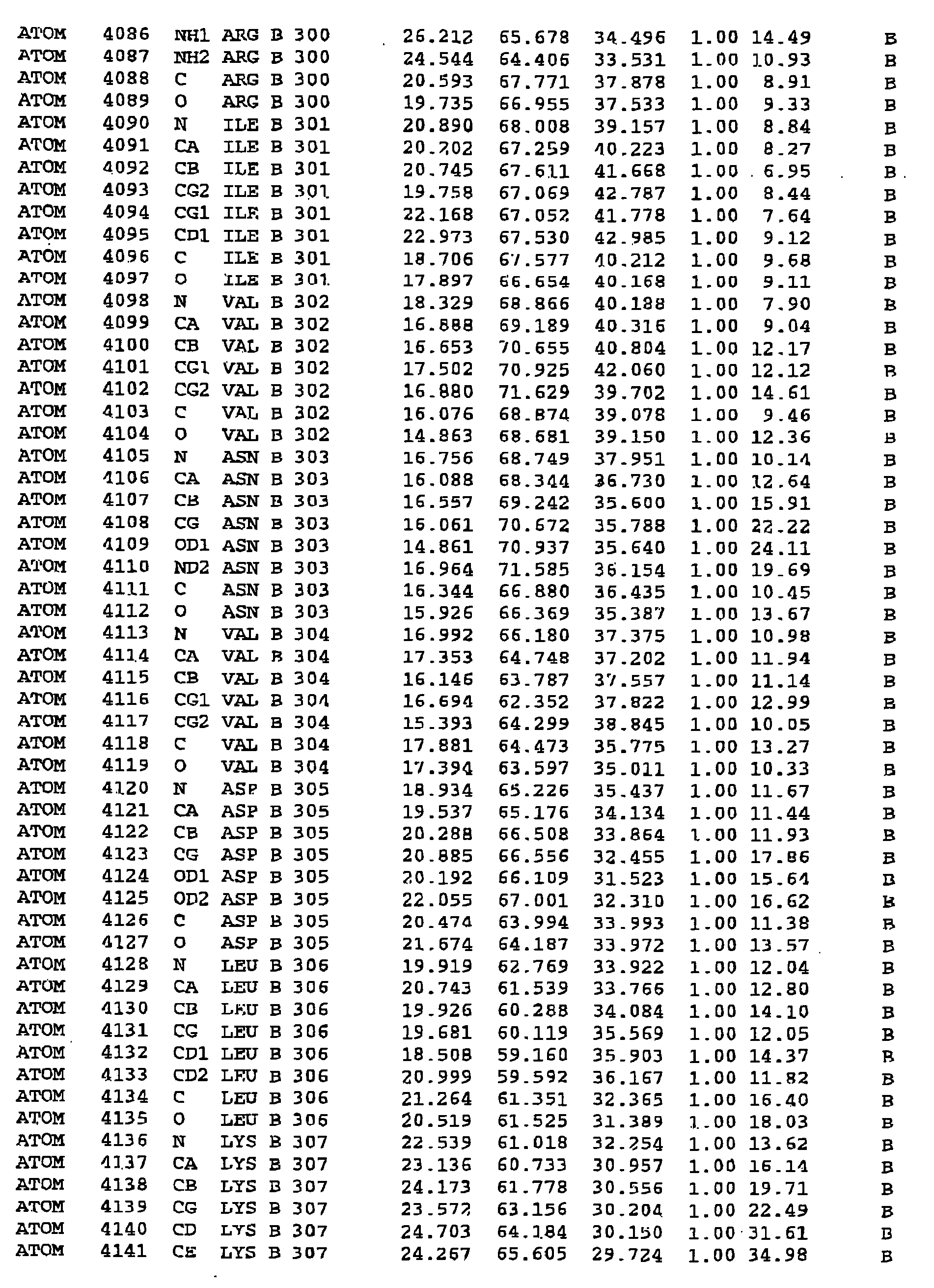

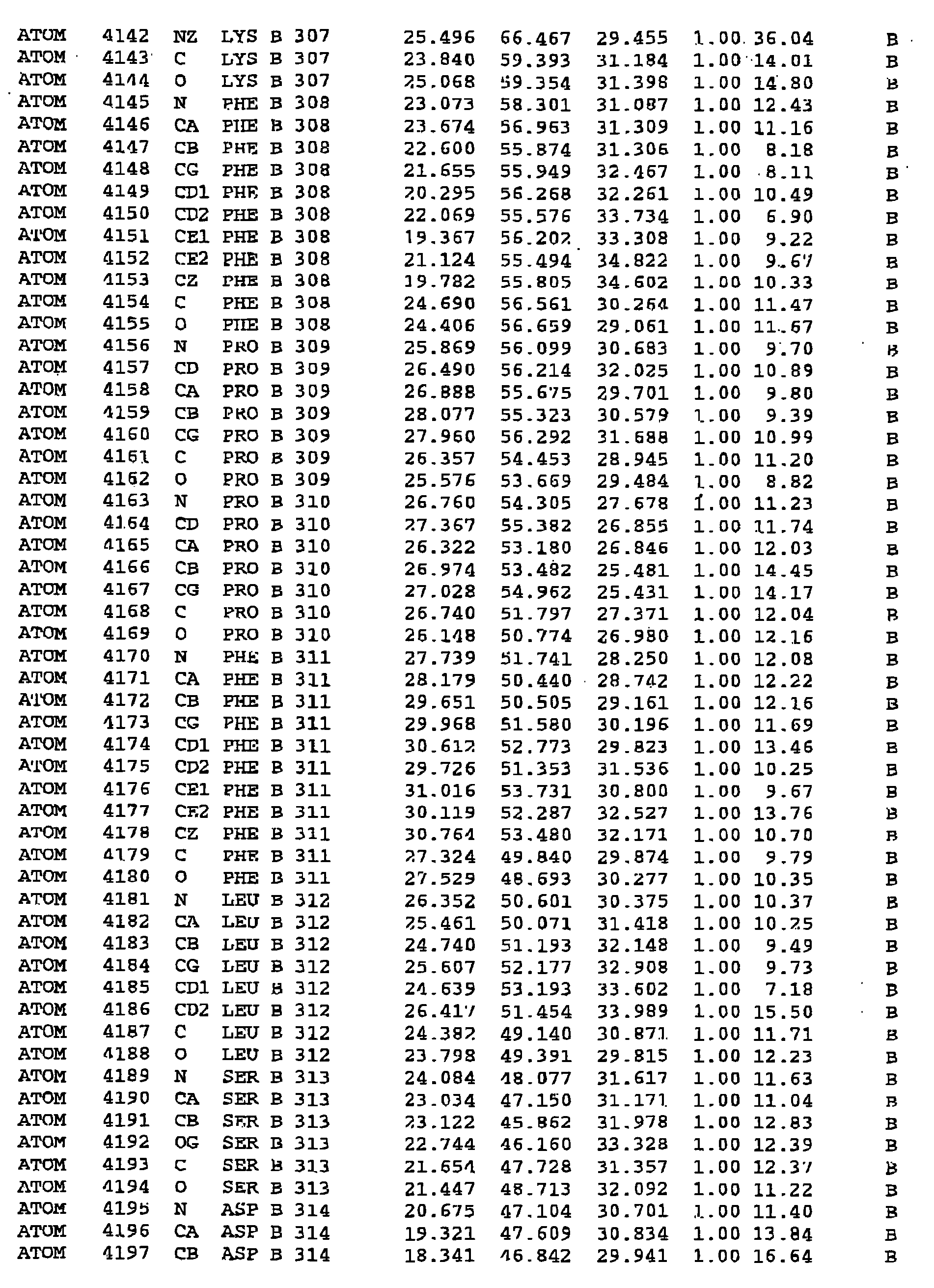

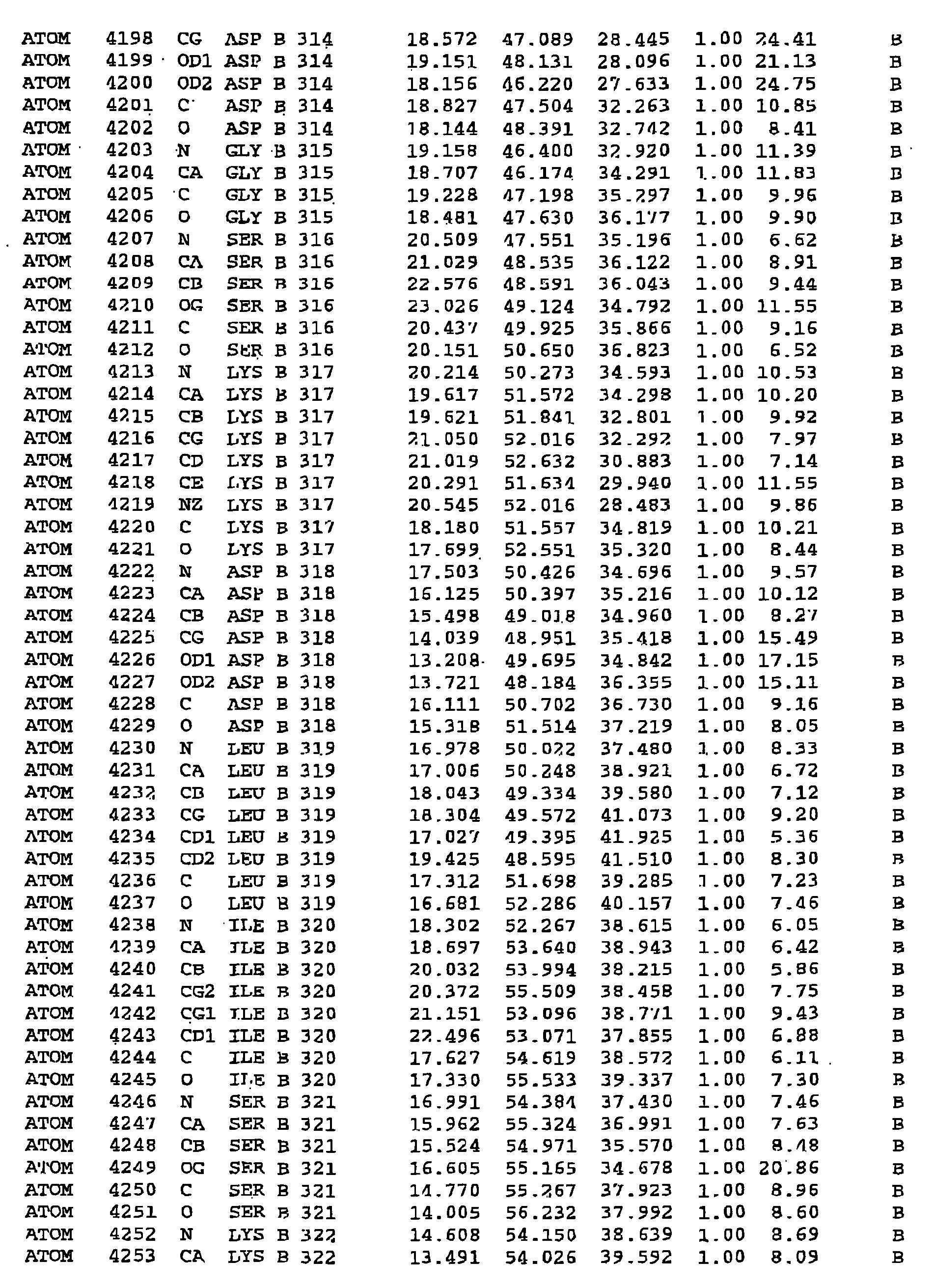

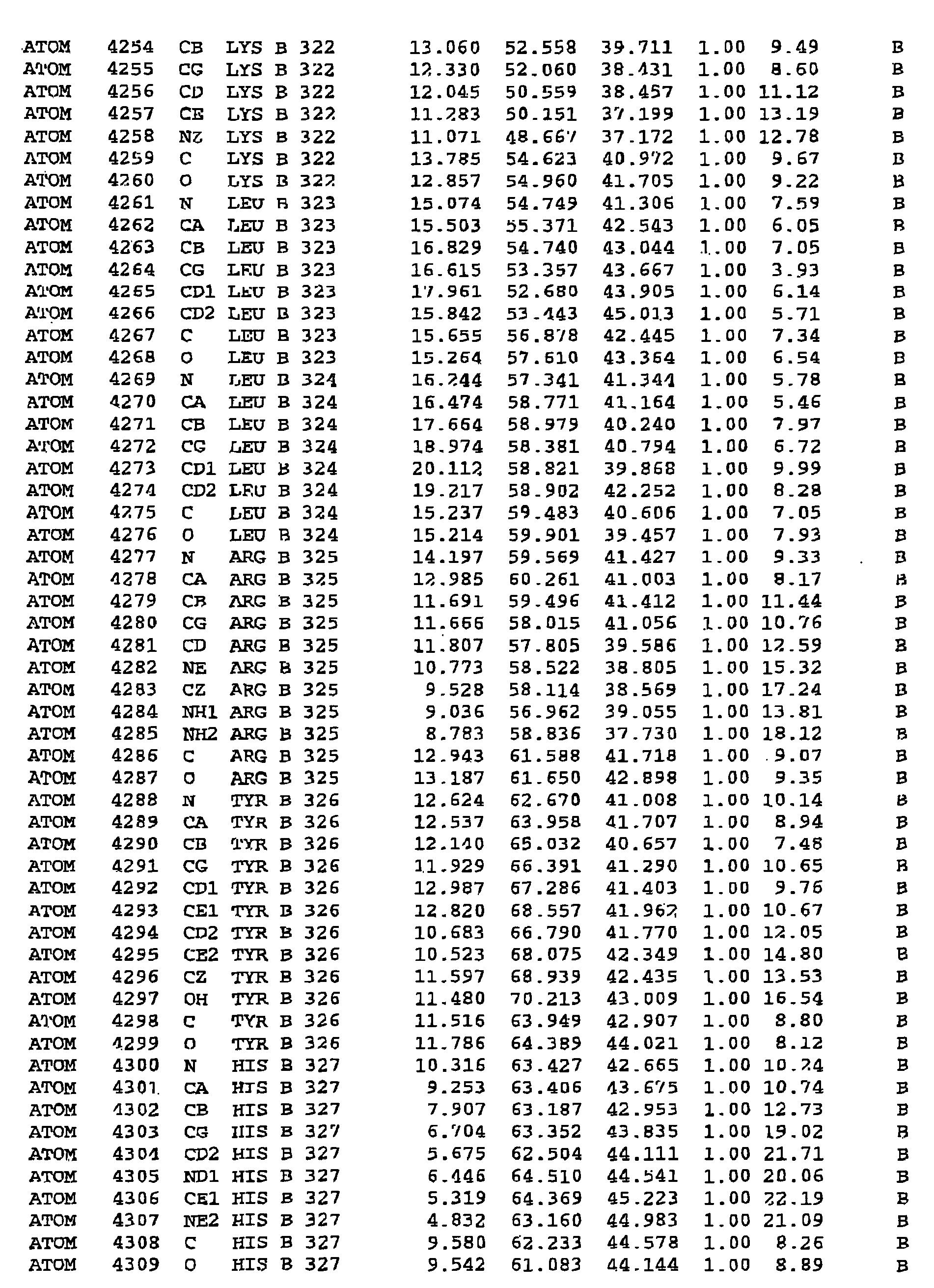

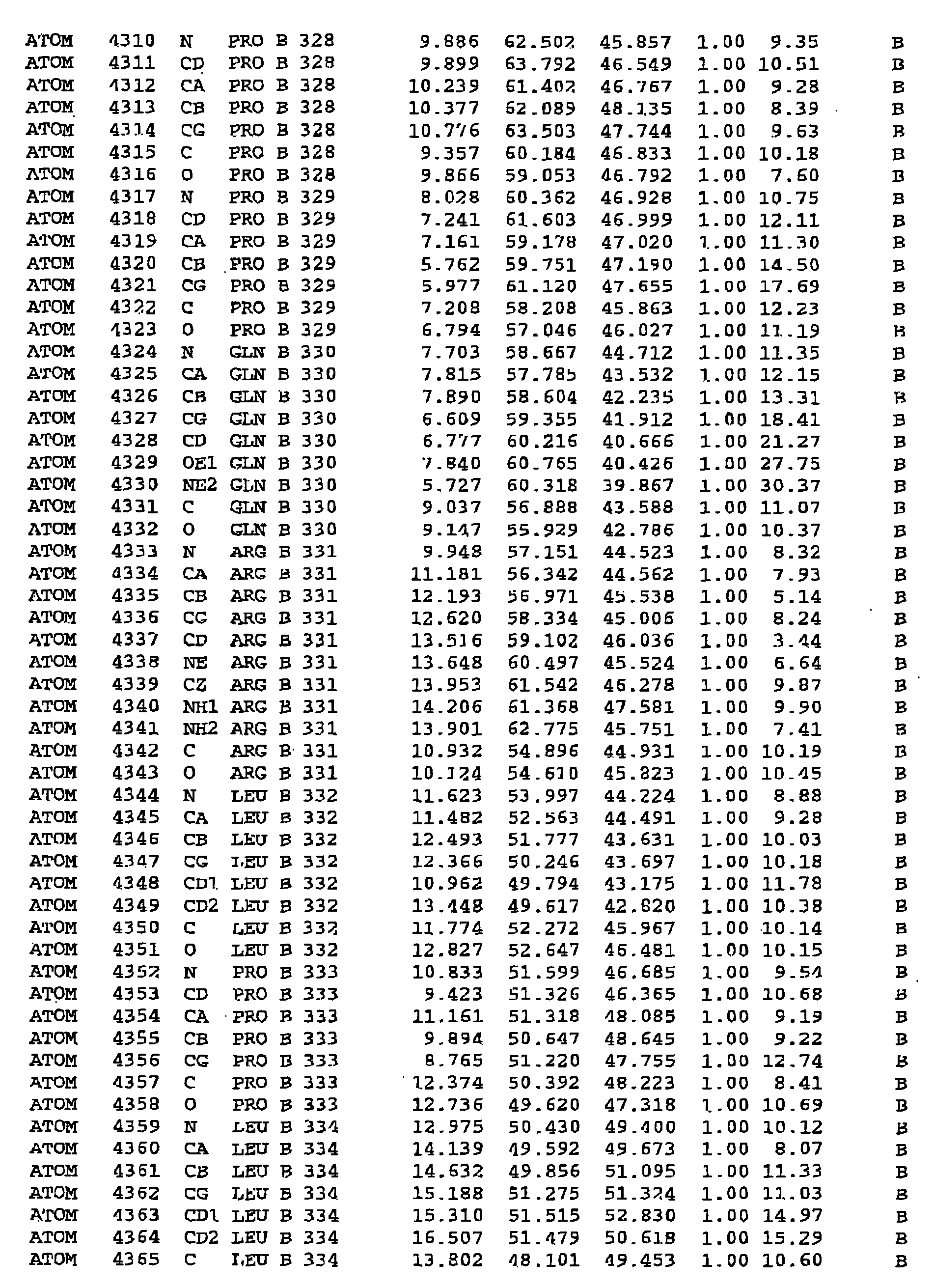

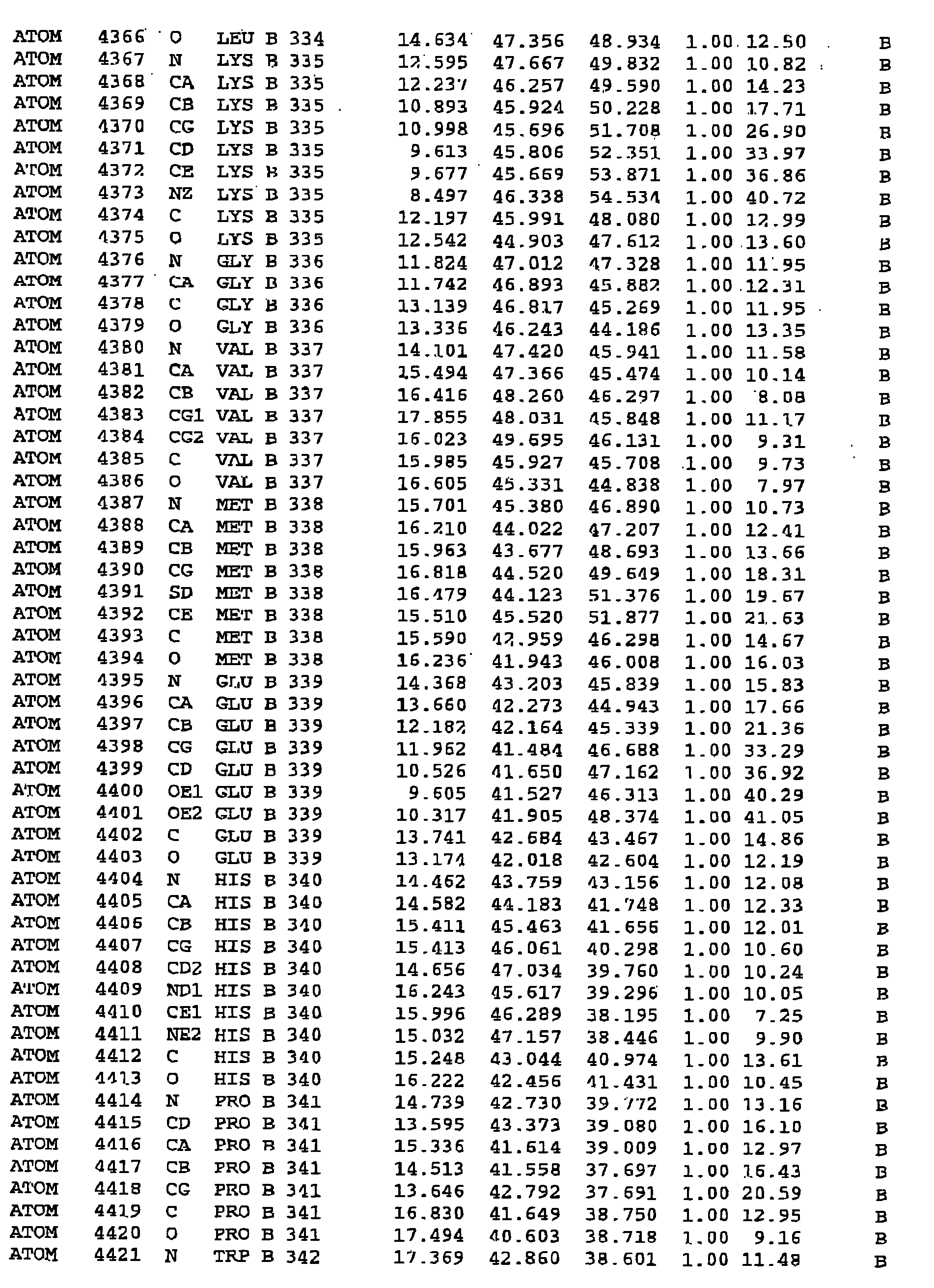

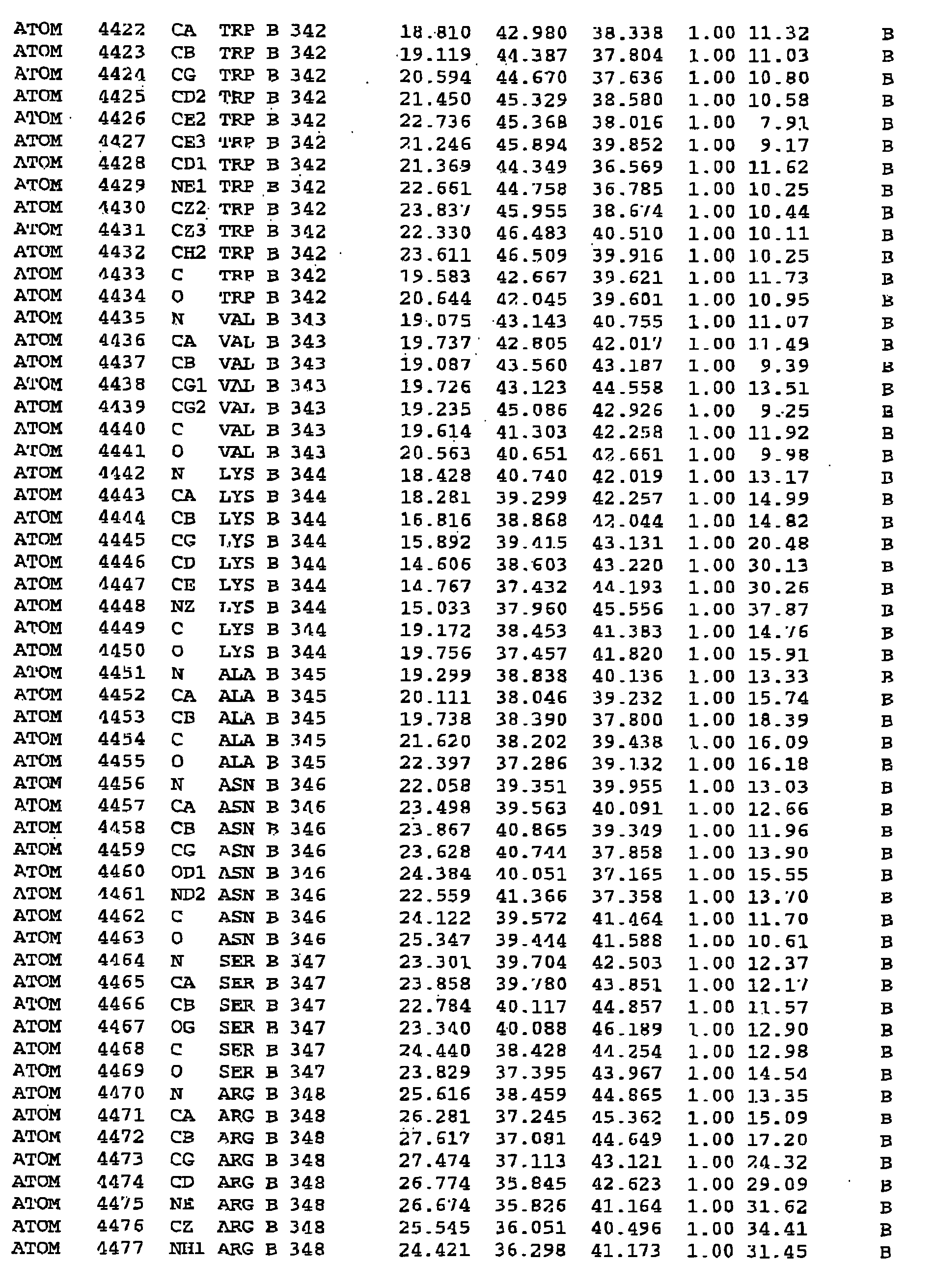

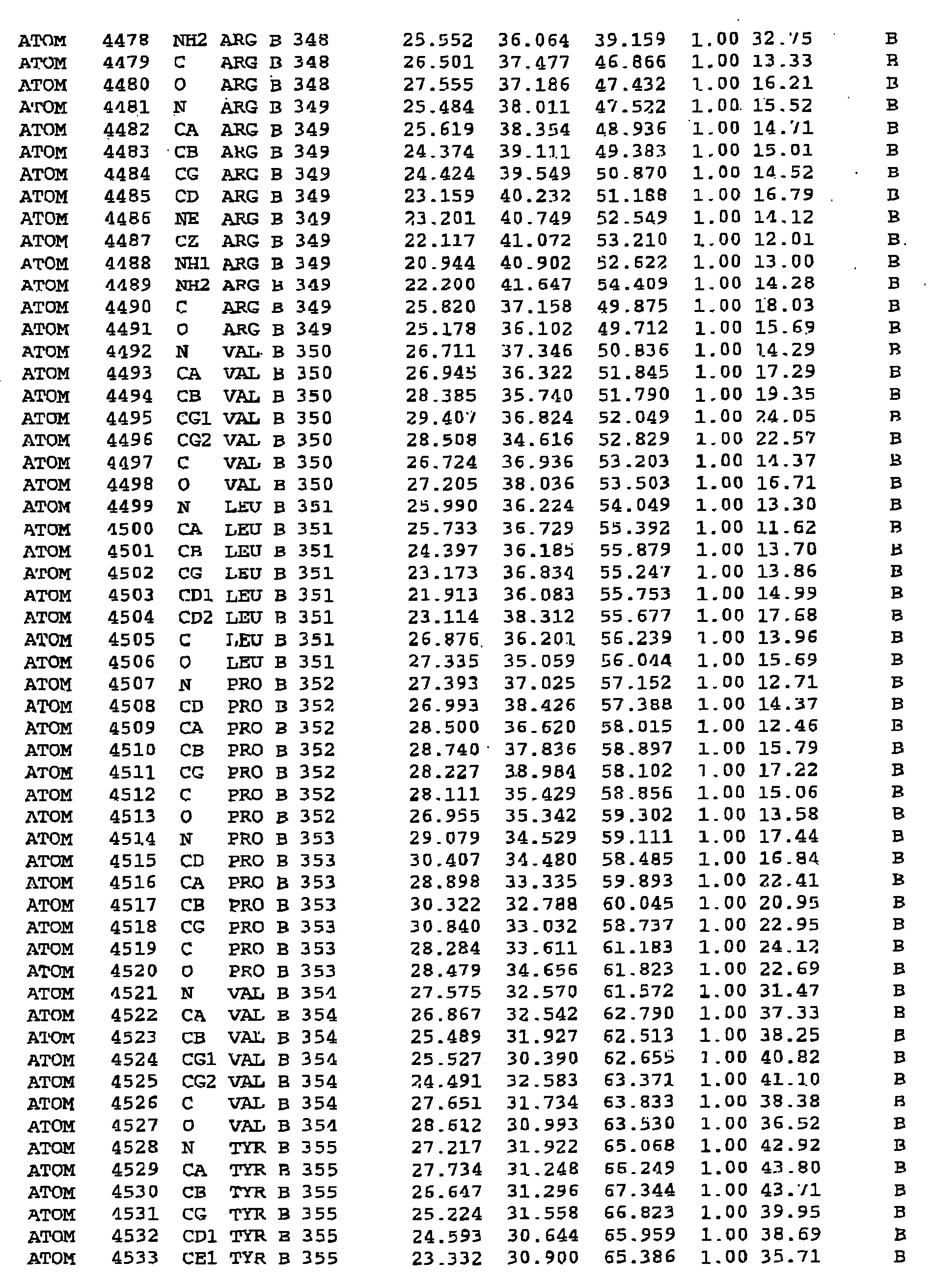

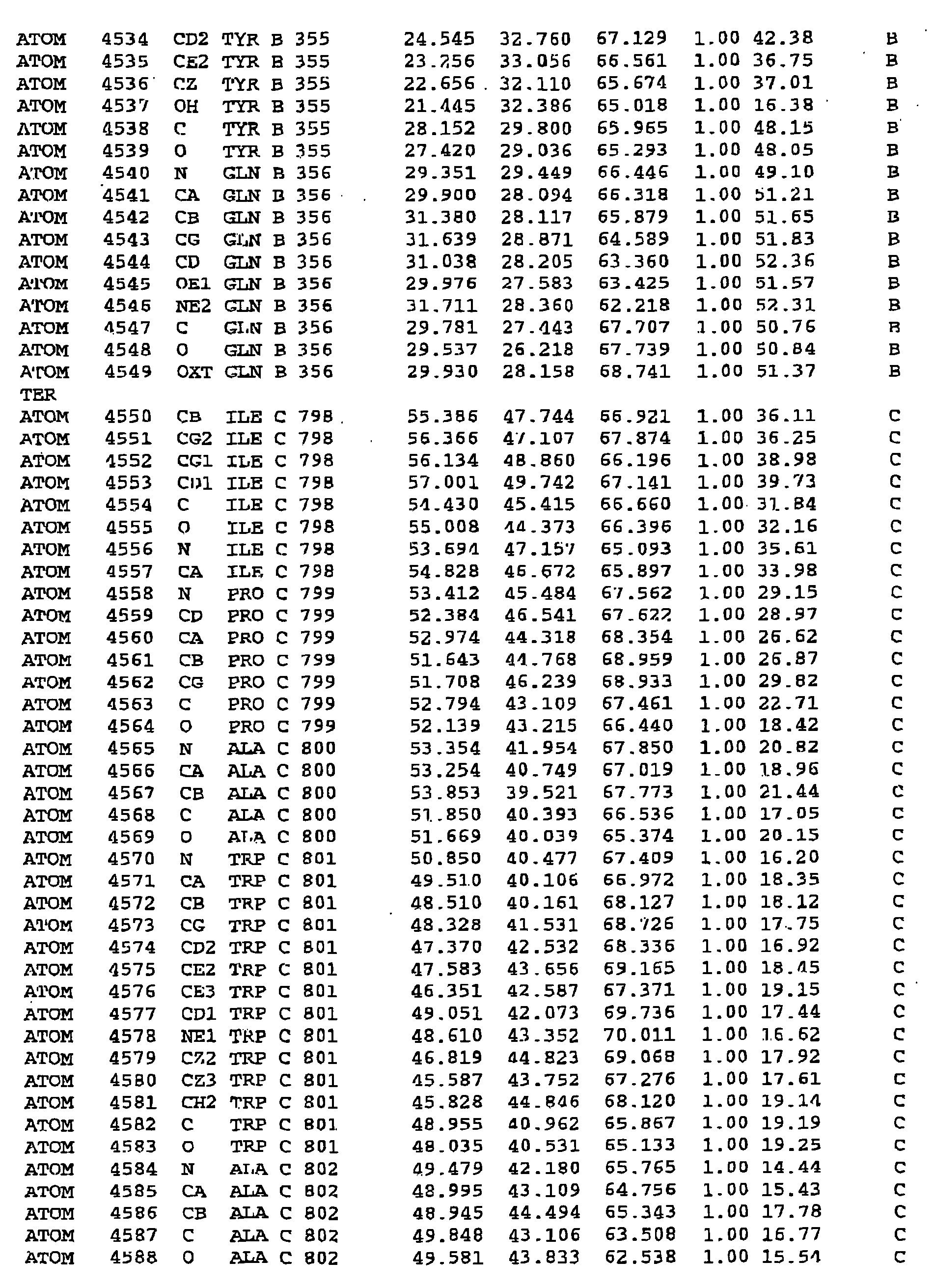

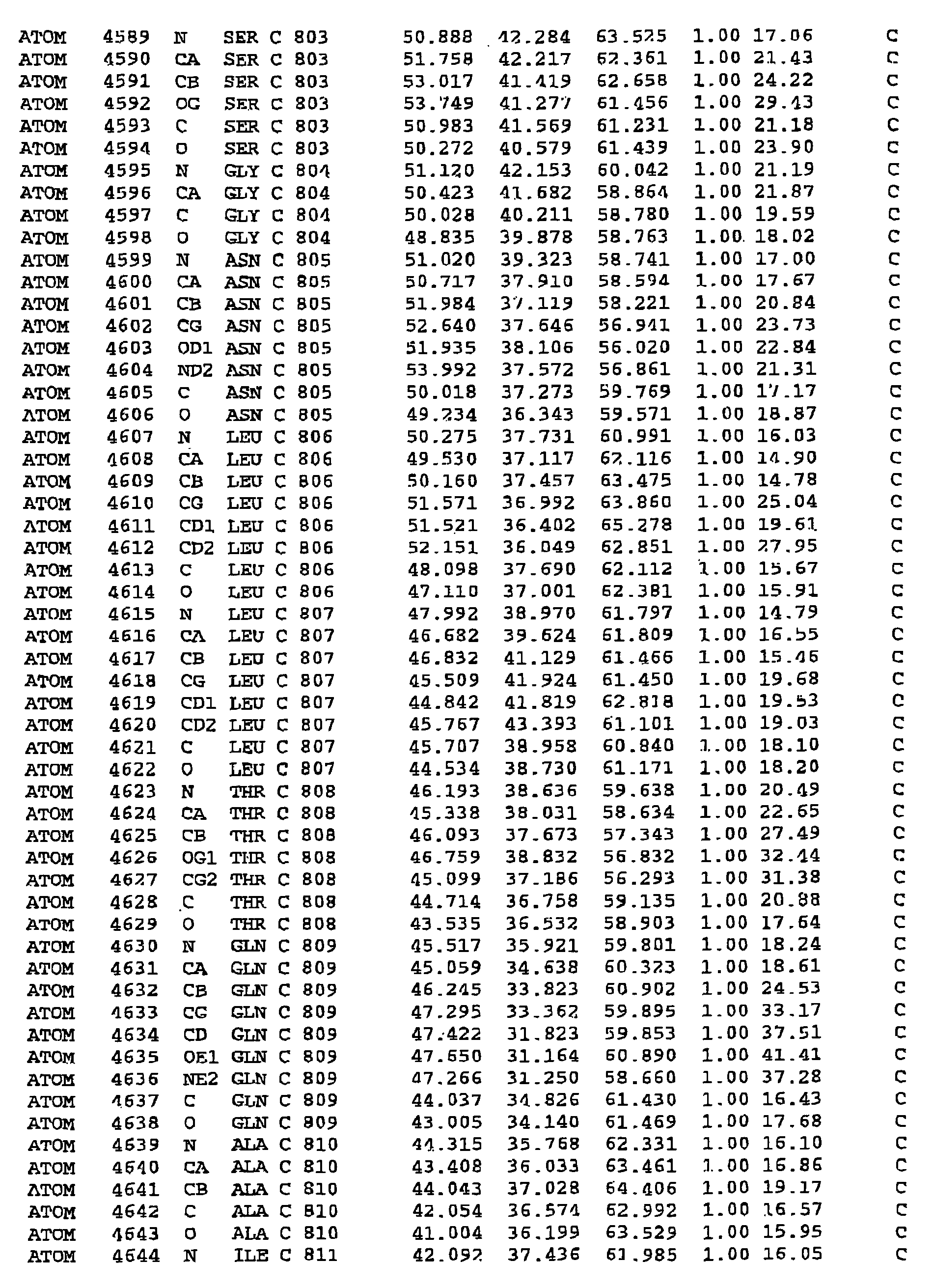

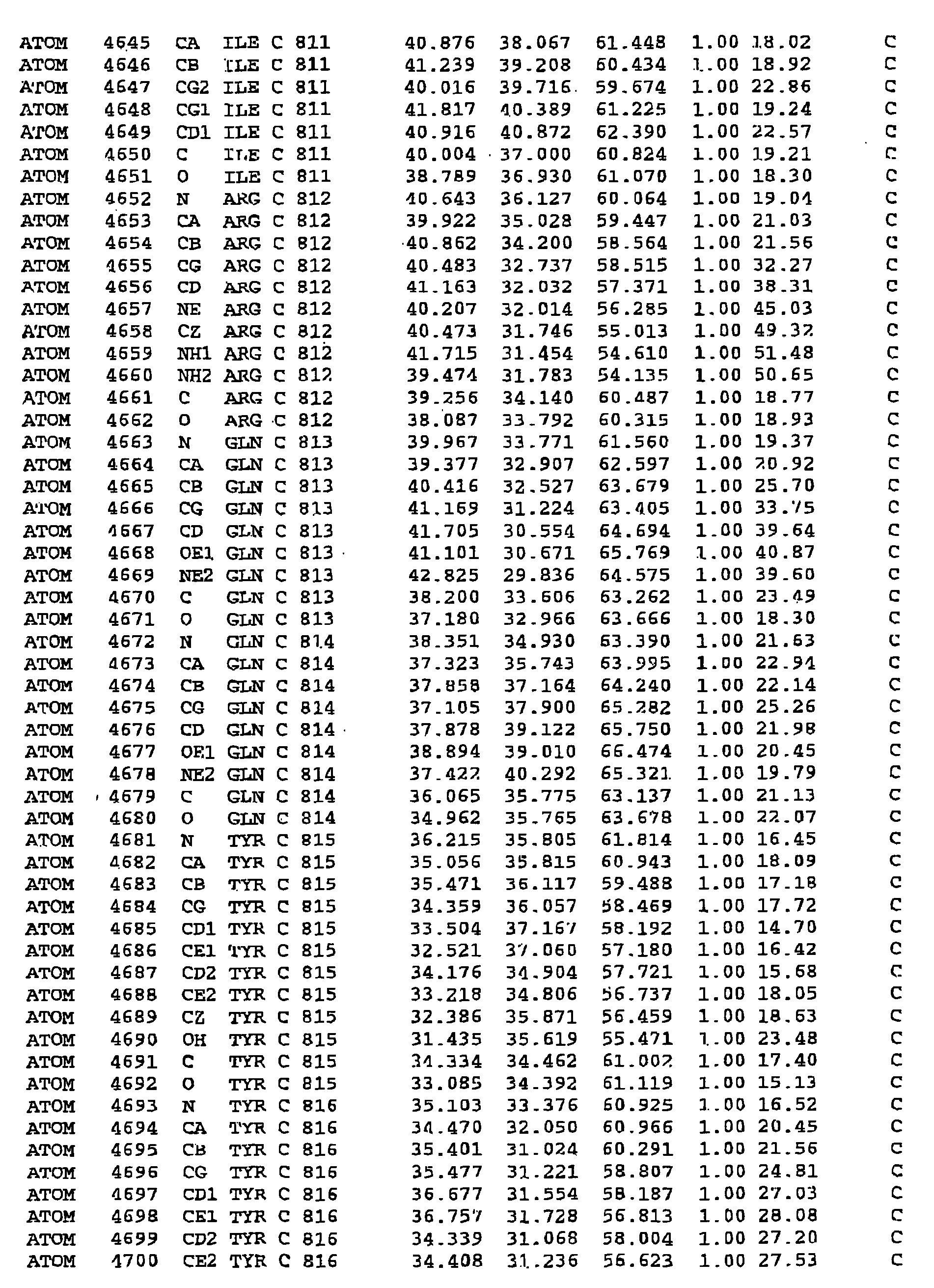

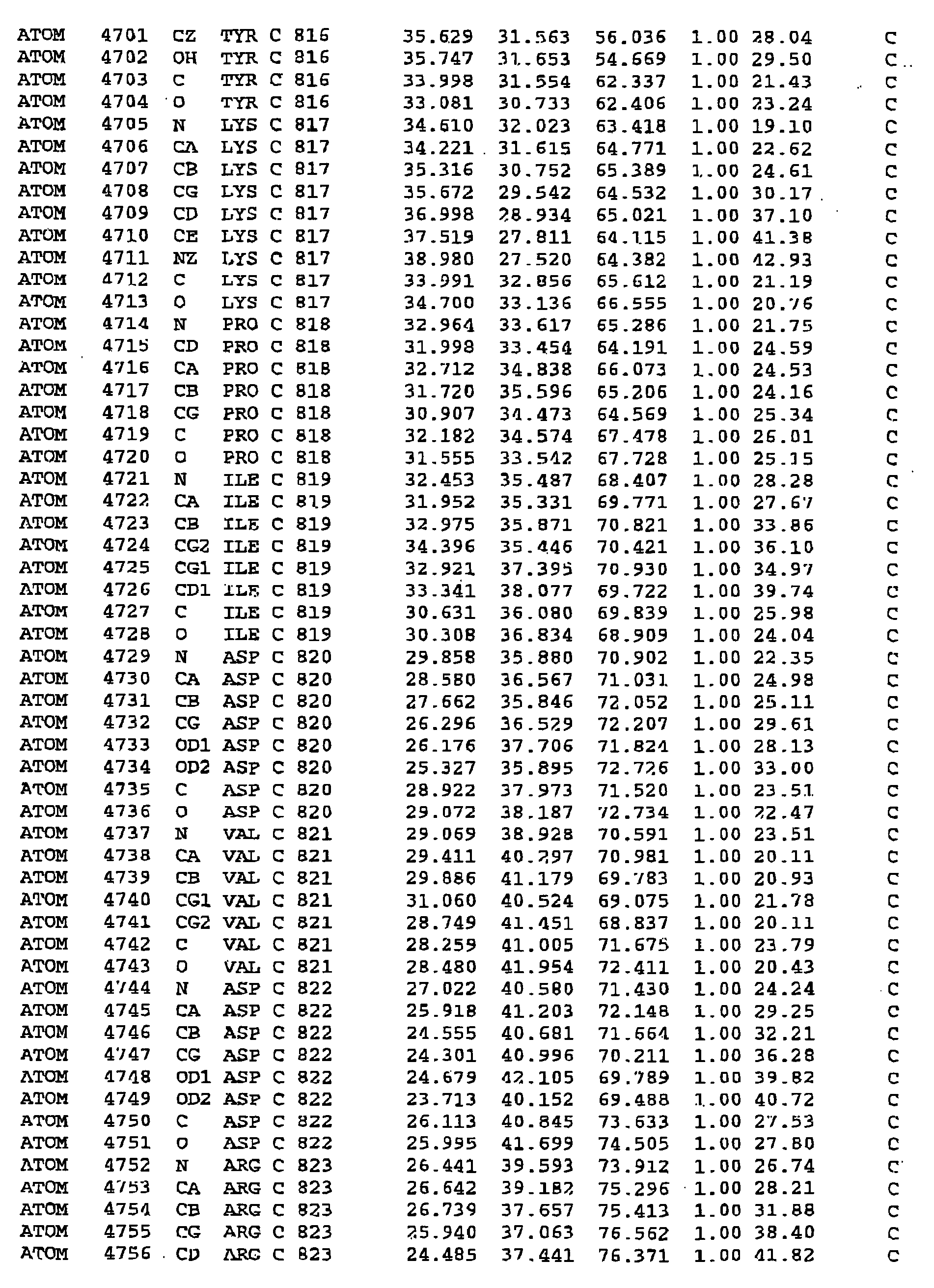

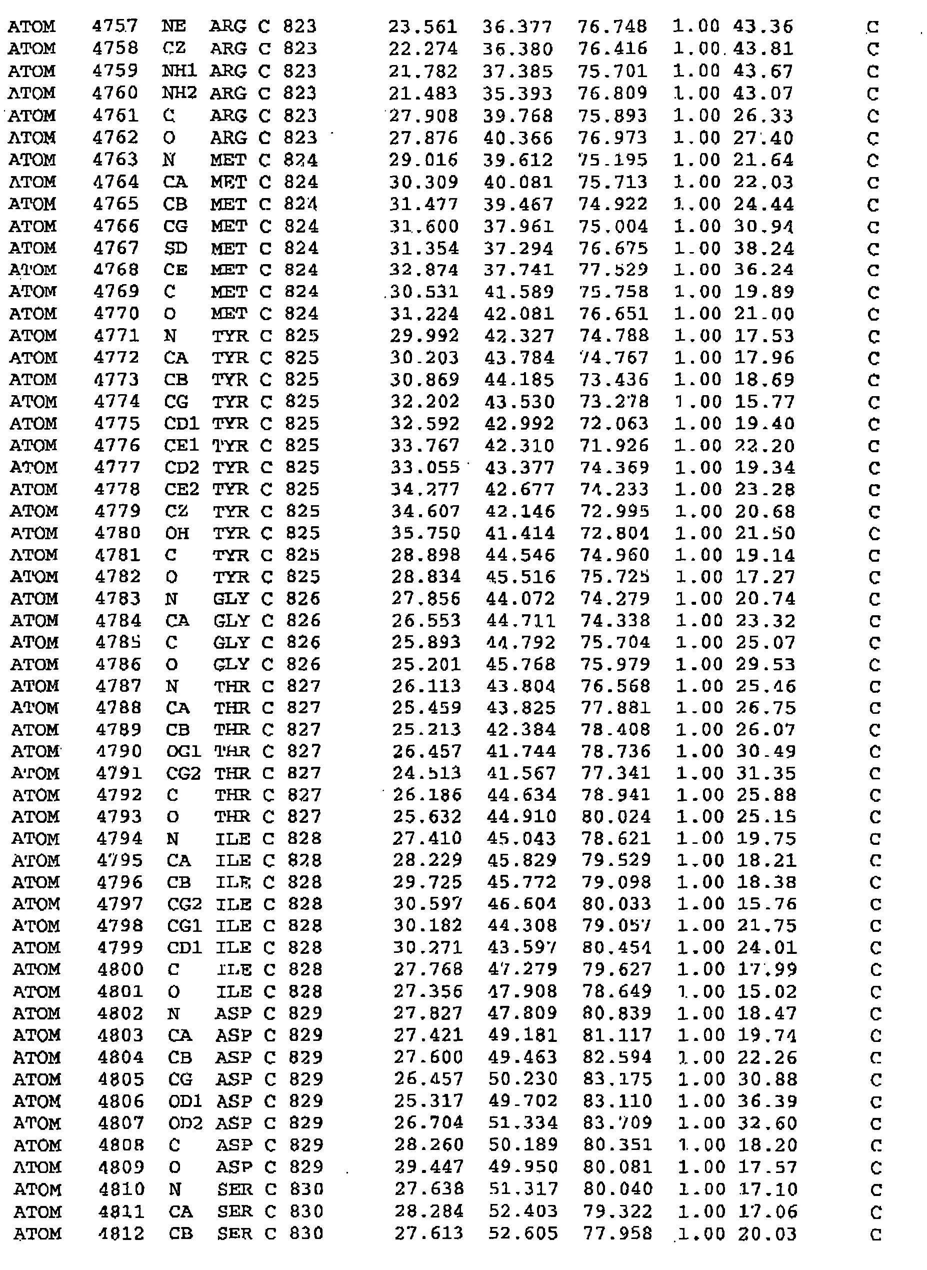

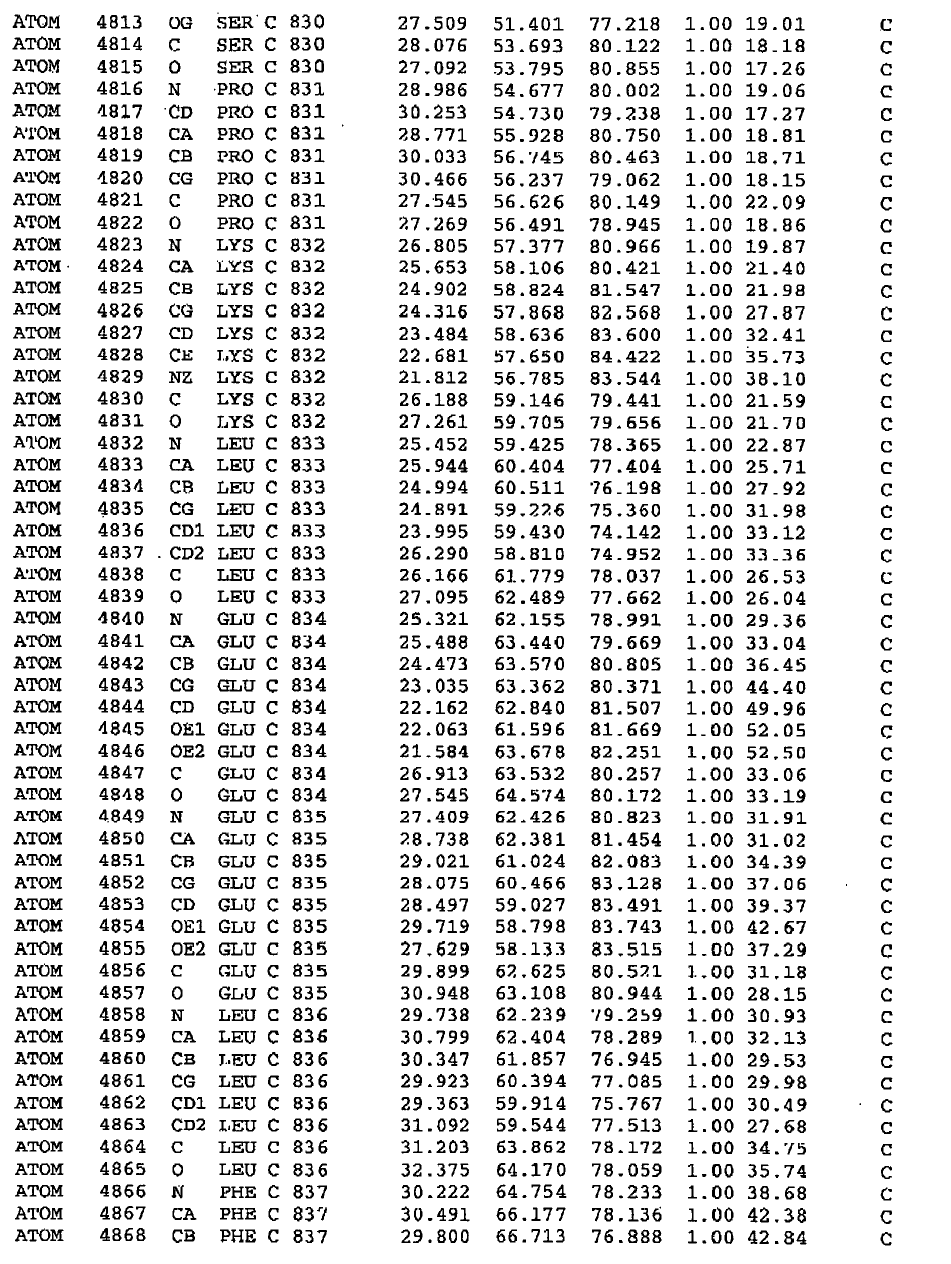

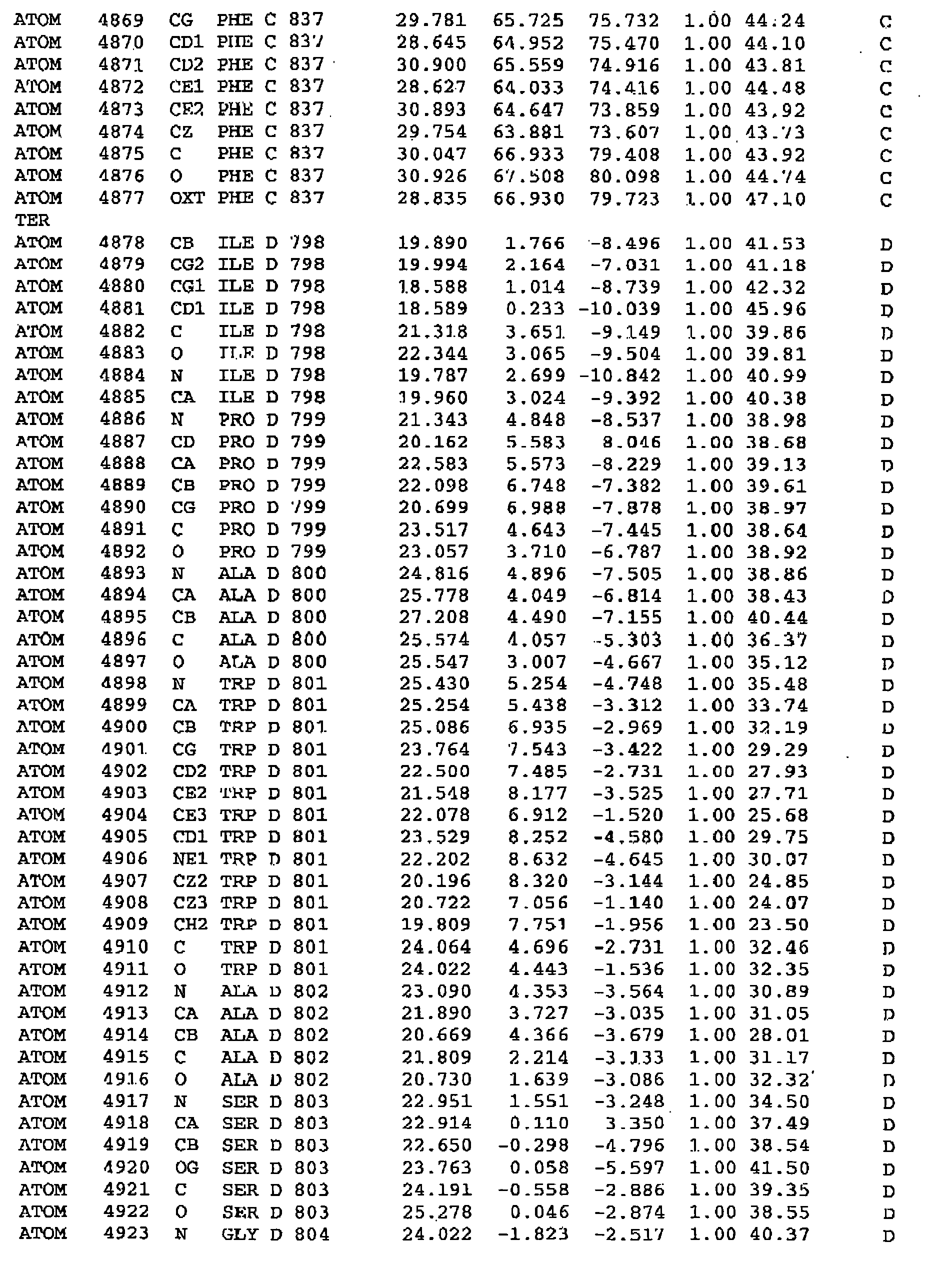

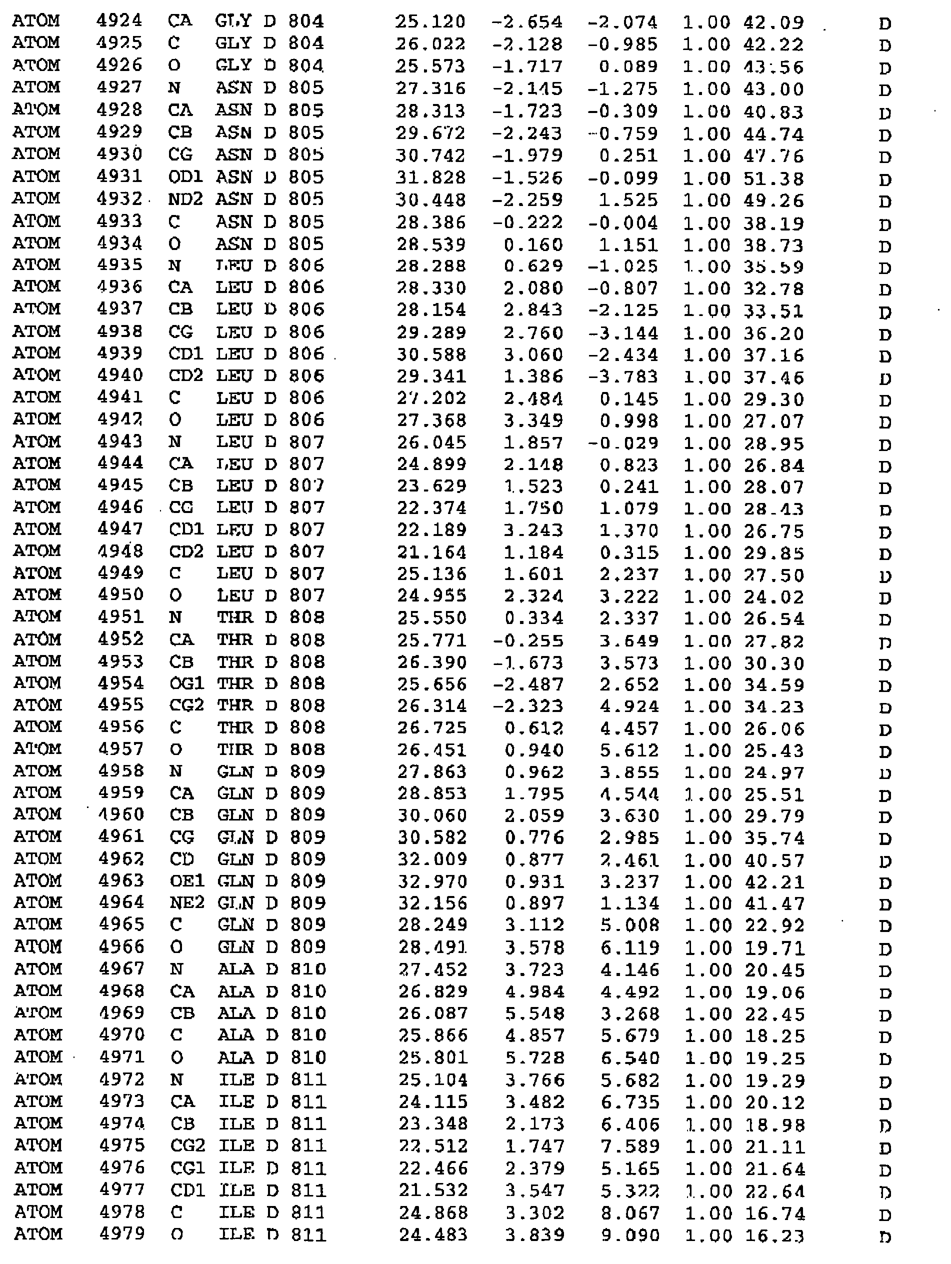

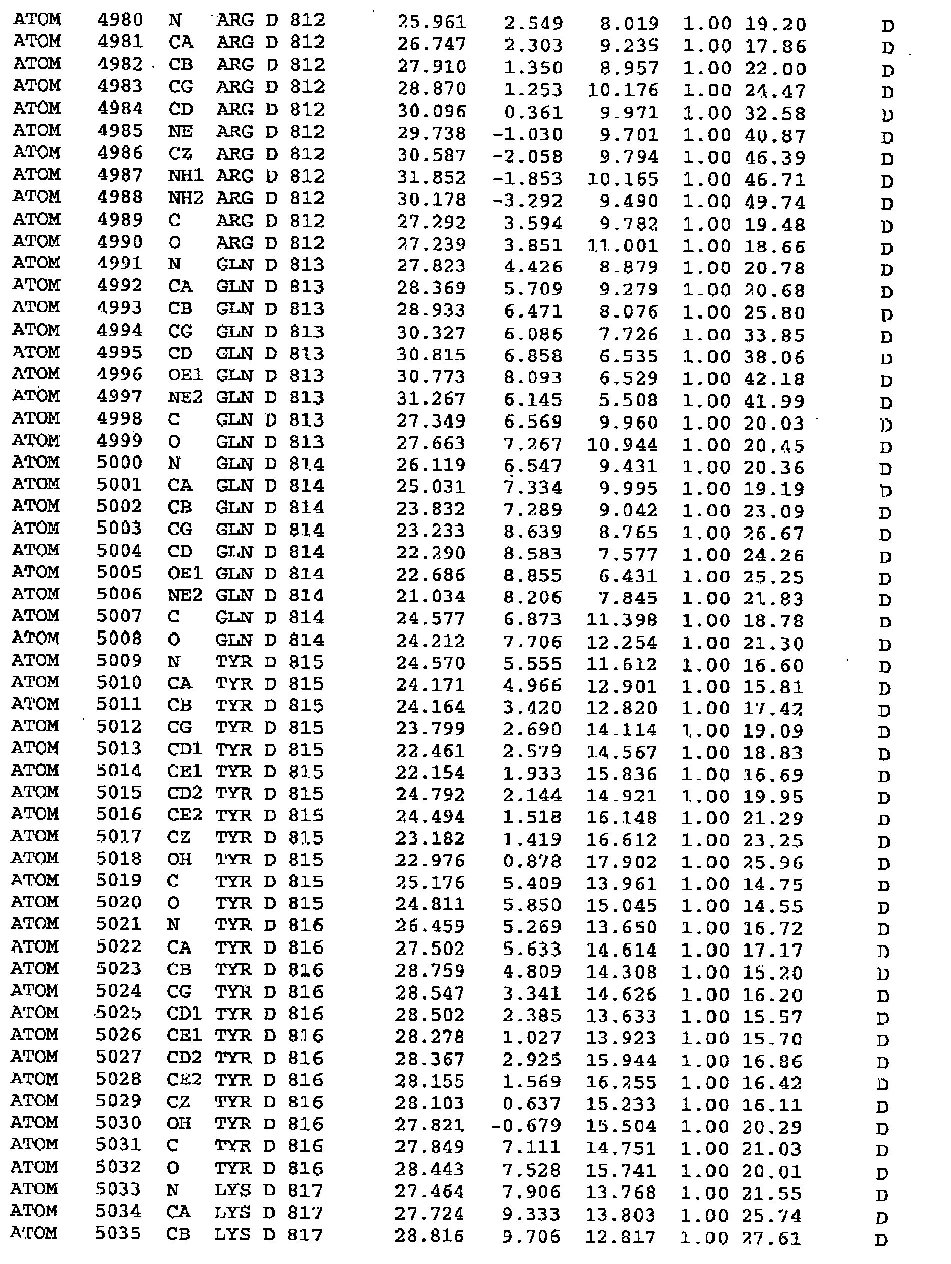

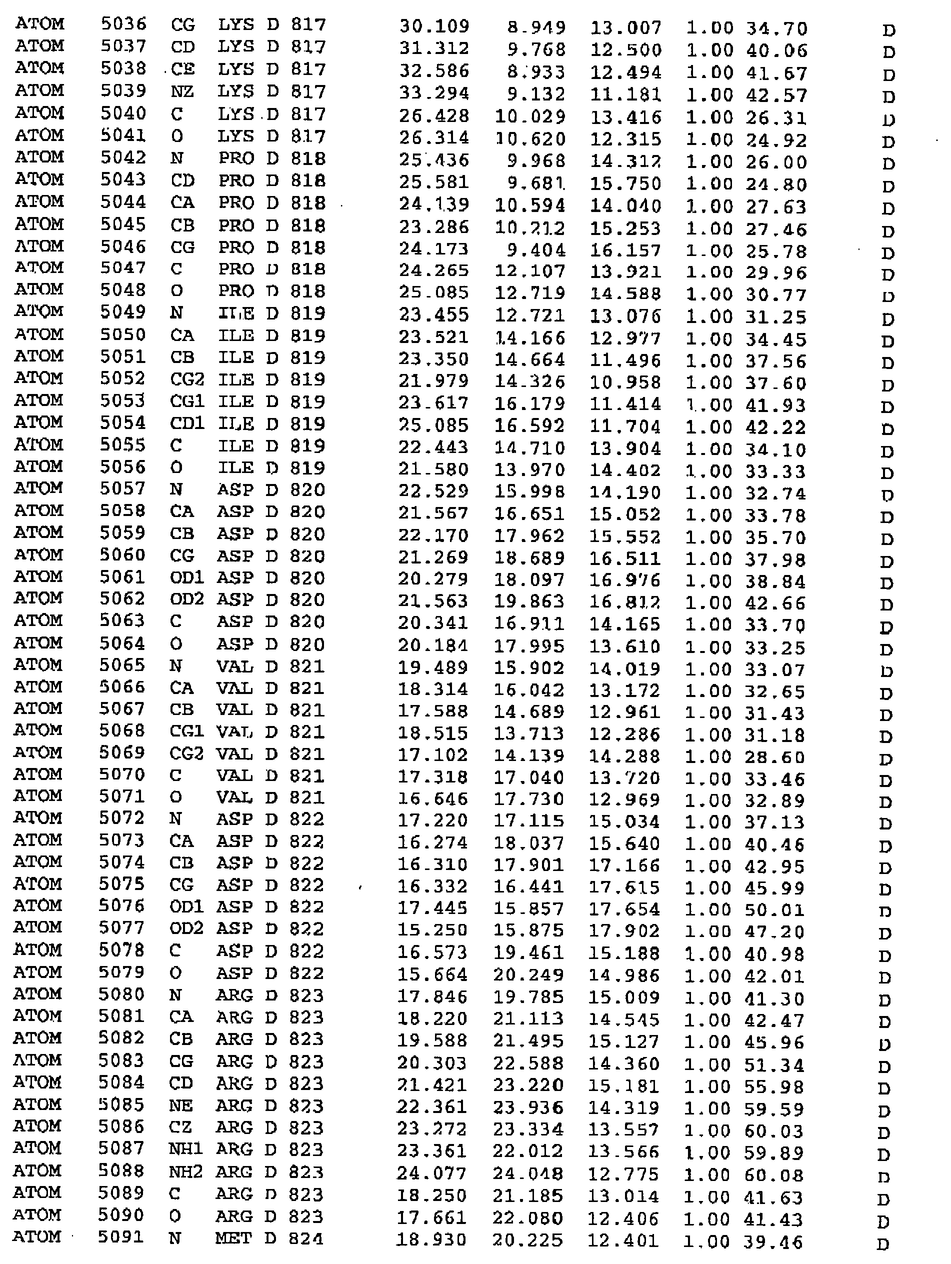

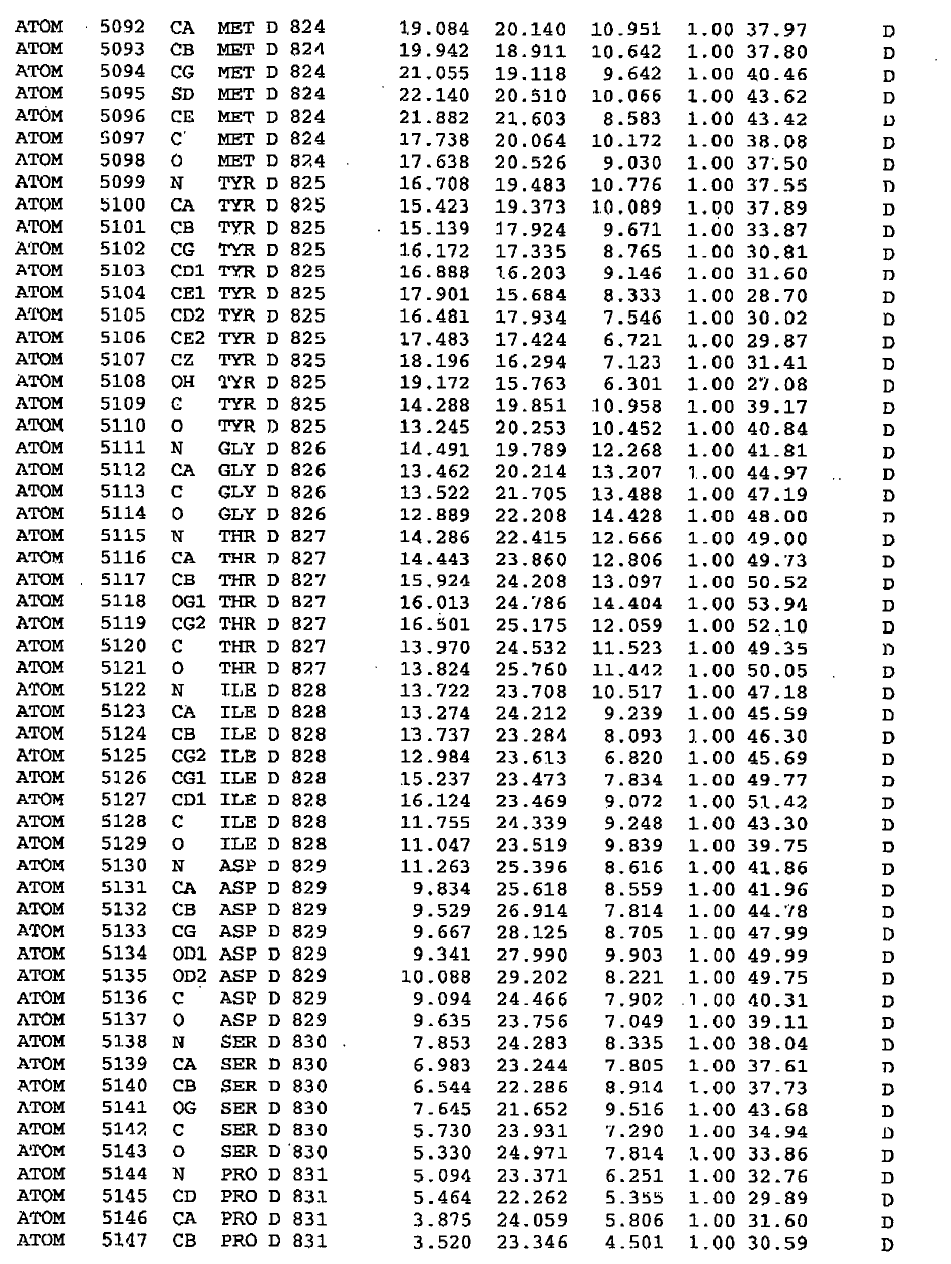

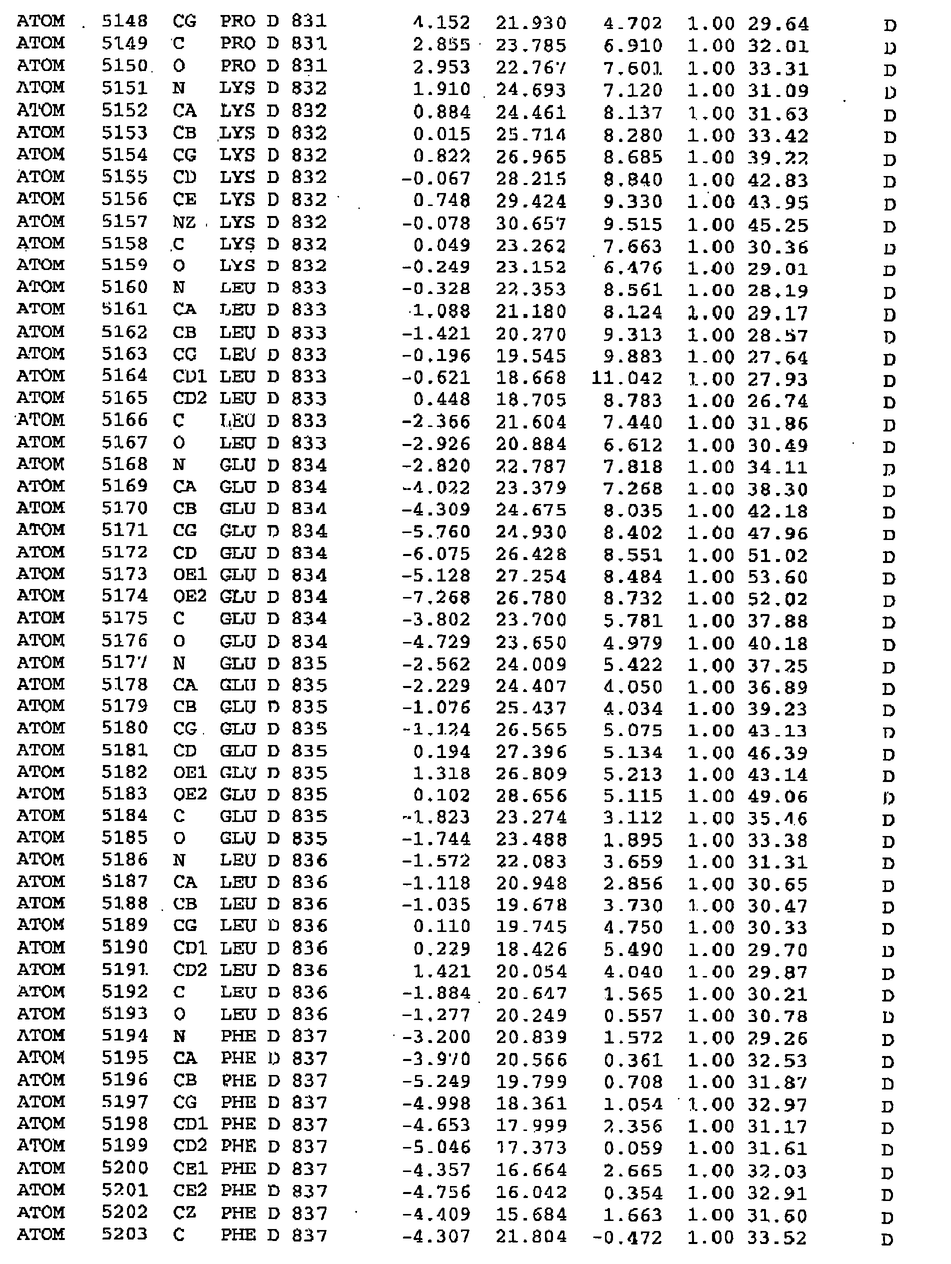

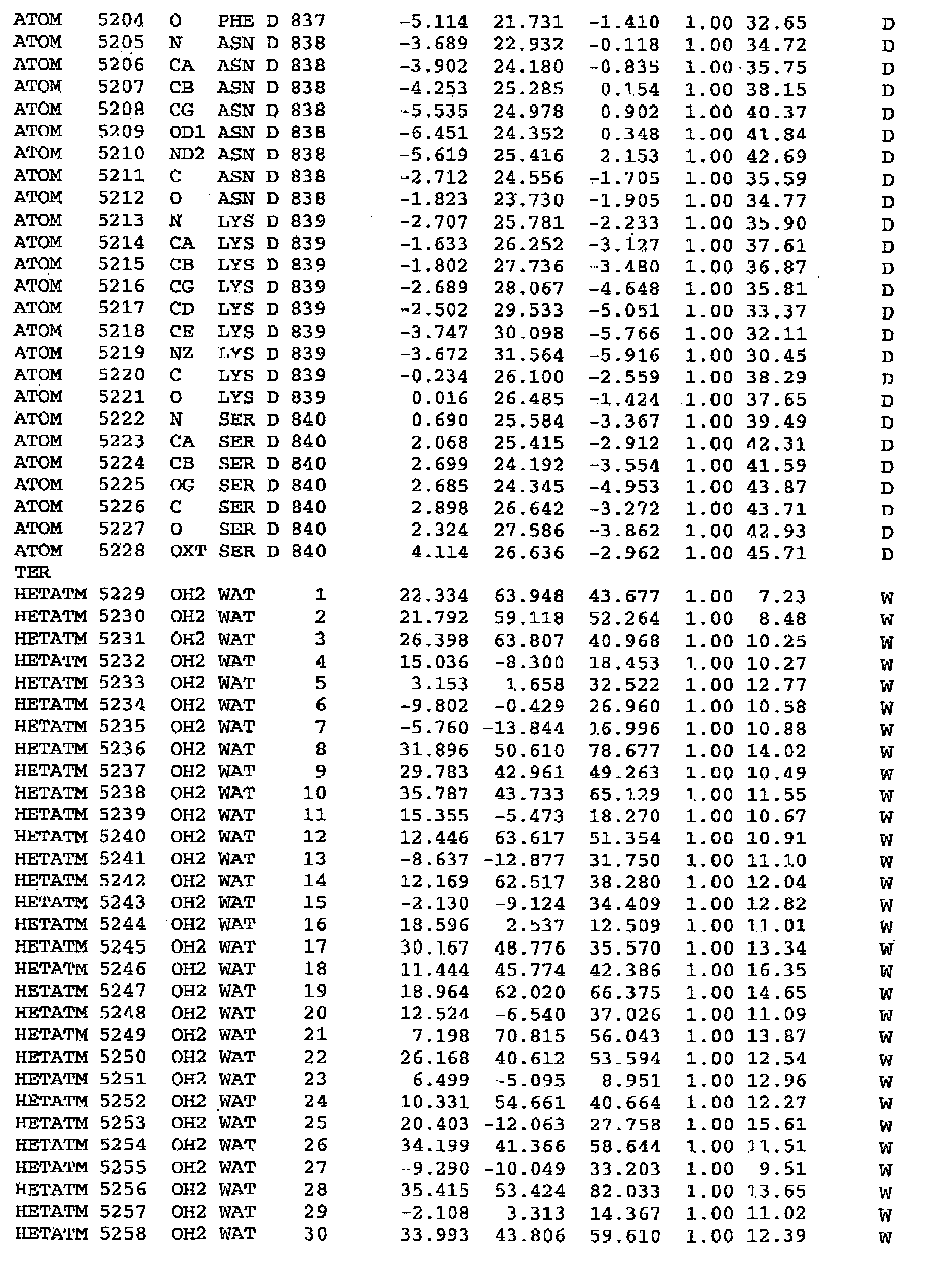

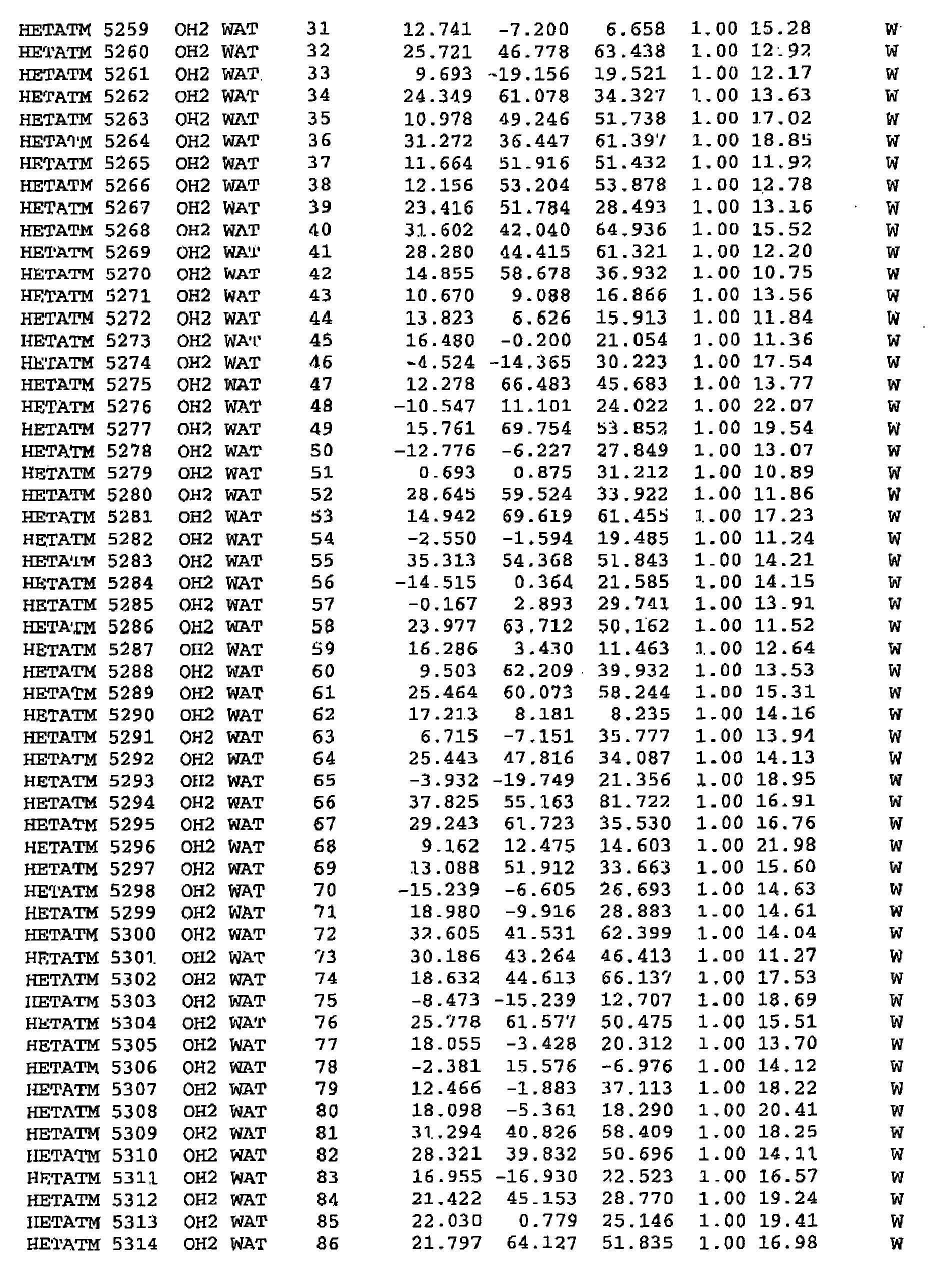

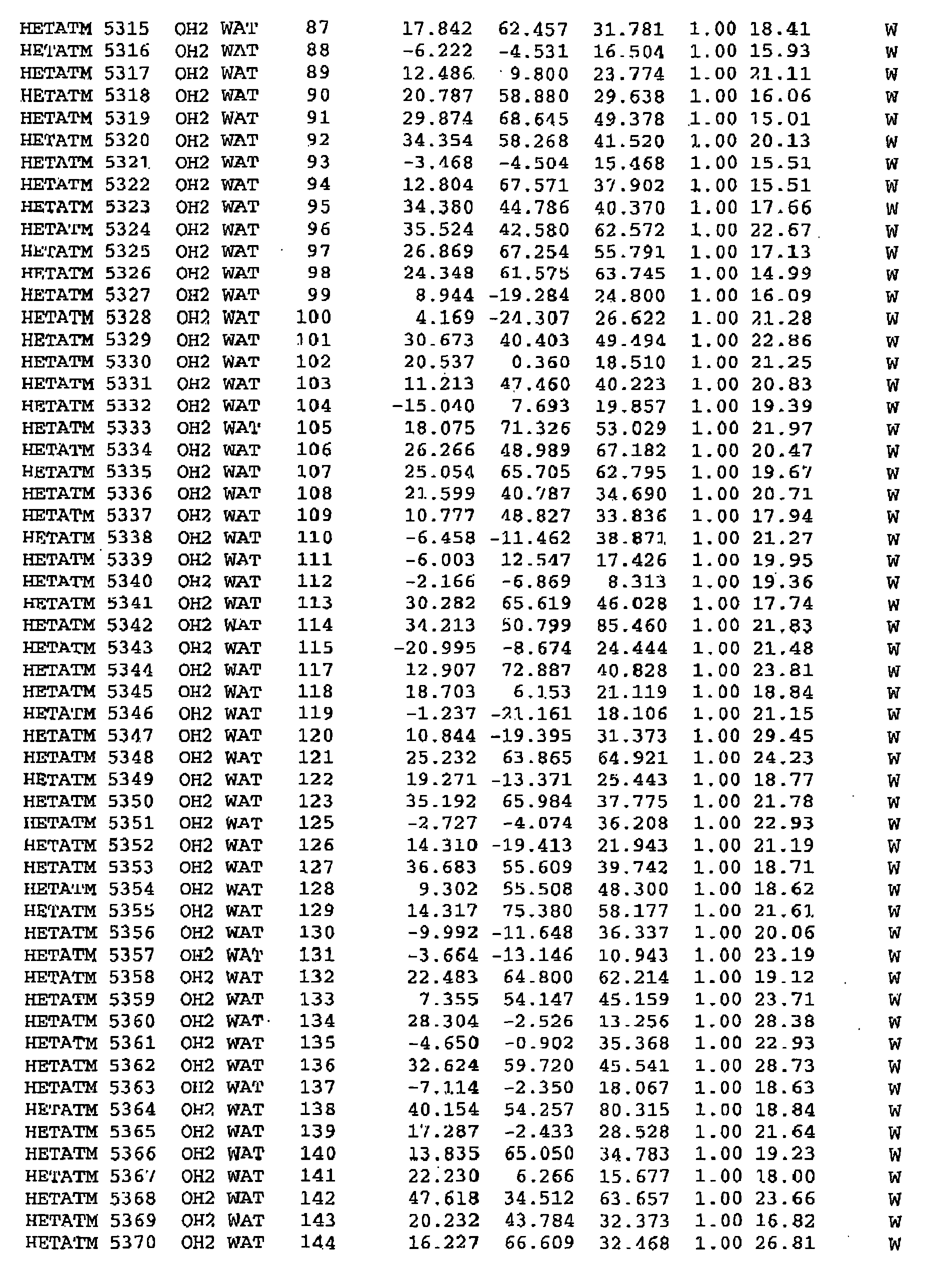

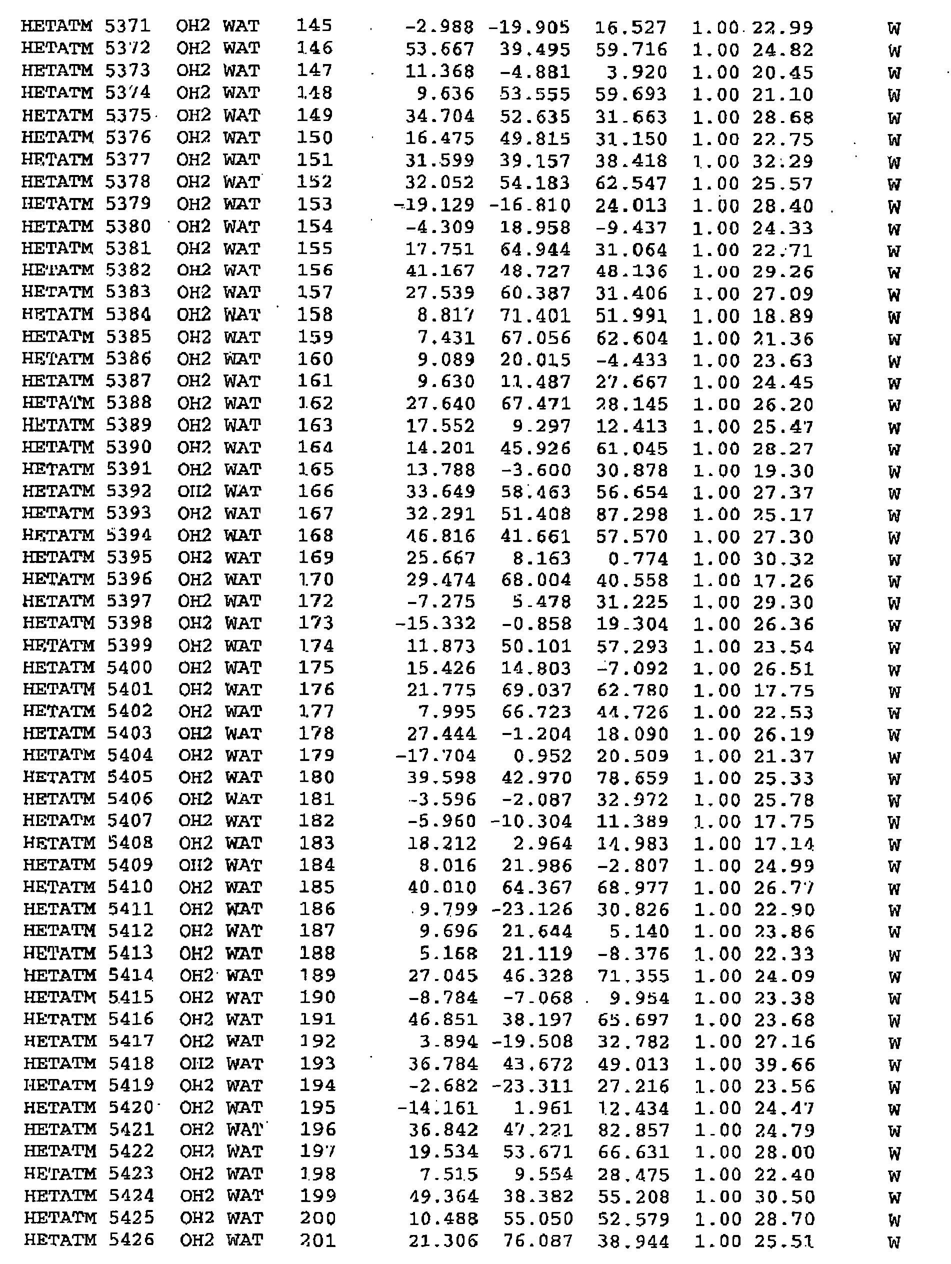

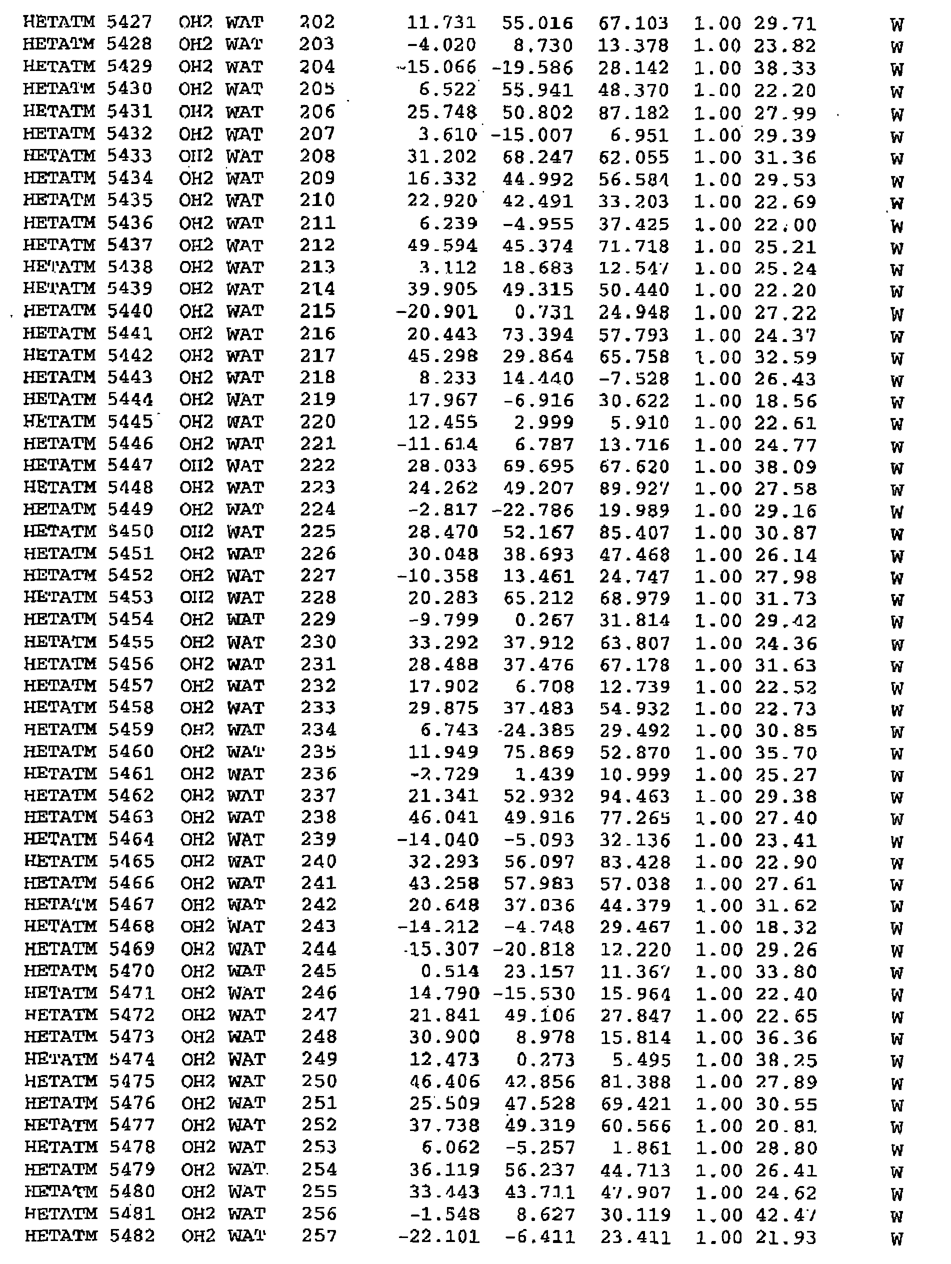

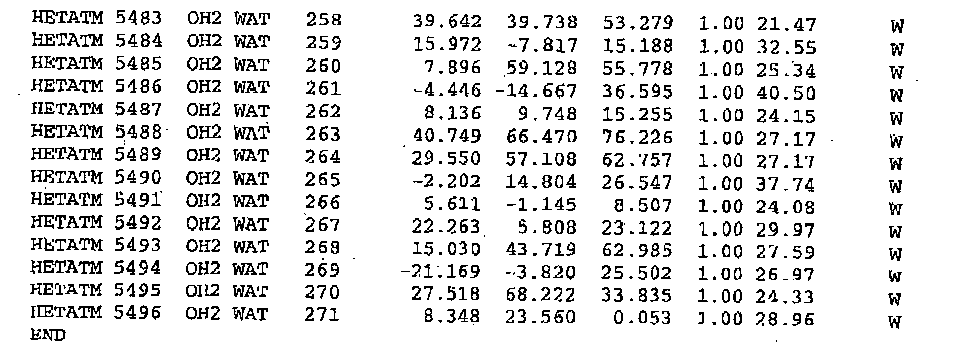

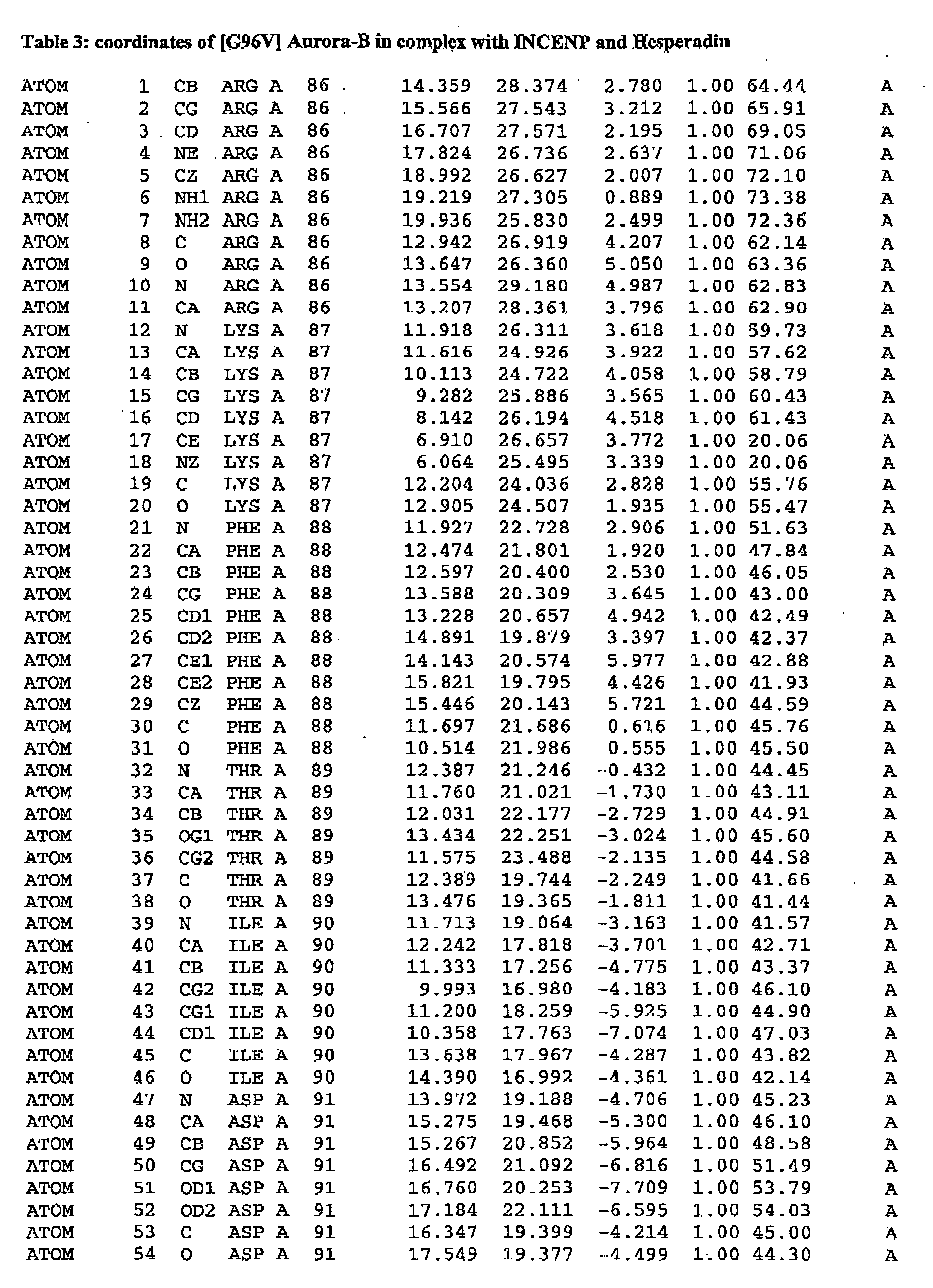

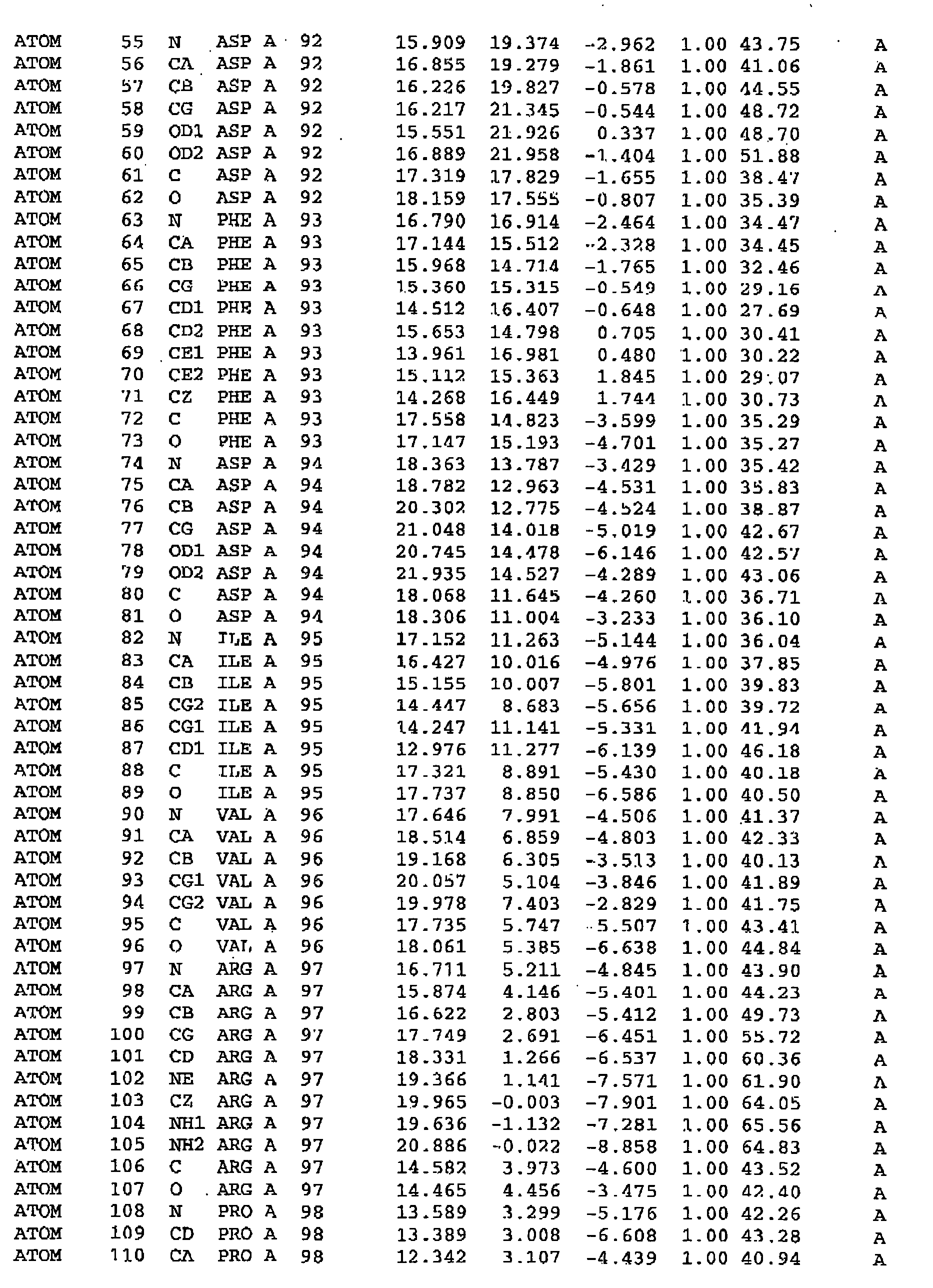

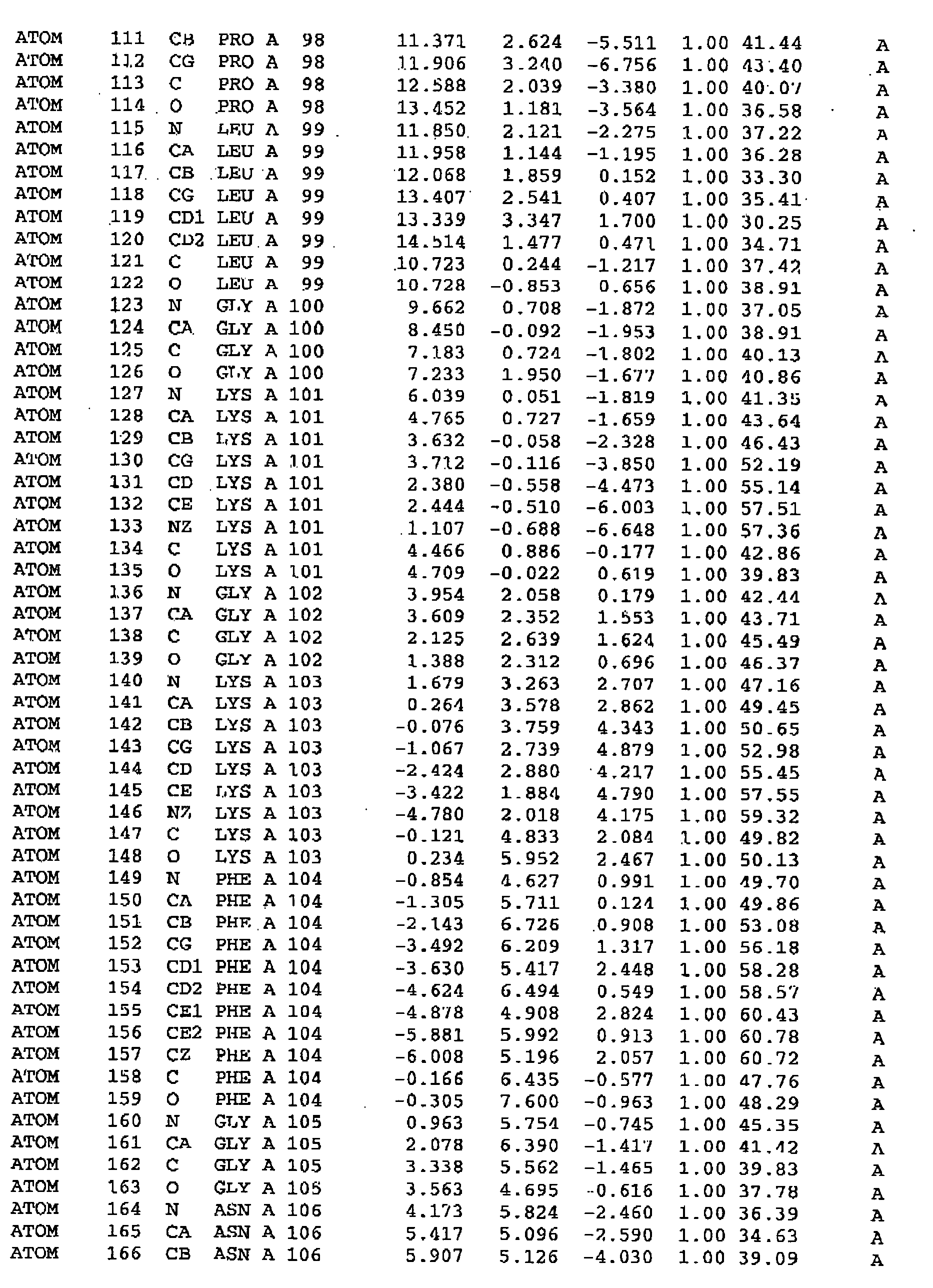

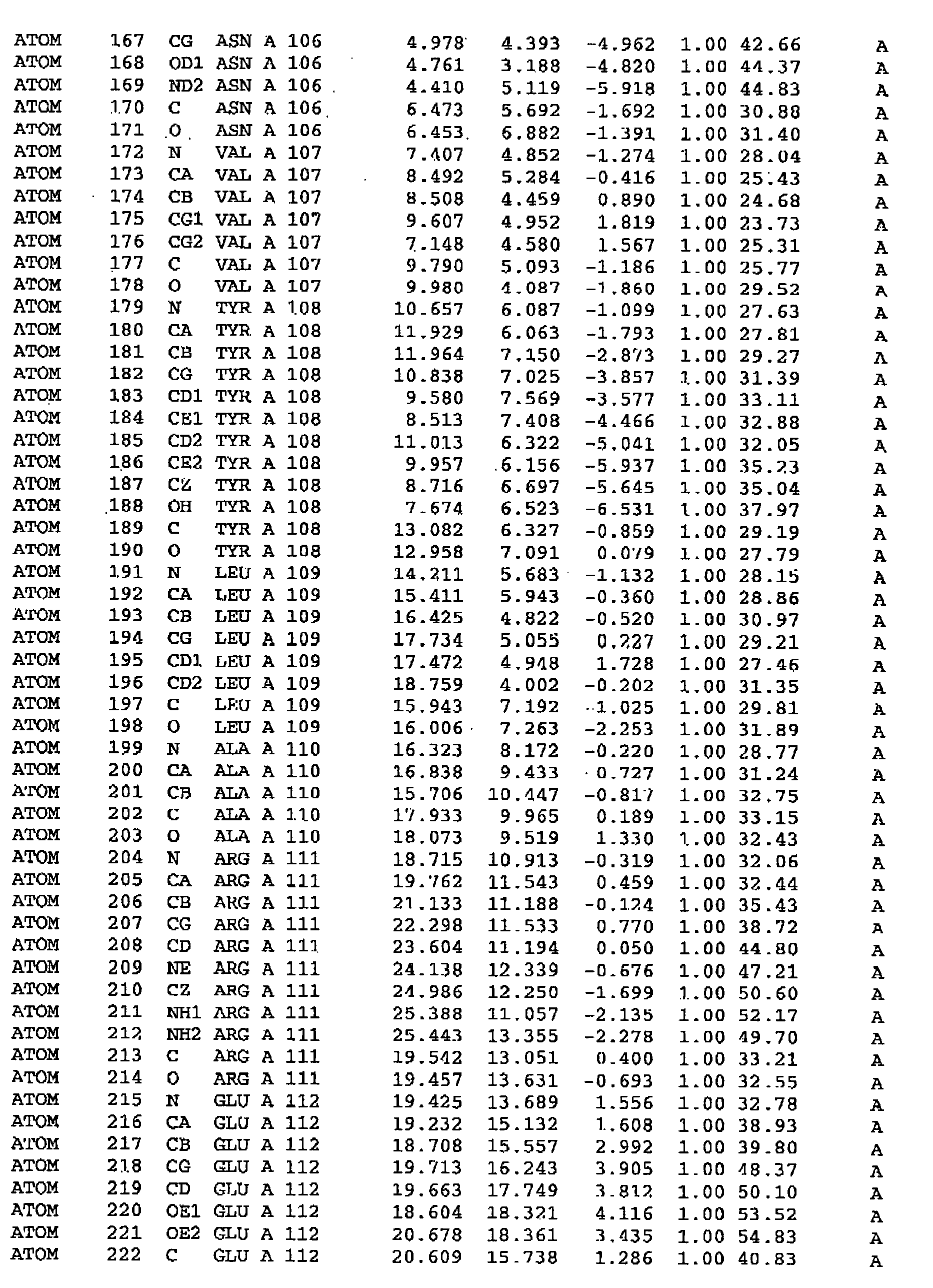

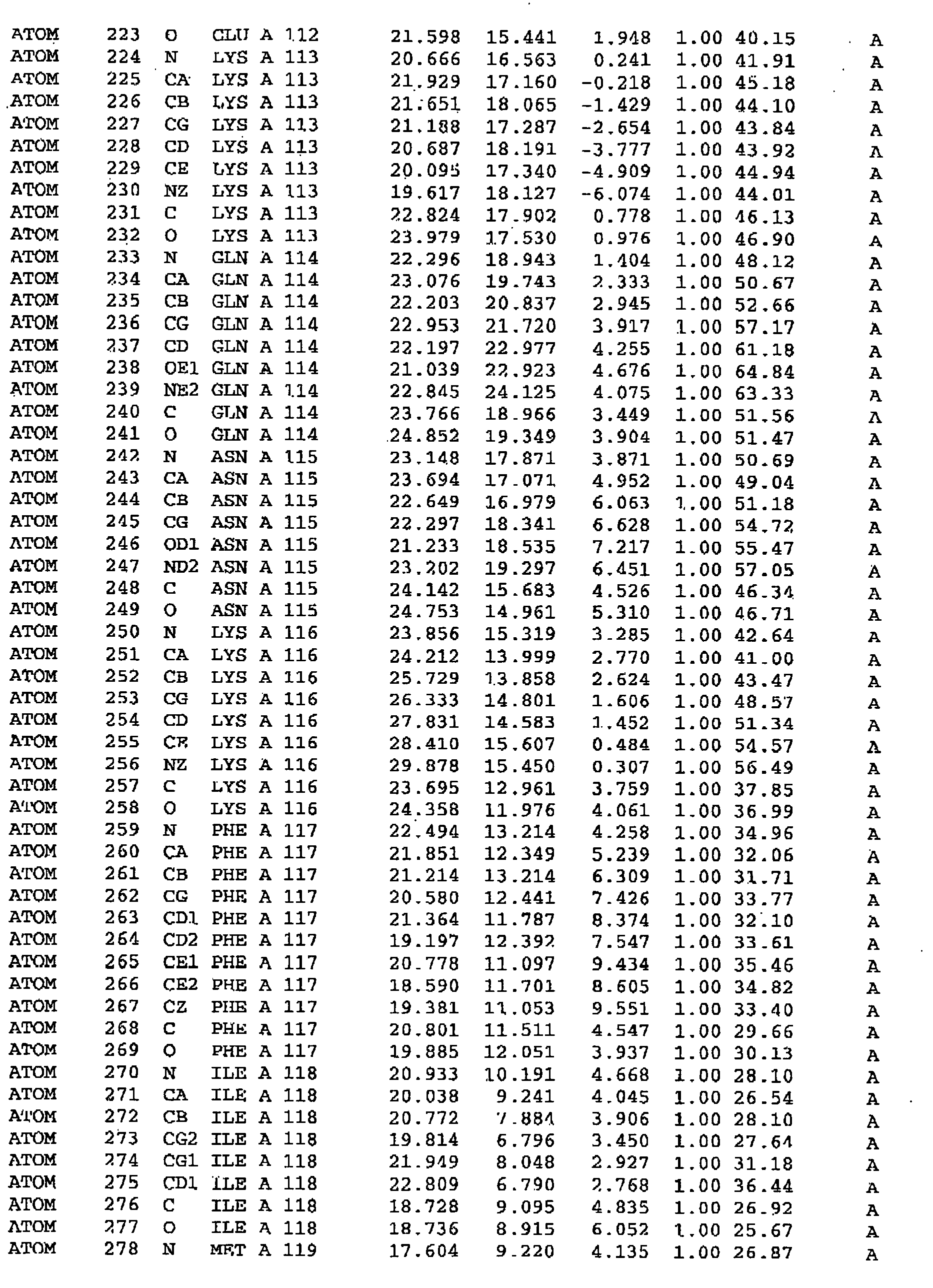

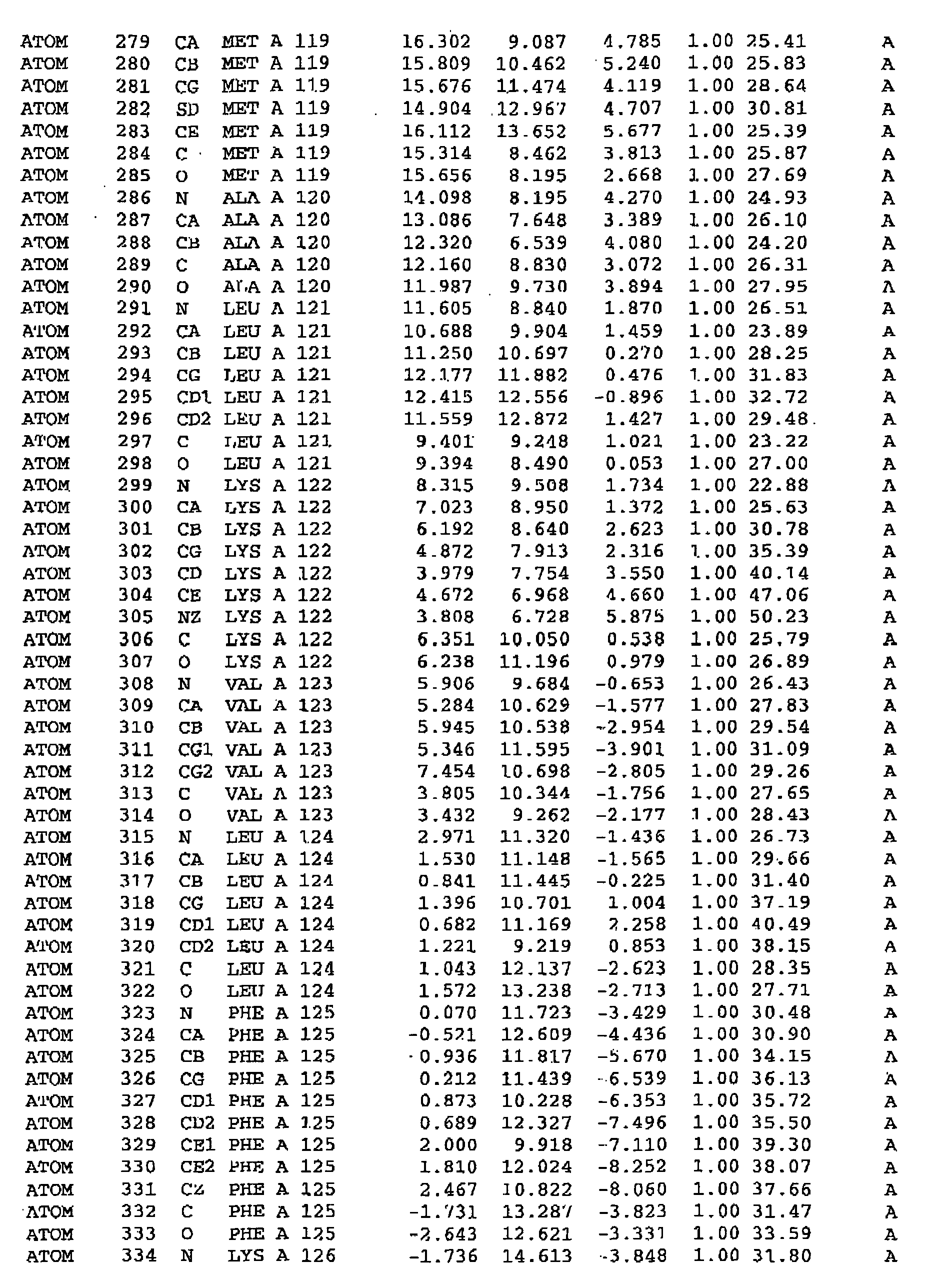

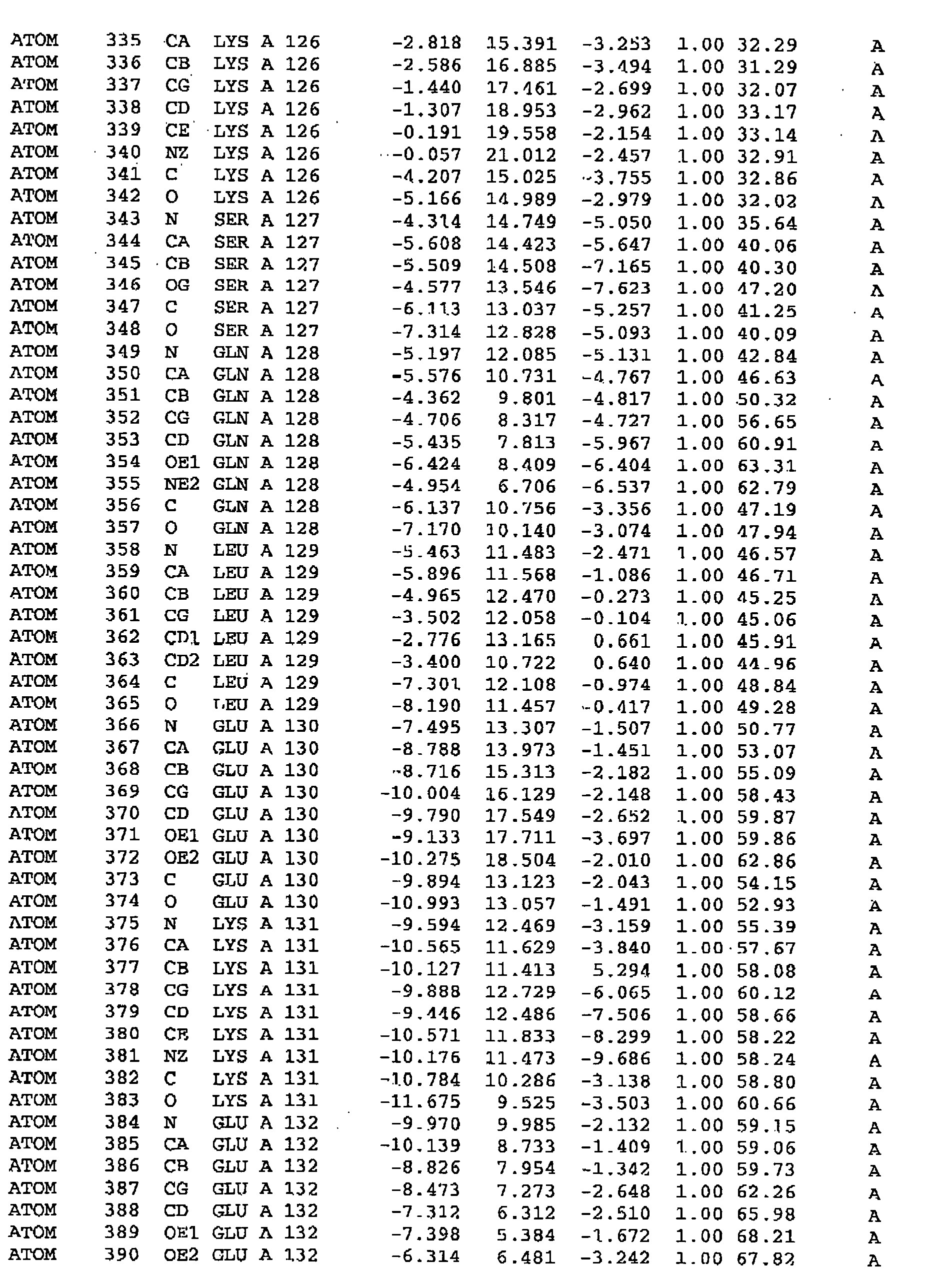

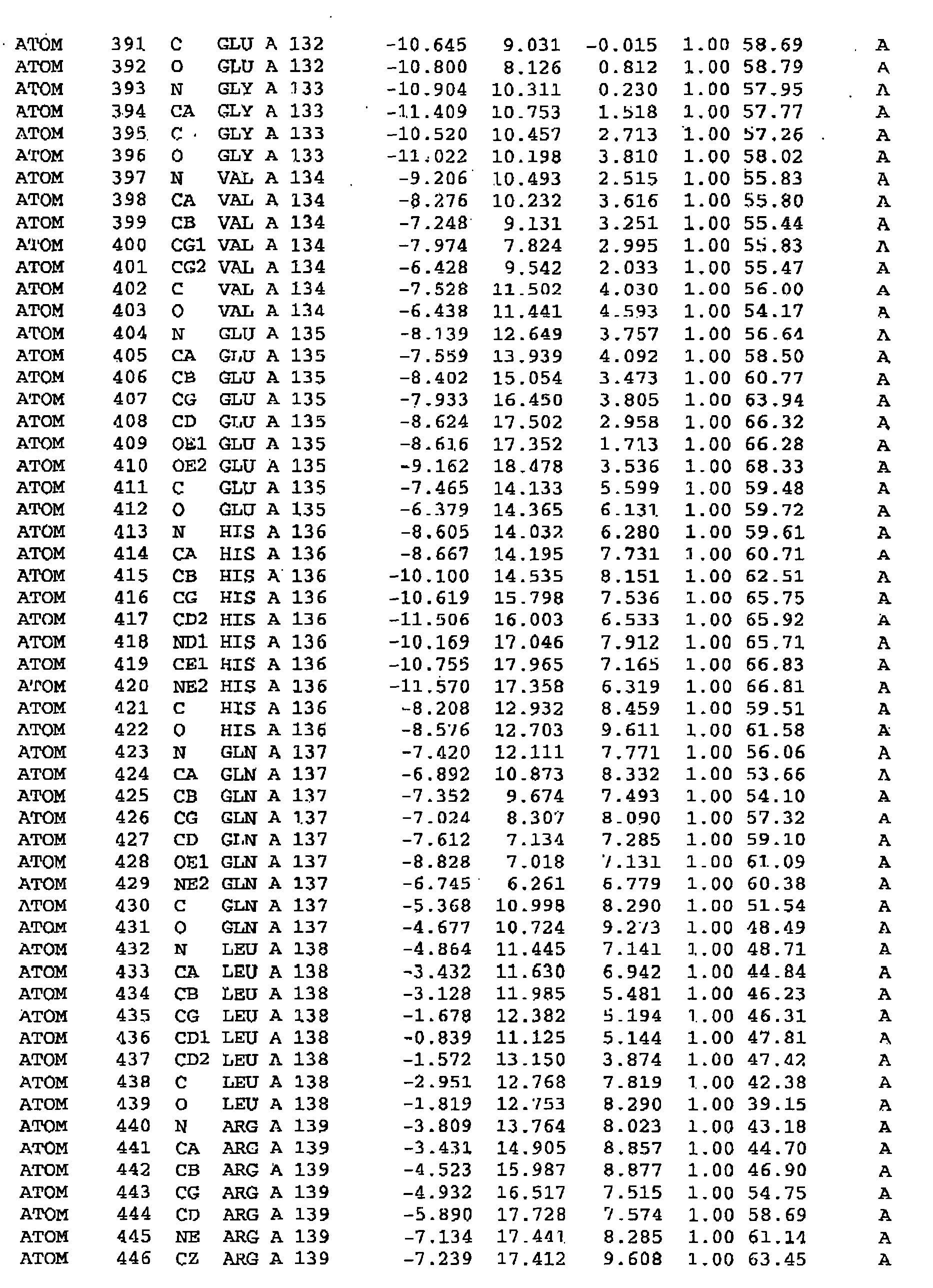

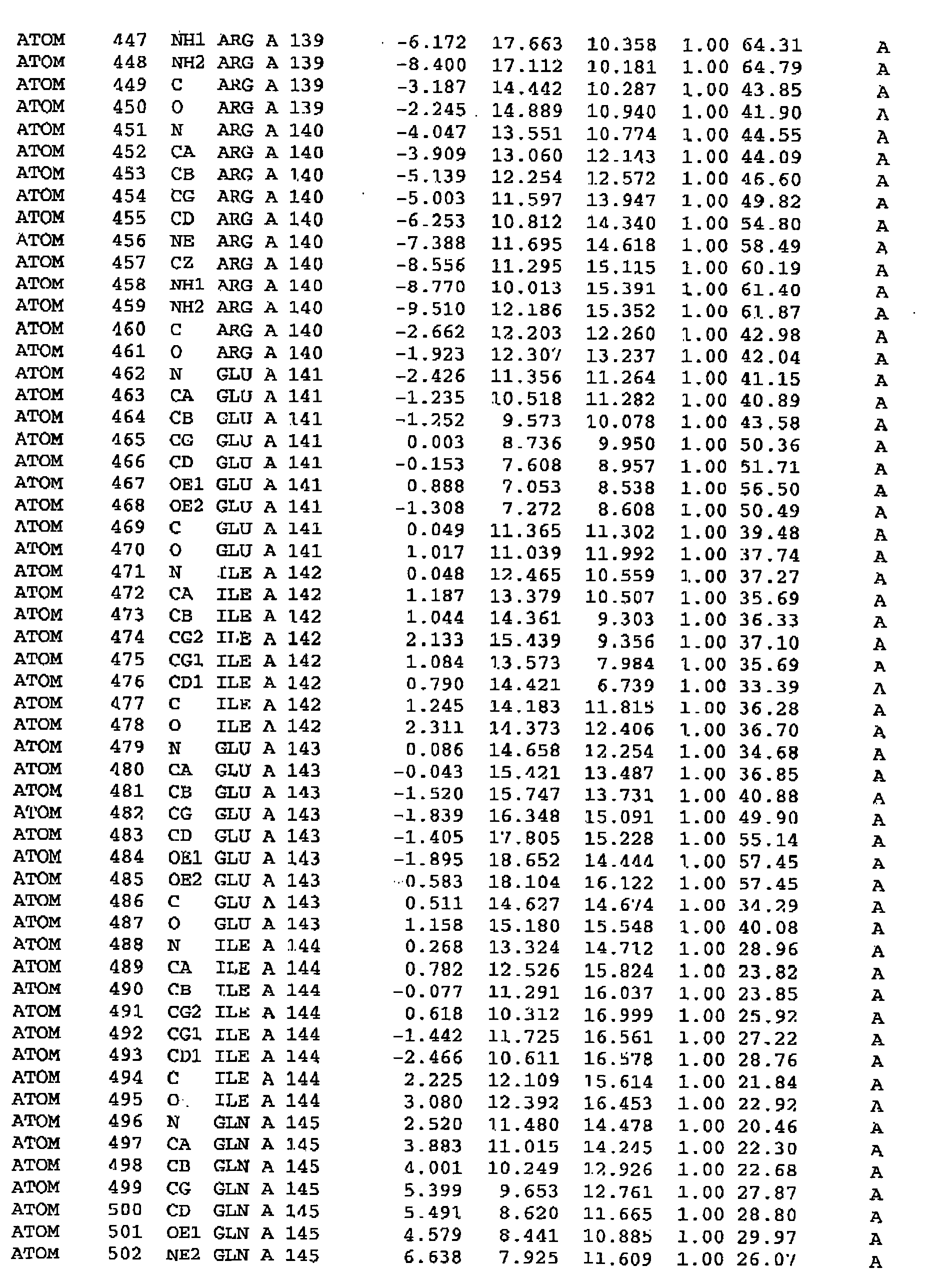

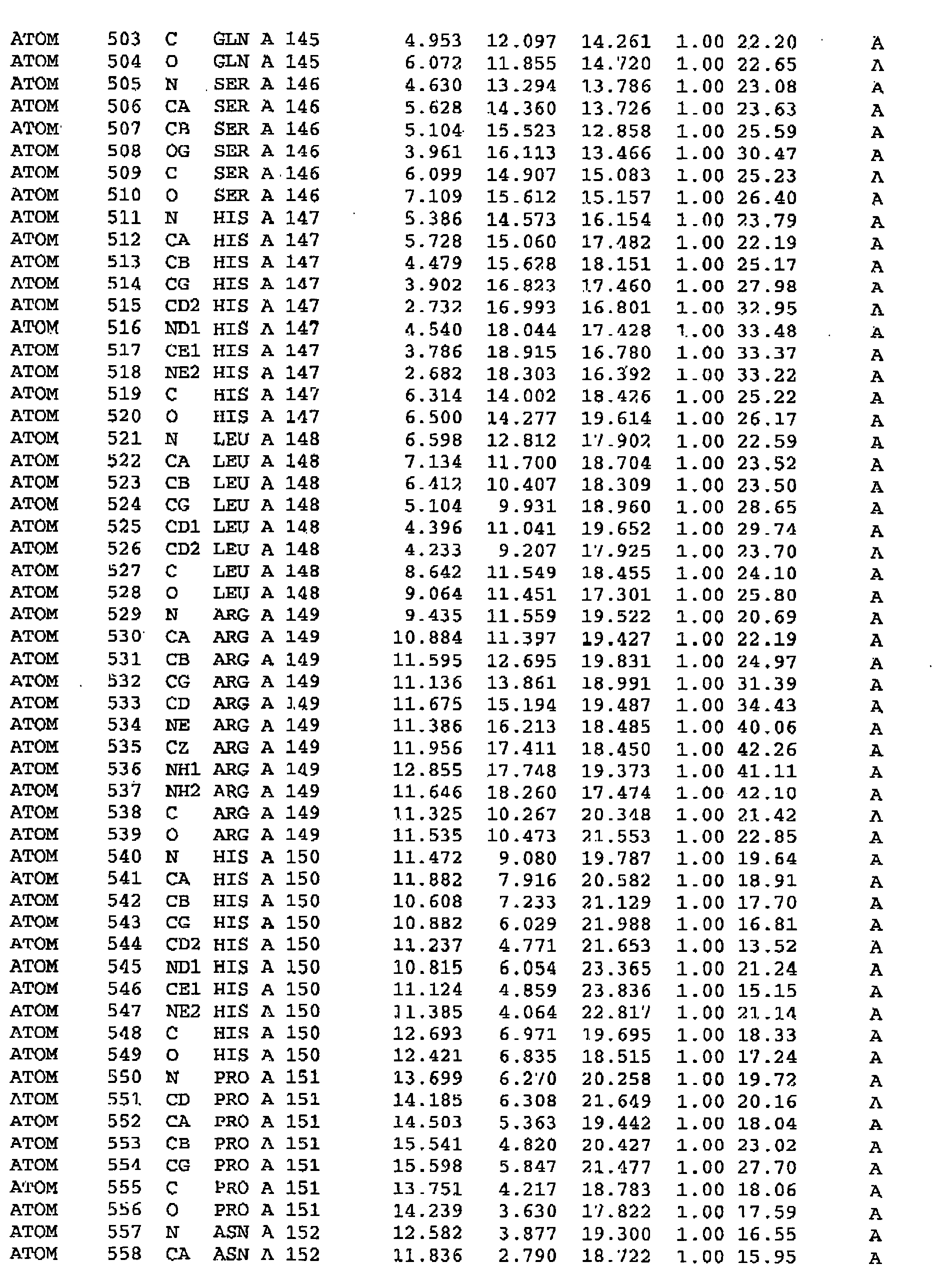

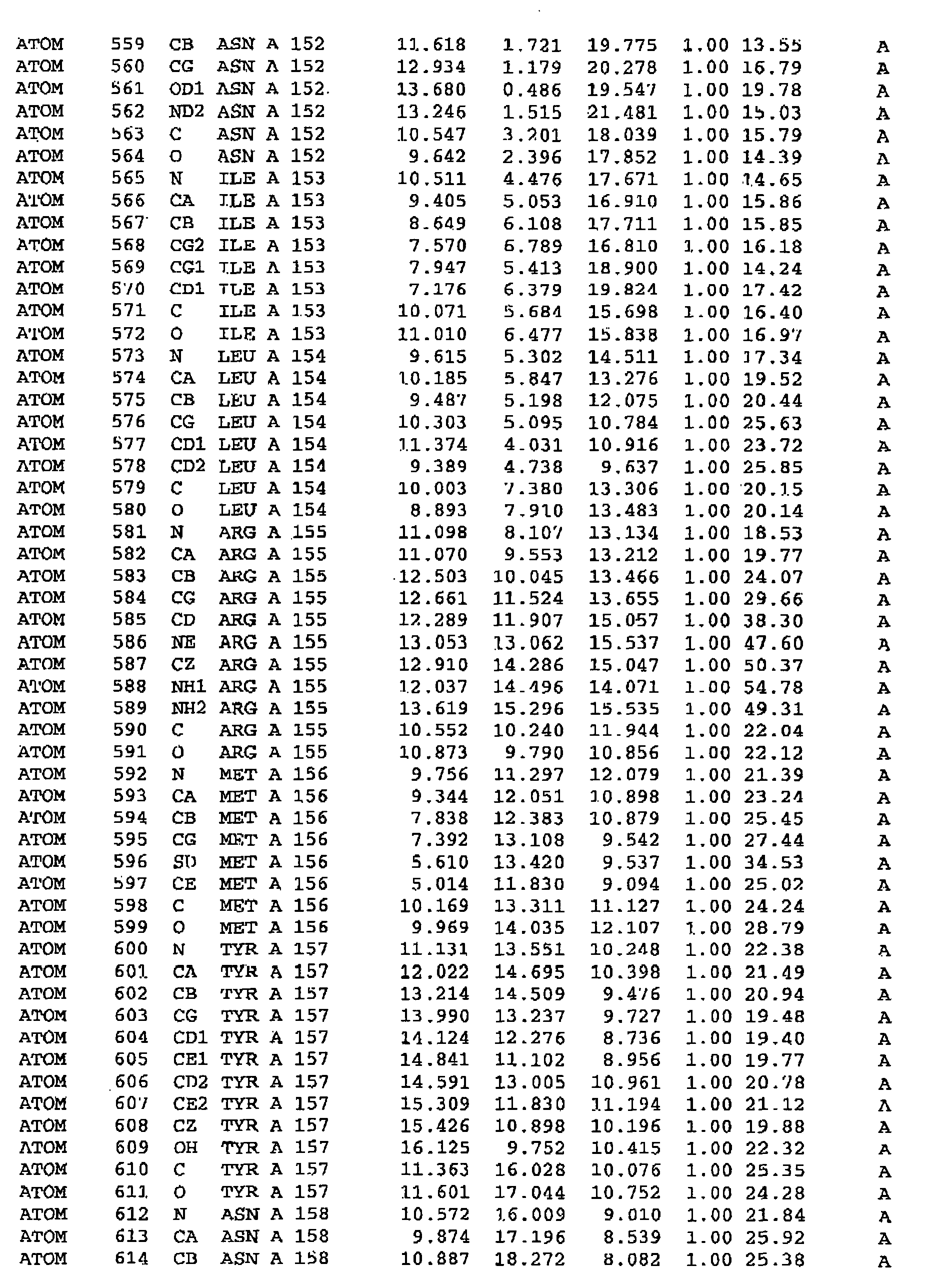

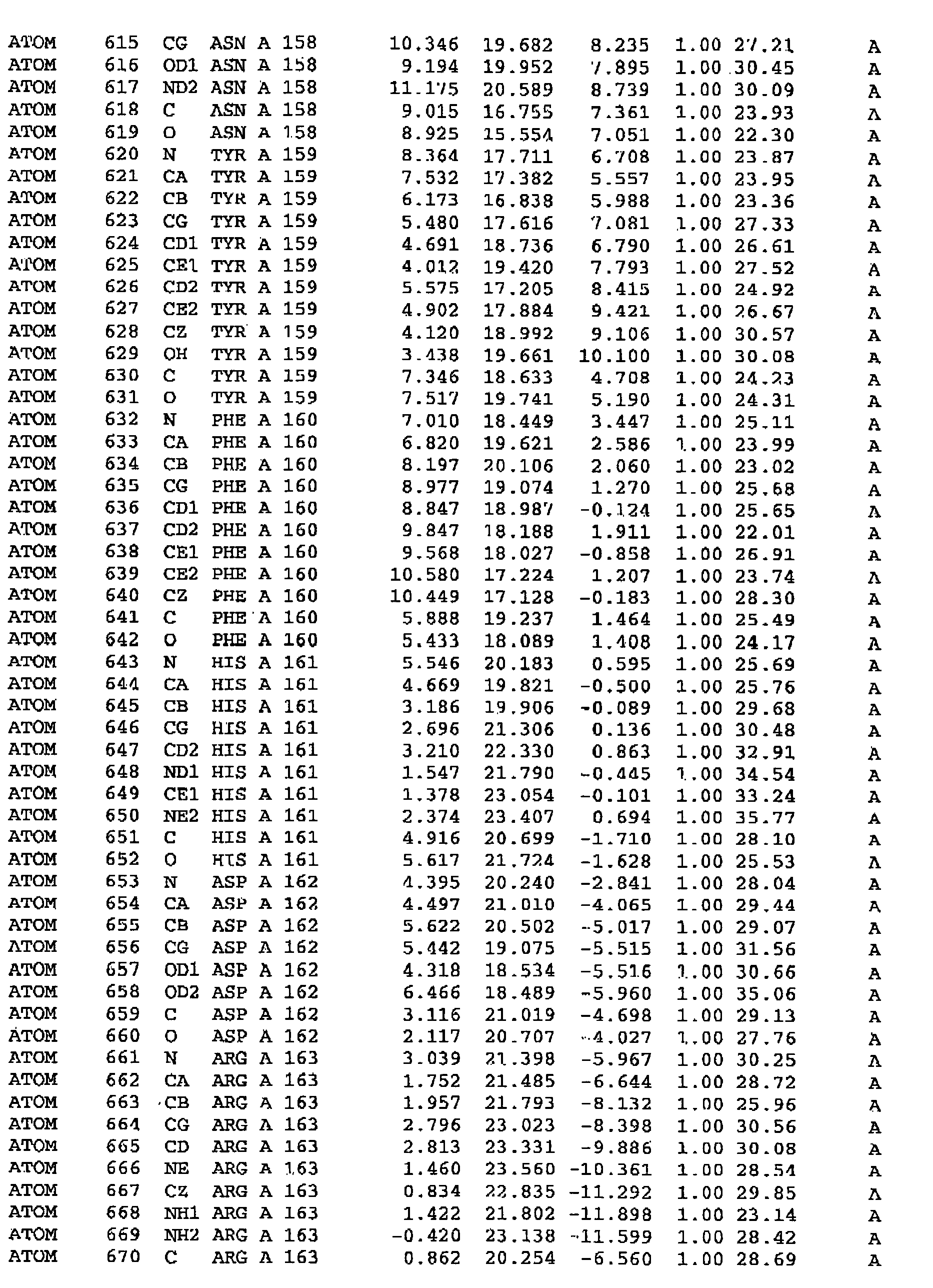

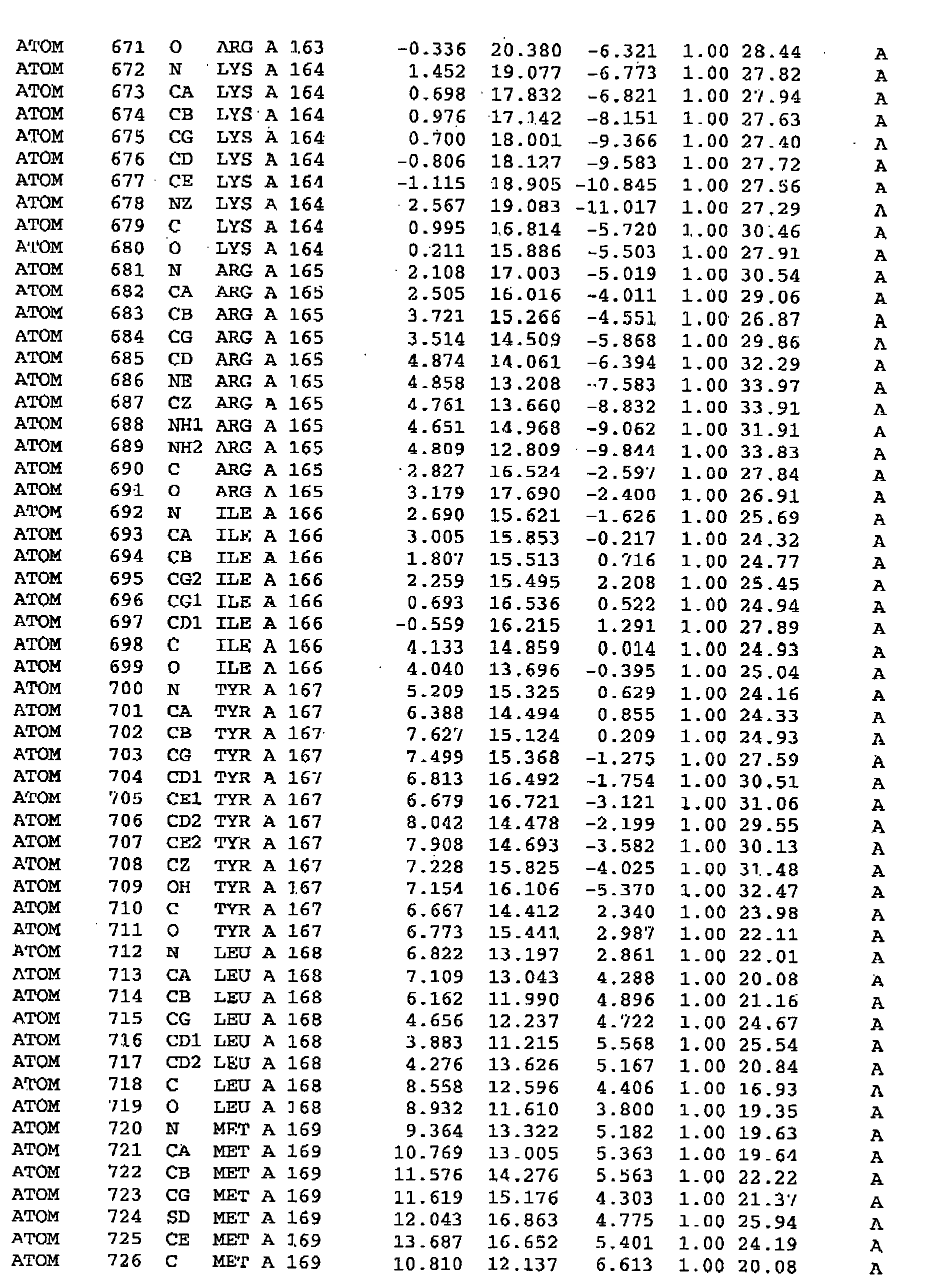

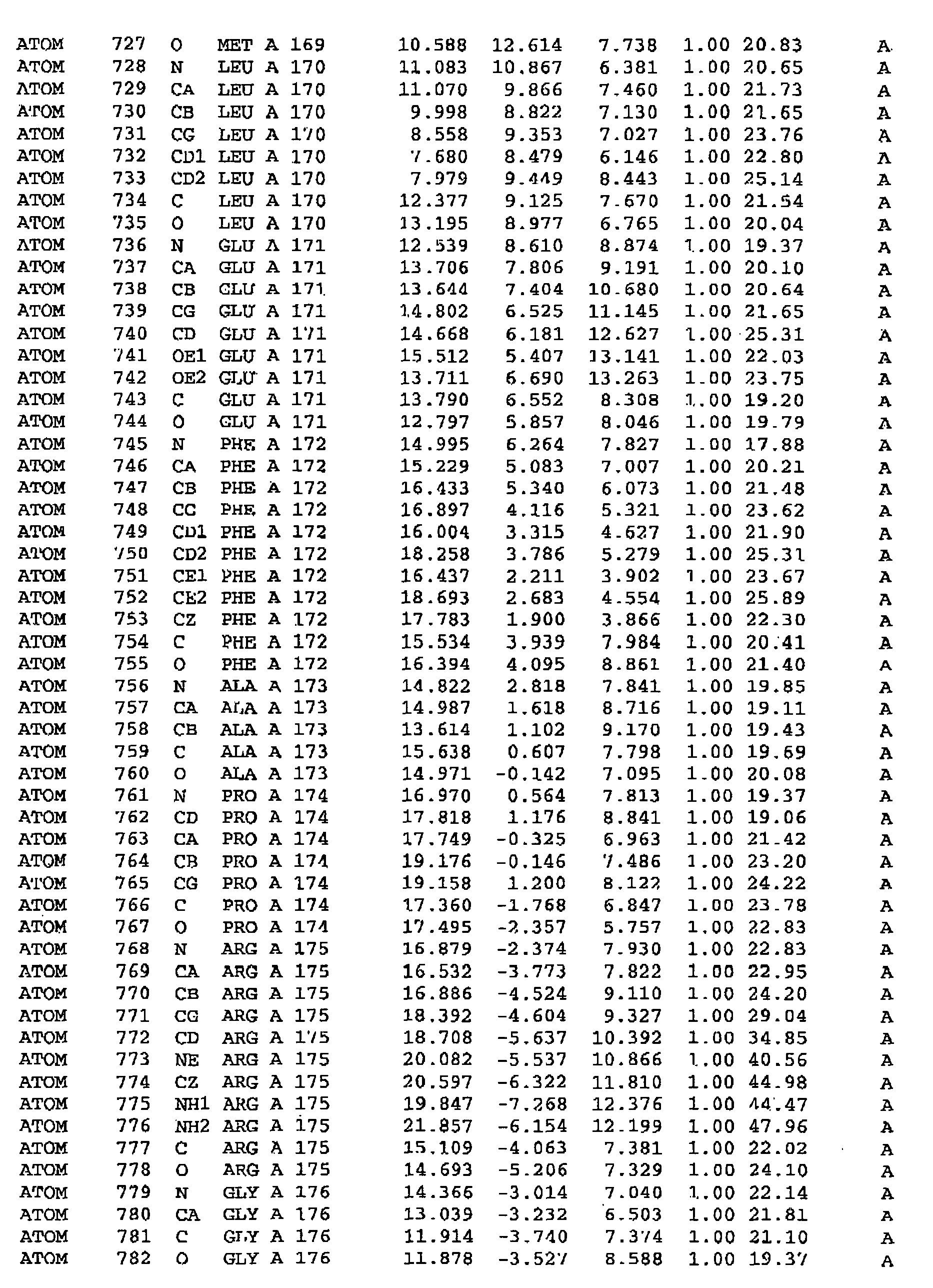

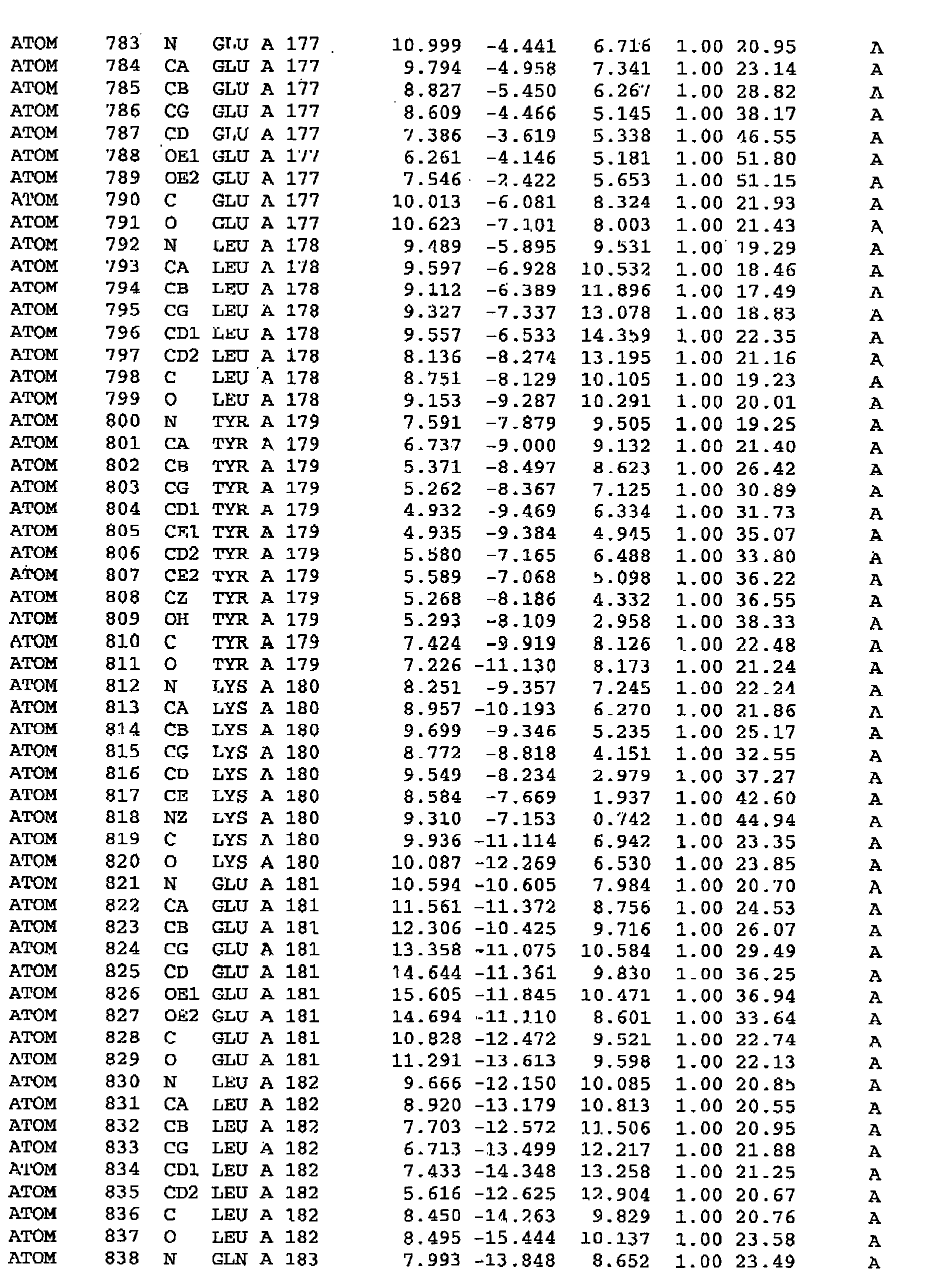

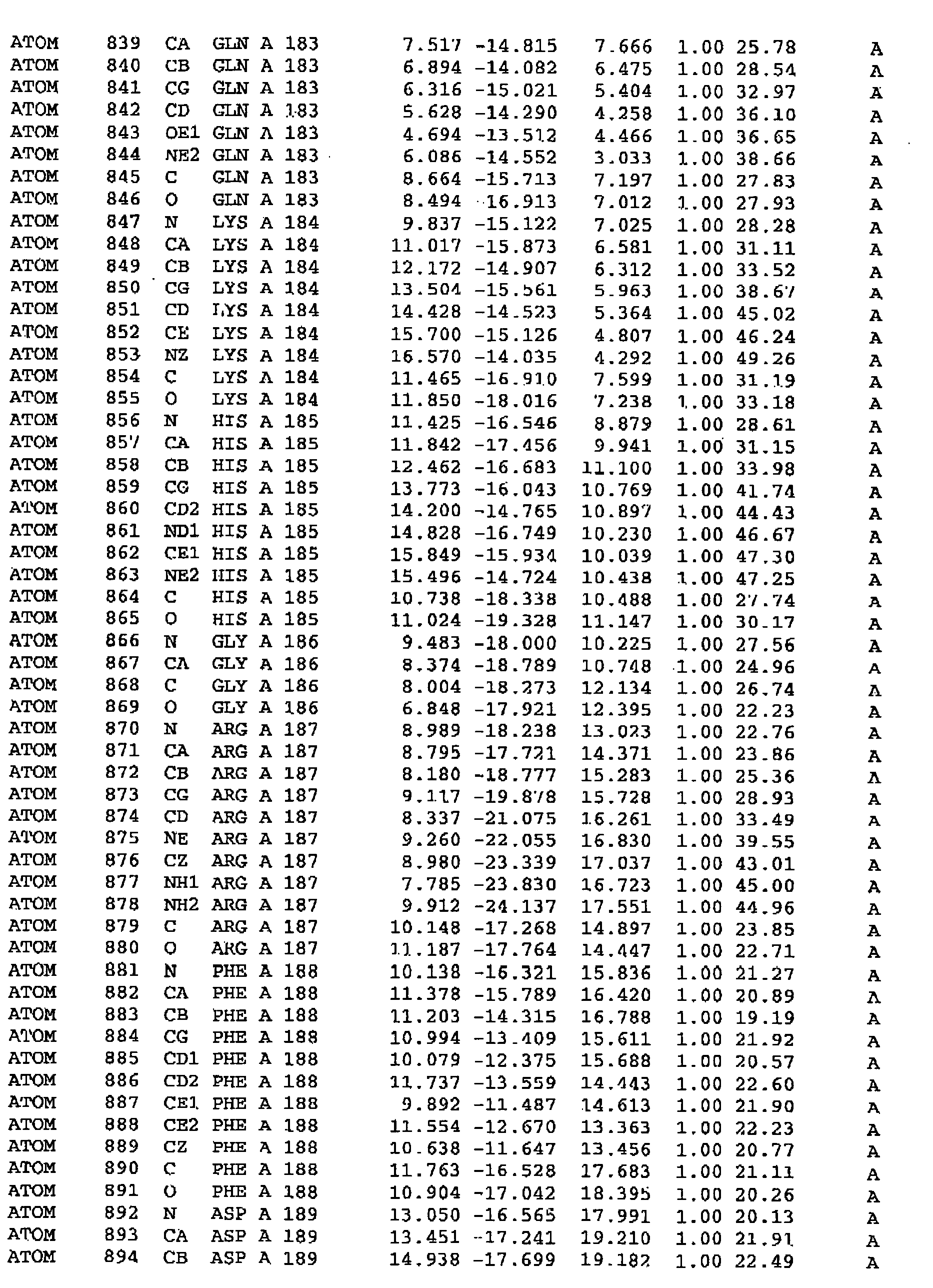

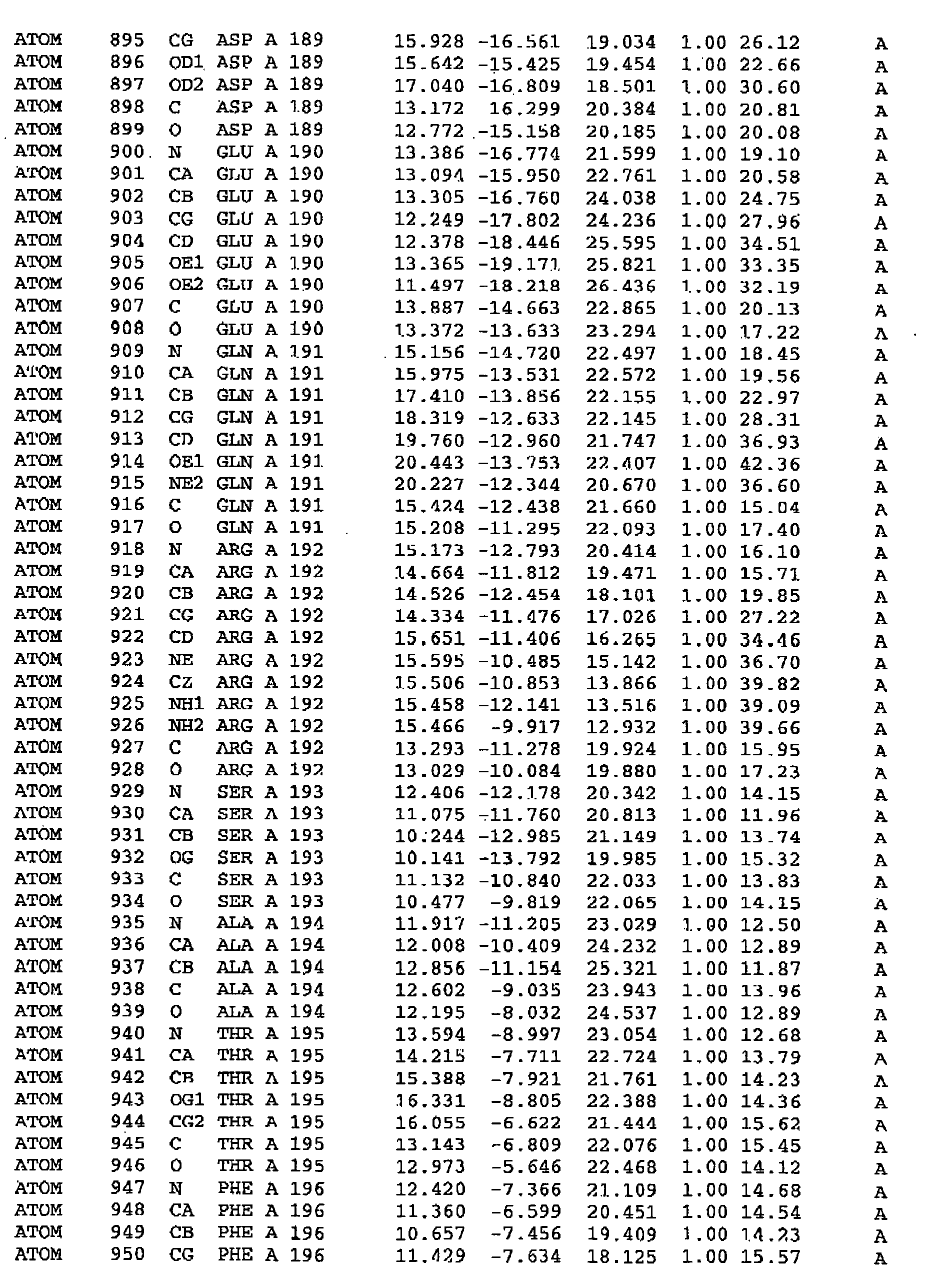

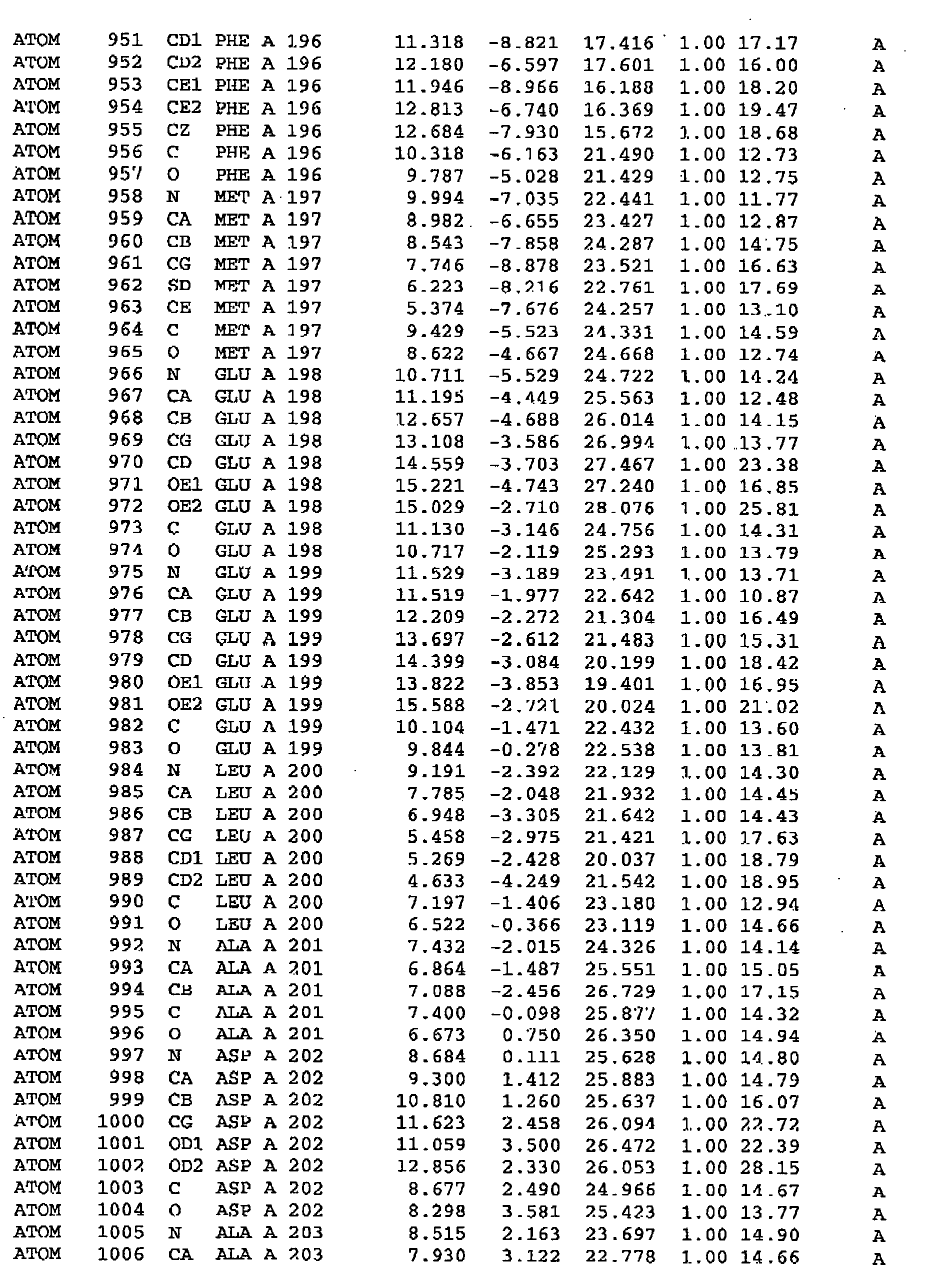

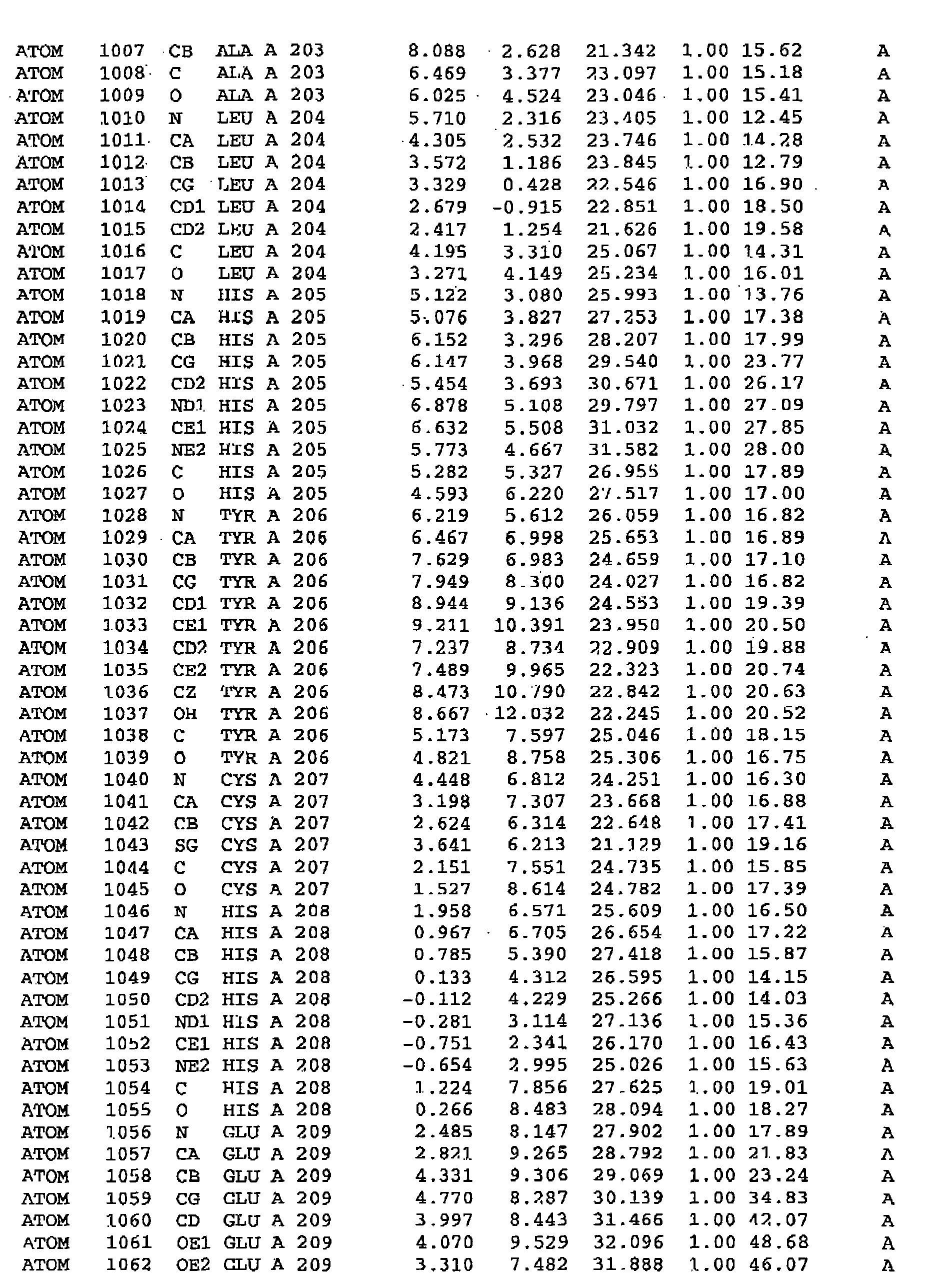

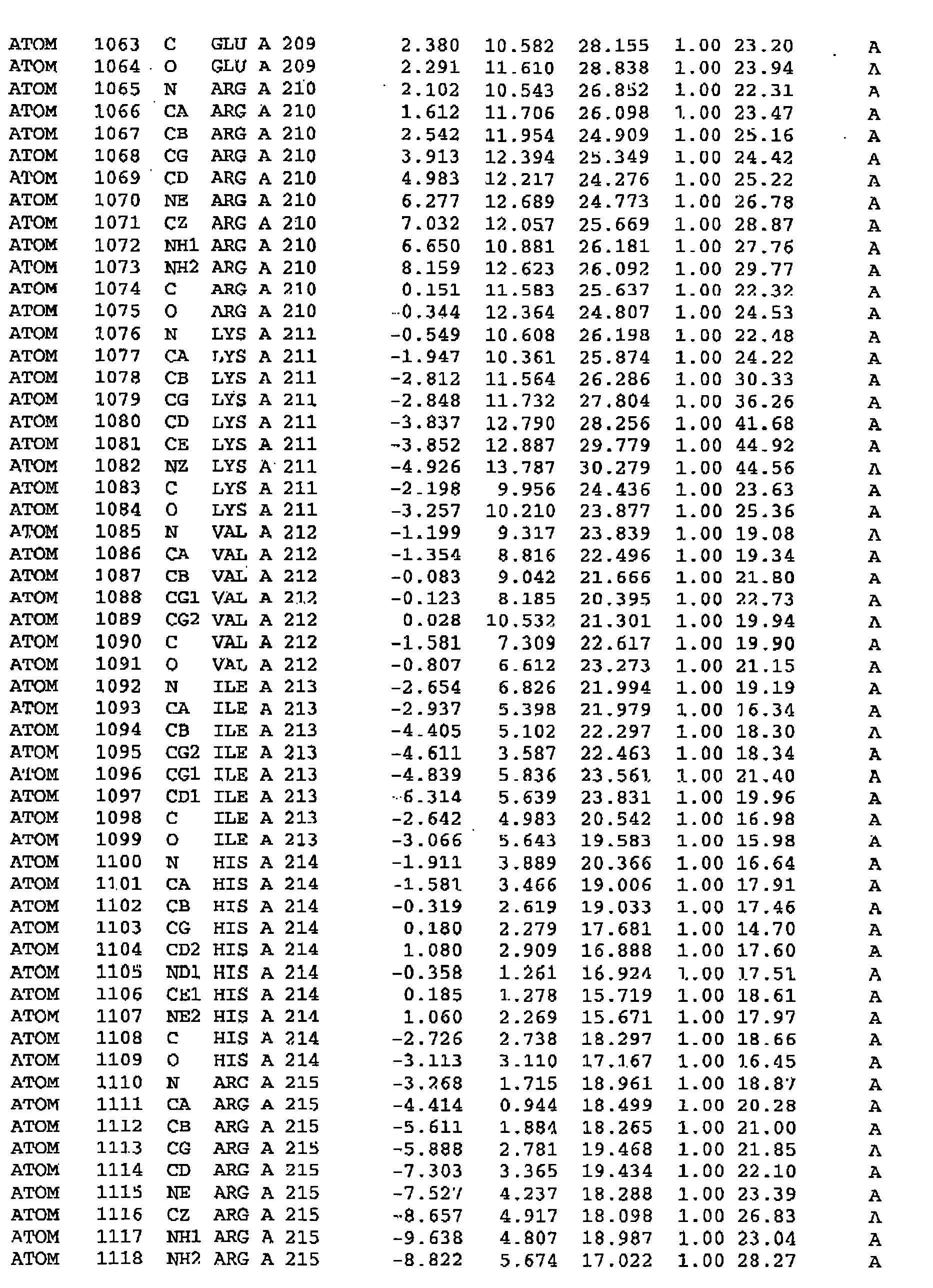

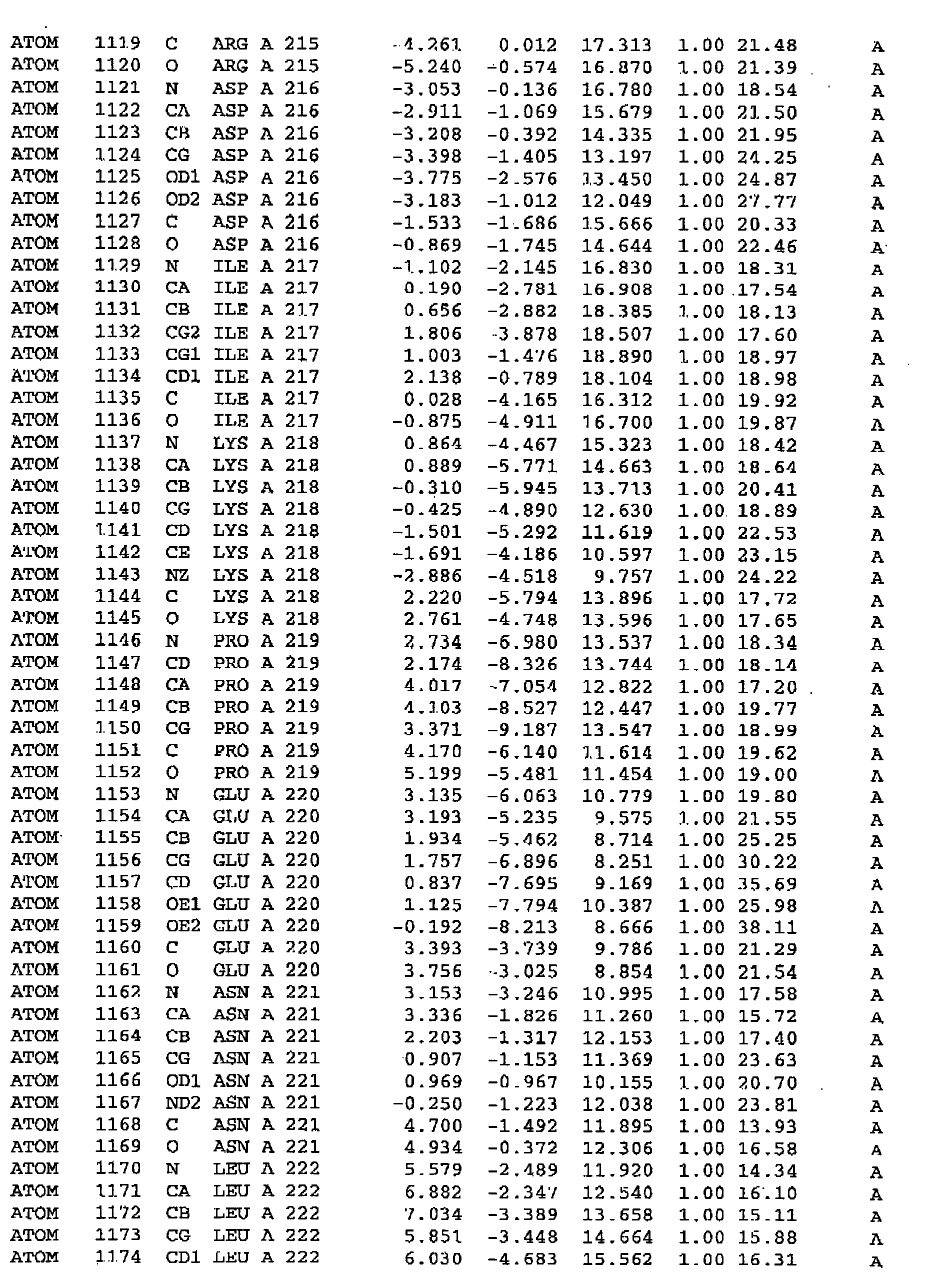

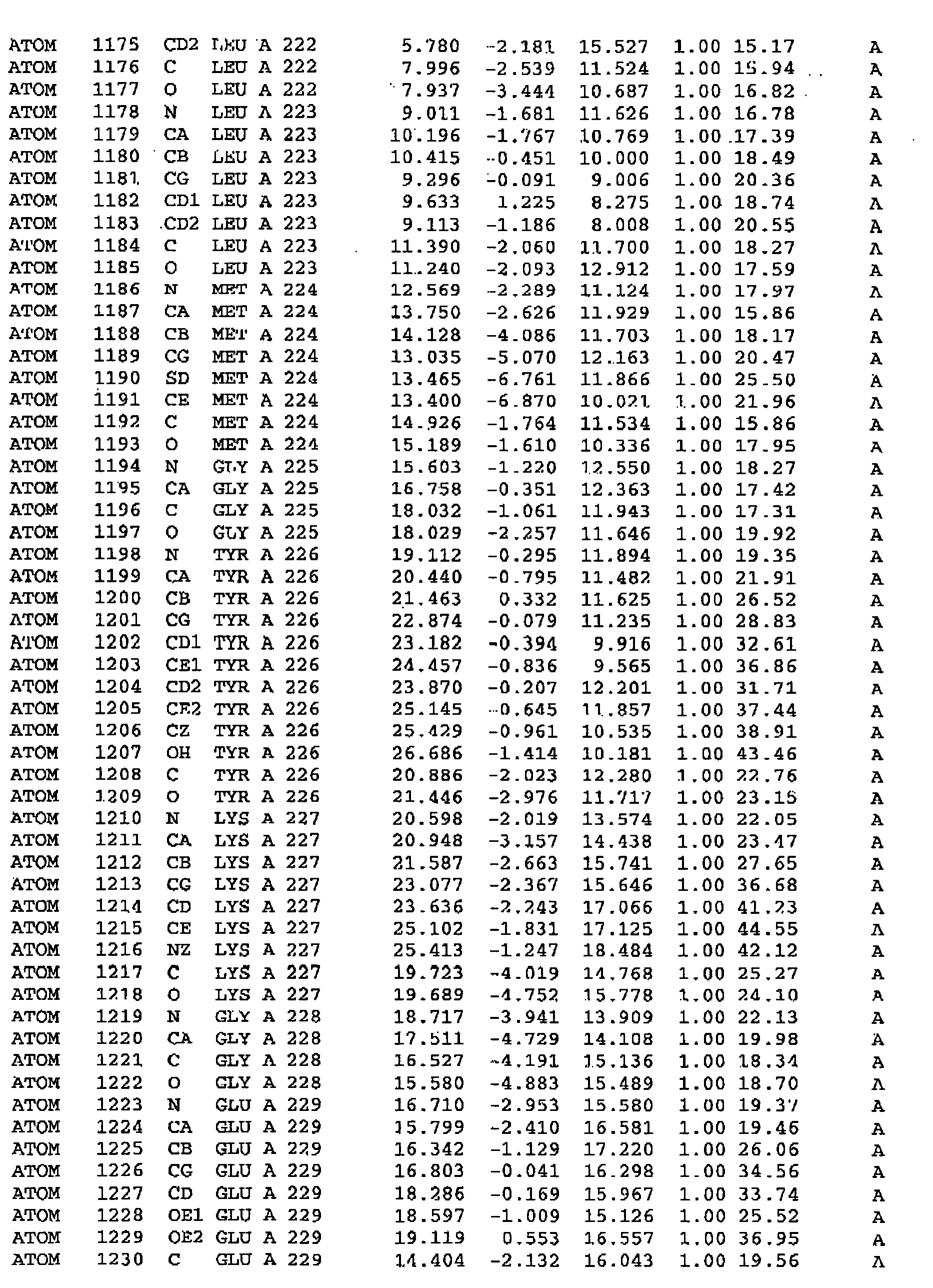

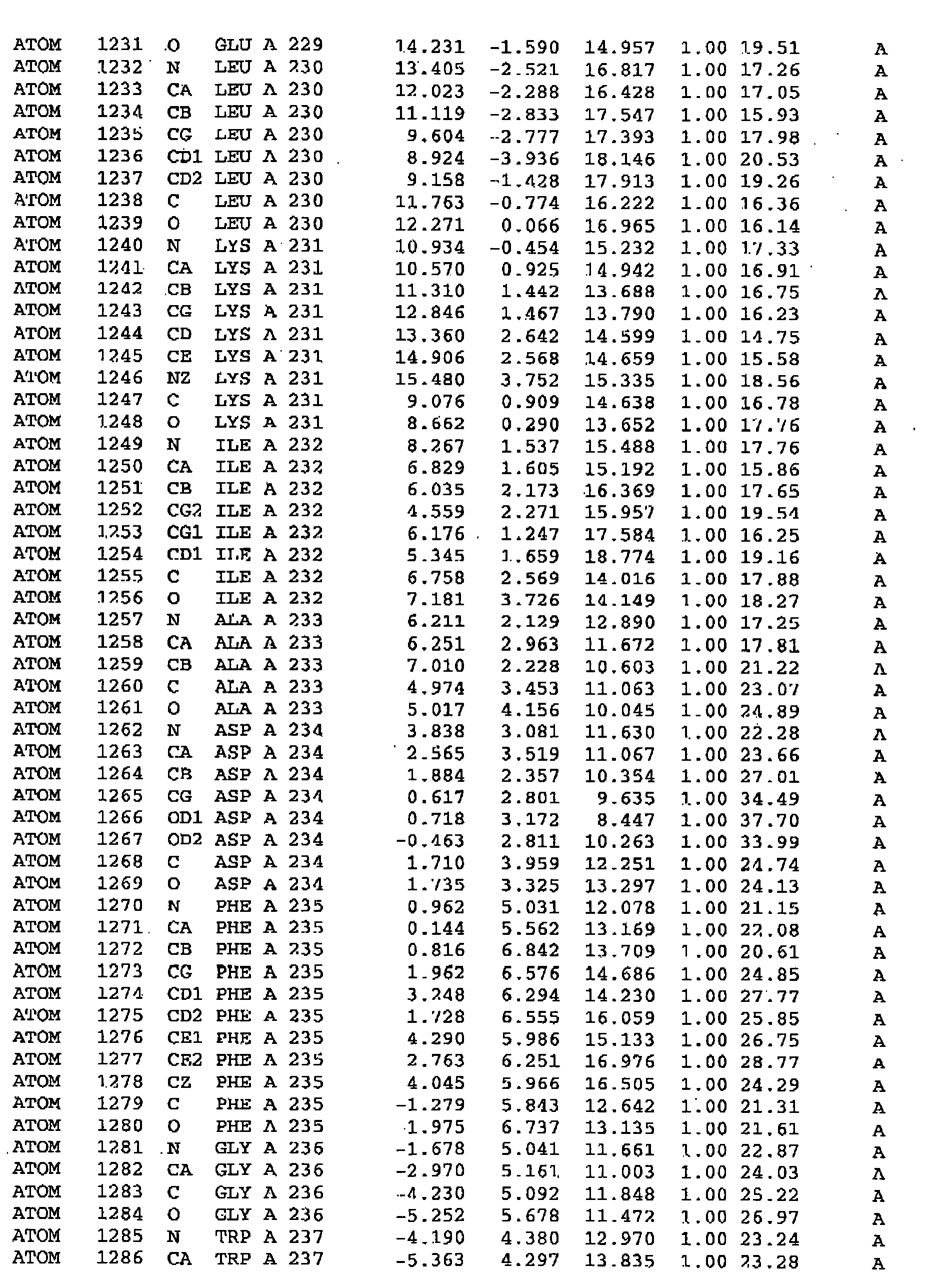

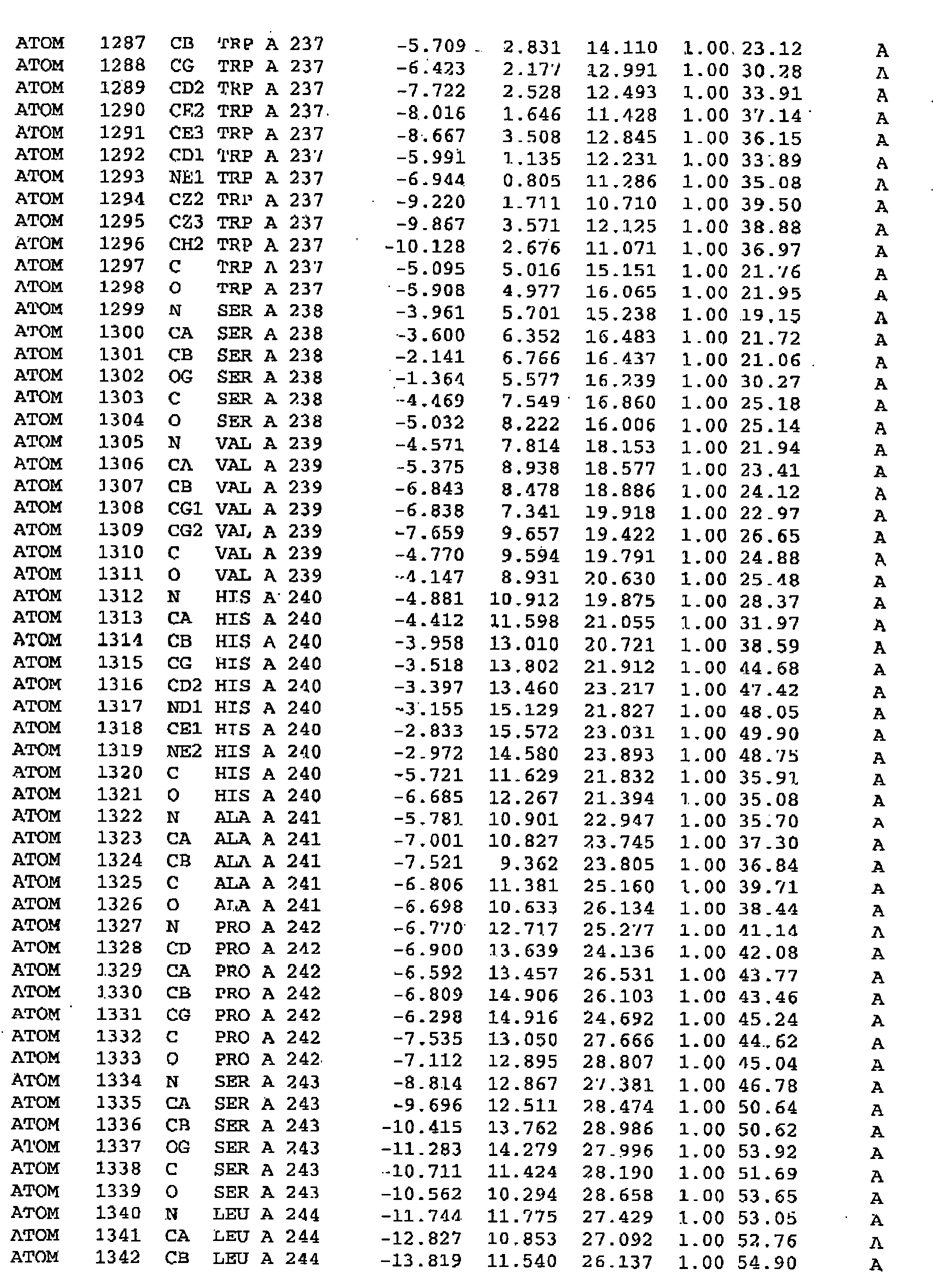

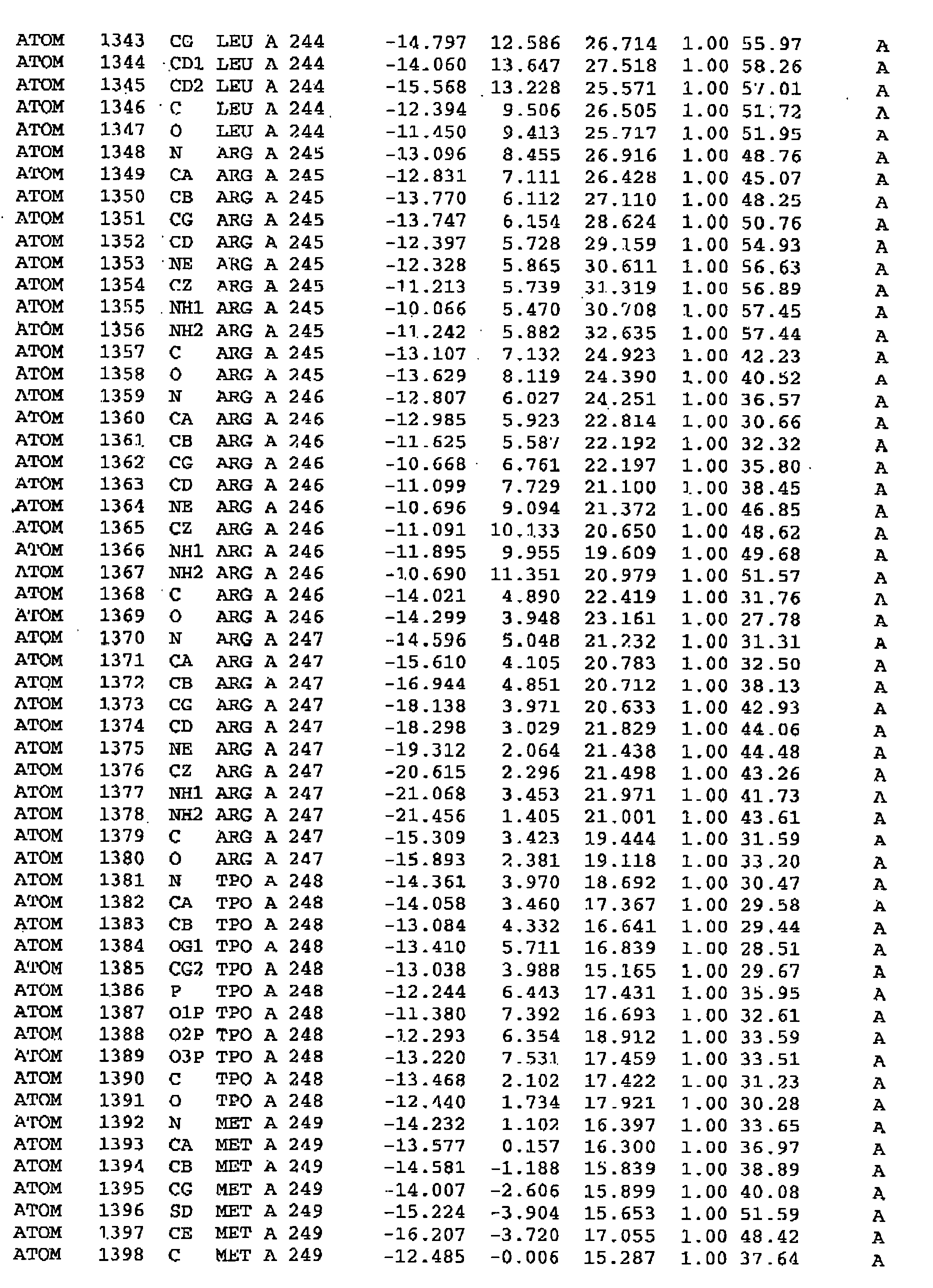

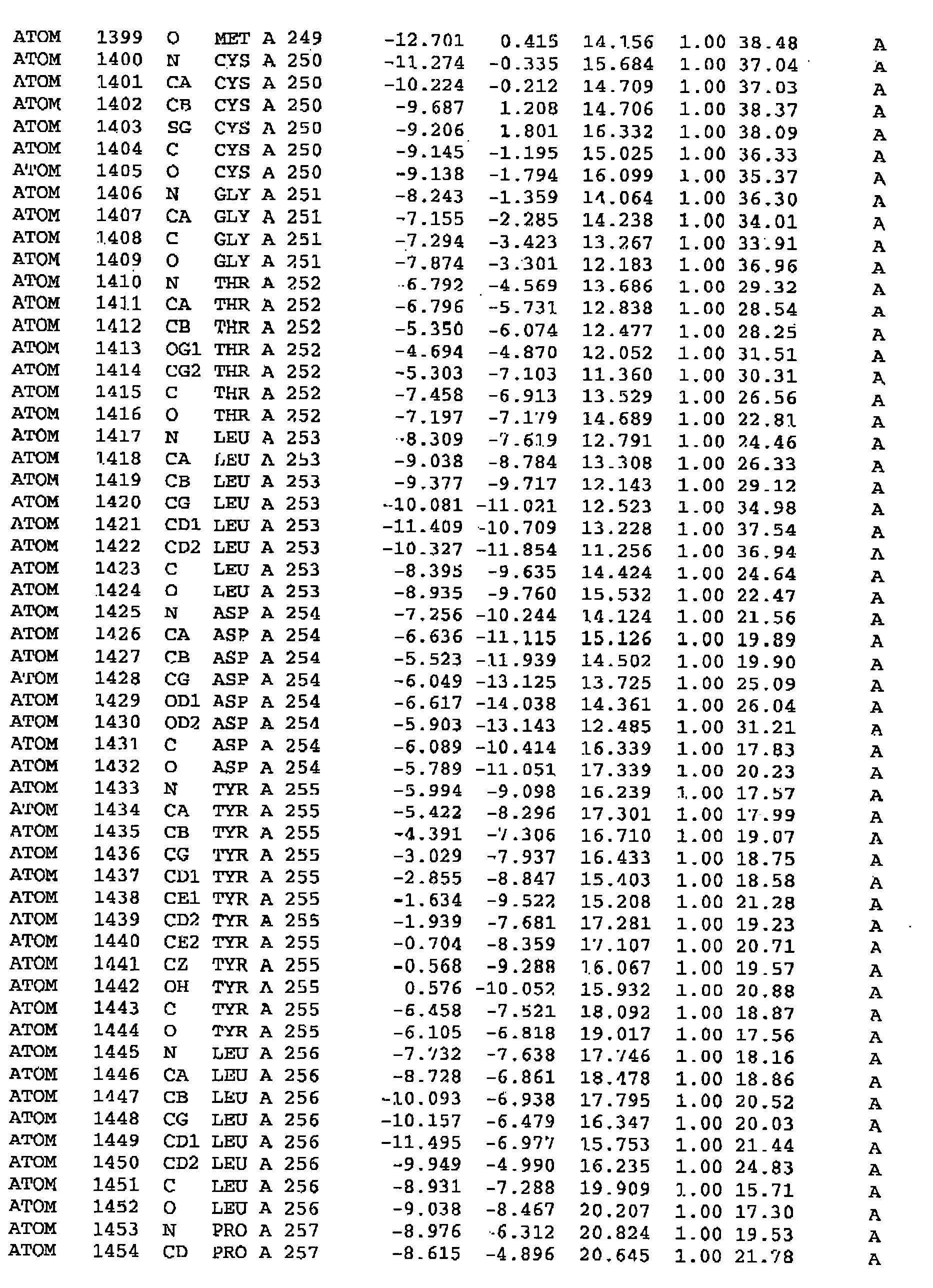

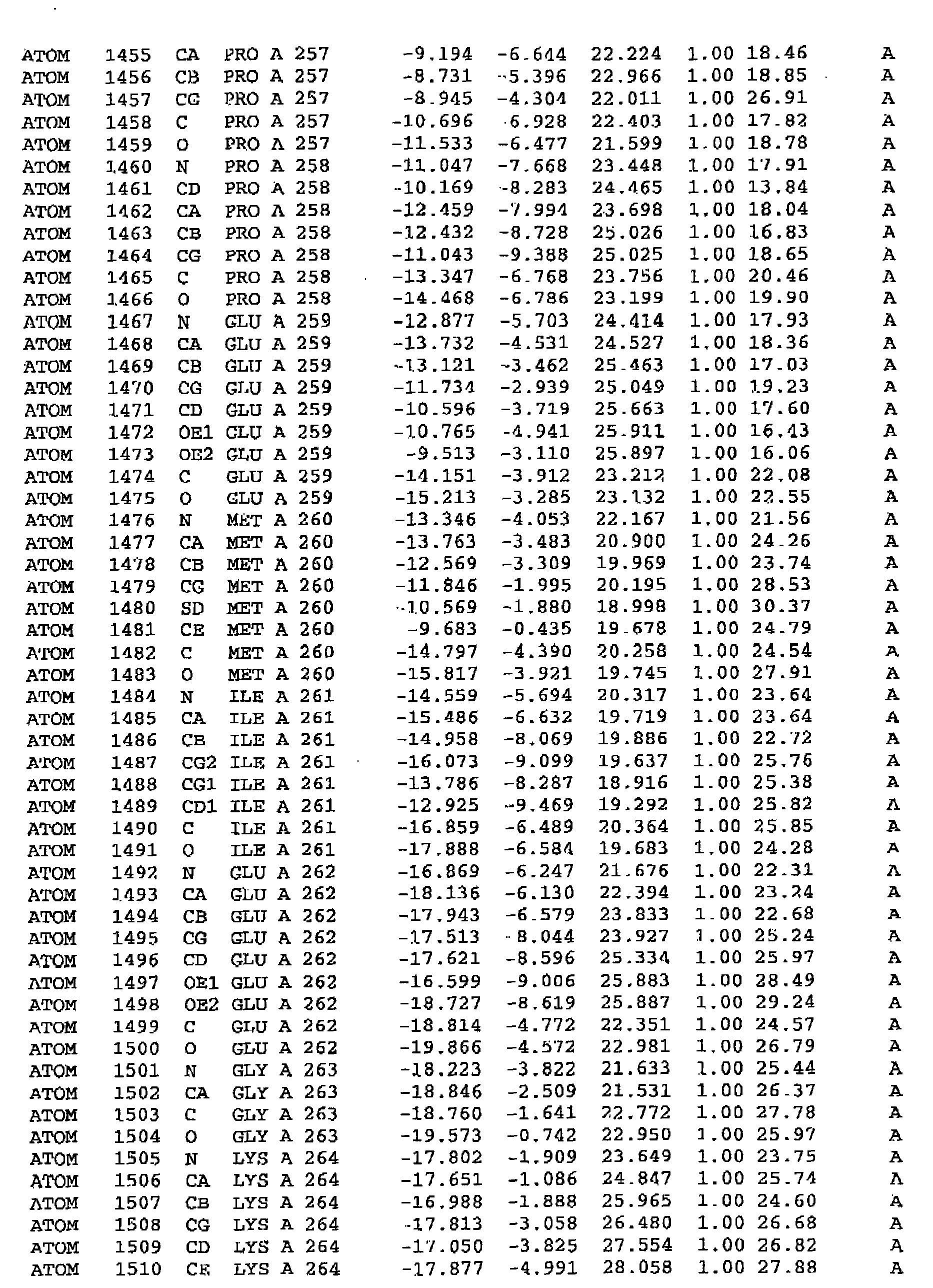

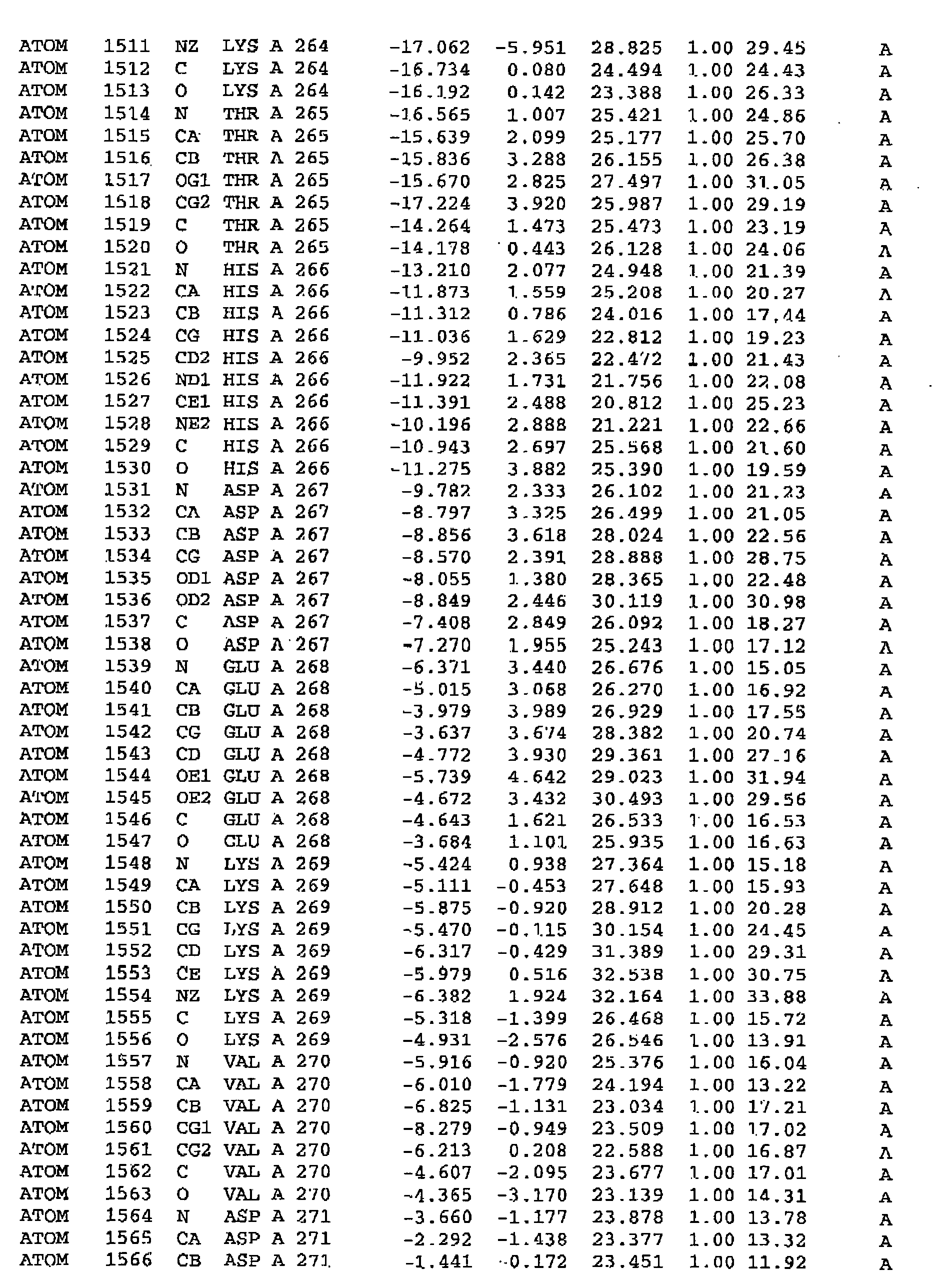

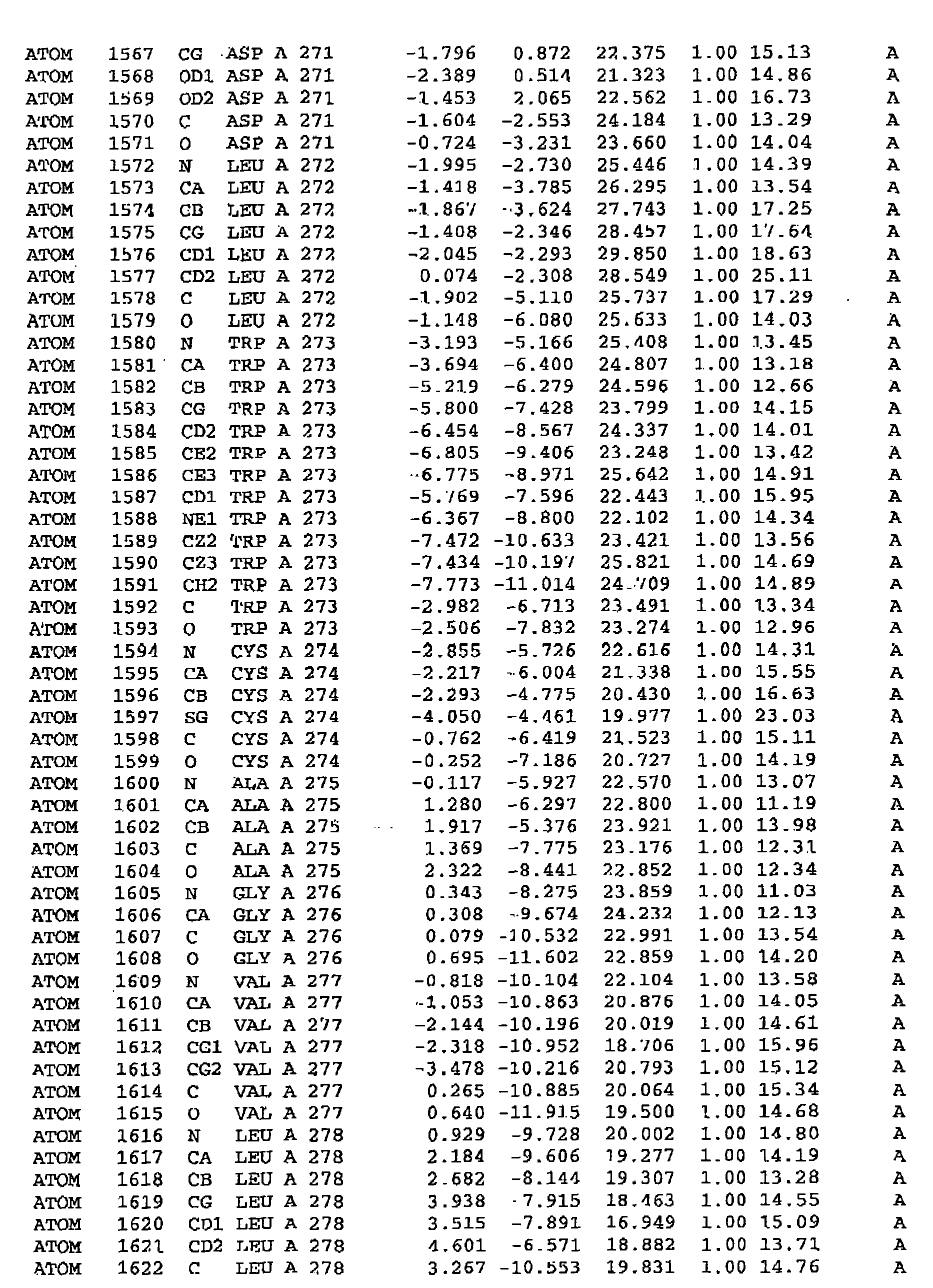

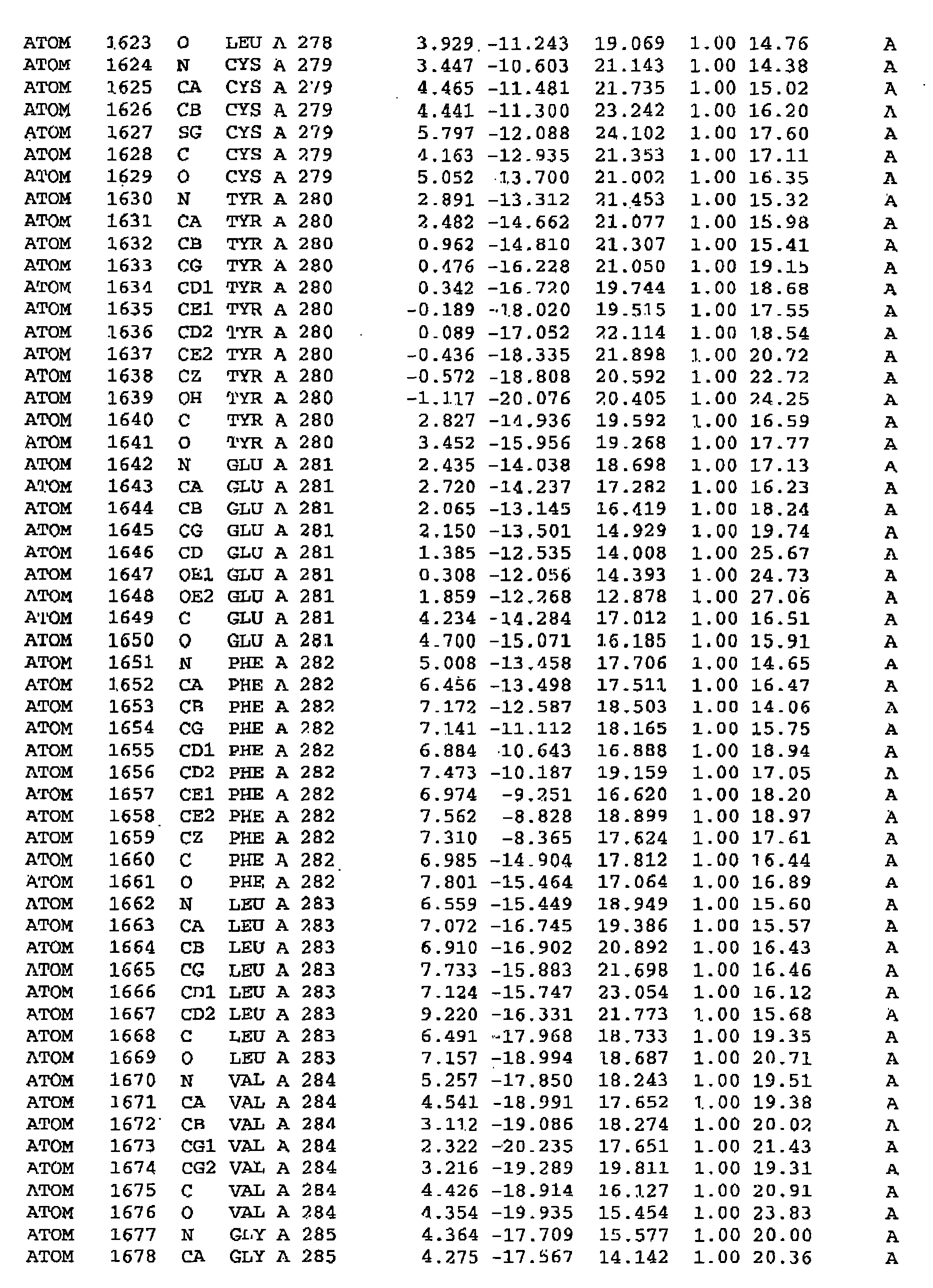

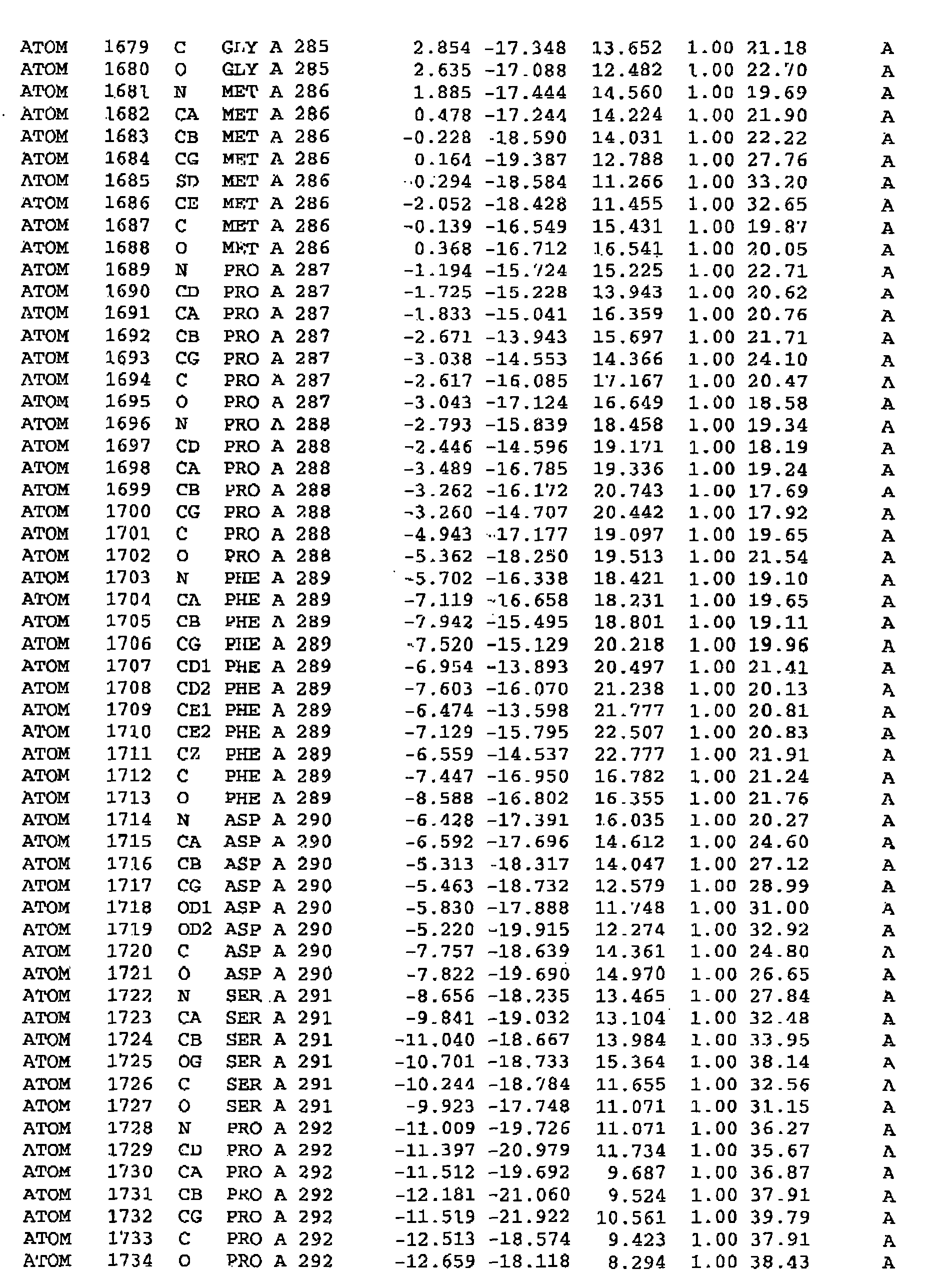

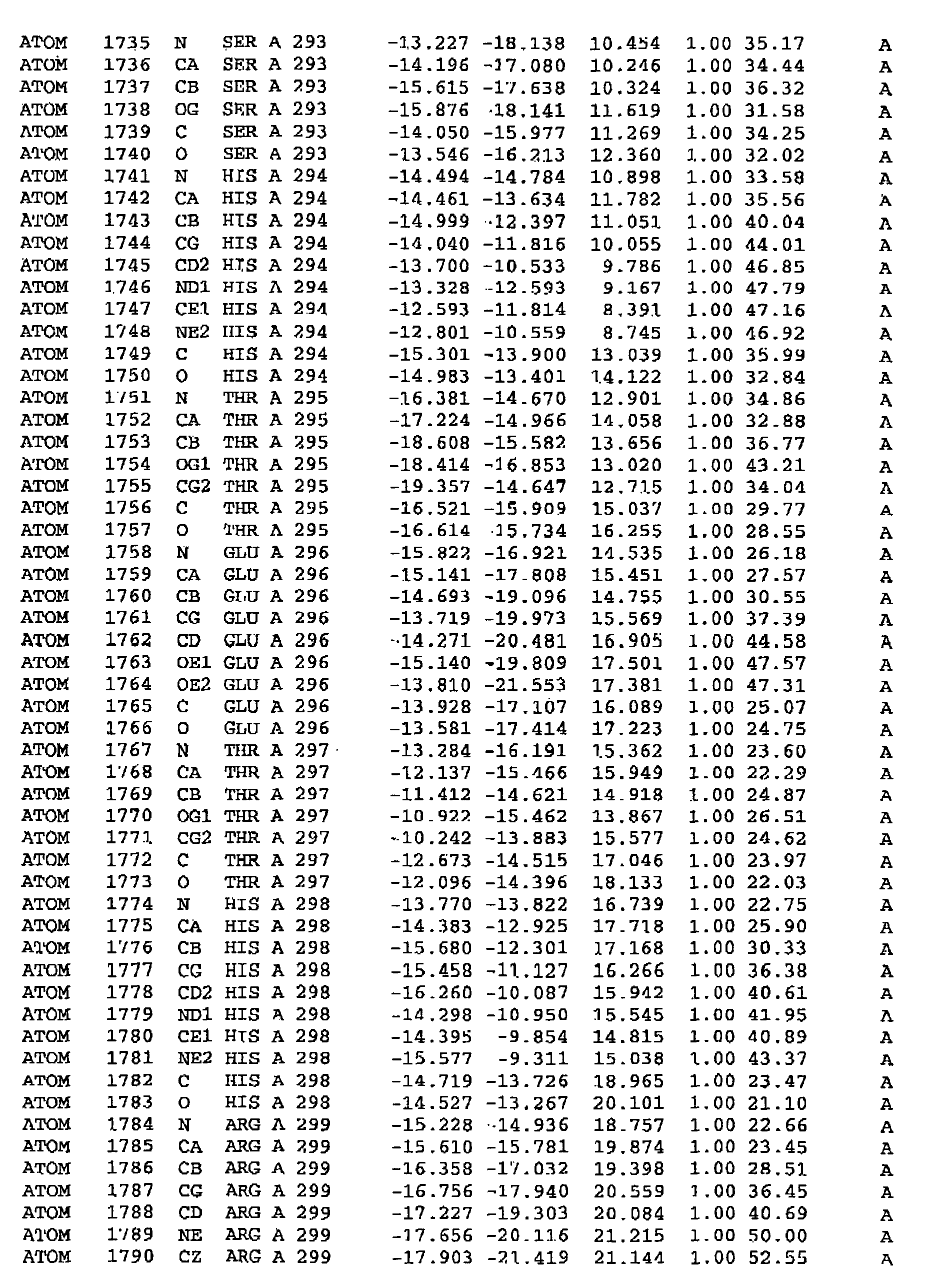

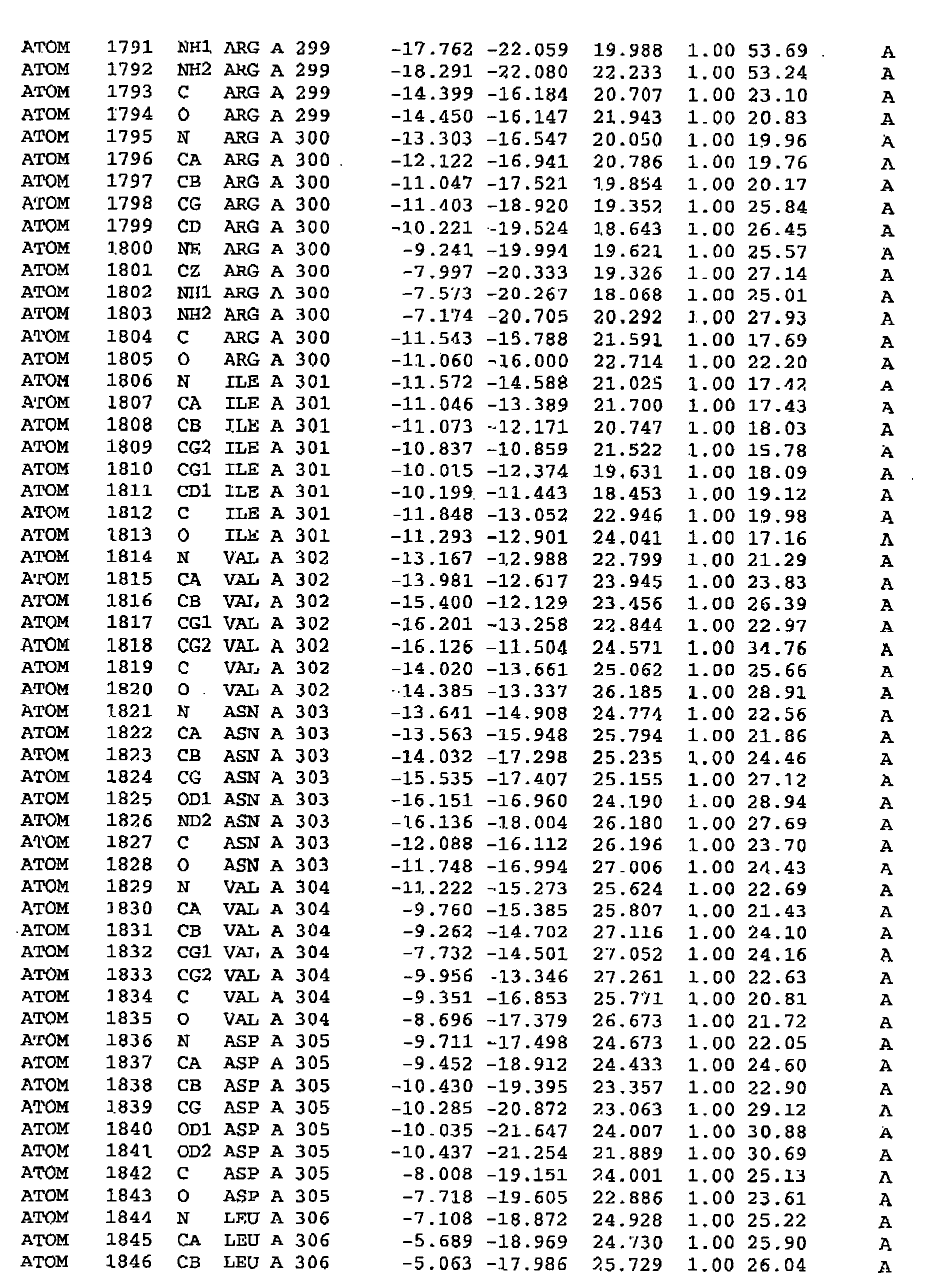

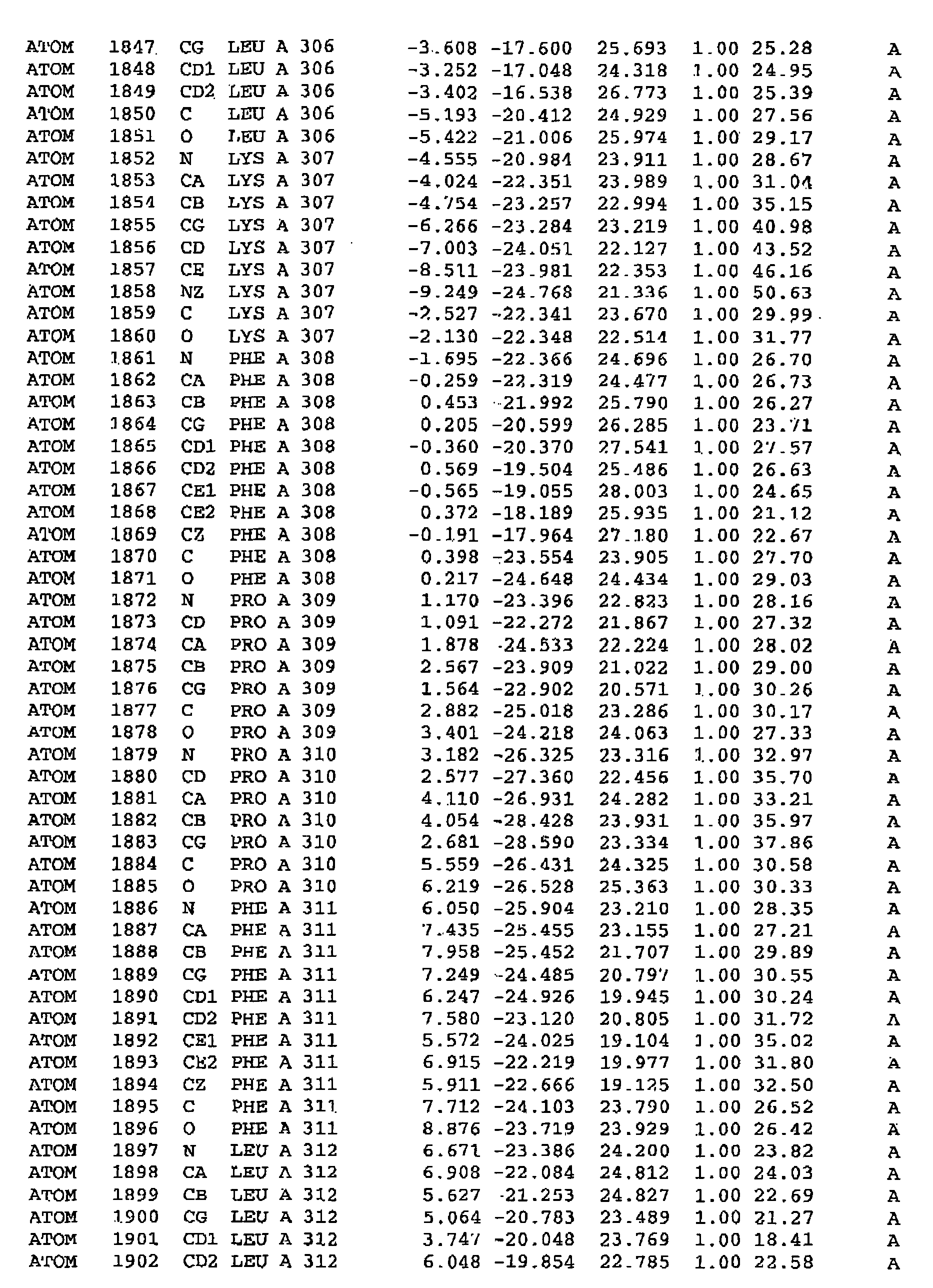

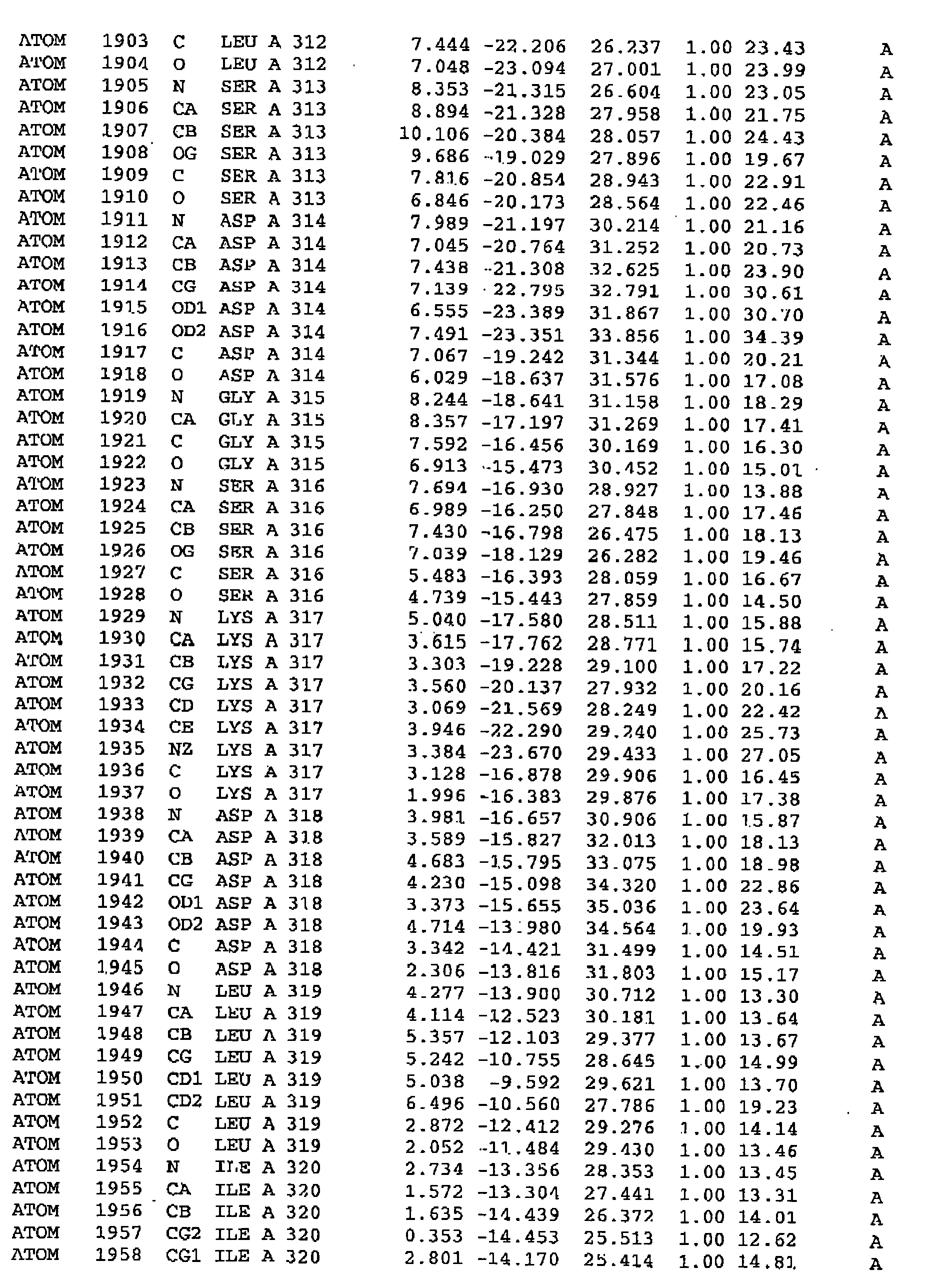

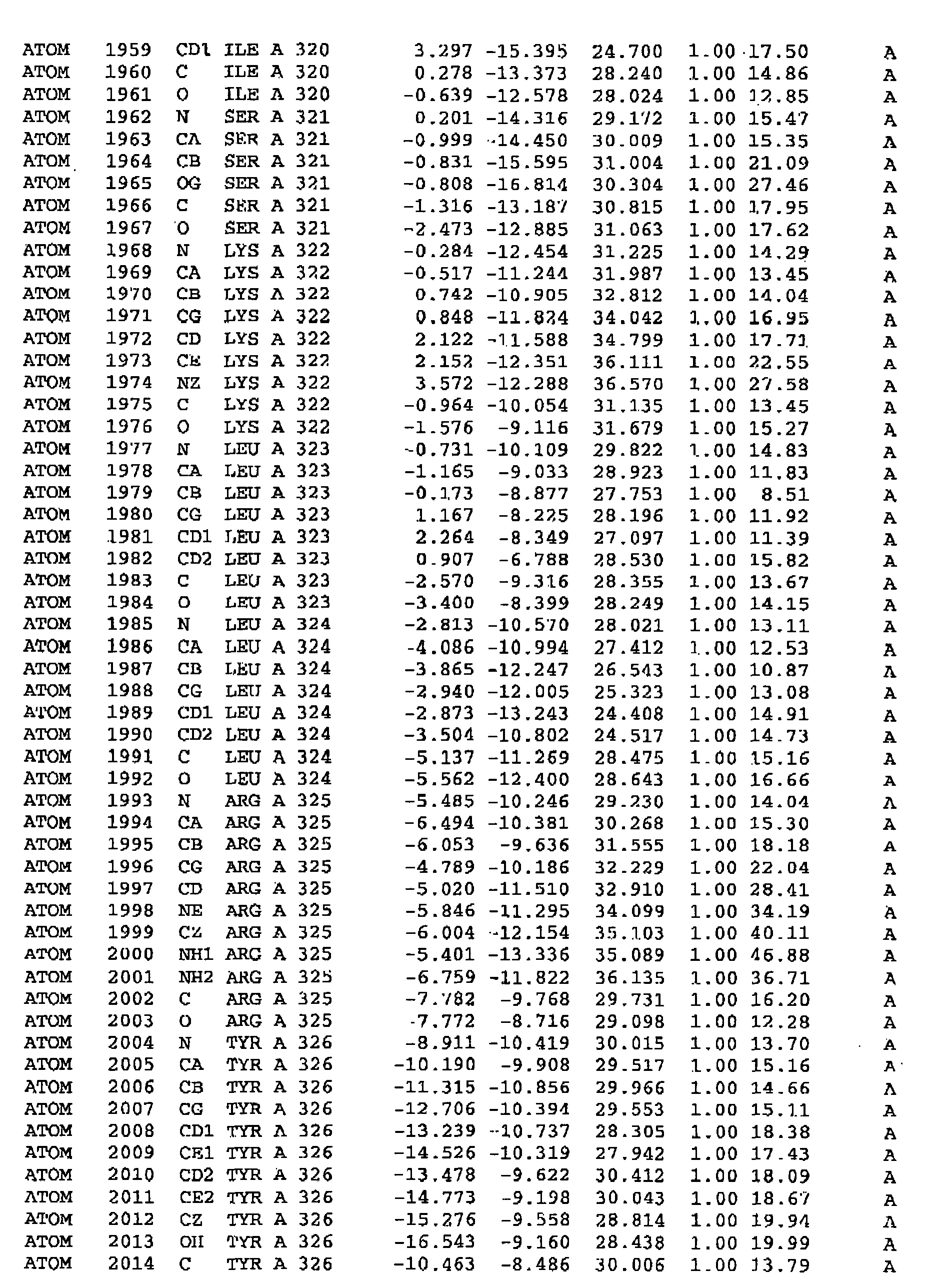

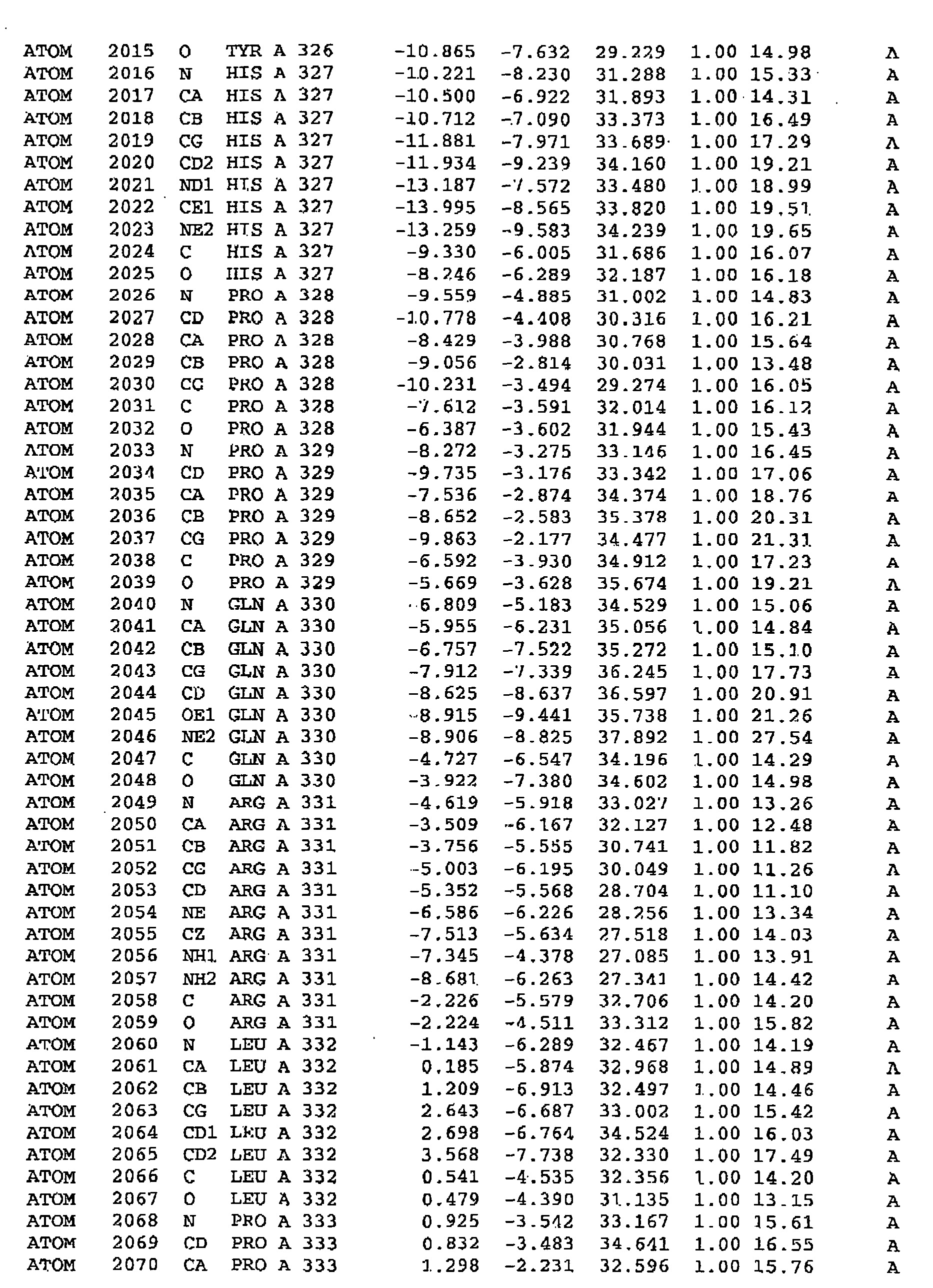

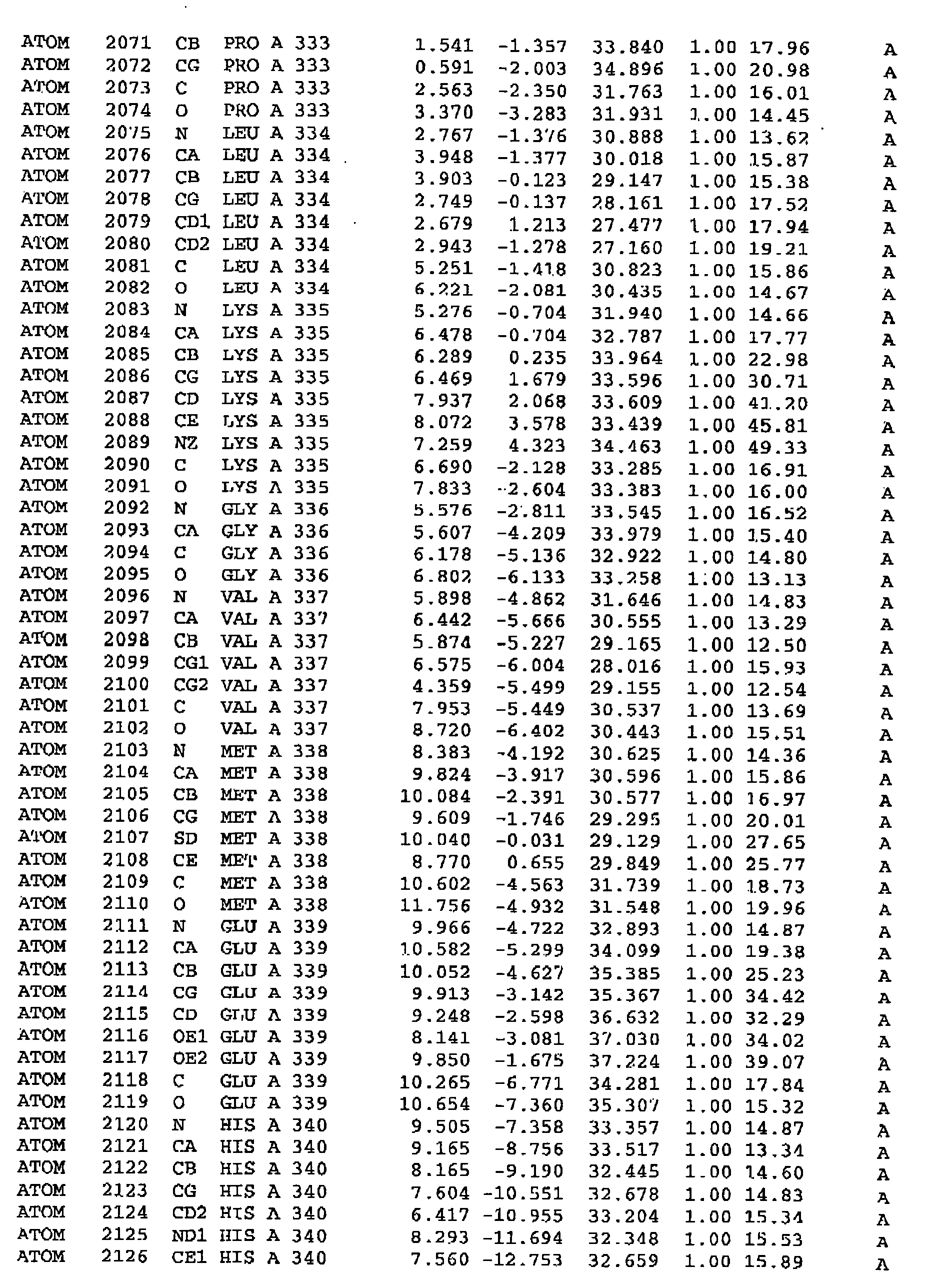

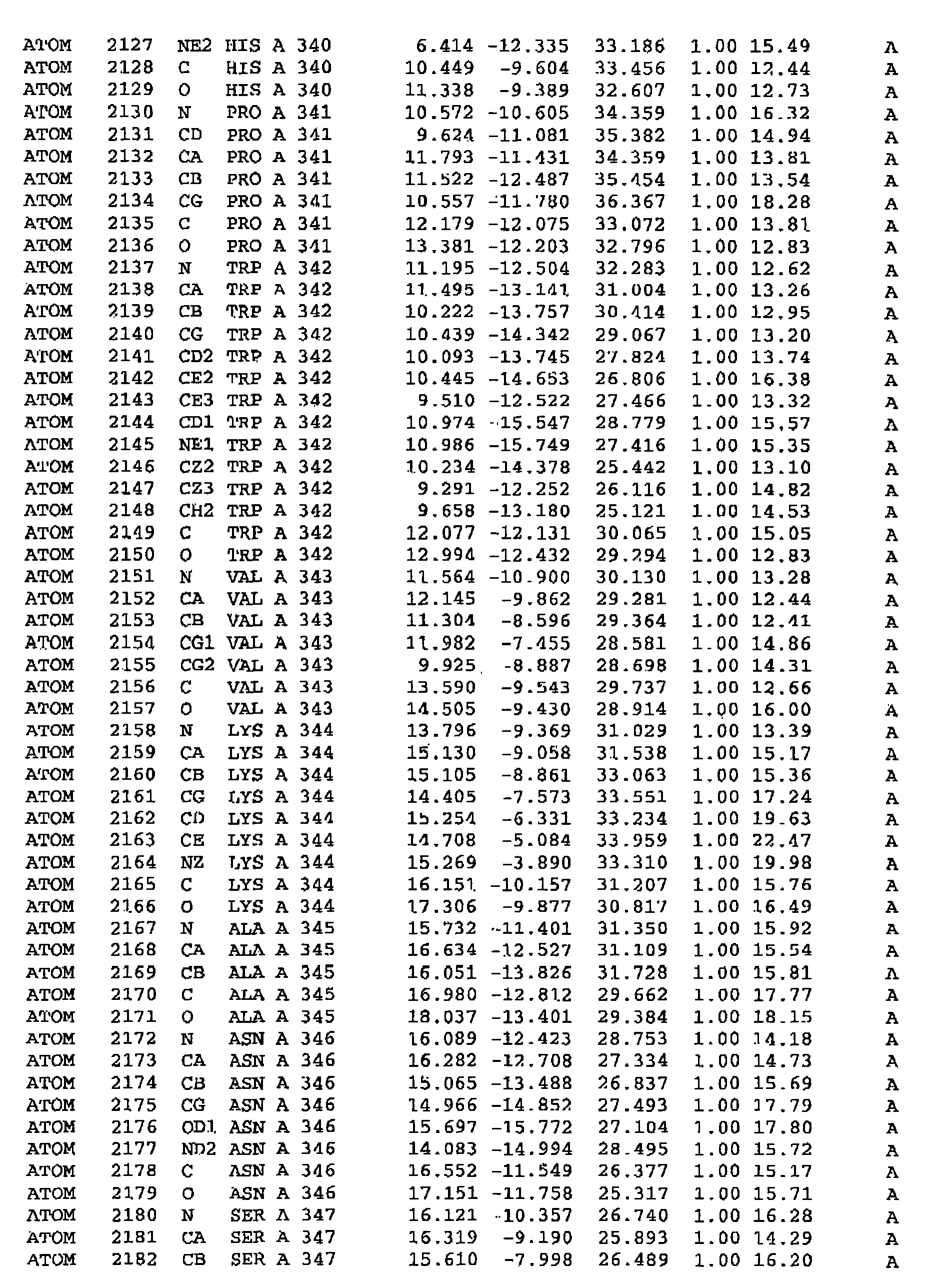

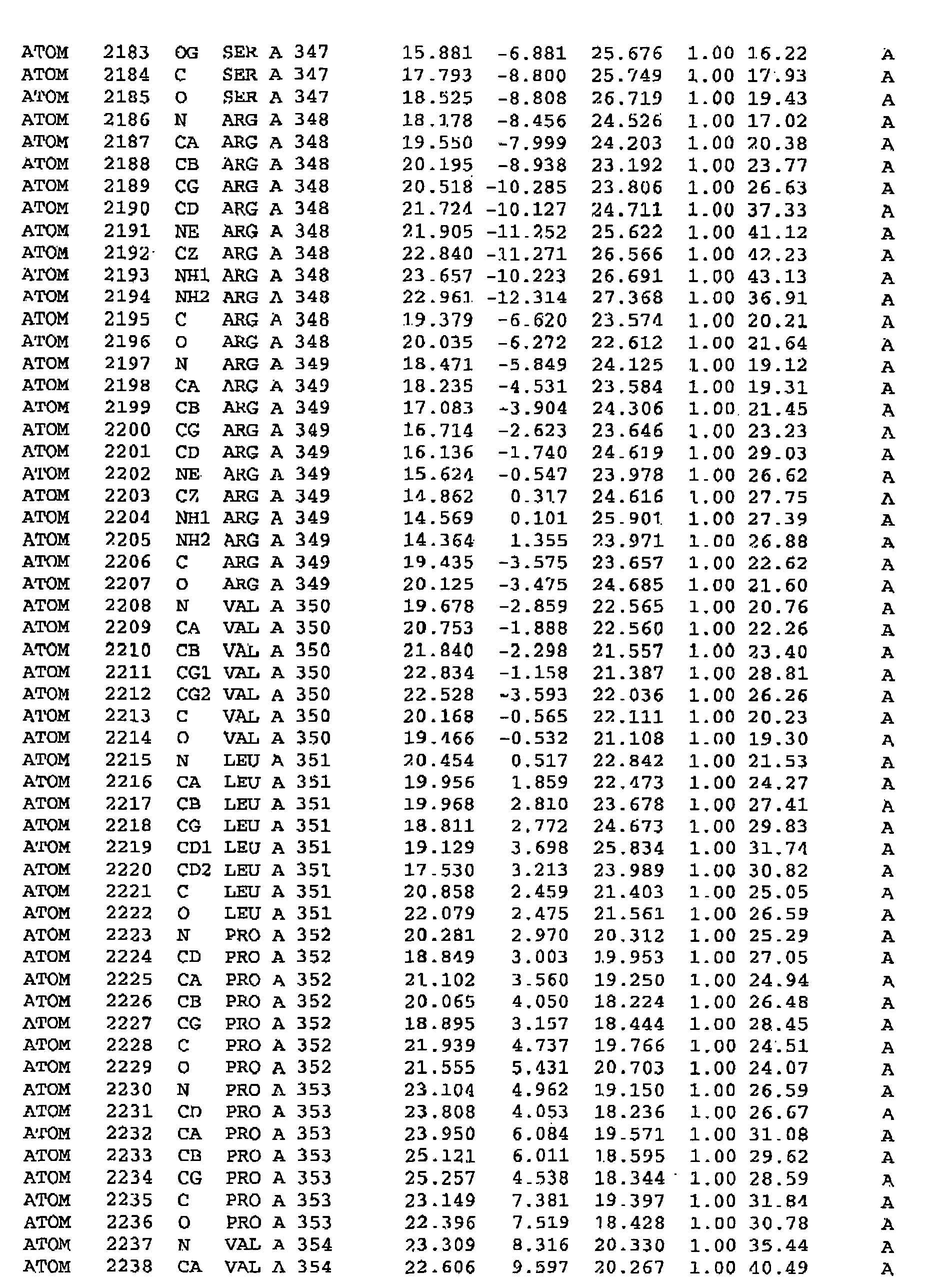

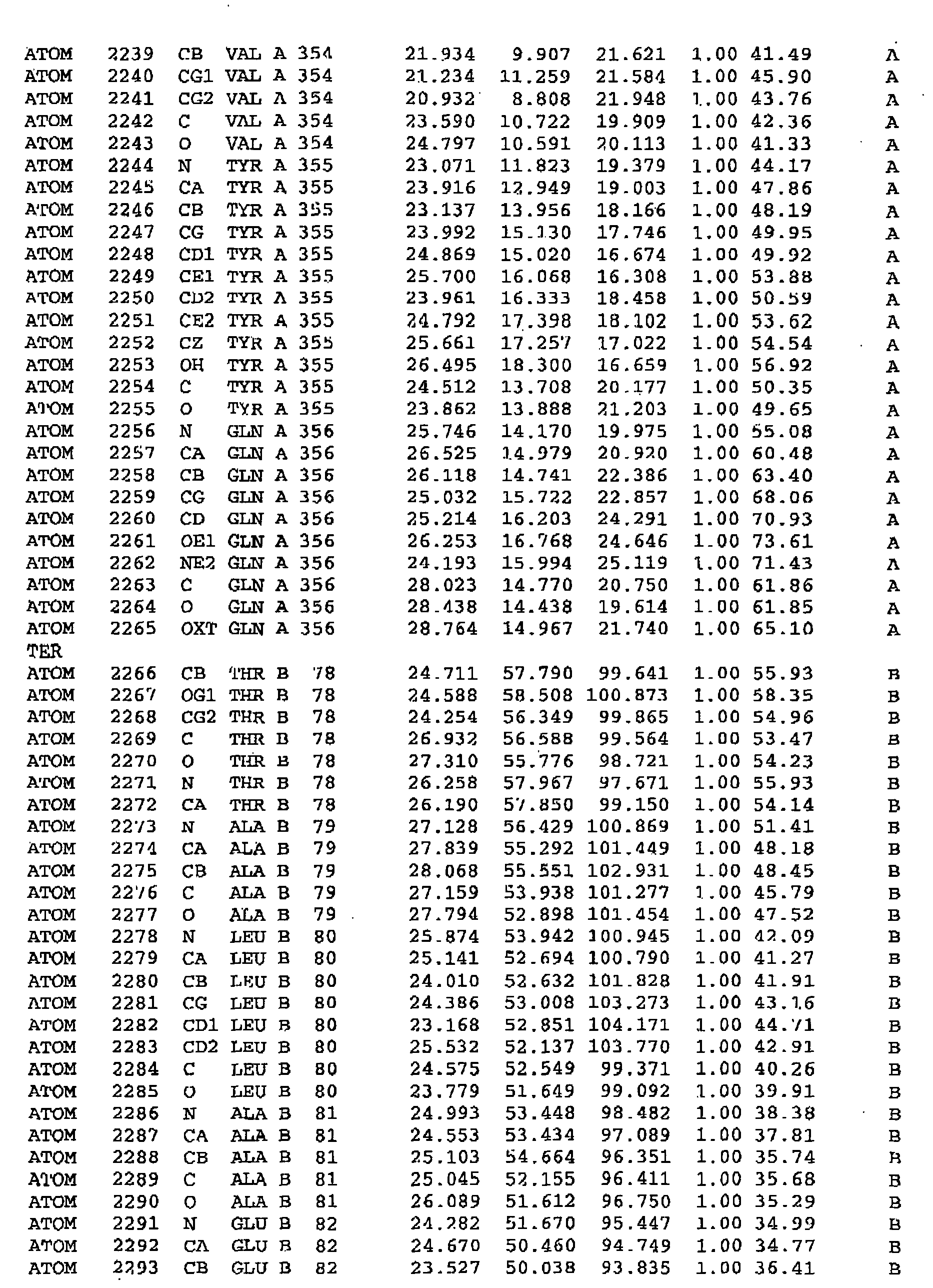

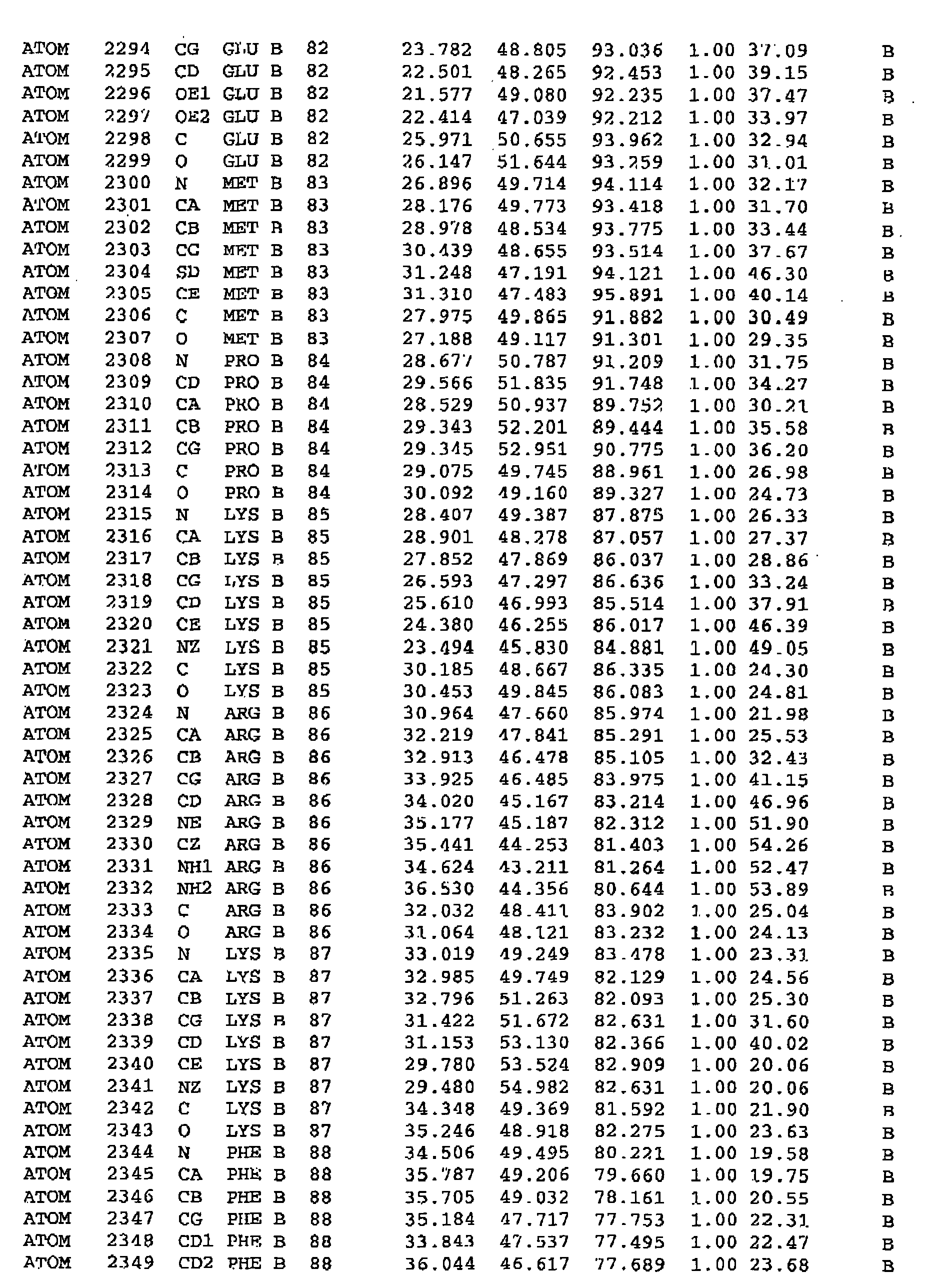

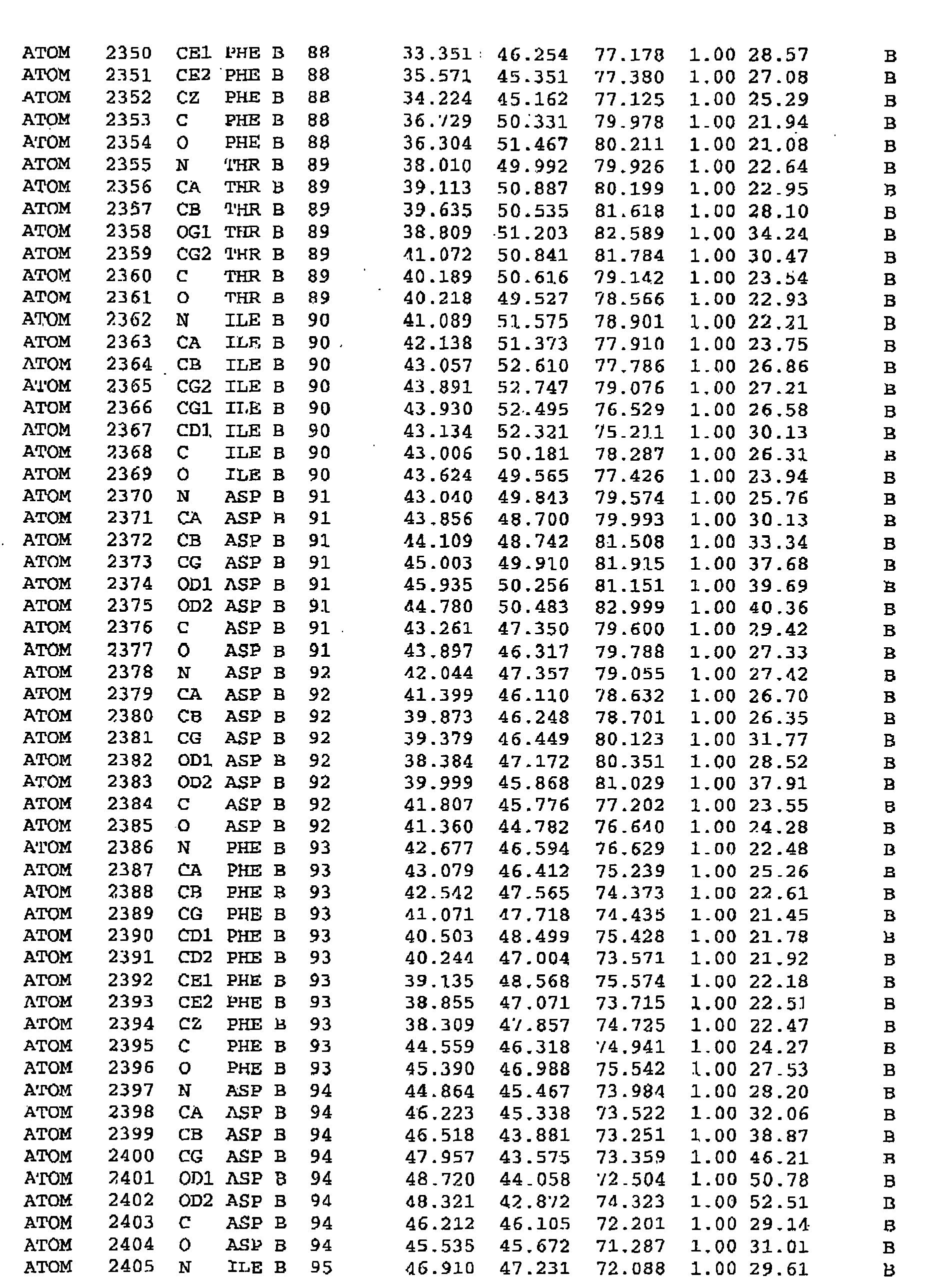

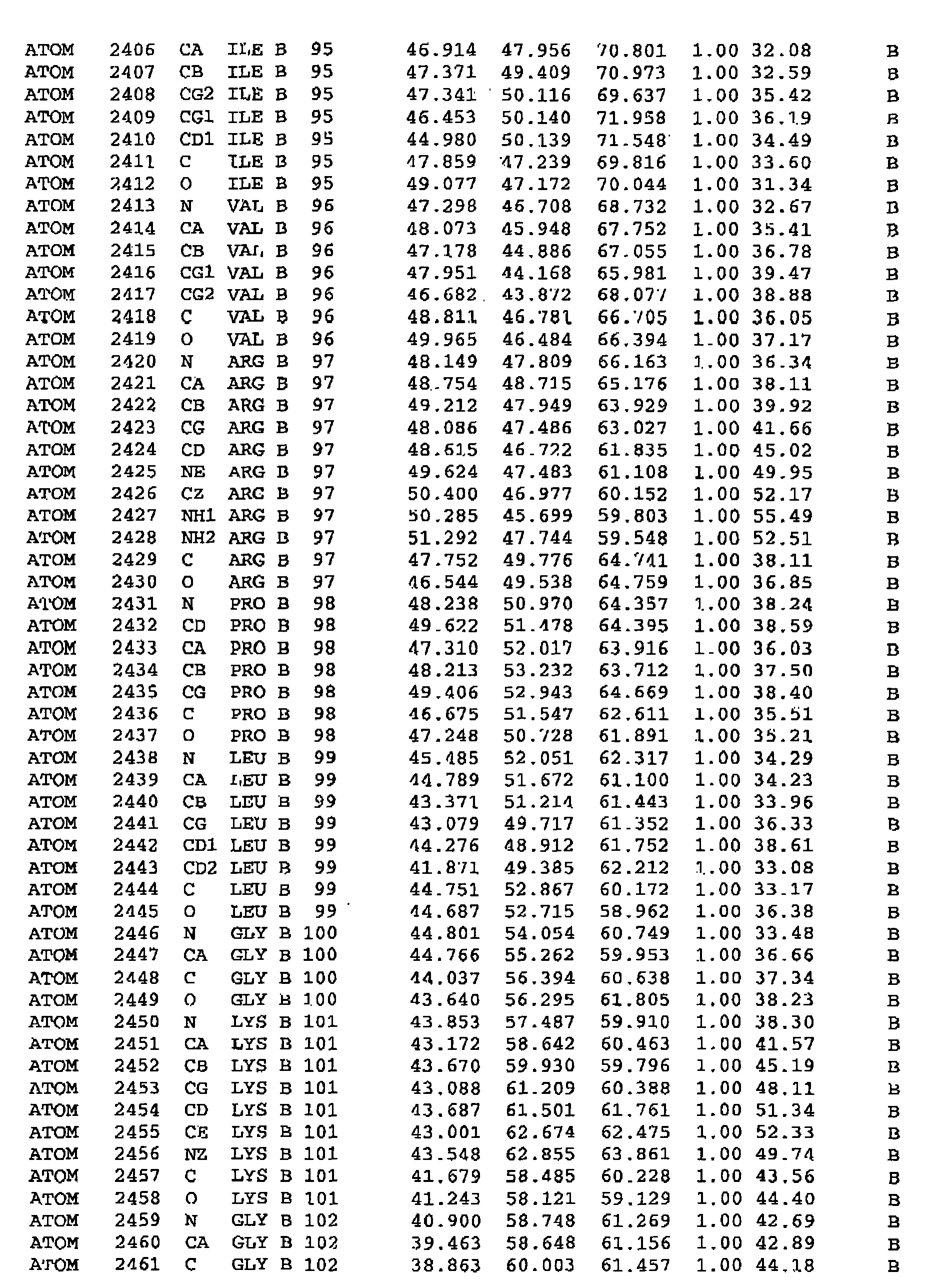

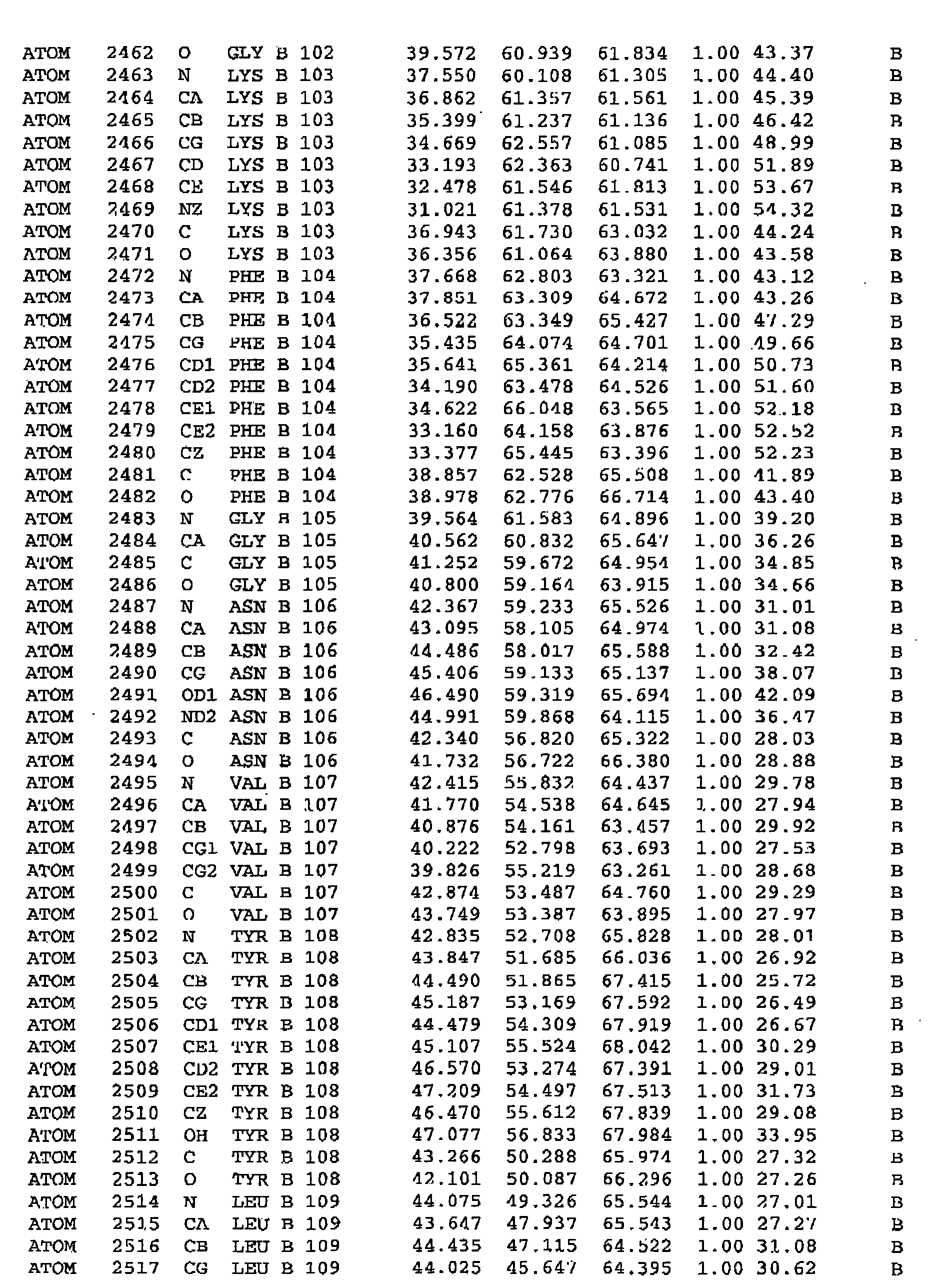

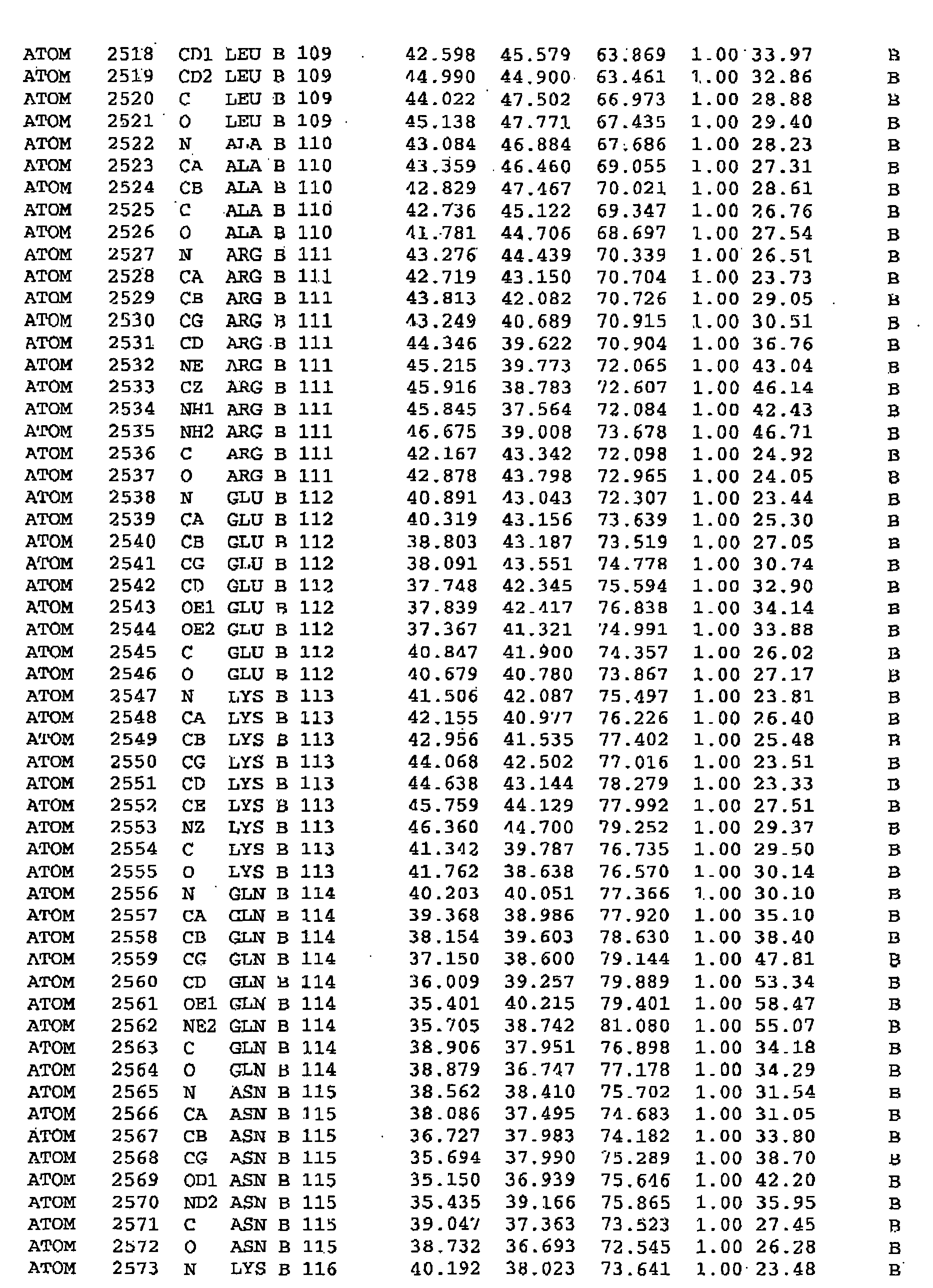

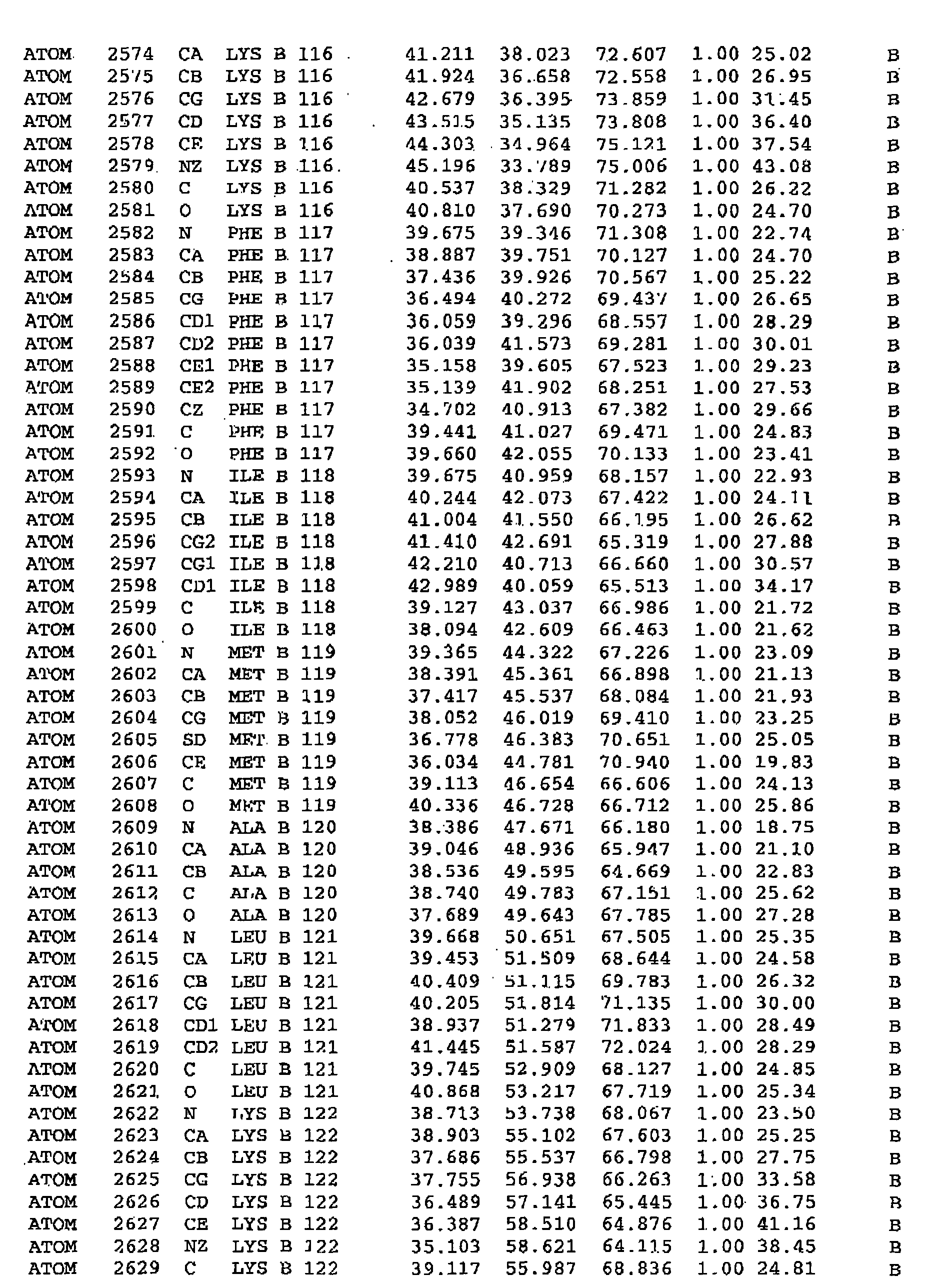

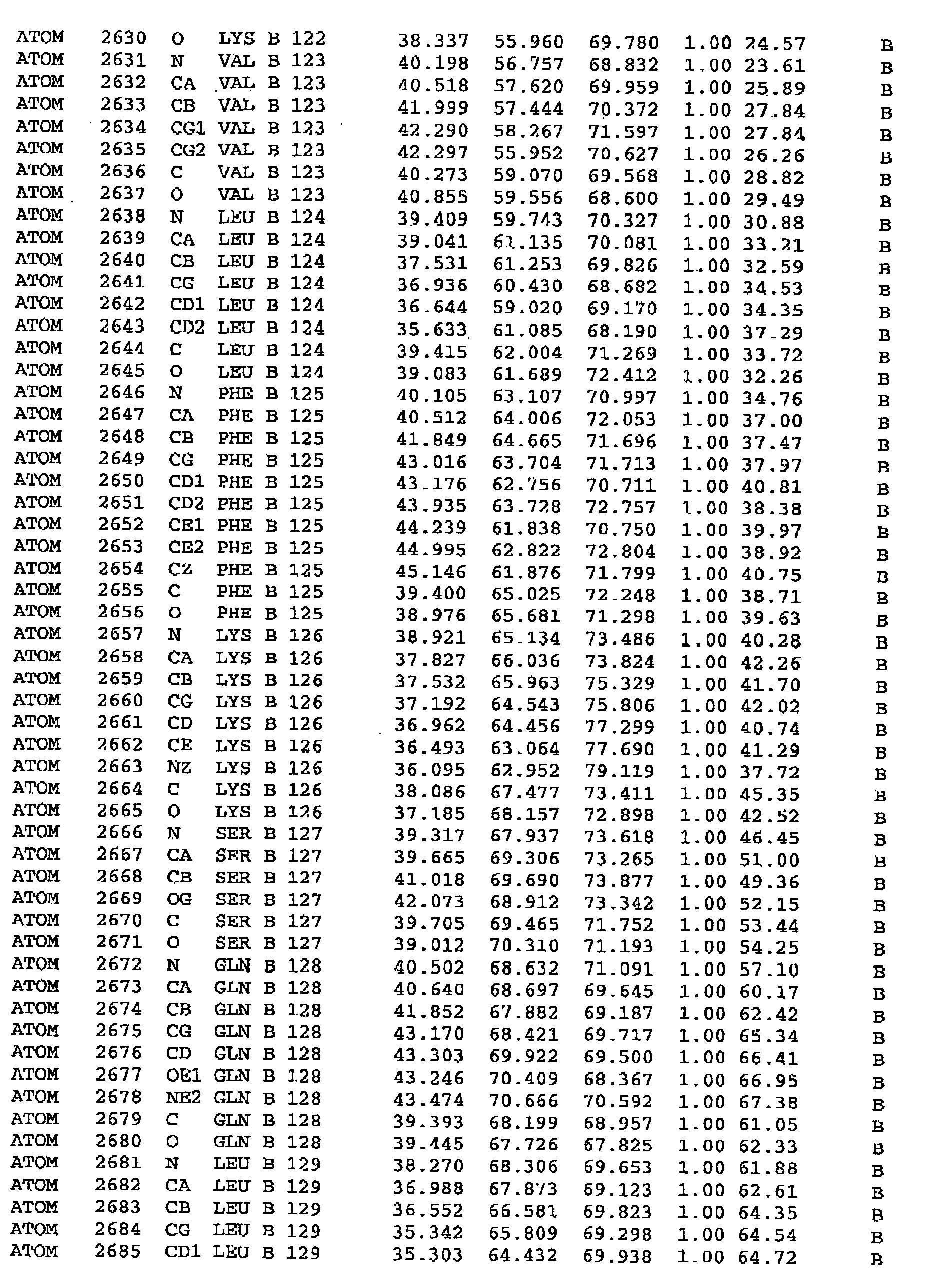

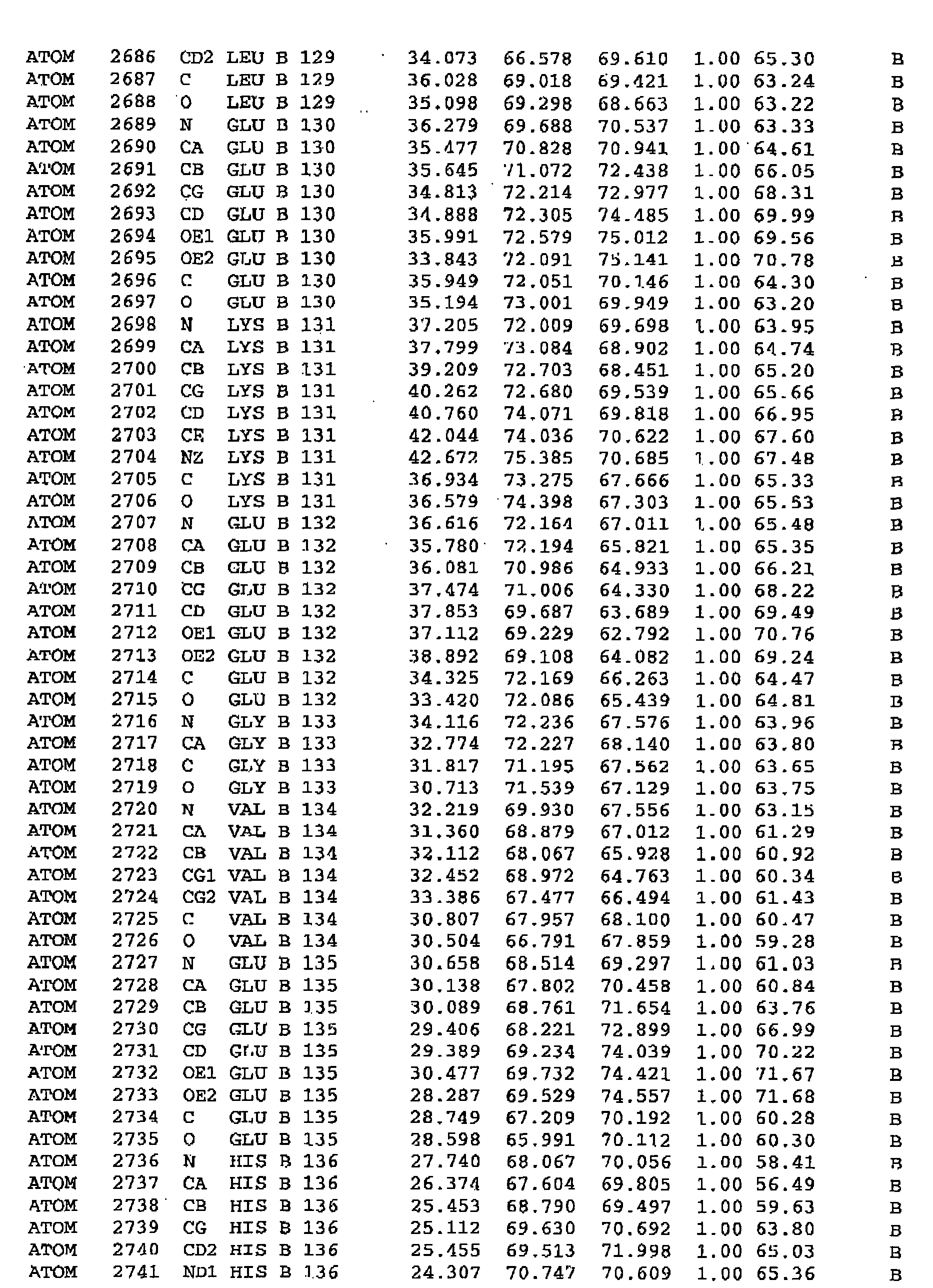

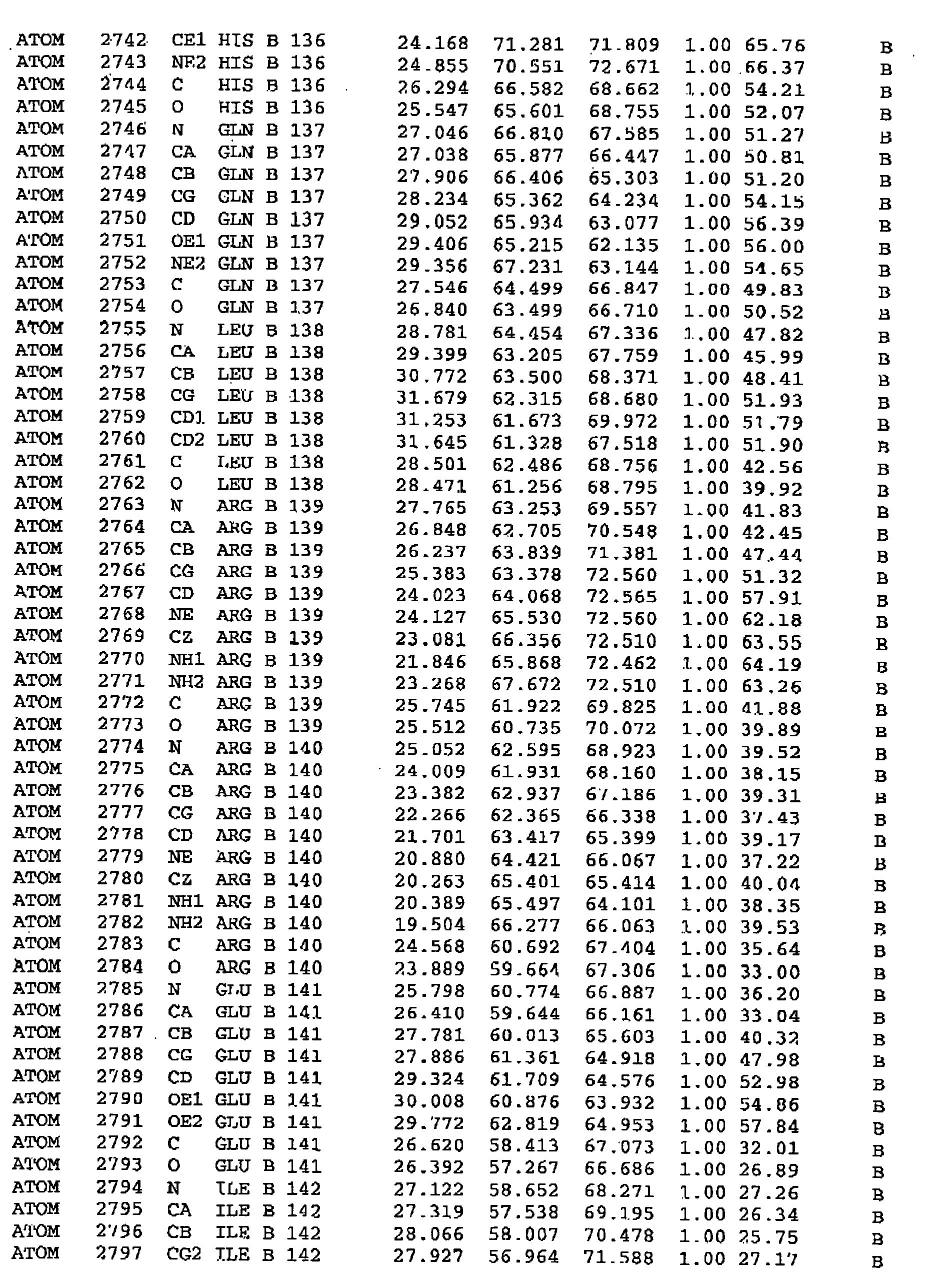

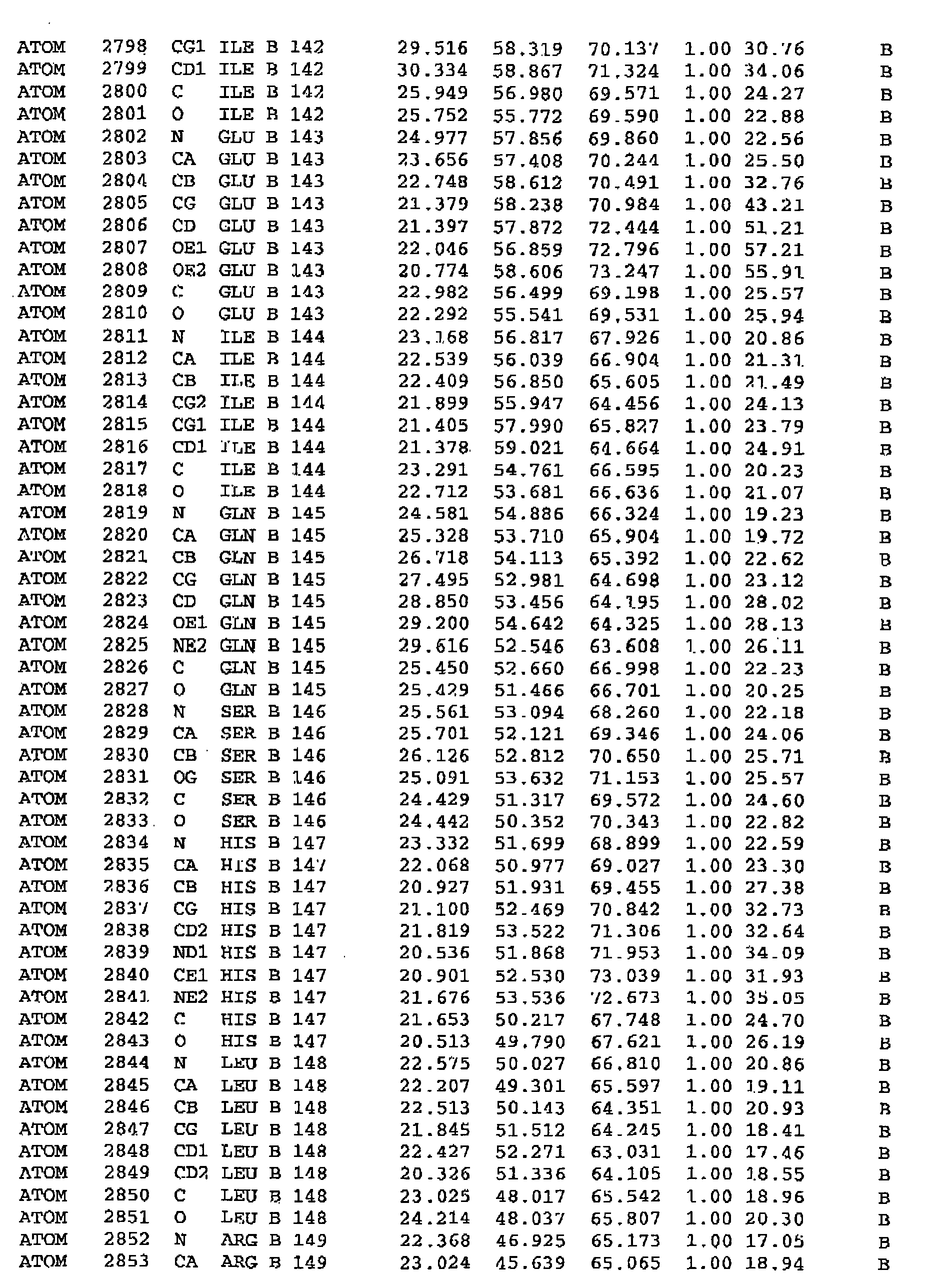

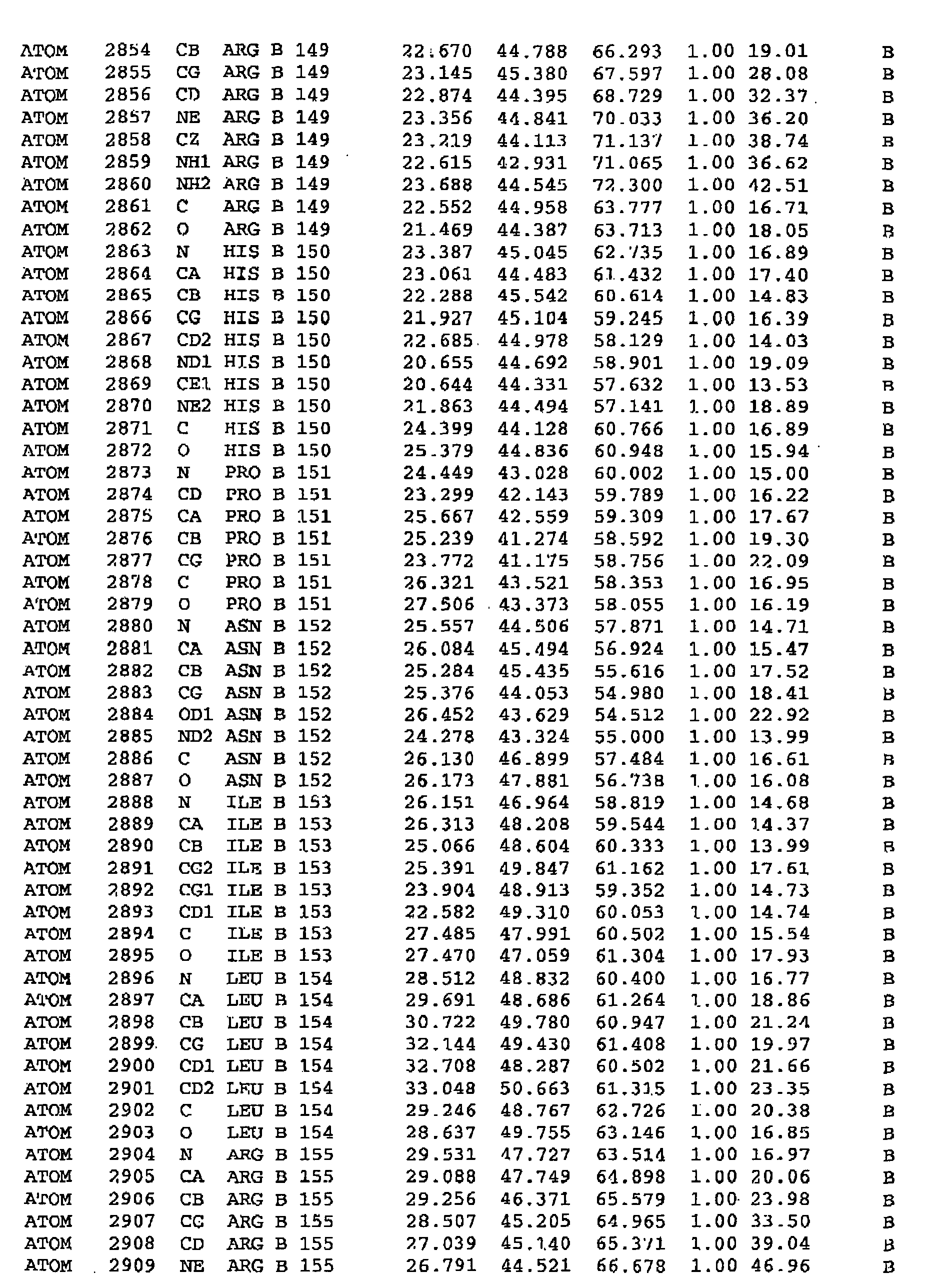

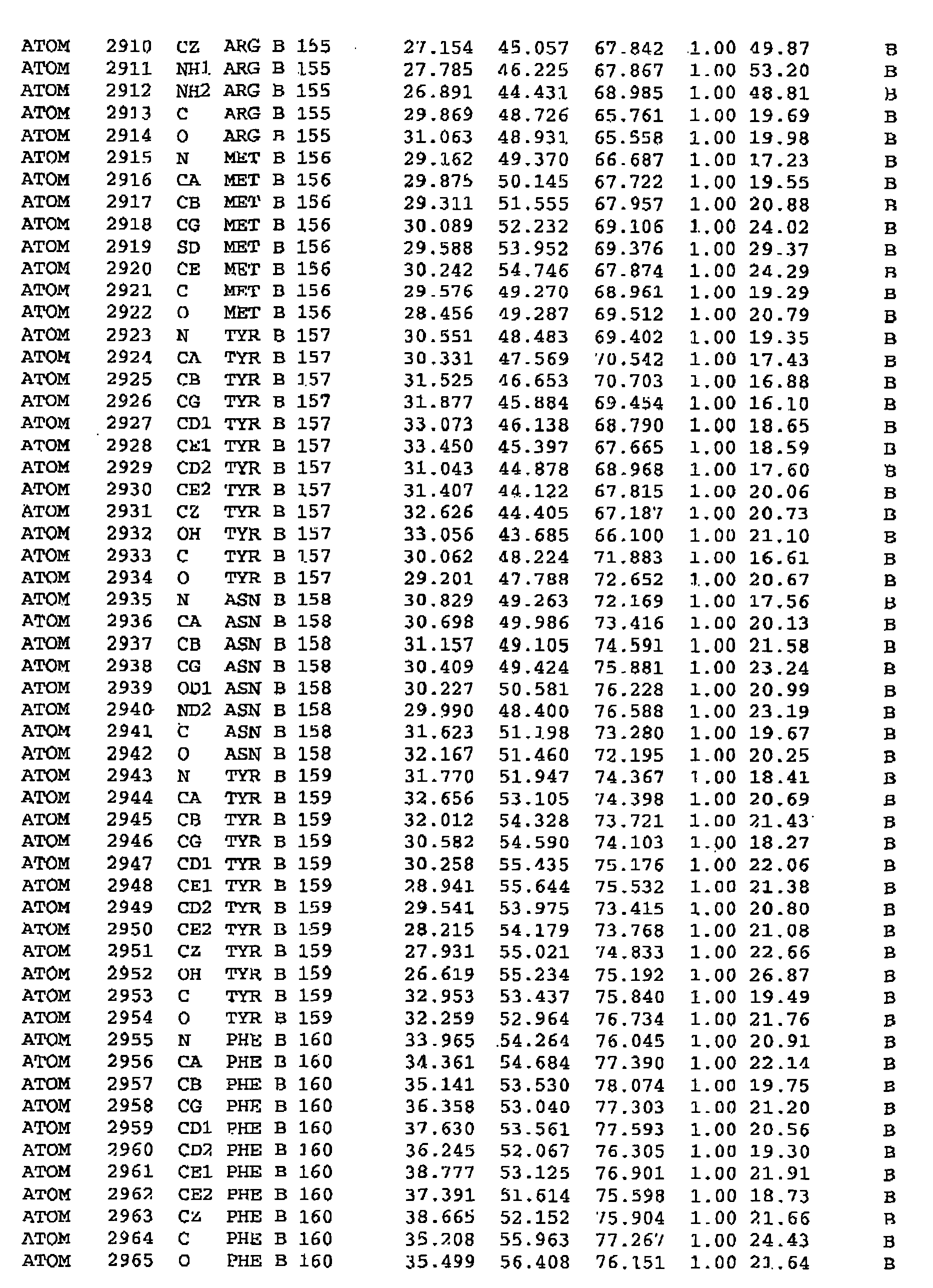

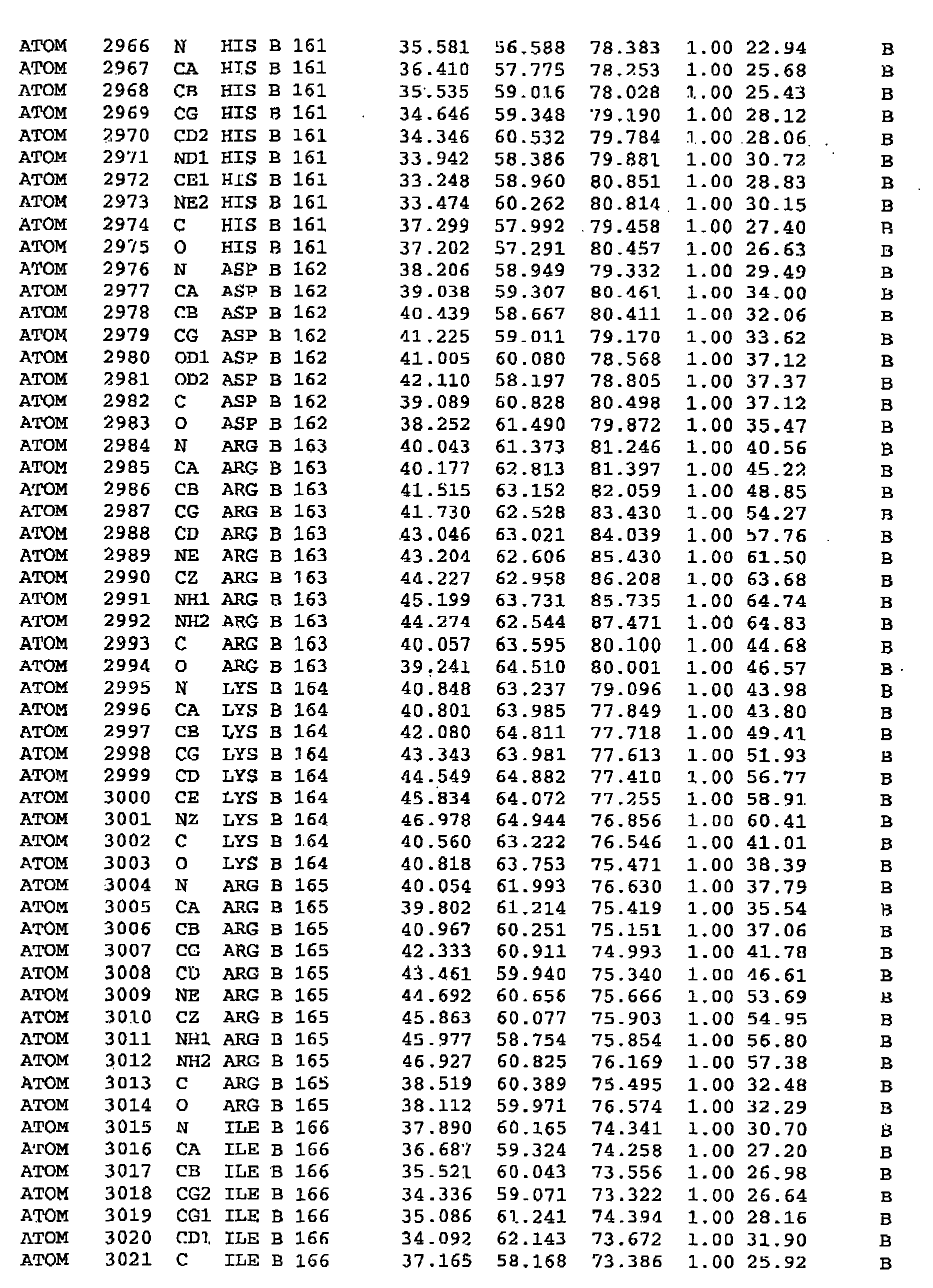

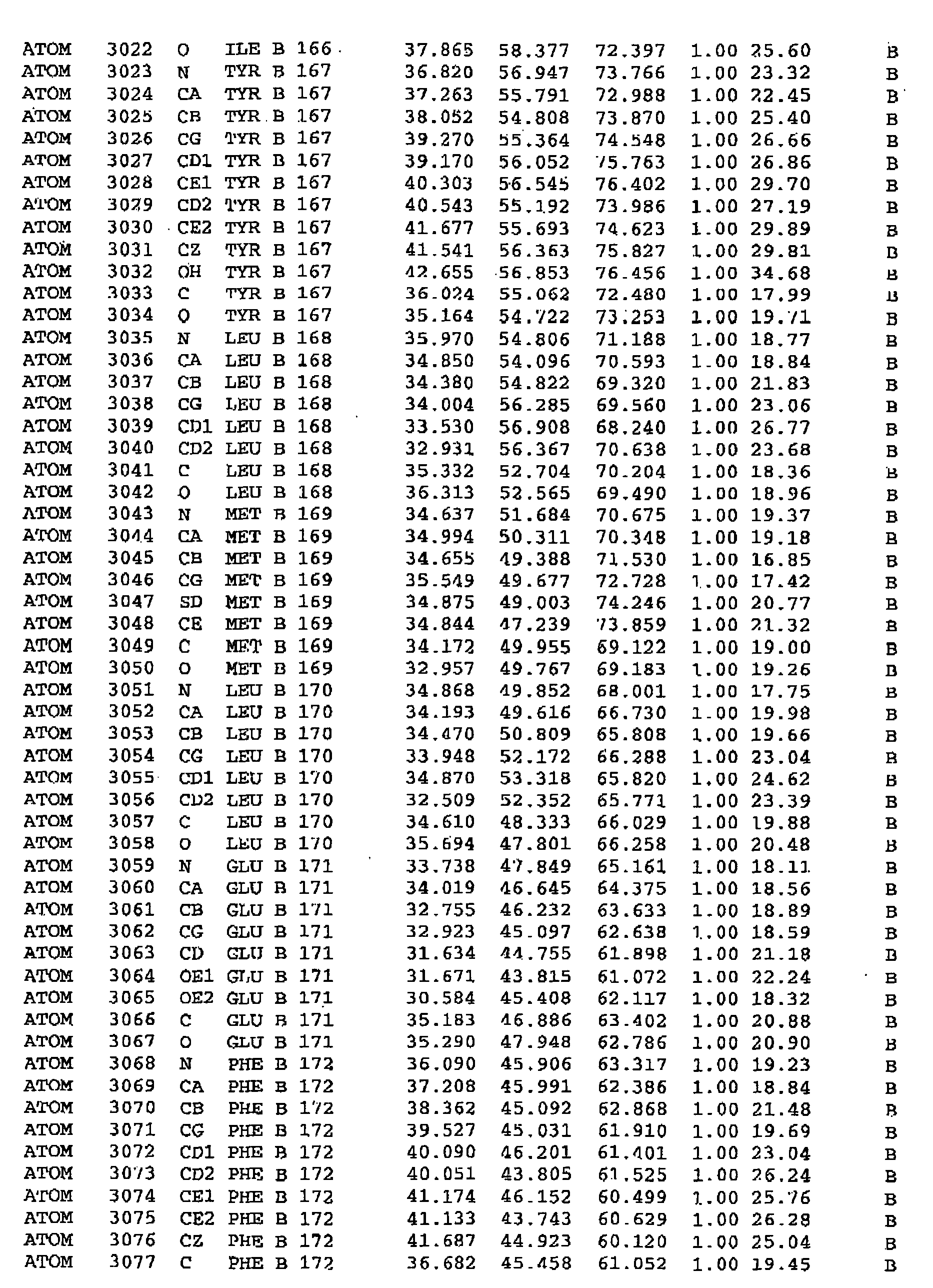

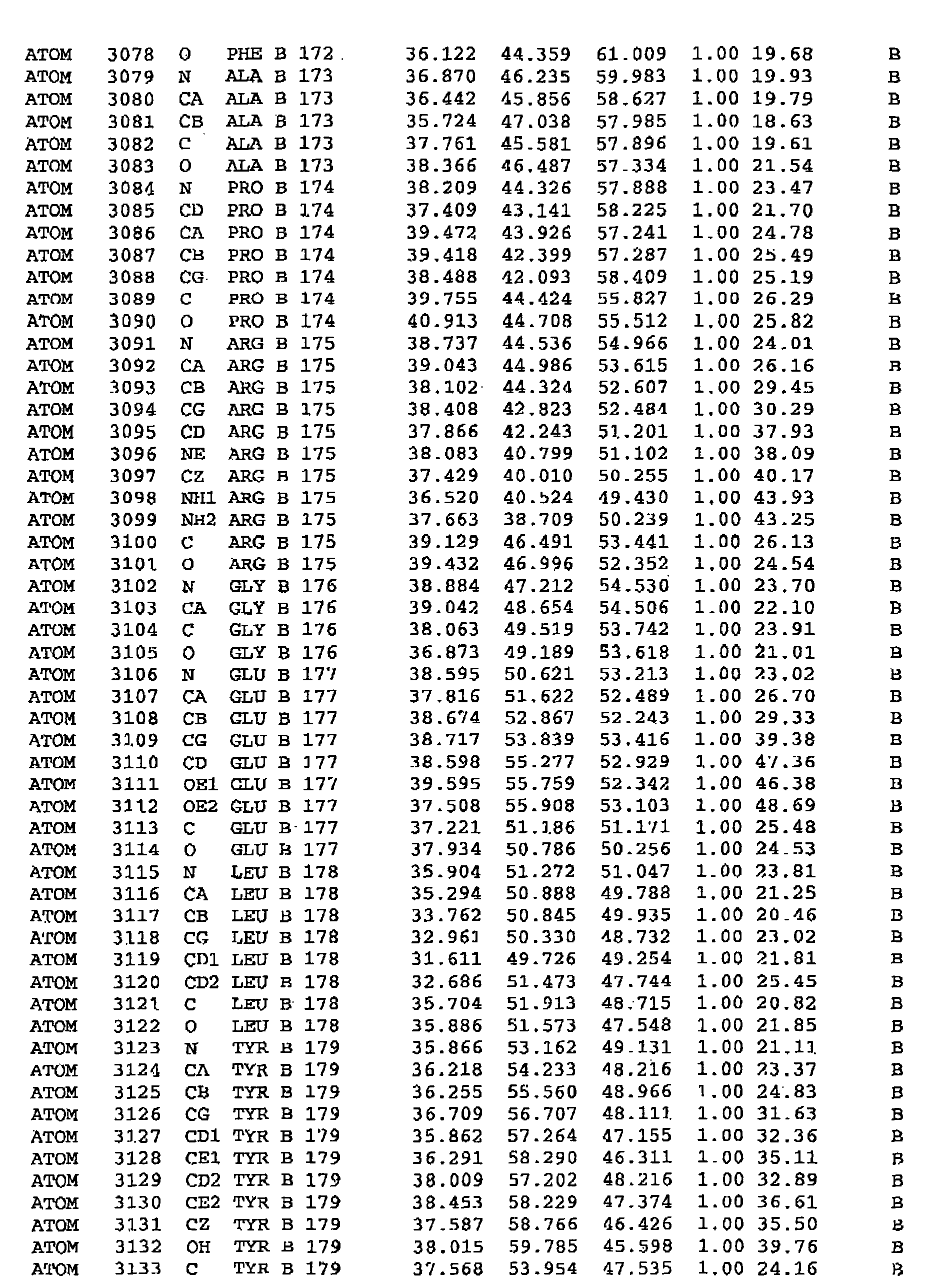

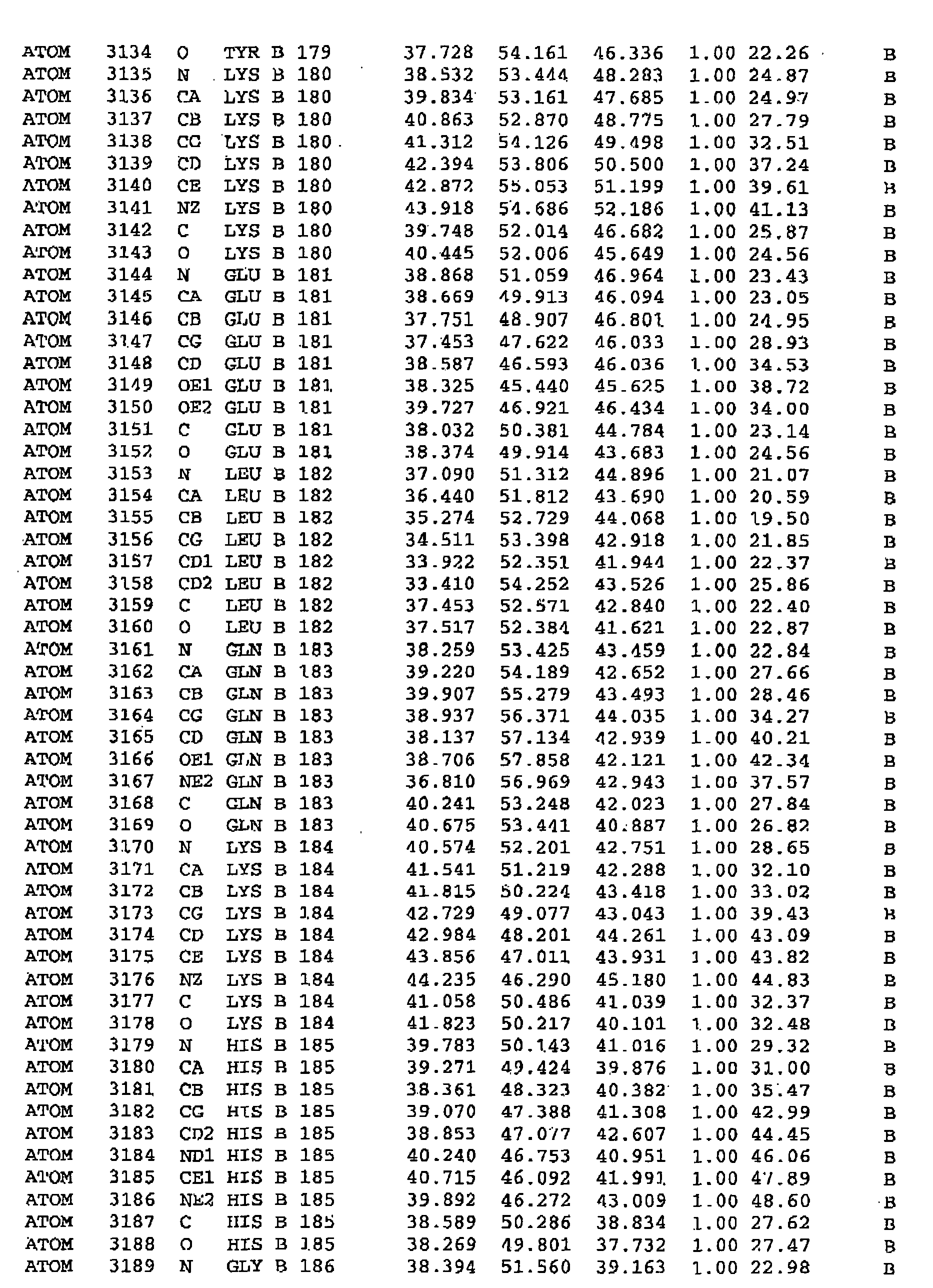

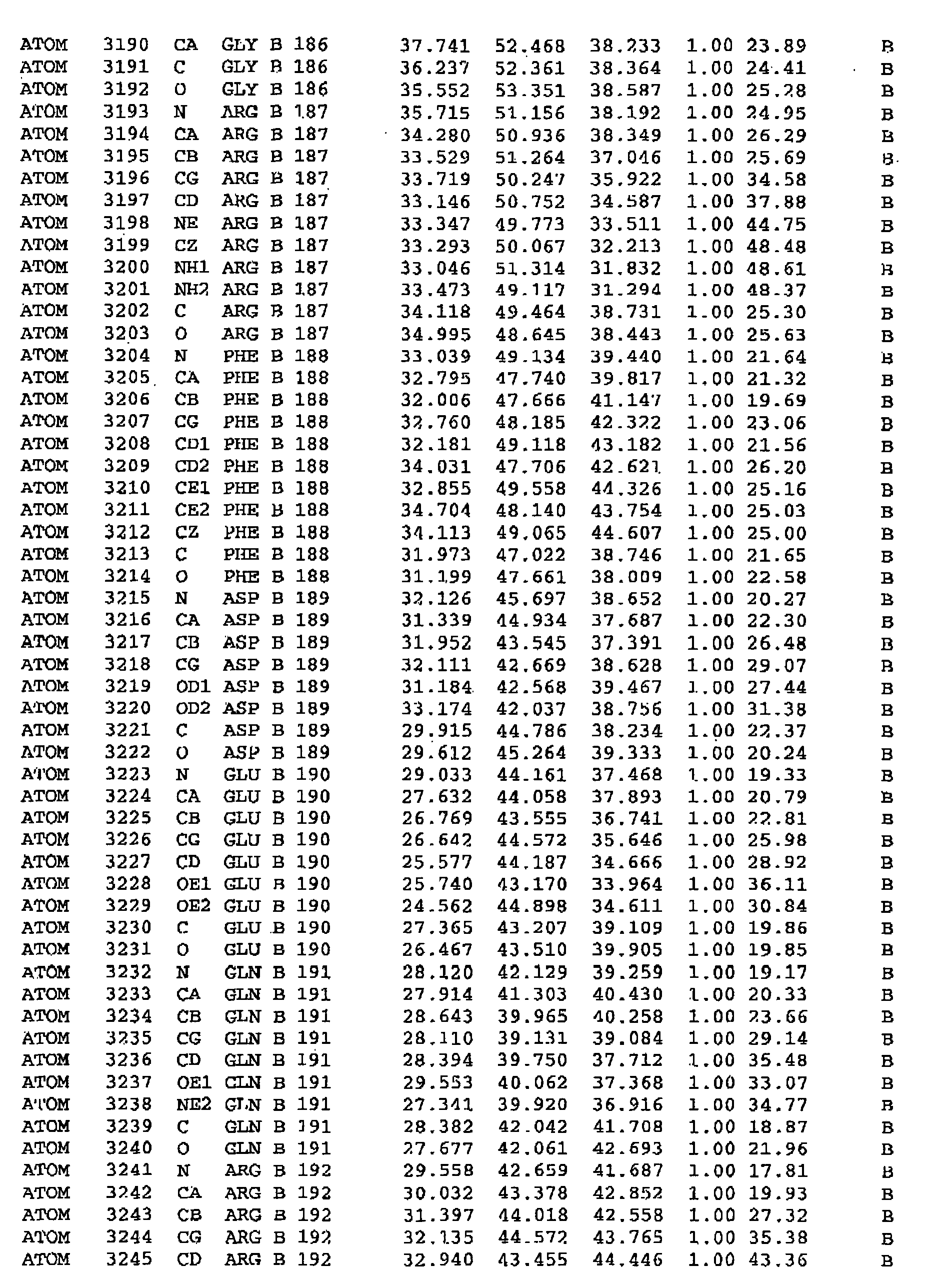

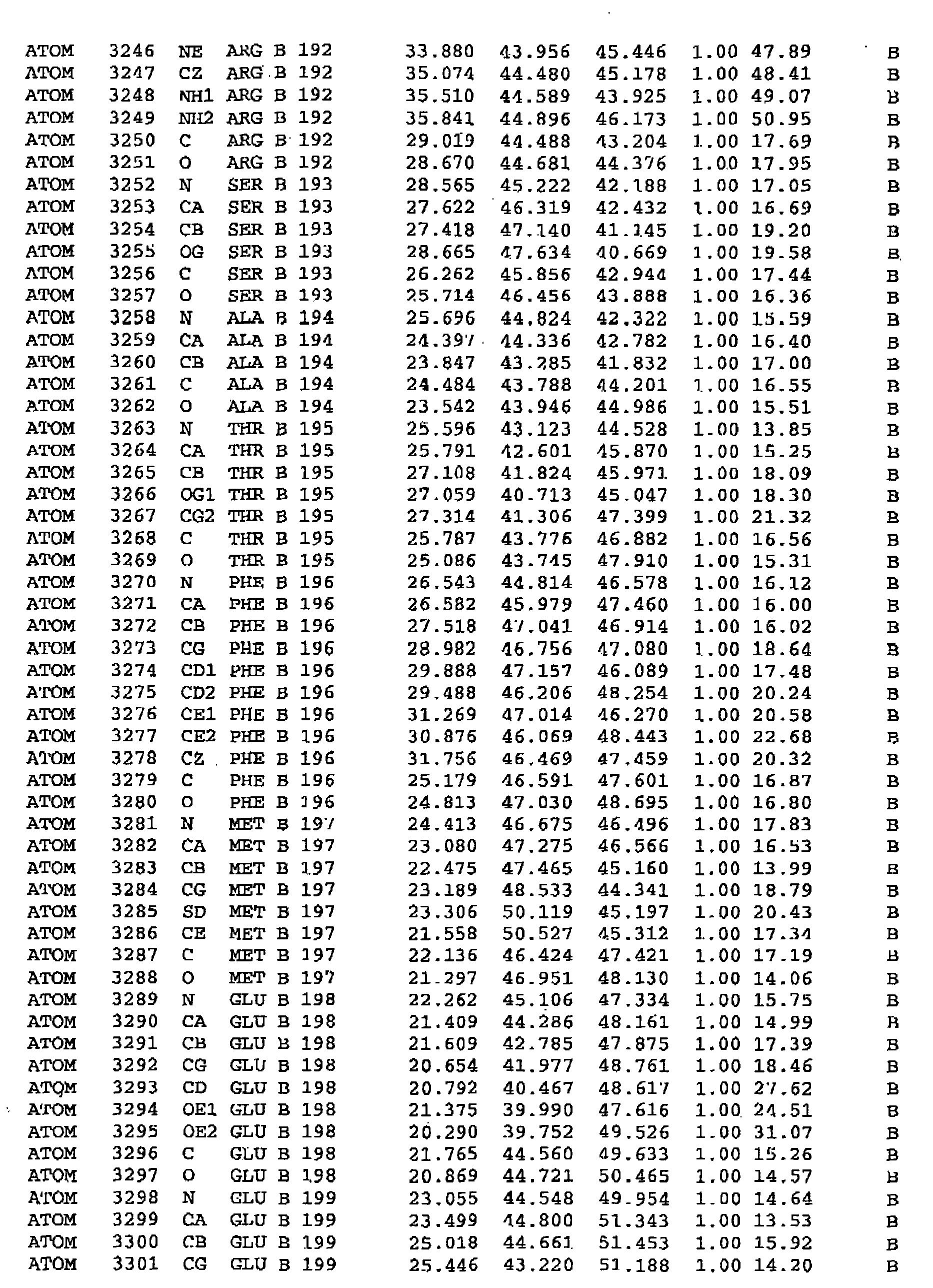

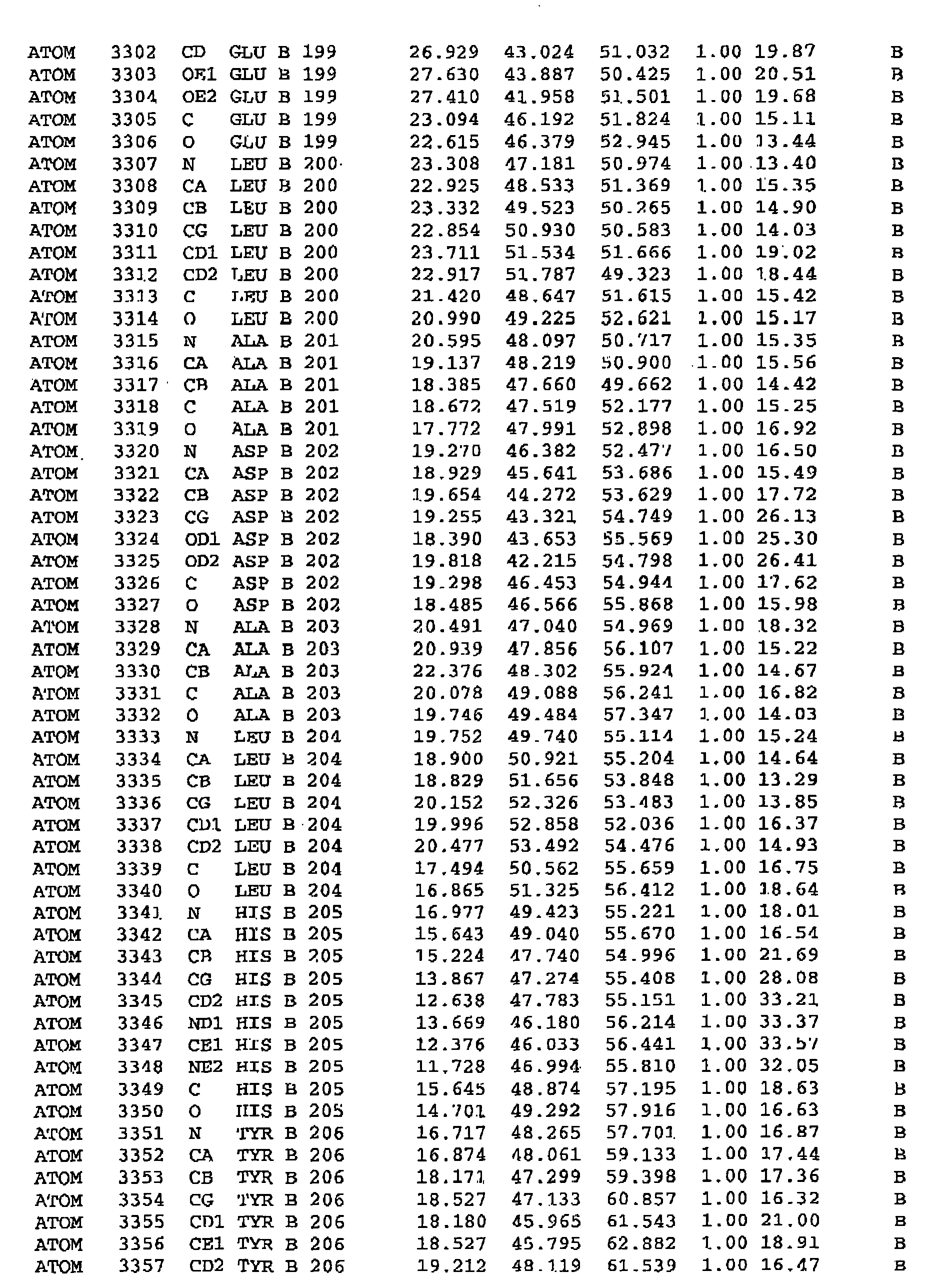

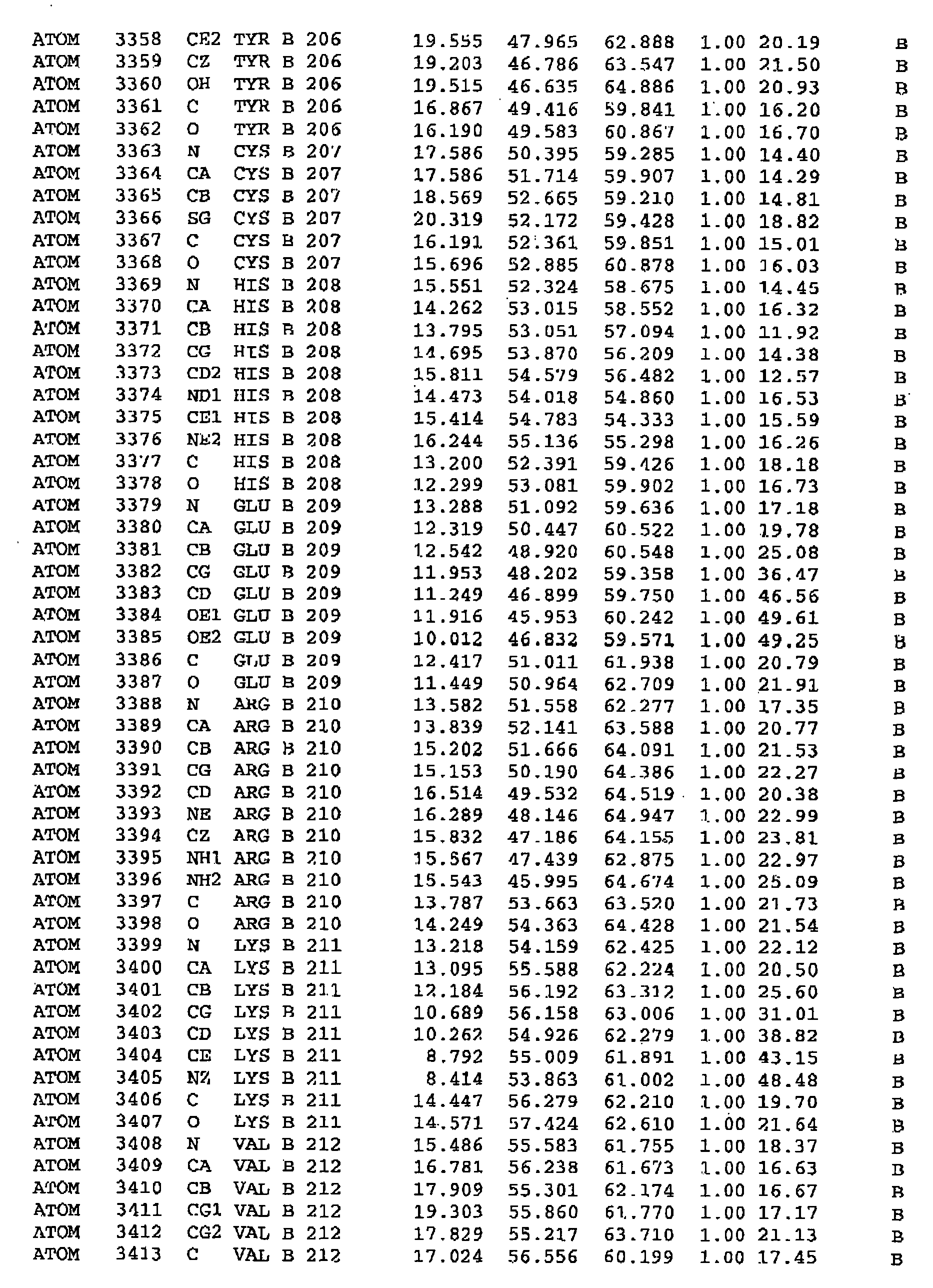

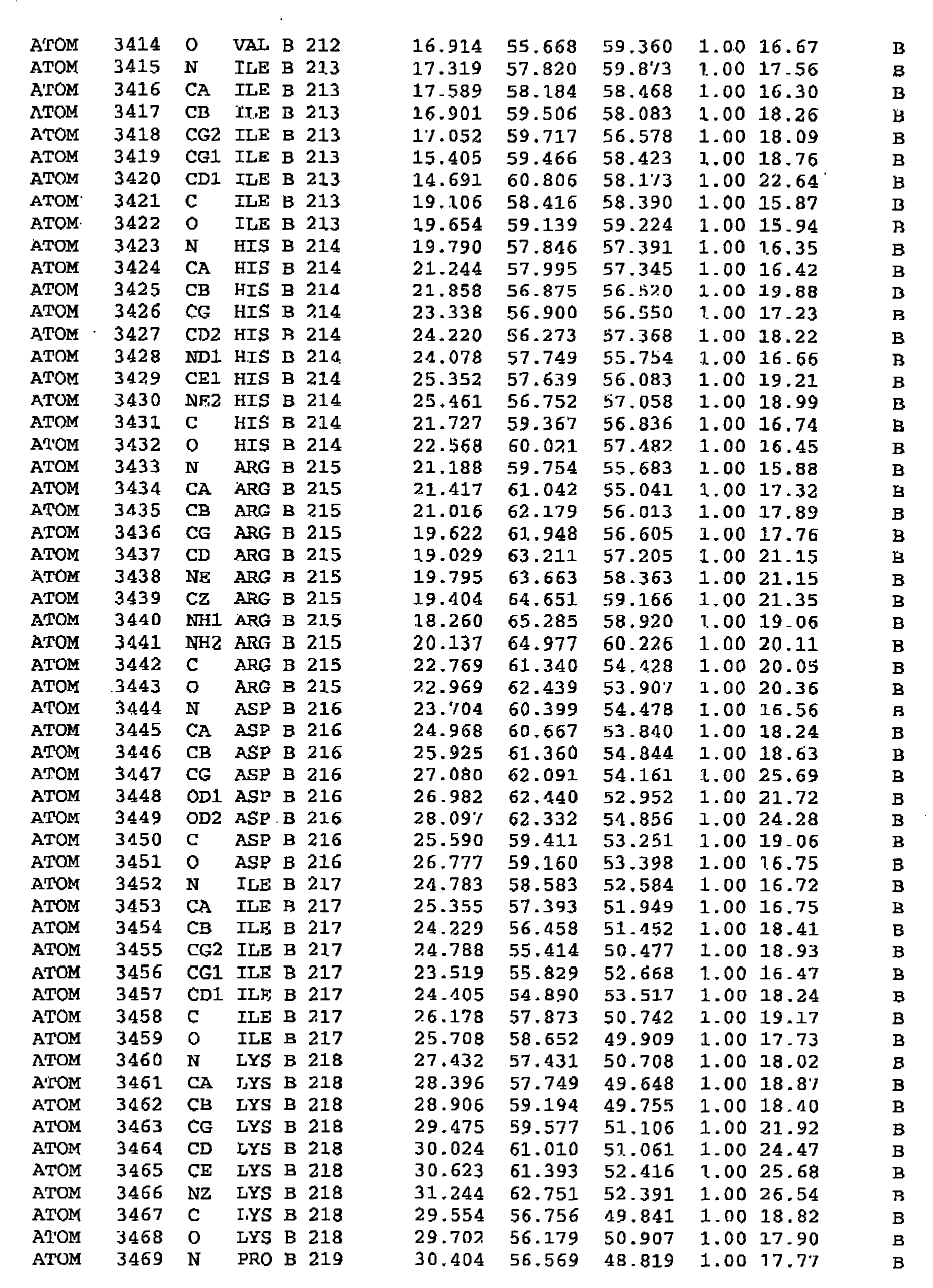

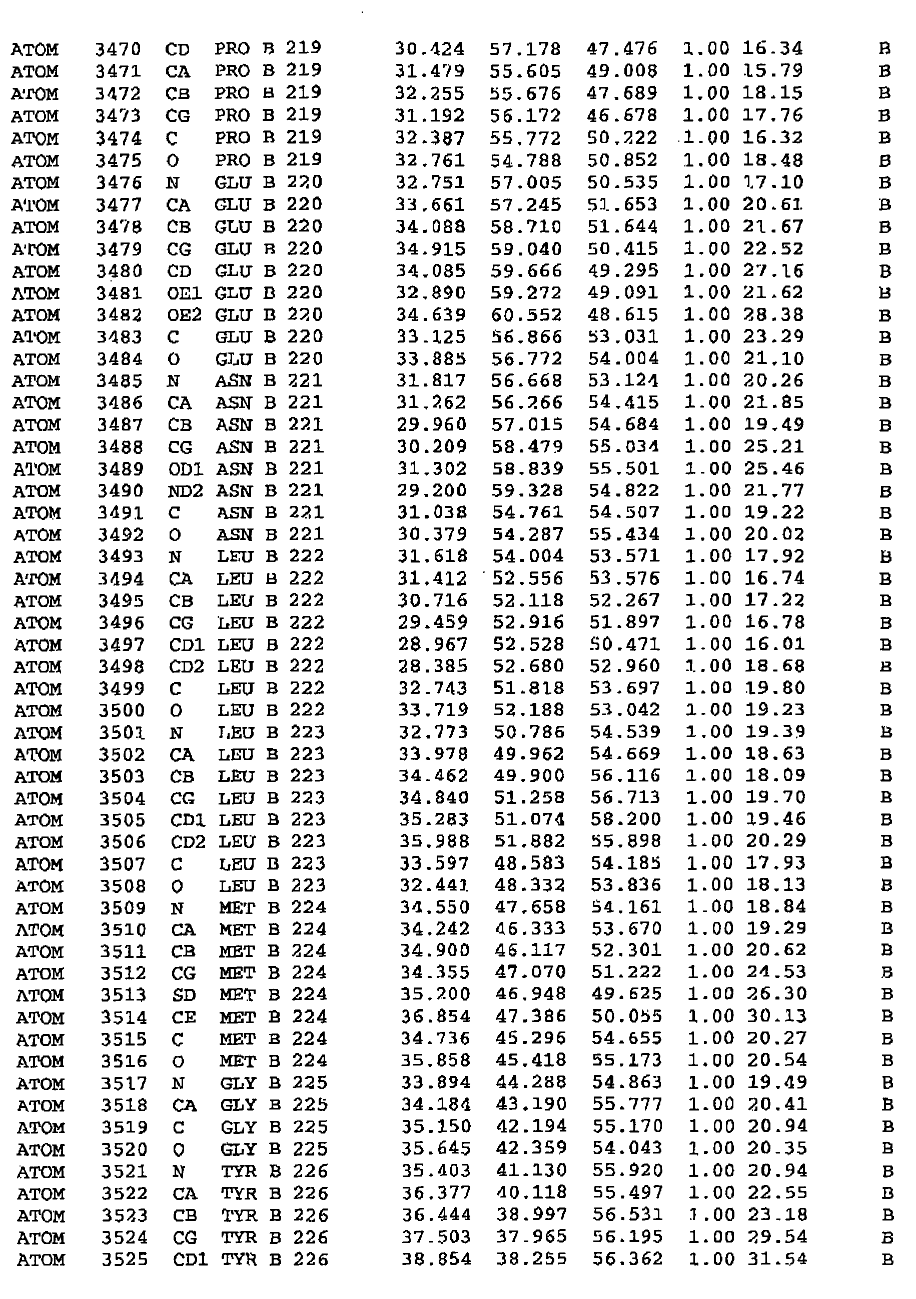

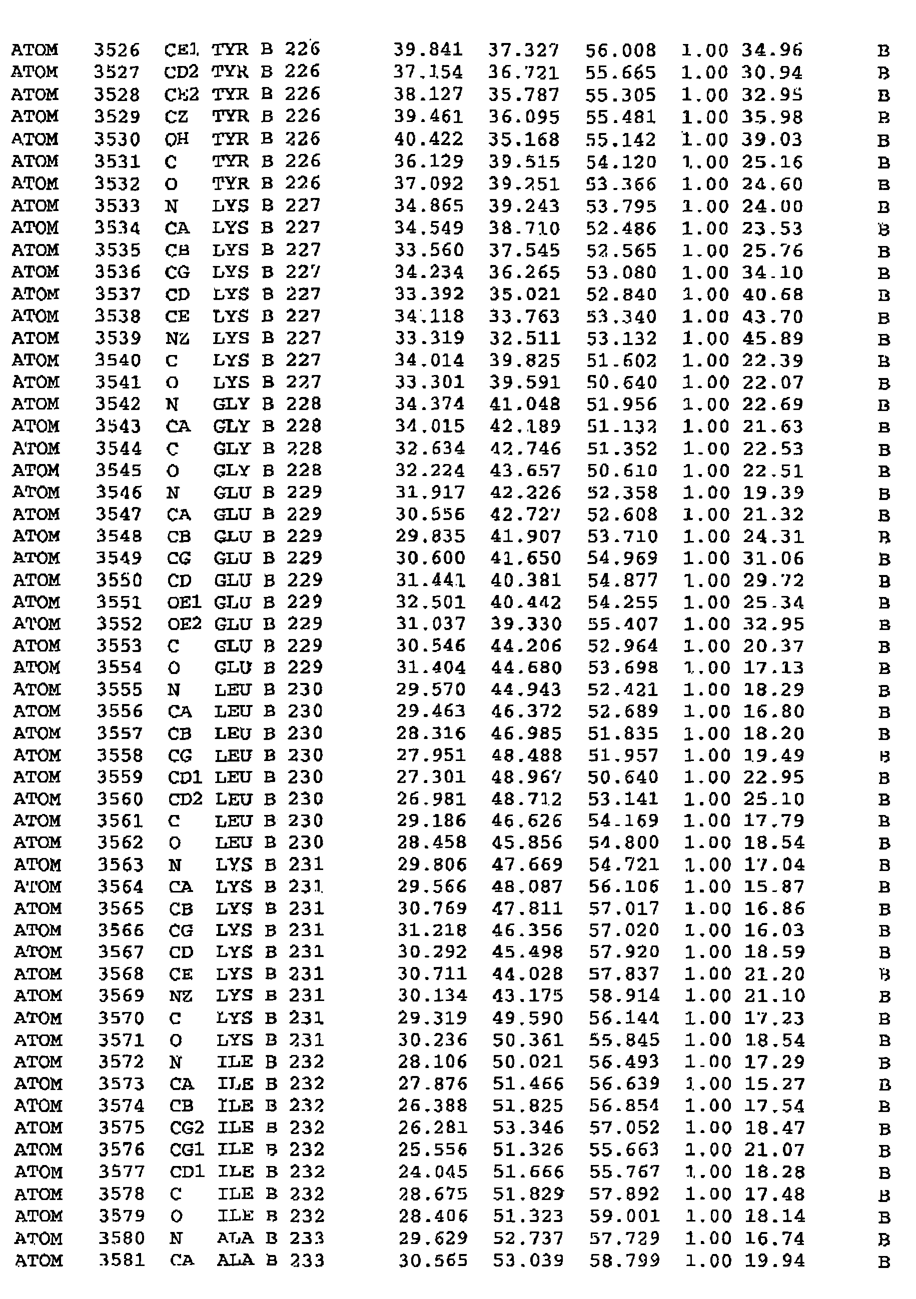

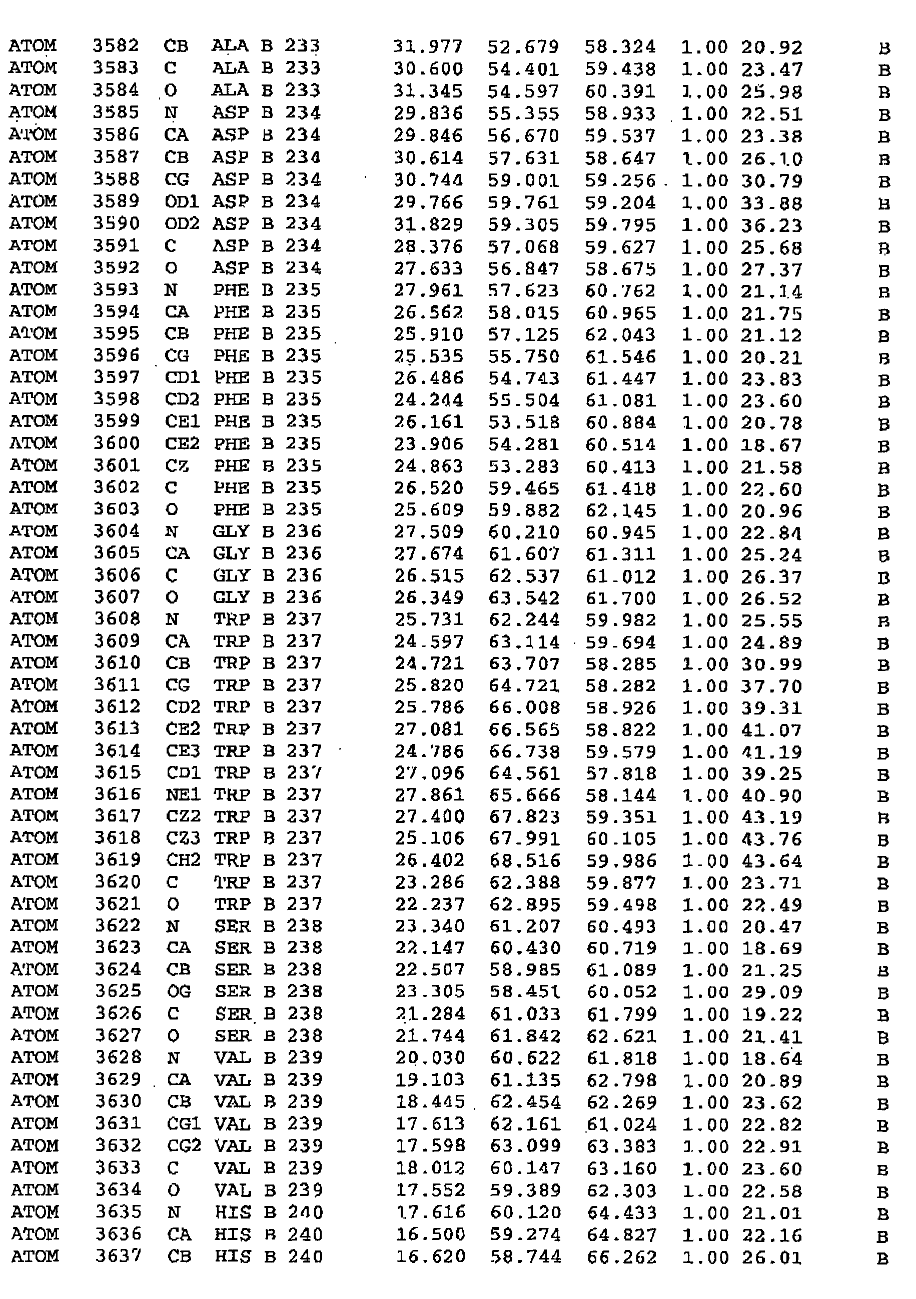

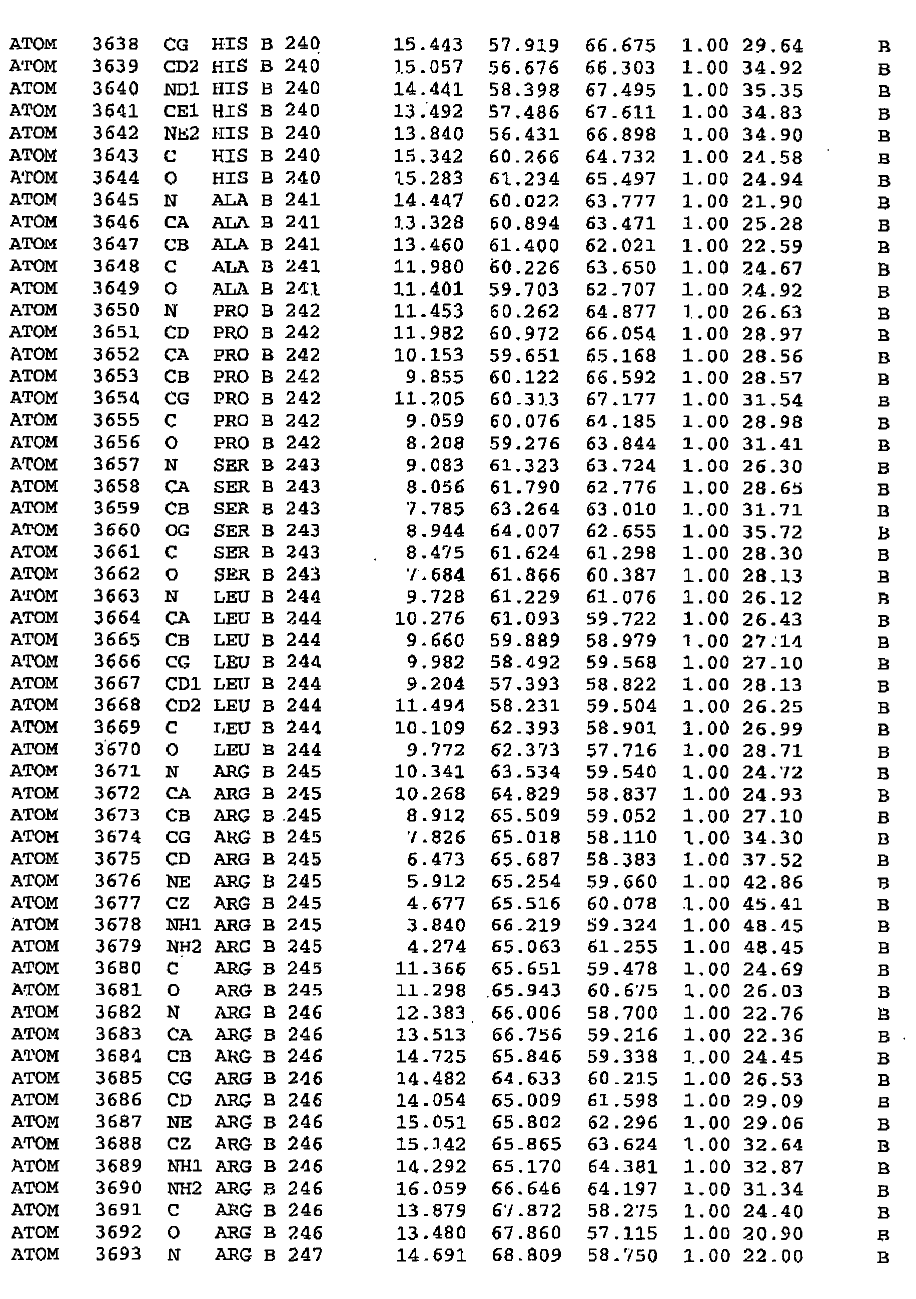

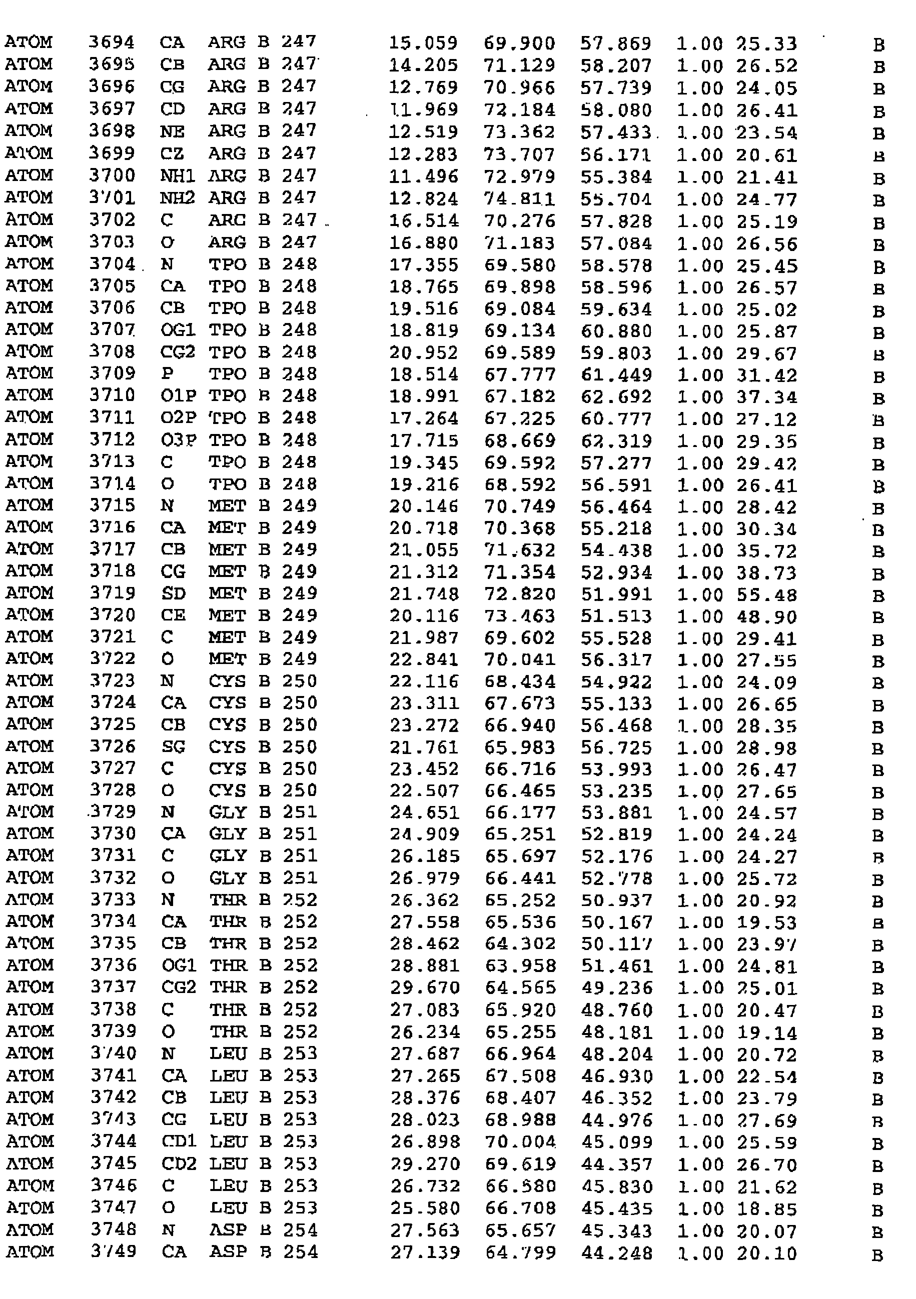

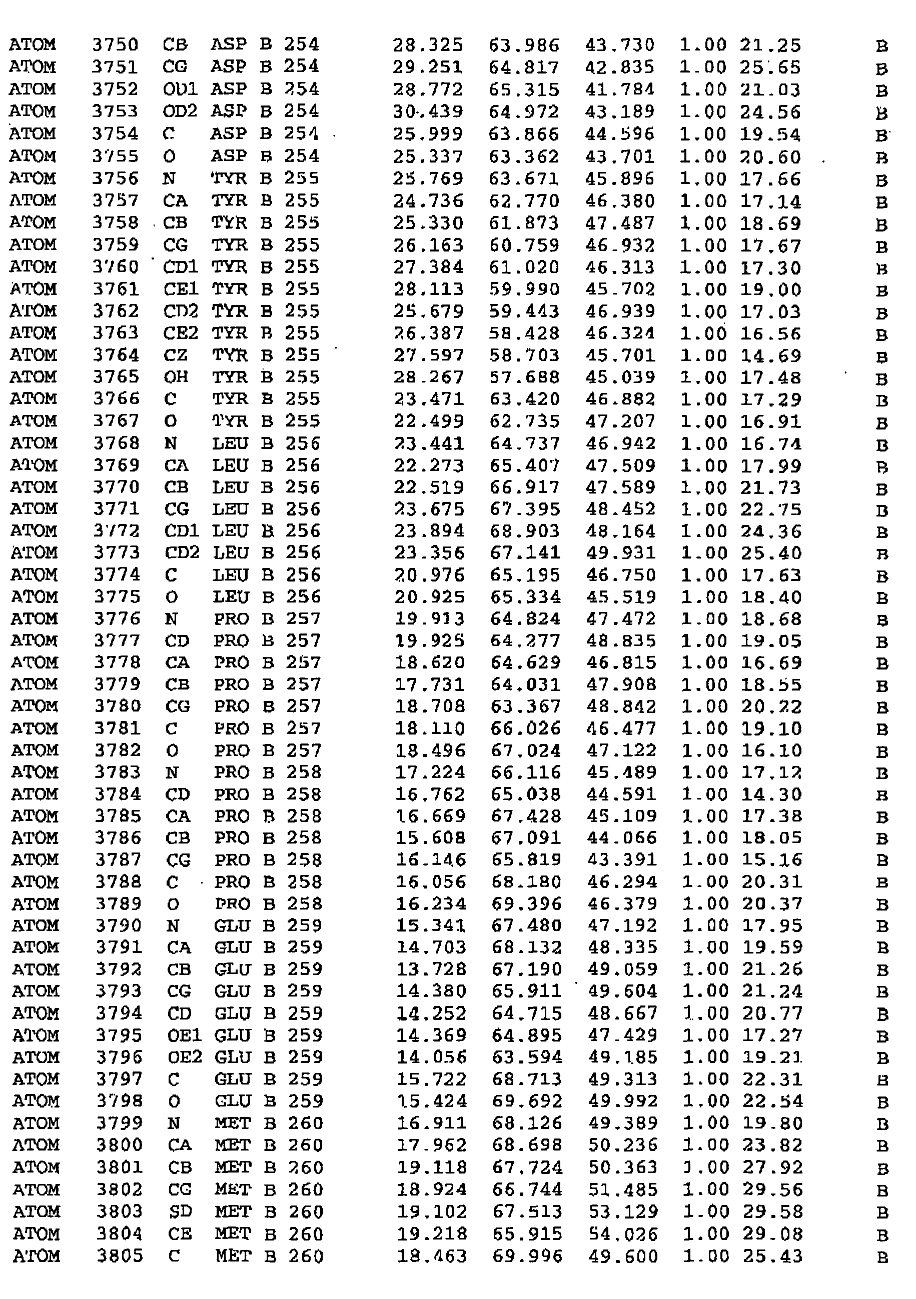

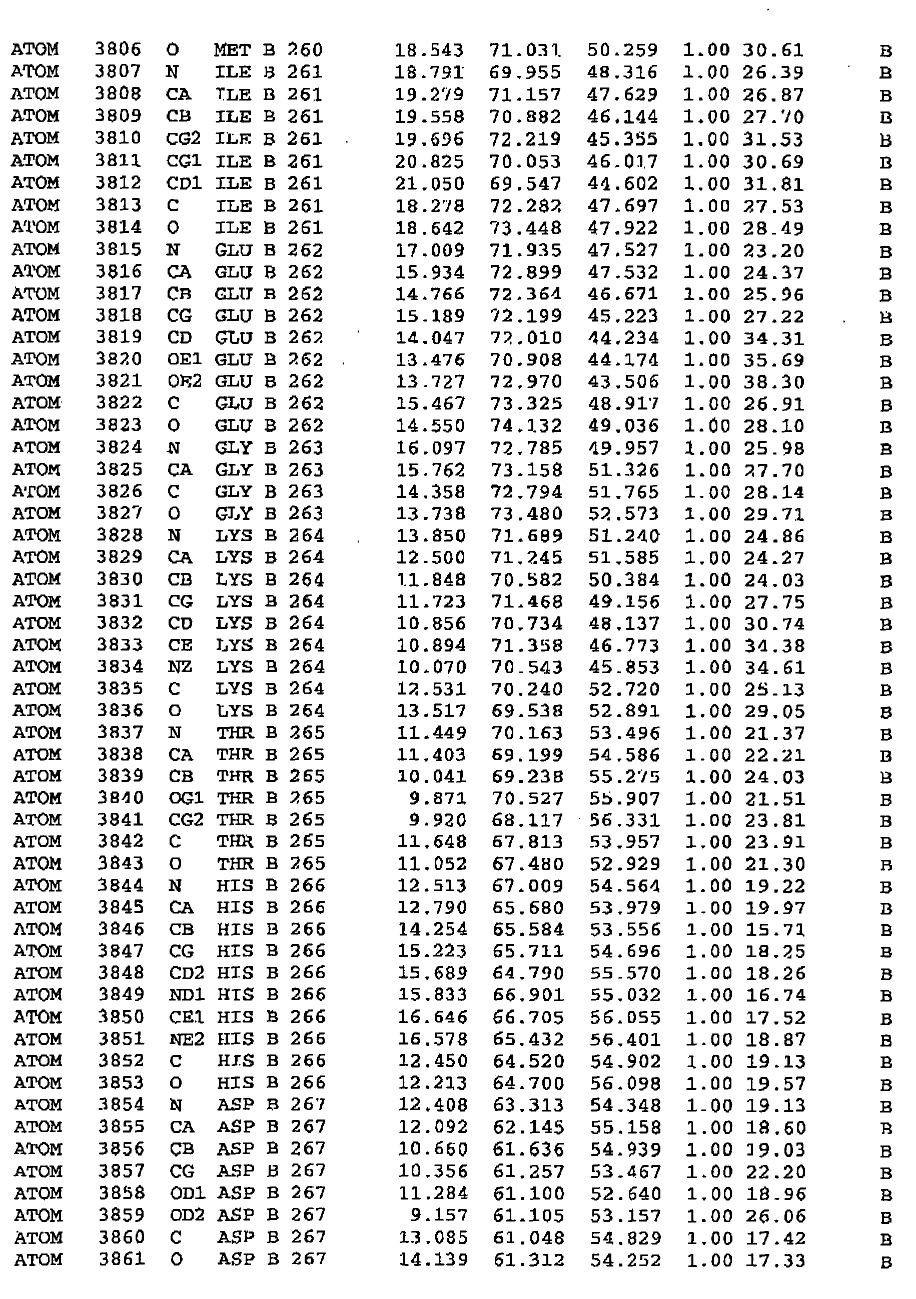

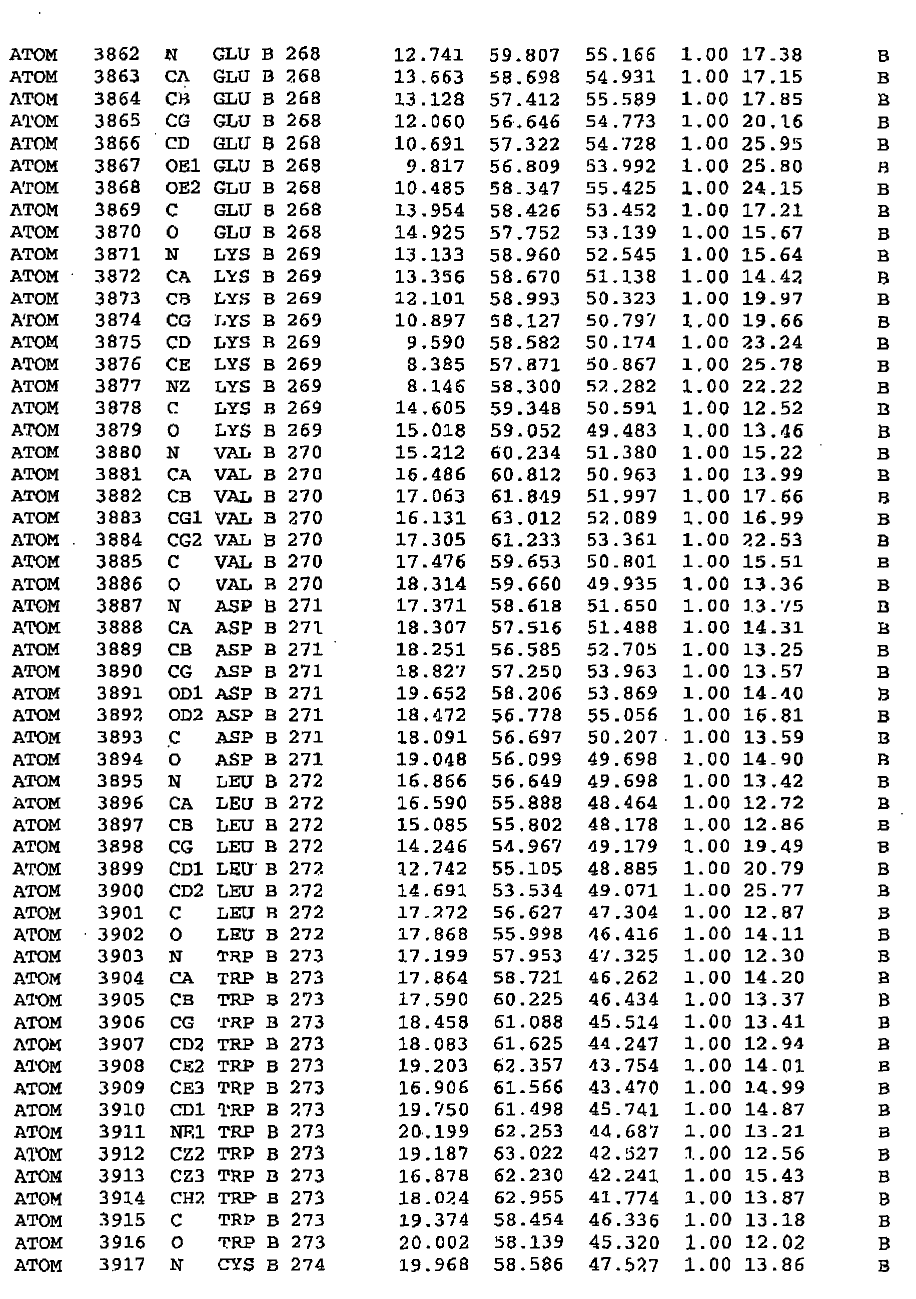

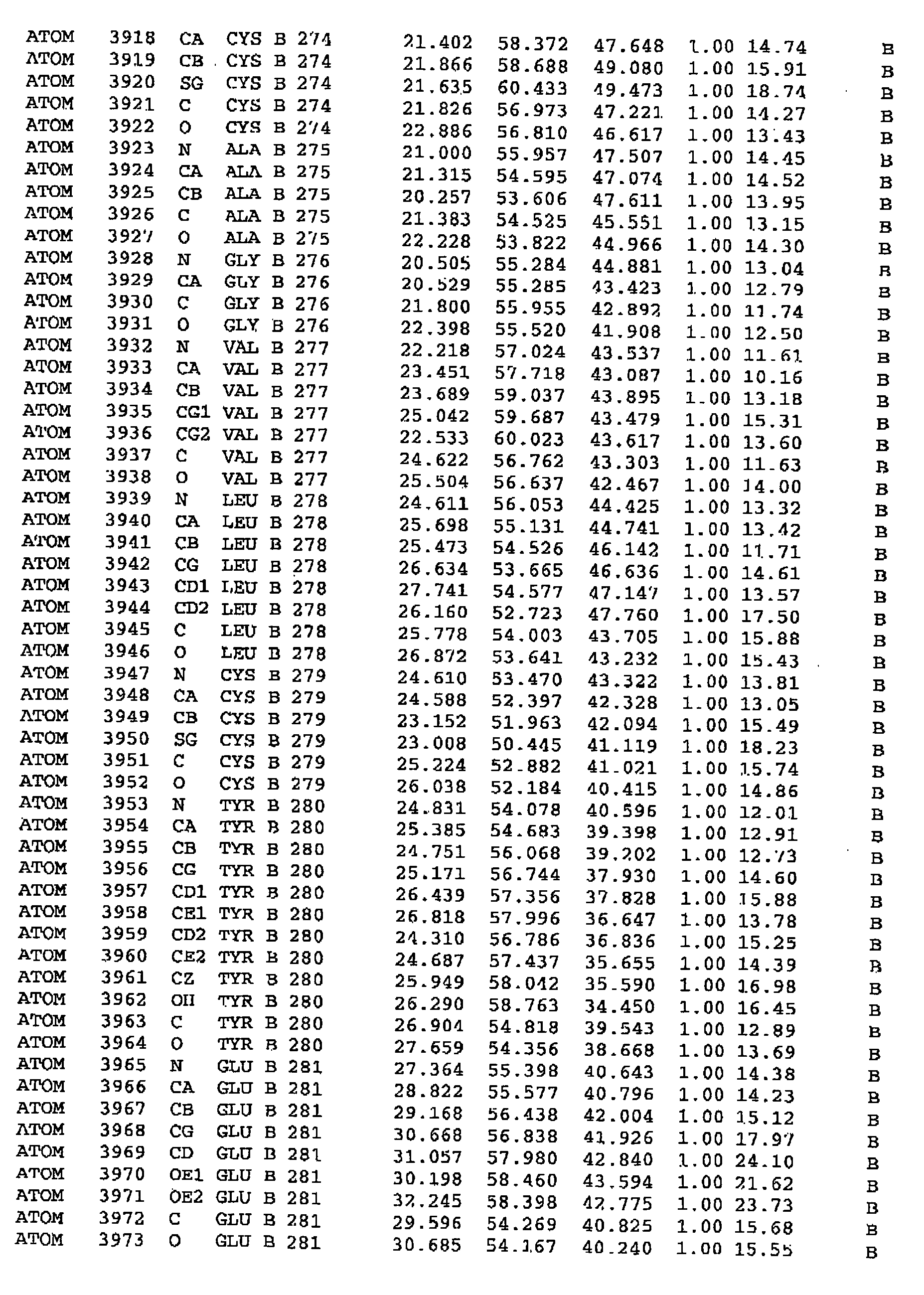

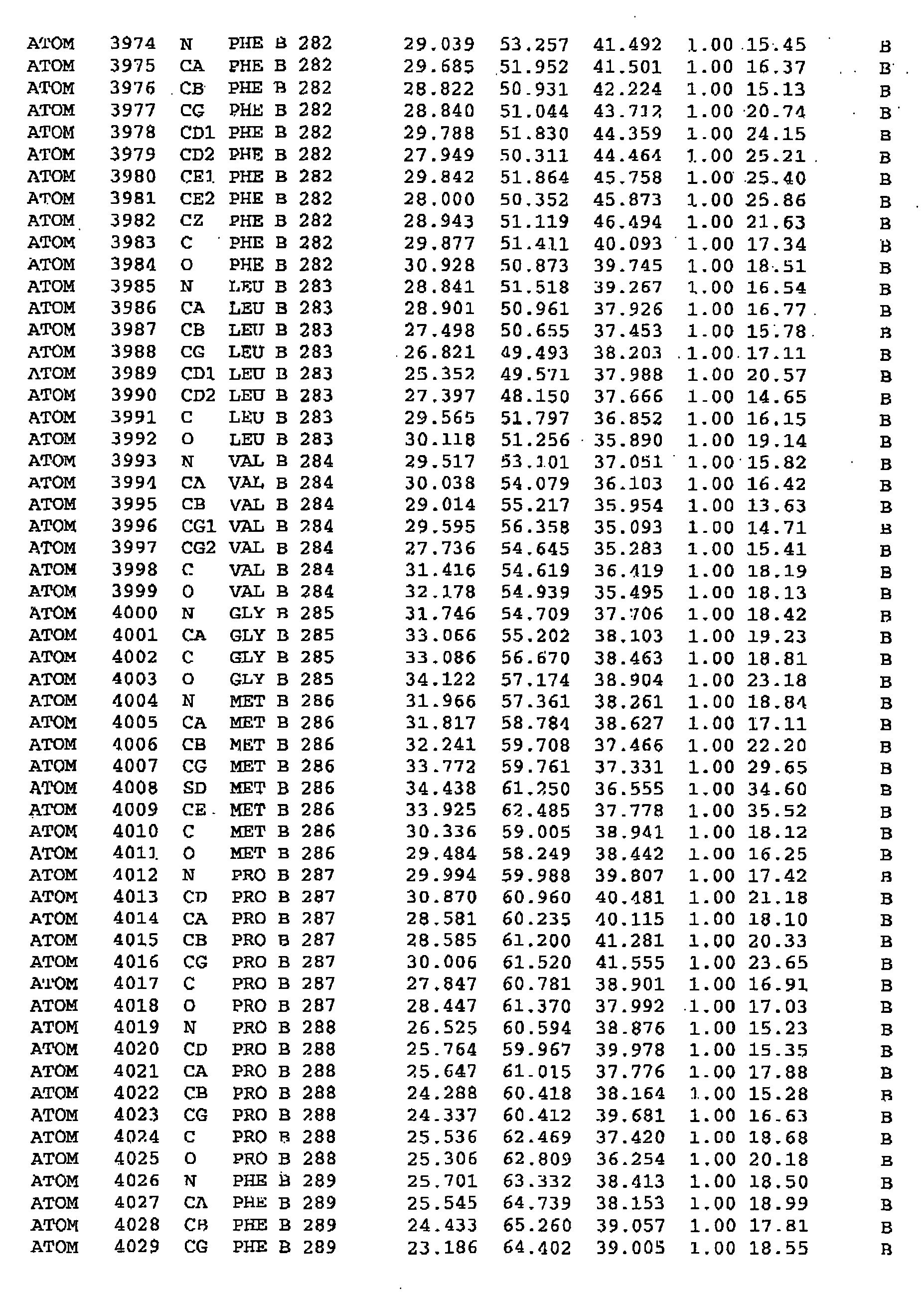

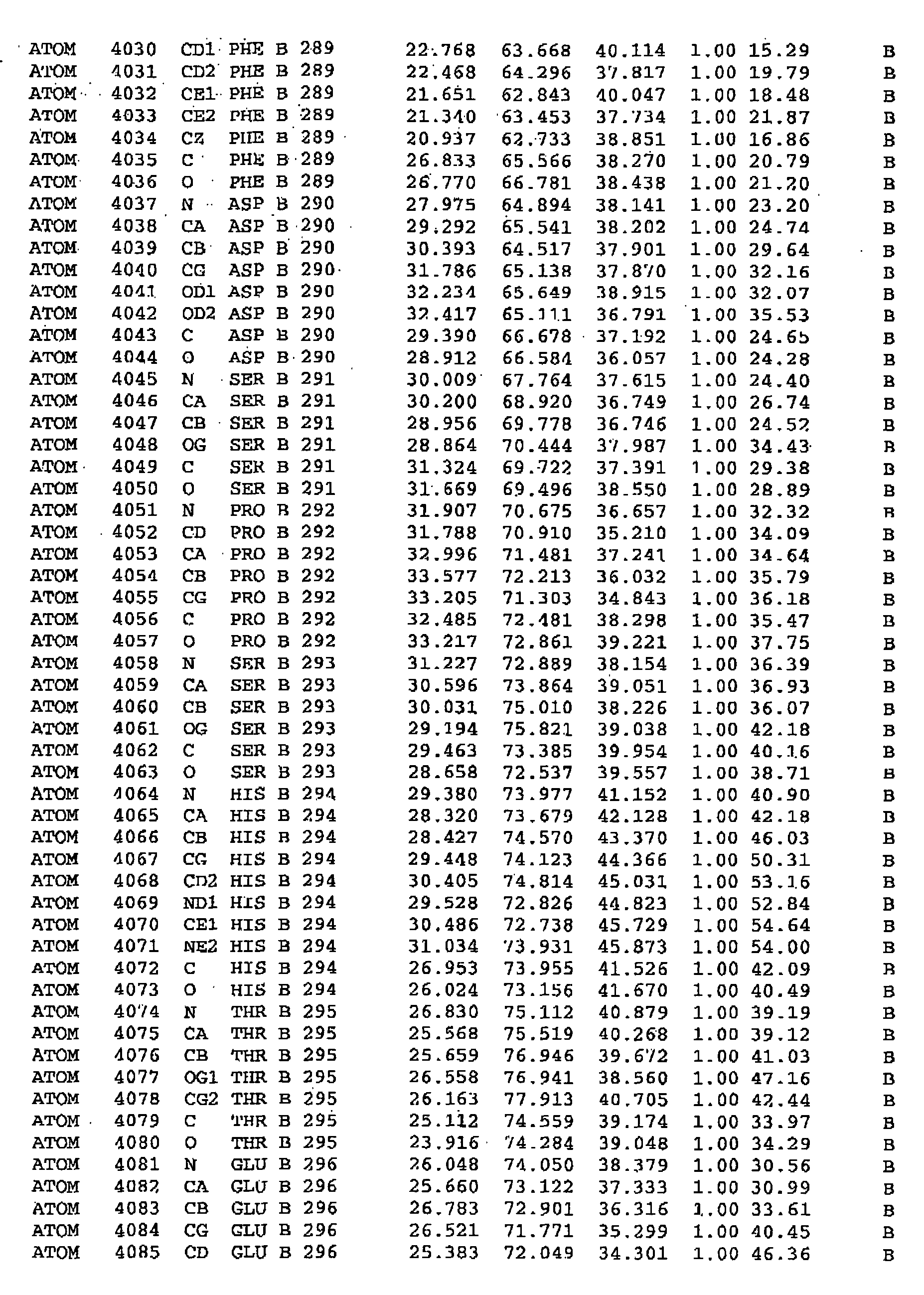

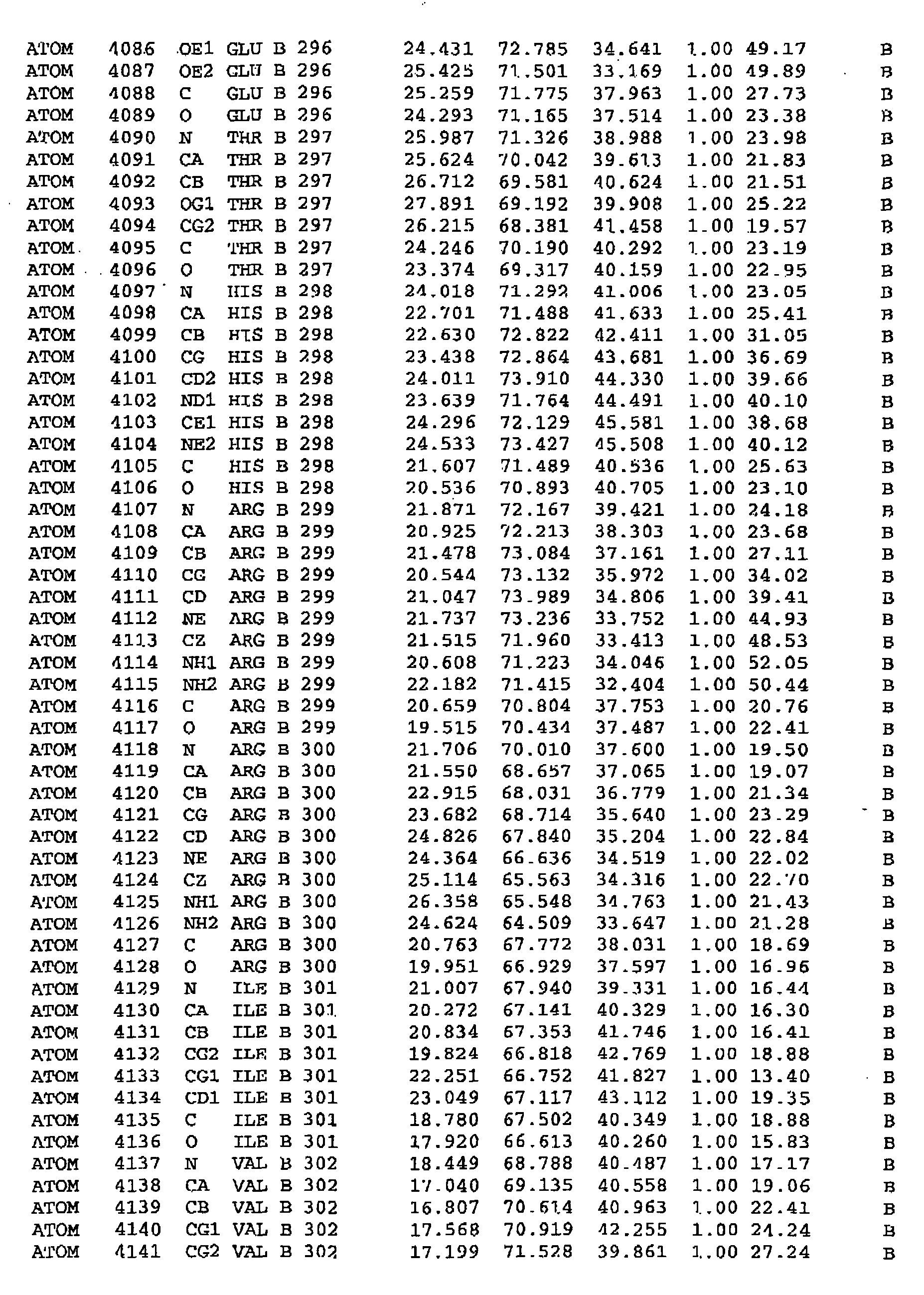

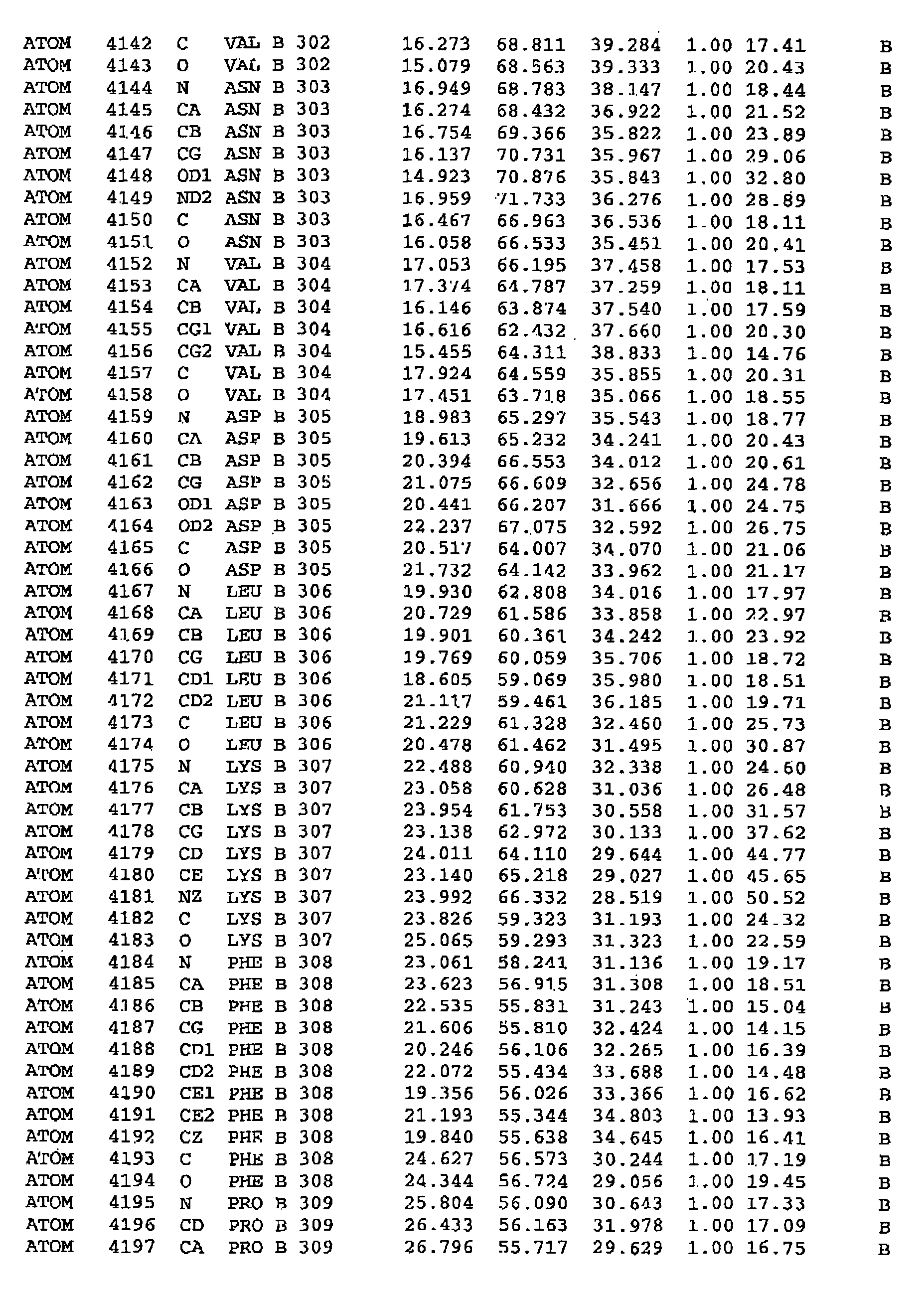

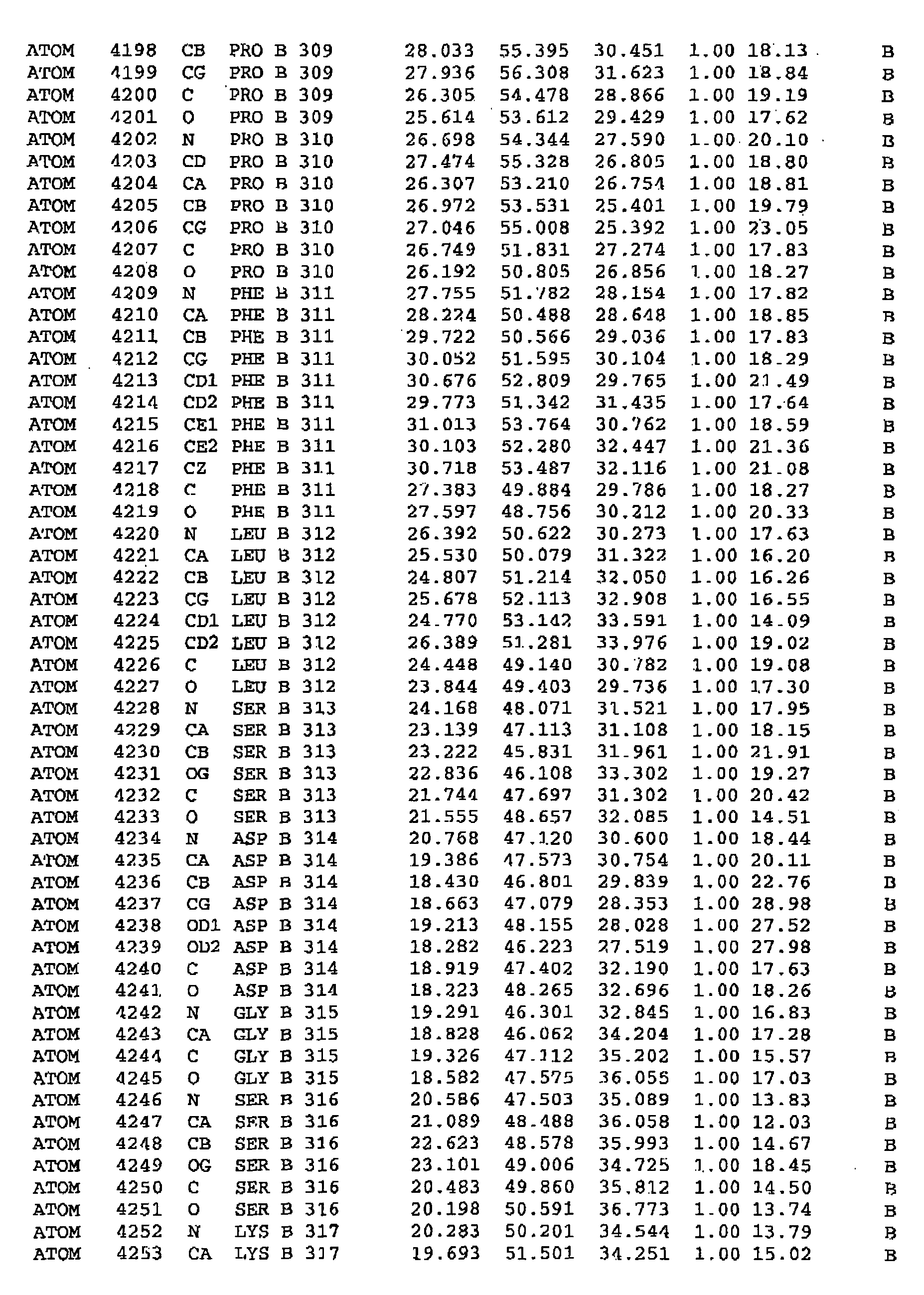

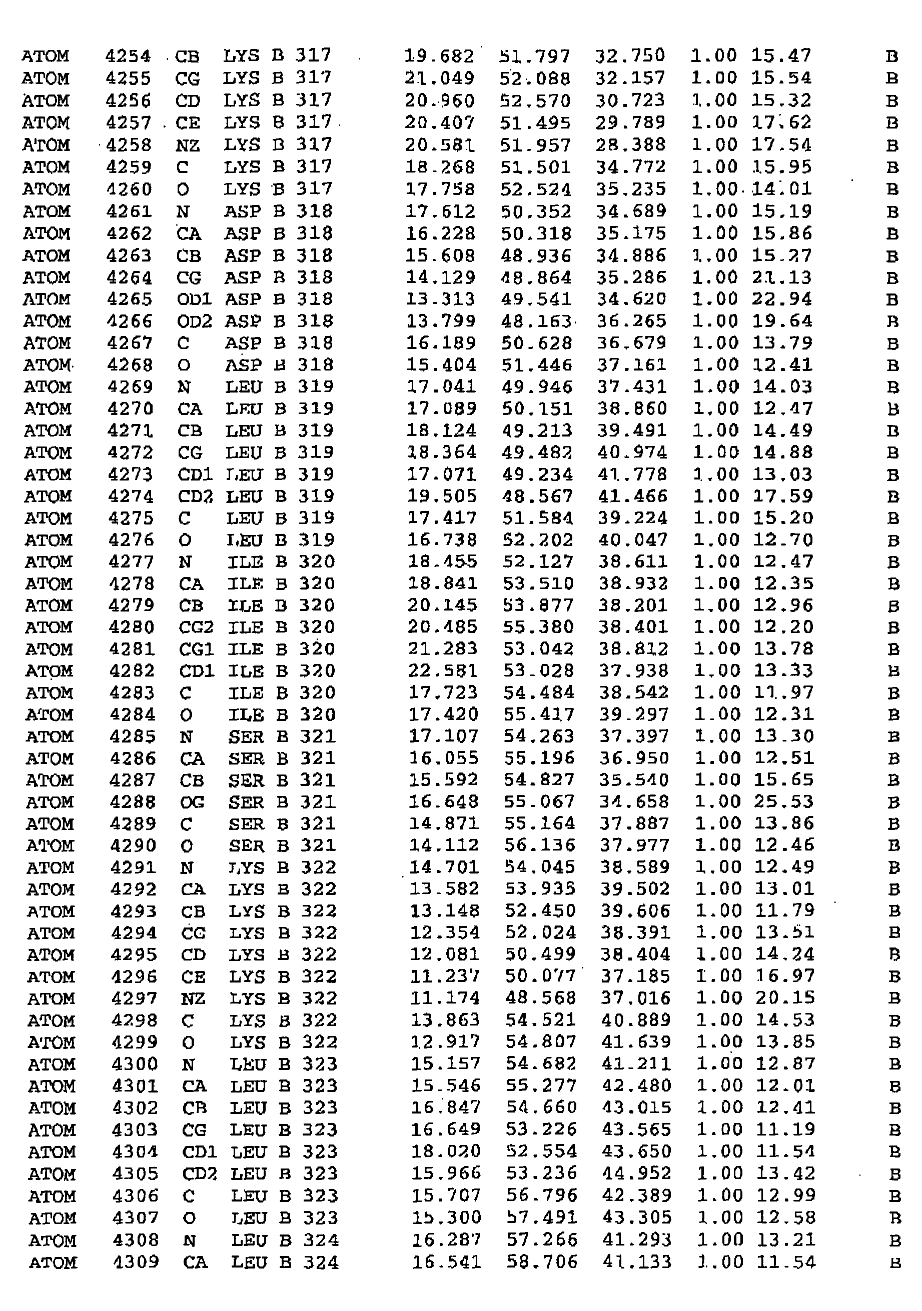

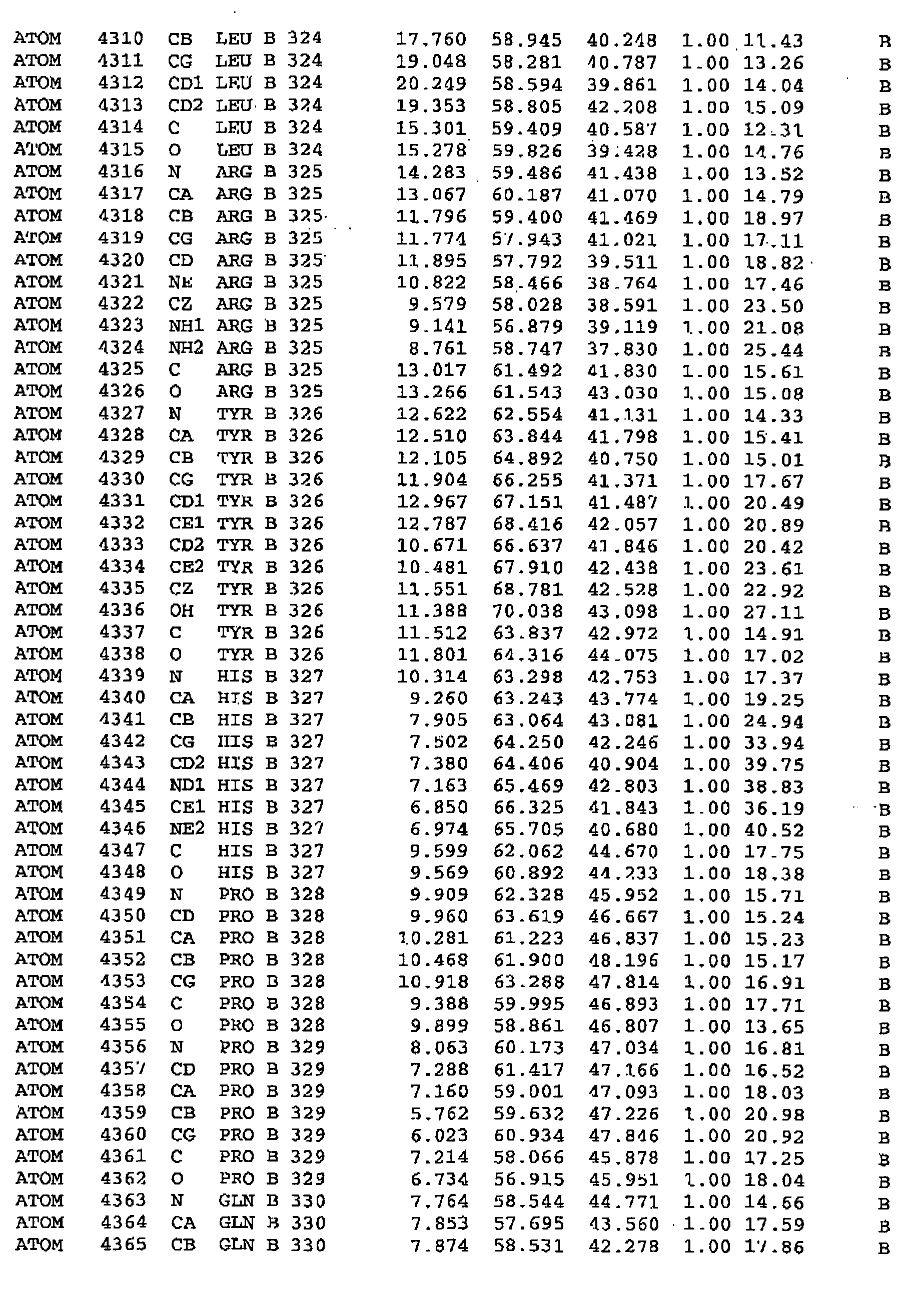

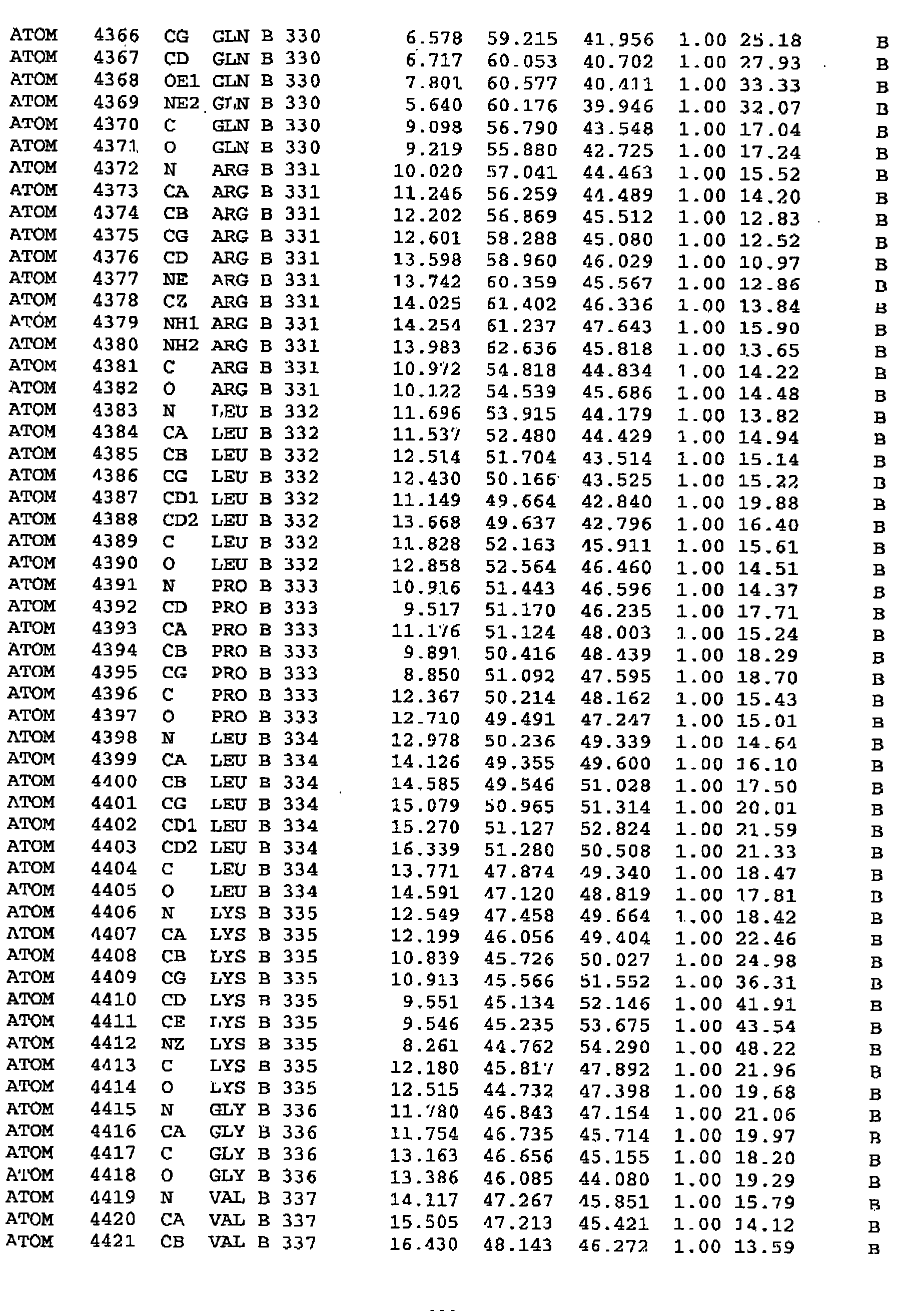

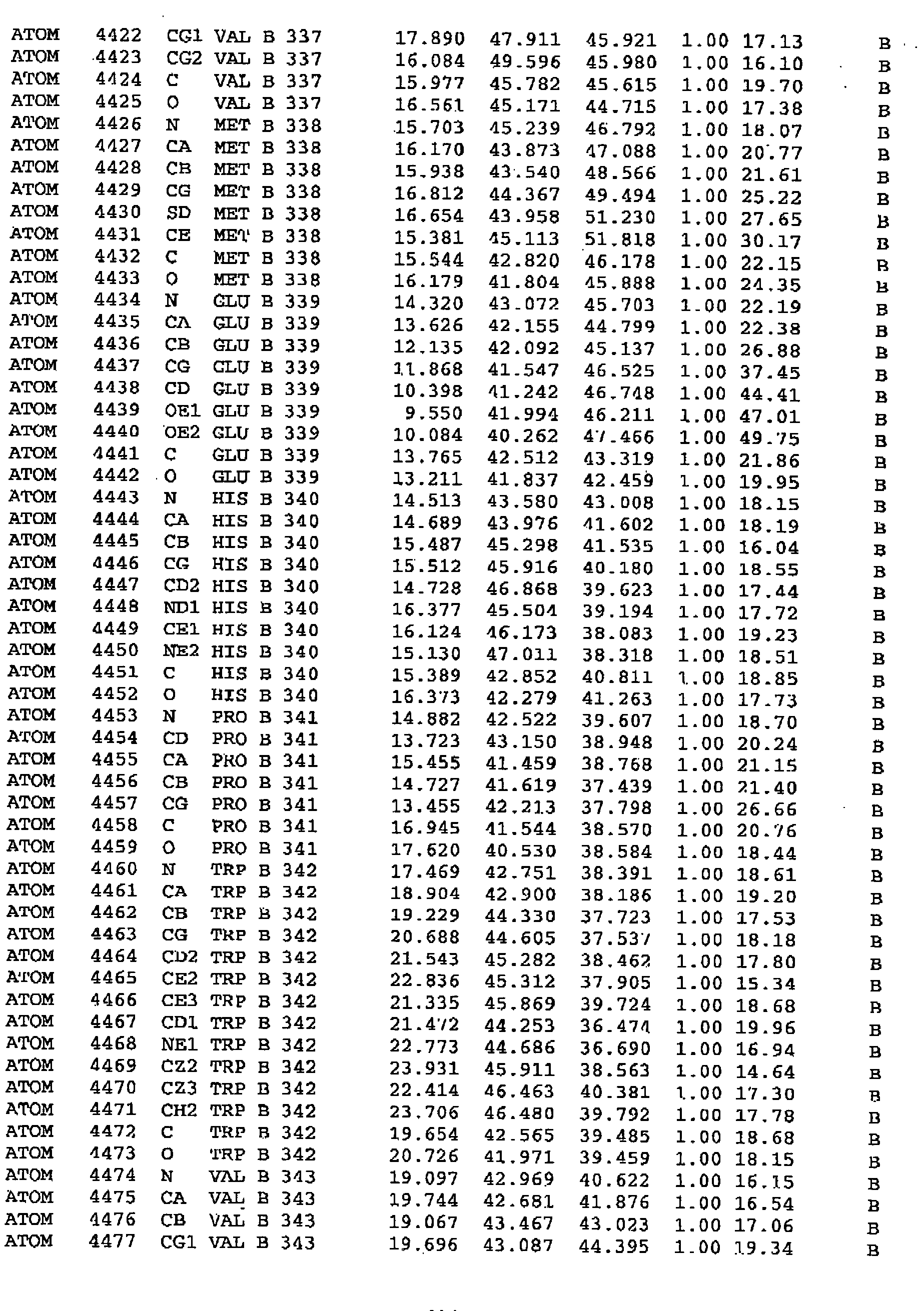

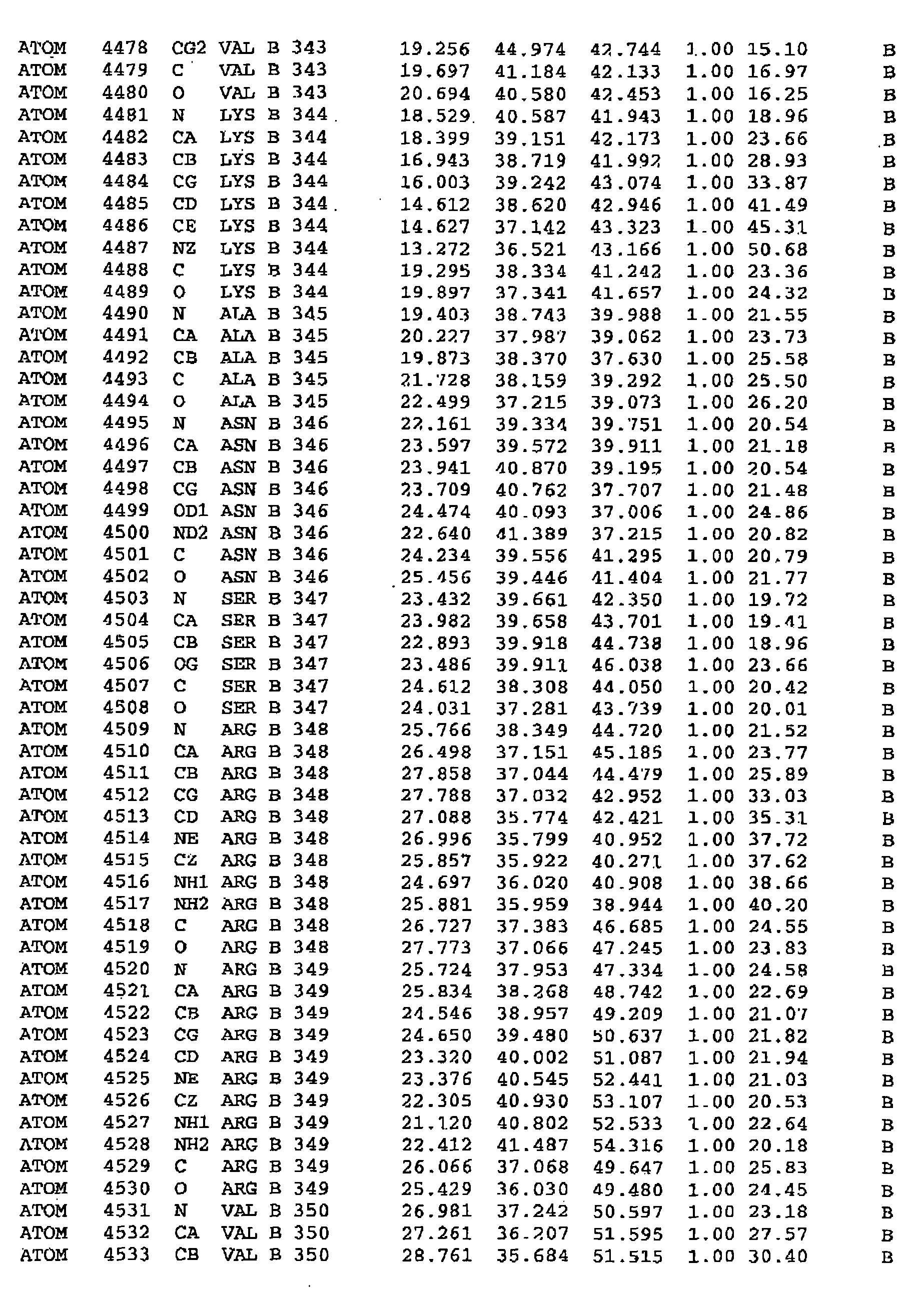

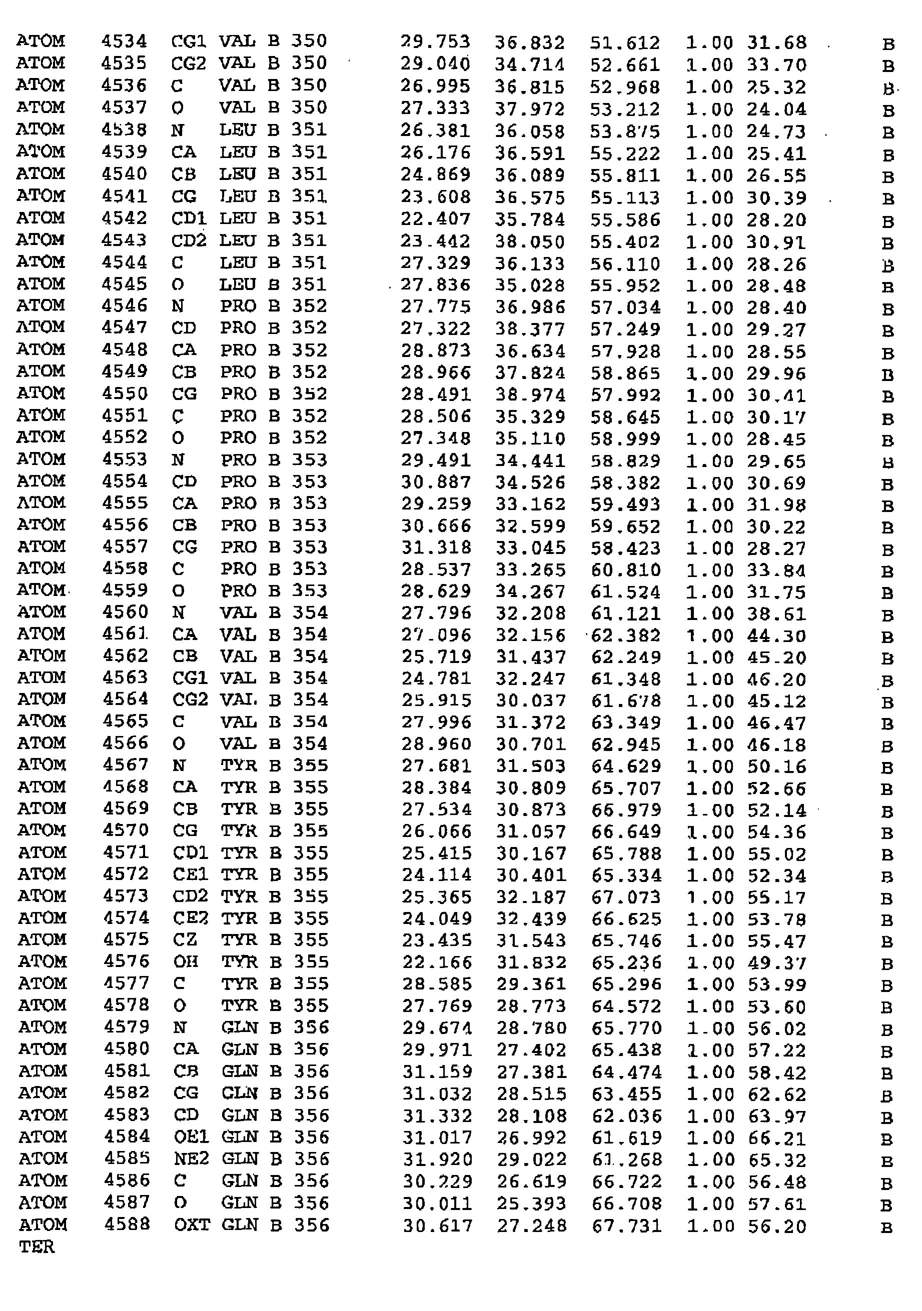

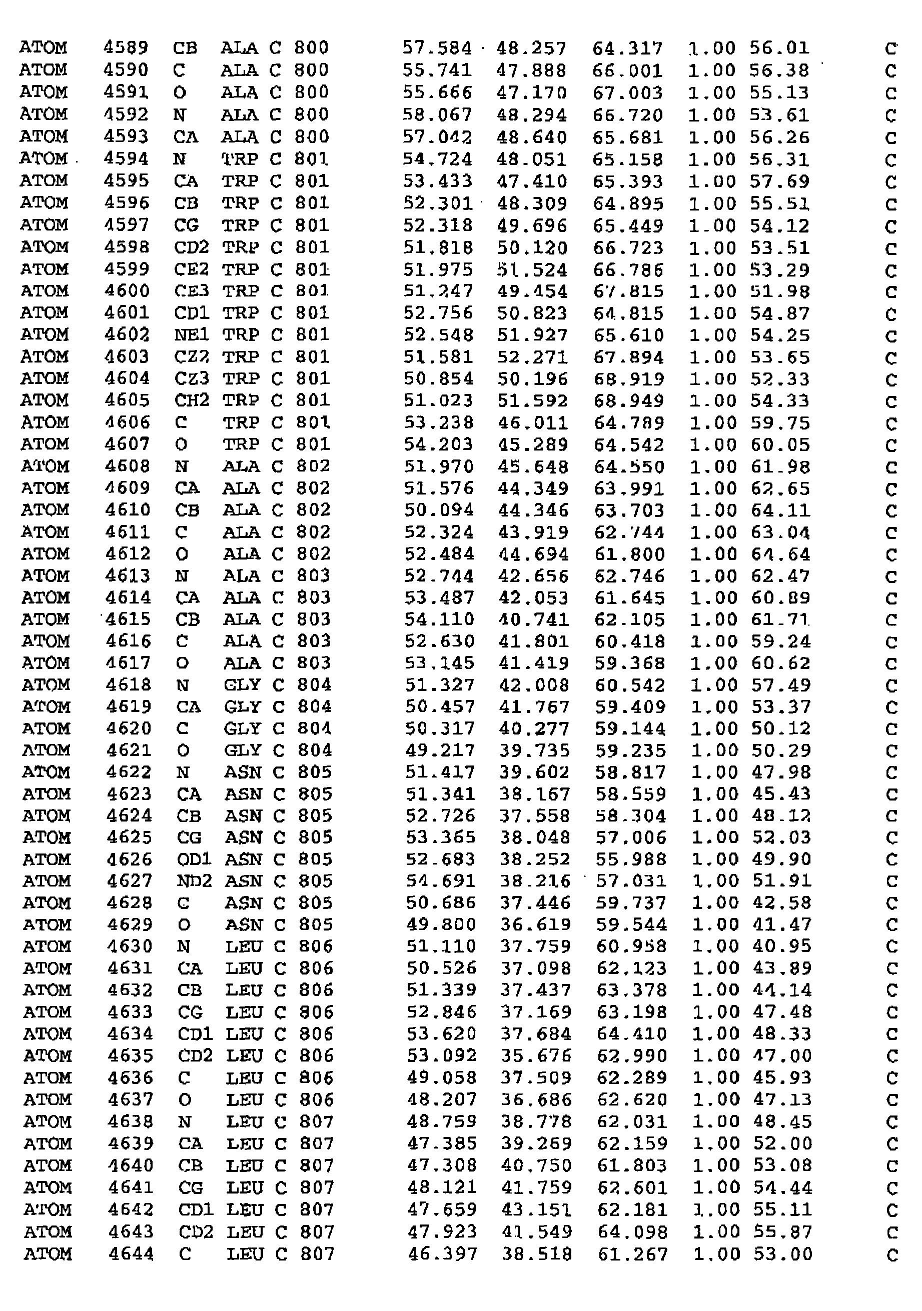

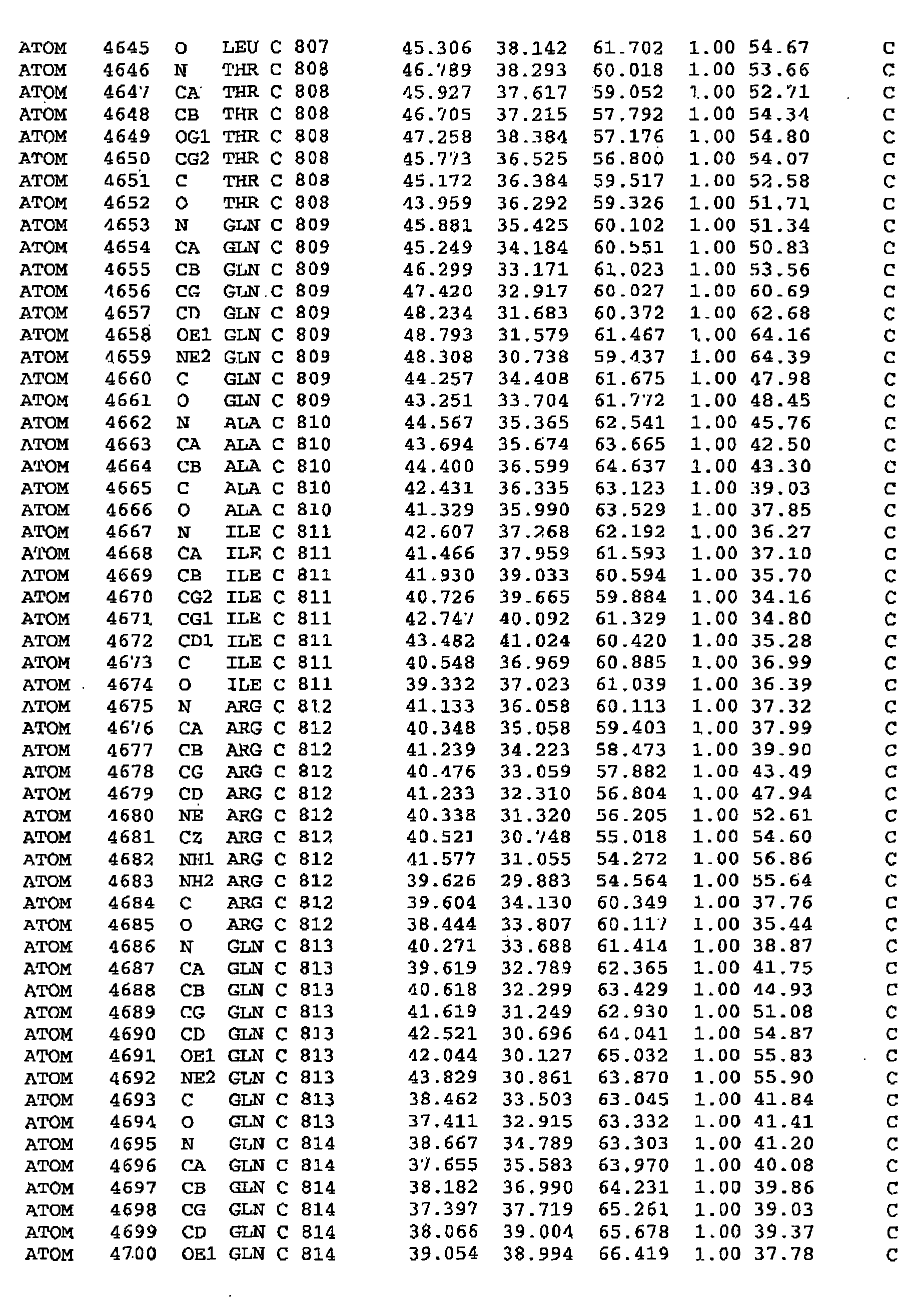

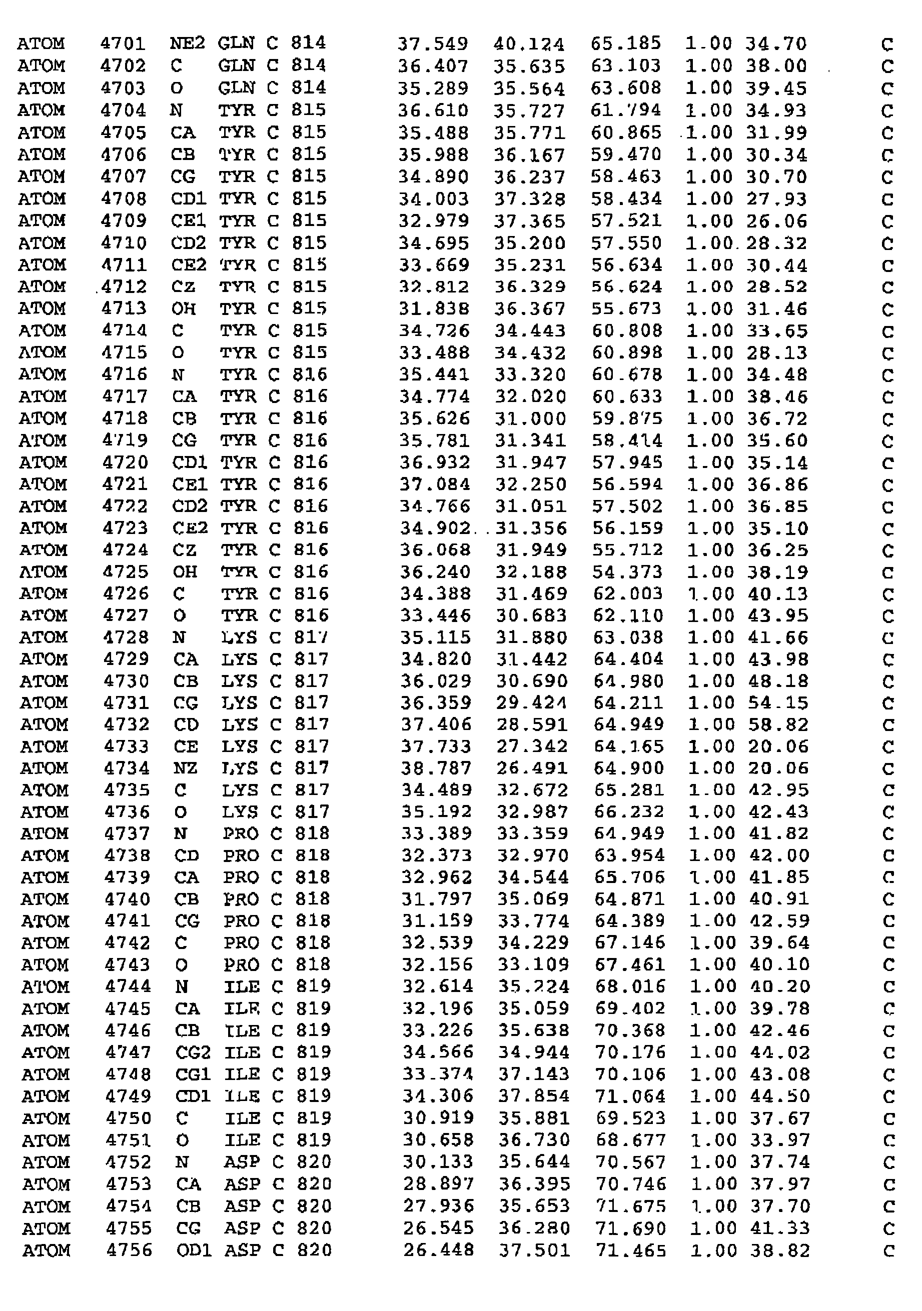

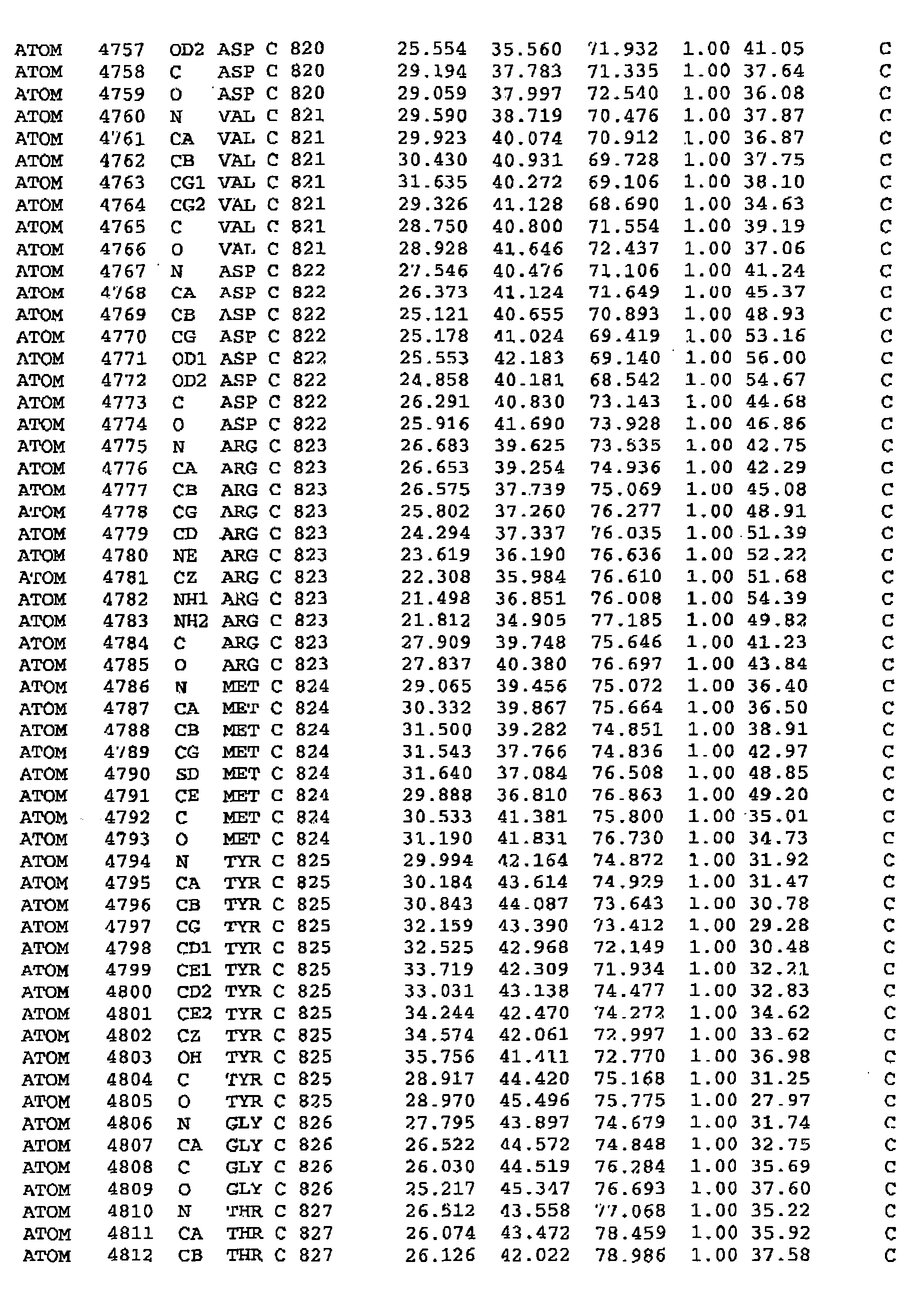

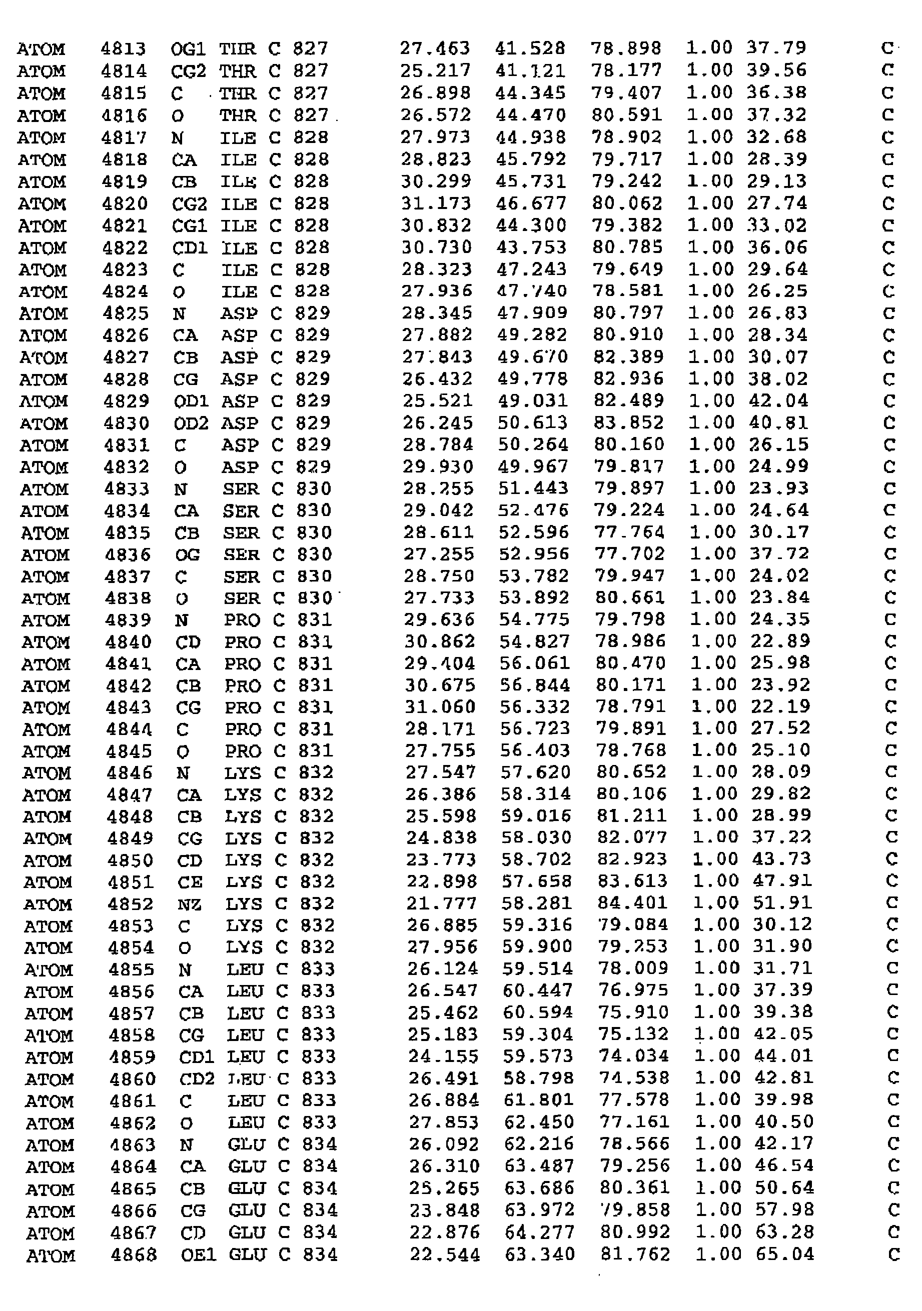

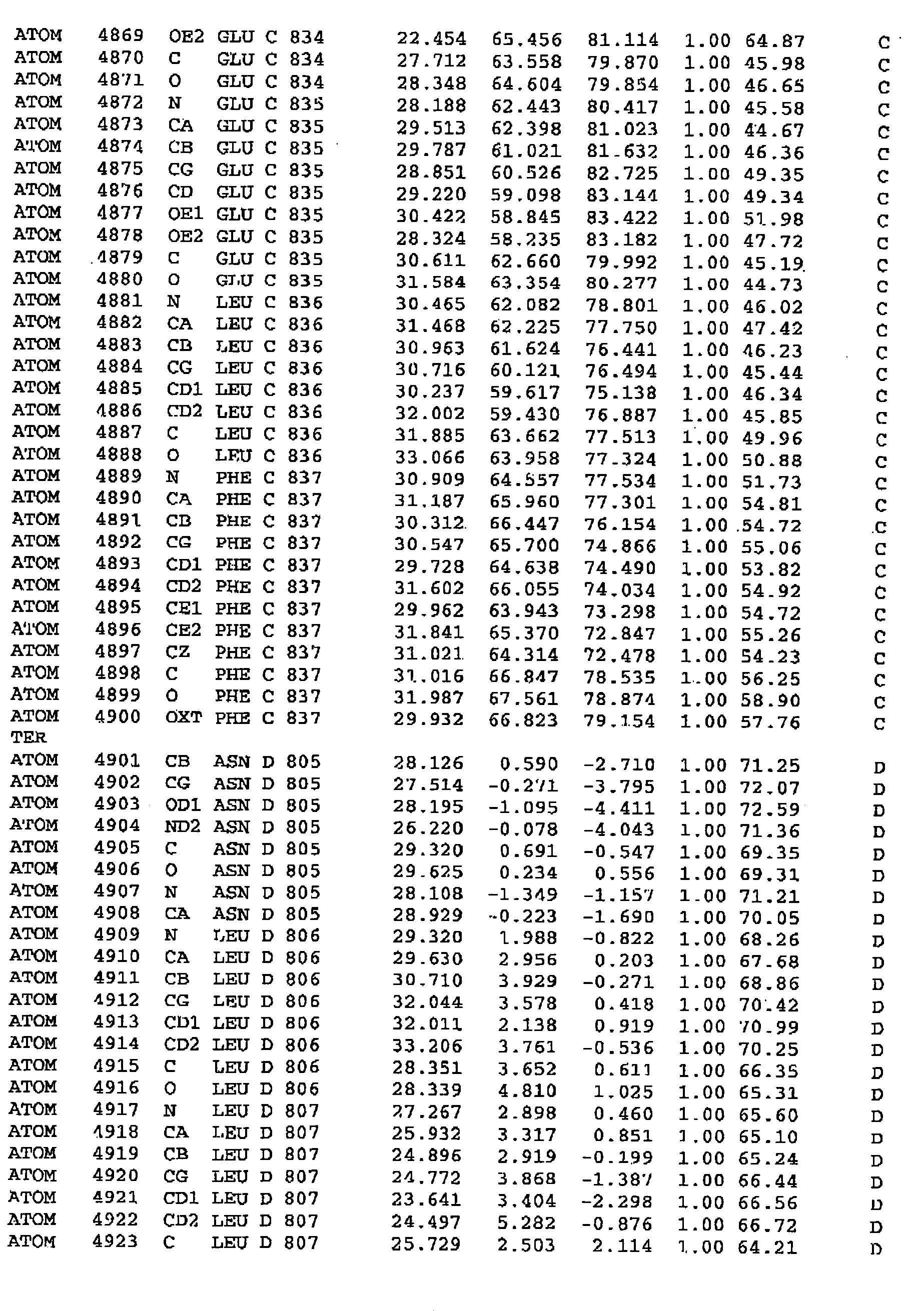

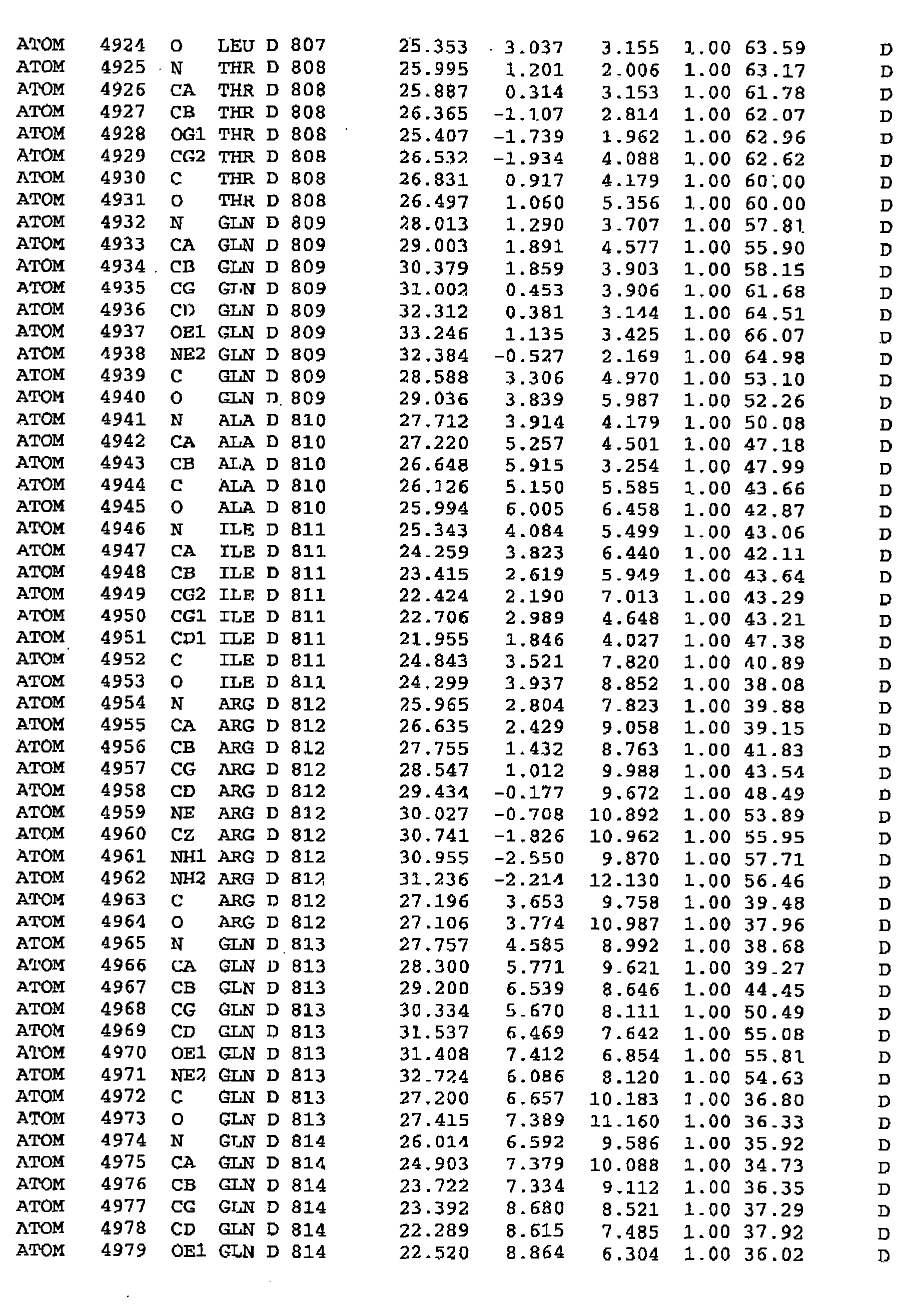

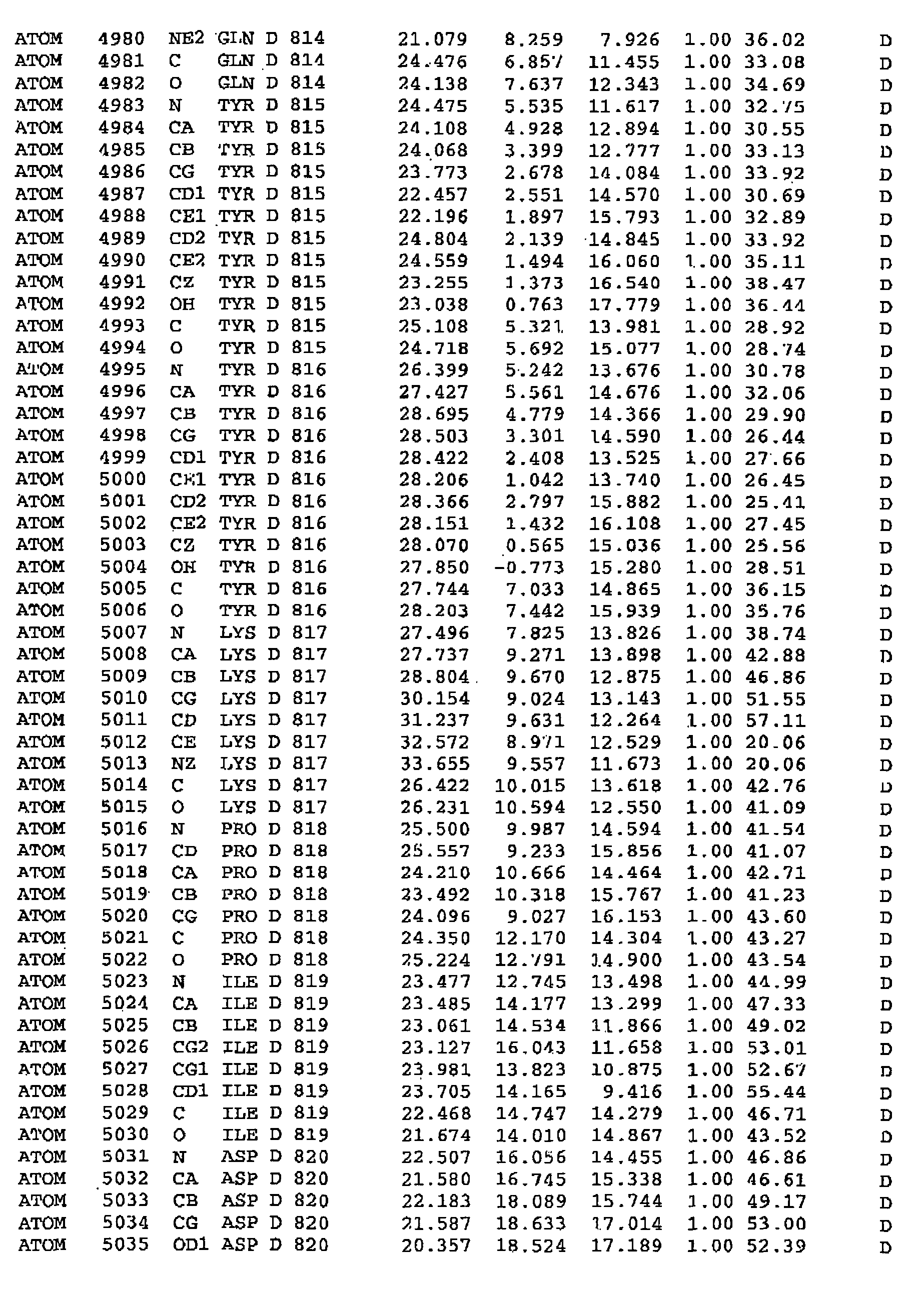

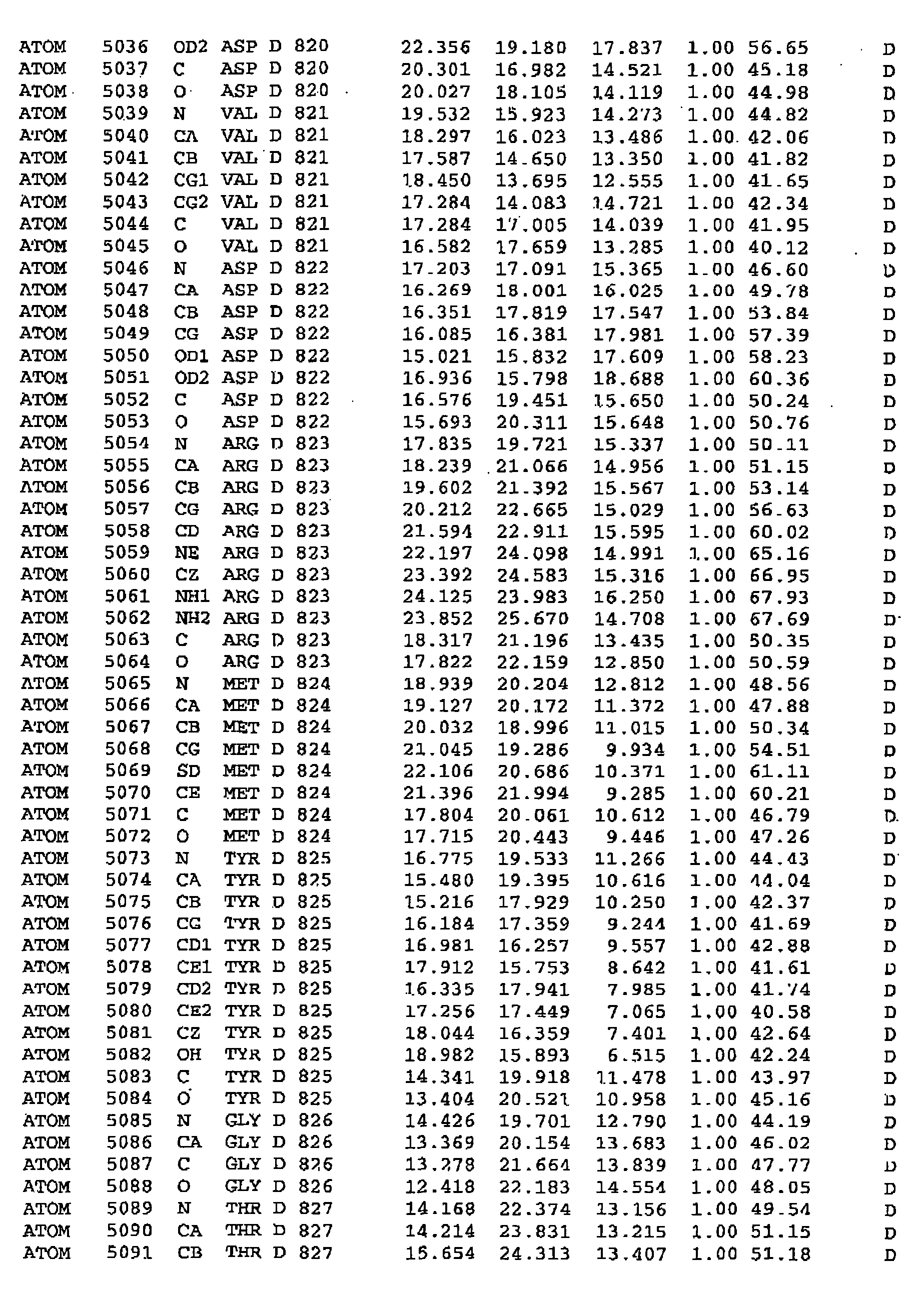

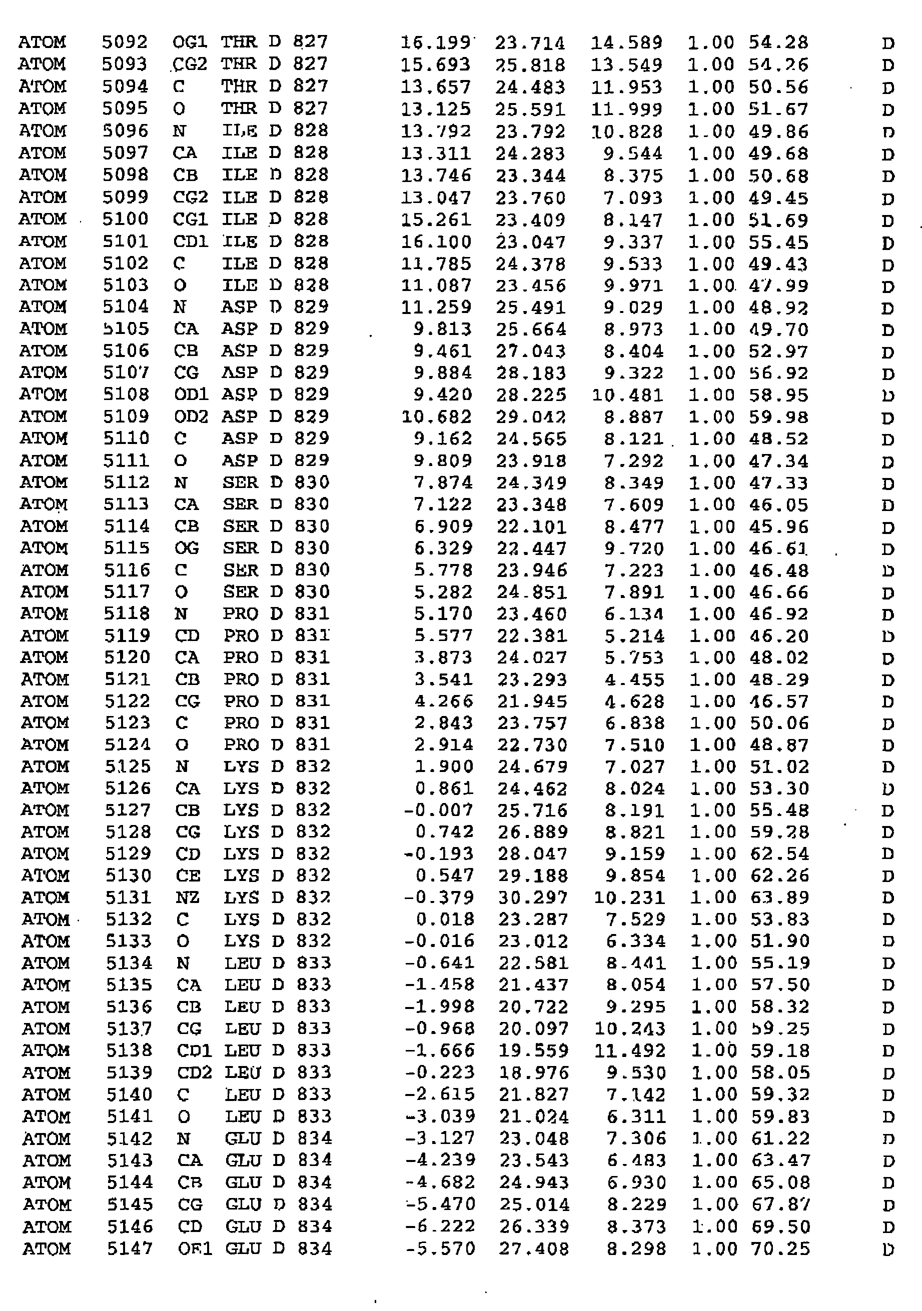

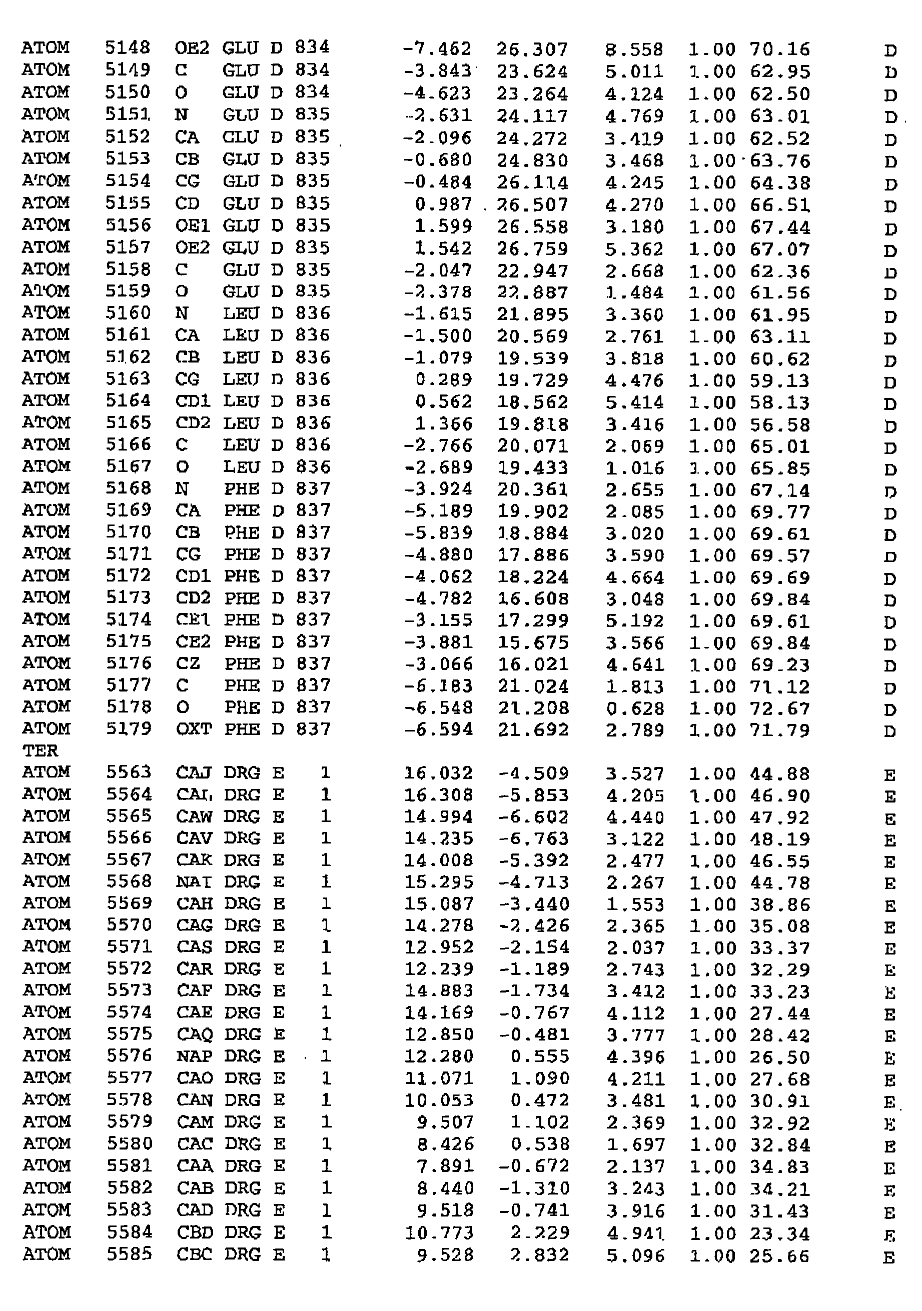

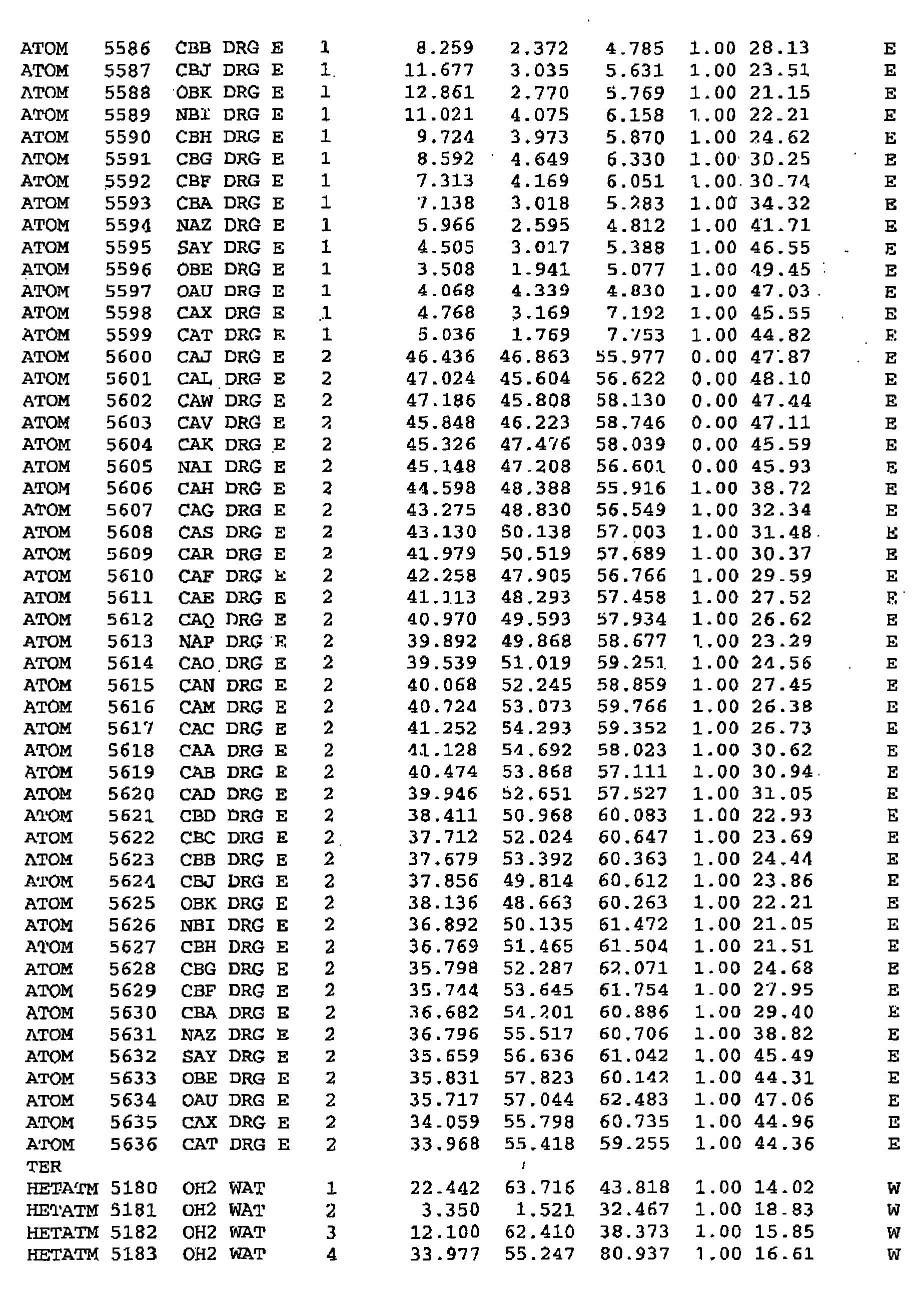

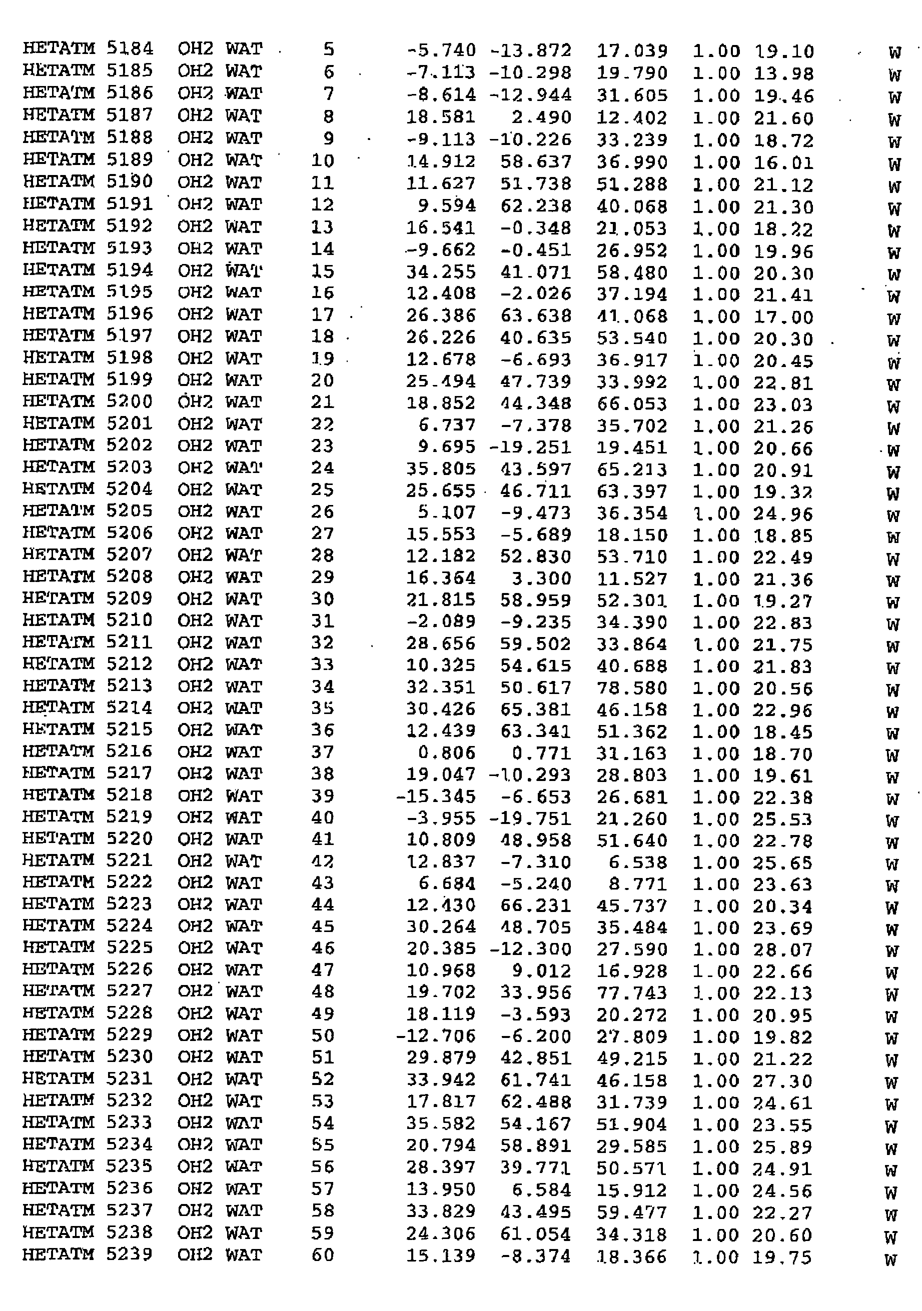

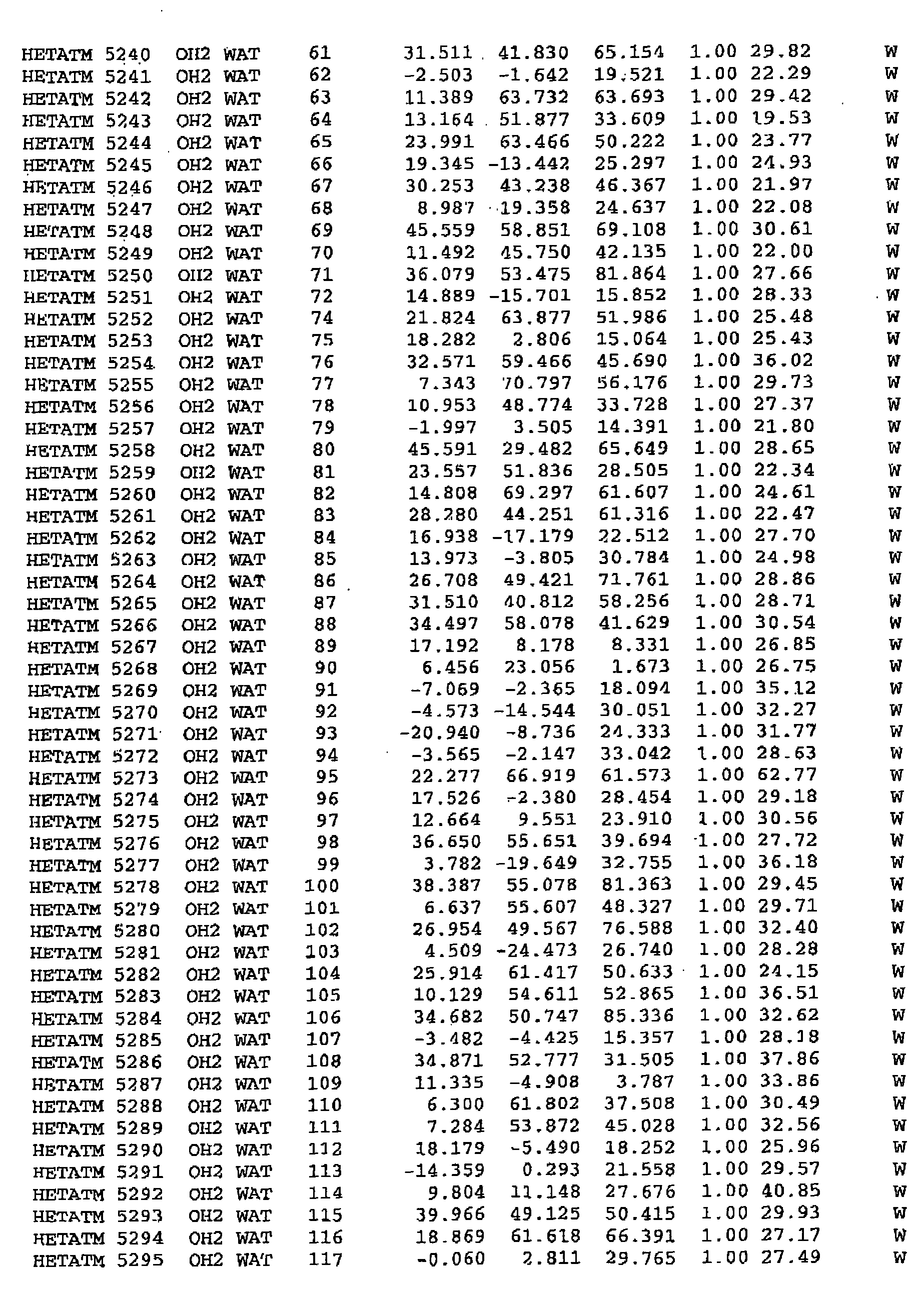

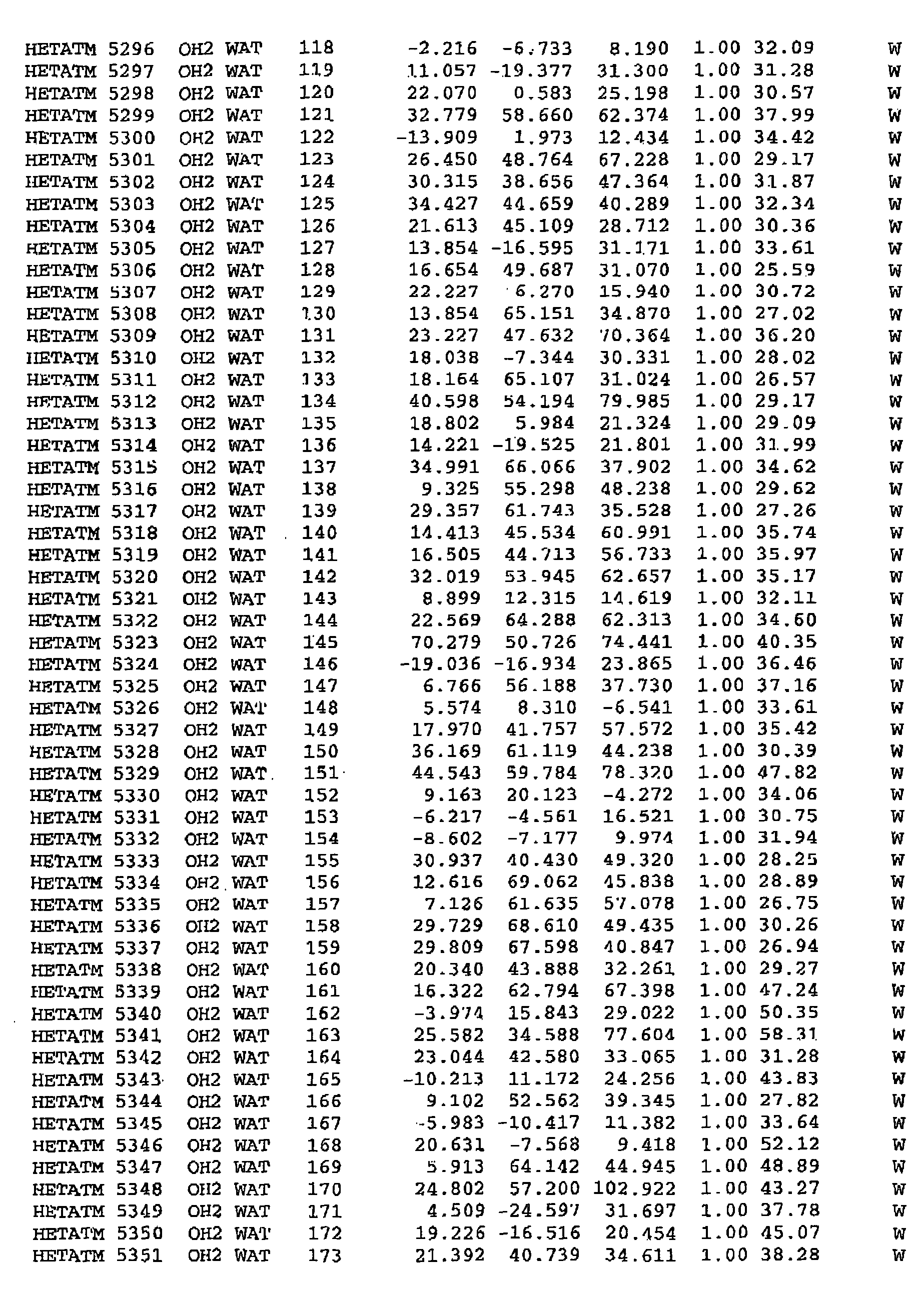

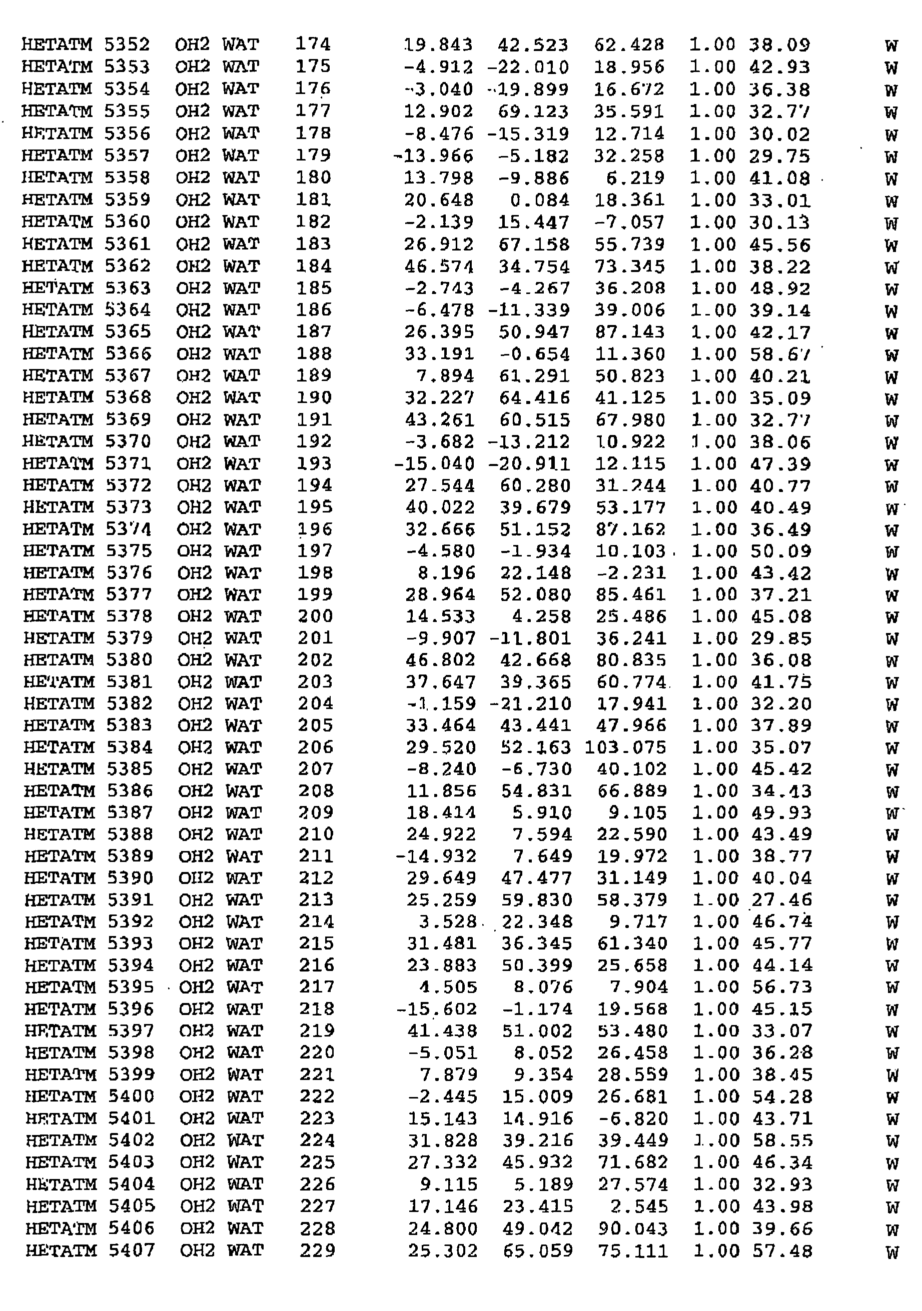

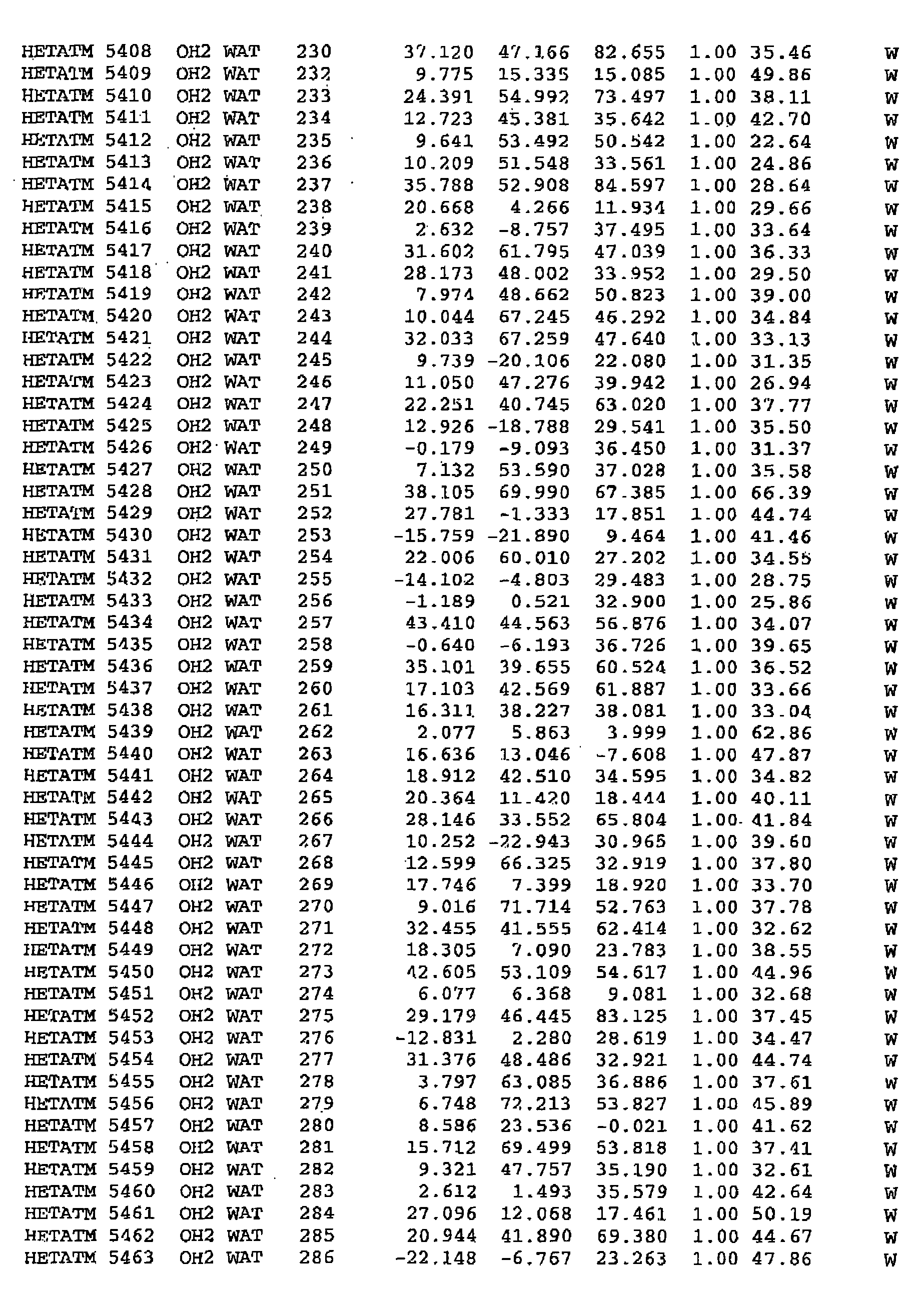

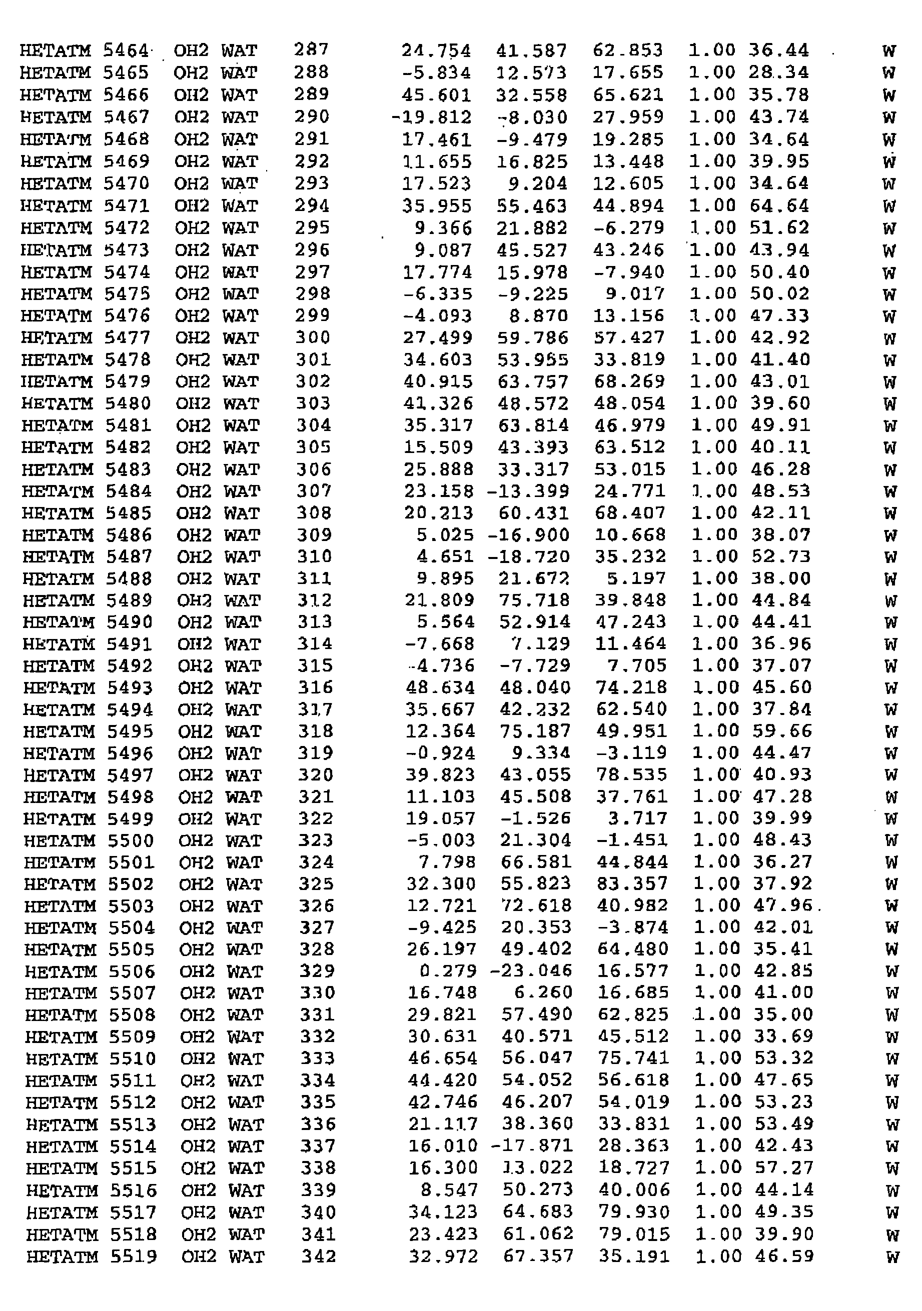

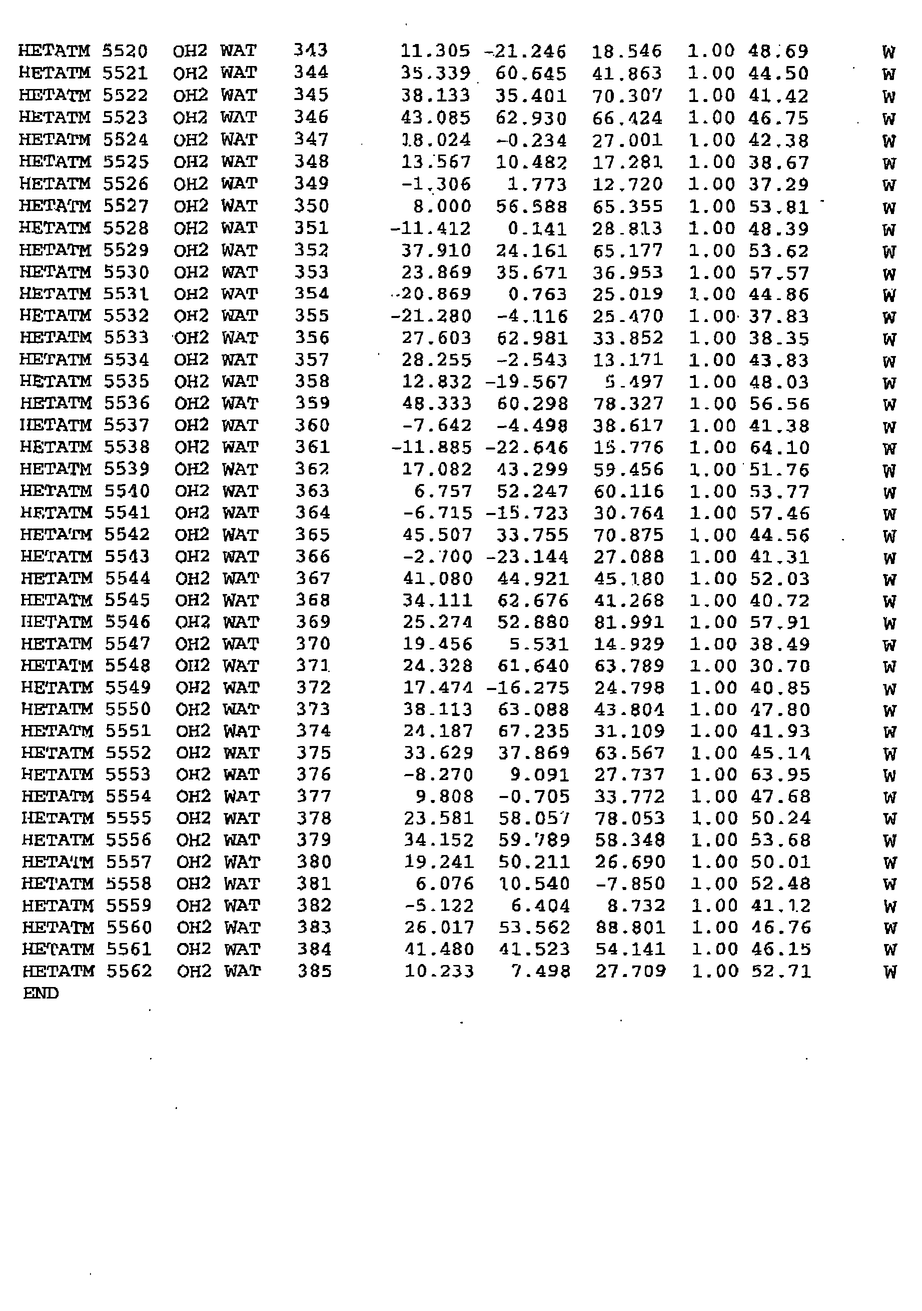

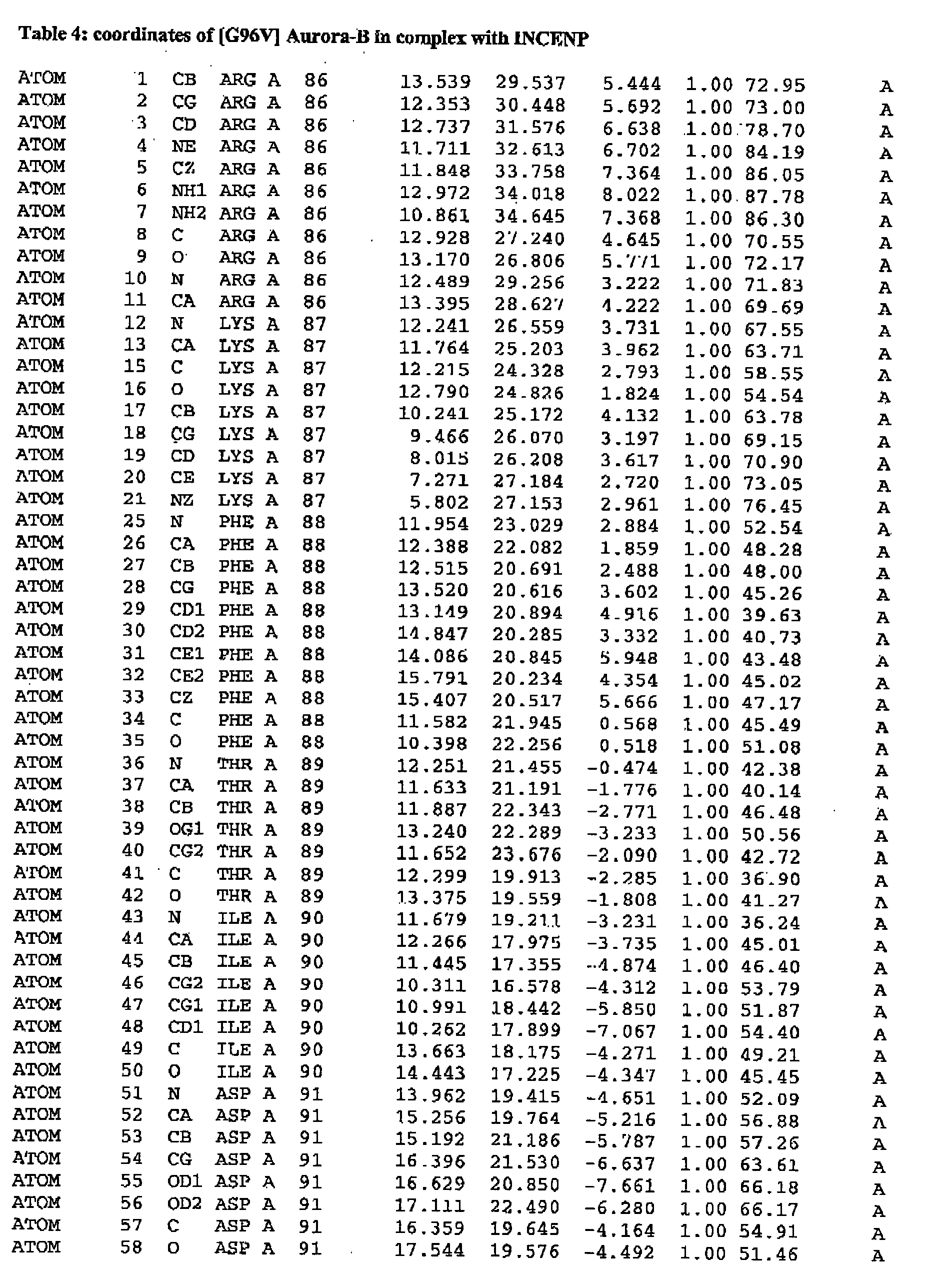

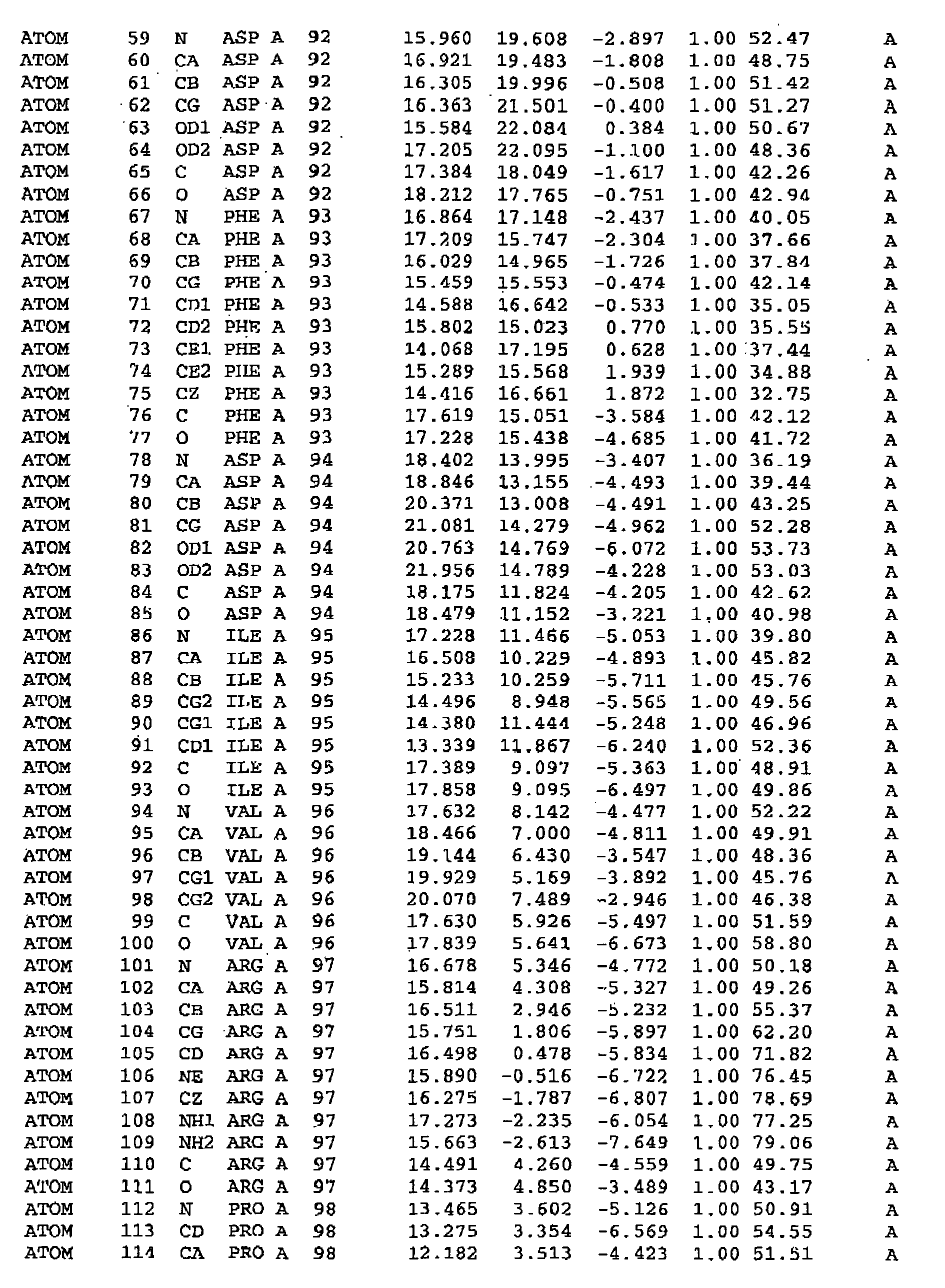

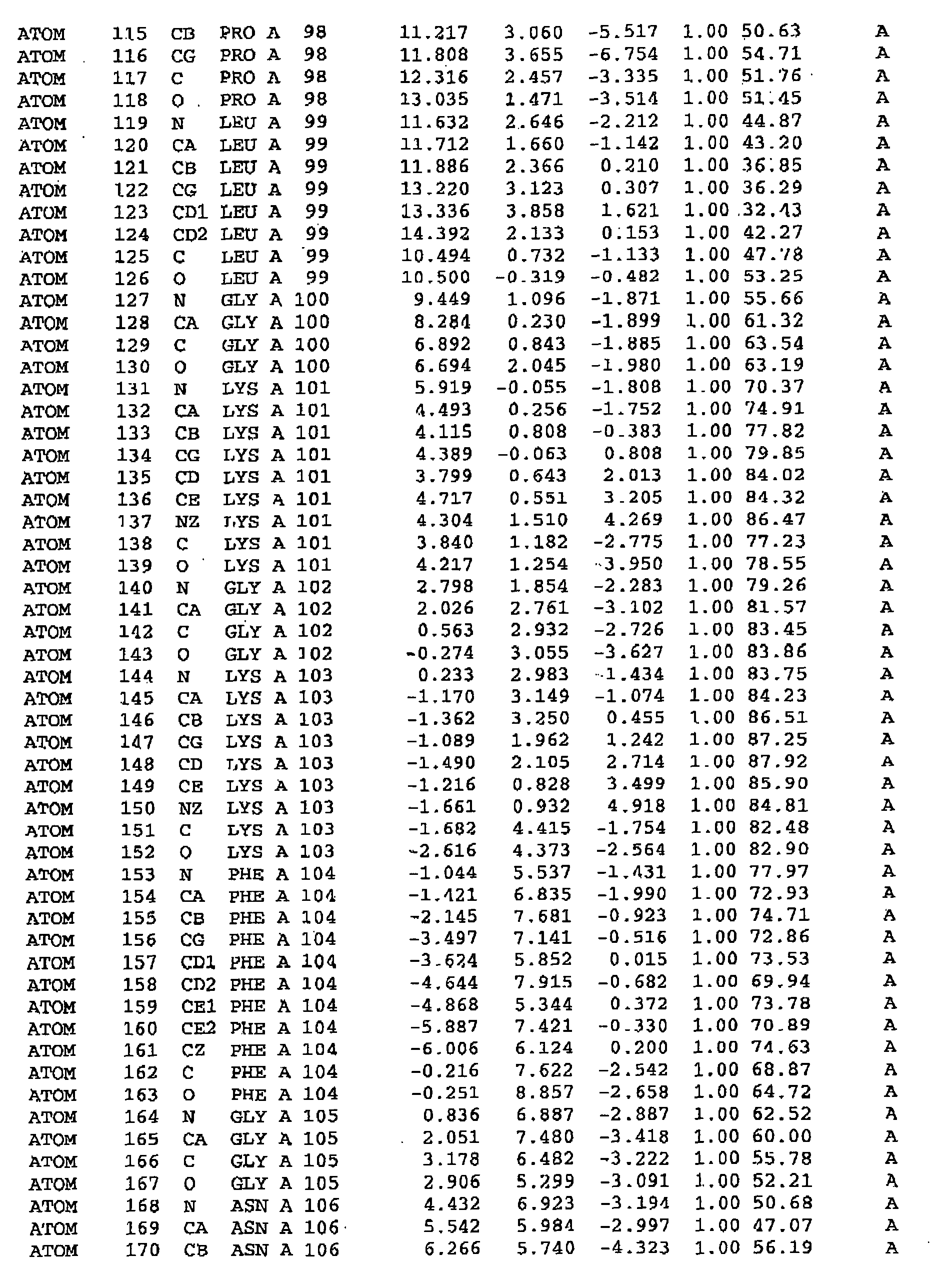

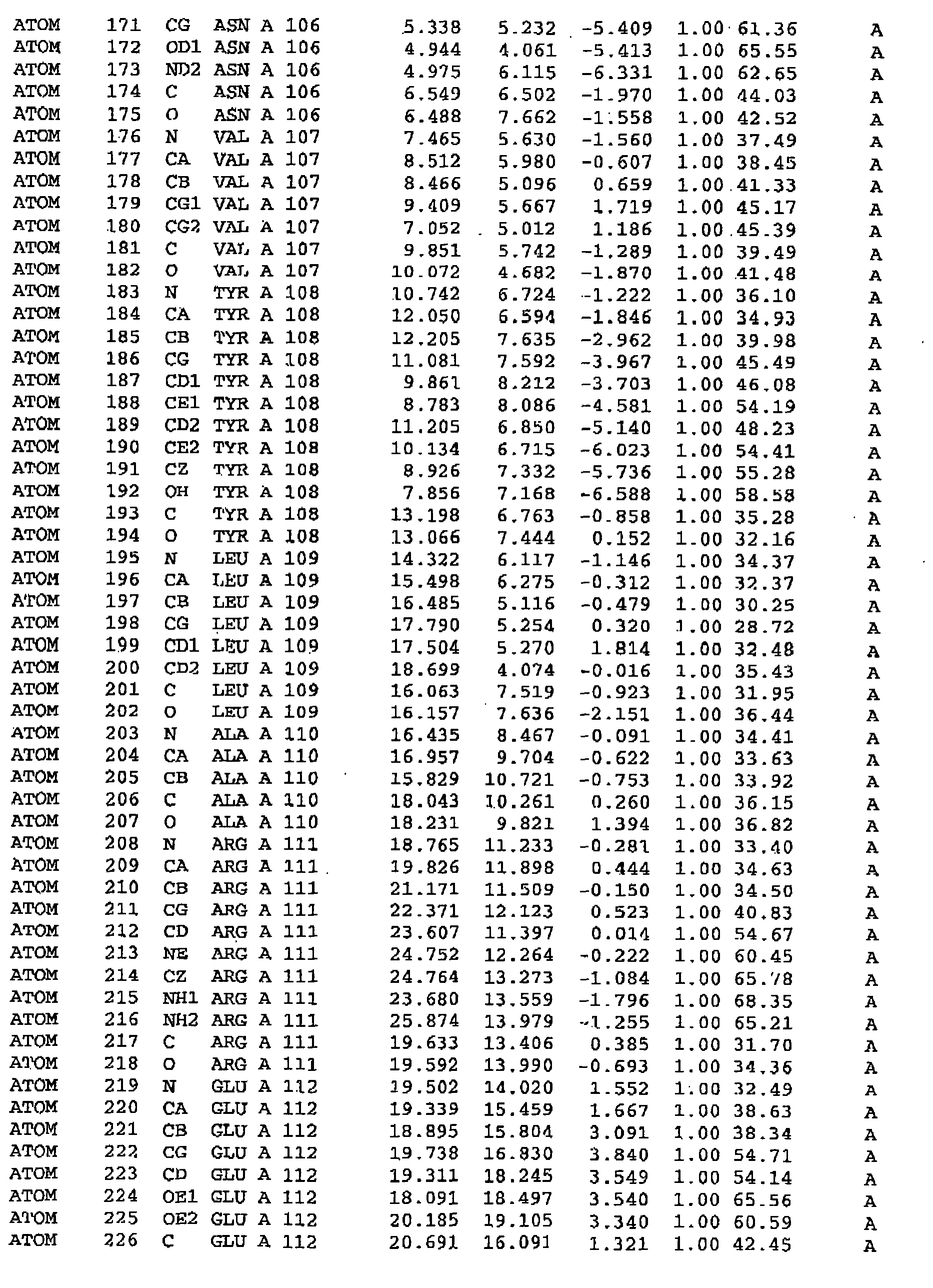

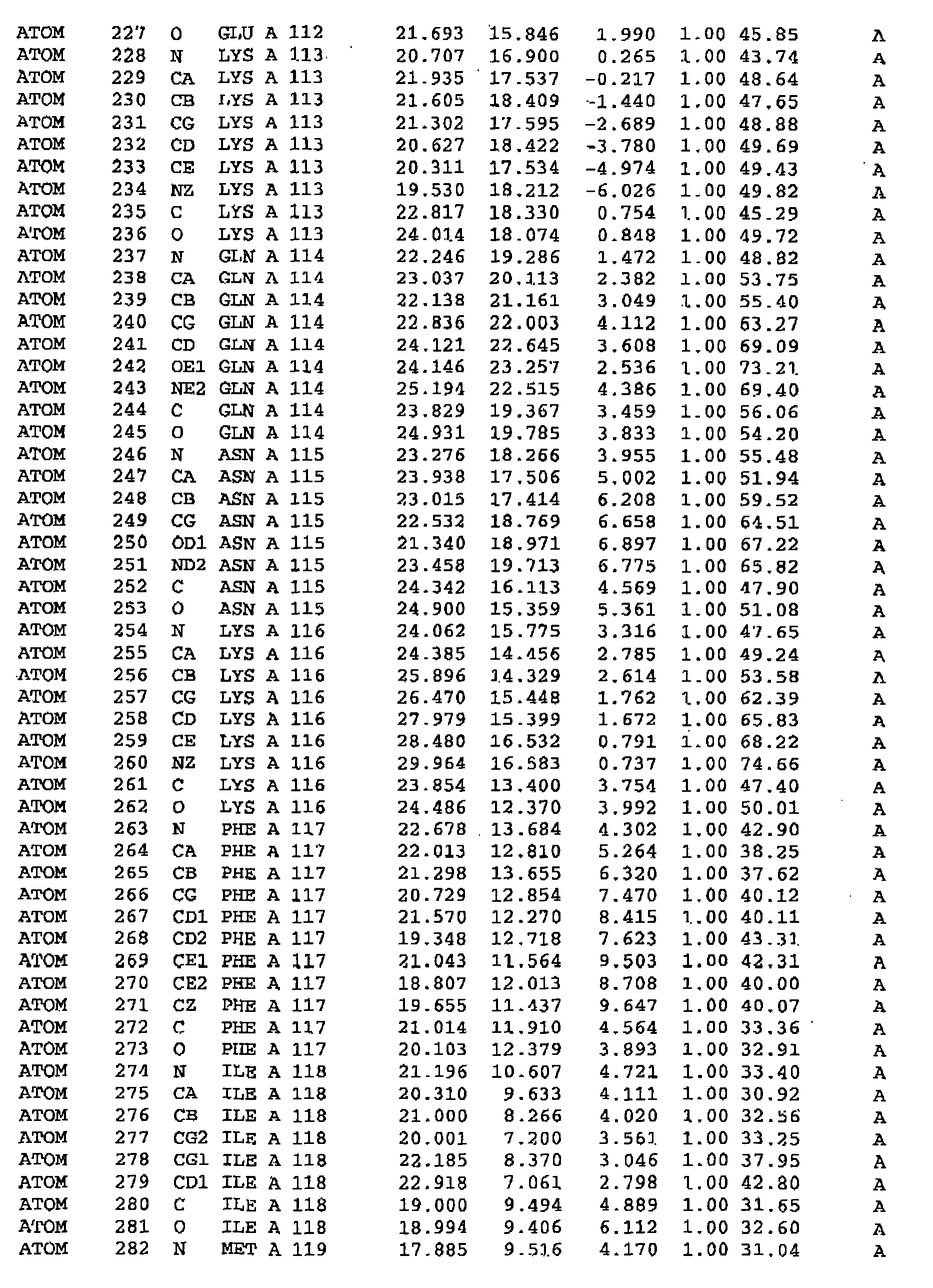

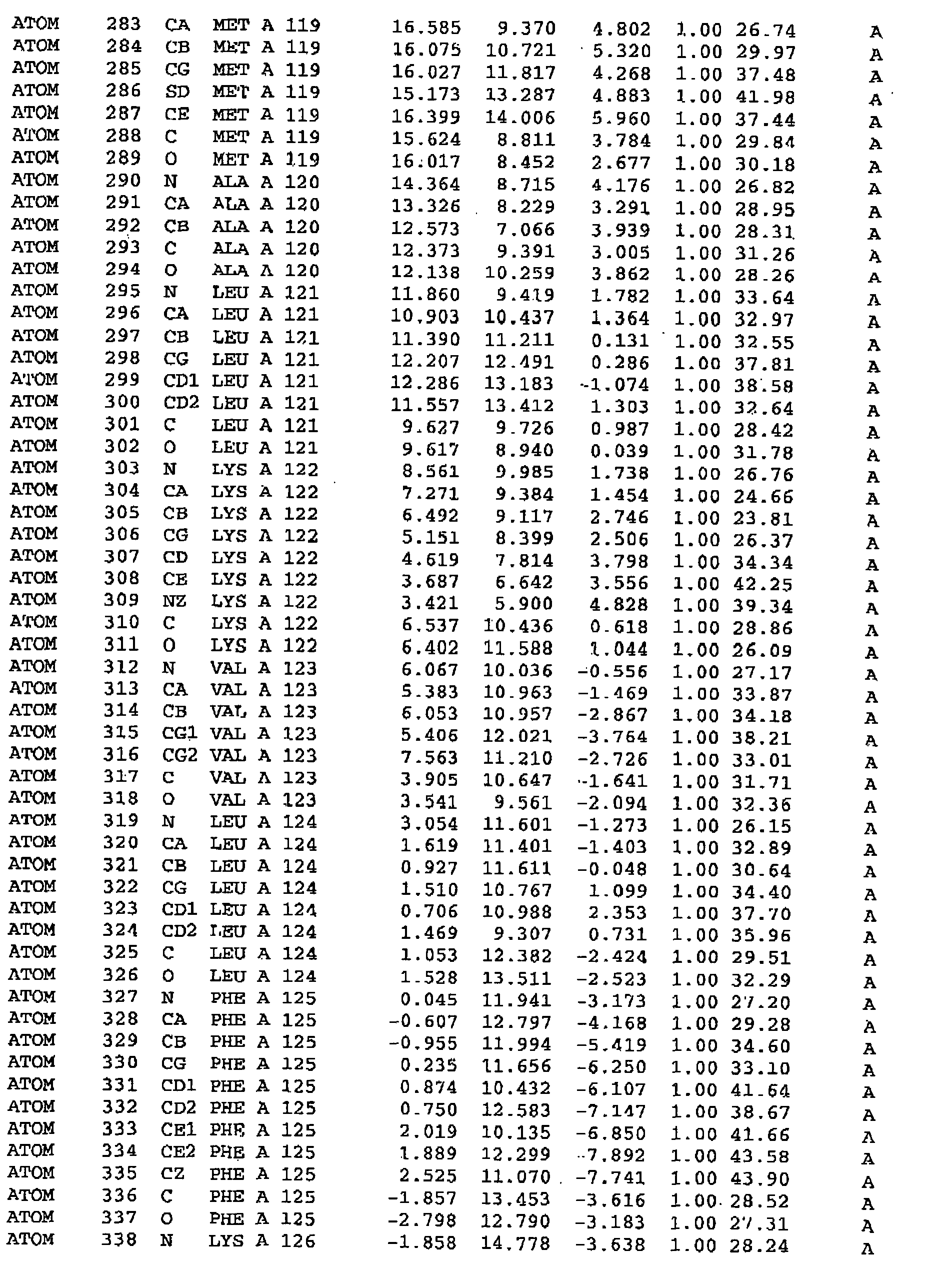

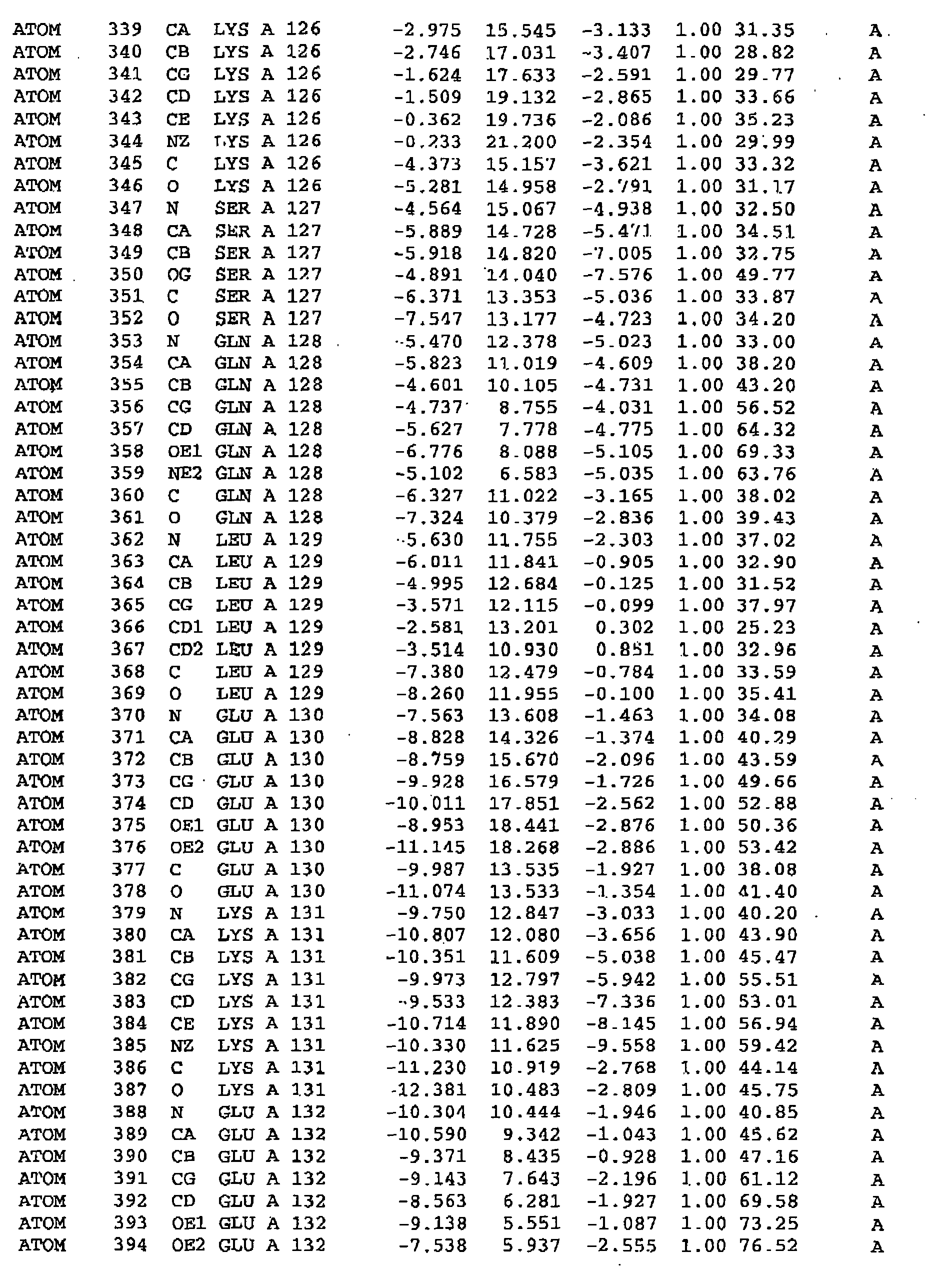

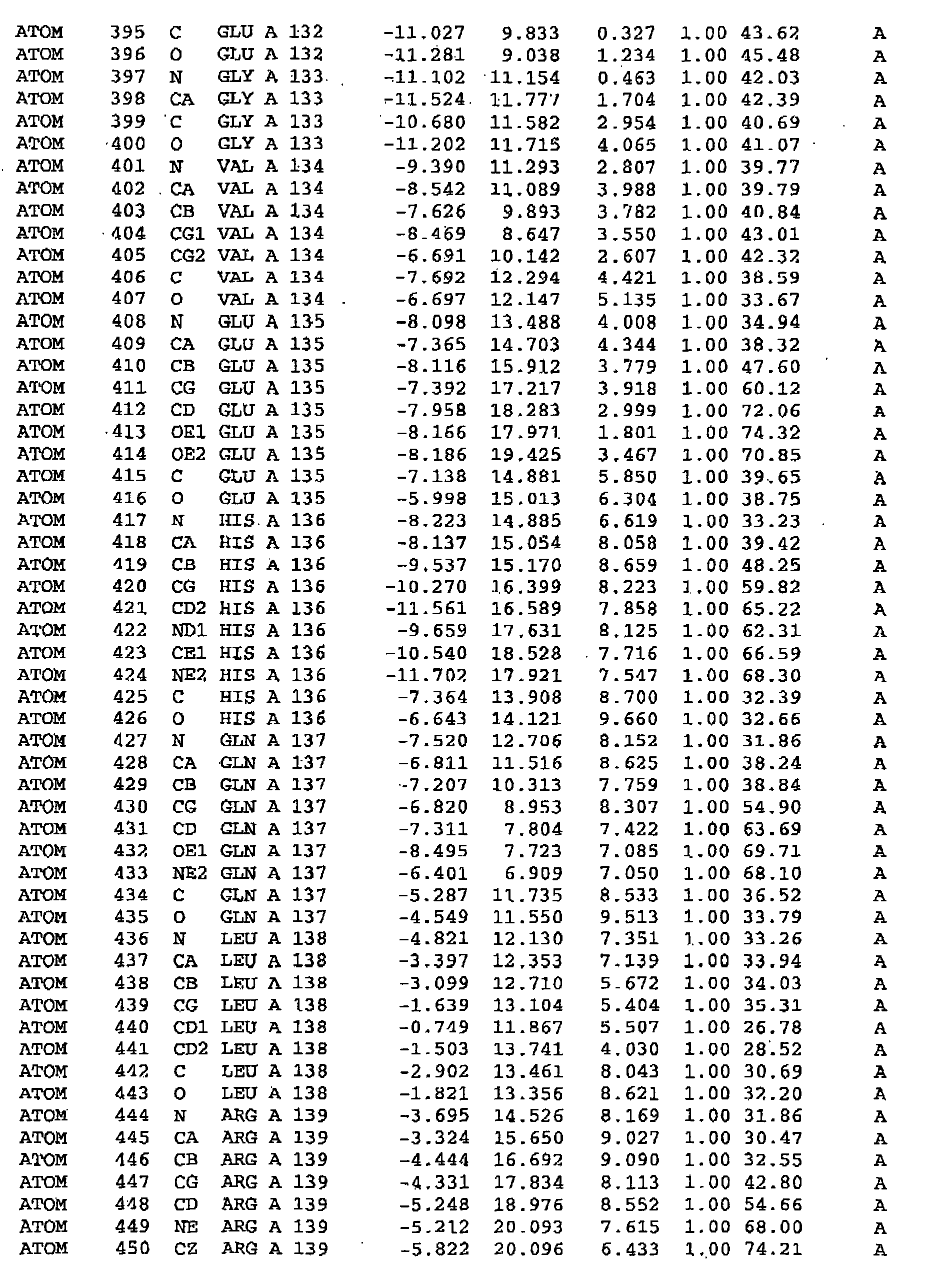

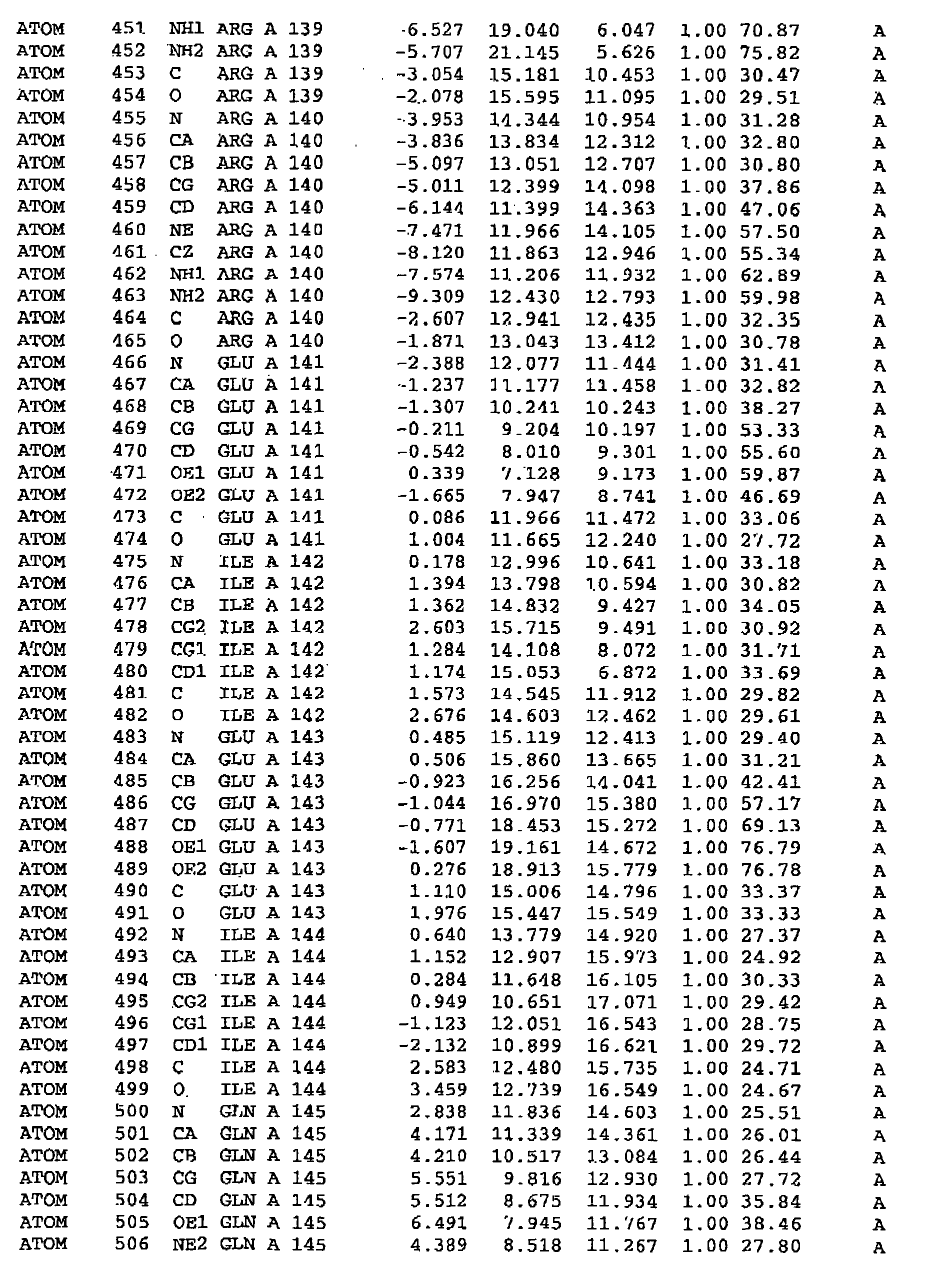

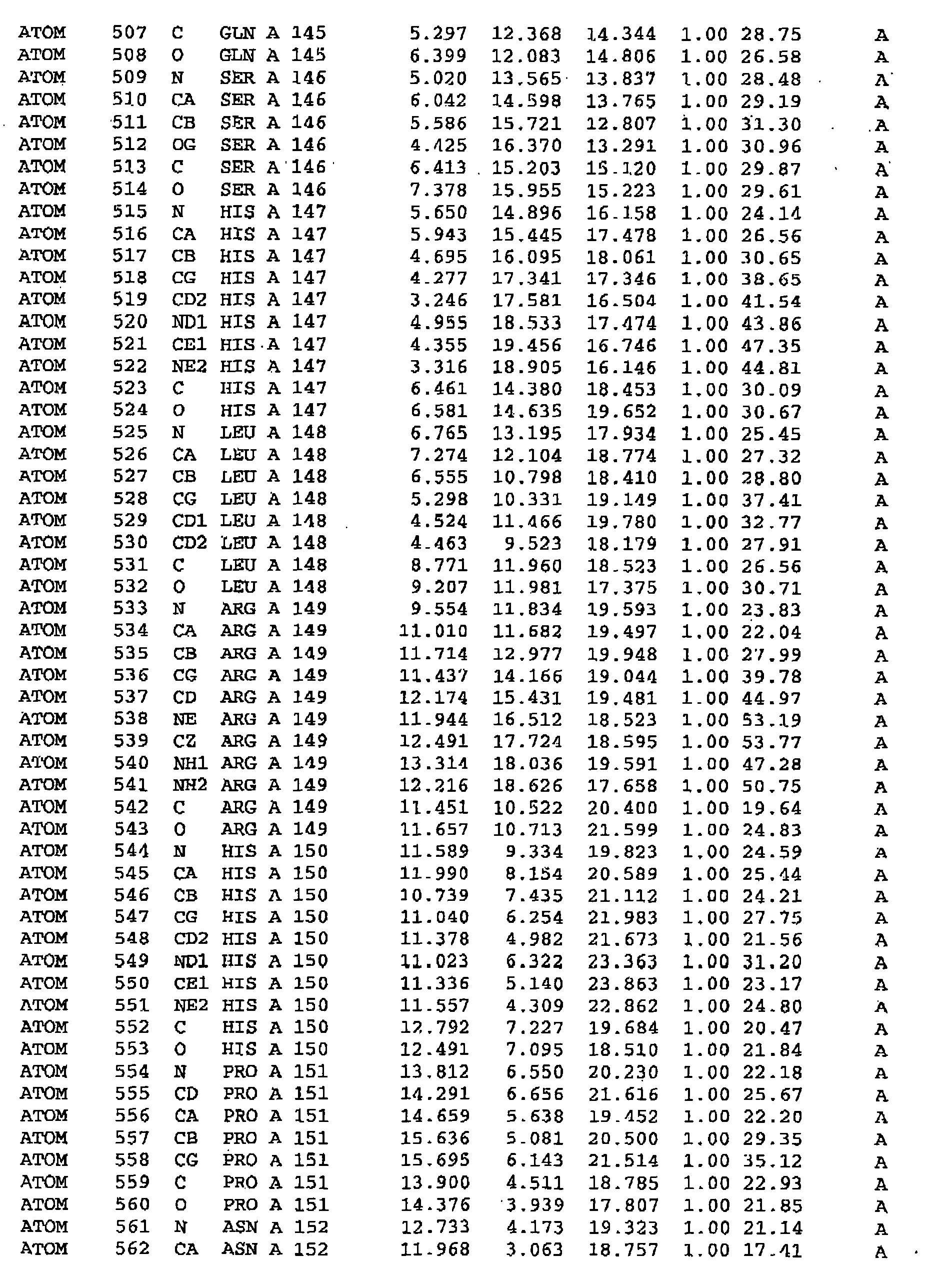

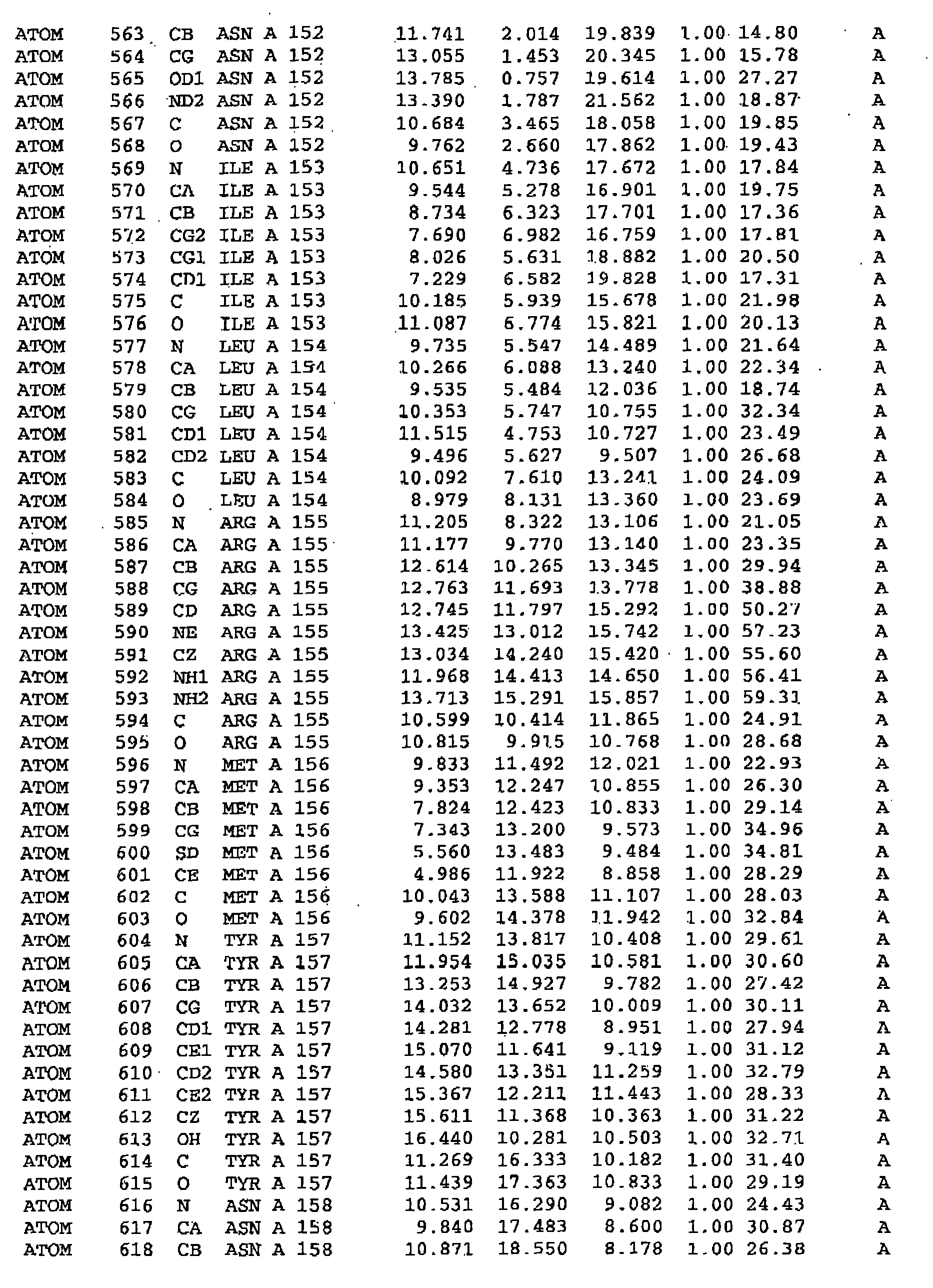

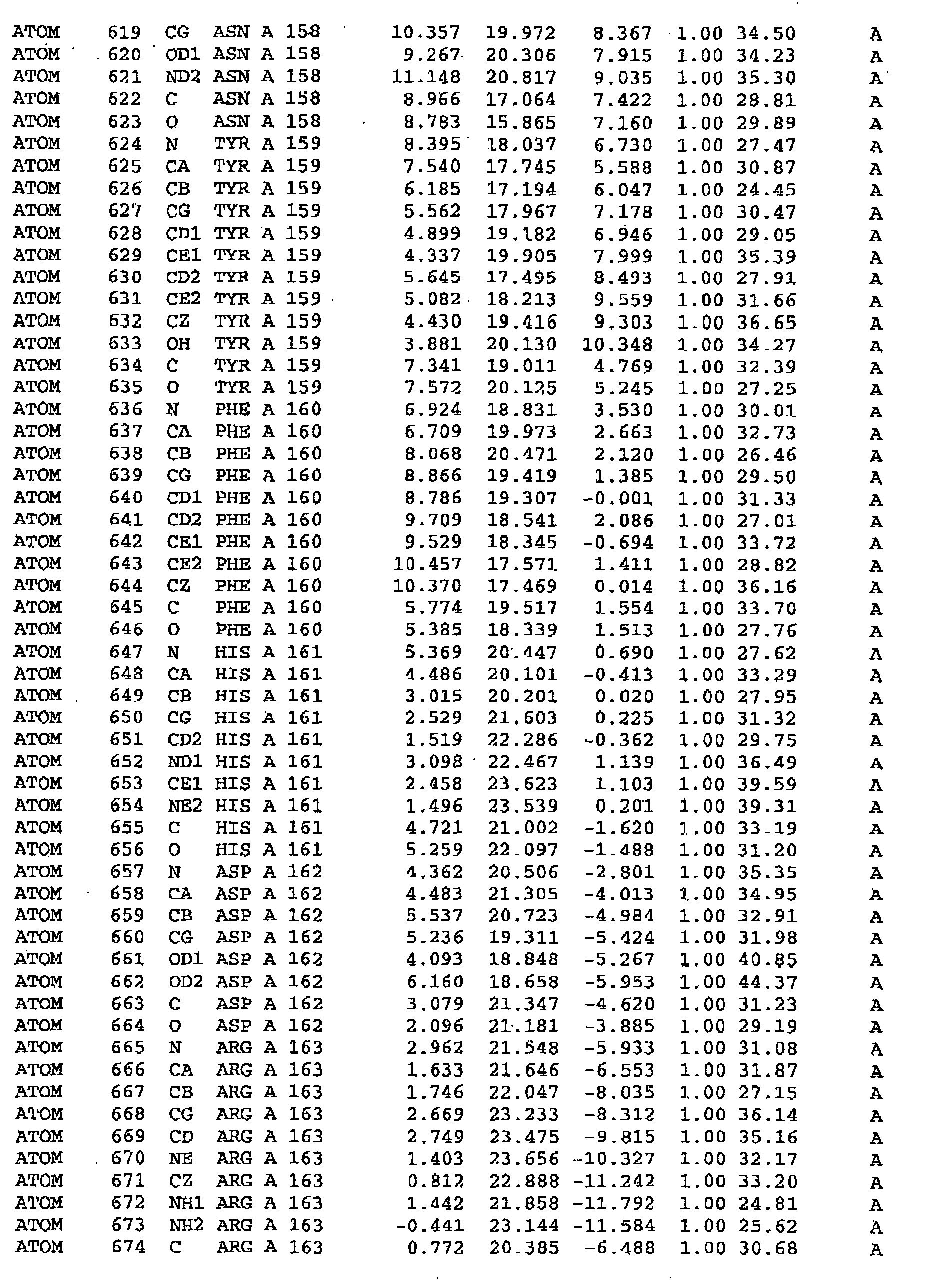

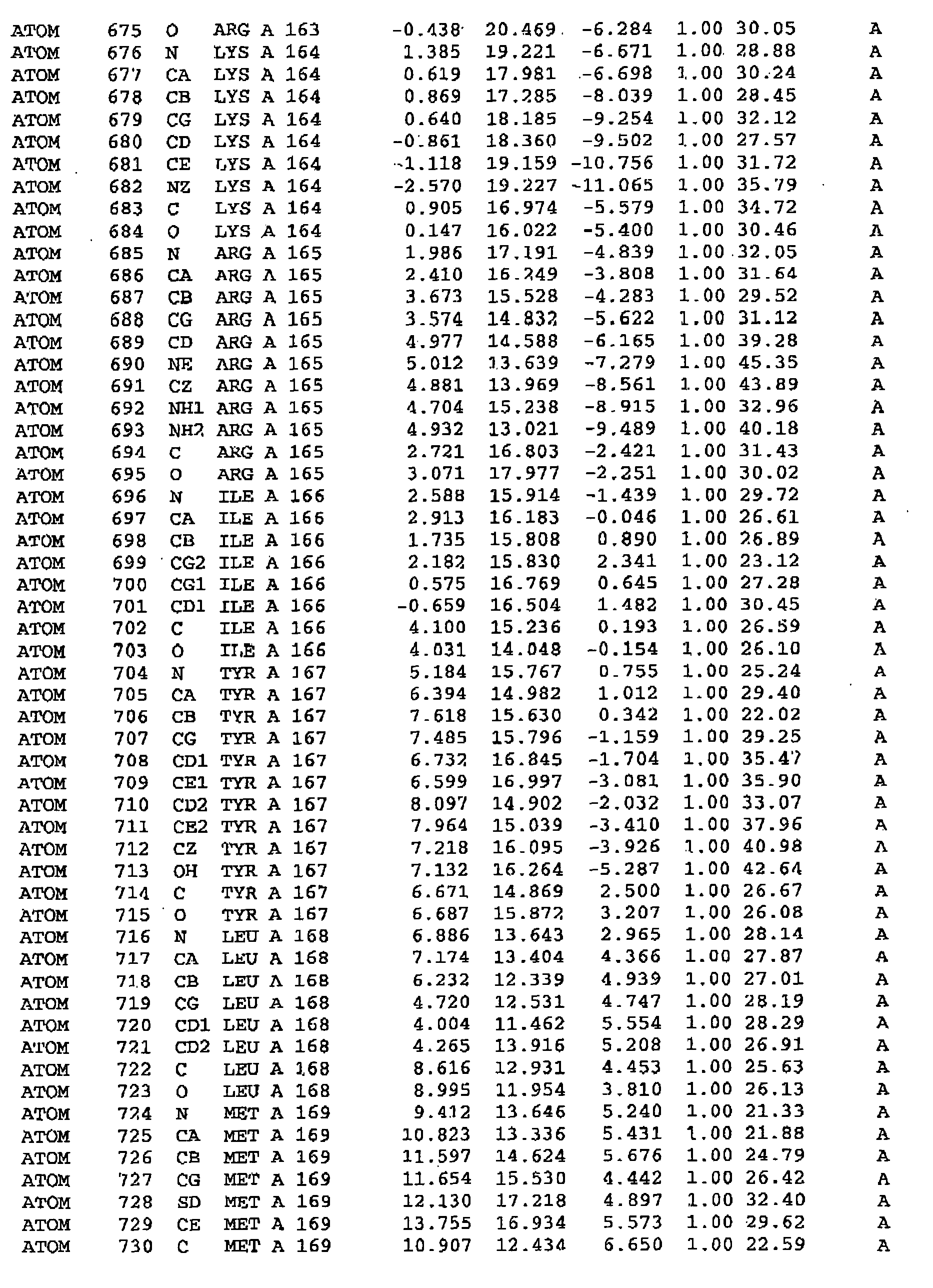

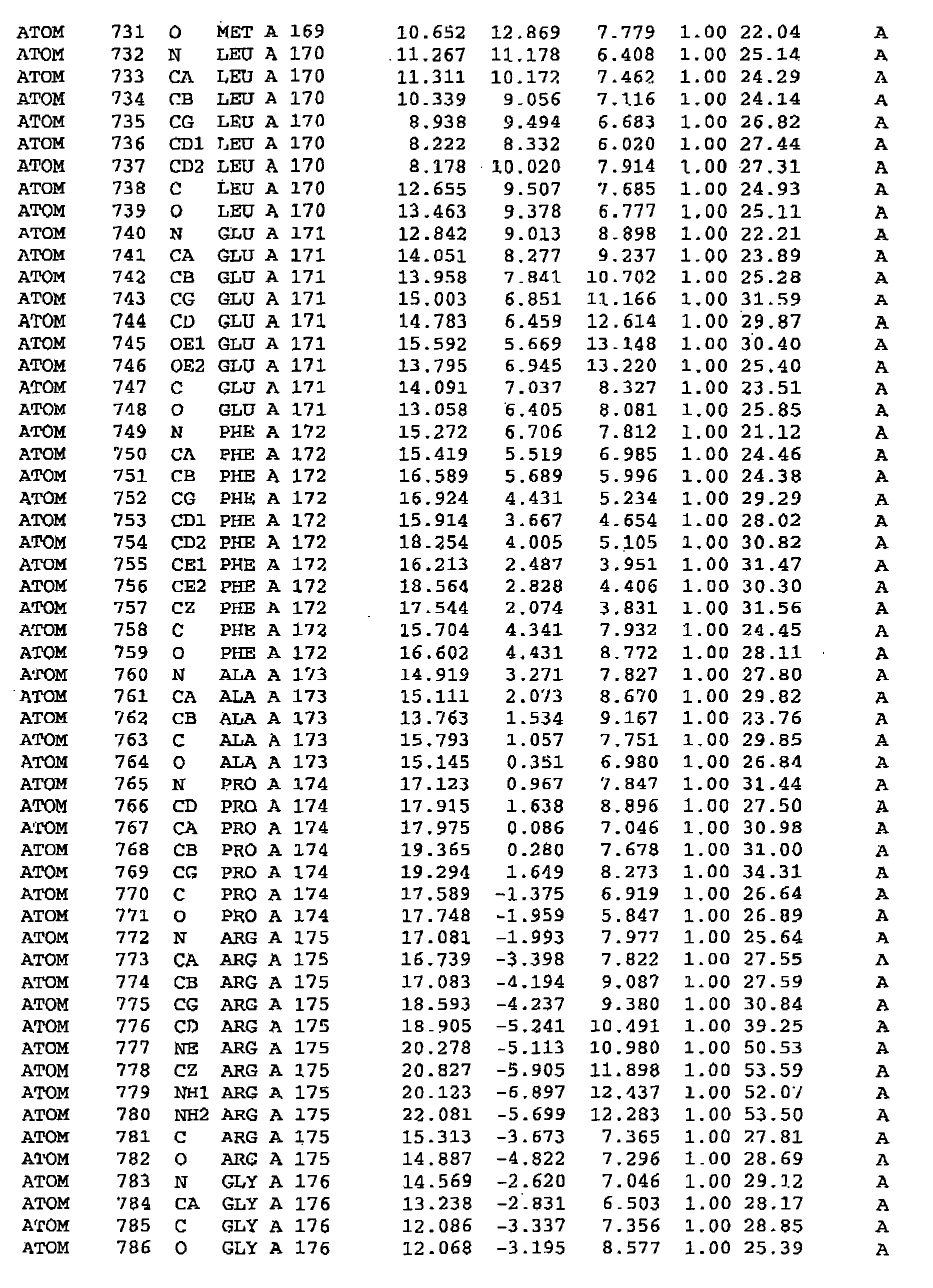

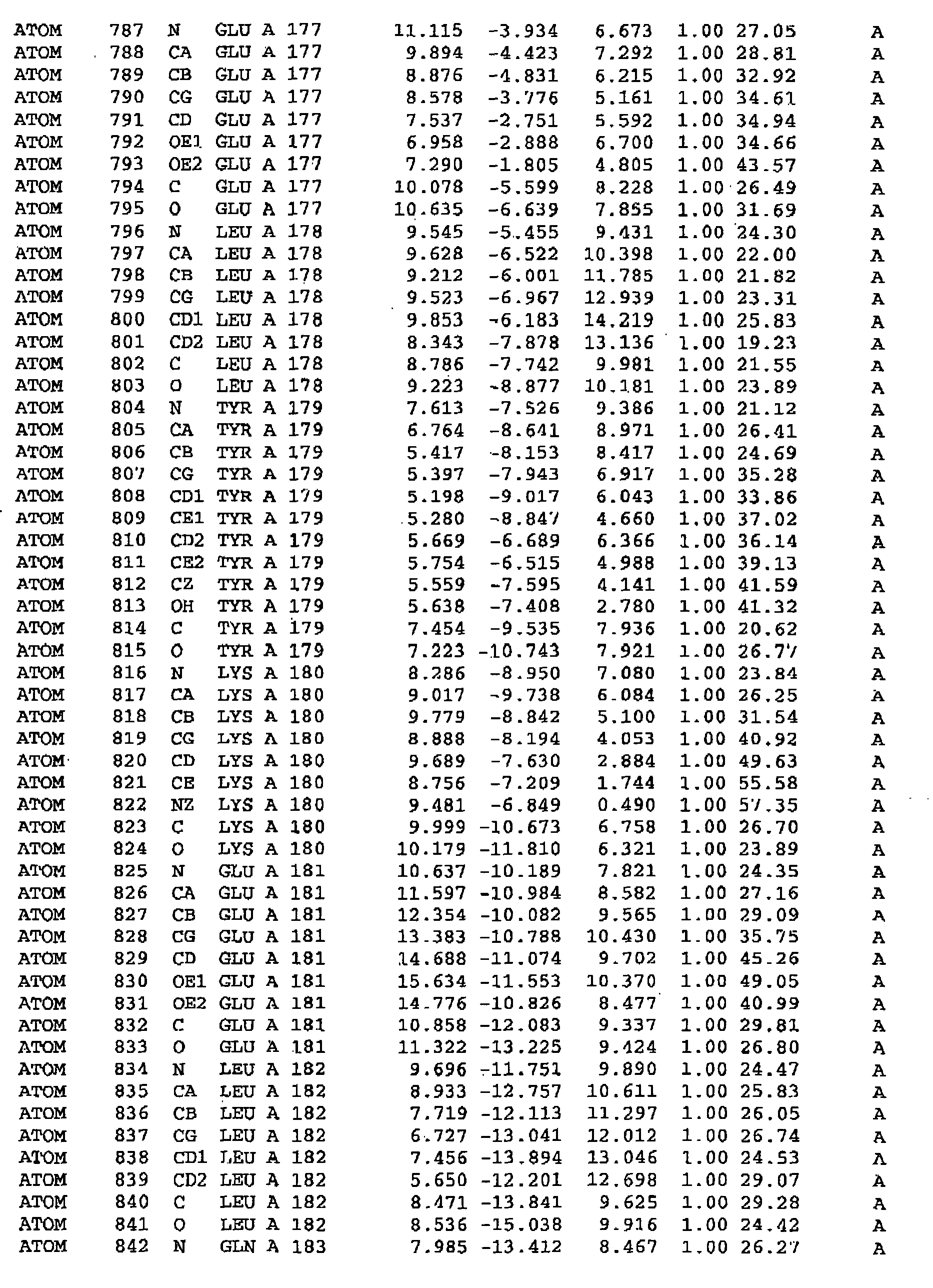

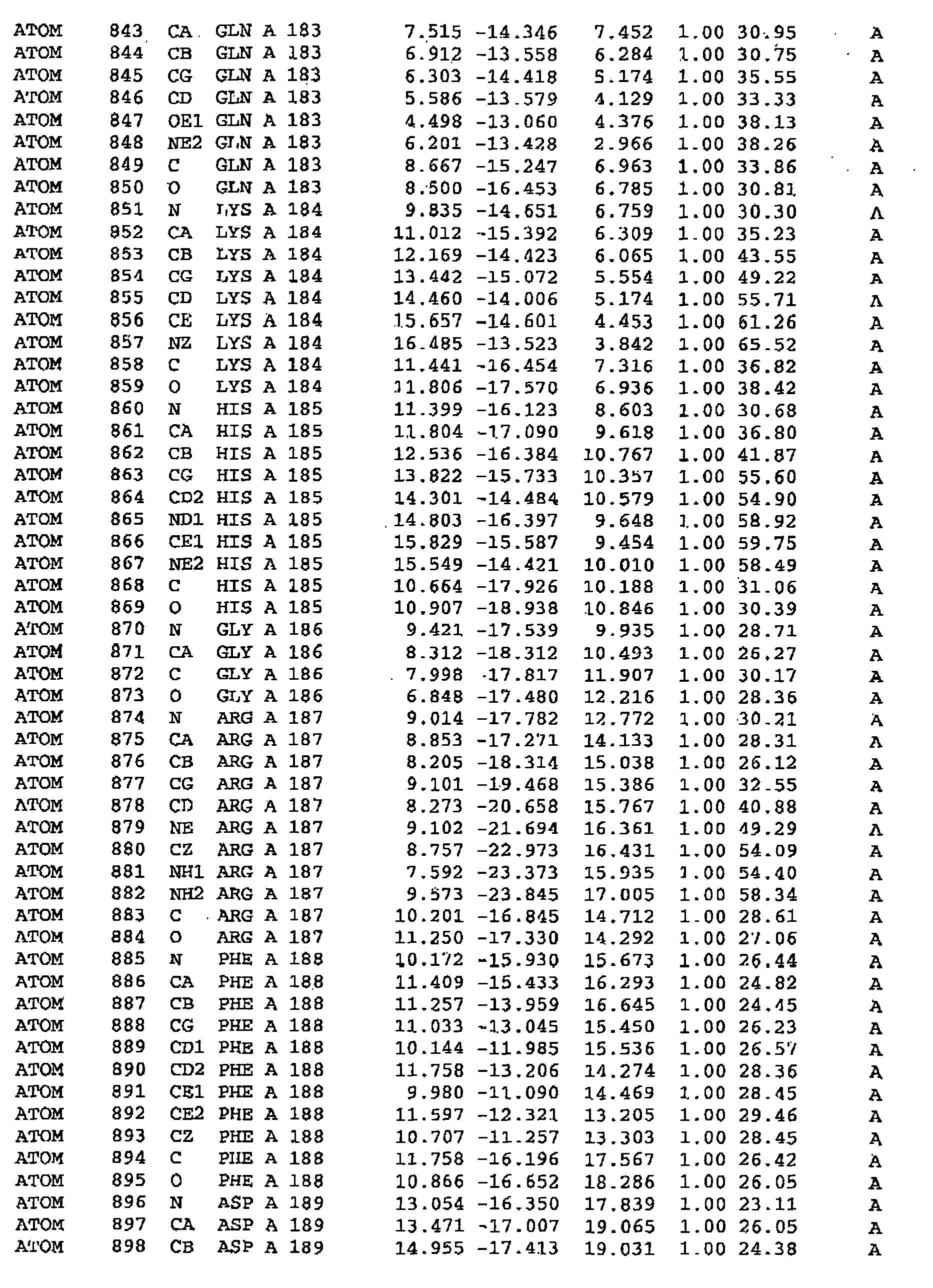

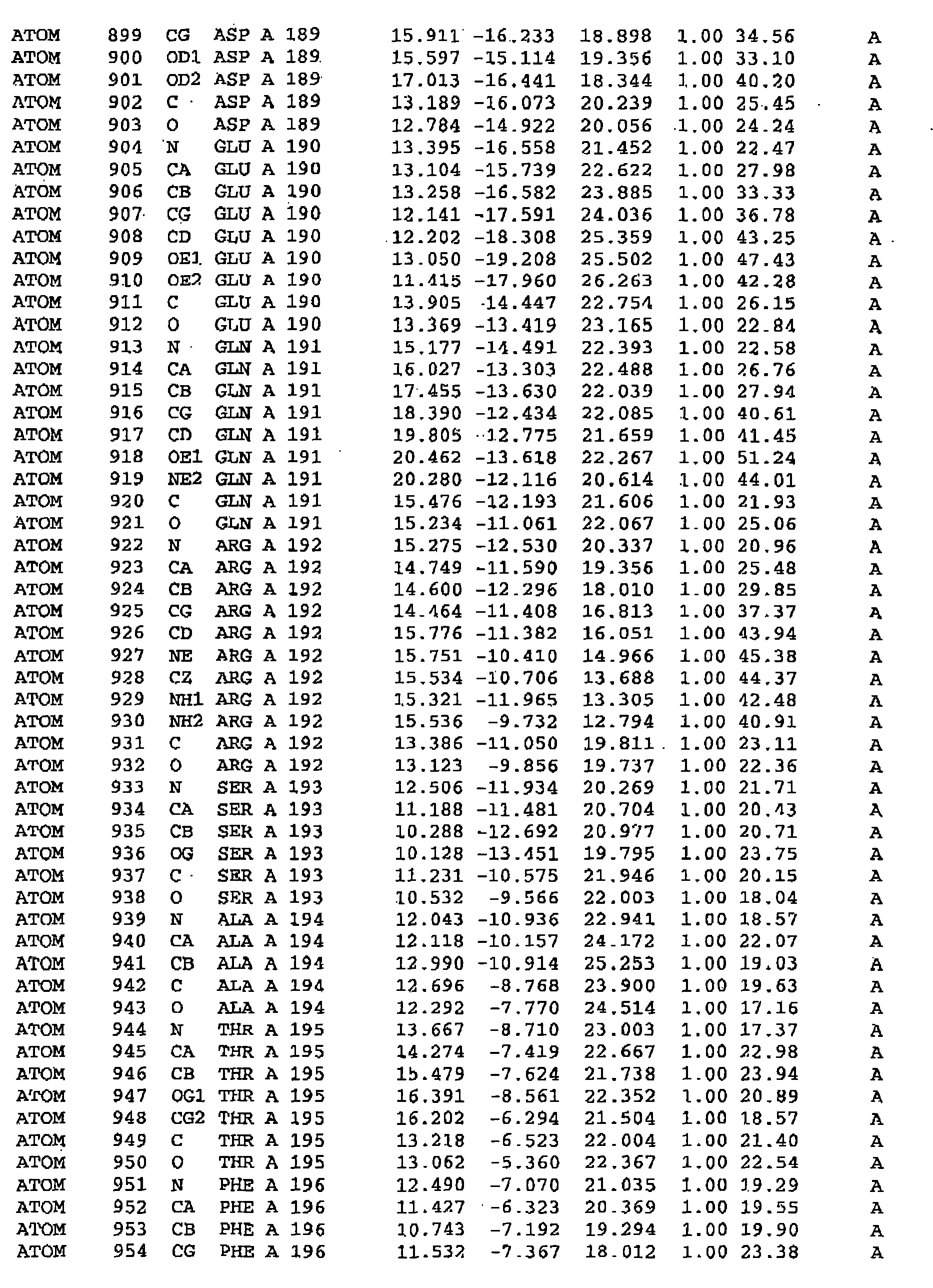

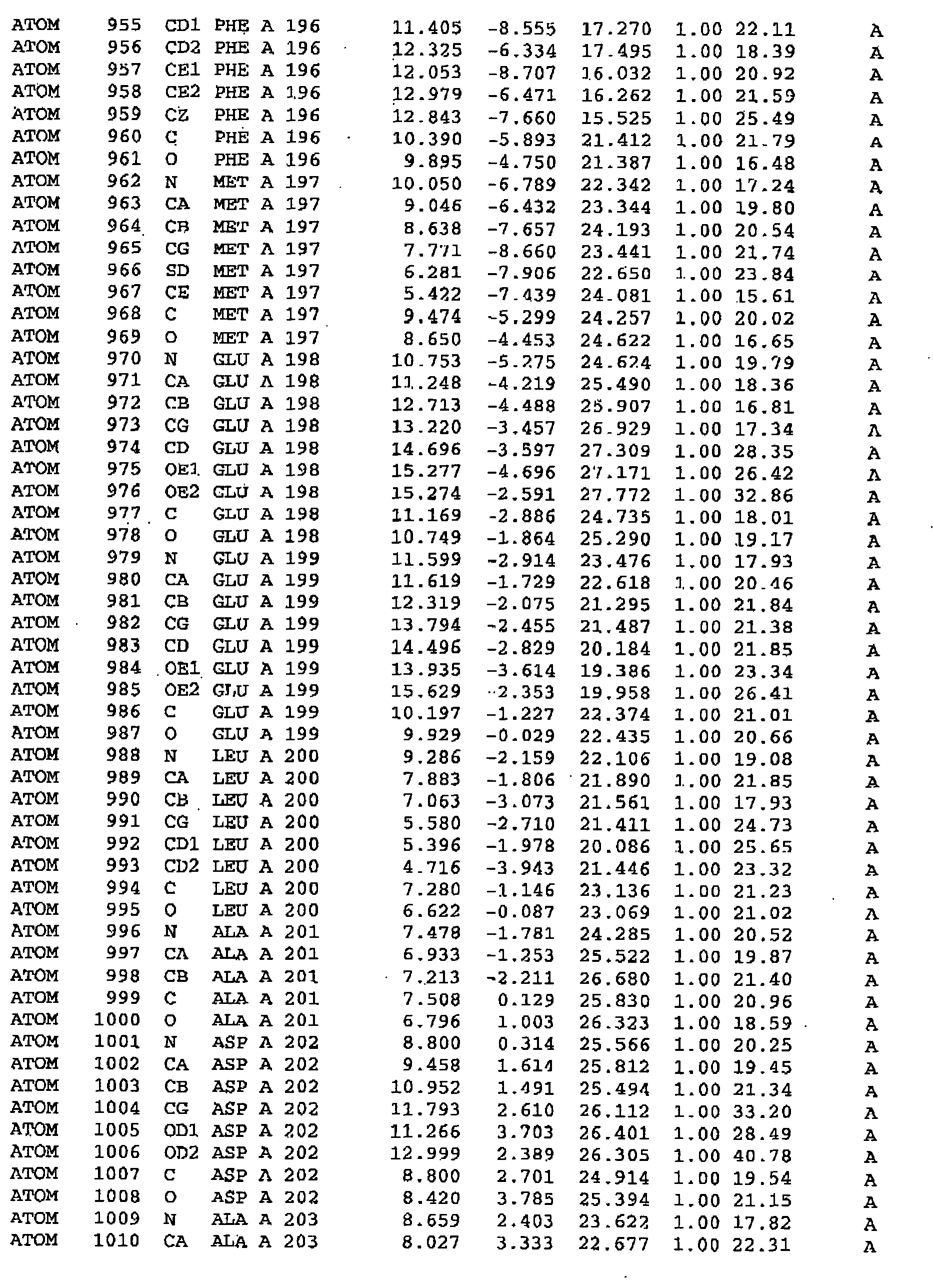

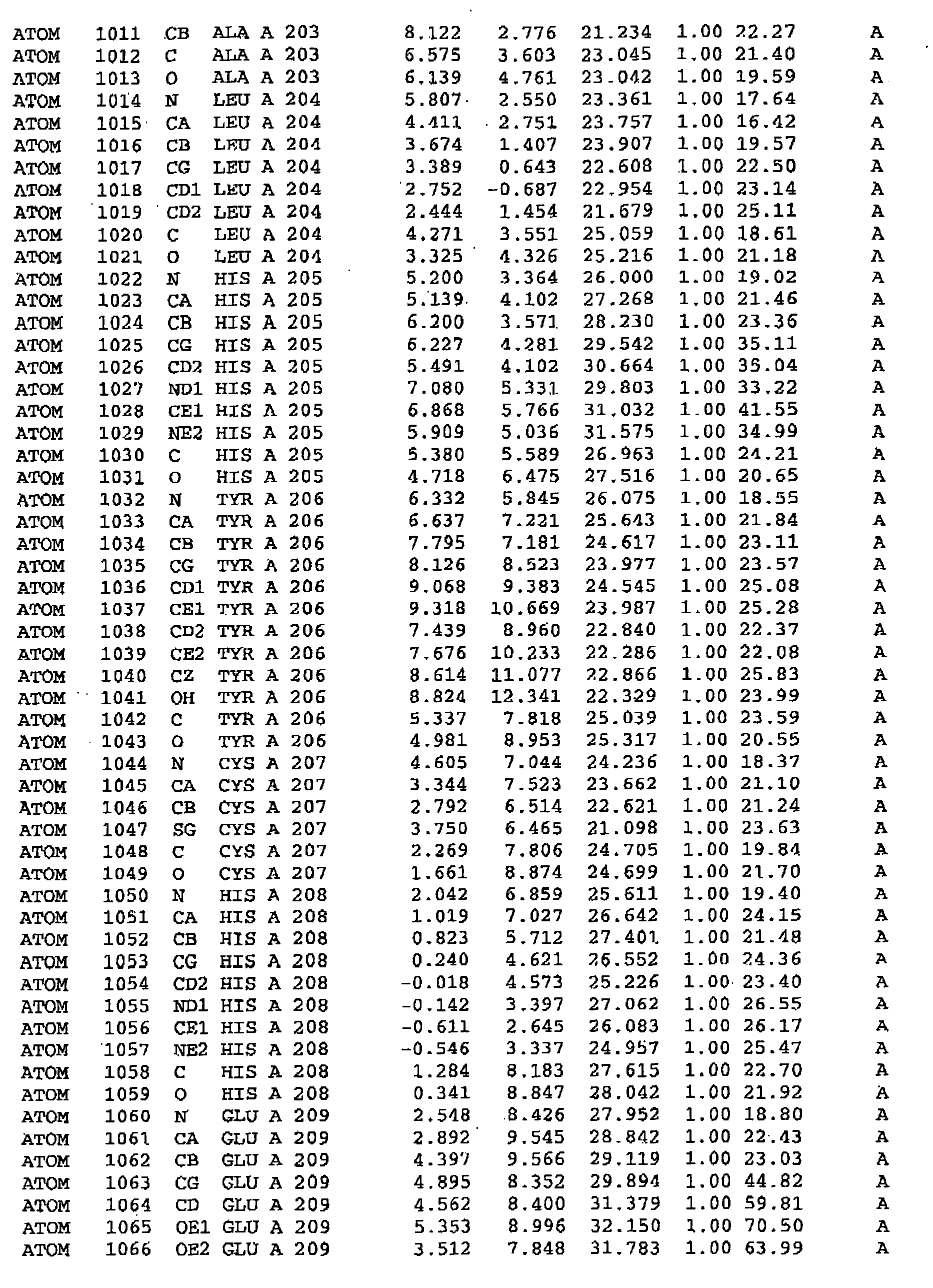

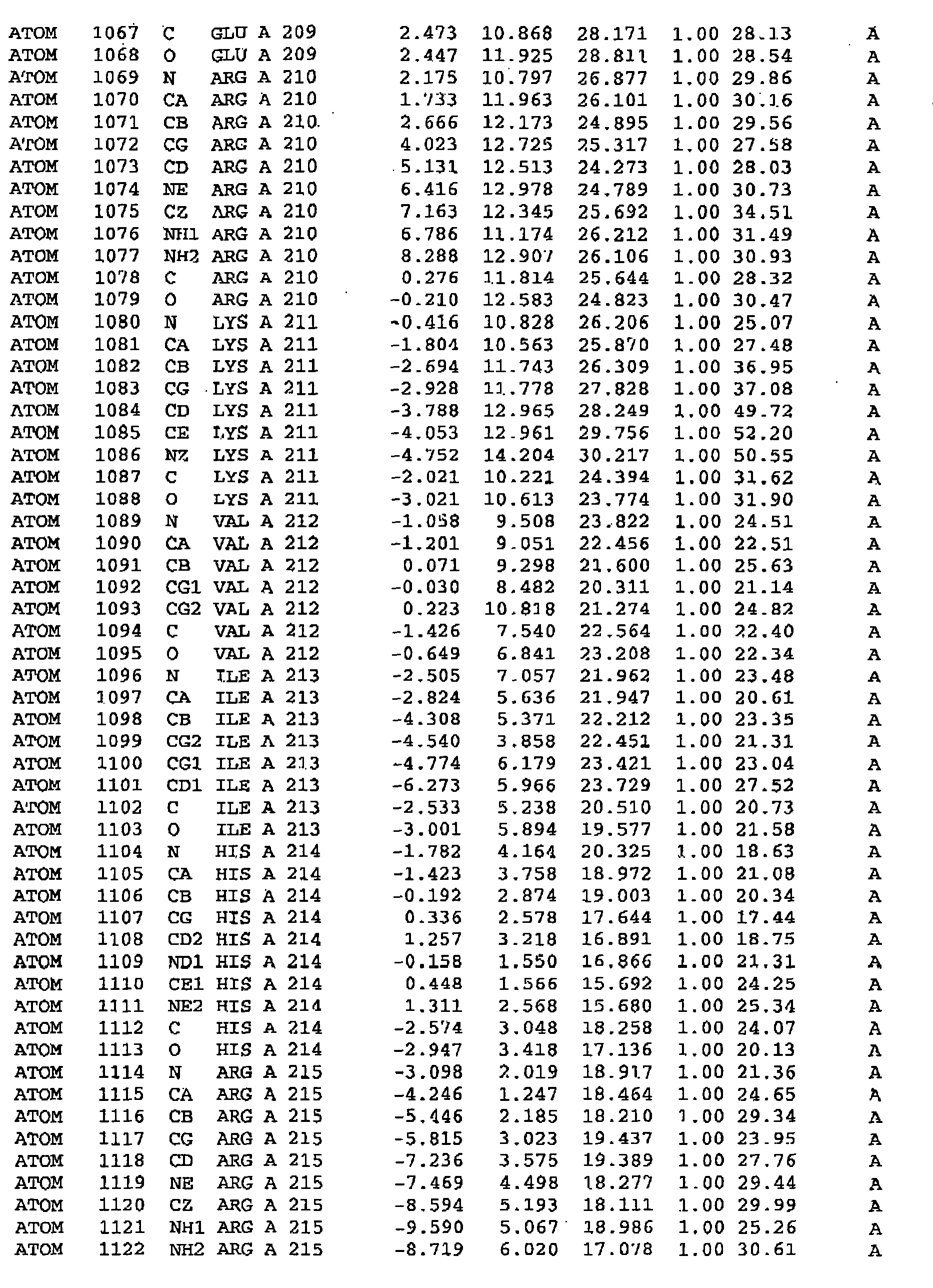

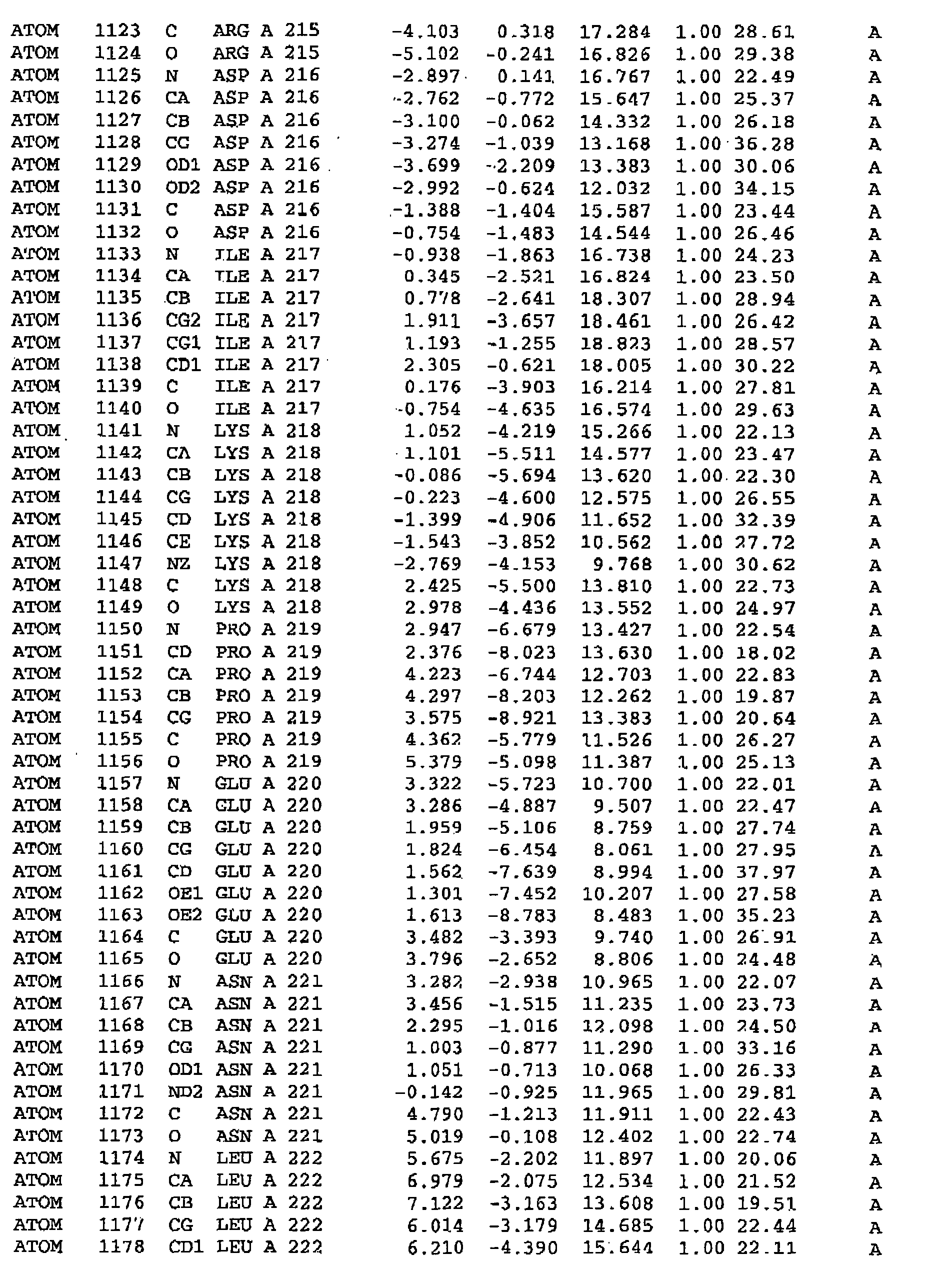

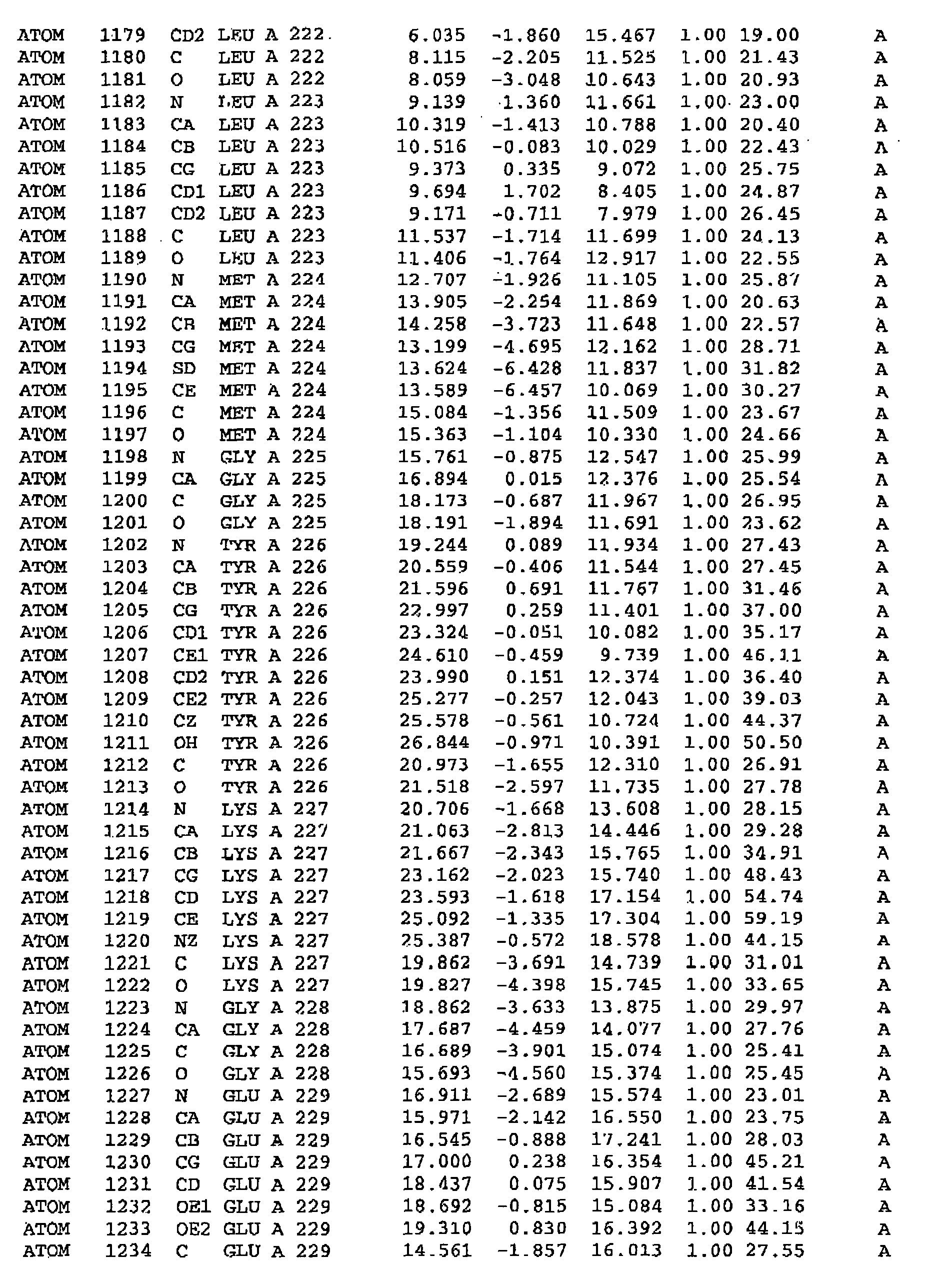

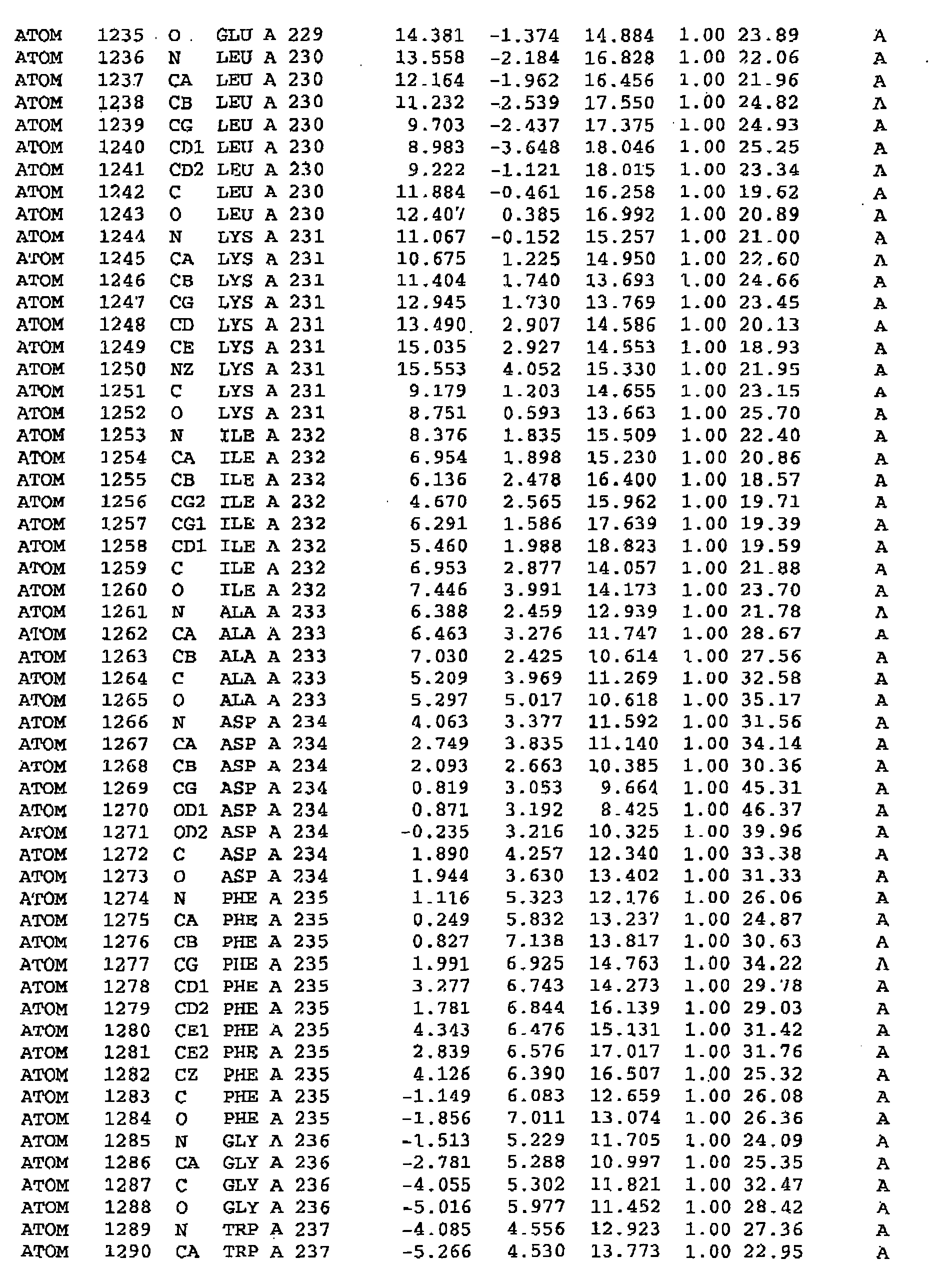

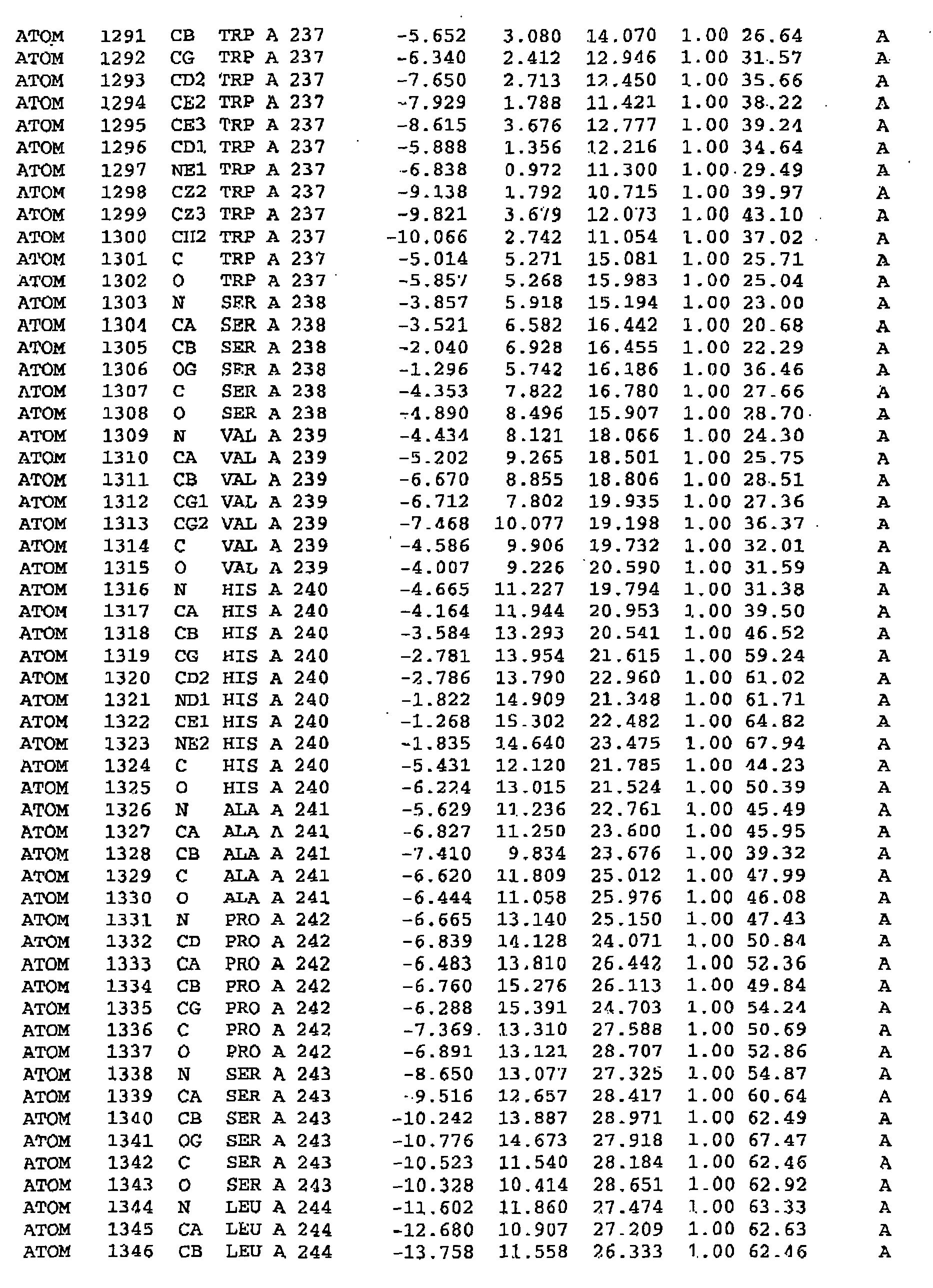

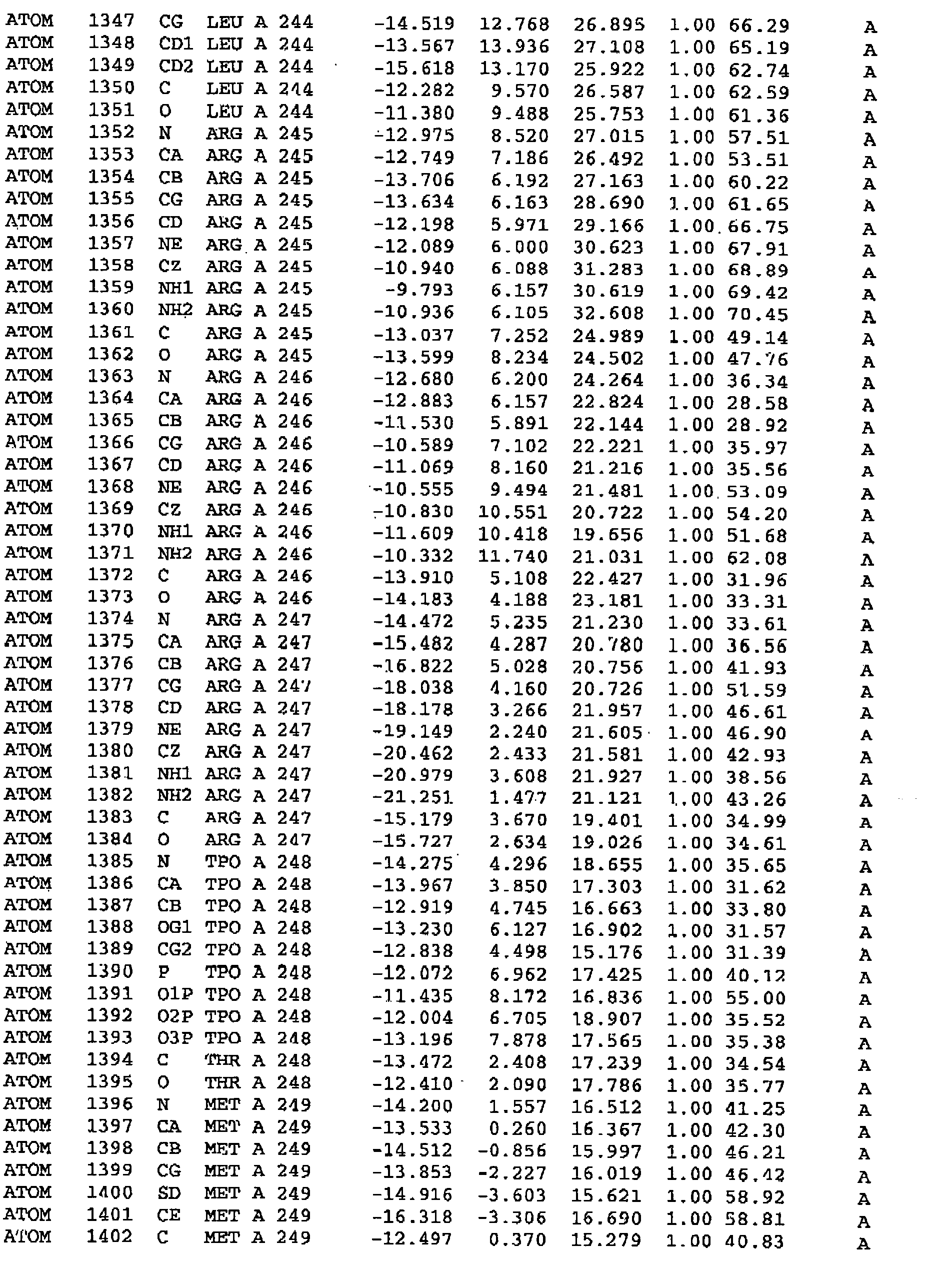

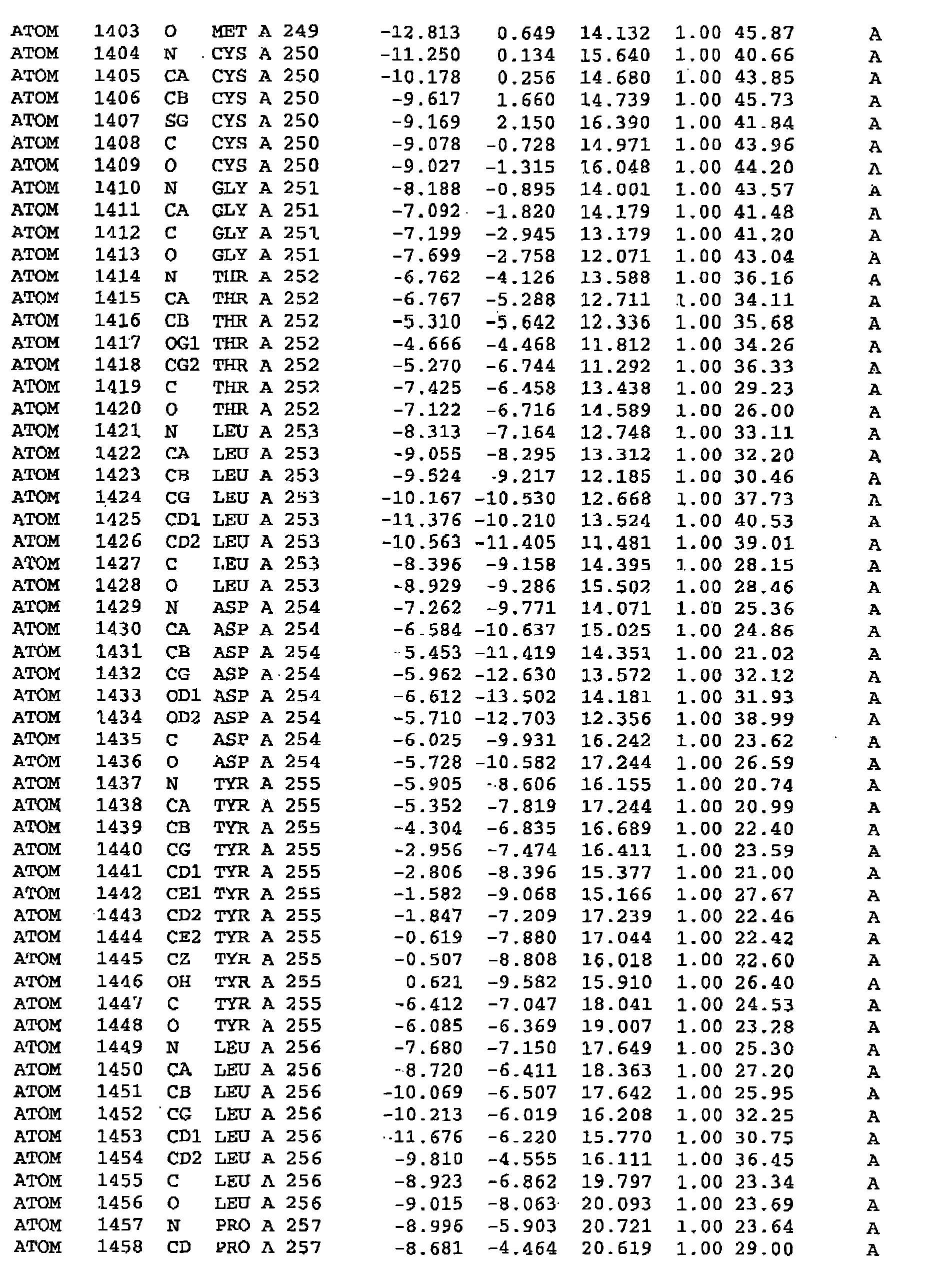

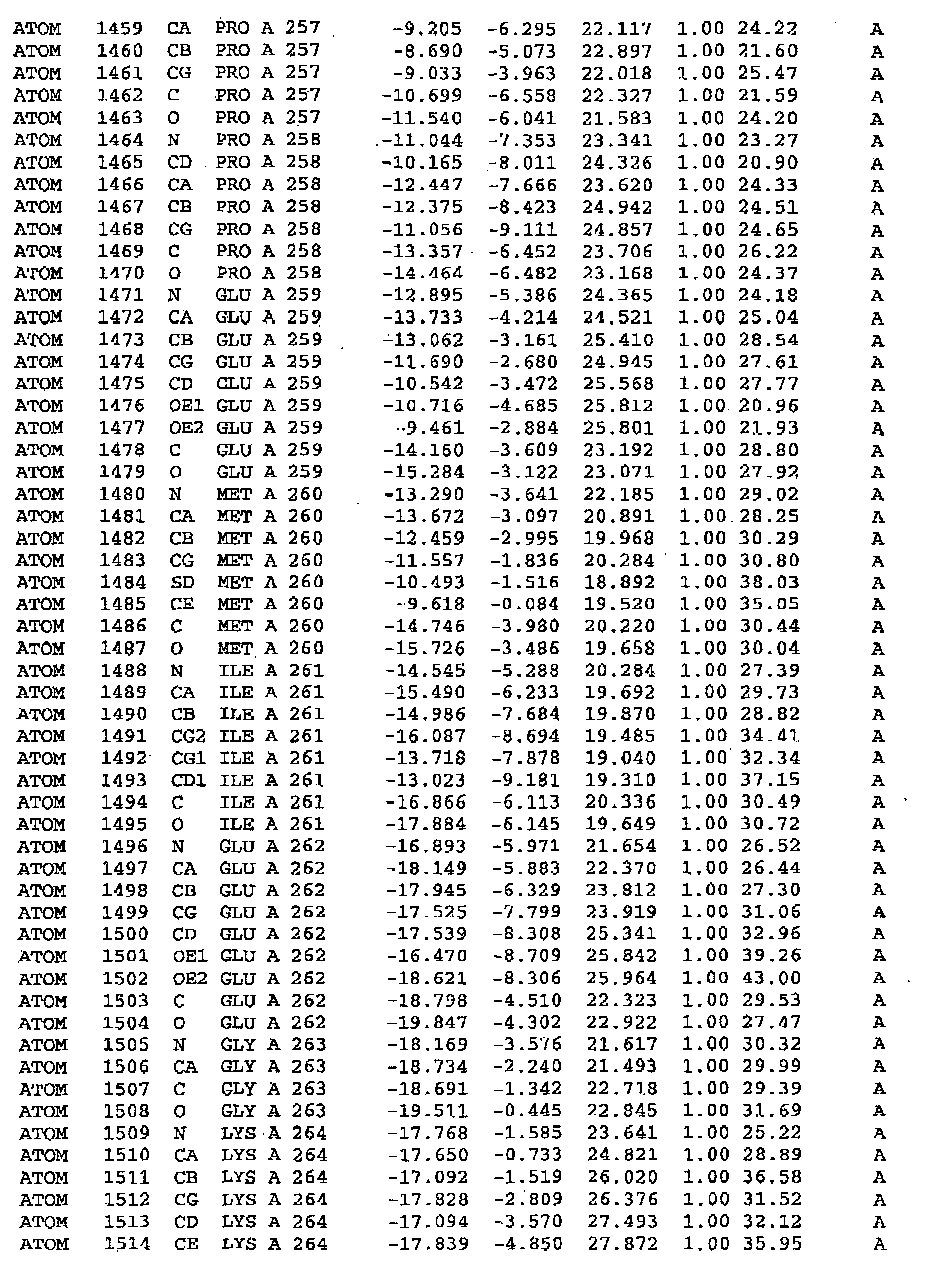

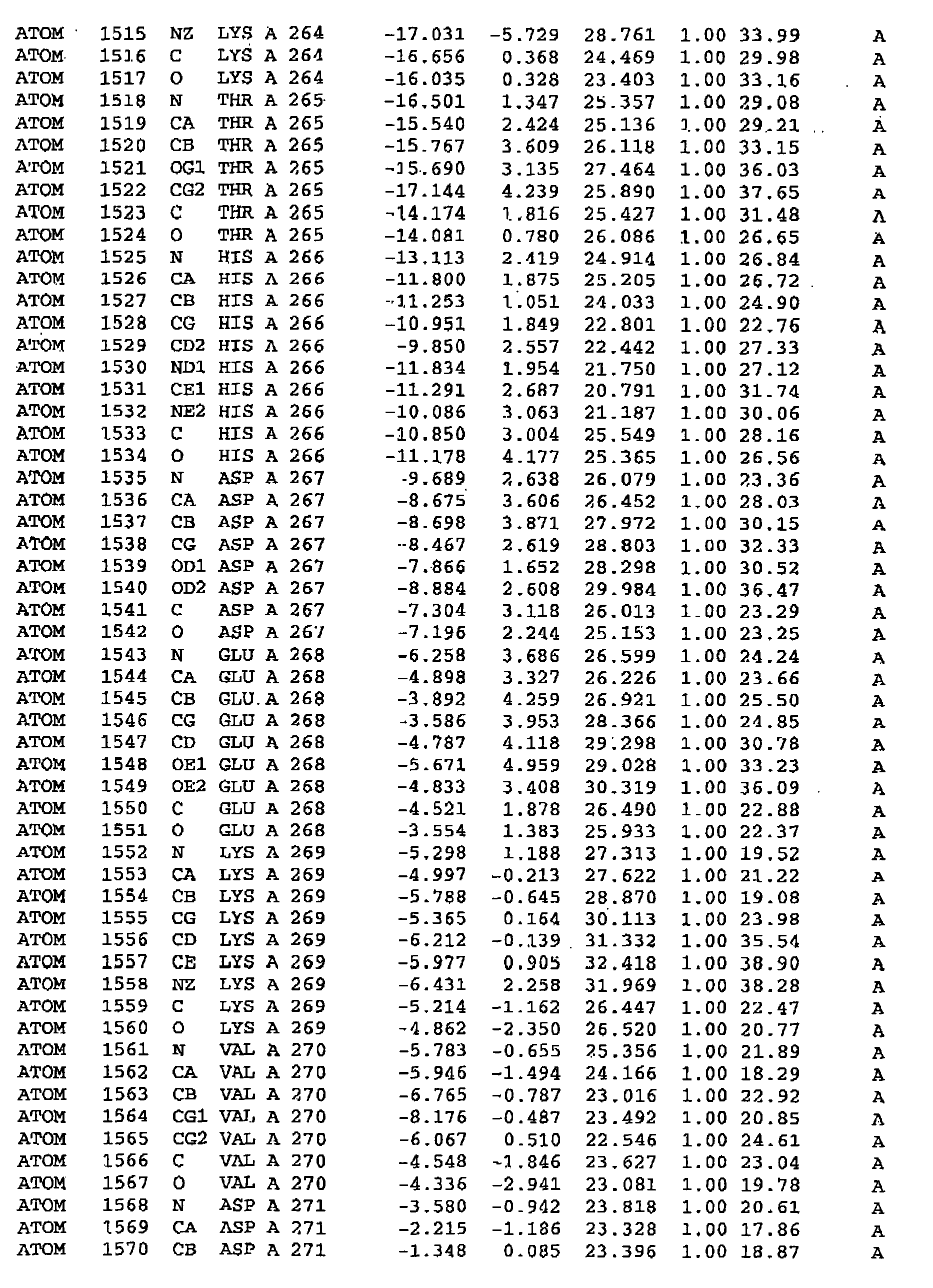

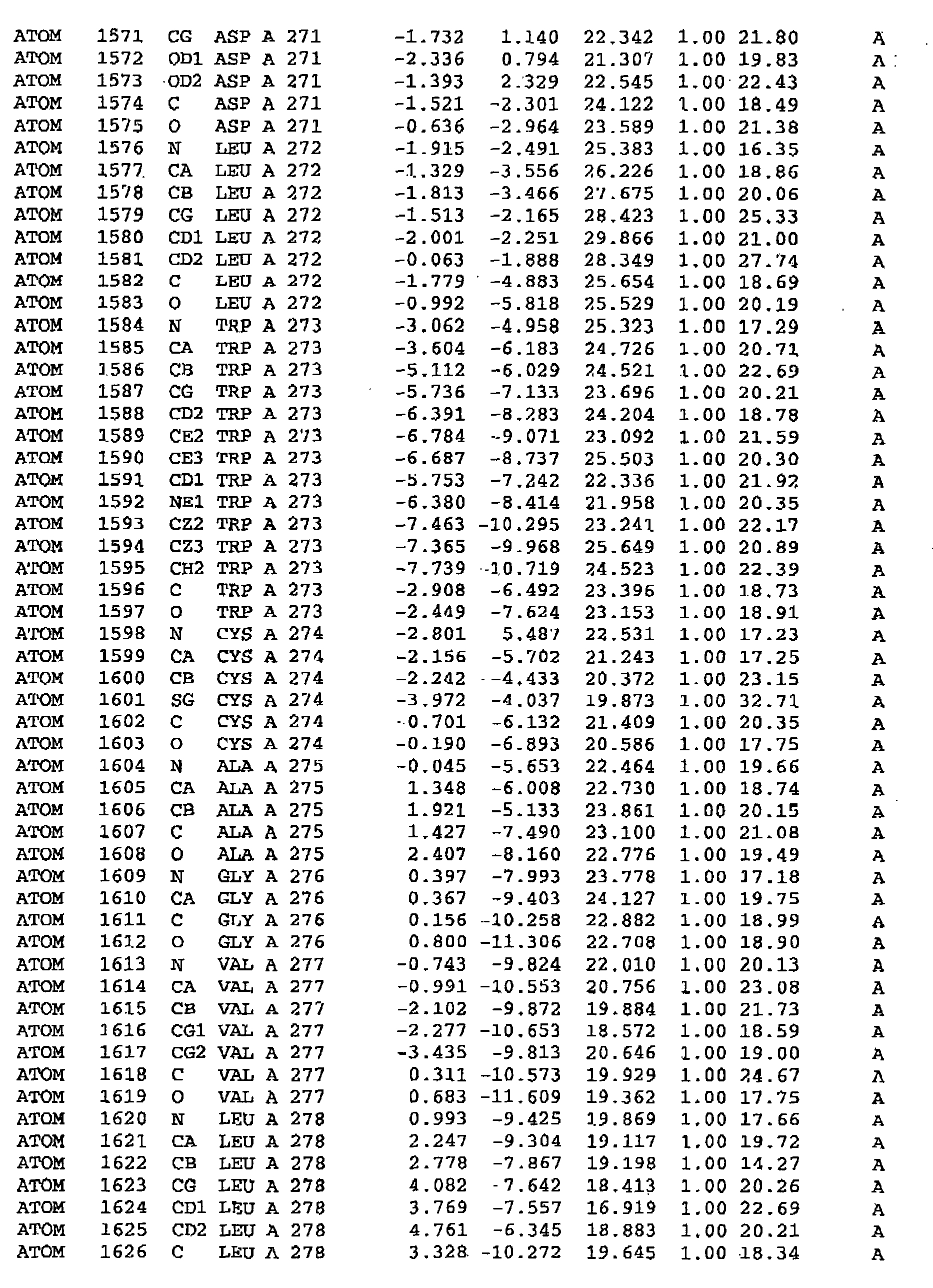

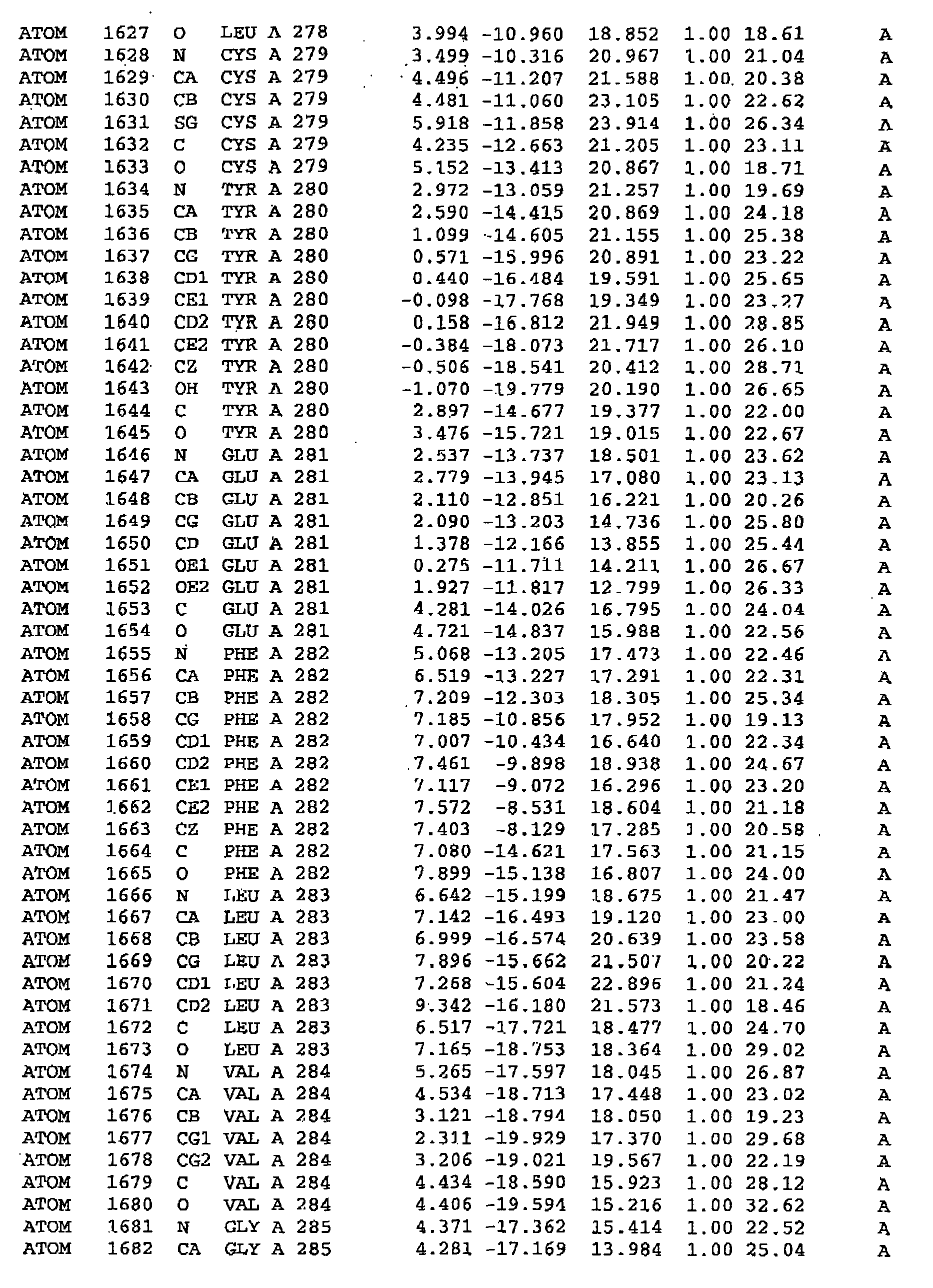

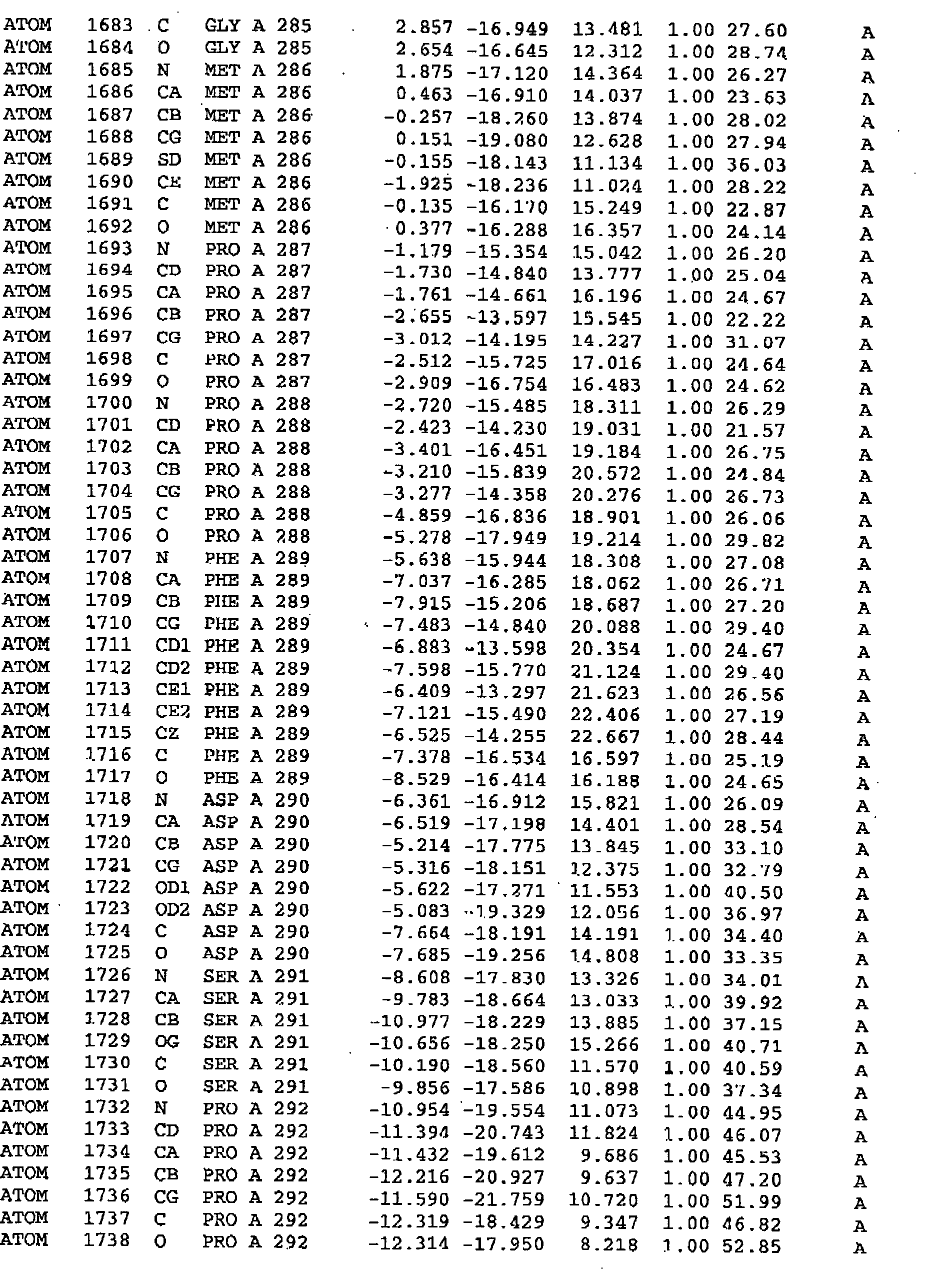

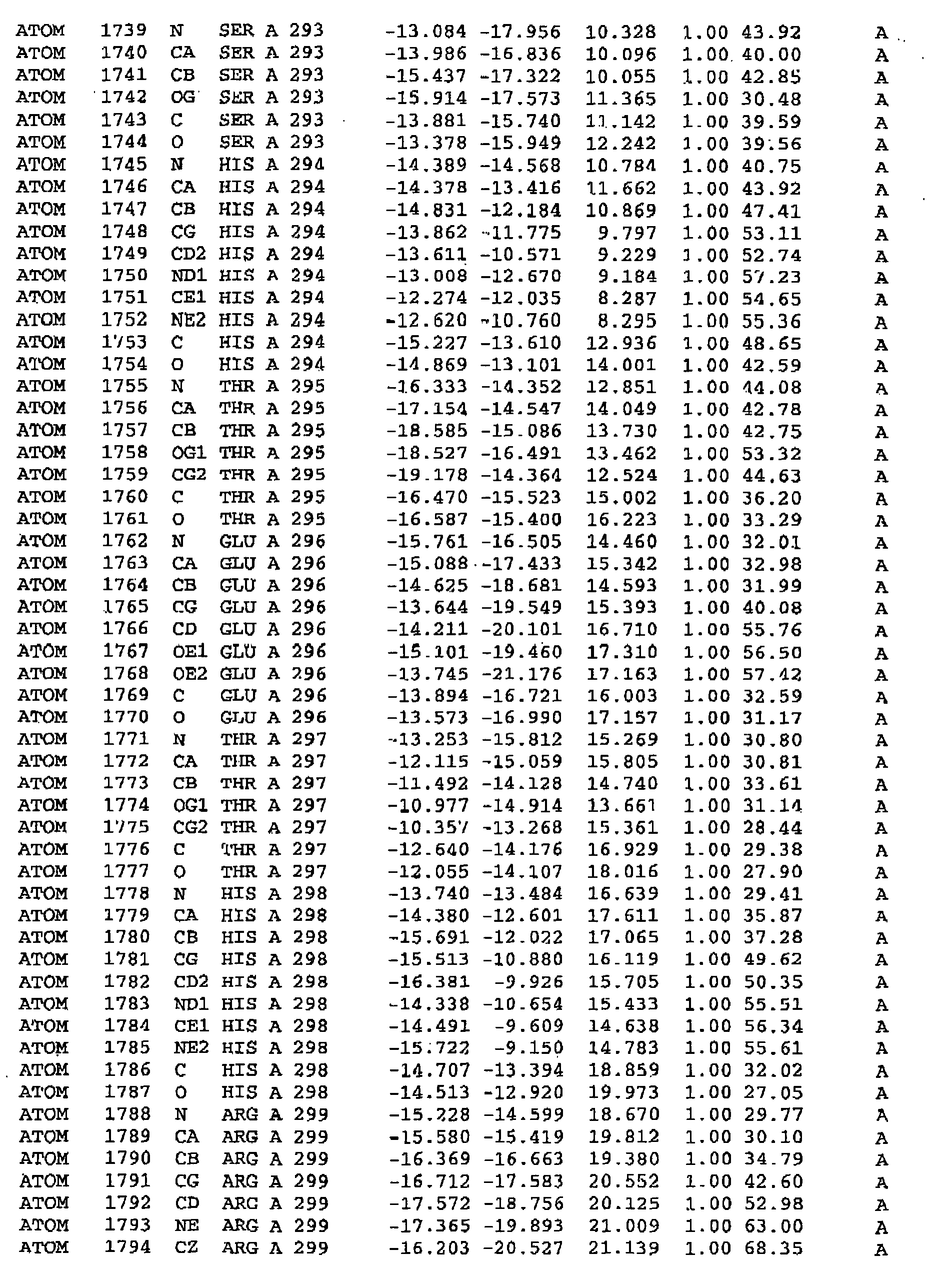

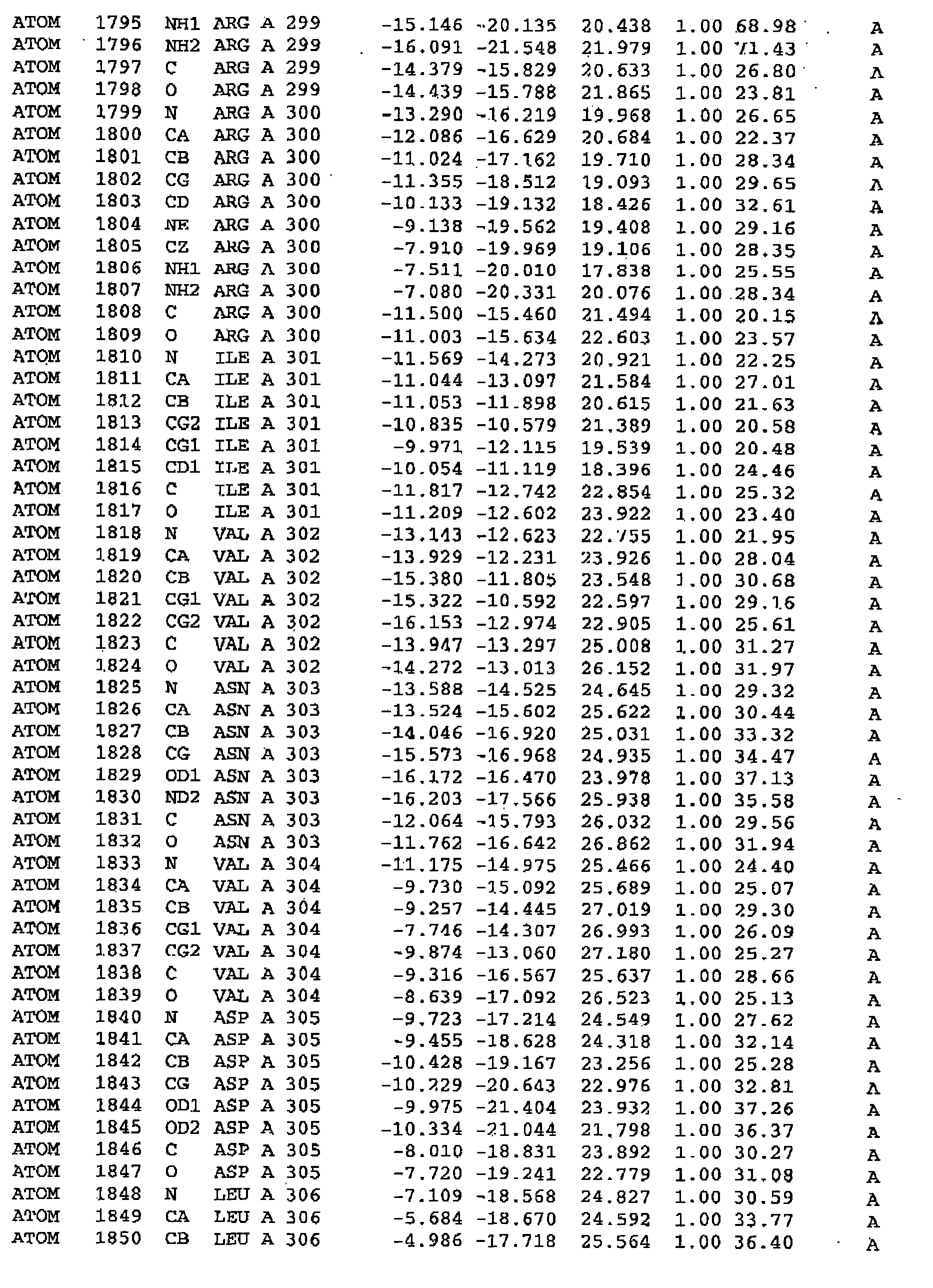

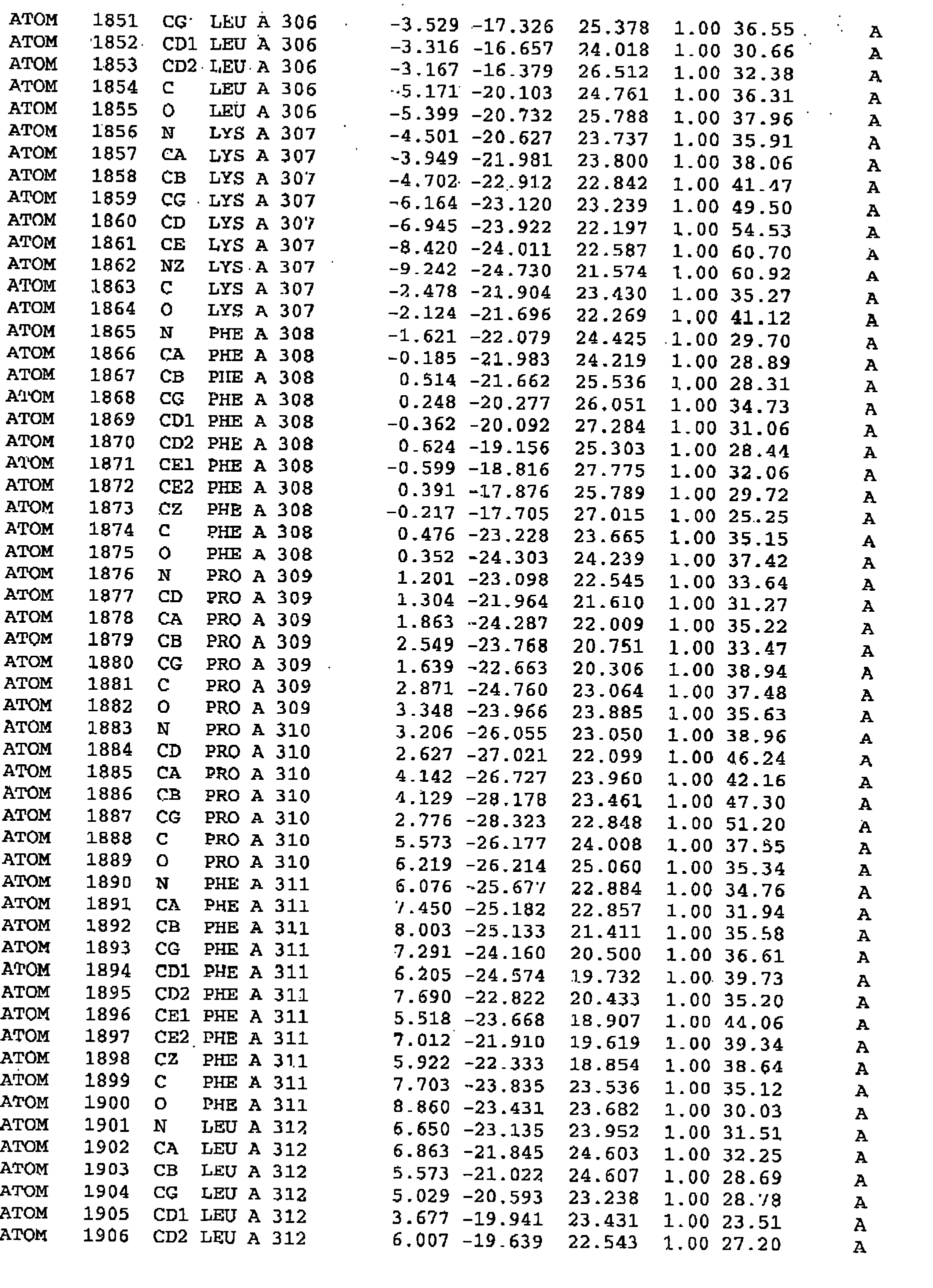

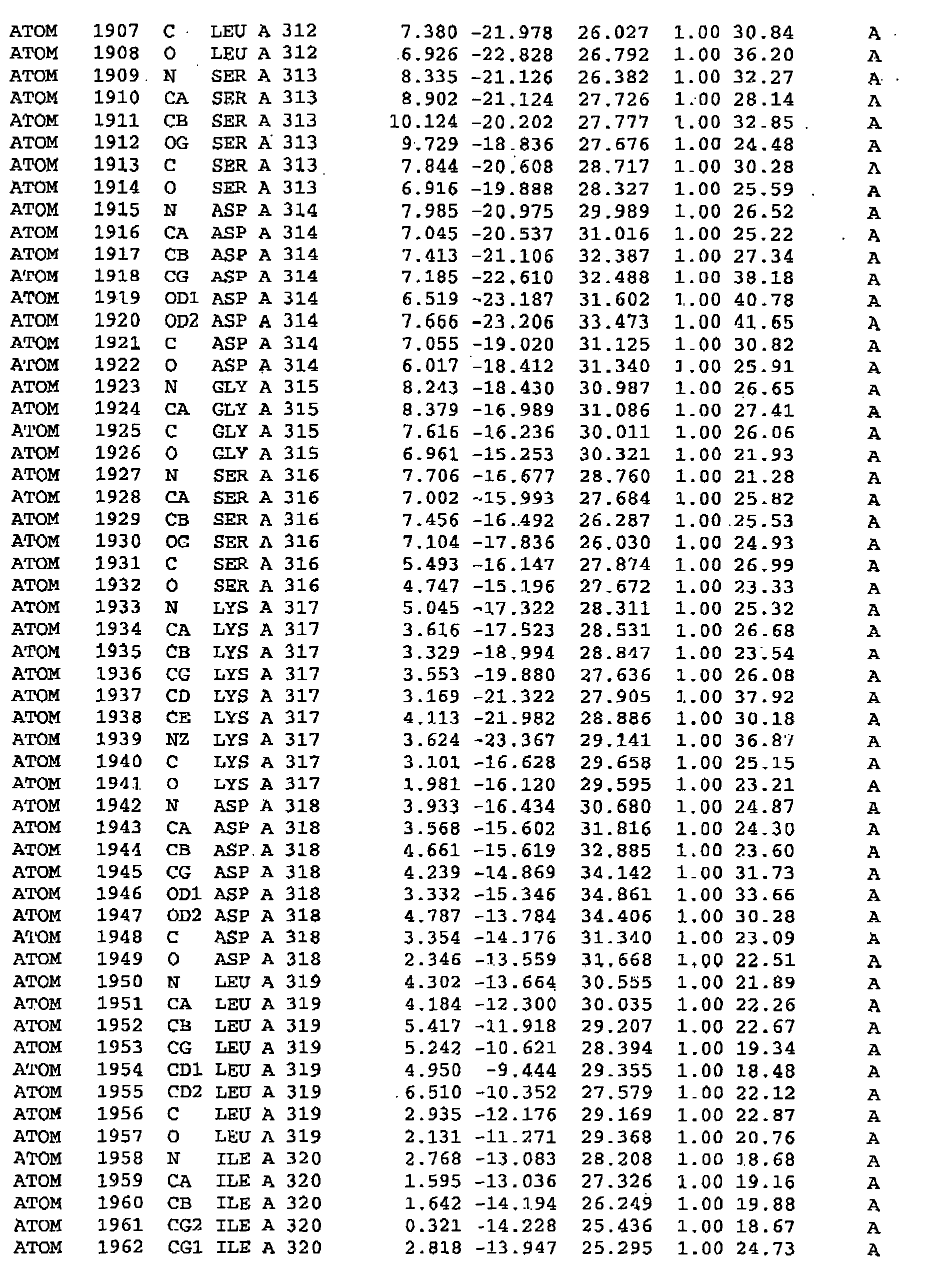

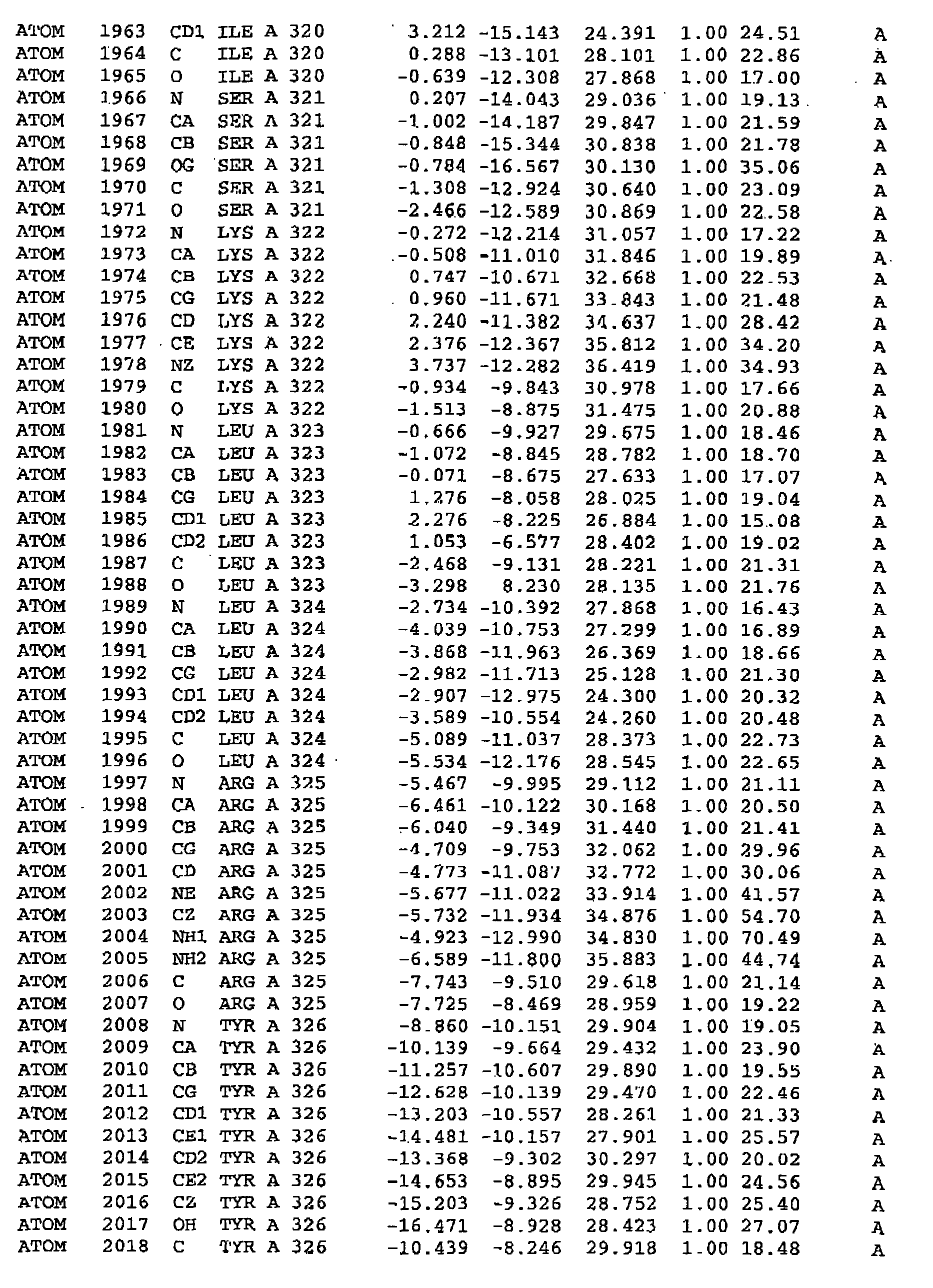

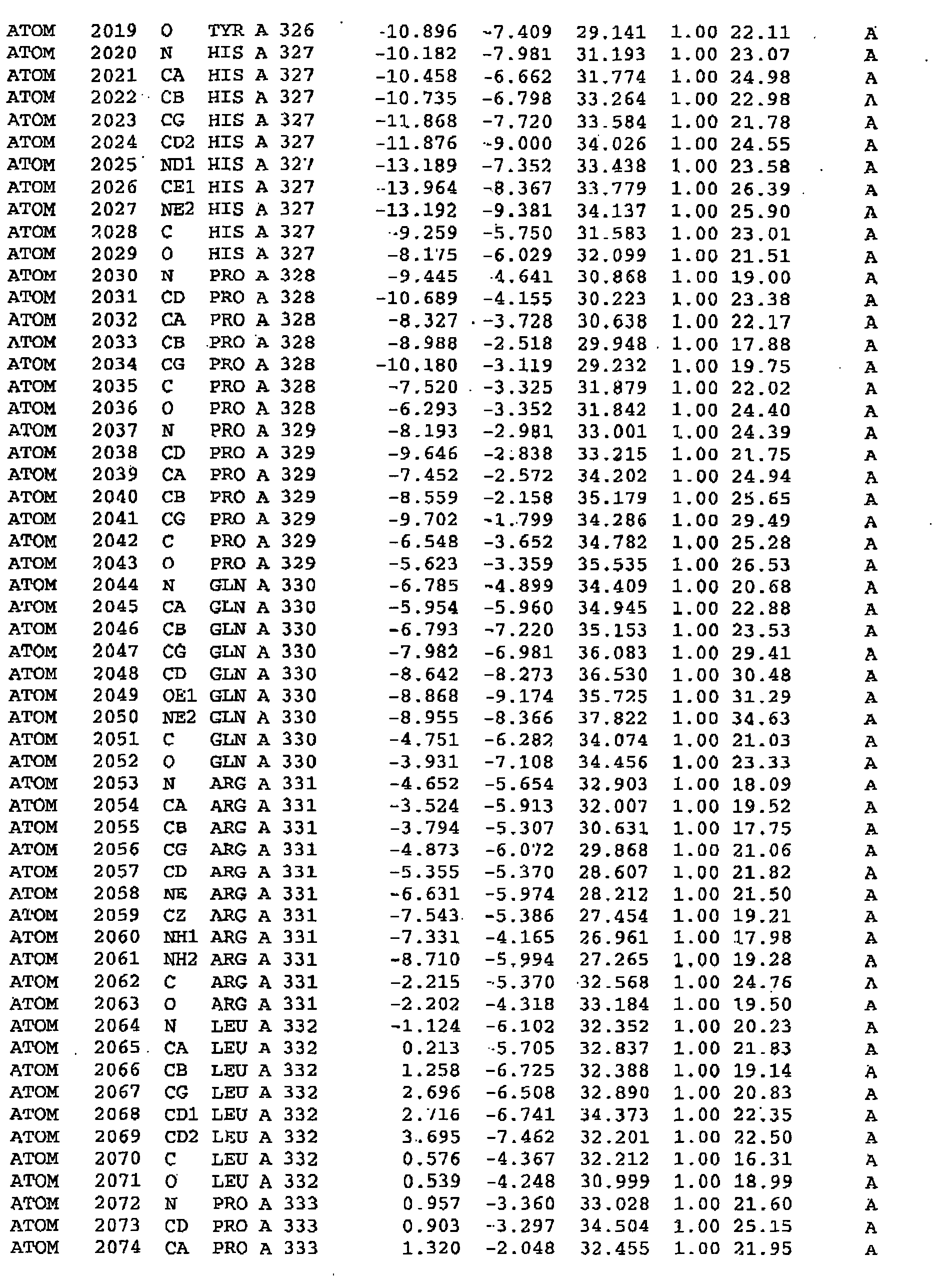

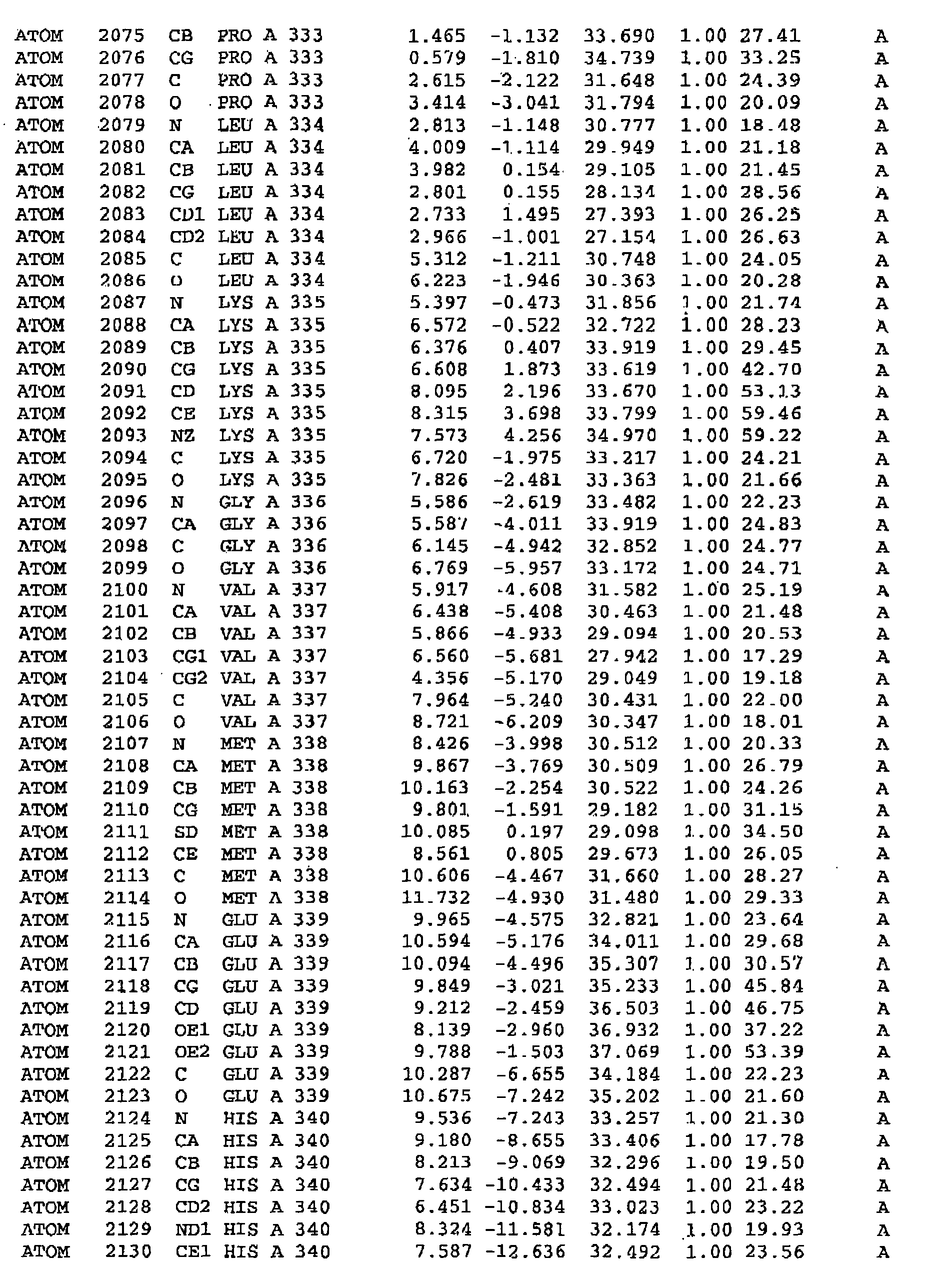

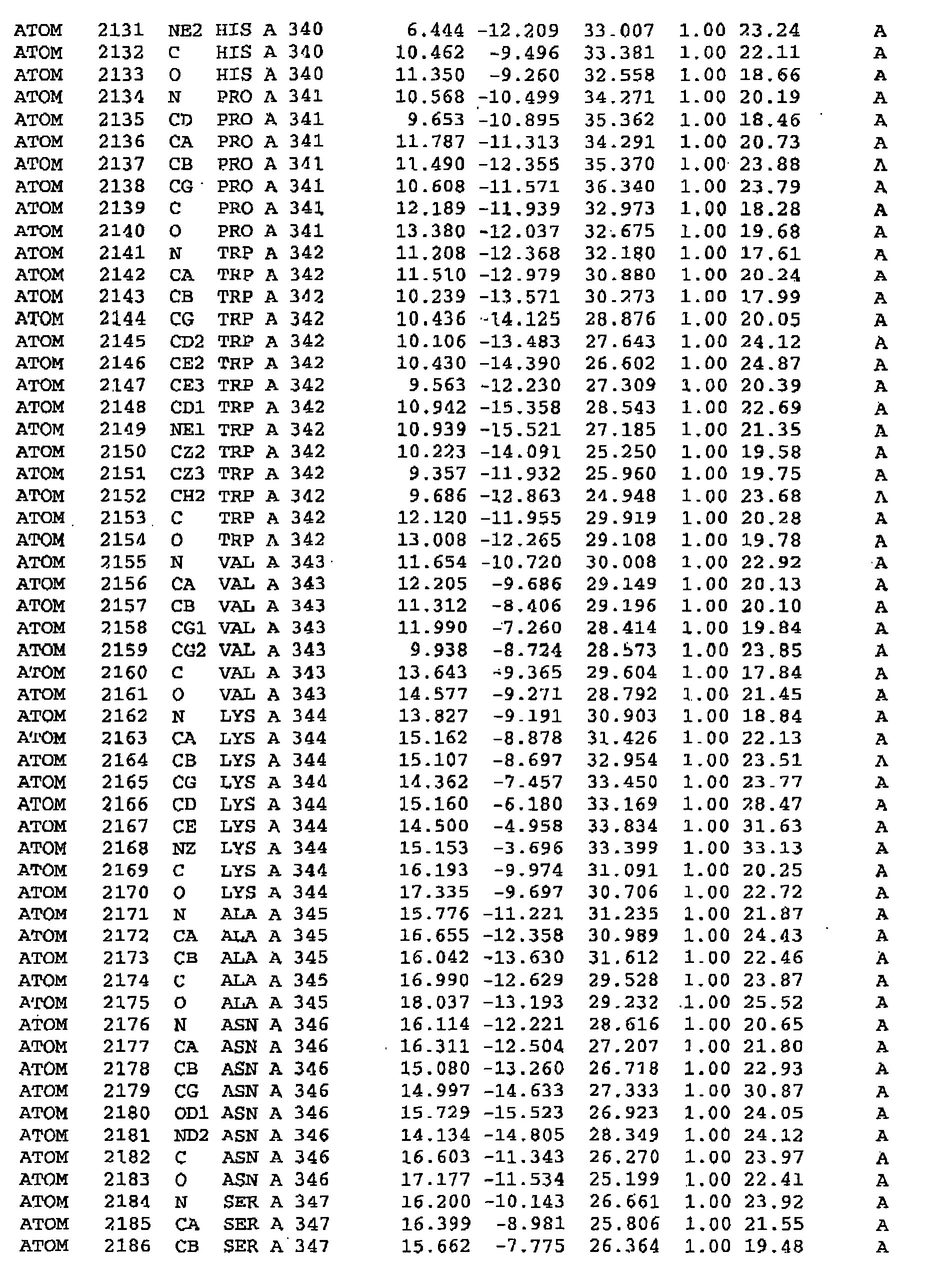

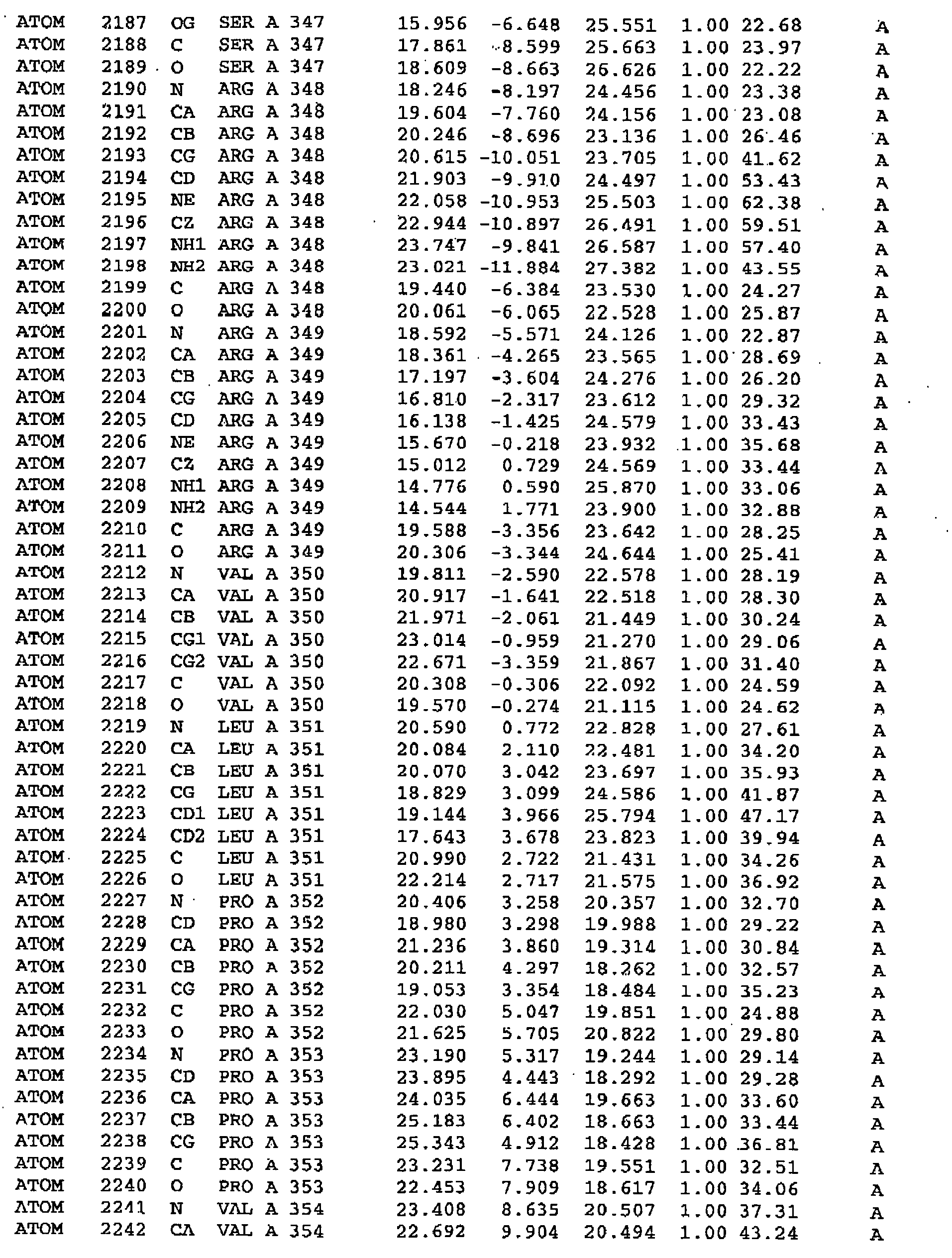

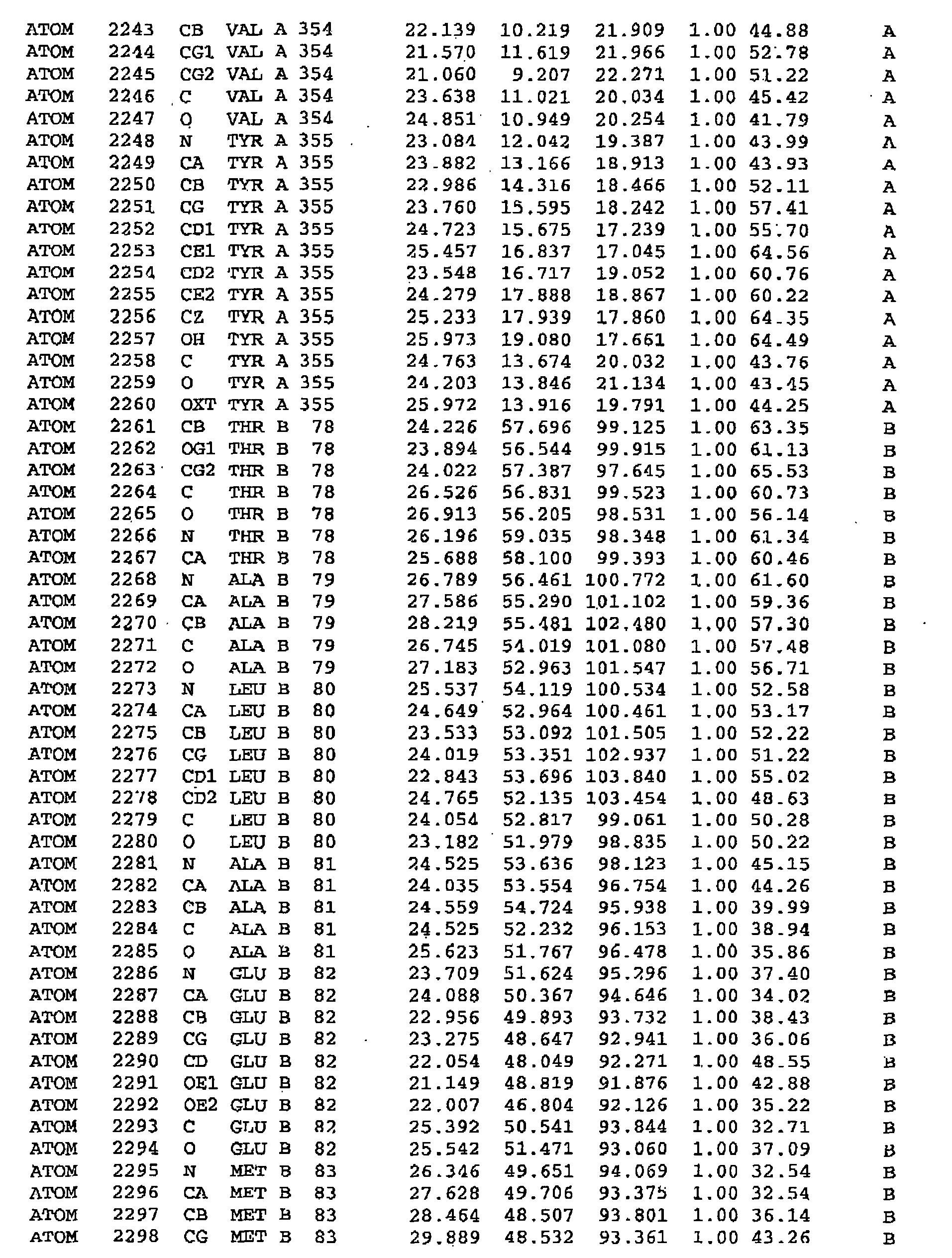

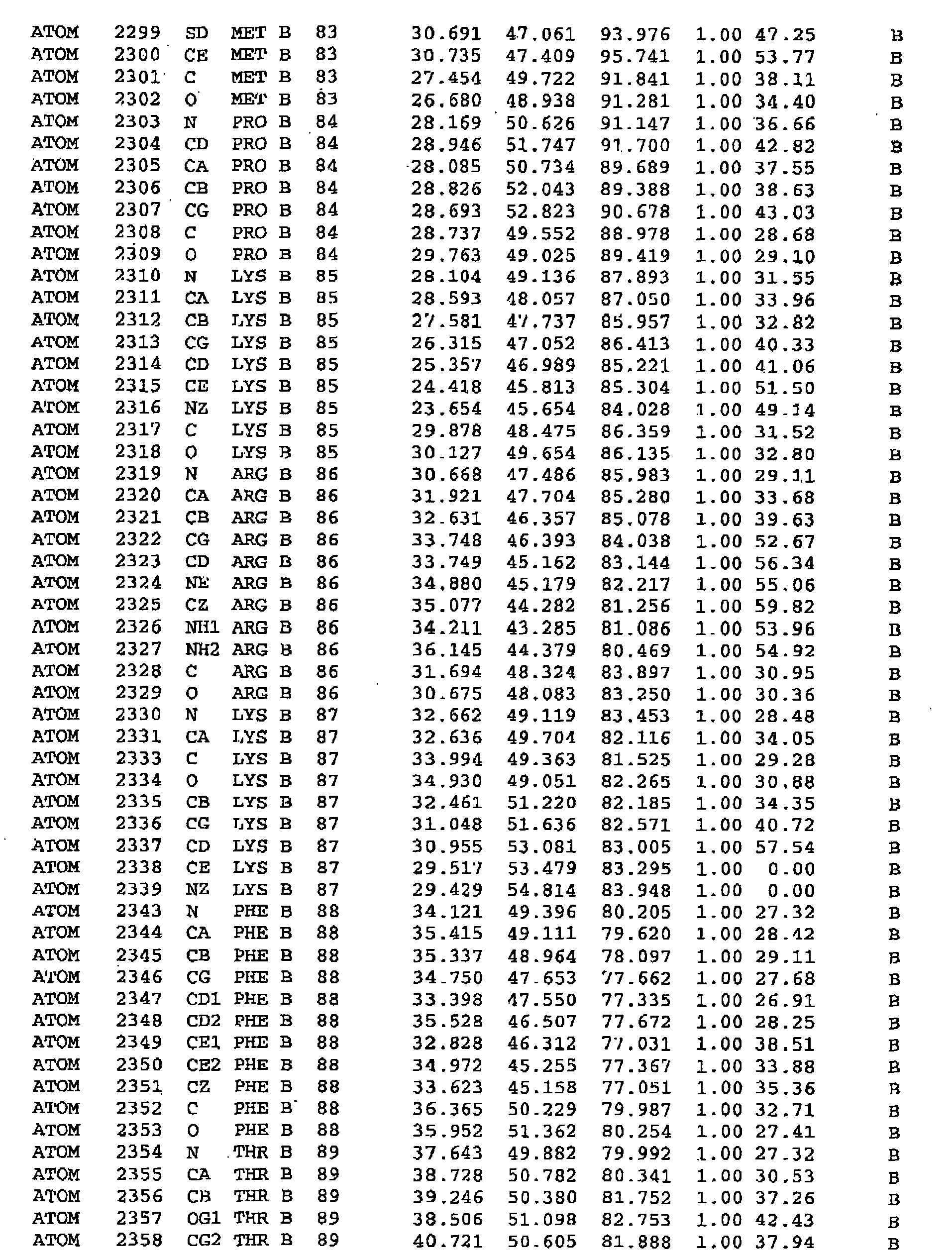

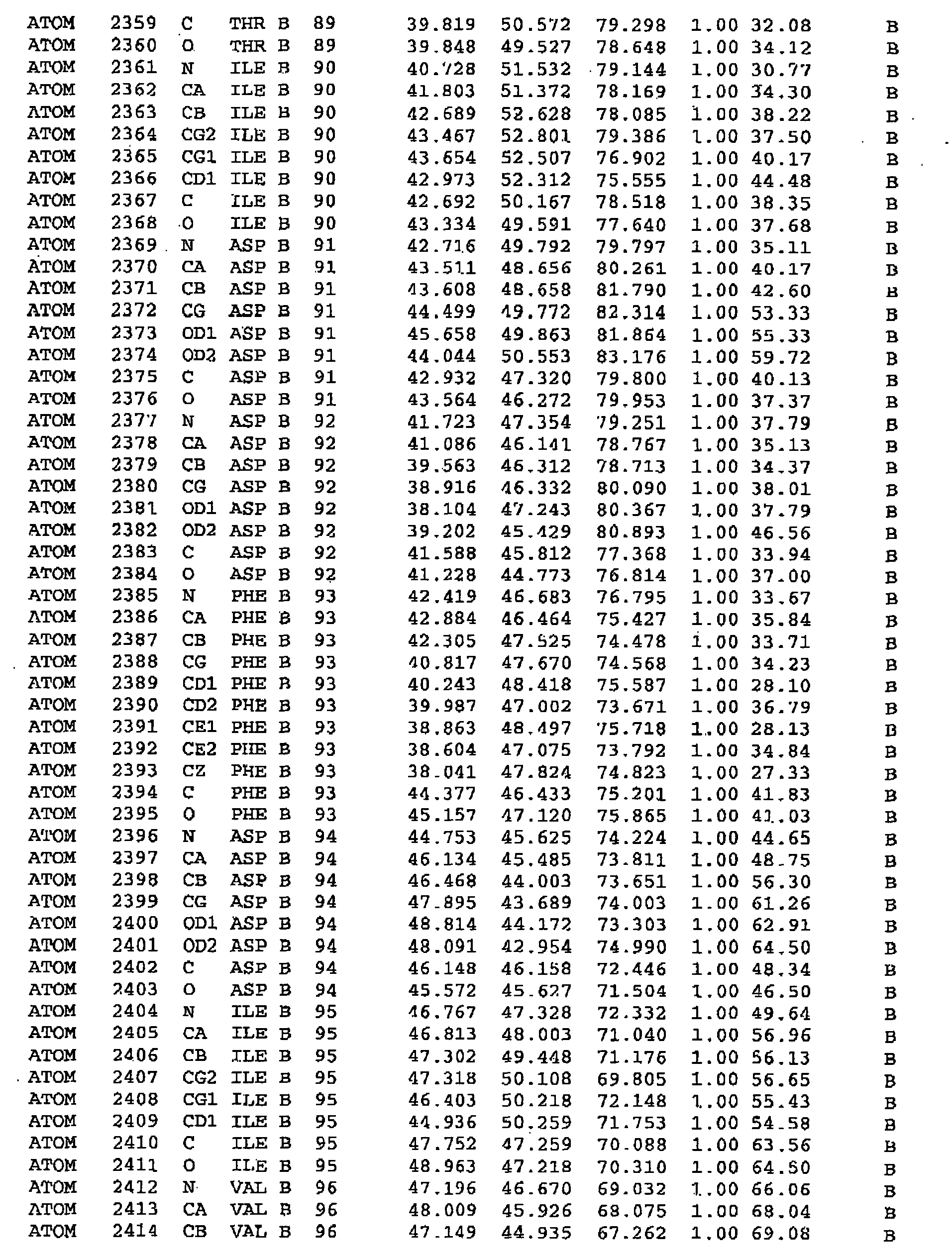

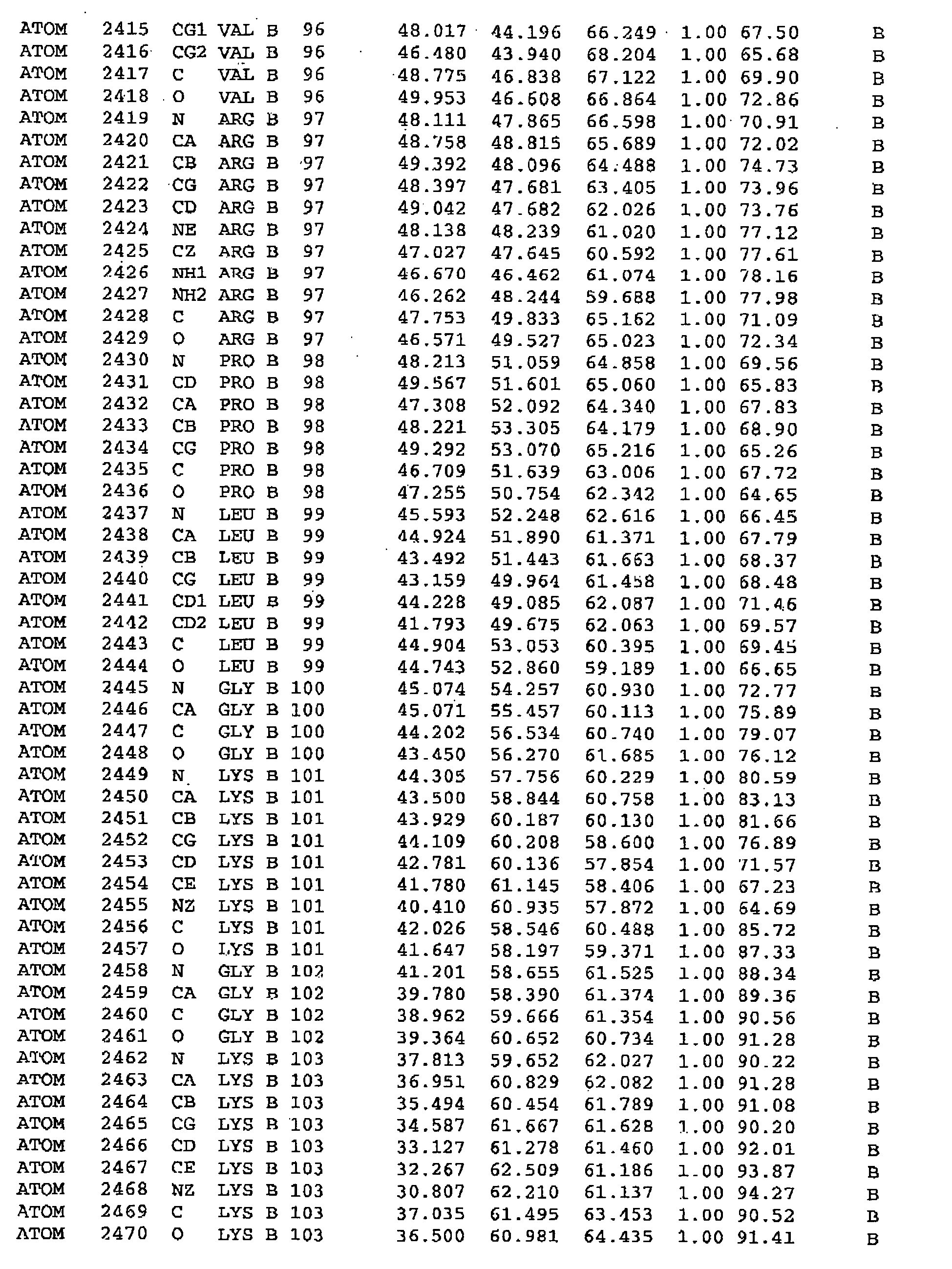

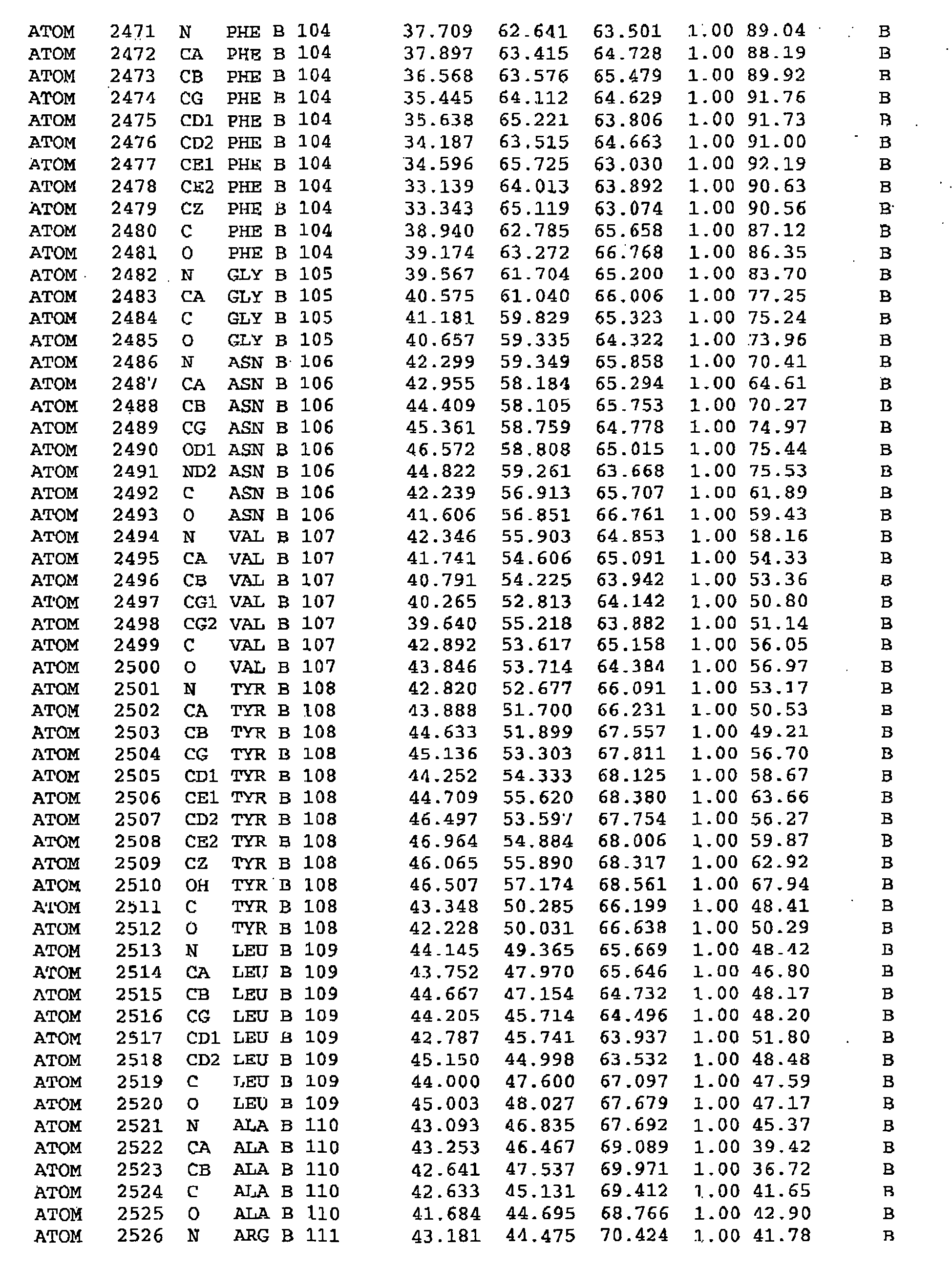

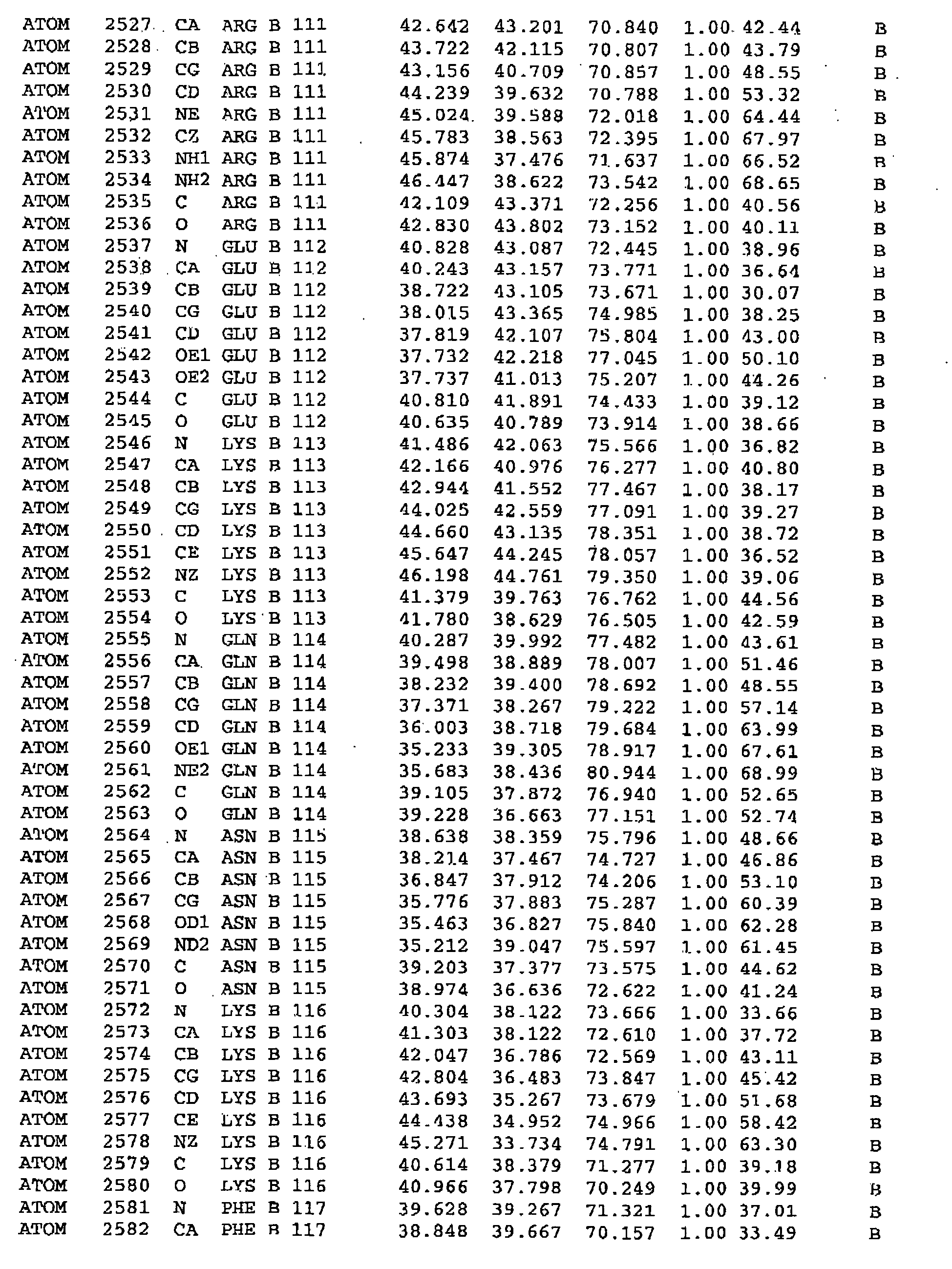

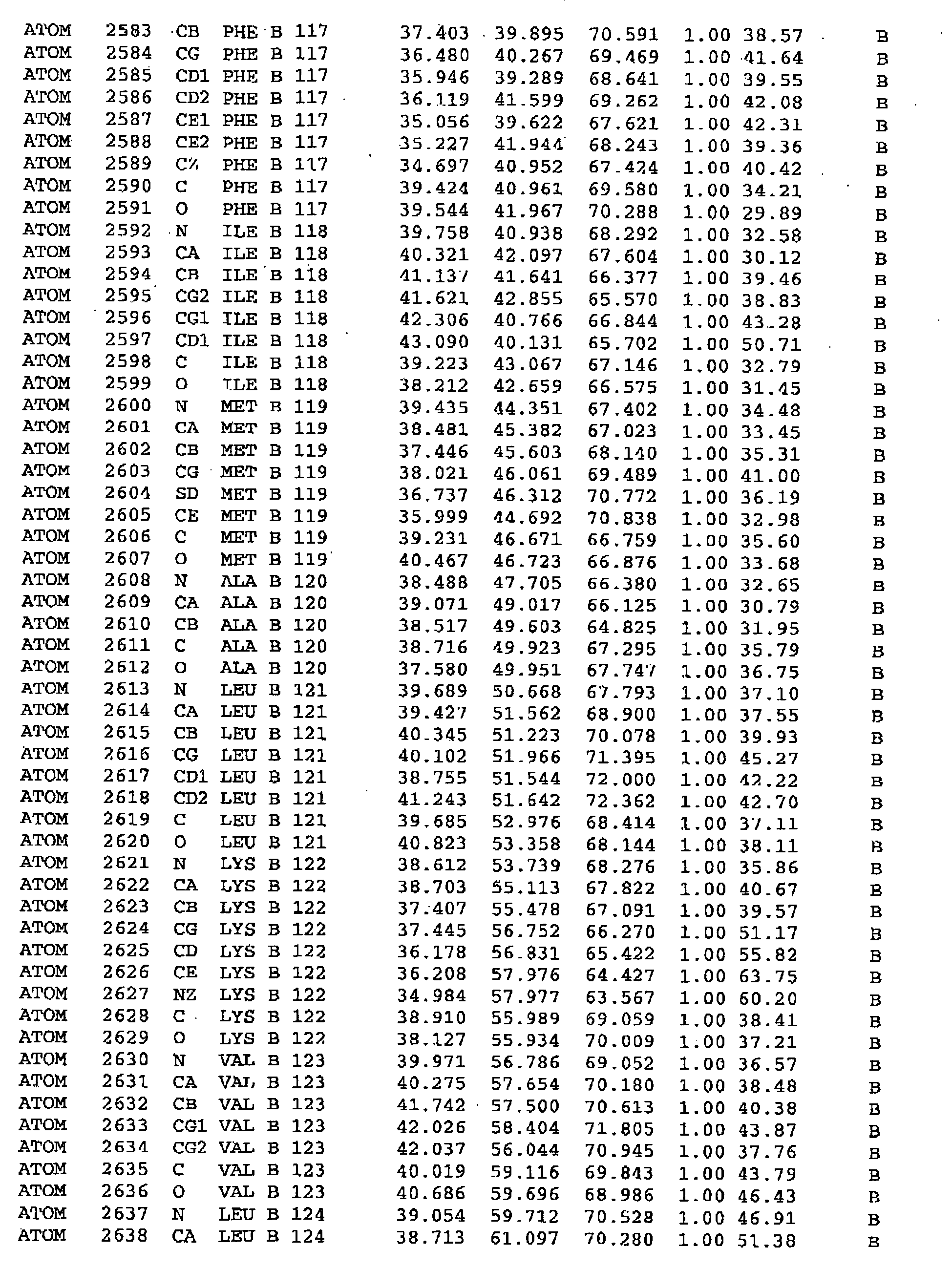

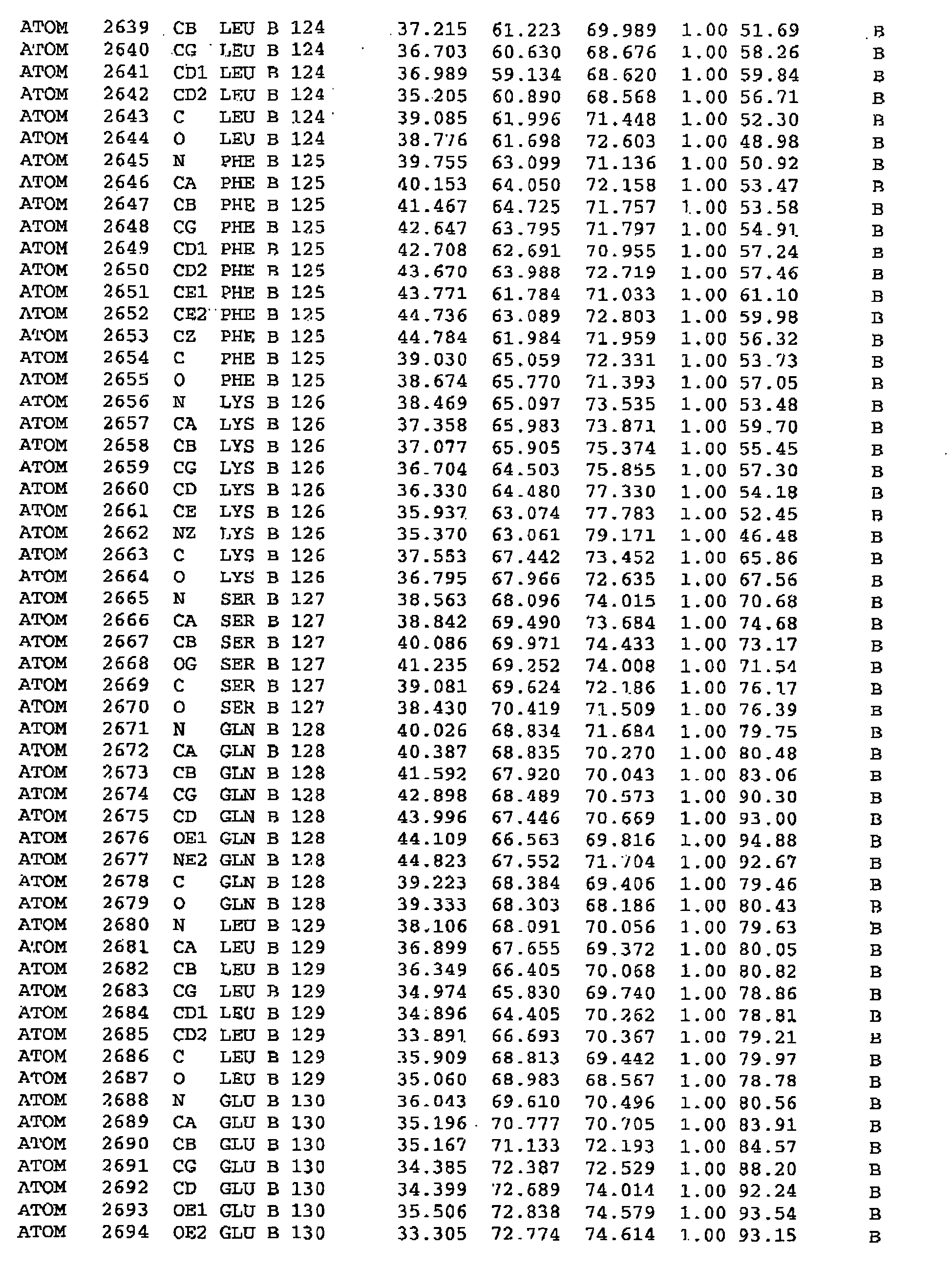

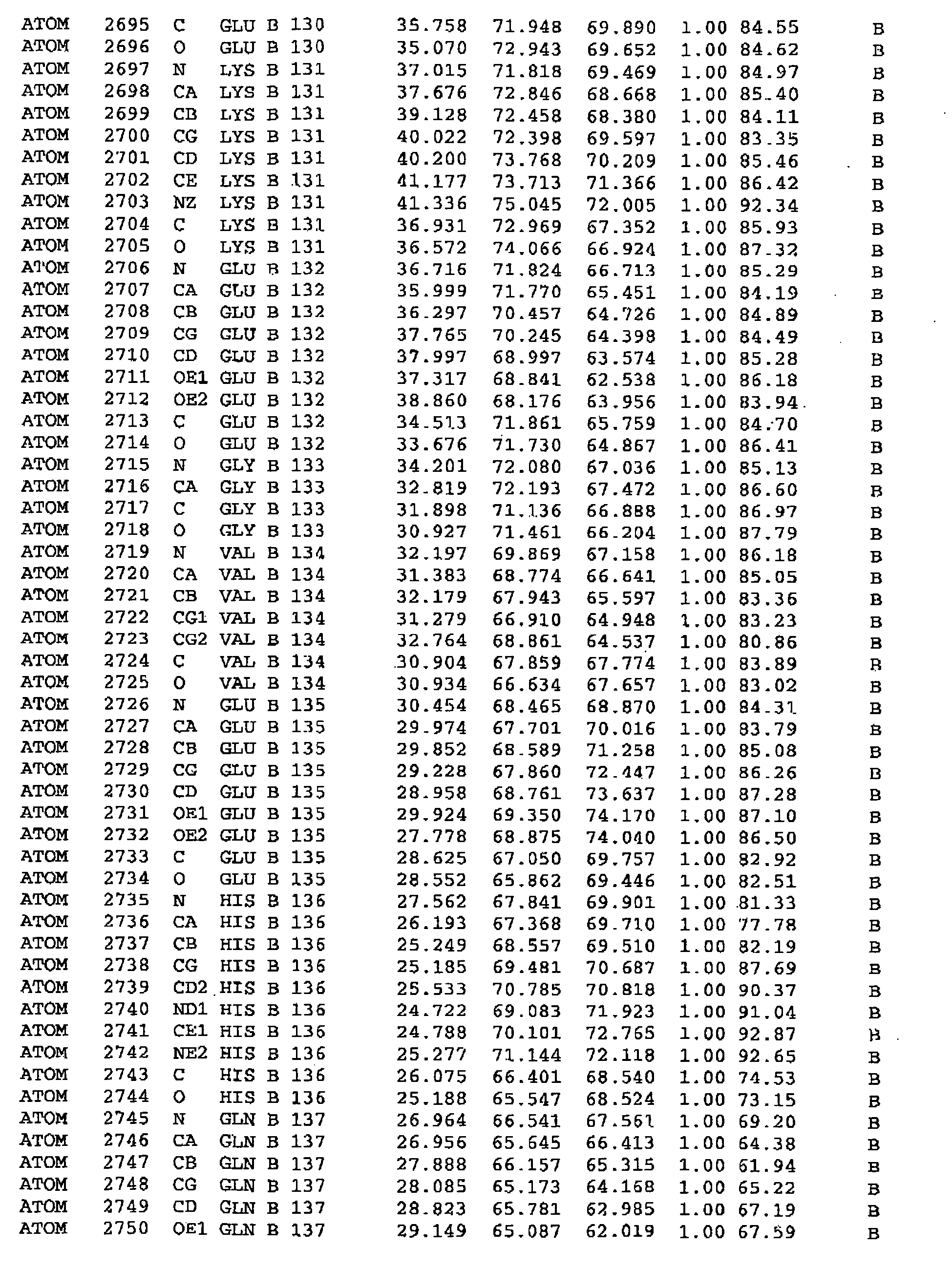

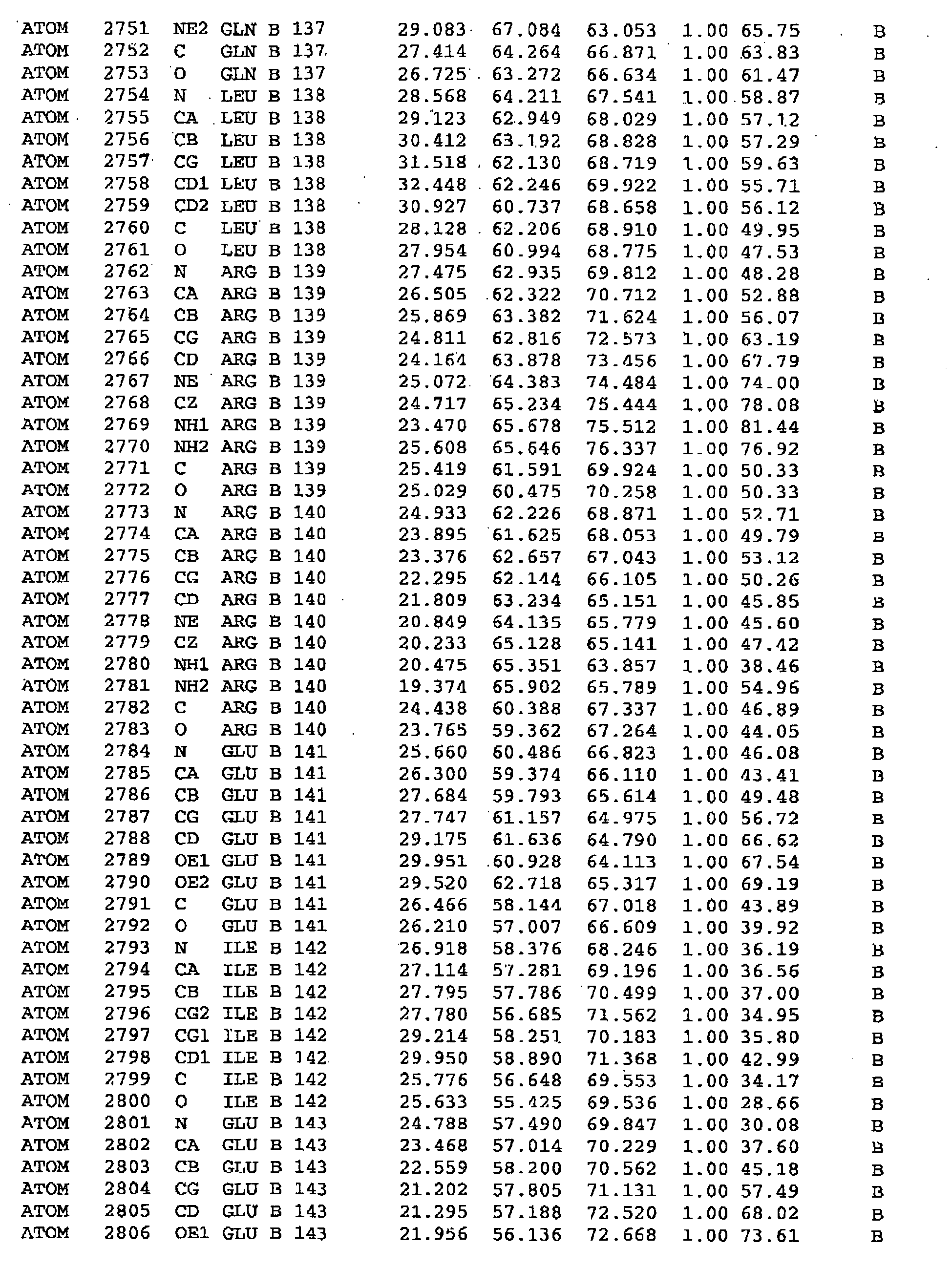

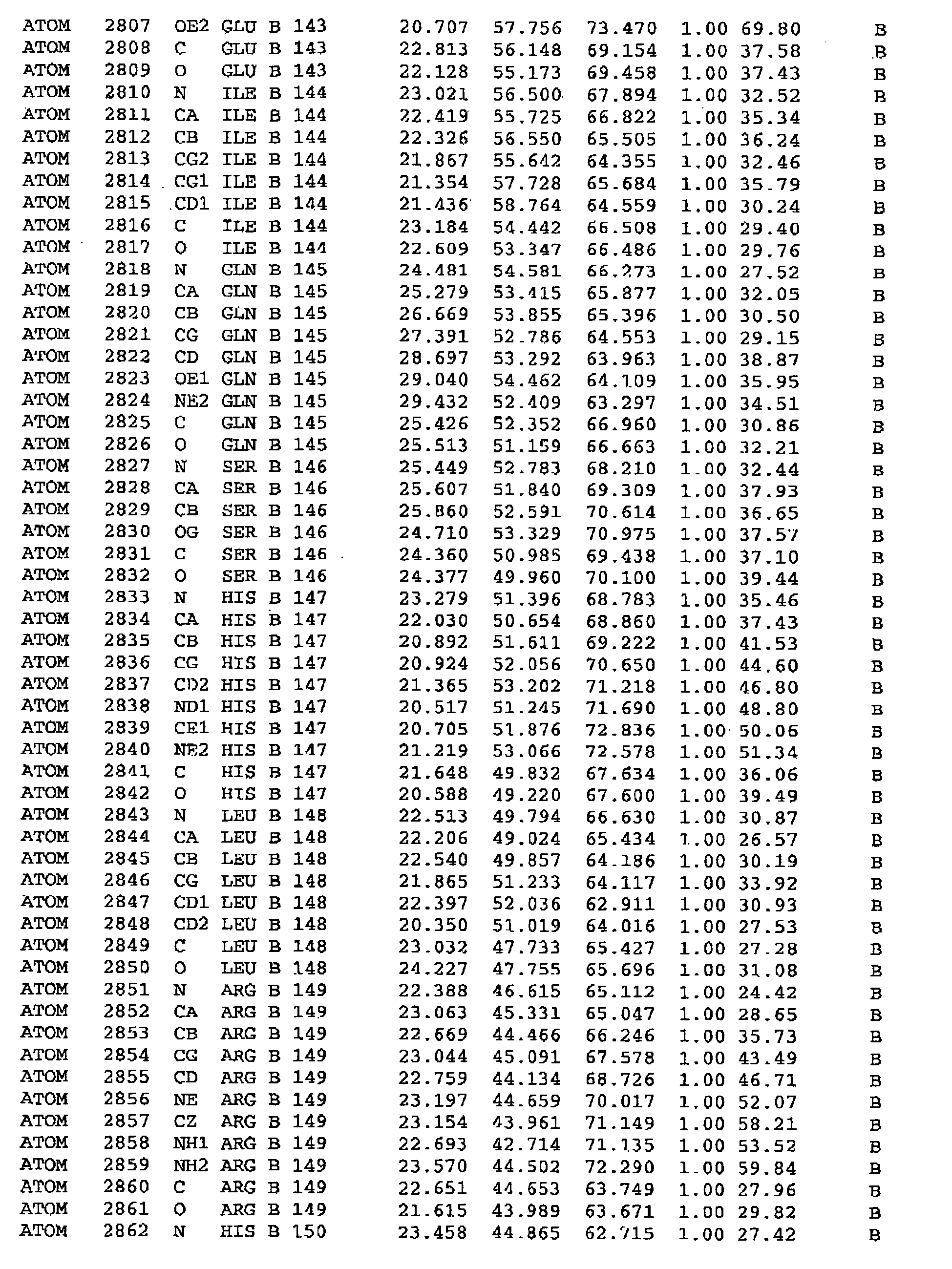

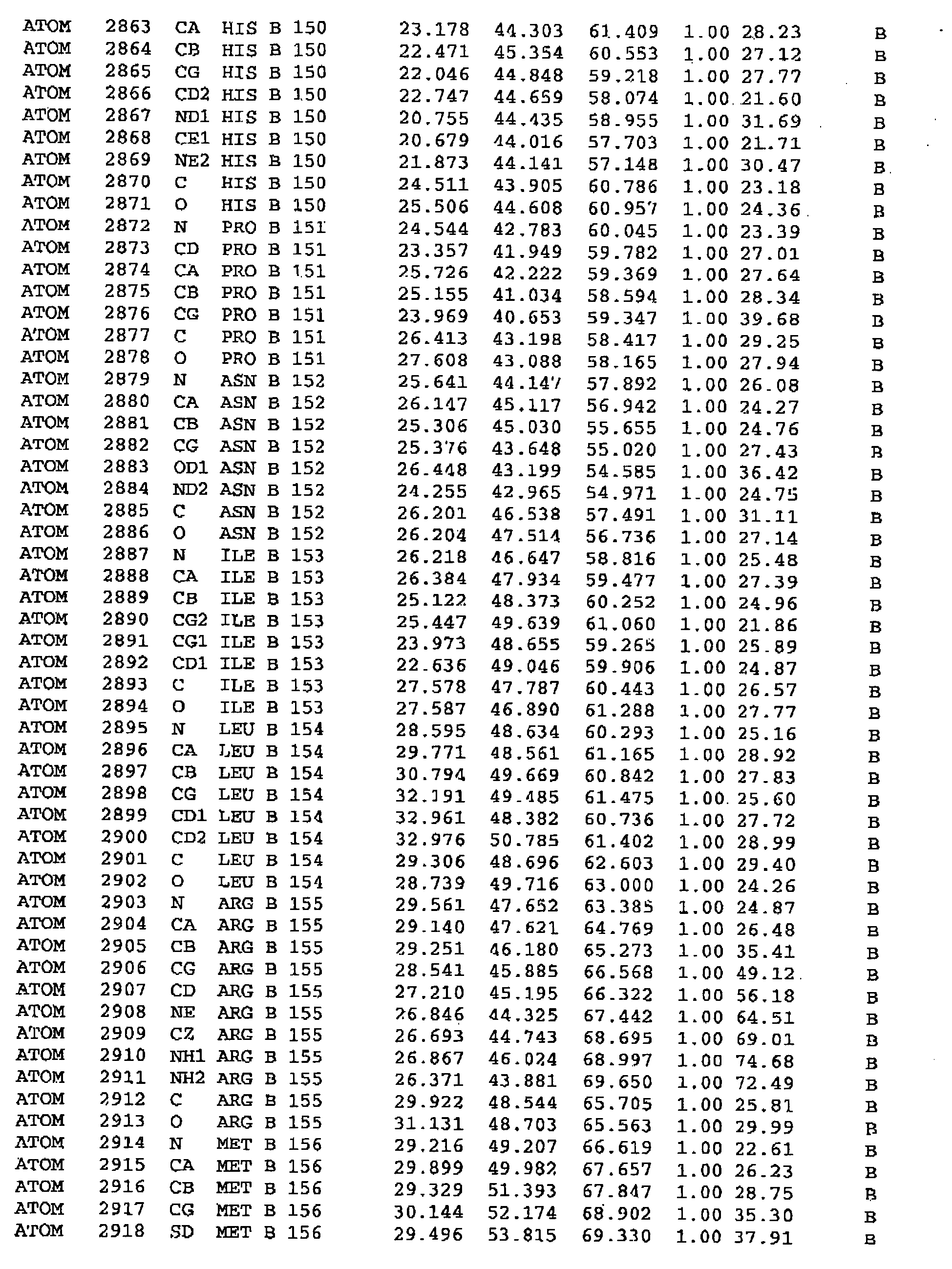

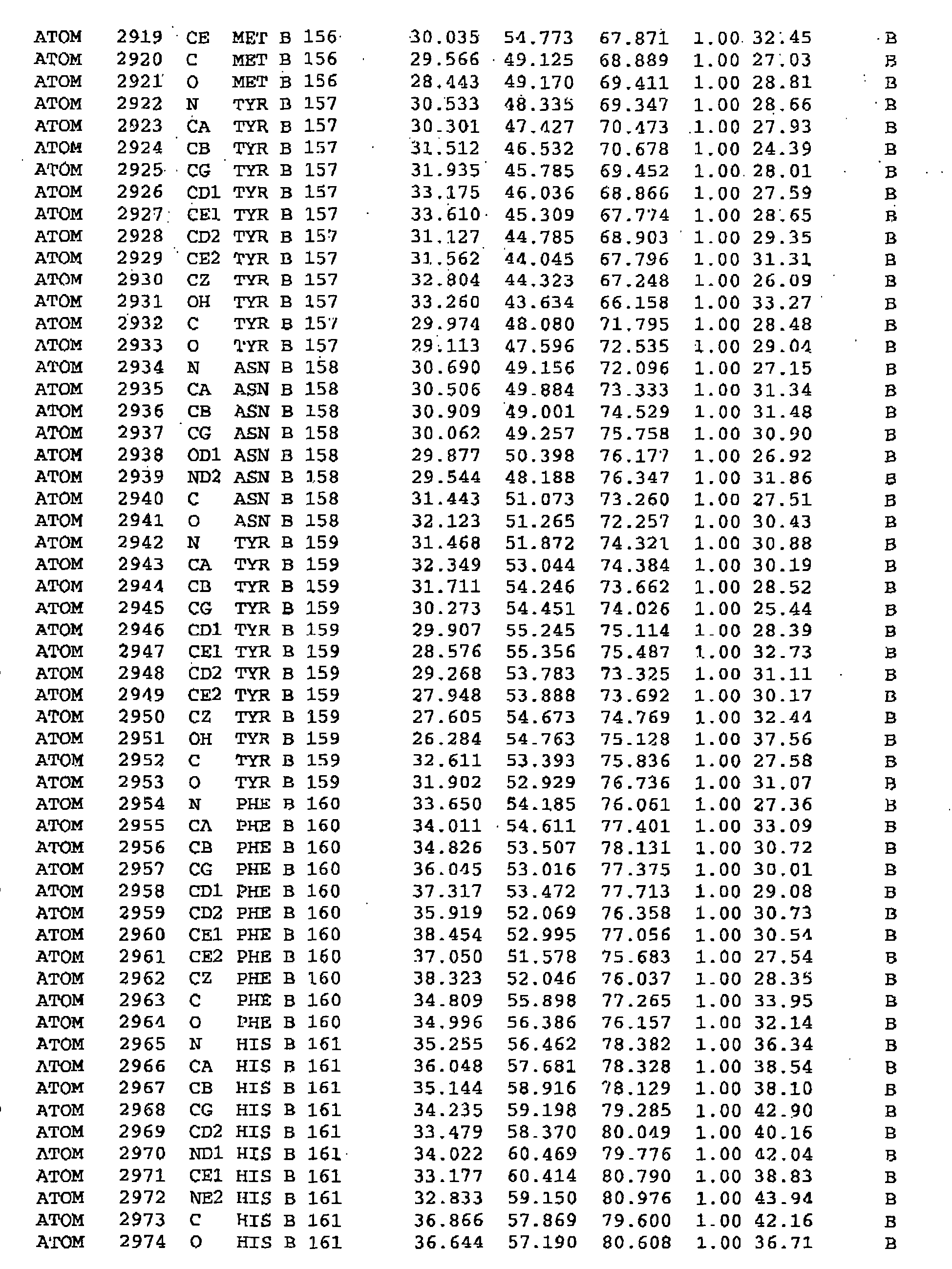

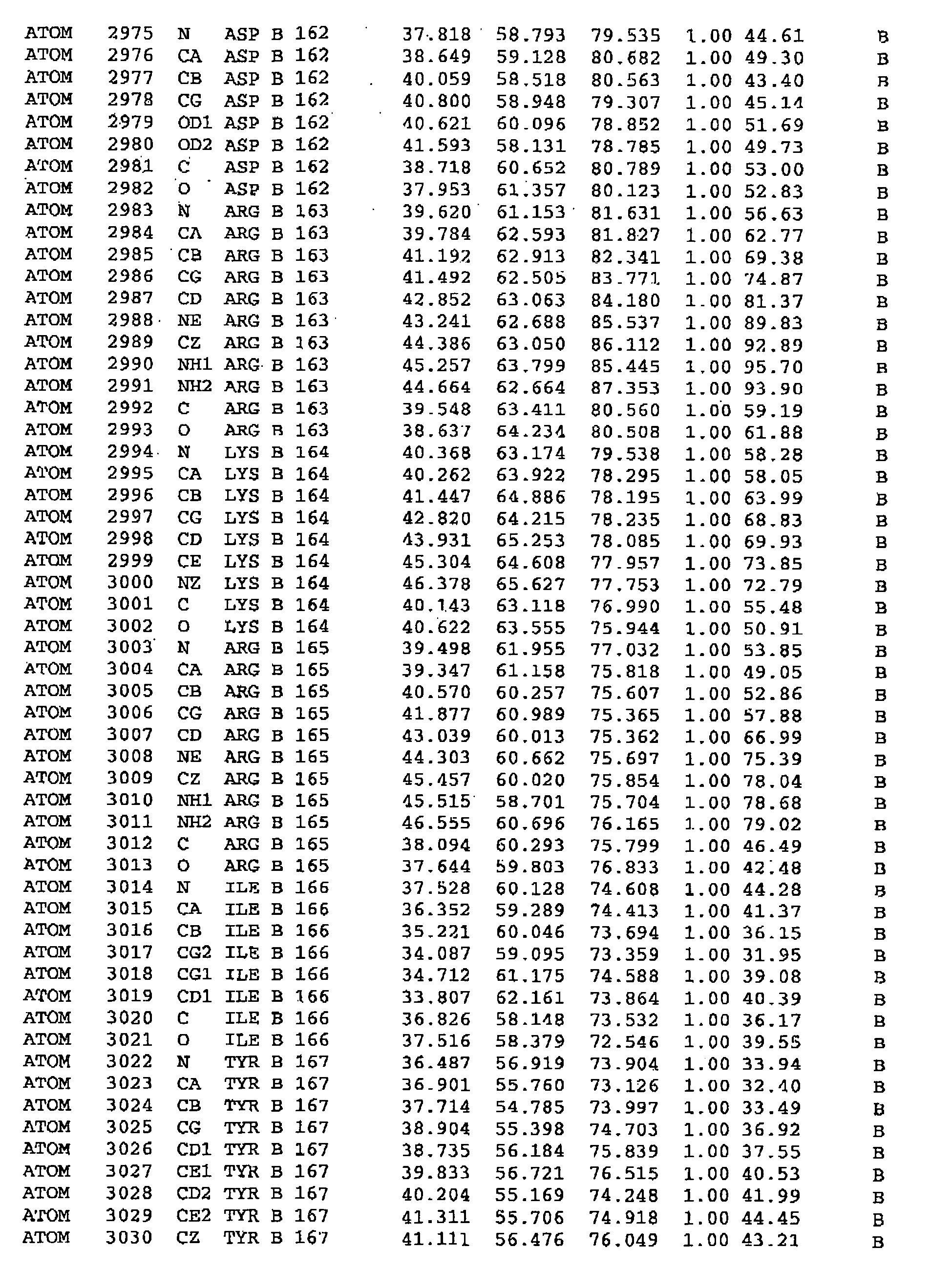

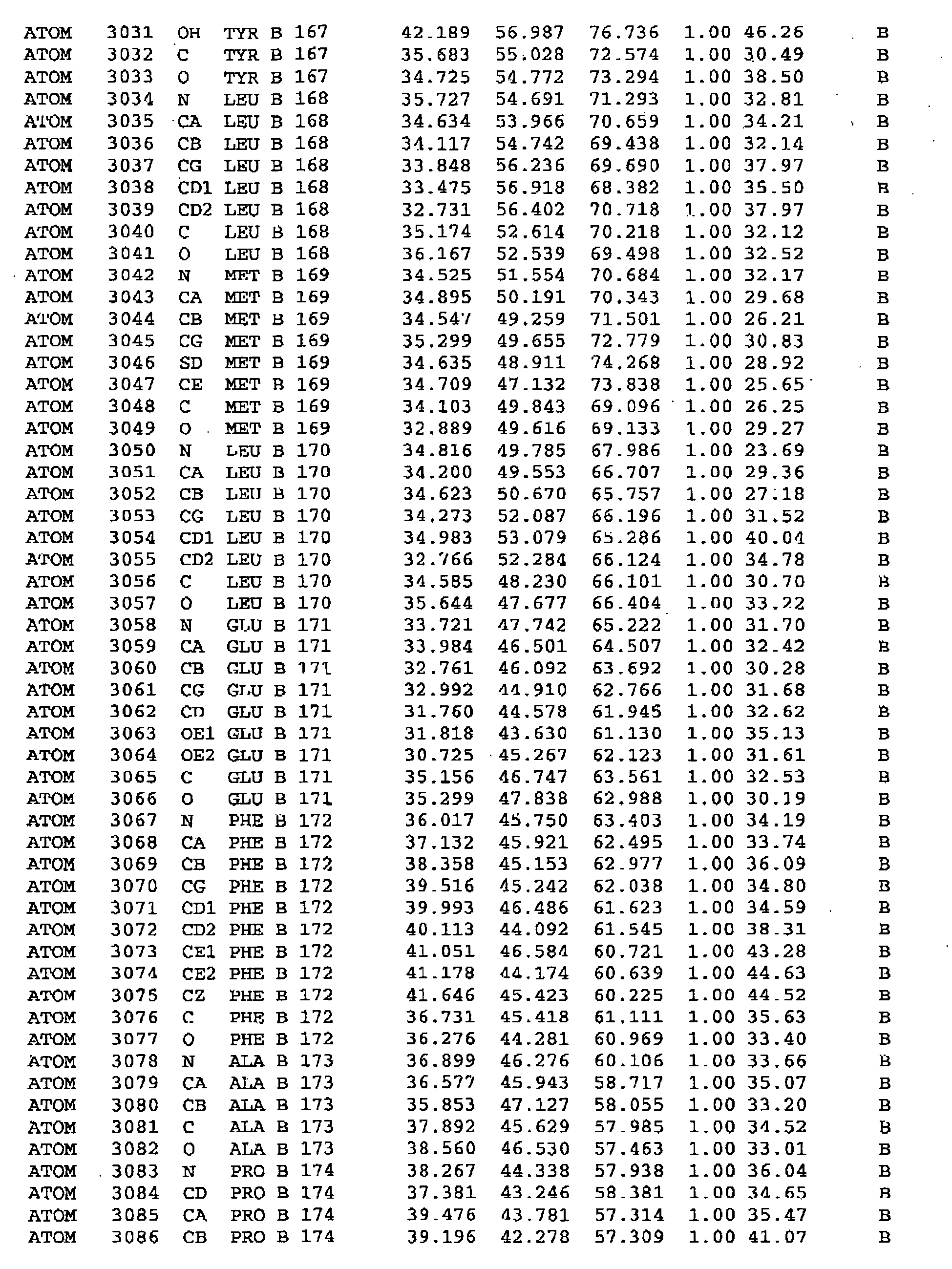

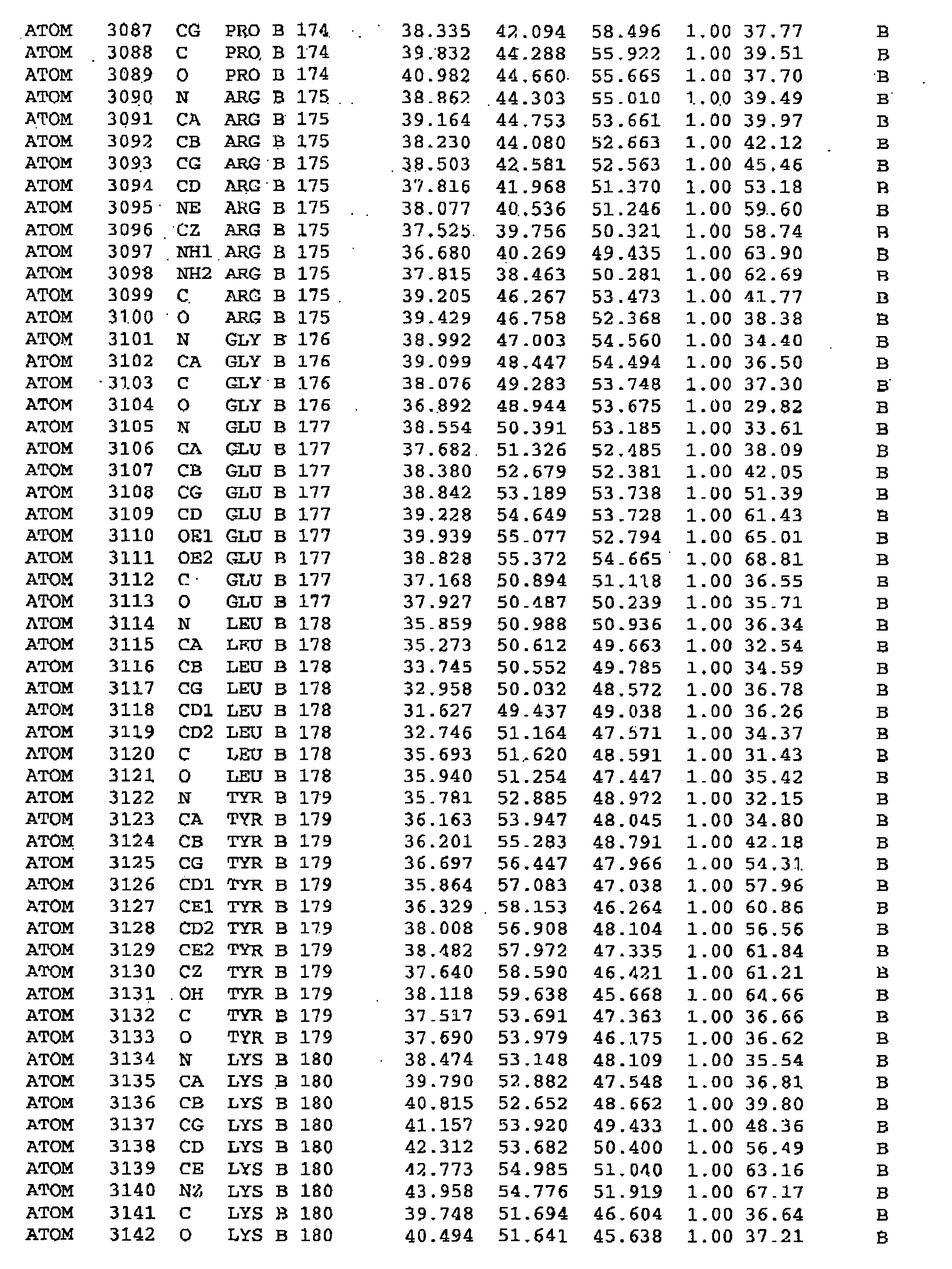

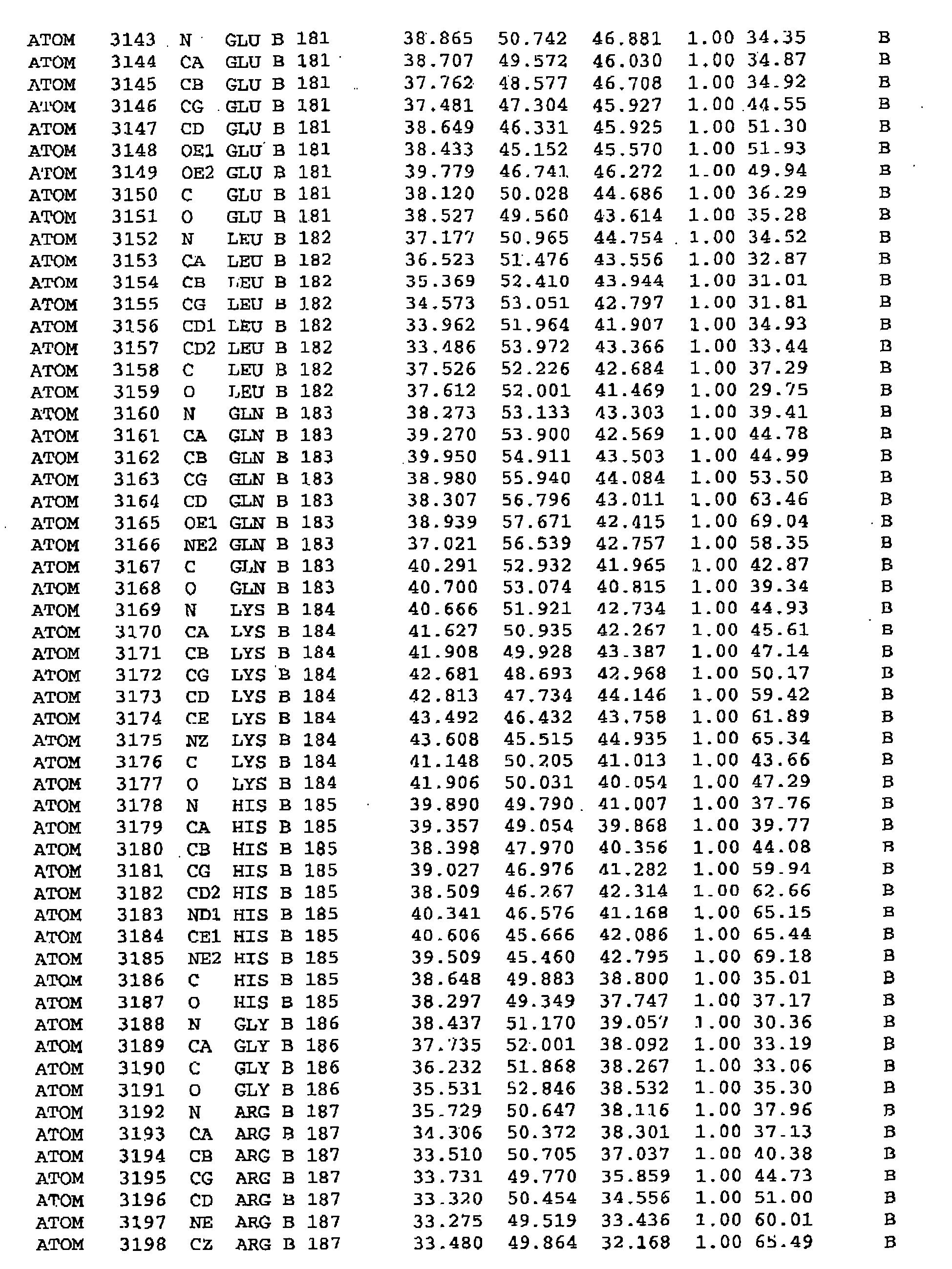

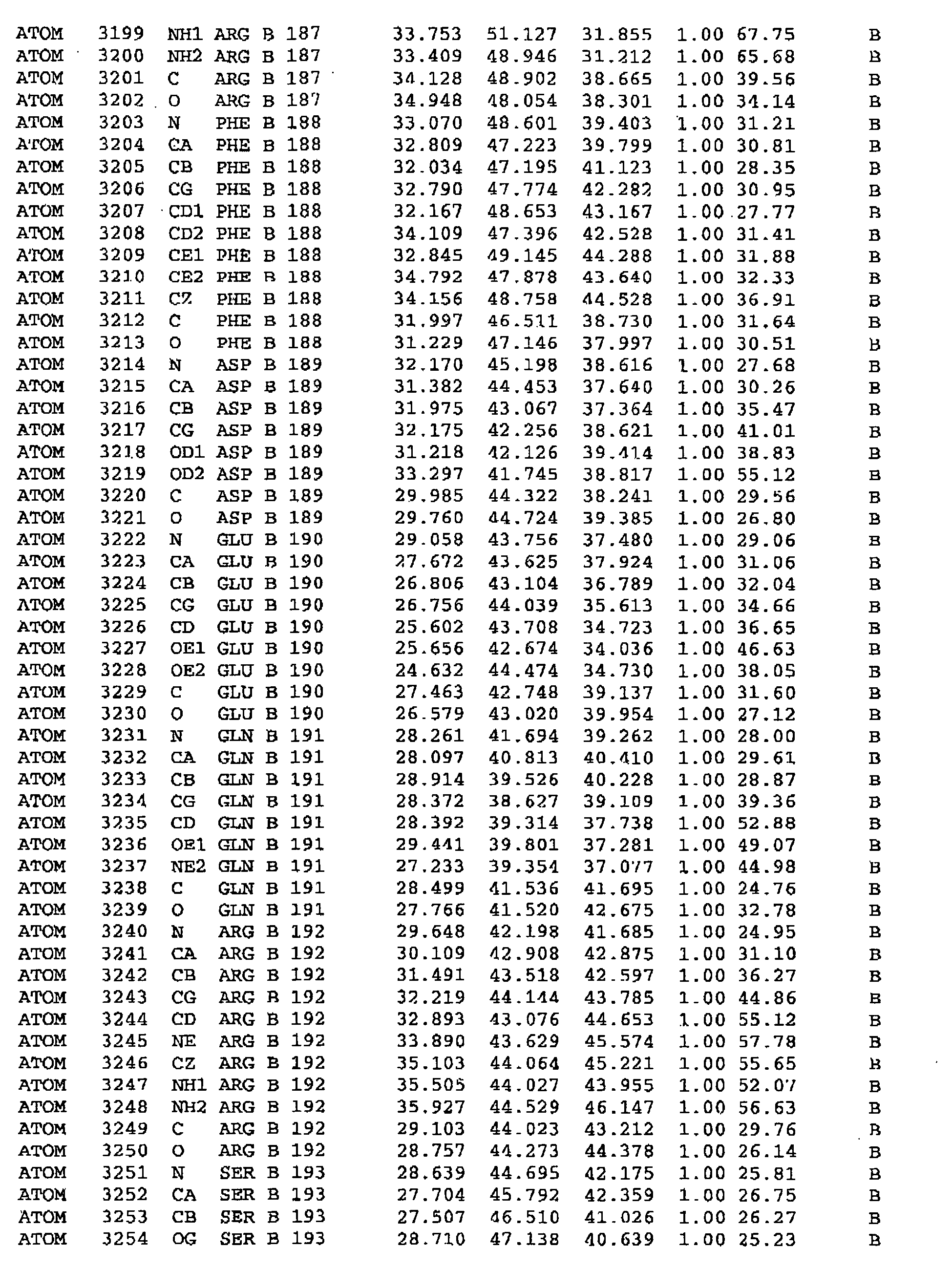

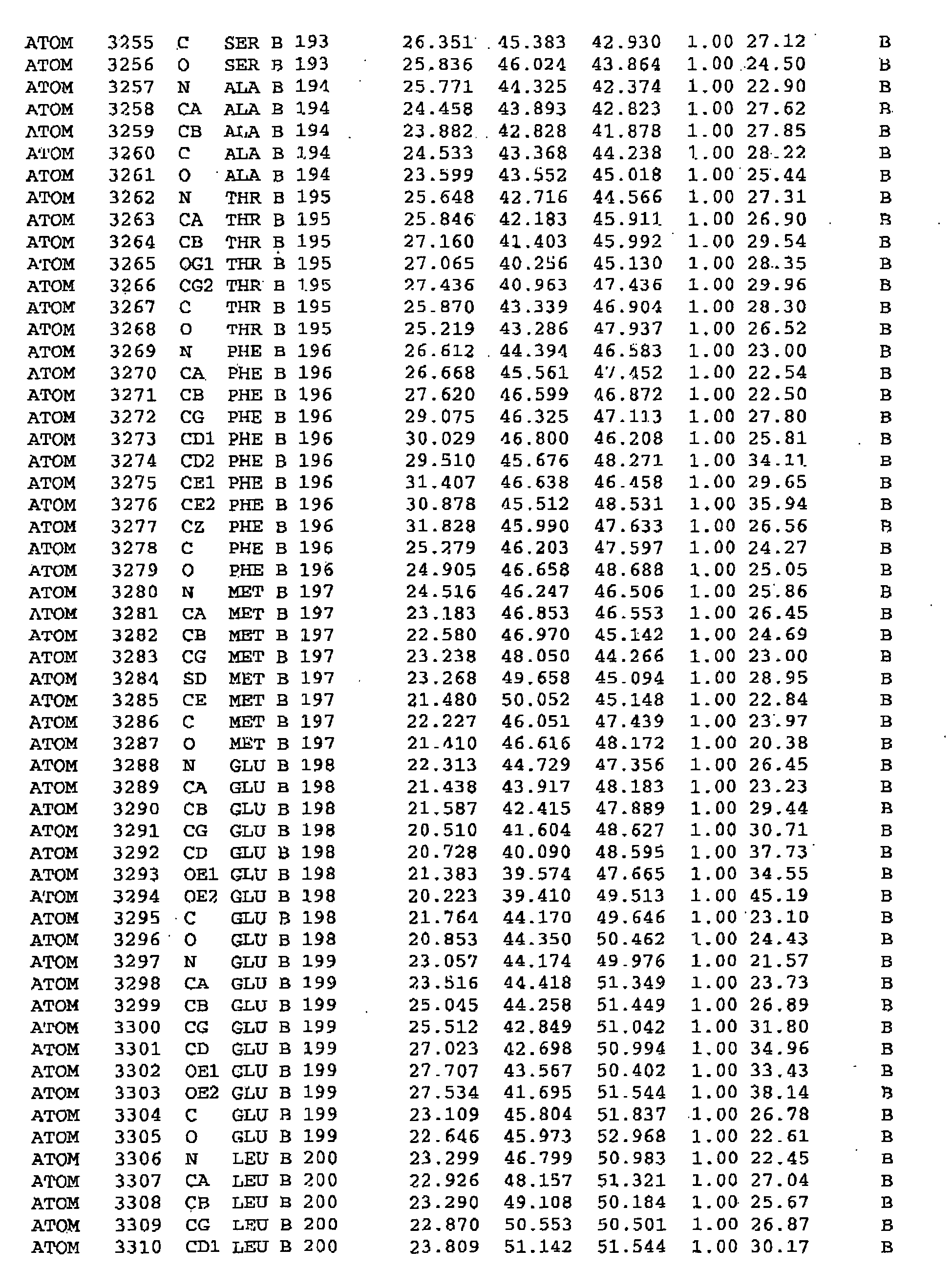

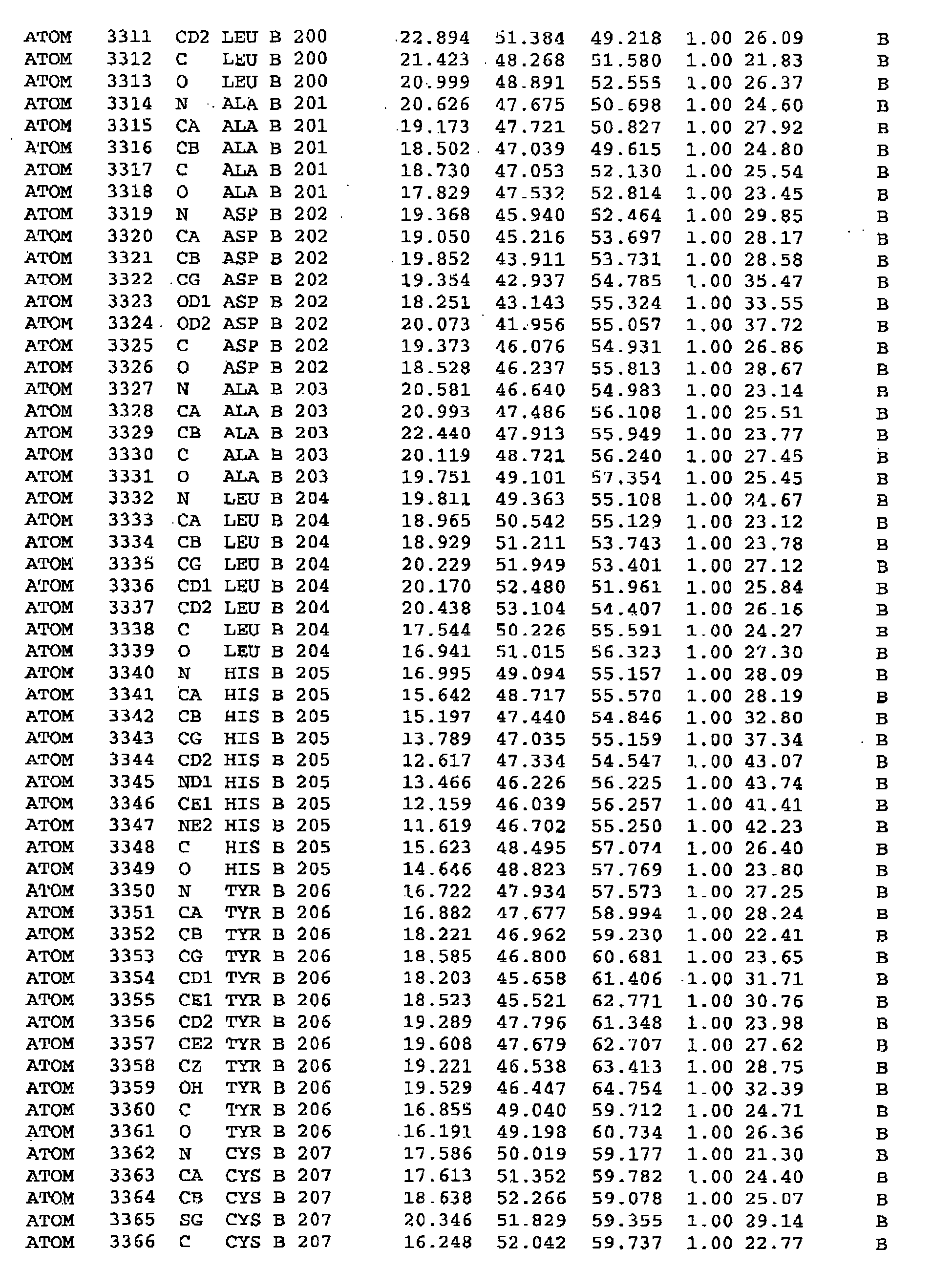

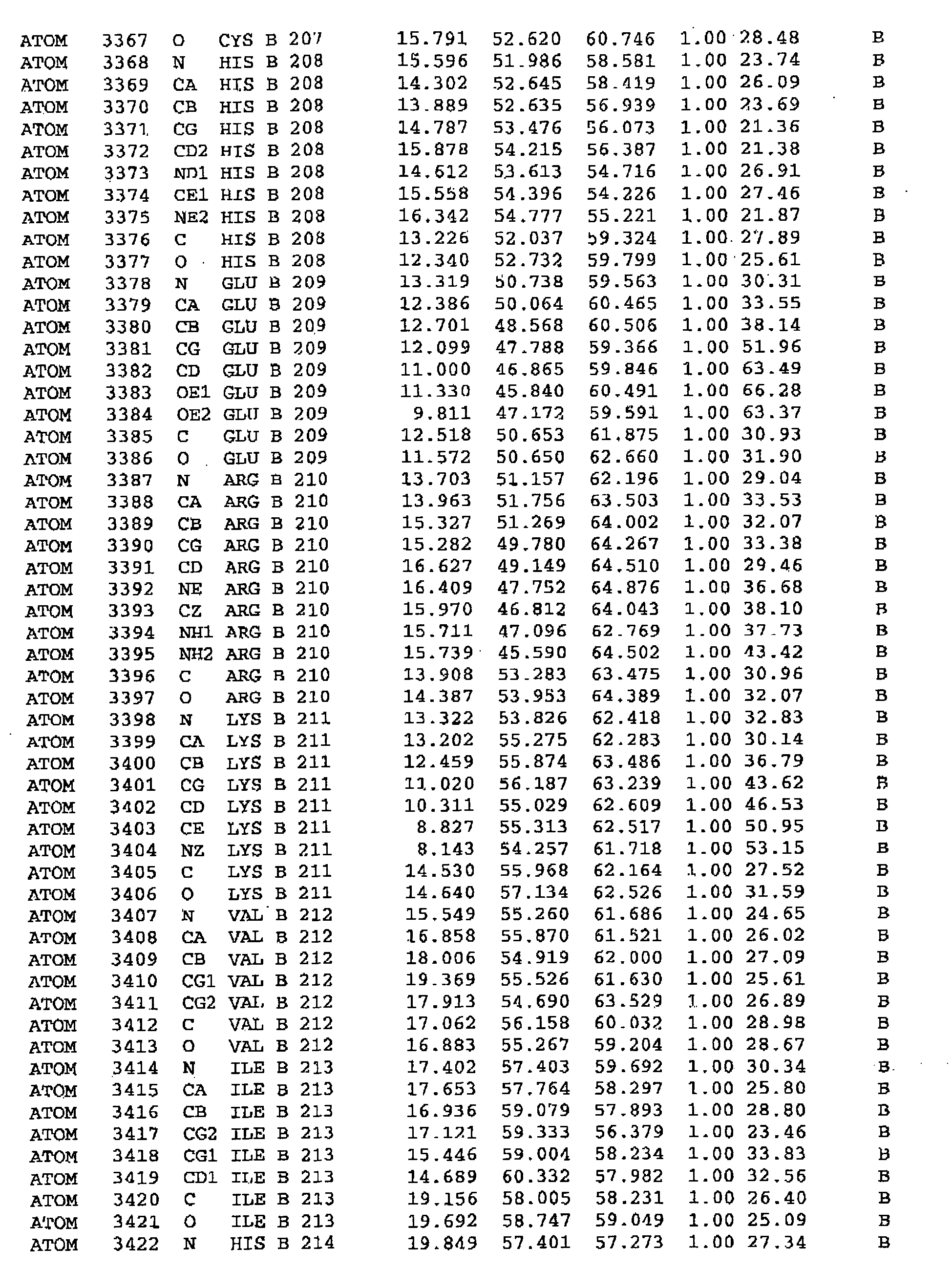

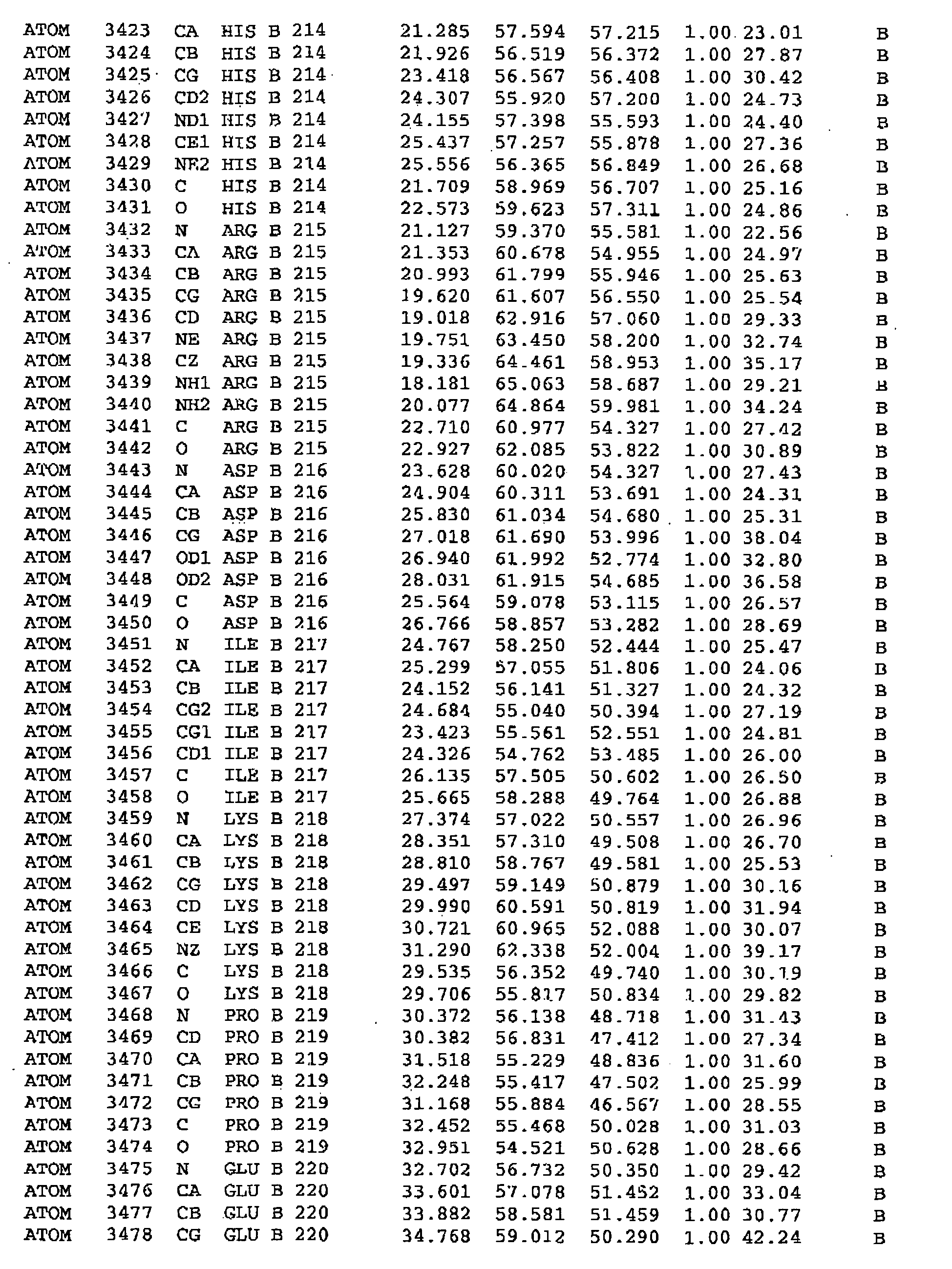

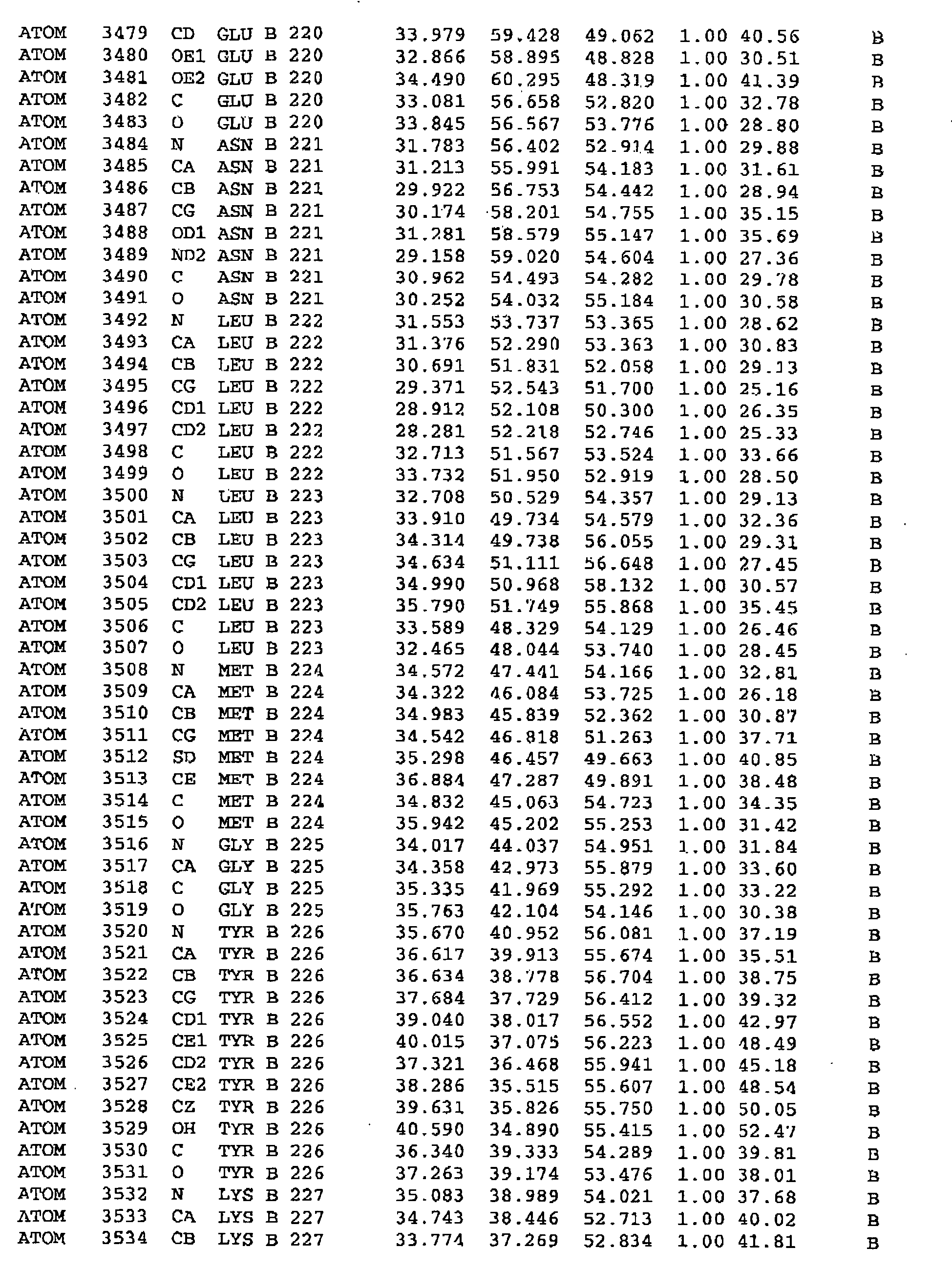

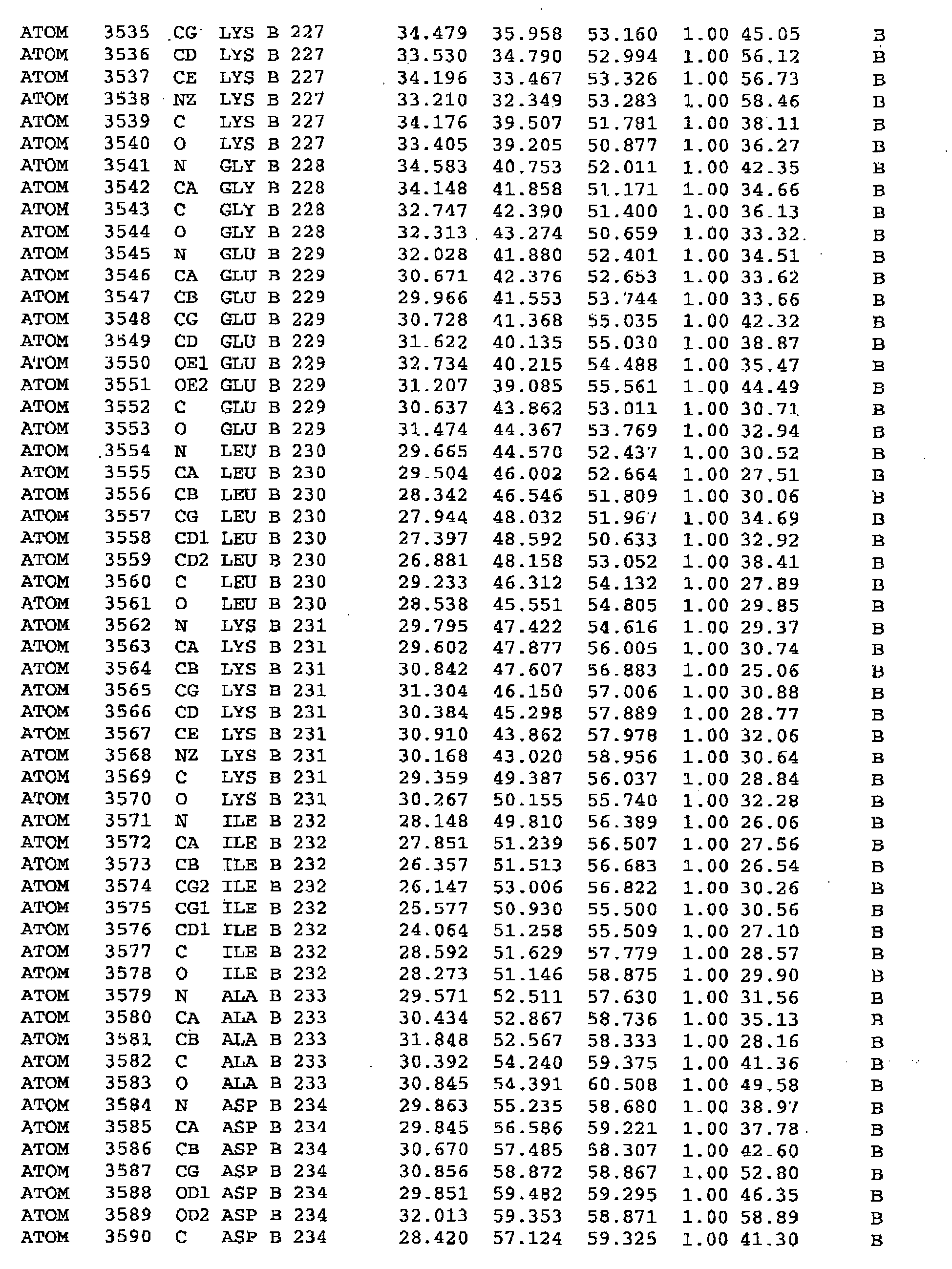

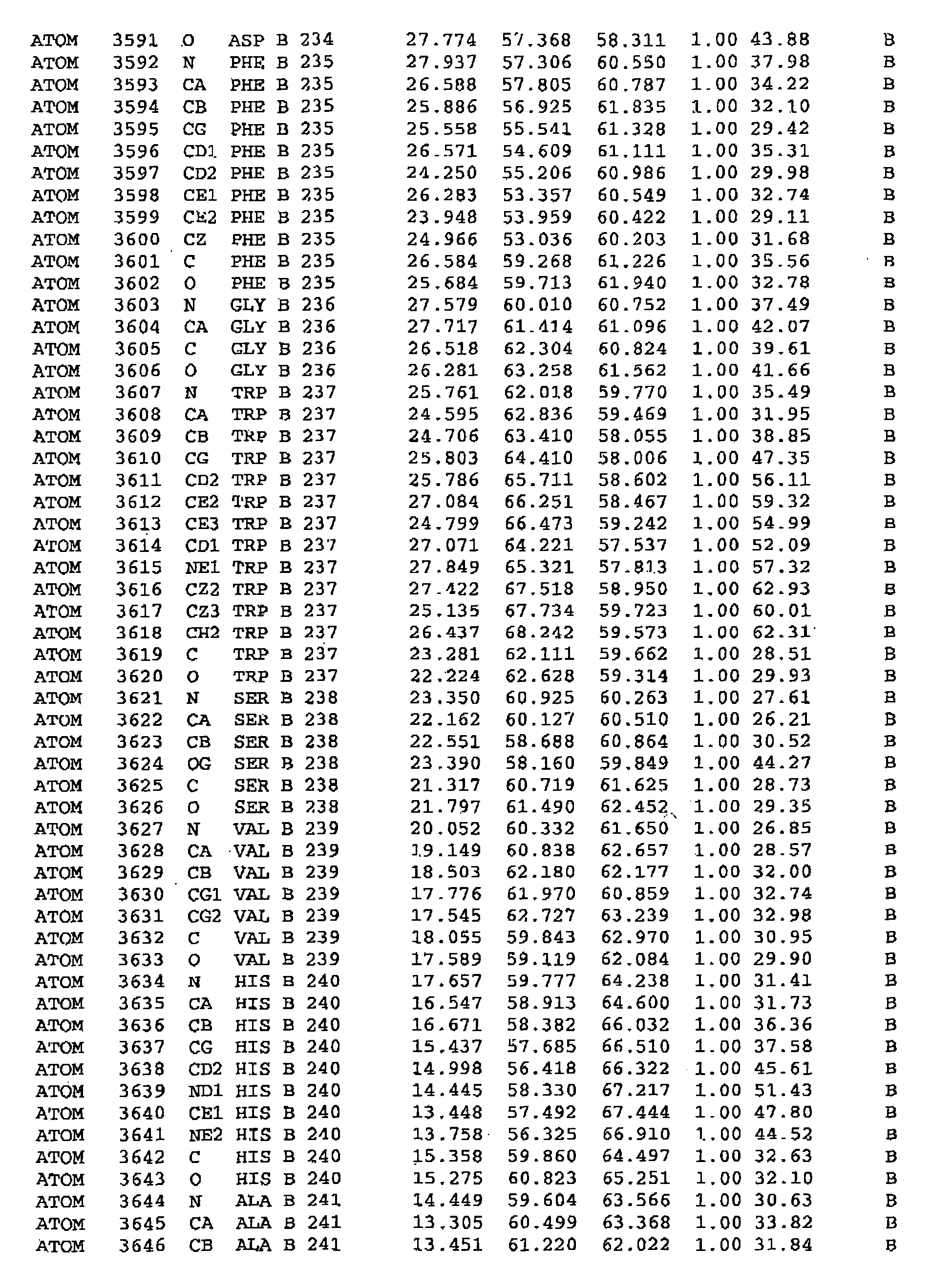

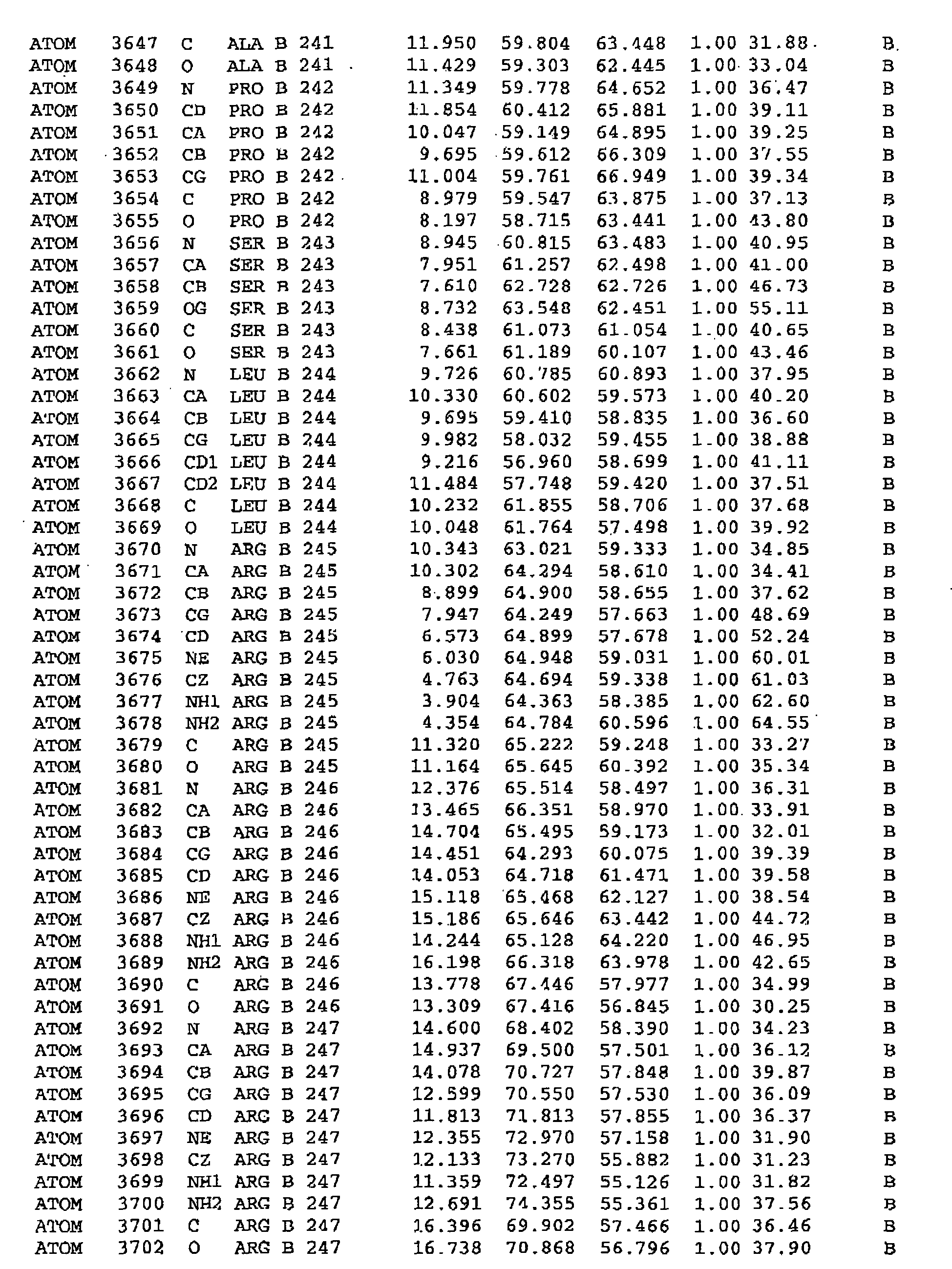

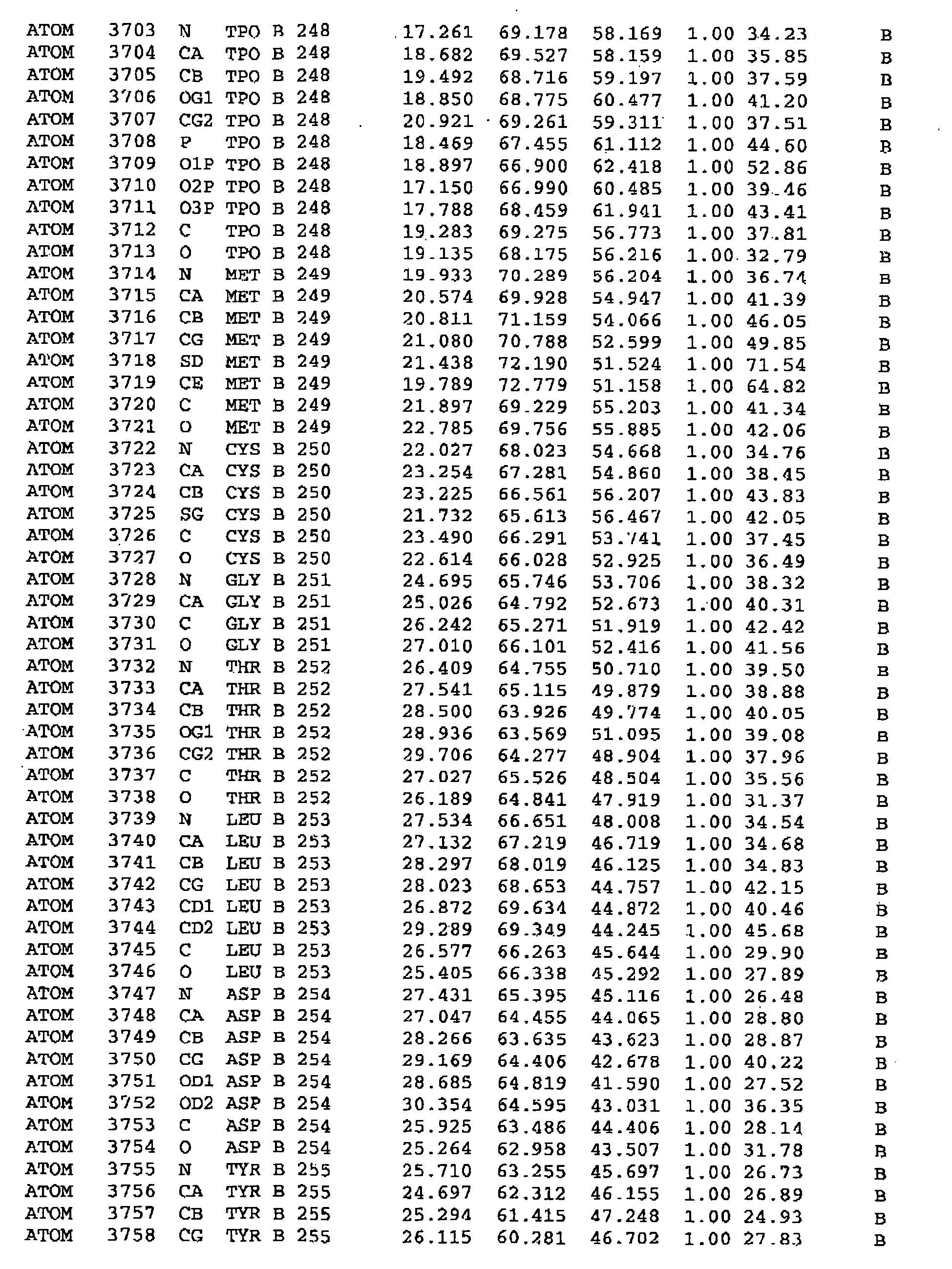

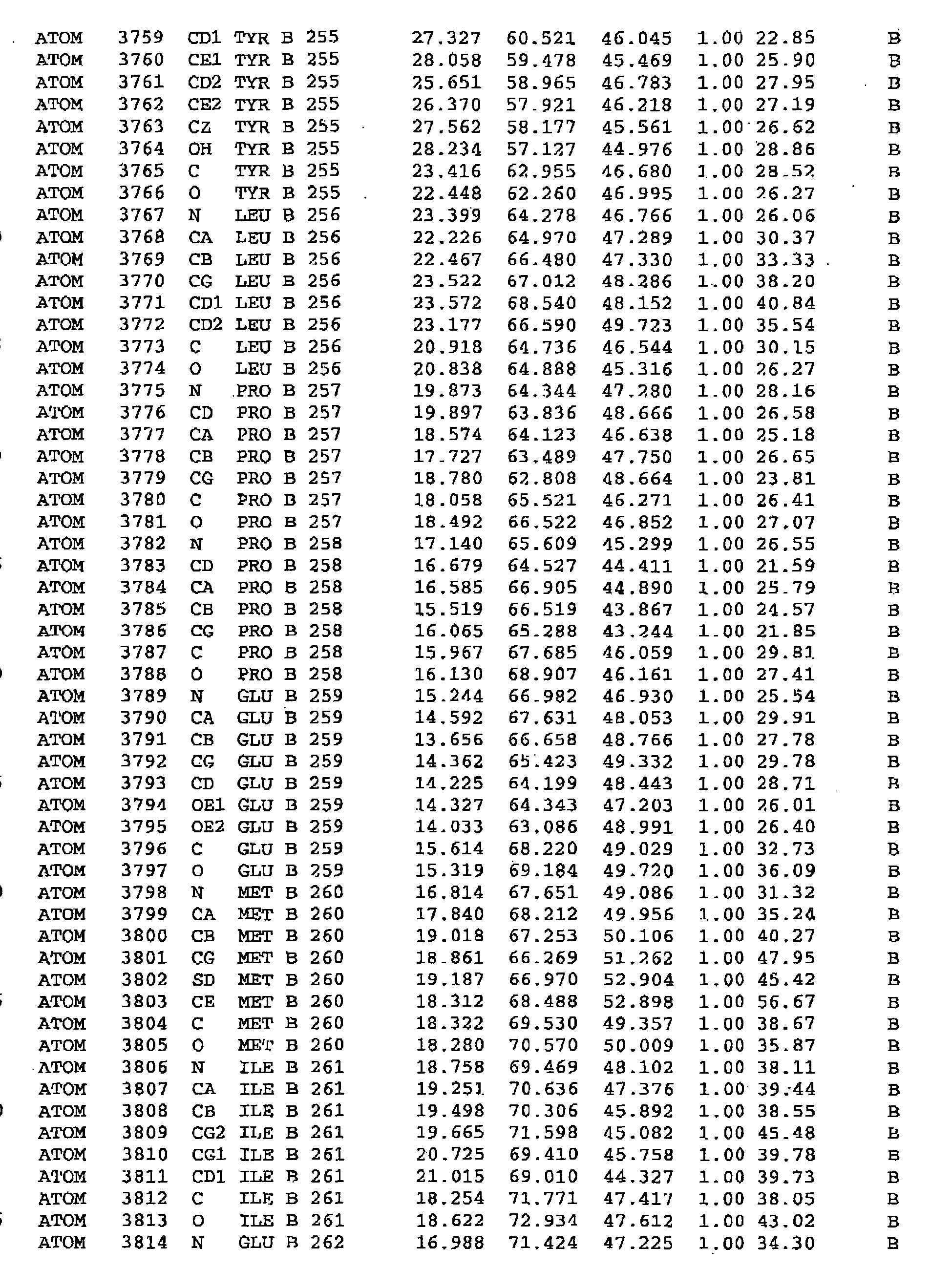

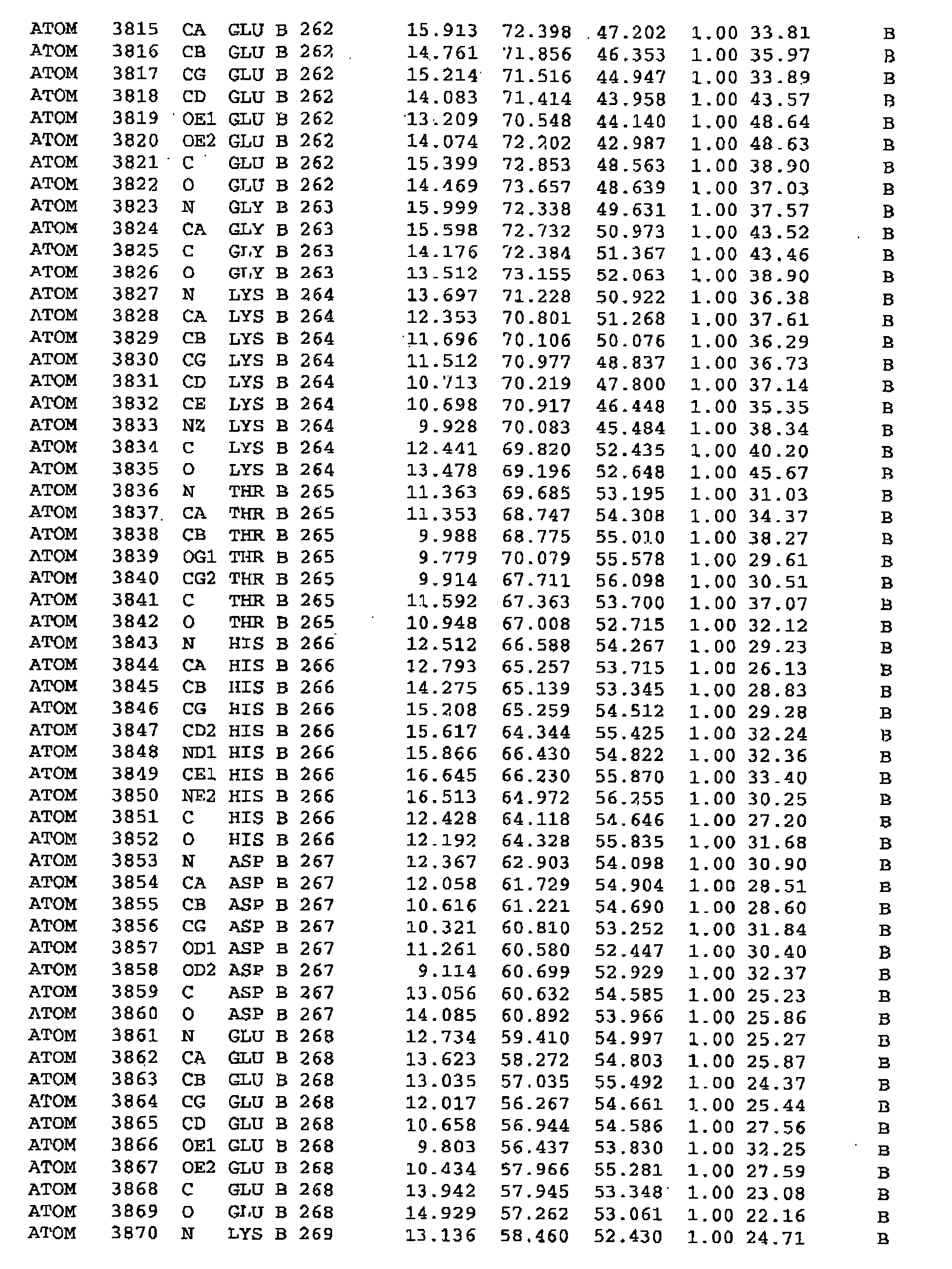

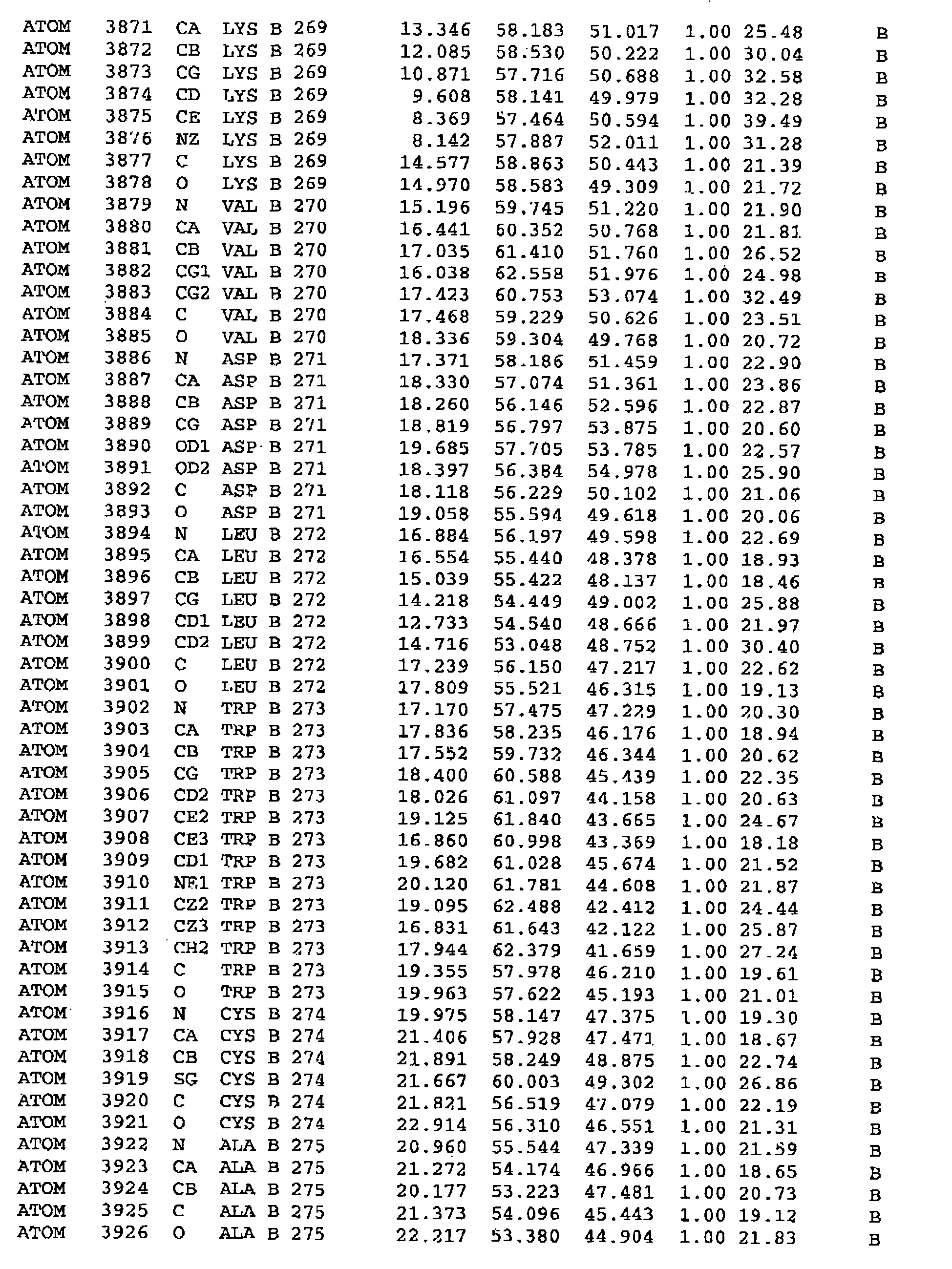

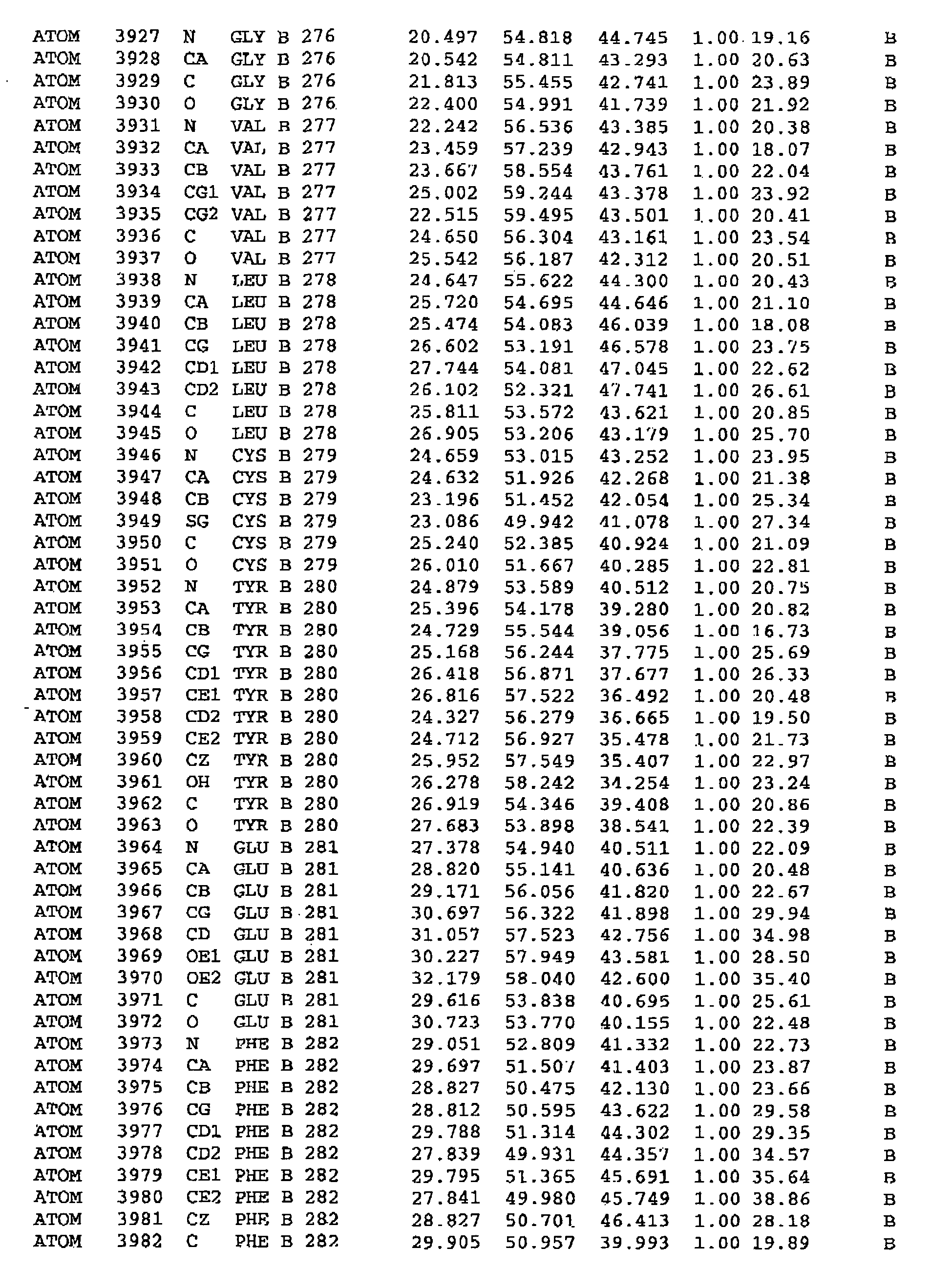

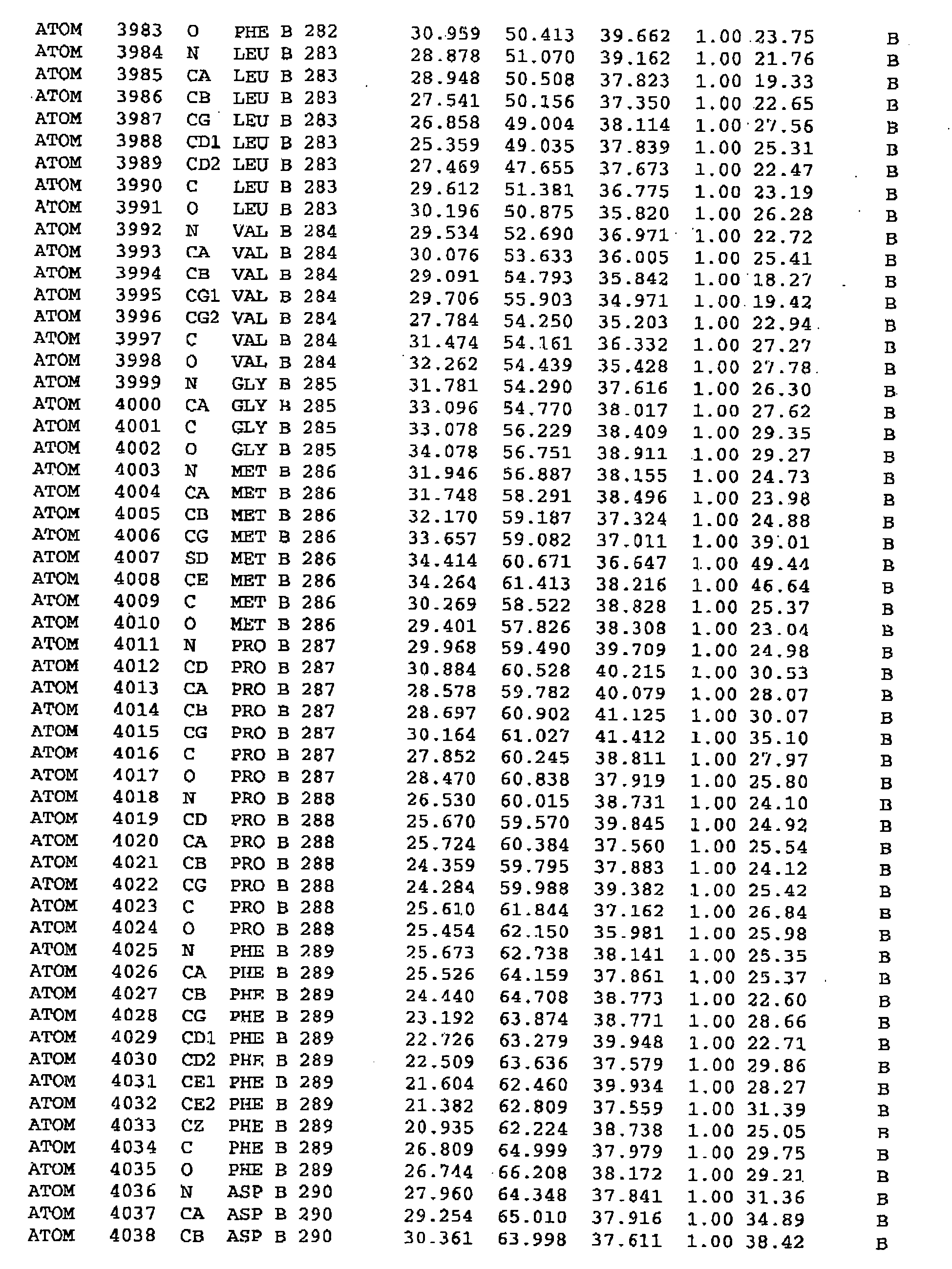

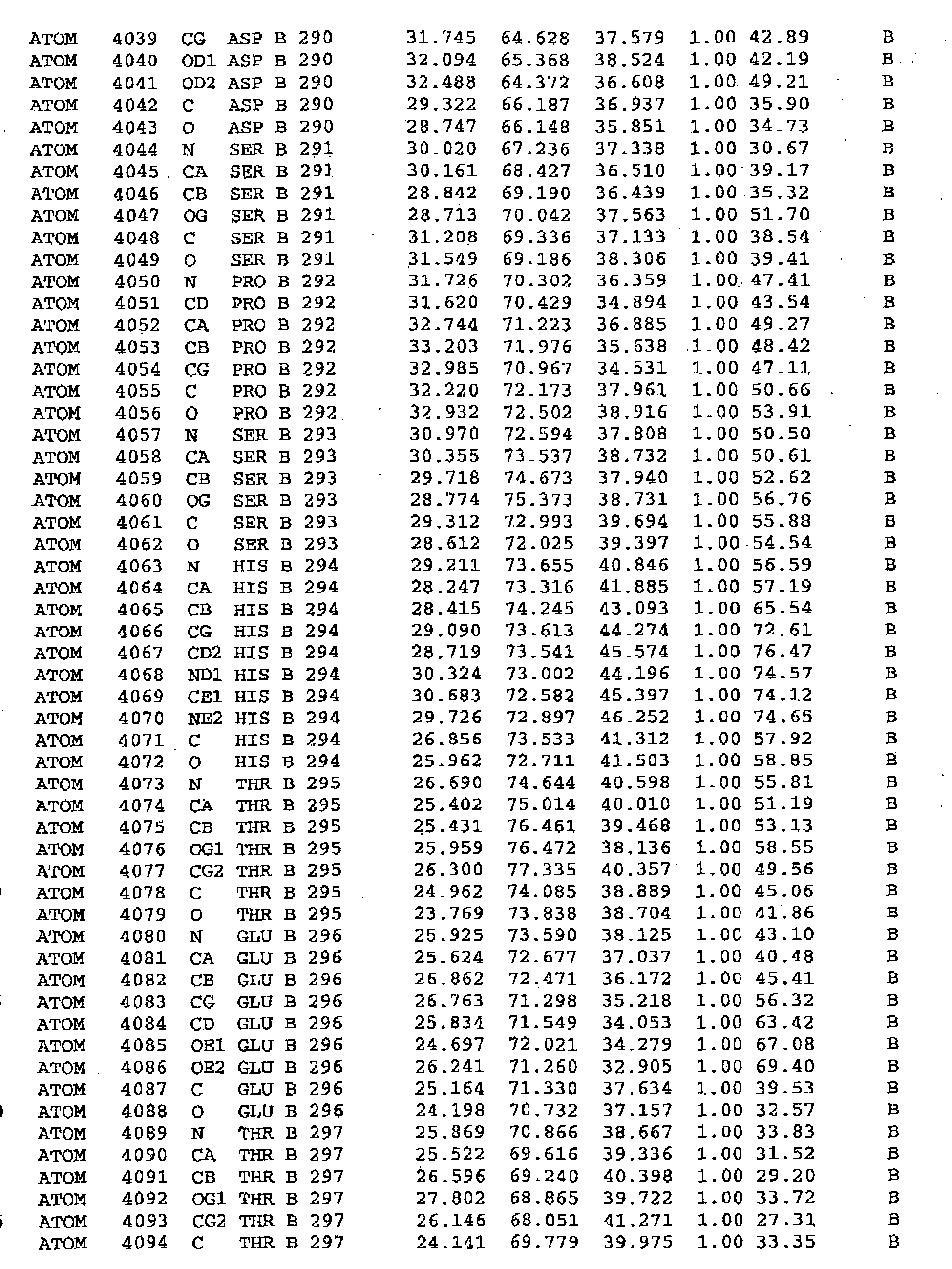

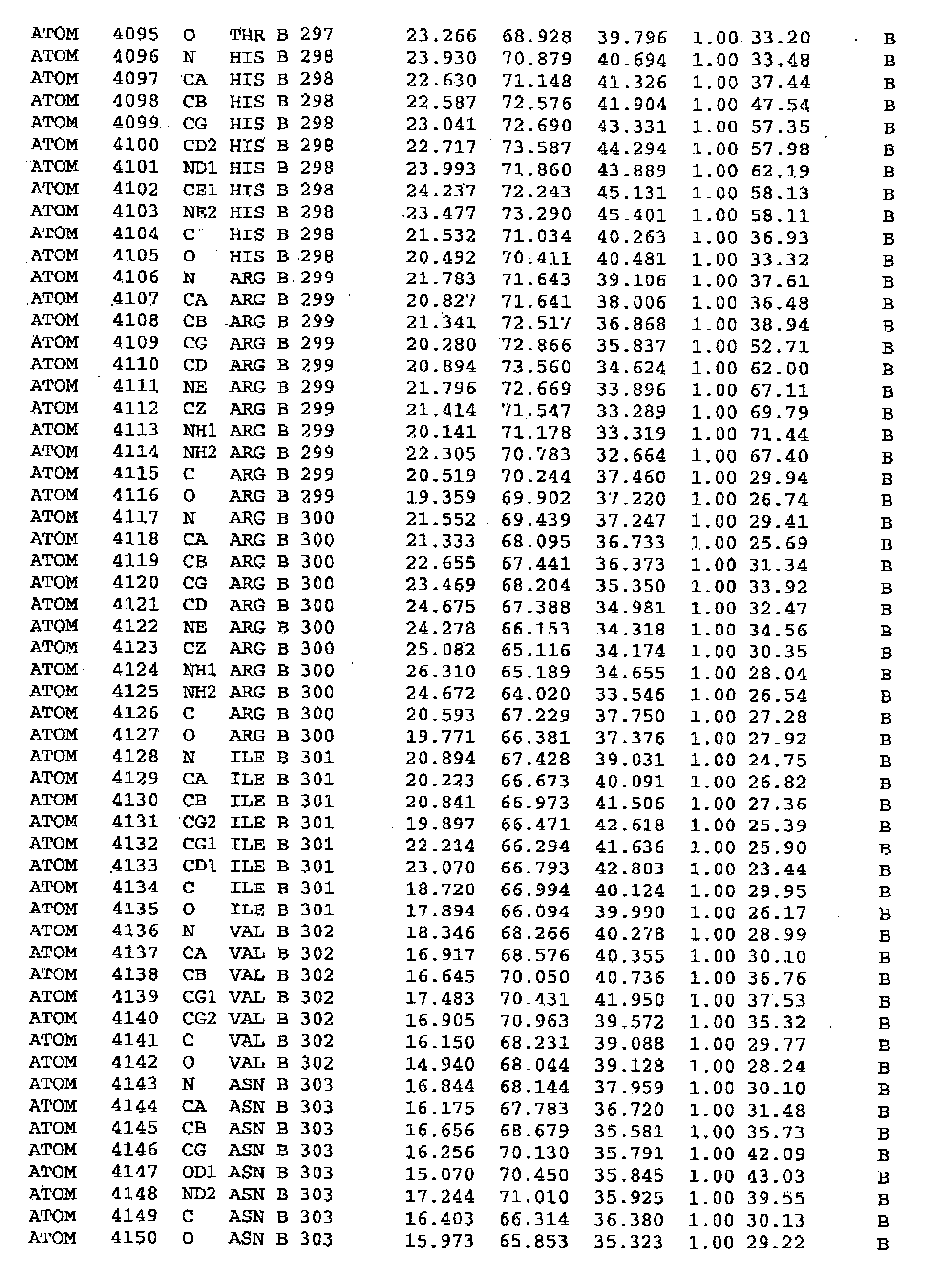

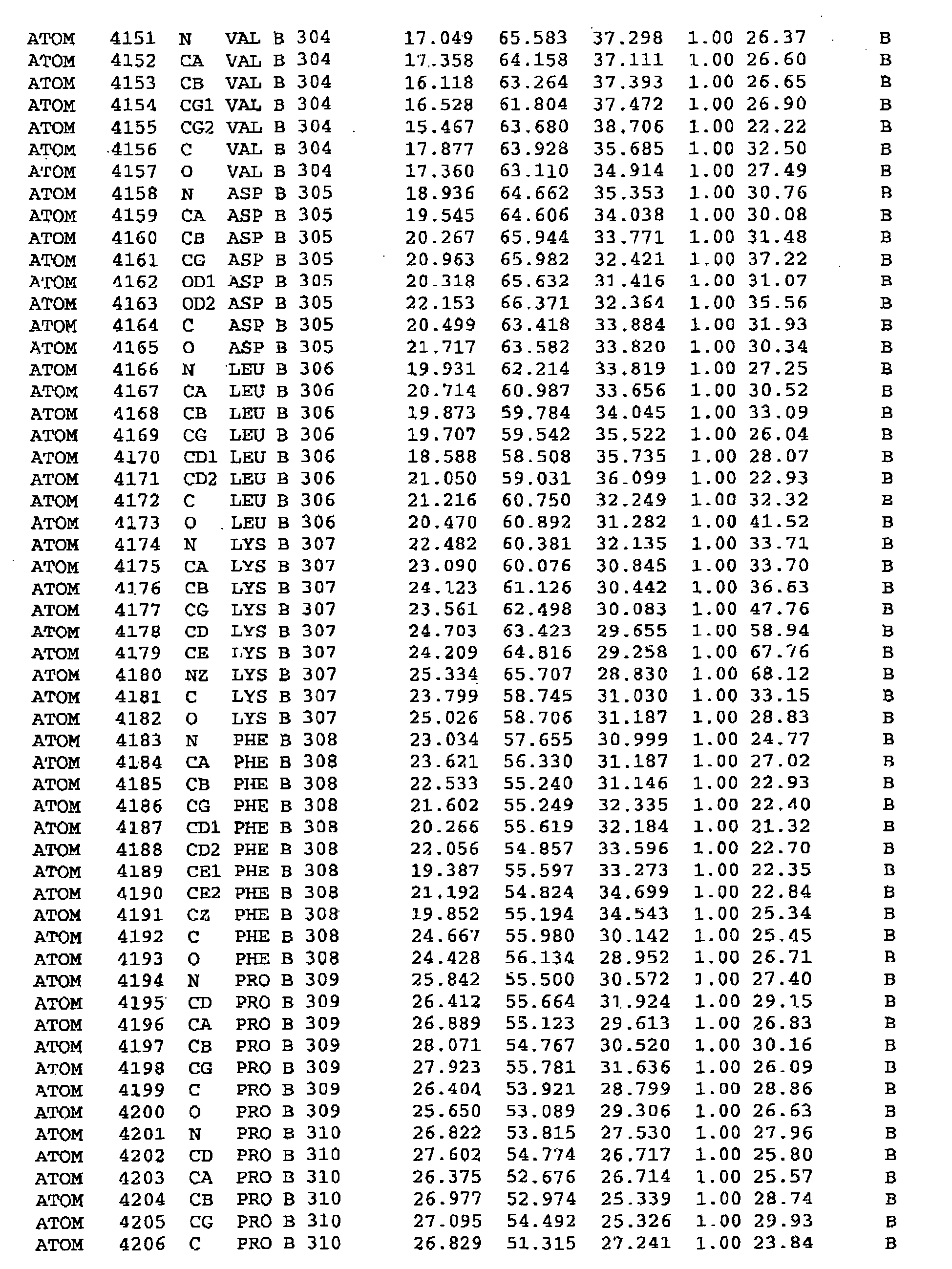

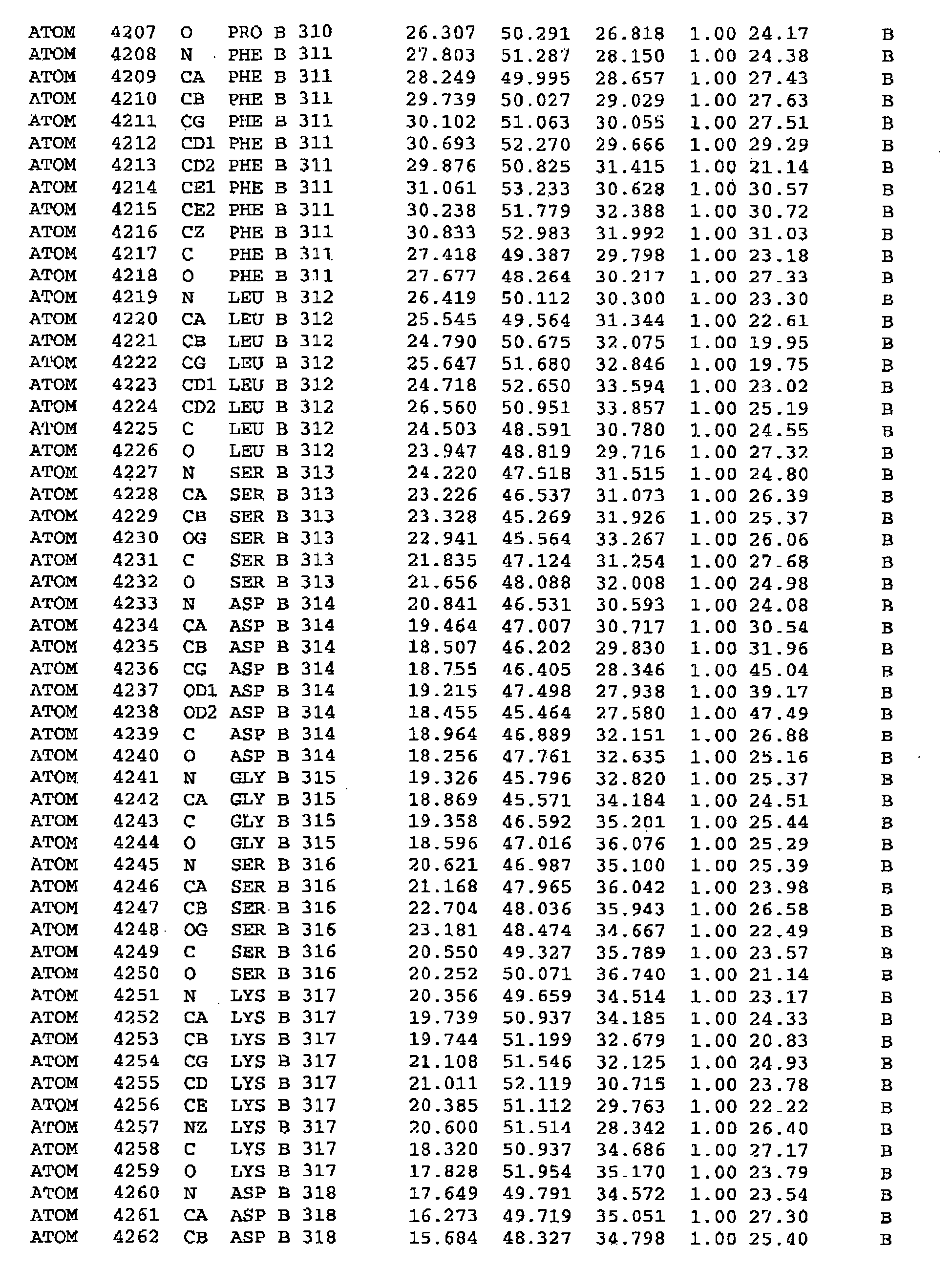

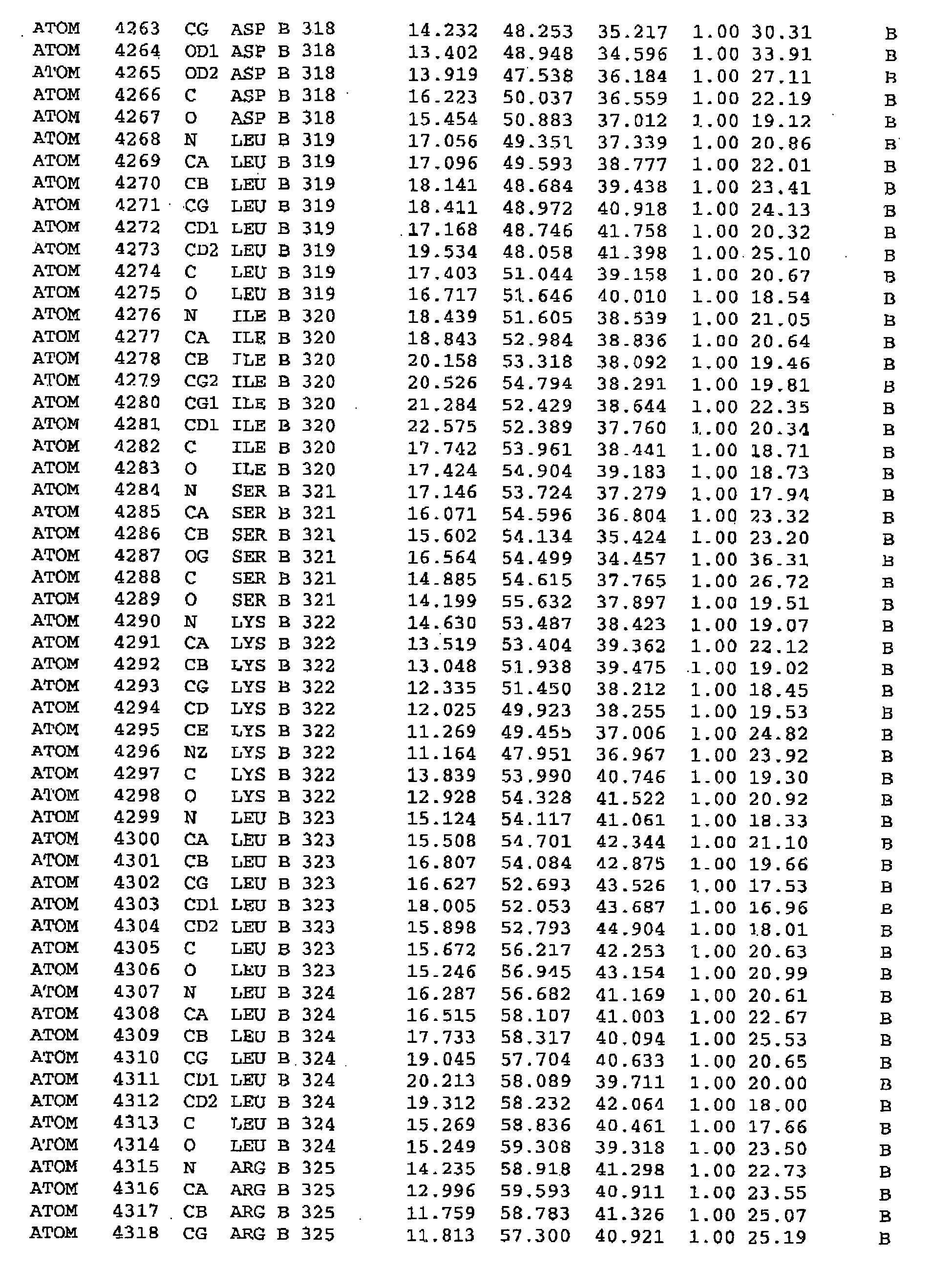

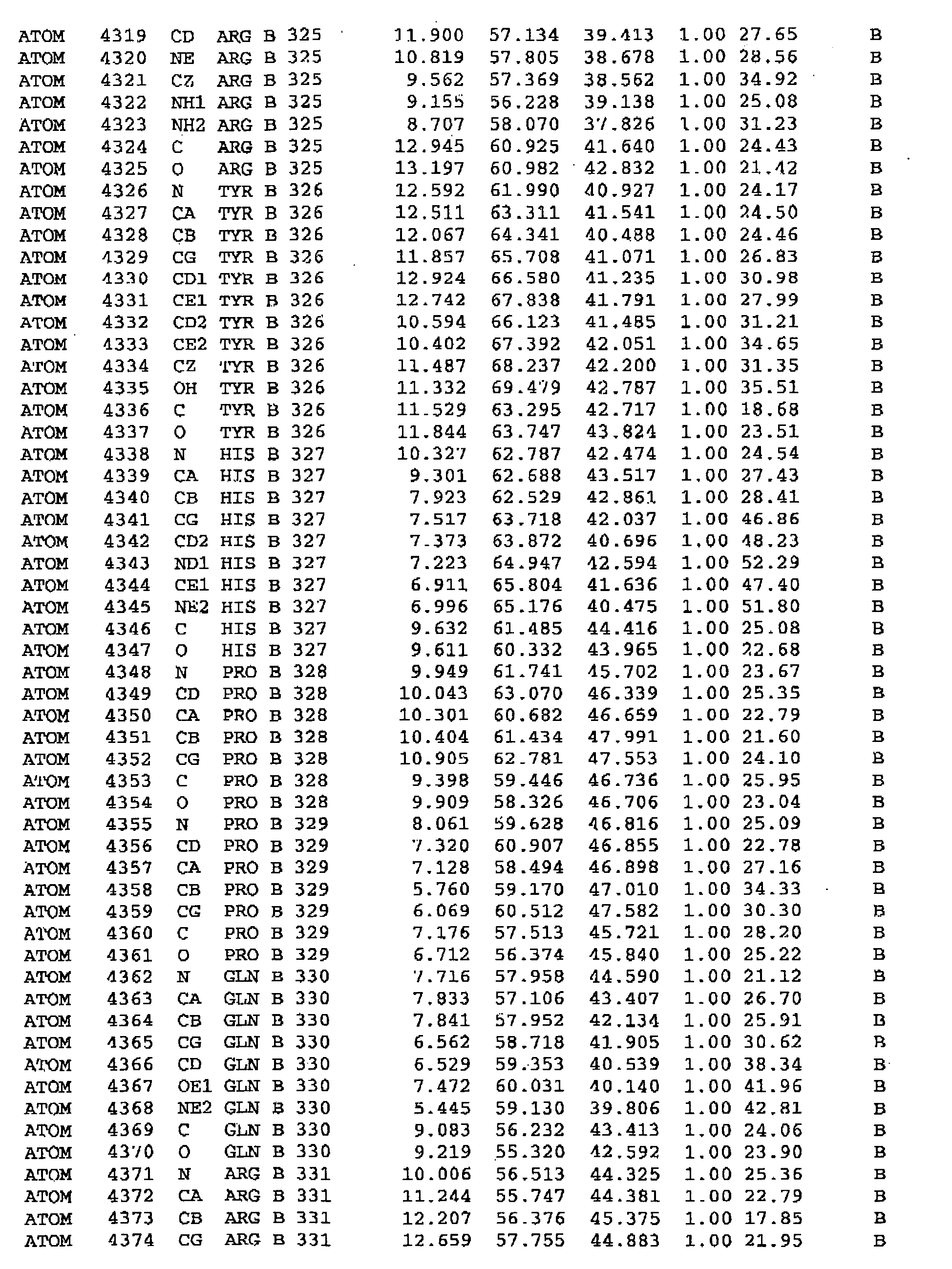

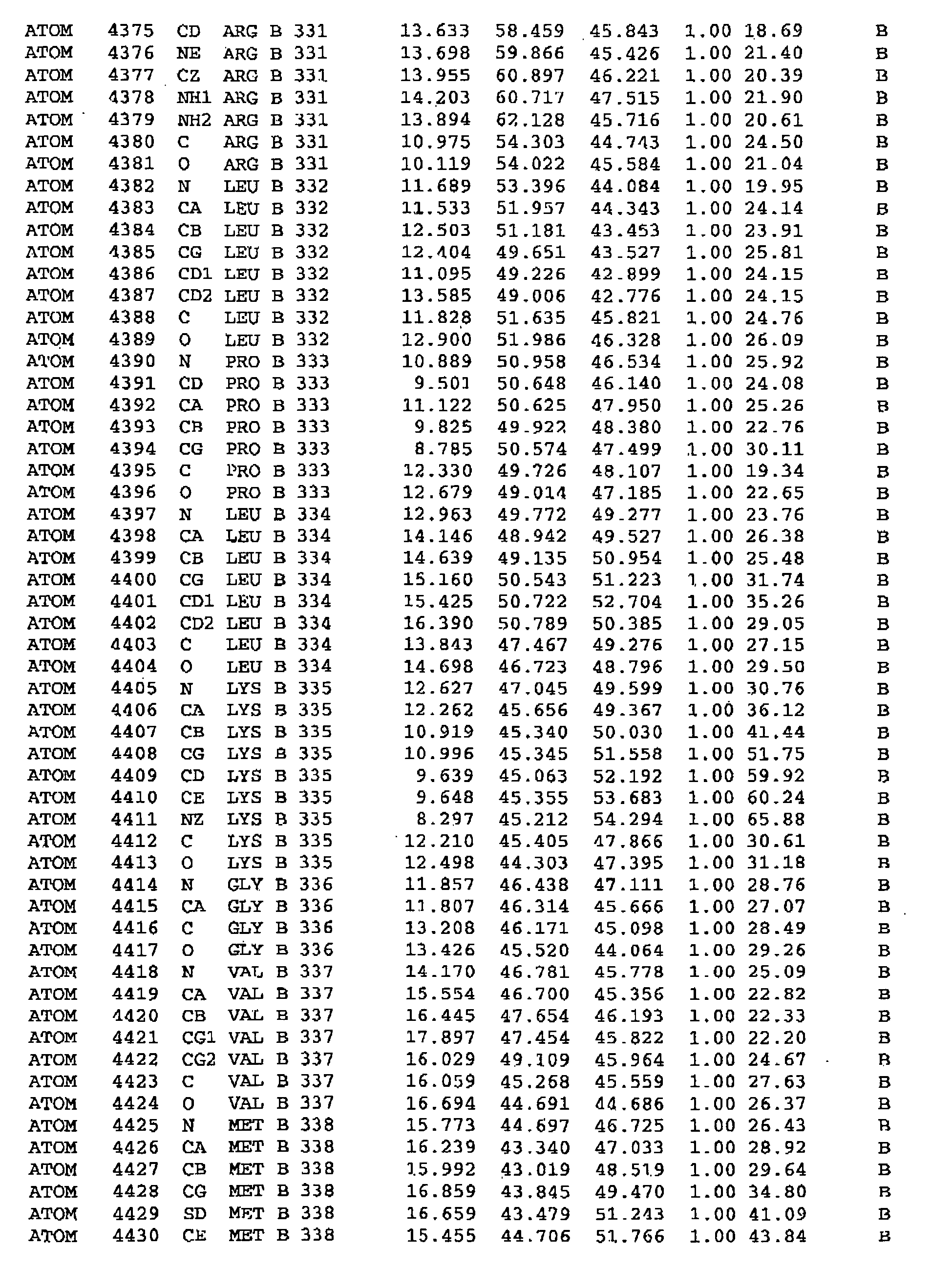

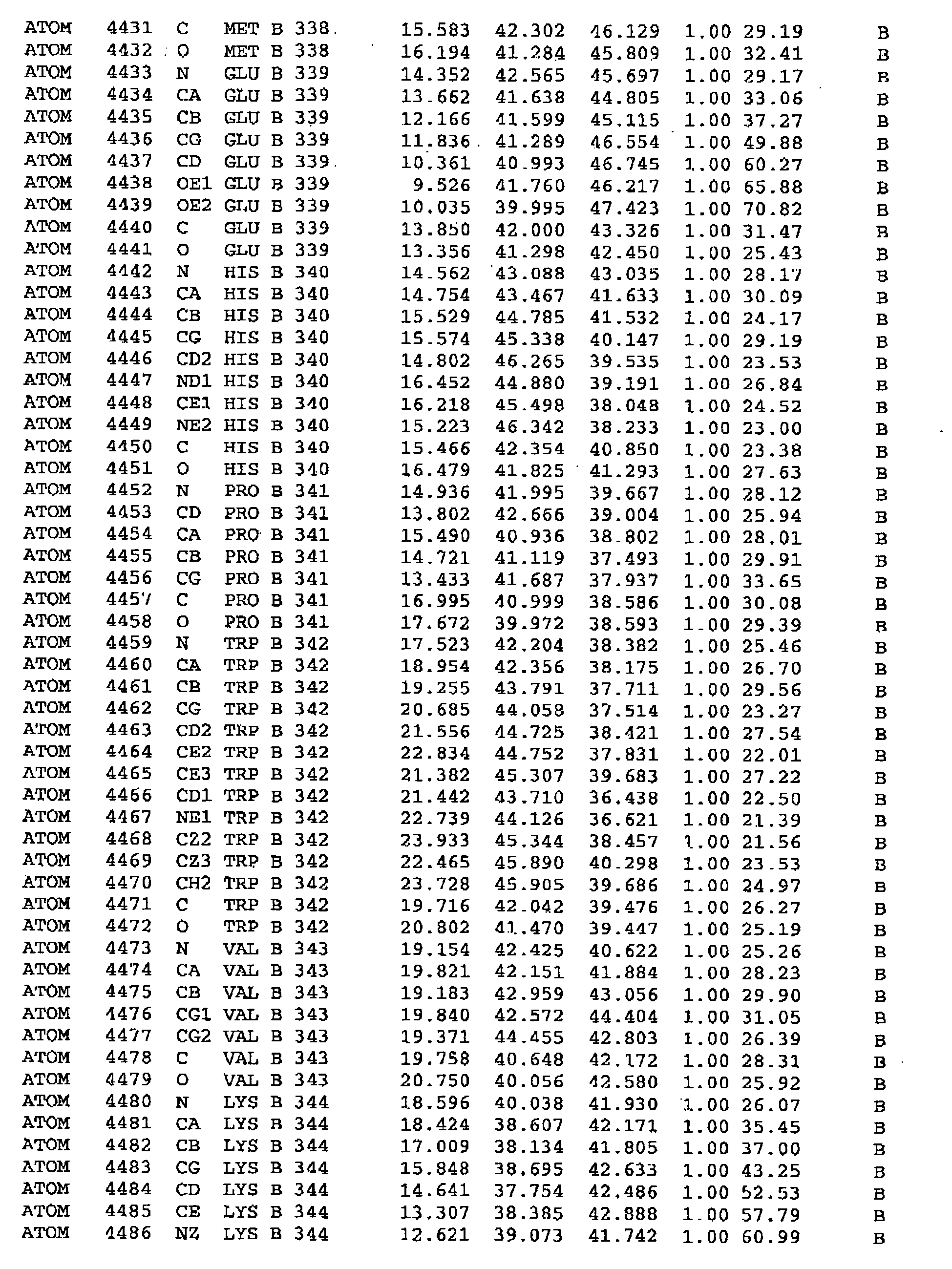

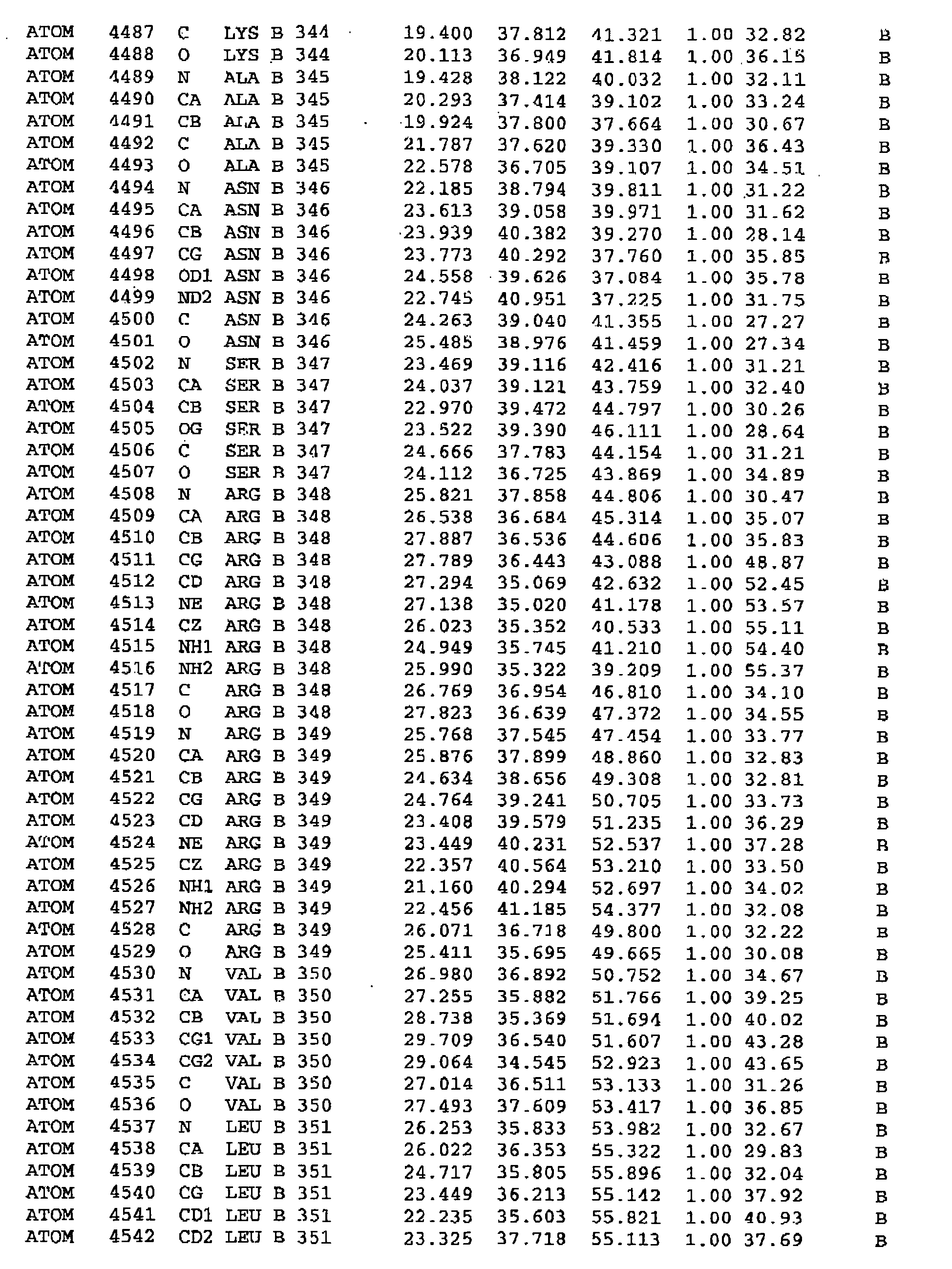

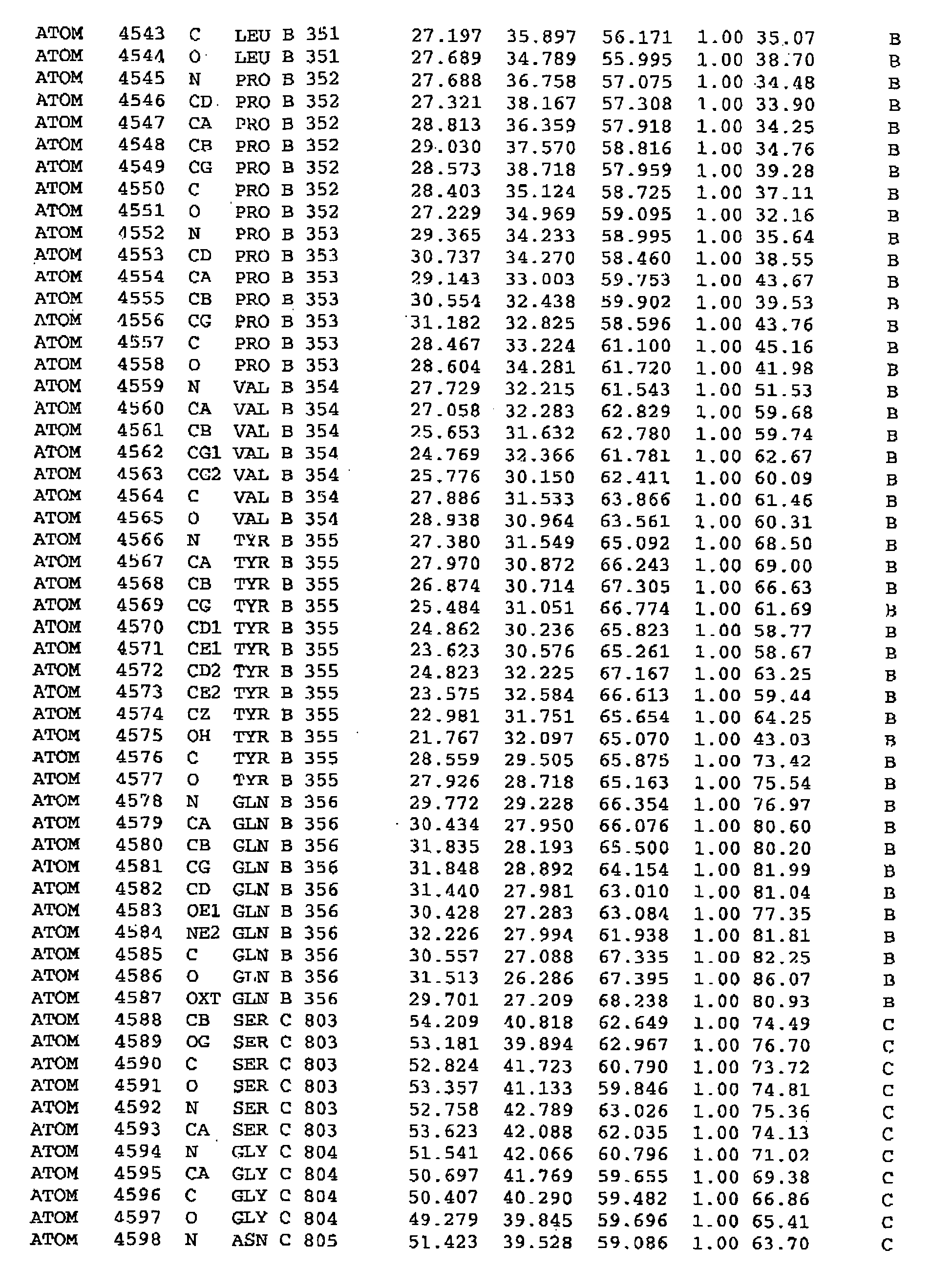

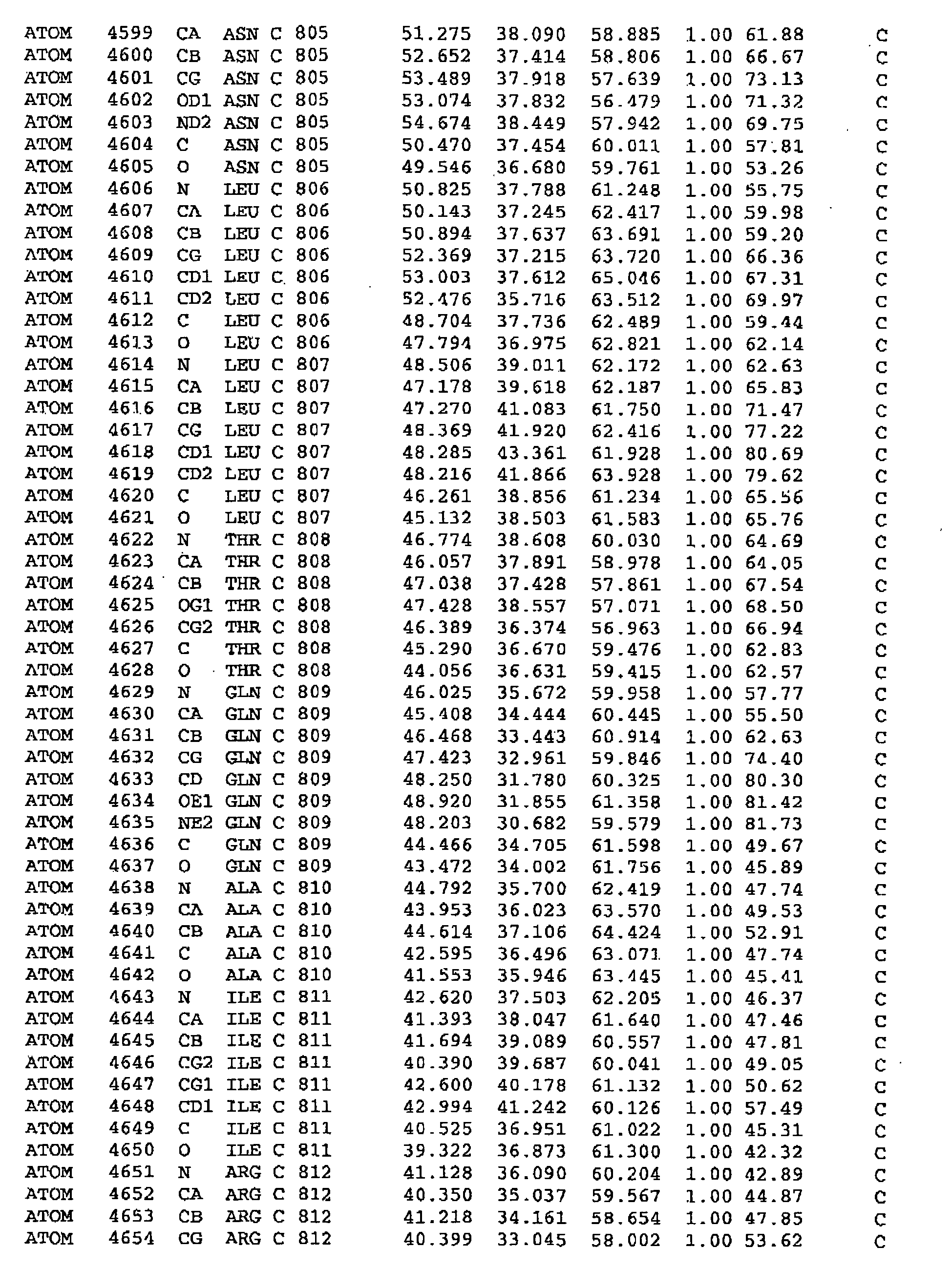

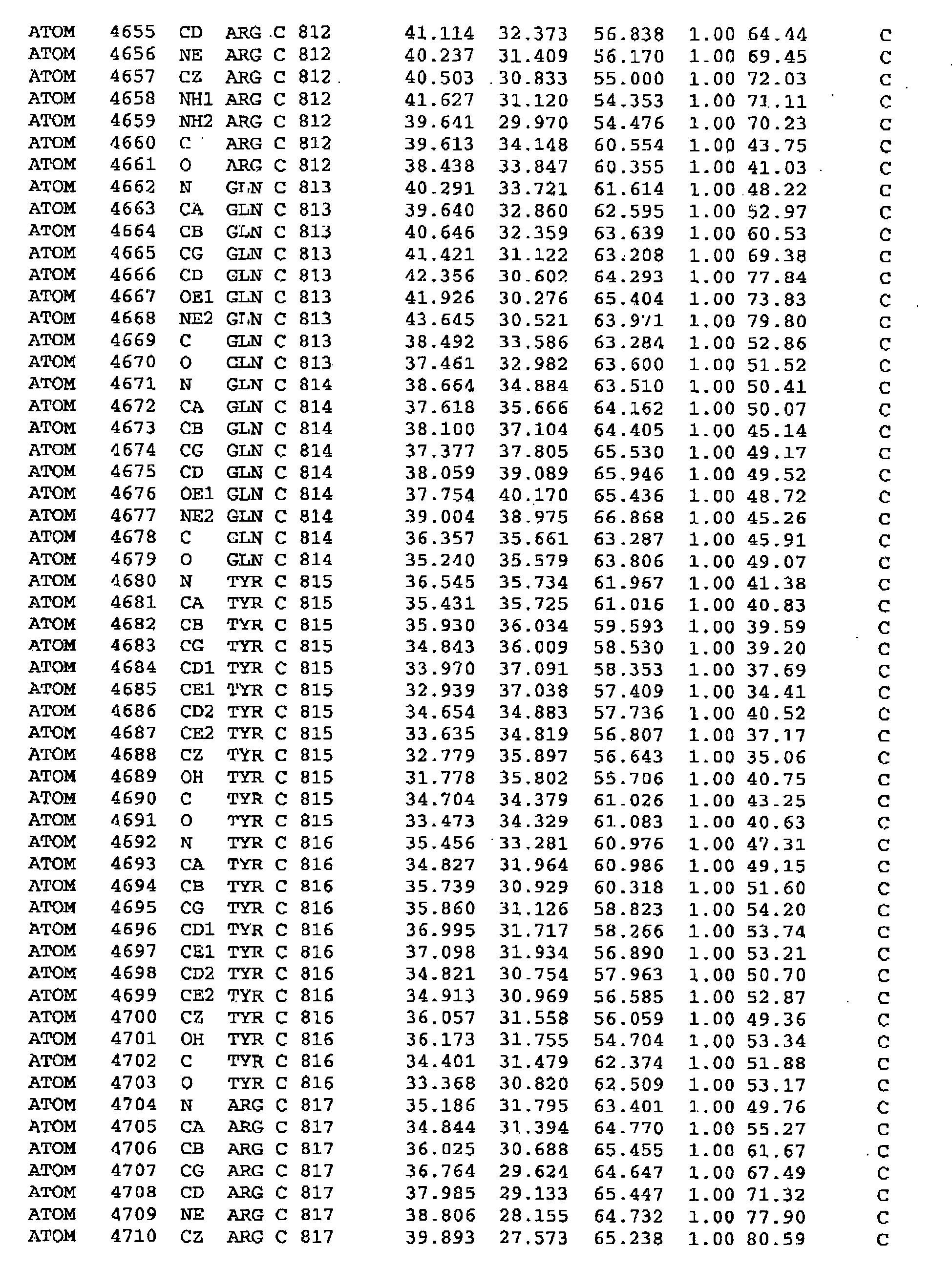

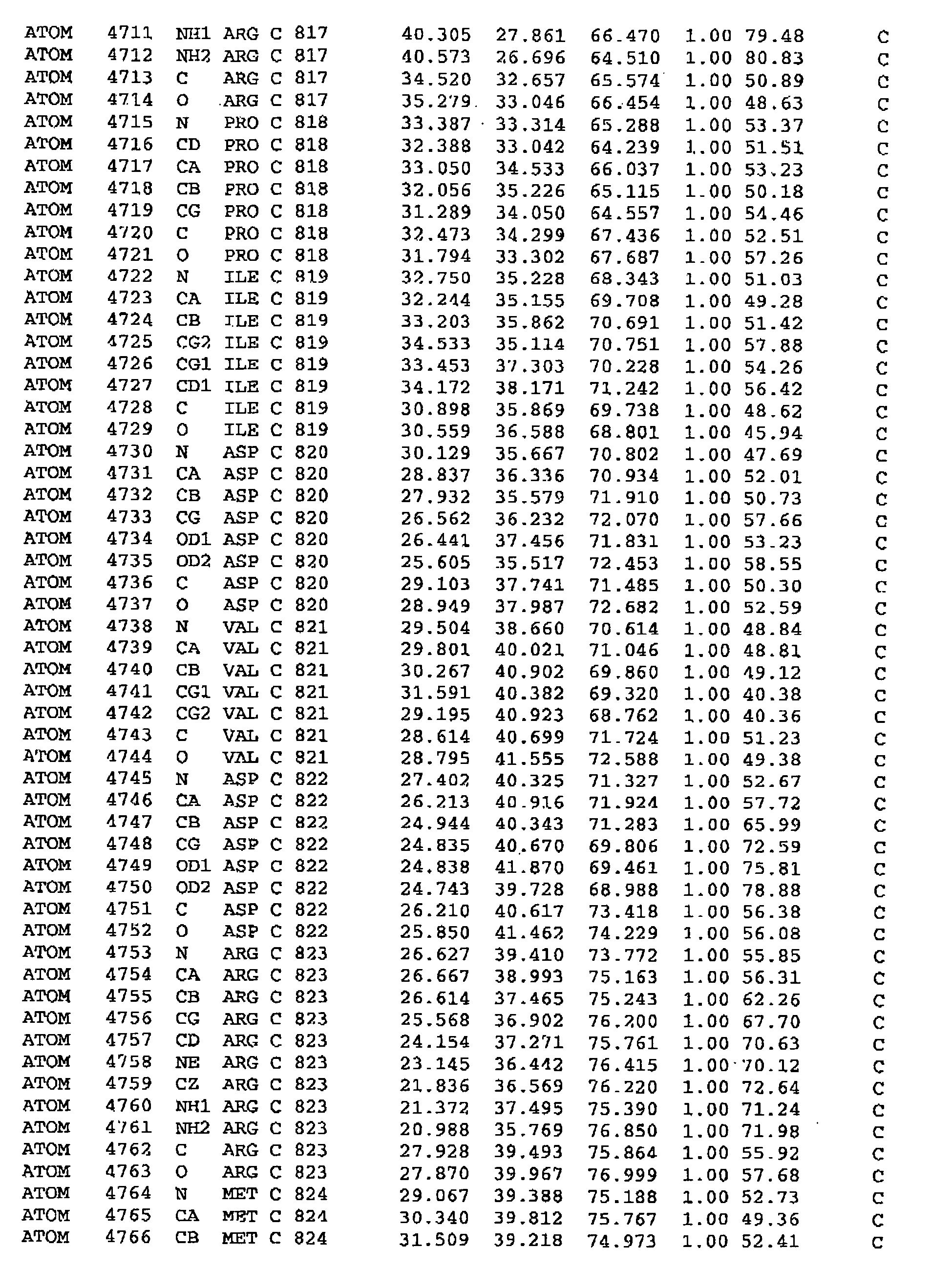

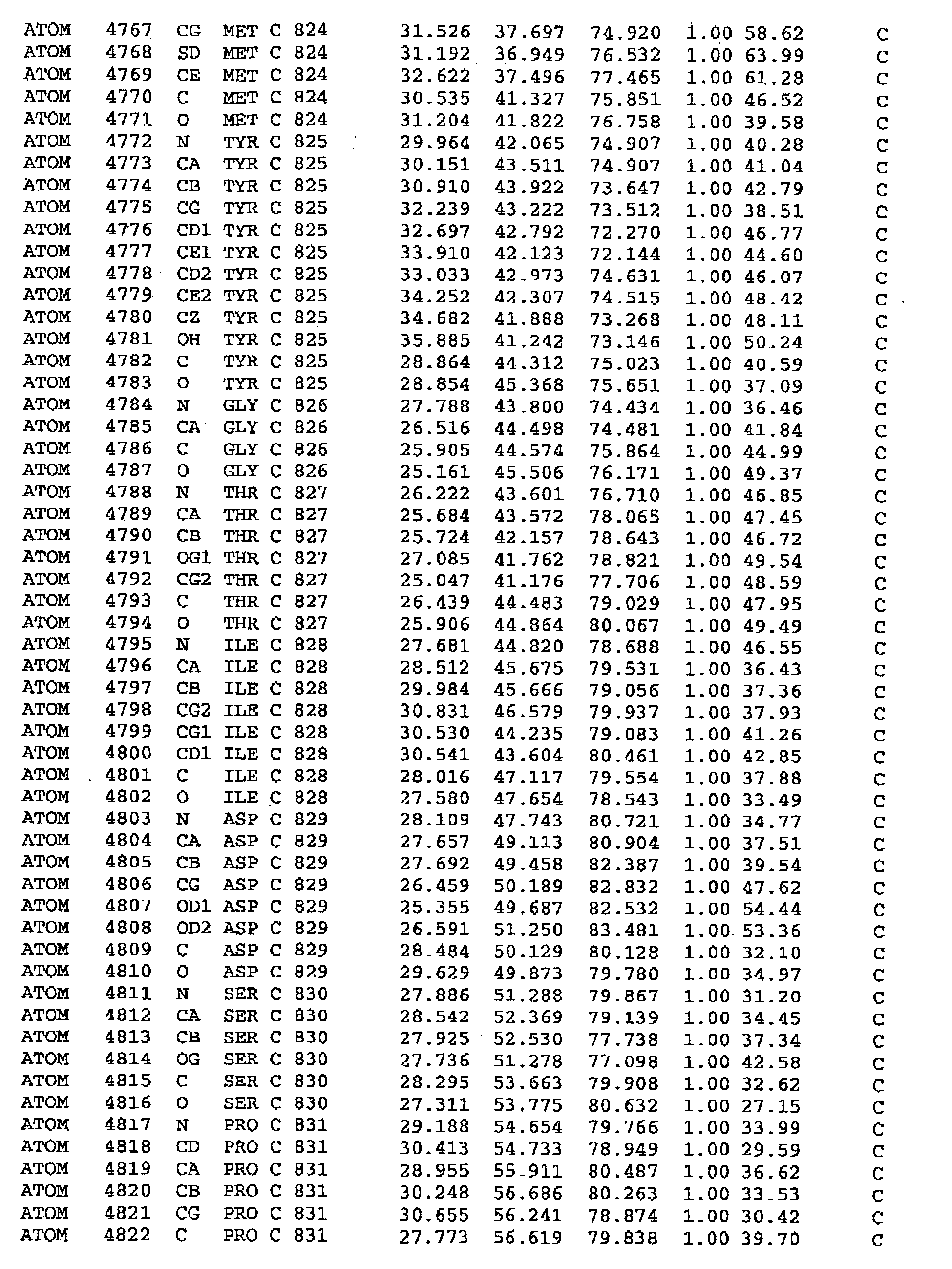

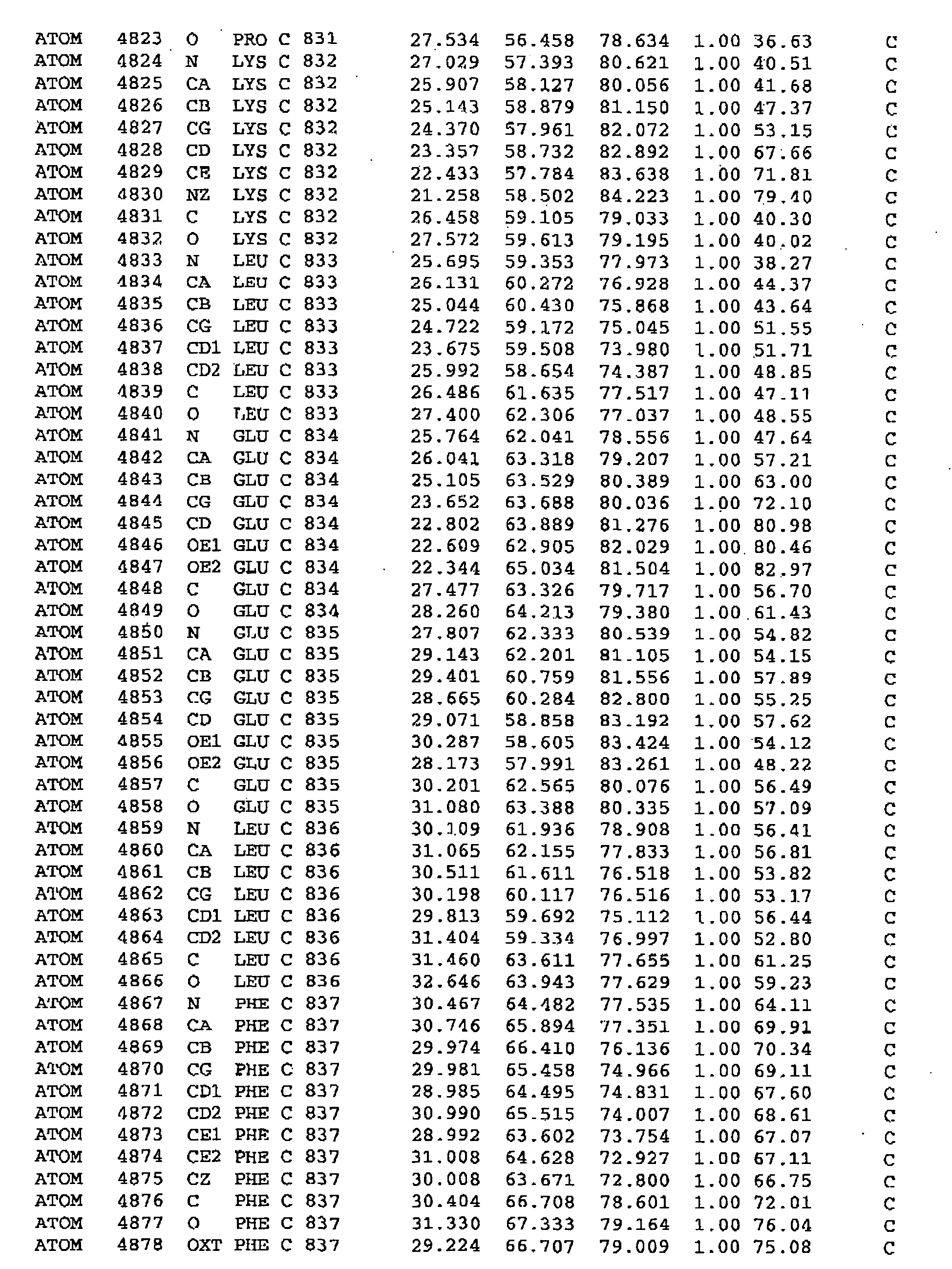

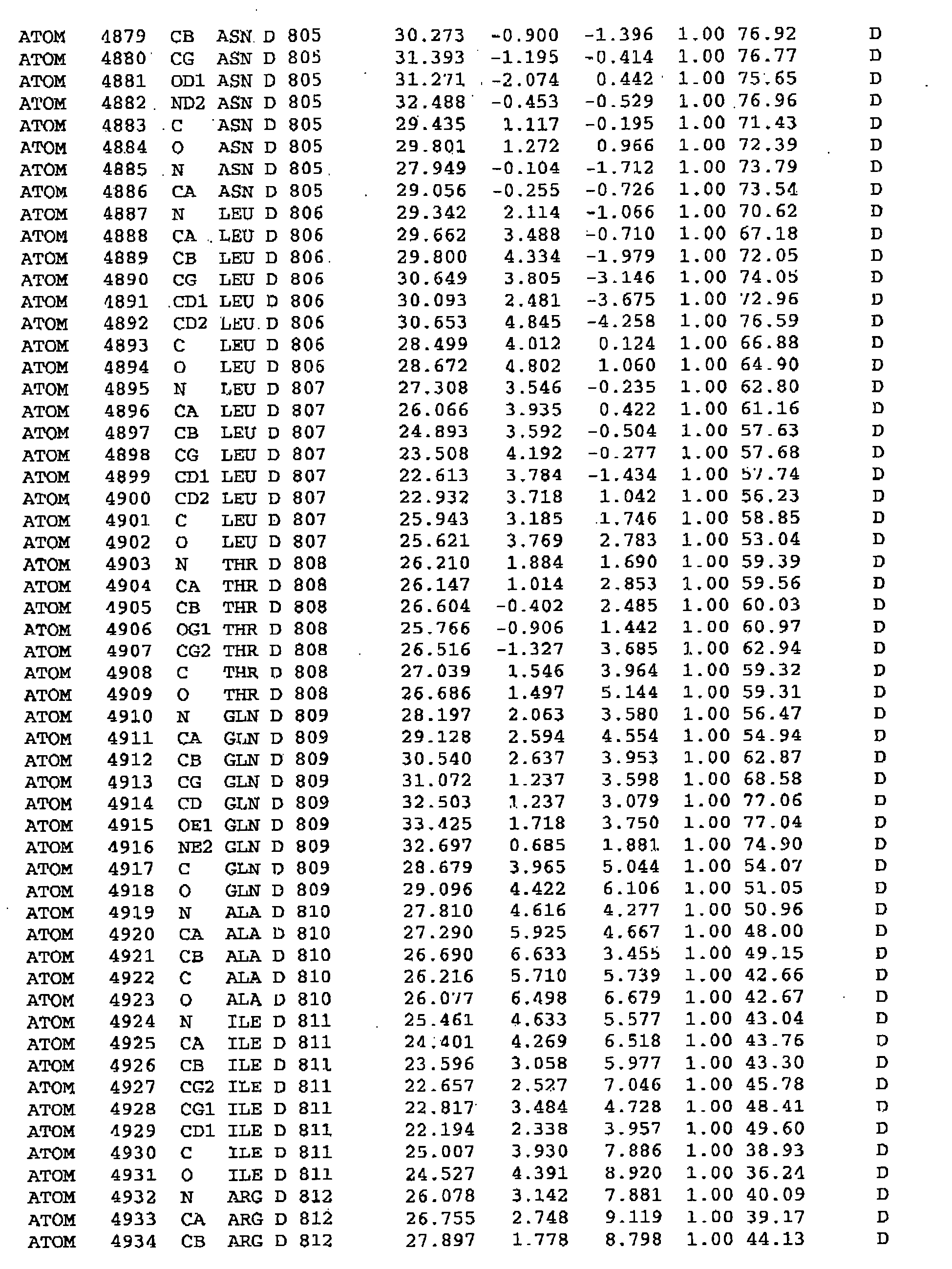

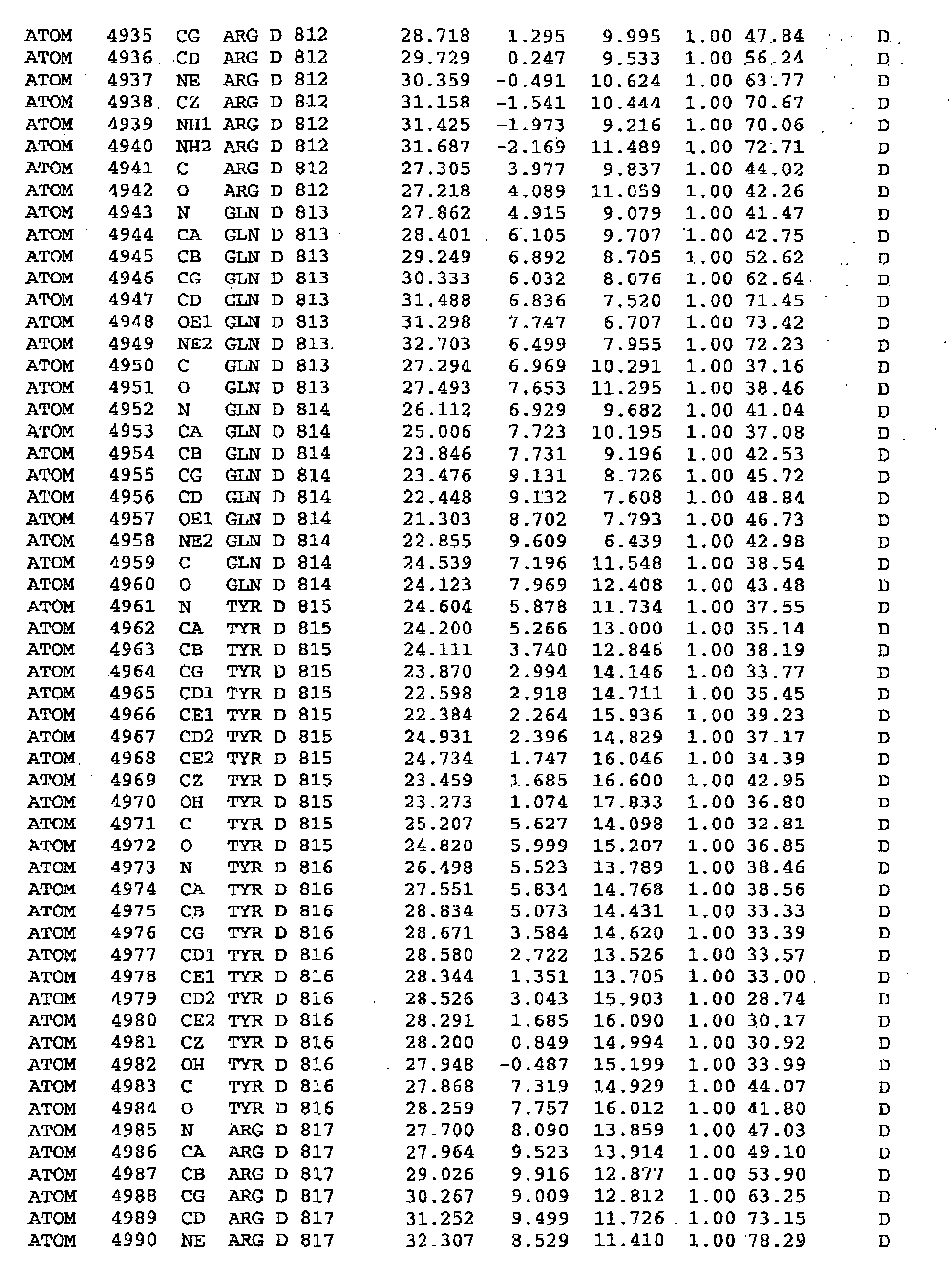

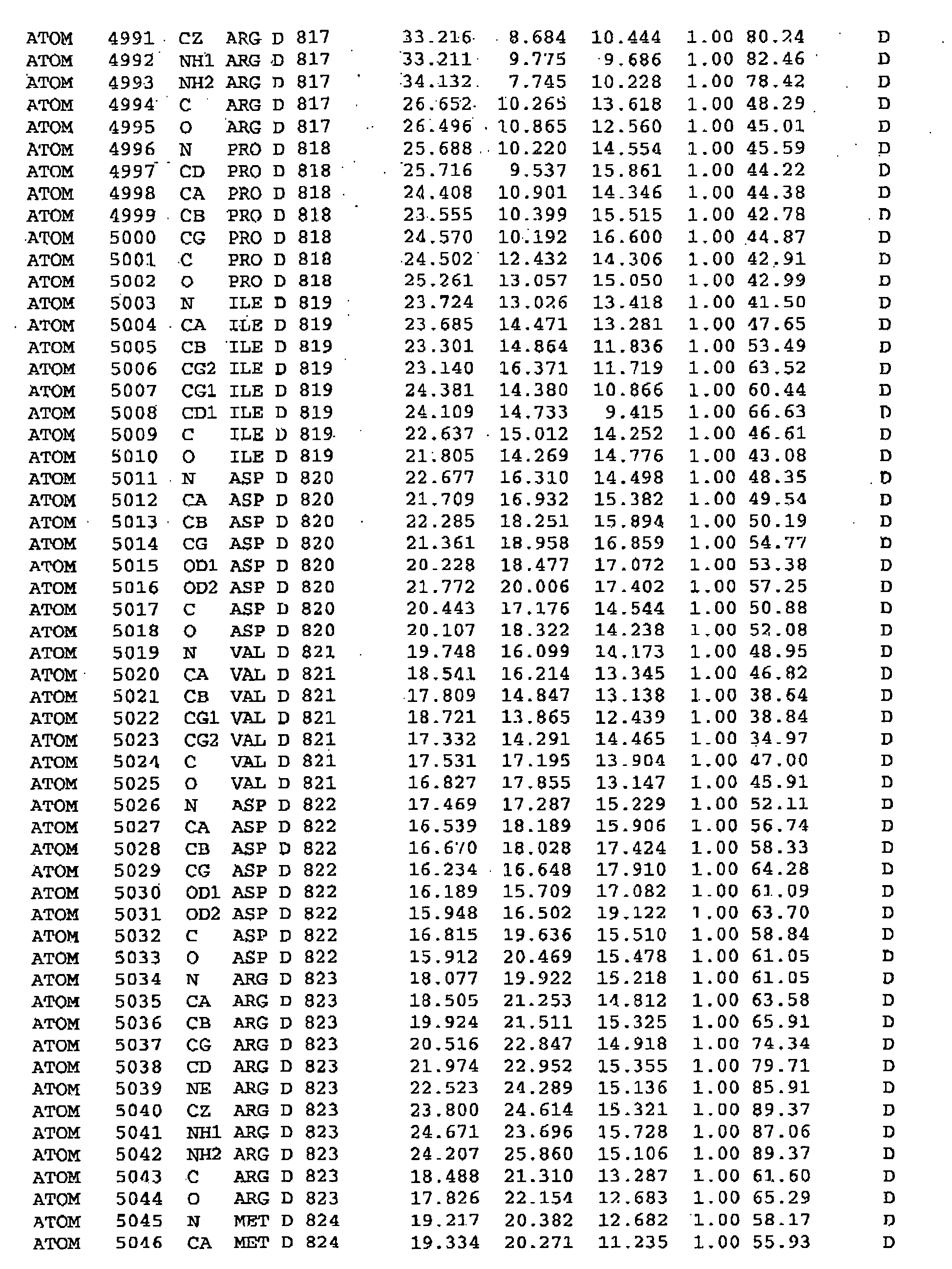

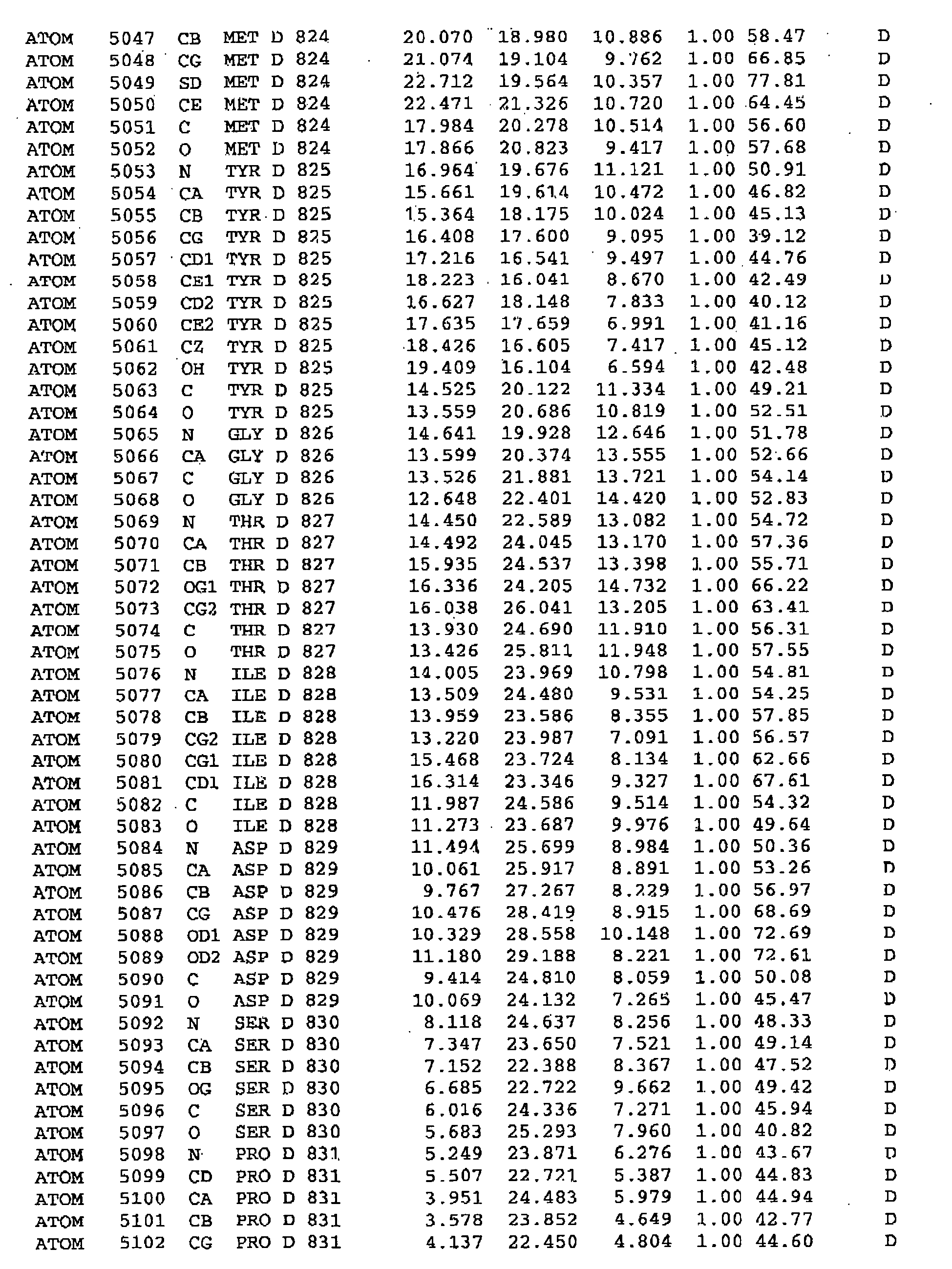

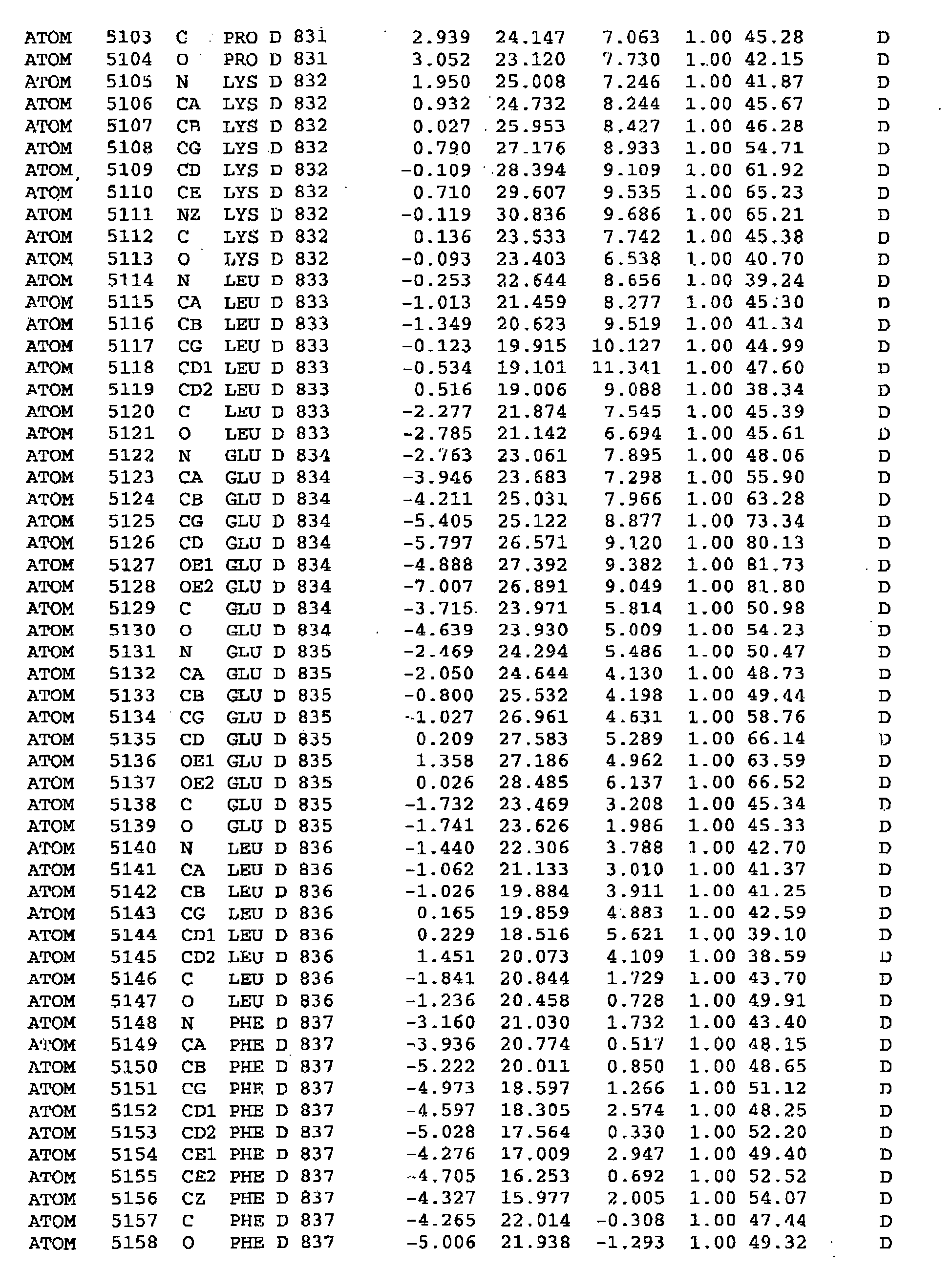

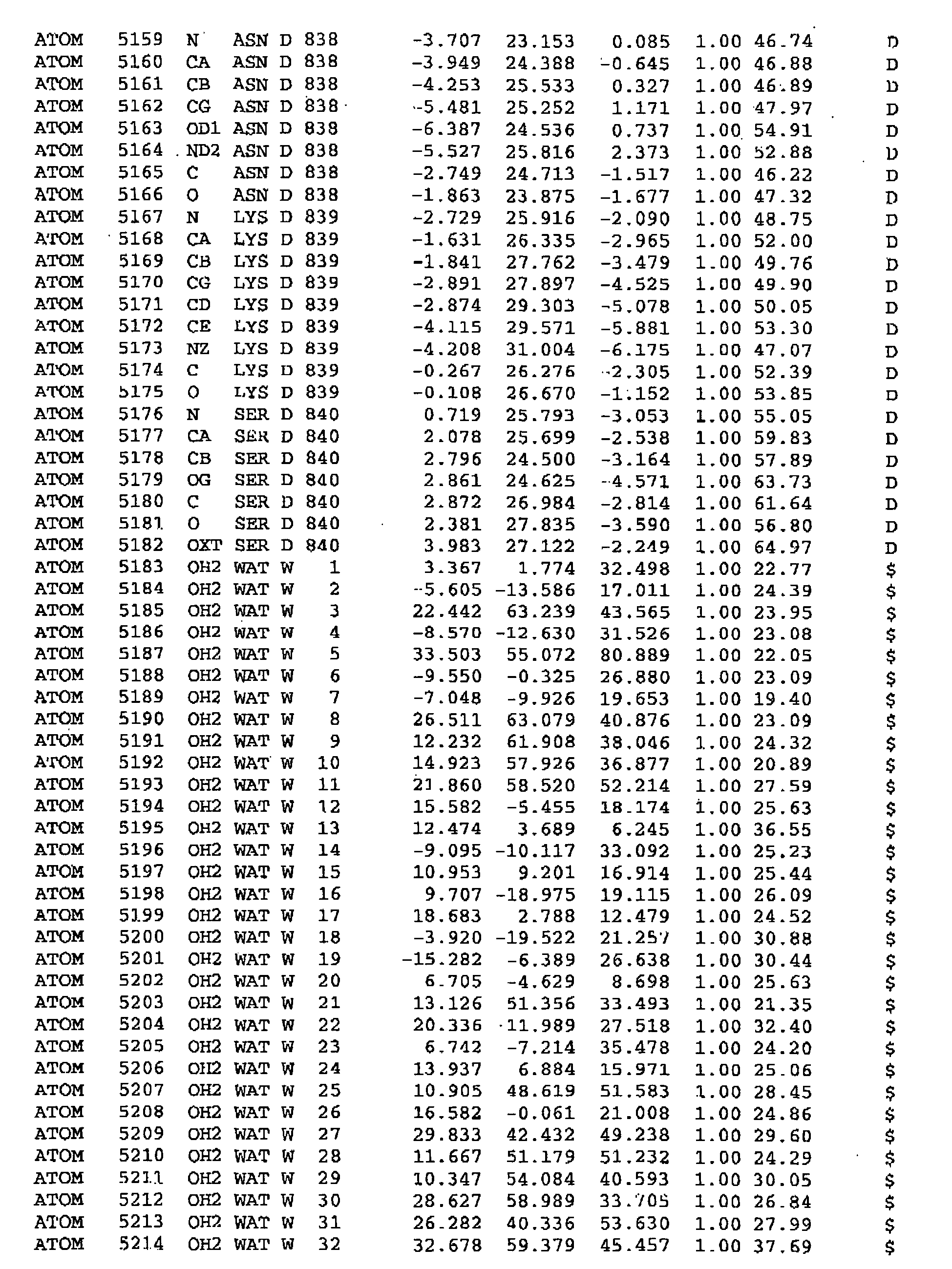

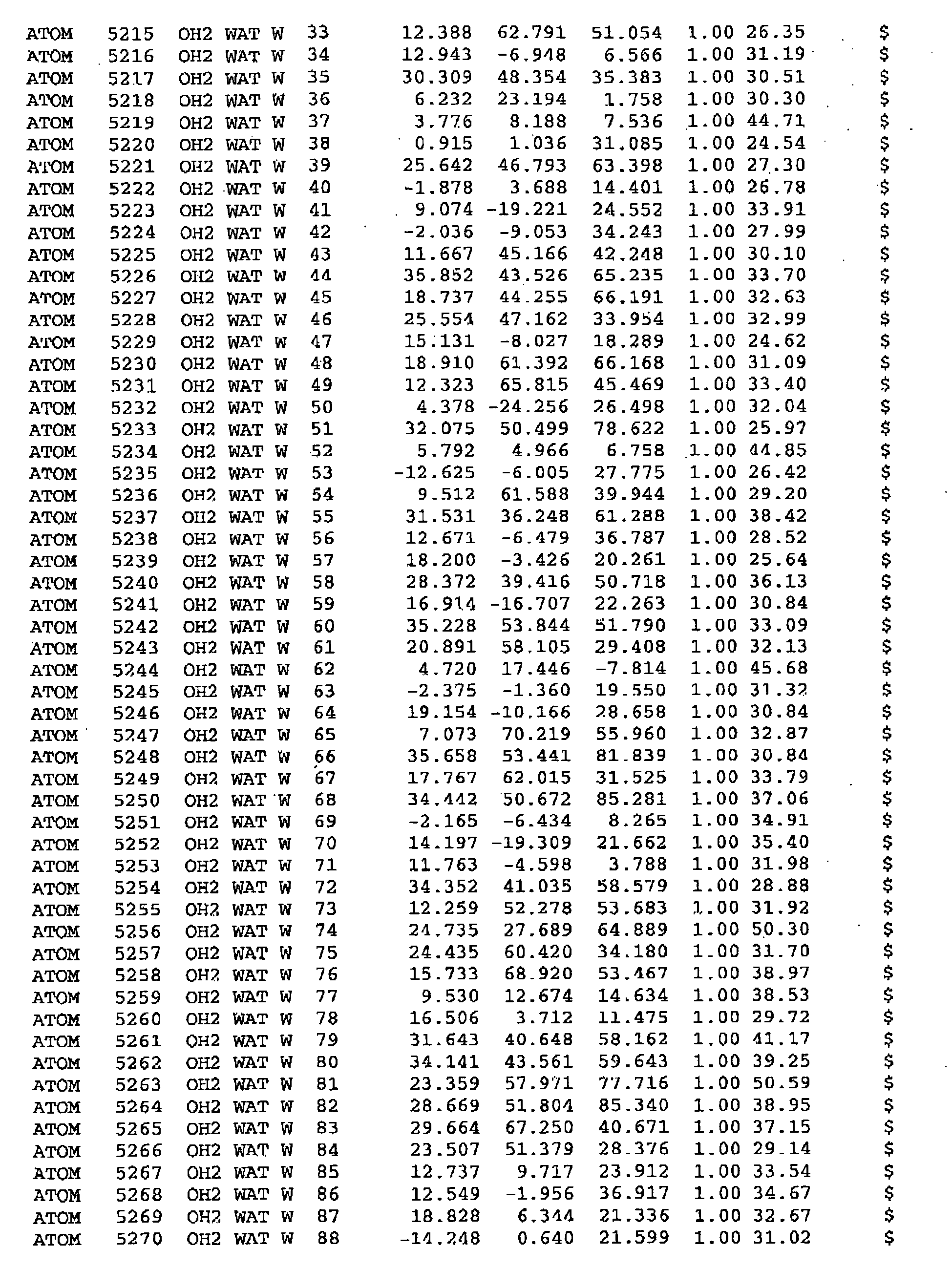

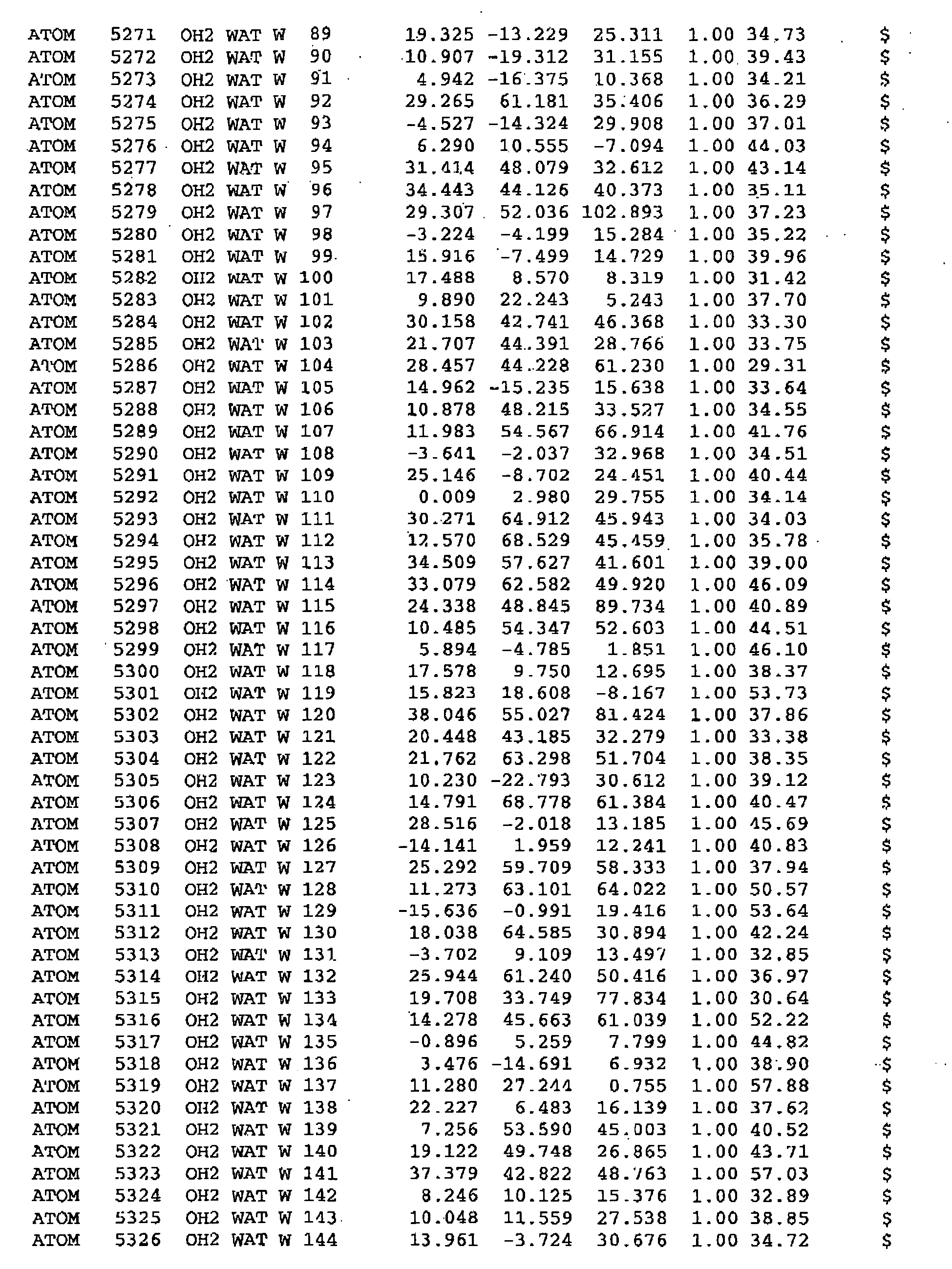

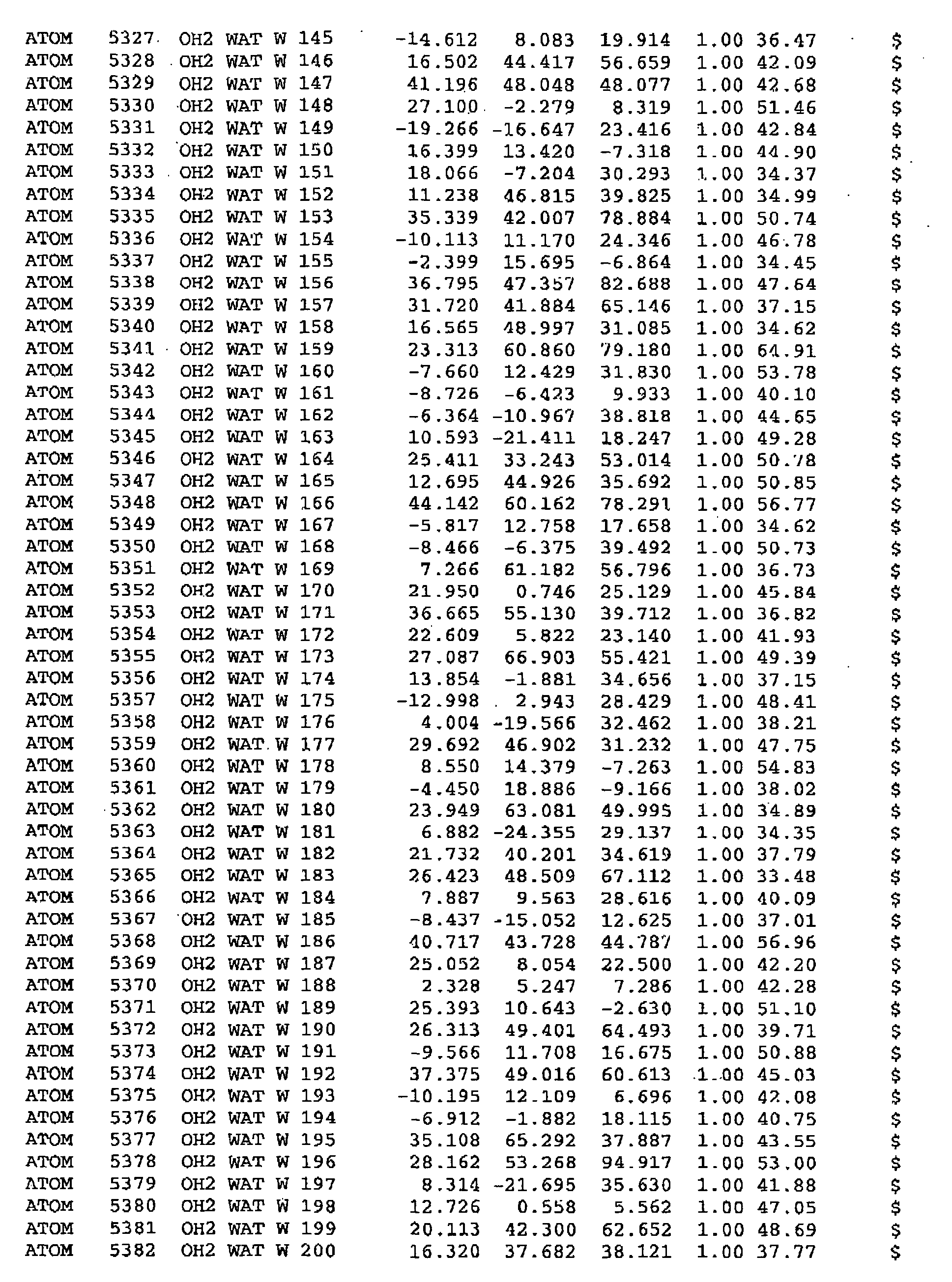

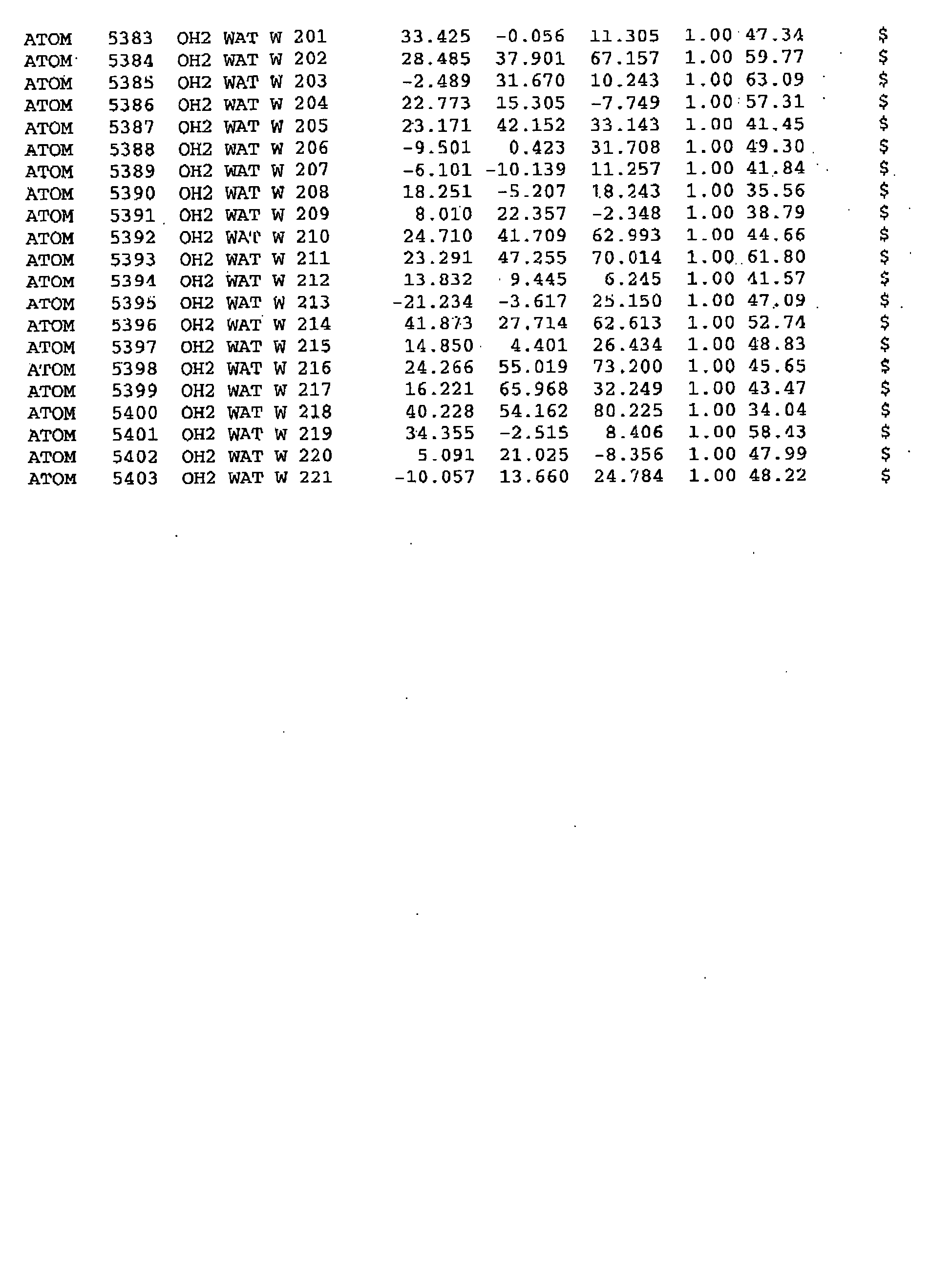

## Claims

**1.** A crystallizable composition comprising an Aurora-B-like polypeptide complexed with an INCENP-like polypeptide.

**2.** The composition according to claim 1 wherein the Aurora-B-like polypeptide is an Aurora-B kinase domain polypeptide or an Aurora-B kinase domain-like polypeptide.

**3.** The composition according to claim 1 or 2, wherein the Aurora-B-like polypeptide is a Xenopus laevis Aurora-B kinase like polypeptide.

**4.** The composition of claim 3, wherein the INCENP-like polypeptide is a Xenopus laevis INCENP-like polypeptide.

**5.** The composition according to claim 3, wherein the Aurora-B-like polypeptide has the amino acid sequence shown in SEQ ID NO:1.

**6.** The composition according to claim 3, wherein the Aurora-B-like polypeptide has the amino acid sequence shown in SEQ ID NO:2.

**7.** The composition according to any one of claims 1 to 6, wherein the INCENP-like polypeptide has the amino acid sequence shown in SEQ ID NO:3.

**8.** The composition according to any one of claims 1 to 6, wherein the INCENP-like-polypeptide has the amino acid sequence shown in SEQ ID NO:4.

**9.** A composition according to claim 4, wherein the Aurora-B-like polypeptide has the amino acid sequence shown in SEQ ID NO:1 and the INCENP-like polypeptide has the amino acid sequence shown in SEQ ID NO:3.

**10.** A composition according to claim 4, wherein the Aurora-B-like polypeptide has the amino acid sequence shown in SEQ ID NO:2 and INCENP-like polypeptide has the amino acid sequence shown in SEQ ID NO:3.

**11.** A crystallizable composition according to claim 1 obtainable by coexpression of an Aurora-B-like polypeptide and an INCENP-like polypeptide.

**12.** A crystal comprising an Aurora-B-like polypeptide complexed with an INCETTP-like polypeptide.

**13.** The crystal according to claim 12, wherein the Aurora-B-like polypeptide is an Aurora-B kinase domain polypeptide or an Aurora-B kinase domain-like polypeptide.

**14.** The crystal according to claim 12 or 13, wherein the Aurora-B-like polypeptide is Xenopus lacvis Aurora-B kinase like polypeptide.

**14.** The crystal of claim 14, wherein the TNCENP-like polypeptide is a Xenopus lae, INCENP-like polypeptide.

**15.** A crystal according to any one of claims 12 to 14, wherein the Aurora-B-like polypeptide has the amino acid sequence shown in SEQ ID NO:1.

**16.** A crystal according to any one of claims 12 to 14, wherein the Aurora-B-like polypeptide has the amino acid sequence shown in SEQ ID NO:2.

**17.** A crystal according to any one of claims 12 to 16, wherein the INCENP-like polypeptide has the amino acid sequence shown in SEQ ID NO:3.

**18.** A crystal according to any one of claims 12 to 16, wherein the INCENP-like-polypeptide has the amino acid sequence shown in SEQ ID NO:4.

**19.** A crystal according to claim 14, wherein the Aurora-B-likc polypeptide has the amino acid sequence shown in SEQ TD NO: 1 and the ThTCENP-like polypeptide has the amino acid sequence shown in SEQ ID NO: 3.

**20.** A crystal according to claim 14, wherein the Aurora-B-like polypeptide has the amino acid sequence shown in SEQ ID NO: 2 and iNCENP-like polypeptide has the amino acid sequence shown in SEQ ID NO: 3.

**21.** The crystal according to any one of claims 12 to 20, additionally comprising a ligand of Aurora-B that is different from 1NCENP or an TNC.F.NY-like polypeptide.

**22.** A method for producing a composition according to any one of claims 1 to 11, **characterized in that** an Aurora-B-like polypeptide and an INCENP-like polypeptide are co-expressed and purified.

**23.** A method for producing a crystallized complex comprising an Aurora-B-like polypeptide and an INCENP-like polypeptide comprising the steps of:
a) obtaining a crystallizable composition comprising an Auora-B-like polypeptide and an INCENP-like polypeptide, and
b) subjecting said composition to conditions which promote crystallization.

**24.** A method of obtaining structural information about a molecule or a molecular complex of unknown structure comprising the steps of:
a) providing the structure coordinates as set forth in Tables 2-4,
b) generating X-ray diffraction data from the crystallized molecule or molecular complex
c) applying at least a portion of the structure coordinates set forth in Tables 2-4 to said X-ray diffraction pattern to generate a three-dimensional electron density map of at least a portion of the molecule or molecular complex.

**25.** The method according to claim 24, wherein the molecule or molecular complex of unknown structure comprises an Aurora-B-like polypeptide in complex with an INCENP-like peptide.

**26.** A model for at least part of the Aurora-B-like polypeptide completed with an INCENP-like polypeptide, made by using a crystal according to any one of claim 12 to 21.

**27.** A method for identifying a potential inhibitor of Aurora B comprising the steps of:
a) providing the atomic coordinates of Aurora-B amino acids forming the binding sites of Aurora-B depicted in Figure 8 or 9 and as set forth in Tables 2-4 ± a root mean square deviation from the backbone atoms of said amino acids of not more than 2.0 Å, to generate a three-dimensional structure of a molecule comprising an Aurora-B-like binding pocket;
b) employing said three-dimensional structure to design or select said inhibitor;
c) obtaining said inhibitor; and
d) determining the ability of said inhibitor to interact with and/or inhibit the activity of Aurora-B.

**28.** A method for determining whether a test compound is a potential inhibitor of an Aurora-B comprising the steps of:
(a) contacting an Aurora-B-like polypeptide/INCENP-like polypeptide composition with a test compound and
(b) determining
(i) the interaction of said test compound with said Aurora-B-like polypeptide/INCENP-like polypeptide composition and/or
(ii) the effect of said compound on the kinase activity or the ATP-consuming activity of said composition.

**29.** A method according to claim 28, wherein the Aurora-B/INCENP composition is substantially pure.

**30.** A peptide having the amino acid sequence shown in SEQ ID NO: 5 or SEQ ID NO: 6, or a multimer thereof.


SEQUENCE LISTING

<110> Boehringer Ingelheim International GmbH

<120> Crystal structure of an Aurora-B/INCENP complex

<130> Case 12-0241

<160> 7

<170> PatentIn version 3.1

<210> 1

<211> 302

<212> PRT

<213> Xenopus laevis

<400> 1

Ser Ser Ser Val Pro Gly Arg Val Ala Val Ser Thr Glu Met Pro Ser
1               5                   10                  15

Gln Asn Thr Ala Leu Ala Glu Met Pro Lys Arg Lys Phe Thr Ile Asp
            20                  25                  30

Asp Phe Asp Ile Gly Arg Pro Leu Gly Lys Gly Lys Phe Gly Asn Val
        35                  40                  45

Tyr Leu Ala Arg Glu Lys Gln Asn Lys Phe Ile Met Ala Leu Lys Val
    50                  55                  60

Leu Phe Lys Ser Gln Leu Glu Lys Glu Gly Val Glu His Gln Leu Arg
65                  70                  75                  80

Arg Glu Ile Glu Ile Gln Ser His Leu Arg His Pro Asn Ile Leu Arg
                85                  90                  95

Met Tyr Asn Tyr Phe His Asp Arg Lys Arg Ile Tyr Leu Met Leu Glu
            100                 105                 110

Phe Ala Pro Arg Gly Glu Leu Tyr Lys Glu Leu Gln Lys His Gly Arg
        115                 120                 125

Phe Asp Glu Gln Arg Ser Ala Thr Phe Met Glu Glu Leu Ala Asp Ala
    130                 135                 140

Leu His Tyr Cys His Glu Arg Lys Val Ile His Arg Asp Ile Lys Pro
145                 150                 155                 160

Glu Asn Leu Leu Met Gly Tyr Lys Gly Glu Leu Lys Ile Ala Asp Phe
                165                 170                 175

Gly Trp Ser Val His Ala Pro Ser Leu Arg Arg Arg Thr Met Cys Gly
            180                 185                 190

Thr Leu Asp Tyr Leu Pro Pro Glu Met Ile Glu Gly Lys Thr His Asp
        195                 200                 205

Glu Lys Val Asp Leu Trp Cys Ala Gly Val Leu Cys Tyr Glu Phe Leu
    210                 215                 220

Val Gly Met Pro Pro Phe Asp Ser Pro Ser His Thr Glu Thr His Arg
225                 230                 235                 240

Arg Ile Val Asn Val Asp Leu Lys Phe Pro Pro Phe Leu Ser Asp Gly
                245                 250                 255

Ser Lys Asp Leu Ile Ser Lys Leu Leu Arg Tyr His Pro Pro Gln Arg
            260                 265                 270

Leu Pro Leu Lys Gly Val Met Glu His Pro Trp Val Lys Ala Asn Ser
        275                 280                 285

Arg Arg Val Leu Pro Pro Val Tyr Gln Ser Thr Gln Ser Lys
    290                 295                 300

<210>  2

<211>  302

<212>  PRT

<213>  Xenopus laevis

<400>  2

Ser Ser Ser Val Pro Gly Arg Val Ala Val Ser Thr Glu Met Pro Ser
1               5                   10                  15

Gln Asn Thr Ala Leu Ala Glu Met Pro Lys Arg Lys Phe Thr Ile Asp
            20                  25                  30

Asp Phe Asp Ile Val Arg Pro Leu Gly Lys Gly Lys Phe Gly Asn Val
        35                  40                  45

Tyr Leu Ala Arg Glu Lys Gln Asn Lys Phe Ile Met Ala Leu Lys Val
    50                  55                  60

Leu Phe Lys Ser Gln Leu Glu Lys Glu Gly Val Glu His Gln Leu Arg
65                  70                  75                  80

Arg Glu Ile Glu Ile Gln Ser His Leu Arg His Pro Asn Ile Leu Arg
                85                  90                  95

Met Tyr Asn Tyr Phe His Asp Arg Lys Arg Ile Tyr Leu Met Leu Glu
            100                 105                 110

Phe Ala Pro Arg Gly Glu Leu Tyr Lys Glu Leu Gln Lys His Gly Arg
        115                 120                 125

Phe Asp Glu Gln Arg Ser Ala Thr Phe Met Glu Glu Leu Ala Asp Ala
    130                 135                 140

Leu His Tyr Cys His Glu Arg Lys Val Ile His Arg Asp Ile Lys Pro
145                 150                 155                 160

Glu Asn Leu Leu Met Gly Tyr Lys Gly Glu Leu Lys Ile Ala Asp Phe
                165                 170                 175

Gly Trp Ser Val His Ala Pro Ser Leu Arg Arg Arg Thr Met Cys Gly
            180                 185                 190

Thr Leu Asp Tyr Leu Pro Pro Glu Met Ile Glu Gly Lys Thr His Asp
        195                 200                 205

Glu Lys Val Asp Leu Trp Cys Ala Gly Val Leu Cys Tyr Glu Phe Leu
    210                 215                 220

Val Gly Met Pro Pro Phe Asp Ser Pro Ser His Thr Glu Thr His Arg
225                 230                 235                 240

Arg Ile Val Asn Val Asp Leu Lys Phe Pro Pro Phe Leu Ser Asp Gly
                245                 250                 255

Ser Lys Asp Leu Ile Ser Lys Leu Leu Arg Tyr His Pro Pro Gln Arg
            260                 265                 270

Leu Pro Leu Lys Gly Val Met Glu His Pro Trp Val Lys Ala Asn Ser
        275                 280                 285

Arg Arg Val Leu Pro Pro Val Tyr Gln Ser Thr Gln Ser Lys
    290                 295                 300

<210>  3

<211>  58

<212>  PRT

<213>  Xenopus laevis

<400>  3

Asp Glu Ser Gln Pro Arg Lys Pro Ile Pro Ala Trp Ala Ser Gly Asn
1               5                   10                  15

Leu Leu Thr Gln Ala Ile Arg Gln Gln Tyr Tyr Lys Pro Ile Asp Val
            20                  25                  30

Asp Arg Met Tyr Gly Thr Ile Asp Ser Pro Lys Leu Glu Glu Leu Phe
        35                  40                  45

Asn Lys Ser Lys Pro Arg Tyr Phe Lys Arg
    50                  55

<210> 4

<211> 61

<212> PRT

<213> Xenopus laevis

<400> 4

Asp Glu Ser Gln Pro Arg Lys Pro Ile Pro Ala Trp Ala Ser Gly Asn
1               5                   10                  15

Leu Leu Thr Gln Ala Ile Arg Gln Gln Tyr Tyr Lys Pro Ile Asp Val
            20                  25                  30

Asp Arg Met Tyr Gly Thr Ile Asp Ser Pro Lys Leu Glu Glu Leu Phe
        35                  40                  45

Asn Lys Ser Lys Pro Arg Tyr Phe Lys Arg Thr Ser Ser Ala Val Trp
    50                  55                  60

His Ser Pro
65

<210> 5

<211> 13

<212> PRT

<213> Homo sapiens

<400>  5

Glu Pro Leu Glu Arg Arg Leu Ser Leu Val Pro Asp Ser
1               5                   10

<210>  6

<211>  13

<212>  PRT

<213>  Artificial sequence

<220>

<223>  Synthetic Peptide

<400>  6

Glu Pro Leu Glu Arg Arg Leu Ser Leu Val Pro Lys Met
1               5                   10

<210>  7

<211>  28

<212>  PRT

<213>  Artificial sequence

<220>

<223>  Synthetic peptide

<400>  7

Leu Arg Arg Trp Ser Leu Gly Leu Arg Arg Trp Ser Leu Gly Leu Arg
1               5                   10                  15

Arg Trp Ser Leu Gly Leu Arg Arg Trp Ser Leu Gly
            20                  25